(19) Europäisches Patentamt
European Patent Office
Office européen des brevets

(11) EP 4 671 242 A1

(12) **EUROPEAN PATENT APPLICATION**
published in accordance with Art. 153(4) EPC

(43) Date of publication:
**31.12.2025 Bulletin 2026/01**

(21) Application number: **24759720.6**

(22) Date of filing: **22.02.2024**

(51) International Patent Classification (IPC):
*C07D 471/04* (2006.01) *A61K 31/519* (2006.01)
*A61P 35/00* (2006.01) *A61P 35/02* (2006.01)
*A61P 31/14* (2006.01) *A61P 31/16* (2006.01)
*A61P 31/18* (2006.01) *A61P 31/20* (2006.01)
*A61P 31/22* (2006.01) *A61P 19/00* (2006.01)
*A61P 37/00* (2006.01) *A61P 29/00* (2006.01)

(52) Cooperative Patent Classification (CPC):
**A61K 31/519; A61P 19/00; A61P 29/00; A61P 31/00; A61P 31/14; A61P 31/16; A61P 31/18; A61P 31/20; A61P 31/22; A61P 35/00; A61P 35/02; A61P 37/00; A61P 37/06; C07D 471/04**

(86) International application number:
**PCT/CN2024/077994**

(87) International publication number:
**WO 2024/175043 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.02.2023 CN 202310155006**

(71) Applicant: **Sunshine Lake Pharma Co., Ltd.**
**Dongguan, Guangdong 523000 (CN)**

(72) Inventors:
- **LIU, Bing**
  **Dongguan, Guangdong 523871 (CN)**
- **GUAN, Mingyu**
  **Dongguan, Guangdong 523871 (CN)**
- **LIU, Lu**
  **Dongguan, Guangdong 523871 (CN)**
- **ZHANG, Yingjun**
  **Dongguan, Guangdong 523871 (CN)**
- **ZHANG, Hang**
  **Dongguan, Guangdong 523871 (CN)**
- **YU, Xinglong**
  **Dongguan, Guangdong 523871 (CN)**
- **PAN, Xiaoguang**
  **Dongguan, Guangdong 523871 (CN)**
- **XI, Yunlong**
  **Dongguan, Guangdong 523871 (CN)**
- **LI, Jiacan**
  **Dongguan, Guangdong 523871 (CN)**
- **ZHAO, Xin**
  **Dongguan, Guangdong 523871 (CN)**

(74) Representative: **Hoffmann Eitle**
**Patent- und Rechtsanwälte PartmbB**
**Arabellastraße 30**
**81925 München (DE)**

# (54) PYRIDOPYRIMIDINE DERIVATIVE AND USE THEREOF

(57) The present invention belongs to the technical field of medicine, relates to a pyridopyrimidine derivative and the use thereof, and specifically relates to a pyridopyrimidine derivative and a pharmaceutical composition comprising the compound, and the use thereof in the preparation of a medicine for treating diseases related to the PD-1/PD-L1 signaling pathway.

EP 4 671 242 A1

**Description**

## FIELD OF THE INVENTION

The present invention belongs to the technical field of medicine, and specifically relates to a pyridopyrimidine derivative and a pharmaceutical composition comprising these compounds, and a use thereof in the preparation of a medicine for treating diseases related to the PD-1/PD-L1 signaling pathway.

## BACKGROUND OF THE INVENTION

Programmed death receptor-1 (PD-1), also known as CD279, is a cell surface receptor expressed on activated T cells, natural killer T cells, B cells, and macrophages (Greenwald et, Annu.Rev. Immunol 2005, 23:515-548; Okazaki and Honjo, Trends Immunol 2006, (4):195-201). It is a type I transmembrane protein composed of 268 amino acids and belongs to the CD28 family. PD-1 is structurally composed of three parts: an extracellular immunoglobulin variable domain, a hydrophobic transmembrane region, and an intracellular domain (Parry et al, Mol Cell Biol 2005, 9543-9553). The intracellular domain includes two phosphorylation sites located in the immunoreceptor tyrosine-based inhibitory motif (ITIM) and the immunoreceptor tyrosine-based translation motif (ITSM), suggesting that PD-1 negatively regulates T cell receptor-mediated signals. As an inherent negative feedback system, PD-1 prevents T cell activation, thereby reducing autoimmunity and promoting self-tolerance. In addition, PD-1 is also believed to play a key role in suppressing antigen-specific T cell responses in diseases such as cancer and viral infection (Sharpe et al, Nat Immunol 2007, 8, 239-245; Postow et al, J. Clinical Oncol 2015, 33,1-9).

Under normal circumstances, the PD-1/PD-L1 signaling pathway can prevent excessive inflammation and autoimmune diseases induced by the immune system's excessive attack on tissues. Under abnormal conditions, such as tumor tissues and chronic HBV-infected tissues, PD-L1 is overexpressed. Overexpression of PD-1/PD-L1 and activation of the signaling pathway inhibit the activation and proliferation of functional T cells, suppress anti-tumor immune responses, and cause the immune system to lose its inhibitory effect on tumor development, thereby accelerating tumor development and deterioration. A variety of drugs have been approved for this pathway. Among them, PD-L1 monoclonal antibodies, such as Atezolizumab, have been approved for indications including urothelial carcinoma and non-small cell lung cancer, with ongoing clinical studies exploring additional oncology applications. However, compared to small-molecule drugs, biologics exhibit notable limitations in tissue penetration, pharmacokinetic properties, cost, and administration methods. Consequently, the development of small-molecule oral drugs targeting the PD-1/PD-L1 pathway remains an unmet clinical need with significant market potential.

Bristol-Myers Squibb (BMS) was among the first pharmaceutical companies to explore small-molecule PD-L1 inhibitors, disclosing a series of compounds capable of directly blocking PD-1/PD-L1 interactions in patent documents (e.g., WO2017066227 and WO2018044963) and related academic publications. Notable small-molecule inhibitors entering clinical development include Incyte Corporation's oral PD-L1 inhibitor (INCB-086550), approved for Phase I clinical trials in December 2018 for advanced solid tumors. Gilead Sciences' GS-4224, which initiated clinical trials in the U.S. on August 8, 2019. Maxinovel Pharmaceuticals' MAX-10181 is the third small-molecule PD-L1 inhibitor to enter clinical trials in the world, which initiated Phase I trials in Australia on October 10, 2019. Additionally, Chase sun Pharmaceutical's IMMH-010, licensed from the Chinese Academy of Medical Sciences, received IND approval in China in April 2020 for clinical trials.

Currently, several monoclonal antibody drugs targeting PD-1 or PD-L1 have been marketed, confirming that PD-1/PD-L1 blockers can be used for the clinical treatment of a variety of tumors. However, antibody drugs have their own characteristics, such as high production cost, poor stability, need for administration by injection, and easy immunogenicity. Small molecule drugs have the advantages of good tissue permeability, convenient storage and transportation, low production cost, non-immunogenicity and can usually be administered orally. Therefore, the development of small molecule inhibitors of PD-1/PD-L1 has significant application value and social value.

## SUMMARY OF THE INVENTION

The present invention provides a pyridopyrimidine derivative having the ability to inhibit PD-1/PD-L1 interaction, which can be used as a novel oral small molecule immunomodulator. The compounds of the present invention have good in *vivo* exposure and sustained exposure time, and are targeted to tumor tissues. They can be enriched in tumor tissues and form higher tumor tissue exposure concentrations, which helps to better exert anti-tumor activity during treatment, thereby achieving better therapeutic effects.

In one aspect, the present invention provides a compound having Formula (I) or a stereoisomer, a tautomer, an *N*-oxide, a hydrate, a solvate, a metabolite, a pharmaceutically acceptable salt or a prodrug thereof,

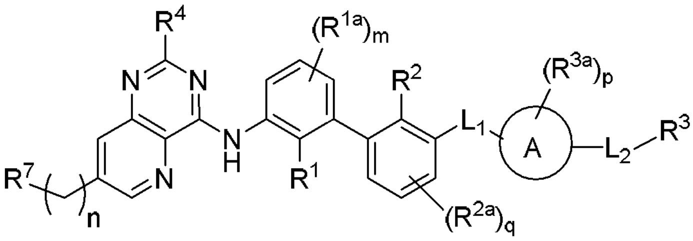

(I),

wherein:

$L_1$ is selected from a bond, $-NR^z-$, -O-, $-(CH_2)_t-$, -HC=CH-, -S- or $-SO_2-$;
$R^Z$ is H, D, -OH, $C_{1-6}$ alkyl, $C_{2-6}$ alkenyl, $C_{2-6}$ alkynyl, $C_{1-6}$ haloalkyl, $C_{1-6}$ alkoxy or $C_{1-6}$ haloalkoxy;
$R^1$, $R^2$, $R^{1a}$ and $R^{2a}$ is independently H, D, F, Cl, Br, I, $-NO_2$, -CN, -OH, $-NH_2$, $C_{1-6}$ alkyl or $C_{1-6}$ haloalkyl;
Ring A is selected from $C_{3-8}$ cycloalkyl, heterocyclic consisting of 3-8 atoms, $C_{6-10}$ aryl, or heteroaryl consisting of 5-6 atoms;
each $R^{3a}$ is independently H, D, F, Cl, Br, I, $-NO_2$, -CN, -OH, $-NH_2$, $C_{1-6}$ alkyl, $C_{2-6}$ alkenyl, $C_{2-6}$ alkynyl, $C_{1-6}$ haloalkyl, $C_{1-6}$ alkoxy or $C_{1-6}$ haloalkoxy, wherein the $C_{1-6}$ alkyl, $C_{2-6}$ alkenyl, $C_{2-6}$ alkynyl and $C_{1-6}$ alkoxy are independently and optionally substituted with 1, 2, 3 or 4 substituents selected from D, F, Cl, Br, I, $C_{1-6}$ alkyl, $C_{2-6}$ alkenyl, $C_{2-6}$ alkynyl, oxo, $-NO_2$, -CN, -OH, $-NH_2$, - $COOCH_3$ and -COOH;
$R^4$ is H, D, F, Cl, Br, I, $-NO_2$, -CN, -OH, $-NH_2$, $C_{1-6}$ alkyl, $C_{1-6}$ haloalkyl or $C_{3-8}$ cycloalkyl, wherein the $C_{1-6}$ alkyl and $C_{3-8}$ cycloalkyl are independently and optionally substituted with 1, 2, 3 or 4 substituents selected from D, F, Cl, Br, I, $C_{1-6}$ alkyl, $C_{1-6}$ haloalkyl, oxo, $-NO_2$, -CN, -OH, $-NH_2$, - $COOCH_3$ and -COOH;
$L_2$ is a bond, $-C_{1-6}$ alkylene- or $-C_{1-6}$ alkylene-$NR^w$-$C_{1-6}$ alkylene-, wherein the each $-C_{1-6}$ alkylene is independently and optionally substituted with 1, 2, 3 or 4 substituents selected from D, F, Cl, Br, I, oxo, -OH, $C_{1-6}$ alkyl and $C_{1-6}$ haloalkyl;
$R^w$ is H, D, -OH, $C_{1-6}$ alkyl, $C_{1-6}$ haloalkyl, $C_{2-6}$ alkenyl or $C_{2-6}$ alkynyl, wherein the $C_{1-6}$ alkyl, $C_{1-6}$ haloalkyl, $C_{2-6}$ alkenyl and $C_{2-6}$ alkynyl are independently and optionally substituted with 1, 2, 3 or 4 substituents selected from D, F, Cl, Br, I, oxo, -OH, $C_{1-6}$ alkyl and $C_{1-6}$ haloalkyl;
$R^3$ is $-NR^5R^6$, $C_{3-12}$ cycloalkyl or heterocyclic consisting of 3-12 atoms, wherein the $C_{3-12}$ cycloalkyl and heterocyclic consisting of 3-12 atoms are independently and optionally substituted with 1, 2, 3 or 4 substituents selected from D, halogen, $-NO_2$, -CN, -OH, $-NH_2$, oxo, $C_{1-6}$ alkyl, - $C(O)CH_3$, -C(O)OH, $-C_{1-4}$ alkylene C(O)OH, $-C(O)OCH_3$, -NHC(O)-$C_{1-4}$ alkyl, $-NHC(O)OCH_3$, - $C(O)NH_2$, $-S(O)_2CH_3$, $-S(O)_2NH_2$ and $-C(O)NHS(O)_2CH_3$;
$R^5$ and $R^6$ are each independently H, D, $C_{1-6}$ alkyl, $C_{3-10}$ cycloalkyl or heterocyclic consisting of 3-12 atoms, wherein the $C_{1-6}$ alkyl, $C_{3-10}$ cycloalkyl and heterocyclic consisting of 3-12 atoms are independently and optionally substituted with 1, 2, 3 or 4 substituents selected from D, F, Cl, Br, I, - $NO_2$, -CN, -OH, $-NH_2$, oxo, $C_{1-6}$ alkyl, $C_{1-6}$ haloalkyl, hydroxy $C_{1-6}$ alkyl, amino $C_{1-6}$ alkyl, carboxy $C_{1-6}$ alkyl, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$; or
$R^5$ and $R^6$ together with the atom to which they are attached form heterocyclic consisting of 3-12 atoms, wherein the heterocyclic consisting of 3-12 atoms optionally contains 1, 2 or 3 heteroatoms independently selected from oxygen, sulfur, or nitrogen, and is optionally substituted with 1, 2, 3 or 4 substituents independently selected from D, F, Cl, Br, I, $-NO_2$, -CN, oxo, $C_{1-6}$ alkyl, $C_{1-6}$ haloalkyl, hydroxy $C_{1-6}$ alkyl, amino $C_{1-6}$ alkyl, carboxy $C_{1-6}$ alkyl, $-OR^a$, $-C(O)R^a$, $-C(O)OR^a$, $-NR^aR^b$, - $NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and - C(O) $NR^aS(O)_2R^b$;
each of $R^a$, $R^b$, $R^c$ and $R^d$ is independently H, D, $C_{1-6}$ alkyl, $C_{3-8}$ cycloalkyl or heterocyclic consisting of 3-8 atoms, wherein the $C_{1-6}$ alkyl, $C_{3-8}$ cycloalkyl and heterocyclic consisting of 3-8 atoms are independently and optionally substituted with 1, 2, 3 or 4 substituents selected from D, F, Cl, Br, I, $C_{1-6}$ alkyl, $-NO_2$, -CN, -OH, $-NH_2$, $-C(O)CH_3$, -C(O) OH, $-C(O)OCH_3$, $-NHC(O)CH_3$, - $NHC(O)OCH_3$, $-C(O)NH_2$, $-S(O)_2CH_3$ and $-S(O)_2NH_2$;
$R^7$ is $C_{3-10}$ cycloalkyl or heterocyclic consisting of 3-12 atoms, wherein the $C_{3-10}$ cycloalkyl and heterocyclic consisting of 3-12 atoms are independently and optionally substituted with 1, 2, 3 or 4 substituents selected from D, F, Cl, Br, I, $-NO_2$, -CN, -OH, $-NH_2$, oxo, $C_{1-6}$ alkyl, $C_{1-6}$ haloalkyl, $C_{1-6}$ hydroxy alkyl, $-OR^e$, $-C(O)R^e$, $-C(O)OR^e$, $-NR^eR^f$, $-NR^eC(O)R^f$, $-NR^eC(O)OR^f$ and $-C(O)NR^eR^f$;
each of $R^e$ and $R^f$ is H, D, $C_{1-6}$ alkyl or $C_{3-8}$ cycloalkyl, wherein the $C_{1-6}$ alkyl and $C_{3-8}$ cycloalkyl are independently and optionally substituted with 1, 2, 3 or 4 substituents selected from D, F, Cl, Br, I, oxo, $-NO_2$, -CN, -OH, $-NH_2$, $-C(O)CH_3$, $-COOCH_3$ and -COOH;
each of m, n, q, p and t is independently 0, 1, 2 or 3.

In some embodiments, $R^1$, $R^2$, $R^{1a}$ and $R^{2a}$ are each independently H, D, F, Cl, Br, I, - $NO_2$, -CN, -OH, $-NH_2$, $C_{1-4}$

alkyl or $C_{1-4}$ haloalkyl;

each $R^{3a}$ is independently H, D, F, Cl, Br, I, -$NO_2$, -CN, -OH, -$NH_2$, $C_{1-4}$ alkyl, $C_{2-4}$ alkenyl, $C_{2-4}$ alkynyl, $C_{1-4}$ haloalkyl, $C_{1-4}$ alkoxy or $C_{1-4}$ haloalkoxy, wherein the $C_{1-4}$ alkyl, $C_{2-4}$ alkenyl, $C_{2-4}$ alkynyl and $C_{1-4}$ alkoxy are independently and optionally substituted with 1, 2, 3 or 4 substituents selected from D, F, Cl, Br, I, $C_{1-4}$ alkyl, $C_{2-4}$ alkenyl, $C_{2-4}$ alkynyl, oxo, -$NO_2$, -CN, -OH, -$NH_2$, - $COOCH_3$ and -COOH;
$R^4$ is H, D, F, Cl, Br, I, -$NO_2$, -CN, -OH, -$NH_2$, $C_{1-4}$ alkyl, $C_{1-4}$ haloalkyl or $C_{3-6}$ cycloalkyl, wherein the $C_{1-4}$ alkyl and $C_{3-6}$ cycloalkyl are independently and optionally substituted with 1, 2, 3 or 4 substituents selected from D, F, Cl, Br, I, $C_{1-4}$ alkyl, $C_{1-4}$ haloalkyl, oxo, -$NO_2$, -CN, -OH, -$NH_2$, - $COOCH_3$ and -COOH.

In some embodiments, $R^1$, $R^2$, $R^{1a}$ and $R^{2a}$ are each independently H, D, F, Cl, Br, I, - $NO_2$, -CN, -OH, -$NH_2$, methyl, ethyl, n-propyl, isopropyl, n-butyl, isobutyl, *sec-butyl, tert-butyl,* - $CH_2F$, -$CHF_2$, -$CF_3$, -$CH_2CHF_2$, -$CHFCH_2F$, -$CH_2CF_3$, -$CH_2Cl$, -$CHCl_2$, -$CH_2CHCl_2$, -$CH_2Br$, -$CHBr_2$ or -$CH_2CHBr_2$;

each of $R^{3a}$ is independently H, D, F, Cl, Br, I, -$NO_2$, -CN, -OH, -$NH_2$, methyl, ethyl, n-propyl, isopropyl, n-butyl, isobutyl, *sec-butyl, tert-butyl,* vinyl, propenyl, allyl, ethynyl, propargyl, 1-propynyl, 1-butynyl, 2-butynyl, 3-butynyl, -$CH_2F$, -$CHF_2$, -$CF_3$, -$CH_2CHF_2$, -$CHFCH_2F$, -$CH_2CF_3$, - $OCH_3$, -$OCH_2CH_3$, -$OCH_2CH_2CH_3$, -$OCH(CH_3)_2$, -$OCHF_2$, -$OCF_3$, -$OCH_2CHF_2$ or -$OCH_2CF_3$, wherein the methyl, ethyl, n-propyl, isopropyl, n-butyl, isobutyl, *sec-butyl, tert-butyl,* vinyl, propenyl, allyl, ethynyl, propargyl, 1-propynyl, 1-butynyl, 2-butynyl, 3-butynyl, -$OCH_3$, -$OCH_2CH_3$, - $OCH_2CH_2CH_3$ and -$OCH(CH_3)_2$ are independently and optionally substituted with 1, 2, 3 or 4 substituents selected from D, F, Cl, Br, I, methyl, ethyl, n-propyl, isopropyl, vinyl, propenyl, allyl, ethynyl, propargyl, 1-propynyl, oxo, -$NO_2$, -CN, -OH, -$NH_2$, -$COOCH_3$ and -COOH;
$R^4$ is H, D, F, Cl, Br, I, -$NO_2$, -CN, -OH, -$NH_2$, methyl, ethyl, n-propyl, isopropyl, n-butyl, isobutyl, *sec-butyl, tert-butyl,* -$CH_2F$, -$CHF_2$, -$CF_3$, -$CH_2CHF_2$, -$CHFCH_2F$, -$CH_2CF_3$, cyclopropyl, cyclobutyl, cyclopentyl or cyclohexyl, wherein the methyl, ethyl, n-propyl, isopropyl, n-butyl, isobutyl, *sec-butyl, tert-butyl,* cyclopropyl, cyclobutyl, cyclopentyl and cyclohexyl are independently and optionally substituted with 1, 2, 3 or 4 substituents selected from D, F, Cl, Br, **I,** methyl, ethyl, *n*-propyl, isopropyl, *n*-butyl, isobutyl, *sec-butyl, tert-butyl,* -$CH_2F$, -$CHF_2$, -$CF_3$, -$CH_2CHF_2$, - $CHFCH_2F$, -$CH_2CF_3$, oxo, -$NO_2$, -CN, -OH, -$NH_2$, -$COOCH_3$ and -COOH.

In some embodiments, ring A is selected from cyclopropyl, cyclobutyl, cyclopentyl, cyclohexyl, oxetanyl, azetidinyl, pyrrolidinyl, tetrahydrofuranyl, piperidinyl, piperazinyl, morpholinyl, phenyl, naphthyl, pyrrolyl, pyrazolyl, imidazolyl, triazolyl, tetrazolyl, furyl, thienyl, thiazolyl, oxazolyl, pyridinyl, pyrimidyl, pyrazinyl or pyridazinyl.

In some embodiments, $R^3$ is -$NR^5R^6$, $C_{3-10}$ cycloalkyl or heterocyclic consisting of 3-10 atoms, wherein the $C_{3-10}$ cycloalkyl and heterocyclic consisting of 3-10 atoms are independently and optionally substituted with 1, 2, 3 or 4 substituents selected from D, halogen, -$NO_2$, -CN, -OH, -$NH_2$, oxo, $C_{1-4}$ alkyl, -$C(O)CH_3$, -C(O)OH, -$C_{1-4}$ alkylene C(O) OH, -$C(O)CH_3$, -NHC(O)-$C_{1-4}$ alkyl, - $NHC(O)OCH_3$, -$C(O)NH_2$, -$S(O)_2CH_3$, -$S(O)_2NH_2$ and -$C(O)NHS(O)_2CH_3$;

$R^5$ and $R^6$ are each independently H, D, $C_{1-4}$ alkyl, $C_{3-8}$ cycloalkyl or heterocyclic consisting of 3-10 atoms, wherein the $C_{1-4}$ alkyl, $C_{3-8}$ cycloalkyl and heterocyclic consisting of 3-10 atoms are independently and optionally substituted with 1, 2, 3 or 4 substituents selected from D, F, Cl, Br, I, - $NO_2$, -CN, -OH, -$NH_2$, oxo, $C_{1-4}$ alkyl, $C_{1-4}$ haloalkyl, hydroxy $C_{1-4}$ alkyl, amino $C_{1-4}$ alkyl, carboxy $C_{1-4}$ alkyl, -$OR^a$, -$C(O)R^a$, -$C(O)OR^a$, -$NR^aR^b$, -$NR^cC(O)R^d$, -$NR^aC(O)OR^b$, -C(O) $NR^aR^b$, -$S(O)_2R^a$, -$S(O)_2NR^aR^b$, -$NR^aS(O)_2R^b$ and -$C(O)NR^aS(O)_2R^b$; or
$R^5$ and $R^6$ together with the atom to which they are attached form heterocyclic consisting of 3-10 atoms, wherein the heterocyclic consisting of 3-10 atoms optionally contains 1, 2 or 3 heteroatoms independently selected from oxygen, sulfur, or nitrogen, and is optionally substituted with 1, 2, 3 or 4 substituents independently selected from D, F, Cl, Br, I, -$NO_2$, -CN, oxo, $C_{1-4}$ alkyl, $C_{1-4}$ haloalkyl, hydroxy $C_{1-4}$ alkyl, amino $C_{1-4}$ alkyl, carboxy $C_{1-4}$ alkyl, -$OR^a$, -$C(O)R^a$, -$C(O)OR^a$, -$NR^aR^b$, - $NR^cC(O)R^d$, -$NR^aC(O)OR^b$, -$C(O)NR^aR^b$, -$S(O)_2R^a$, -$S(O)_2NR^aR^b$, -$NR^aS(O)_2R^b$ and - C(O) $NR^aS(O)_2R^b$;
each of $R^a$, $R^b$, $R^c$ and $R^d$ is independently H, D, $C_{1-4}$ alkyl, $C_{3-6}$ cycloalkyl or heterocyclic consisting of 3-6 atoms, wherein the $C_{1-4}$ alkyl, $C_{3-6}$ cycloalkyl and heterocyclic consisting of 3-6 atoms are independently and optionally substituted with 1, 2, 3 or 4 substituents selected from D, F, Cl, Br, I, $C_{1-4}$ alkyl, -$NO_2$, -CN, -OH, -$NH_2$, -$C(O)CH_3$, -C(O) OH, -$C(O)OCH_3$, -$NHC(O)CH_3$, - $NHC(O)OCH_3$, -$C(O)NH_2$, -$S(O)_2CH_3$ and -$S(O)_2NH_2$.

In some embodiments, $R^3$ is -$NR^5R^6$, $C_{5-8}$ cycloalkyl or heterocyclic consisting of 4-9 atoms, wherein the $C_{5-8}$ cycloalkyl and heterocyclic consisting of 4-9 atoms are independently and optionally substituted with 1, 2, 3 or 4 substituents selected from D, halogen, -$NO_2$, -CN, -OH, -$NH_2$, oxo, $C_{1-4}$ alkyl, -$C(O)CH_3$, -C(O)OH, -$C_{1-4}$ alkylene C(O)OH, -$C(O)OCH_3$, -NHC(O)-$C_{1-4}$ alkyl, - $NHC(O)OCH_3$, -$C(O)NH_2$, -$S(O)_2CH_3$, -$S(O)_2NH_2$ and -$C(O)NHS(O)_2CH_3$;

$R^5$ and $R^6$ are each independently H, D, $C_{1-4}$ alkyl, $C_{5-8}$ cycloalkyl or heterocyclic consisting of 4-9 atoms, wherein the $C_{1-4}$ alkyl, $C_{5-8}$ cycloalkyl and heterocyclic consisting of 4-9 atoms are independently and optionally substituted with 1, 2, 3 or 4 substituents selected from D, F, Cl, Br, I, - $NO_2$, -CN, -OH, -$NH_2$, oxo, $C_{1-4}$ alkyl, $C_{1-4}$ haloalkyl, hydroxy $C_{1-4}$ alkyl, amino $C_{1-4}$ alkyl, carboxy $C_{1-4}$ alkyl, -$OR^a$, -C(O)$R^a$, -C(O)$OR^a$, -$NR^aR^b$, -$NR^c$C(O)$R^d$, -$NR^a$C(O)$OR^b$, -C(O)$NR^aR^b$, -S$(O)_2R^a$, -S$(O)_2NR^aR^b$, -$NR^a$S$(O)_2R^b$ and -C(O)$NR^a$S$(O)_2R^b$;or

$R^5$ and $R^6$ together with the atom to which they are attached form heterocyclic consisting of 4-9 atoms, wherein the heterocyclic consisting of 4-9 atomsoptionally contains 1, 2 or 3 heteroatoms independently selected from oxygen, sulfur, or nitrogen, and is optionally substituted with 1, 2, 3 or 4 substituents independently selected from D, F, Cl, Br, I, -$NO_2$, -CN, oxo, $C_{1-4}$ alkyl, $C_{1-4}$ haloalkyl, hydroxy $C_{1-4}$ alkyl, amino $C_{1-4}$ alkyl, carboxy $C_{1-4}$ alkyl, -$OR^a$, -C(O)$R^a$, -C(O)$OR^a$, -$NR^aR^b$, - $NR^c$C(O)$R^d$, -$NR^a$C(O)$OR^b$, -C(O)$NR^aR^b$, -S$(O)_2R^a$, -S$(O)_2NR^aR^b$, -$NR^a$S$(O)_2R^b$ and - C(O)$NR^a$S$(O)_2R^b$;

each of $R^a$, $R^b$, $R^c$ and $R^d$ is independently H, D, $C_{1-4}$ alkyl, $C_{3-6}$ cycloalkyl or heterocyclic consisting of 3-6 atoms, wherein the $C_{1-4}$ alkyl, $C_{3-6}$ cycloalkyl and heterocyclic consisting of 3-6 atoms are independently and optionally substituted with 1, 2, 3 or 4 substituents selected from D, F, Cl, Br, I, $C_{1-4}$ alkyl, -$NO_2$, -CN, -OH, -$NH_2$, -C(O)$CH_3$, -C(O)OH, -C(O)$OCH_3$, -NHC(O)$CH_3$, - NHC(O)$OCH_3$, -C(O)$NH_2$, -S$(O)_2CH_3$ and -S$(O)_2NH_2$.

In some embodiments, $R^3$ is -$NR^5R^6$, cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, 2-oxa-5-azabicyclo[2.2.1]heptan-5-yl, 2-azabicyclo[2.2.1]heptyl, 2-azabicyclo[2.2.2]octyl, 3-azabicyclo[3.1.0]hexyl, 2,6-diazaspiro[3.3]heptyl, 2-oxa-6-azaspiro[3.3]heptyl, 2,6-diazaspiro[3.4]octyl, 1,7-diazaspiro[4.4]nonyl, azetidinyl, pyrrolidyl, tetrahydrofuranyl, piperidinyl, piperazinyl or morpholinyl, wherein the cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, 2-oxa-5-azabicyclo[2.2.1]heptan-5-yl, 2-azabicyclo[2.2.1]heptyl, 2-azabicyclo[2.2.2]octyl, 3-azabicyclo[3.1.0]hexyl, 2,6-diazaspiro[3.3]heptyl, 2-oxa-6-azaspiro[3.3]heptyl, 2,6-diazaspiro[3.4]octyl, 1,7-diazaspiro[4.4]nonyl, azetidinyl, pyrrolidyl, tetrahydrofuranyl, piperidinyl, piperazinyl and morpholinyl are independently and optionally substituted with 1, 2, 3 or 4 substituents selected from D, F, Cl, Br, I, -$NO_2$, -CN, -OH, -$NH_2$, oxo, methyl, ethyl, n-propyl, isopropyl, n-butyl, isobutyl, *sec-butyl, tert-butyl,* -C(O)$CH_3$, -C(O)OH, -methylene C(O)OH, -C(O)$OCH_3$, -NHC(O)$CH_3$, -NHC(O)$OCH_3$, -C(O)$NH_2$, -S$(O)_2CH_3$, -S$(O)_2NH_2$ and -C(O)NHS$(O)_2CH_3$;

each of $R^5$ and $R^6$ is independently H, D, methyl, ethyl, *n*-propyl, isopropyl, *n*-butyl, isobutyl, *sec-butyl, tert-butyl,* cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, azetidinyl, pyrrolidyl, tetrahydrofuranyl, piperidinyl, piperazinyl or morpholinyl, wherein the methyl, ethyl, *n*-propyl, isopropyl, *n*-butyl, isobutyl, *sec*-butyl, *tert-butyl,* cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, azetidinyl, pyrrolidyl, tetrahydrofuranyl, piperidinyl, piperazinyl and morpholinyl are independently and optionally substituted with 1, 2, 3 or 4 substituents selected from D, F, Cl, Br, I, -$NO_2$, -CN, -OH, - $NH_2$, oxo, methyl, ethyl, n-propyl, isopropyl, *n*-butyl, isobutyl, *sec-butyl, tert-butyl,* -$CH_2F$, -$CHF_2$, - $CF_3$, -$CH_2CHF_2$, -$CHFCH_2F$, -$CH_2CF_3$, -$CH_2OH$, -$CH_2CH_2OH$, -$CH_2NH_2$, -$CH_2CH_2NH_2$, -$CH_2COOH$, -$CH_2CH_2COOH$, -$OR^a$, -C(O)$R^a$, -C(O)$OR^a$, -$NR^aR^b$, -$NR^c$C(O)$R^d$, -$NR^a$C(O)$OR^b$, - C(O)$NR^aR^b$, -S$(O)_2R^a$, -S$(O)_2NR^aR^b$, -$NR^a$S$(O)_2R^b$ and -C(O)$NR^a$S$(O)_2R^b$; or

$R^5$ and $R^6$ together with the atoms to which they are attached form a structure selected from the following:

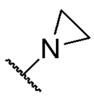 , 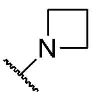 , 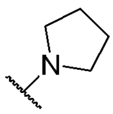 , 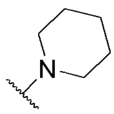 , 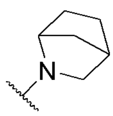 , 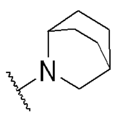 , 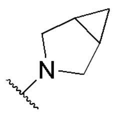 , 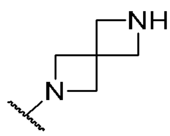 ,

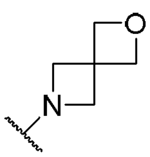 , 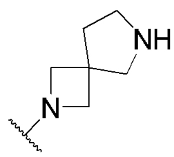 or 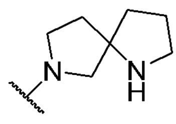 ,

wheiein the

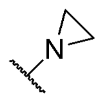, 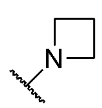, 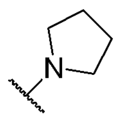, 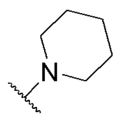,

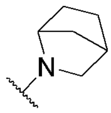, 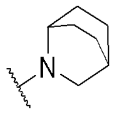, 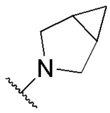, 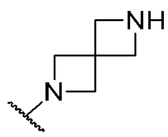, 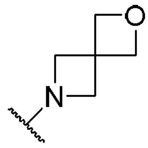, 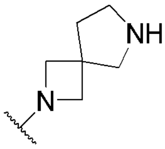 and 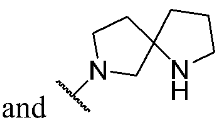

are independently and optionally substituted with 1, 2, 3 or 4 substituents selected from D, F, Cl, Br, I, - $NO_2$, -CN, oxo, methyl, ethyl, *n*-propyl, isopropyl, *n*-butyl, isobutyl, *sec-butyl, tert-butyl,* $-CH_2F$, - $CHF_2$, $-CF_3$, $-CH_2CHF_2$, $-CHFCH_2F$, $-CH_2CF_3$, $-CH_2OH$, $-CH_2CH_2OH$, $-CH_2NH_2$, $-CH_2CH_2NH_2$, - $CH_2COOH$, $-CH_2CH_2COOH$, $-OR^a$, $-C(O)R^a$, $-C(O)OR^a$, $-NR^aR^b$, $-NR^cC(O)R^d$, $-NR^aC(O)OR^b$, - $C(O)NR^aR^b$, $-S(O)_2R^a$, $-S(O)_2NR^aR^b$, $-NR^aS(O)_2R^b$ and $-C(O)NR^{a}$-$S(O)_2R^b$;

each of $R^a$, $R^b$, $R^c$ and $R^d$ is independently H, D, methyl, ethyl, *n*-propyl, isopropyl, *n*-butyl, isobutyl, *sec-butyl, tert-butyl,* cyclopropyl, cyclobutyl, cyclopentyl, cyclohexyl, azetidinyl, pyrrolidyl, tetrahydrofuranyl, piperidinyl, piperazinyl or morpholinyl, wherein the methyl, ethyl, *n*-propyl, isopropyl, *n*-butyl, isobutyl, *sec*-butyl, *tert-butyl,* cyclopropyl, cyclobutyl, cyclopentyl, cyclohexyl, azetidinyl, pyrrolidyl, tetrahydrofuranyl, piperidinyl, piperazinyl and morpholinyl are independently and optionally substituted with 1, 2, 3 or 4 substituents selected from D, F, Cl, Br, I, methyl, ethyl, *n*-propyl, isopropyl, *n*-butyl, isobutyl, *sec-butyl, tert-butyl,* $-NO_2$, -CN, -OH, $-NH_2$, - $C(O)CH_3$, -C(O)OH, $-C(O)OCH_3$, $-NHC(O)CH_3$, $-NHC(O)OCH_3$, $-C(O)NH_2$, $-S(O)_2CH_3$ and - $S(O)_2NH_2$.

In some embodiments, $L_2$ is a bond, $-C_{1-3}$ alkylene- or $-C_{1-3}$ alkylene-$NR^w$-$C_{1-3}$ alkylene-, wherein the each $-C_{1-3}$ alkylene is independently and optionally substituted with 1, 2, 3 or 4 substituents selected from D, F, Cl, Br, I, oxo, -OH, $C_{1-4}$ alkyl and $C_{1-4}$ haloalkyl;

$R^w$ is H, D, -OH, $C_{1-4}$ alkyl, $C_{1-4}$ haloalkyl, $C_{2-4}$ alkenyl or $C_{2-4}$ alkynyl, wherein the $C_{1-4}$ alkyl, $C_{1-4}$ haloalkyl, $C_{2-4}$ alkenyl and $C_{2-4}$ alkynyl are independently and optionally substituted with 1, 2, 3 or 4 substituents selected from D, F, Cl, Br, I, oxo, -OH, $C_{1-4}$ alkyl and $C_{1-4}$ haloalkyl;

$R^z$ is H, D, -OH, $C_{1-4}$ alkyl, $C_{2-4}$ alkenyl, $C_{2-4}$ alkynyl, $C_{1-4}$ haloalkyl, $C_{1-4}$ alkoxy or $C_{1-4}$ haloalkoxy;

$R^7$ is $C_{3-8}$ cycloalkyl or heterocyclic consisting of 3-10 atoms, wherein the $C_{3-8}$ cycloalkyl and heterocyclic consisting of 3-10 atoms are independently and optionally substituted with 1, 2, 3 or 4 substituents selected from D, F, Cl, Br, I, $-NO_2$, -CN, -OH, $-NH_2$, oxo, $C_{1-4}$ alkyl, $C_{1-4}$ haloalkyl, $C_{1-4}$ hydroxy alkyl, $-OR^e$, $-C(O)R^e$, $-C(O)OR^e$, $-NR^eR^f$, $-NR^eC(O)R^f$, $-NR^eC(O)OR^f$ and $-C(O)NR^eR^f$;

each of $R^e$ and $R^f$ is H, D, $C_{1-4}$ alkyl or $C_{3-6}$ cycloalkyl, wherein the $C_{1-4}$ alkyl and $C_{3-6}$ cycloalkyl are independently and optionally substituted with 1, 2, 3 or 4 substituents selected from D, F, Cl, Br, I, oxo, $-NO_2$, -CN, -OH, $-NH_2$, $-C(O)CH_3$, $-COOCH_3$ and -COOH.

In some embodiments, $L_2$ is a bond, -methylene-, -ethylene-, -methylene-$NR^w$-methylene-, or -ethylene-$NR^w$-ethylene-, wherein the -methylene- and -ethylene- are independently and optionally substituted with 1, 2, 3 or 4 substituents selected from D, F, Cl, Br, I, oxo, -OH, methyl, ethyl, *n*-propyl, isopropyl, *n*-butyl, isobutyl, *sec-butyl, tert-butyl,* $-CH_2F$, $-CHF_2$, $-CF_3$, $-CH_2CHF_2$, - $CHFCH_2F$ and $-CH_2CF_3$;

$R^w$ is H, D, -OH, methyl, ethyl, *n*-propyl, isopropyl, *n*-butyl, isobutyl, *sec-butyl, tert-butyl,* - $CH_2F$, $-CHF_2$, $-CF_3$, $-CH_2CHF_2$, $-CHFCH_2F$, $-CH_2CF_3$, vinyl, propenyl, allyl, ethynyl, propargyl, 1-propynyl, 1-butynyl, 2-butynyl or 3-butynyl, wherein the methyl, ethyl, *n*-propyl, isopropyl, *n*-butyl, isobutyl, *sec-butyl, tert-butyl,* $-CH_2F$, $-CHF_2$, $-CH_2CHF_2$, $-CHFCH_2F$, $-CH_2CF_3$, vinyl, propenyl, allyl, ethynyl, propargyl, 1-propynyl, 1-butynyl, 2-butynyl and 3-butynyl are independently and optionally substituted with 1, 2, 3 or 4 substituents selected from D, F, Cl, Br, I, oxo, -OH, methyl, ethyl, *n*-propyl, isopropyl, *n*-butyl, isobutyl, *sec-butyl, tert-butyl,* $-CH_2F$, $-CHF_2$, $-CF_3$, $-CH_2CHF_2$, - $CHFCH_2F$ and $-CH_2CF_3$;

$R^z$ is H, D, -OH, methyl, ethyl, *n*-propyl, isopropyl, *n*-butyl, isobutyl, *sec-butyl, tert-butyl,* vinyl, propenyl, allyl, ethynyl, propargyl, 1-propynyl, 1-butynyl, 2-butynyl, 3-butynyl, $-CH_2F$, $-CHF_2$, $-CF_3$, $-CH_2CHF_2$, $-CHFCH_2F$, $-CH_2CF_3$, $-OCH_3$, $-OCH_2CH_3$, $-OCH_2CH_2CH_3$, $-OCH(CH_3)_2$, $-OCHF_2$, - $OCF_3$, $-OCH_2CHF_2$ or $-OCH_2CF_3$;

$R^7$ is cyclopropyl, cyclobutyl, cyclopentyl, cyclohexyl, cycloheptyl, cyclooctyl, bicyclo[1.1.1]pentyl, bicyclo[2.1.1]hexyl, bicyclo[2.2.1]heptyl, bicyclo[2.2.2]octyl, azetidinyl, oxetanyl, pyrrolidinyl, tetrahydrofuranyl, tetrahydrothiophenyl, thiazolidinyl, pyrazolidinyl, oxazolidinyl, imidazolidinyl, isoxazolidinyl, piperidinyl, piperazinyl or morpholinyl, wherein the cyclopropyl, cyclobutyl, cyclopentyl, cyclohexyl, cycloheptyl, cyclooctyl, bicyclo[1.1.1]pentyl, bicyclo [2.1.1]hexyl, bicyclo[2.2.1]heptyl, bicyclo[2.2.2]octyl, azetidinyl, oxetanyl, pyrrolidinyl, tetrahydrofuranyl, tetrahydrothiophenyl, thiazolidinyl, pyrazolidinyl, oxazolidinyl, imidazolidinyl, isoxazolidinyl, piperidinyl, piperazinyl and morpholinyl are independently and optionally substituted with 1, 2, 3 or 4 substituents selected from D, F, Cl, Br, I, $-NO_2$, -CN, -OH, $-NH_2$, oxo, methyl, ethyl, n-propyl, isopropyl, n-butyl, isobutyl, *sec-butyl, tert-butyl,* $-CH_2F$, $-CHF_2$, $-CF_3$, $-CH_2CHF_2$, $-CHFCH_2F$, $-CH_2CF_3$, $-CH_2OH$, $-CH_2CH_2OH$, $-CH(OH)CH_3$, $-OR^e$, $-C(O)R^e$, $-C(O)OR^e$, $-NR^eR^f$, $-NR^eC(O)R^f$, $-NR^eC(O)OR^f$ and $-C(O)NR^eR^f$;

each of $R^e$ and $R^f$ is H, D, methyl, ethyl, *n*-propyl, isopropyl, *n*-butyl, isobutyl, *sec-butyl, tert-butyl,* cyclopropyl, cyclobutyl, cyclopentyl or cyclohexyl, wherein the methyl, ethyl, *n*-propyl, isopropyl, *n*-butyl, isobutyl, *sec-butyl, tert-butyl,* cyclopropyl, cyclobutyl, cyclopentyl and cyclohexyl are independently and optionally substituted with 1, 2, 3 or 4 substituents selected from D, F, Cl, Br, I, oxo, $-NO_2$, -CN, -OH, $-NH_2$, $-C(O)CH_3$, $-COOCH_3$ and -COOH.

In some embodiments, the present invention provides a compound of formula (II) or a stereoisomer, a tautomer, an N-oxide, a solvate, a metabolite, a pharmaceutically acceptable salt or a prodrug thereof,

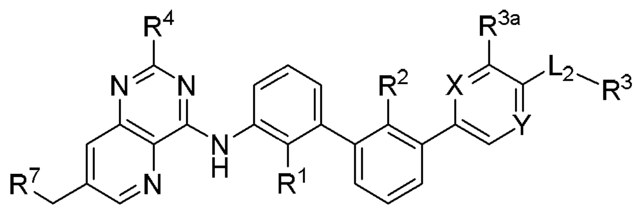

(II)

wherein, X is $CR^x$ or N;

Y is $CR^y$ or N;

$R^x$ and $R^y$ are independently H, D, F, Cl, Br, I, $-NO_2$, -CN, -OH, $-NH_2$, $C_{1-6}$ alkyl, $C_{1-6}$ haloalkyl, $C_{1-6}$ alkoxy or $C_{1-6}$ haloalkoxy;

wherein, each of $R^1$, $R^2$, $R^3$, $R^{3a}$, $R^4$, $R^7$ and $L_2$ is as defined herein.

In some embodiments, $R^x$ and $R^y$ are each independently H, D, F, Cl, Br, I, $-NO_2$, -CN, -OH, $-NH_2$, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, $-OCH_3$, $-OCH_2CH_3$, $-OCH_2CH_2CH_3$, $-OCH(CH_3)_2$, $-OCHF_2$, $-OCF_3$, $-OCH_2CHF_2$ or $-OCH_2CF_3$.

In some embodiments, the present invention provides a compound of formula (III) or a stereoisomer, a tautomer, an N-oxide, a solvate, a metabolite, a pharmaceutically acceptable salt or a prodrug thereof,

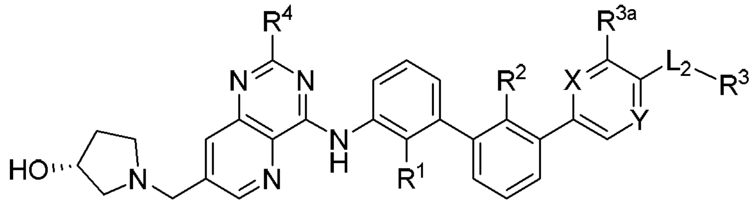

(III)

wherein, X, Y, $R^1$, $R^2$, $R^3$, $R^{3a}$ and $L_2$ are as defined herein.

In some embodiments, the present invention provides a compound of formula (IV) or a stereoisomer, a geometric iomers, a tautomer, an N-oxide, a solvate, a metabolite, a pharmaceutically acceptable salt or a prodrug thereof,

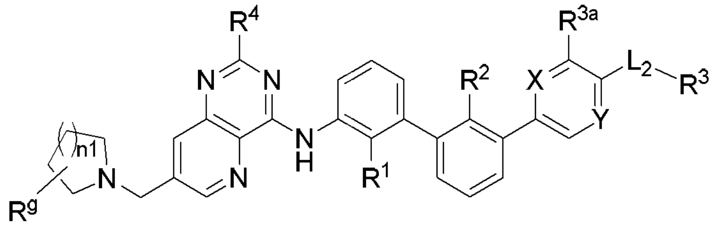

(IV)

wherein, n1 is 0, 1, 2, 3, 4 or 5;

$R^g$ is H, D, F, Cl, Br, I, $-NO_2$, -CN, -OH, $-NH_2$, oxo, $C_{1-6}$ alkyl, $C_{1-6}$ haloalkyl, $C_{1-6}$ hydroxy alkyl, $-OR^e$, $-C(O)R^e$, $-C(O)OR^e$, $-NR^eR^f$, $-NR^eC(O)R^f$, $-NR^eC(O)OR^f$ and $-C(O)NR^eR^f$;
wherein, each of X, Y, $R^1$, $R^2$, $R^3$, $R^{3a}$, $R^4$, $R^e$, $R^f$ and $L_2$ is as defined herein.

In some embodiments, $R^g$ is H, D, F, Cl, Br, I, $-NO_2$, -CN, -OH, $-NH_2$, oxo, $C_{1-4}$ alkyl, $C_{1-4}$ haloalkyl, $C_{1-4}$ hydroxy alkyl, $-OR^e$, $-C(O)R^e$, $-C(O)OR^e$, $-NR^eR^f$, $-NR^eC(O)R^f$, $-NR^eC(O)OR^f$ and $-C(O)NR^eR^f$;
wherein, each of $R^e$ and $R^f$ is as defined herein.

In some embodiments, $R^g$ is H, D, F, Cl, Br, I, $-NO_2$, -CN, -OH, $-NH_2$, oxo, methyl, ethyl, n-propyl, isopropyl, n-butyl, isobutyl, *sec*-butyl, t*ert*-butyl, $-CH_2F$, $-CHF_2$, $-CF_3$, $-CH_2CHF_2$, - $CHFCH_2F$, $-CH_2CF_3$, $-CH_2OH$, $-CH_2CH_2OH$, $-CH(OH)CH_3$, $-OR^e$, $-C(O)R^e$, $-C(O)OR^e$, $-NR^eR^f$, - $NR^eC(O)R^f$, $-NR^eC(O)OR^f$ or $-C(O)NR^eR^f$;
wherein, each of $R^e$ and $R^f$ is as defined herein.

In one aspect, provided herein is a pharmaceutical composition comprising a compound of formula (I), (II), (III) or (IV), or a stereoisomer, a tautomer, an *N*-oxide, a hydrate, a solvate, a metabolite, an eater, a pharmaceutically acceptable salt or a prodrug thereof,

In some embodiments, the pharmaceutical composition disclosed herein further comprises pharmaceutically acceptable carriers, excipients, diluents, adjuvants, vehicles, or combination thereof.

In other aspect, provided herein is use of the compound of formula (I), (II), (III) or (IV) or the pharmaceutical composition disclosed herein in the manufacture a medicament for treating a disease mediated by the PD-1/PD-L1 signaling pathway.

In some embodiments, the disease mediated by the PD-1/PD-L1 signaling pathway disclosed herein is cancer, infectious disease, or autoimmune disease.

In other embodiments, the cancer disclosed herein is selected from acute lymphoblastic leukemia, acute myeloid leukemia, chronic lymphocytic leukemia, chronic myelogenous leukemia, multiple myeloma, T-cell lymphoma, B-cell lymphoma, Waldenström's macroglobulinemia, pancreatic cancer, bladder cancer, colorectal cancer, breast cancer, prostate cancer, kidney cancer, liver cancer, lung cancer, ovarian cancer, cervical cancer, gastric cancer, esophageal cancer, head and neck cancer, melanoma, neuroendocrine cancer, CNS cancer, brain cancer, or bone cancer.

In other embodiments, the infectious disease disclosed herein is selected from acquired immunodeficiency syndrome (HIV), hepatitis A, hepatitis B, hepatitis C, hepatitis D, herpes virus infection, papillomavirus infection, or influenza virus infection.

In other embodiments, the autoimmune disease according to the present invention is selected from Chronic lymphocytic thyroiditis, hyperthyroidism, insulin-dependent diabetes mellitus, myasthenia gravis, ulcerative colitis, pernicious anemia with chronic atrophic gastritis, Goodpasture's syndrome, primary biliary cholangitis, multiple sclerosis, acute idiopathic polyneuritis, rheumatoid arthritis, systemic lupus erythematosus, systemic vasculitis, scleroderma, pemphigus, dermatomyositis, mixed connective tissue disease, or autoimmune hemolytic anemia.

In another aspect, the present invention provides a method for modulating an immune response mediated by the PD-1/PD-L1 signaling pathway in a patient, comprising administering to the subject a therapeutically effective amount of a compound of the present invention, thereby modulating the immune response in the patient.

In a further aspect, the present invention relates to methods for preparing, separating, and purifying the compounds of Formula (I), (II), (III) or (IV).

The foregoing merely summarizes certain aspects disclosed herein and is not intended to be limiting in nature. These aspects and other aspects and embodiments are described more fully below.

## DETAILED DESCRIPTION OF THE INVENTION

## DEFINITIONS AND GENERAL TERMINOLOGY

Reference will now be made in detail to certain embodiments of the invention, examples of which are illustrated in the accompanying structures and formulas. The invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application, (including but not limited to defined terms, term usage, described techniques, or the like), this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as is commonly understood by one skilled in the art to which this invention belongs. All patents and publications referred to herein are incorporated by reference in their entirety.

As used herein, the following definitions shall apply unless otherwise indicated. For purposes of this invention, the chemical elements are identified in accordance with the Periodic Table of the Elements, CAS version, and *the* Handbook of Chemistry and Physics, 75th Ed. 1994. Additionally, general principles of organic chemistry are described in "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are hereby incorporated by reference.

The grammatical articles "a", "an" and "the", as used herein, are intended to include "at least one" or "one or more" unless otherwise indicated herein or clearly contradicted by the context. Thus, the articles are used herein to refer to one or more than one (i.e. at least one) of the grammatical objects of the article. By way of example, "a component" means one or more components, and thus, possibly, more than one component is contemplated and may be employed or used in an implementation of the described embodiments.

As used herein, the term "subject" refers to an animal. Typically, the animal is a mammal. A subject also refers to for example, primates (e.g., humans, male or female), cows, sheep, goats, horses, dogs, cats, rabbits, rats, mice, fish, birds and the like. In **c**ertain embodiments, the subject is a primate. In yet other embodiments, the subject is a human.

As used herein, "patient" refers to a human (including adults and children) or other animal. In one embodiment, "patient" refers to a human.

The term "comprise" is an open expression, it means comprising the contents disclosed herein, but don't exclude other contents.

The term "stereoisomers" refers to compounds which have identical chemical constitution, but differ with regard to the arrangement of the atoms or groups in space. Stereoisomers include enantiomer, diastereomers, conformer (rotamer), geometric (cis/trans) isomer, atropisomer, etc.

The term "chiral molecule" refers to a molecule that is not superimposable on its mirror image; whereas "achiral molecule" is one that is superimposable on its mirror image.

The term "enantiomers" refer to two stereoisomers of a compound which are non-superimposable mirror images of one another.

The term "diastereomer" refers to a stereoisomer with two or more centers of chirality and whose molecules are not mirror images of one another. Diastereomers have different physical properties, e.g. melting points, boling points, spectral properties or biological activities. Mixture of diastereomers may separate under high resolution analytical procedures such as electrophoresis and chromatography such as HPLC.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994.

Many organic compounds exist in optically active forms, i.e., they have the ability to rotate the plane of plane-polarized light. **In** describing an optically active compound, the prefixes D and *L,* or *R* and S, are used to denote the absolute configuration of the molecule about its chiral center. The prefixes *d* and *l* or (+) and (-) are employed to designate the sign of rotation of plane-polarized light by the compound, with (-) or *l* meaning that the compound is levorotatory. A compound prefixed with (+) or *d* is dextrorotatory. A specific stereoisomer may be referred to as an enantiomer, and a mixture of such stereoisomers is called an enantiomeric mixture. A 50:50 mixture of enantiomers is referred to as a racemic mixture or a racemate, which may occur where there has been no stereoselection or stereospecificity in a chemical reaction or process.

Any asymmetric atom (e.g., carbon etc.) of the compound disclosed herein can be present in racemic or enantiomerically enriched, for example the (*R*)-, (S)- or *(R, S*)- configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (*R*)- or (*S*)- configuration.

Depending on the choice of the starting materials and procedures, the compounds can be present in the form of one of the possible stereoisomers or as mixtures thereof, such as racemates and diastereoisomer mixtures, depending on the number of asymmetric carbon atoms. Optically active (*R*)- and (*S*)- isomers may be prepared using chiral synthons or chiral reagents, or resolved using conventional techniques. If the compound contains a double bond, the substituent may be E or *Z* configuration. If the compound contains a disubstituted cycloalkyl, the cycloalkyl substituent may have a *cis*- or trans-configuration.

Any resulting mixtures of stereoisomers can be separated on the basis of the physicochemical differences of the constituents, into the pure or substantially pure geometric isomers, enantiomers, diastereomers, for example, by chromatography and/or fractional crystallization. *Cis* and *trans* isomers are diastereomer.

Any resulting racemates of final products or intermediates can be resolved into the optical antipodes by methods

known to those skilled in the art, e.g., by separation of the diastereomeric salts thereof. Racemic products can also be resolved by chiral chromatography, e.g., high performance liquid chromatography (HPLC) using a chiral adsorbent. Preferred enantiomers can also be prepared by asymmetric syntheses.See, for example, Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

The term "tautomer" or "tautomeric form" refers to structural isomers of different energies which are interconvertible via a low energy barrier. Where tautomerization is possible (e.g. in solution), a chemical equilibrium of tautomers can be reached. For example, proton tautomers (also known as prototropic tautomers) include interconversions via migration of a proton, such as keto-enol and imine-enamine isomerizations. Valence tautomers include interconversions by reorganization of some of the bonding electrons. A specific example of keto-enol tautomerization is the interconversion of pentane-2,4-dione and 4-hydroxypent-3-en-2-one tautomers. Another example of tautomerization is phenol-keto tautomerization. The specific example of phenol-keto tautomerisms is pyridin-4-ol and pyridin-4(1H)-one tautomerism. Unless otherwise stated, all tautomeric forms of the compounds disclosed herein are within the scope of the invention.

The term "optionally substituted with..." may be used interchangeably with the term "unsubstituted or substituted with...", meaning that the structure is either unsubstituted or substituted with one or more substituents according to the present invention. The substituents of the present invention include, but are not limited to: D, F, Cl, Br, I, $-CHF_2$, $-CF_3$, $-CH_2CF_3$, -OH, -COOH, - $CONH_2$, $-C(=O)NHCH_3$, $-C(=O)N(CH_3)_2$, -C(=O)-alkyl, -C(=O)-alkoxy, alkyl, alkenyl, alkynyl, haloalkyl, cycloalkyl, heterocyclic, aryl, heteroaryl, -alkylene-cycloalkyl, -alkylene-heterocyclyl, - alkylene-aryl, or -alkylene-heteroaryl, etc.

In general, the term "substituted" refers to the replacement of one or more hydrogen radicals in a given structure or group with the radical of a specified substituent. Unless otherwise indicated, a substituted group may have a substituent at each substitutable position of the group. When more than one position in a given structure can be substituted by one or more specified substituents selected, then the substituents may be identical or different and may substitute at each chemically feasible position in the structure.

The term "unsubstituted" means that the specified group bears no substituents.

Furthermore, what need to be explained is that the phrase "each...is independently" and "each of...and...is independently", unless otherwise stated, should be broadly understood. The specific options expressed by the same symbol are independent of each other in different groups; or the specific options expressed by the same symbol are independent of each other in same groups.

At various places in the present specification, substituents of compounds disclosed herein are disclosed in groups or in ranges. It is specifically intended that the invention include each and every individual subcombination of the members of such groups and ranges. For example, the term "$C_1$-$C_6$ alkyl" or "$C_{1-6}$ alkyl" is specifically intended to individually disclose methyl, ethyl, $C_3$ alkyl, $C_4$ alkyl, $C_5$ alkyl, and $C_6$ alkyl.

The term "D" refers to a single deuterium atom.

The term "halogen" refers to fluorine (F), chlorine (Cl), bromine (Br) or iodine (I).

The term "alkyl" or "alkyl group" refers to a saturated linear or branched-chain monovalent hydrocarbon radical of 1 to 20 carbon atoms, wherein the alkyl radical may be optionally and independently substituted with one or more substituents described herein. In some embodiments, the alkyl group contains 1-10 carbon atoms. In other embodiments, the alkyl group contains 1-8 carbon atoms. In other embodiments, the alkyl group contains 1-6 carbon atoms. In still other embodiments, the alkyl group contains 1-4 carbon atoms. In yet other embodiments, the alkyl group contains 1-3 carbon atoms.

Some non-limiting examples of the alkyl group include, methyl (Me, $-CH_3$), ethyl (Et, - $CH_2CH_3$), *n*-propyl (*n*-Pr, $-CH_2CH_2CH_3$), isopropyl (*i*-Pr, $-CH(CH_3)_2$), *n*-butyl (*n*-Bu, - $CH_2CH_2CH_2CH_3$), 2-methyl-propyl or isobutyl (*i*-Bu, $-CH_2CH(CH_3)_2$), 1-methyl-propyl or *sec*-butyl (*s*-Bu, $-CH(CH_3)CH_2CH_3$), *tert*-butyl (*t-Bu,* $-C(CH_3)_3$), *n*-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), *n*-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$, n-heptyl and n-octyl, etc. The term "alkyl" or the prefix "alk-" is inclusive of both straight chain and branched saturated carbon chain.

The term "alkenyl" refers to linear or branched-chain monovalent hydrocarbon radical of 2 to 12 carbon atoms with at least one site of unsaturation, i.e., a carbon-carbon, $sp^2$ double bond, wherein the alkenyl radical may be optionally substituted independently with one or more substituents described herein, and includes radicals having *"cis"* and *"trans"*

orientations, or alternatively, "*E*" and "*Z*" orientations. Examples of alkenyl groups include, but are not limited to, vinyl, propenyl, allyl, and the like.

The term "alkynyl" refers to a linear or branched monovalent hydrocarbon radical of 2 to 12 carbon atoms with at least one site of unsaturation, i.e., a carbon-carbon, sp triple bond, wherein the alkynyl radical may be optionally substituted independently with one or more substituents described herein. In some embodiments, the alkynyl contains 2 to 8 carbon atoms. In other embodiments, the alkynyl contains 2 to 6 carbon atoms. In still other embodiments, the alkynyl contains 2 to 4 carbon atoms. Some non-limiting examples of the alkynyl group include ethynyl ($-C\equiv CH$), propargyl ($-CH_2C\equiv CH$), 1-propynyl ($-C\equiv C-CH_3$), 1-butynyl ($-CH_2CH_2C\equiv CH$), 2-butynyl ($-CH_2C\equiv CCH_3$), 3-butynyl ($-C\equiv CCH_2CH_3$), and the like.

The term "haloalkyl" refers to an alkyl group substituted with one or more identical or different halogen atoms. Wherein the alkyl group is as defined herein. Some non-limiting examples of the haloalkyl group include trifluoromethyl, difluoromethyl, fluoromethyl, 2,2-chloroethyl, 3,3,3-trifluoropropyl, and the like. The haloalkyl may be optionally substituted with one or more substituents disclosed herein.

The term "alkoxy" refers to an alkyl group, as previously defined, attached to the parent molecular moiety via an oxygen atom. Unless otherwise specified, the alkoxy group contains 1-12 carbon atoms. In one embodiment, the alkoxy group contains 1-6 carbon atoms. In other embodiment, the alkoxy group contains 1-4 carbon atoms. In still other embodiment, the alkoxy group contains 1-3 carbon atoms. The alkoxy group may be optionally substituted with one or more substituents disclosed herein. Some non-limiting examples of the alkoxy group include, but are not limited to, methoxy (MeO, $-OCH_3$), ethoxy (EtO, $-OCH_2CH_3$), 1-propoxy (*n*-PrO, *n*-propoxy, $-OCH_2CH_2CH_3$), 2-propoxy (*i*-PrO, *i*-propoxy, $-OCH(CH_3)_2$), 1-butoxy (*n*-BuO, *n*-butoxy, $-OCH_2CH_2CH_2CH_3$), 2-methyl-1-propoxy (*i*-BuO, *i*-butoxy, $-OCH_2CH(CH_3)_2$), 2-butoxy (*s*-BuO, *s*-butoxy, $-OCH(CH_3)CH_2CH_3$), 2-methyl-2-propoxy (*t*-BuO, t-butoxy, $-OC(CH_3)_3$), 1-pentoxy (*n*-pentoxy, $-OCH_2CH_2CH_2CH_2CH_3$), 2-pentoxy ($-OCH(CH_3)CH_2CH_2CH_3$), 3-pentoxy ($-OCH(CH_2CH_3)_2$), 2-methyl-2-butoxy ($-OC(CH_3)_2CH_2CH_3$), 3-methyl-2-butoxy ($-OCH(CH_3)CH(CH_3)_2$), 3-methyl-1-butoxy ($-OCH_2CH_2CH(CH_3)_2$), 2-methyl-1-butoxy ($-OCH_2CH(CH_3)CH_2CH_3$), and the like.

The term "haloalkoxy" respectively refers to an alkoxy group, as the case may be, substituted with one or more halogen atoms, and wherein the alkoxy is defined as described herein. Examples of such groups include, but are not limited to, difluoromethoxy ($-OCHF_2$), trifluoromethoxy ($-OCF_3$), 2,2-difluoroethoxy ($-OCH_2CHF_2$), 2,2,2-trifluoroethoxy ($-OCH_2CF_3$) and the like.

The term "hydroxyalkyl" refers to an alkyl group substituted with one or more hydroxy groups. In some embodiments, hydroxyalkyl refers to an alkyl group substituted with 1, 2, 3 or 4 hydroxy groups. In some embodiments, hydroxyalkyl refers to an alkyl group substituted with one or two hydroxy groups. In some embodiments, hydroxyalkyl refers to hydroxy $C_{1-6}$ alkyl, namely a $C_{1-6}$ alkyl group substituted with one or more hydroxyl groups, preferably, "hydroxy $C_{1-6}$ alkyl" refers to a $C_{1-6}$ alkyl group substituted with one hydroxyl group. In some embodiments, hydroxyalkyl refers to hydroxy $C_{1-4}$ alkyl. In some embodiments, hydroxyalkyl refers to hydroxy $C_{1-3}$ alkyl. Some non-limiting examples of the hydroxyalkyl include, but are not limited to, $-CH_2OH$, $-CH(OH)CH_3$, $-CH_2CH_2OH$, $-CH_2CH(OH)CH_2CH_2OH$, and the like.

The term "aminoalkyl" refers to an alkyl group substituted with one or more amino groups, wherein the alkyl group is as defined herein. In some embodiments, aminoalkyl refers to amino $C_{1-6}$ alkyl, namely a $C_{1-6}$ alkyl group substituted with one or more amino groups, preferably, "amino $C_{1-6}$ alkyl" refers to a $C_{1-6}$ alkyl group substituted with one amino group. Some non-limiting examples of such groups include, aminomethyl ($-CH_2NH_2$), aminoethyl (e.g., $-CH_2CH_2NH_2$), and the like.

The term "carboxyalkyl" refers to an alkyl group substituted with one or two carboxy substituents, wherein the carboxy is -COOH and alkyl is as defined herein. In some embodiments, carboxyalkyl refers to an alkyl group substituted with one or two carboxy groups. In some embodiments, carboxyalkyl refers to carboxy $C_{1-6}$ alkyl, namely a $C_{1-6}$ alkyl group substituted with one or more carboxy groups, preferably, "carboxy $C_{1-6}$ alkyl" refers to a $C_{1-6}$ alkyl group substituted with one carboxy group. Some non-limiting examples of such groups include, $-CH_2COOH$, $-CH_2CH_2COOH$, $-CH_2CH_2CH_2COOH$, and the like.

The term "cycloalkyl" refers to a monovalent or polyvalent, saturated or partially unsaturated, non-aromatic saturated monocyclic, bicyclic, tricyclic, or tetracyclic carbon ring system containing 3-12 ring carbon atoms, wherein the bicyclic, tricyclic, or tetracyclic carbon ring systems are formed in a fused, bridged, or spiro-fused configuration. In some embodiments, the cycloalkyl contains 3 to 10 ring carbon atoms. In still other embodiments, the cycloalkyl contains 3 to 8 ring carbon atoms. In yet other embodiments, the cycloalkyl contains 3 to 6 ring carbon atoms. Examples of cycloalkyl groups include, but are not limited to: cyclopropyl, cyclobutyl, cyclopentyl, cyclohexyl, cycloheptyl, cyclooctyl, bicyclo [1.1.1]pentanyl, bicyclo[2.1.1]hexanyl, bicyclo[2.2.1]heptanyl, and bicyclo[2.2.2]octanyl, etc. The cycloalkyl group is optionally substituted with one or more substituents described herein.

The terms "heterocyclyl" and "heterocycle" are used interchangeably herein to refer to a monovalent or polyvalent, saturated or partially unsaturated, non-aromatic monocyclic, bicyclic, or tricyclic system containing 3-12 ring atoms, wherein at least one ring atom is selected from nitrogen, sulfur, and oxygen atoms. Unless otherwise specified, the heterocyclyl group may be carbon or nitrogen linked, and a $-CH_2-$ group can be optionally replaced by a $-C(=O)-$ group. The

sulfur of ring atom can be optionally oxygenized to S-oxide. The nitrogen of ring atom can be optionally oxygenized to N-oxide. The heterocyclic group includes a saturated heterocyclic group (ie, a heterocycloalkyl group) and a partially unsaturated heterocyclic group. In some embodiments, the heterocyclyl group is heterocyclyl group consisting of 3-8 atoms; in other embodiments, the heterocyclyl group is heterocyclyl group consisting of 3-6 atoms. Some non-limiting examples of the heterocyclyl group include oxiranyl, azetidinyl, oxetanyl, pyrrolidinyl, tetrahydrofuranyl, tetrahydrothiophenyl, thiazolidinyl, pyrazolidinyl, oxazolidinyl, imidazolidinyl, isoxazolidinyl, piperidinyl, piperazinyl or morpholinyl, etc. As described in the present invention, the heterocyclic group may be composed of 3-12 atoms, 3-8 atoms or 3-6 atoms, and the atoms are optionally selected from C, N, O or S, and at least one atom is N, O or S; wherein the heterocyclic group consisting of 3-8 atoms includes a heterocyclic group consisting of 3-6 atoms; and the heterocyclic group consisting of 3-6 atoms includes a heterocyclic group consisting of 3-5 atoms. Specifically, the heterocyclic groups composed of 3-6 atoms include, but are not limited to, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidinyl, piperazinyl, or morpholinyl.

The heterocyclyl may be a carbon atom or heteroatom atom. The heterocyclyl group also includes heterocyclic groups, bridged heterocyclic groups, or spiro heterocyclic groups formed by the fusion, bridging, or spiro linkage of a heterocyclic group with a saturated or partially unsaturated carbocyclic ring or heterocyclic ring. Some non-limiting examples of the heterocyclyl group include pyrrolidinyl, tetrahydrofuranyl, dihydrofuranyl, tetrahydrothienyl, tetrahydropyranyl, dihydropyranyl, tetrahydrothiopyranyl, *N*-piperidinyl, 2-piperidinyl, 3-piperidinyl, 4-piperidinyl, thioxayl, azetidinyl, oxetanyl, thietanyl, homopiperidinyl, oxiranyl, azepanyl, oxepanyl, thiepanyl, N-morpholinyl, 2-morpholinyl, 3-morpholinyl, thiomorpholinyl, *N*-piperazinyl, 2-piperazinyl, 3-piperazinyl, homopiperazinyl, 1,2,3,6-tetrahydropyrid-1-yl, oxazepinyl, diazepinyl, thiazepinyl, pyrrolin-1-yl, 2-pyrrolinyl, 3-pyrrolinyl, dihydroindolyl, 2-indolinyl, 2*H*-pyranyl, 4*H*-pyranyl, dioxanyl, 1,3-dioxolanyl, pyrazolinyl, dithianyl, dithiolanyl, dihydrothienyl, imidazolidinyl, 1,2,3,4-tetrahydroisoquinolinyl, 1,2,6-thidiazine-1,1-dioxo-2-yl, hexahydro-2*H*-[1,4]dioxin[2,3-c]pyrrolyl, quinolizinyl, 1,1-dioxothiomorpholinyl, 2,3,3*a*,7*a*-tetrahydro-1*H*-isoindolyl, isoindolinyl, 1,2,3,4-tetrahydroquinolyl, dibenzofuranyl, dihydrobenzisothiazinyl, dihydrobenzisoxazinyl, dioxolanyl, dihydropyrazinyl, dihydropyridyl, dihydropyrazolyl, dihydropyrimidinyl, dihydropyrrolyl, 1,4-dithianyl, furanonyl, furyl, imidazolidinyl, imidazolinyl, imidazolyl, imidazopyridyl, imidazothiazolyl, indazolyl, dihydroindolyl, indolizinyl, isobenzotetrahydrofuryl, isobenzotetrahydrothienyl, isobenzothienyl, isobenzodihydropyranyl, isocoumarinyl, isoindolinyl, isothiazolidinyl, isooxazolidinyl, morpholinyl, decahydroindolyl, decahydroisoindolyl, oxadiazolyl, oxazolidenedionyl, oxazolidinyl, oxazolopyridinyl, oxiranyl, 4-piperidonyl, pyrrolyl, quinolyl, tetrahydroisoquinolinyl, tetrahydrothienyl, thiomorpholinyl, thiazolidinyl, 1,3,5-trithianyl, 2-oxopyrrolidyl, oxo-1,3-thiazolidinyl, 2-piperidonyl, 3,5-dioxopiperidinyl, 1,2,3,4-tetrahydroisoquinolyl, 1,3-benzodioxlyl, 2-oxa-5-azabicyclo[2.2.1]hept-5-yl, 2-azabicyclo[2.2.1]heptanyl, 2-azabicyclo[2.2.2]octanyl, 3-azabicyclo[3.1.0]hexanyl, 2,6-diazaspiro[3.3]heptanyl, 2,6-diazaspiro[3.4]octanyl, 1,7-diazaspiro[4.4]nonanyl, 4-oxomorpholinyl, 2,6-diazaspiro[3.3]heptanyl, 2,5-diazaspiro[3.4]octanyl, 2-oxa-6-azaspiro[3.4]octanyl, 1,7-diazaspiro[4.4]nonanyl, 1,9-diazaspiro[5.5]undecanyl and pyrimidinedionyl.

The phrases "consisting of j-k ring atoms", "consisting of j-k atoms" or "j-k-membered" mean that the cyclic group is formed by j-k atoms forming the ring, the ring atoms including carbon atoms and/or heteroatoms such as O, N, S, P, etc. The j and k are each independently any non-zero natural number, and k > j; the "j-k" includes j, k, and any natural number between j and k, inclusive. For example, "consisting of 3-8 atoms or 3-8-membered", "consisting of 3-6 atoms or 3-6-membered", "consisting of 5-10 atoms or 5-10-membered", or "consisting of 5-6 atoms or 5-6-membered" mean that the cyclic group is formed by 3-8, 3-6, 5-10, or 5-6 ring atoms, respectively. The ring atoms include carbon atoms and/or heteroatoms such as O, N, S, P, etc. Specifically, for example, "heteroaryl consisting of 5-10 ring atoms" or "5-10-membered heteroaryl" denote a heteroaryl group having 5, 6, 7, 8, 9, or 10 ring atoms, wherein the 5, 6, 7, 8, 9, or 10 refers to the number of atoms forming the ring, for example, pyridinyl is a heteroaryl group composed of 6 ring atoms or a 6-membered heteroaryl.

The term "heteroatom" refers to one or more of oxygen, sulfur, nitrogen, phosphorus and silicon, including any oxidized form of nitrogen, sulfur, or phosphorus; the quaternized form of any basic nitrogen; or a substitutable nitrogen of a heterocyclic ring, for example, N (as in 3,4-dihydro-2H-pyrrolyl), NH (as in pyrrolidinyl) or NR (as in N-substituted pyrrolidinyl, R is a substituent as defined herein).

The term "aryl" refers to monocyclic, bicyclic and tricyclic aromatic unsaturated carbocyclic system containing 6-14 ring atoms, 6-12 ring atoms, or 6-10 ring atoms, wherein each ring system comprises rings of 3-7 atoms, and has one or more attachment points to the remainder of the molecule. The term "aryl" and "aromatic ring" can be used interchangeably herein. Examples of aryl ring may include phenyl, naphthyl and anthryl. The aryl group may be optionally and independently substituted with one or more substituents disclosed herein.

The term "heteroaryl" refers to monocyclic, bicyclic and tricyclic carbocyclic ring systems containing 5-12 ring atoms, 5-10 ring atoms, or 5-6 ring atoms, wherein at least one ring is aromatic, at least one ring contains 1, 2, 3, or 4 ring heteroatoms selected from nitrogen, oxygen, and sulfur, and the heteroaryl has one or more attachment points to the remainder of the molecule. When a $-CH_2-$ group is present in the heteroaryl group, the $-CH_2-$ group may be optionally replaced by - C(=O)-. Unless otherwise specified, the heteroaryl group may be attached to the rest of the molecule (eg, the main structure in the general formula) via any reasonable position (eg, C in CH, or N in NH). The term "hetreroaryl" and

"heteroaromatic ring" or "heteroaromatic compound" can be used interchangeably herein. Some non-limiting examples of hetreroaryl group included furyl, imidazolyl, pyrrolyl, pyrazolyl, pyridinyl, pyrimidyl and pyrazinyl, etc. The heteroaryl group may be optionally substituted with one or more substituents disclosed herein. In some embodiments, the heteroaryl group is heteroaryl group consisting of 5-10 atoms, meaning it comprises 1-9 ring carbon atoms and 1, 2, 3, or 4 ring heteroatoms selected from O, S and N. In other embodiments, the heteroaryl group is heteroaryl consisting of 5-6 atoms, meaning it comprises 1-5 ring carbon atoms and 1, 2, 3, or 4 ring heteroatoms selected from O, S, and N. Examples of heteroaryl groups consisting of 5-6 atoms include, but are not limited to, furyl, imidazolyl, isoxazolyl, oxazolyl, pyrrolyl, pyrazolyl, pyridinyl, pyrimidinyl, pyridazinyl, pyrazinyl, thienyl, thiazolyl, etc.

In other embodiments, some non-limiting examples of the heteroaryl system (including heteroaryl and heteroaromatic ring) include furan-2-yl, furan-3-yl, *N*-imidazolyl, imidazol-2-yl, imidazol-4-yl, imidazol-5-yl, isoxazol-3-yl, isoxazol-4-yl, isoxazol-5-yl, oxazol-2-yl, oxazol-4-yl, oxazol-5-yl, 4-methylisoxazol-5-yl, *N*-pyrrolyl, pyrrol-2-yl, pyrrol-3-yl, pyridin-2-yl, pyridin-3-yl, pyridin-4-yl, pyrimidin-2-yl, pyrimidin-4-yl, pyrimidin-5-yl, pyridazinyl (e.g., pyridazin-3-yl), thiazol-2-yl, thiazol-4-yl, thiazol-5-yl, tetrazolyl (e.g., tetrazol-5-yl), triazolyl (e.g., triazol-2-yl and triazol-5-yl), thiophen-2-yl, thiophen-3-yl, pyrazolyl (e.g., pyrazol-2-yl), isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, 1,3,4-thiadiazol-2-yl, pyrazinyl, pyrazin-2-yl, 1,3,5-triazinyl, benzo[*d*]thiazol-2-yl, imidazo[1,5-*a*]pyridin-6-yl, benzimidazolyl, benzoxazolyl, quinoxalinyl, 1,8-naphthyridinyl, benzothienyl, benzothiazolyl, indolyl (e.g., indol-2-yl), purinyl, quinolinyl (e.g., quinolin-2-yl, quinolin-3-yl and quinolin-4-yl), isoquinolyl (e.g., isoquinol-1-yl, isoquinol-3-yl or isoquinol-4-yl), benzopyrazolyl, acridinyl, benzimidazolyl, benzindolyl, benzoisoxazinyl, benzo[4,6]imidazo[1,2-*a*]pyridinyl, benzo[d]imidazo[2,1-*b*]thiazolyl, benzothiadiazolyl, benzothiazolyl, benzothienyl, benzotriazolyl, benzothiopyranyl, benzoxazinyl, benzoxazolyl, benzothiazolyl, β-carbolinyl, carbazolyl, cinnolinyl, dibenzofuranyl, imidazopyridinyl, imidazothiazolyl, indazolyl, indolizinyl, indolyl, isobenzothienyl, isoquinolinyl, isothiazolidinyl, isothiazolyl, naphthyridinyl, oxazolidinedionyl, oxazolidinyl, oxazolopyridinyl, oxazolyl, perimidinyl, phenanthridinyl, phenanthrolinyl, phenarsazinyl, phenazinyl, phenothiazinyl, phenoxazinyl, phthalazinyl, pteridinyl, pyridopyridyl, quinazolinyl, quinoxalinyl, thiophenyl, triazinyl, 2*H*-pyrrolo[3,4-*c*]pyridinyl,pyrazolo[2',1':2,3]oxazolo[4,5-*c*]pyridinyl,imidazo[2',1':2,3]thiazolo[4,5-*c*]pyridinyl,imidazo[2',1':2,3]thiazolo[4,5-*b*]pyridinyl, imidazo [2',1':2,3]thiazolo[5,4-b]pyridinyl, pyrazolo[2',1':2,3]thiazolo[4,5-*b*]pyrazinyl, *1H*-benzo[4,5]thieno[2,3-*d*]imidazolyl, 1H-benzo[4,5]thieno[2,3-*d*]imidazolyl, benzo[4,5]thieno[2,3-d]imidazolyl, imidazo[2',1':2,3]thiazolo[4,5-*b*]pyrazinyl, imidazo [2',1':2,3]thiazolo[5,4-*b*]pyridinyl, imidazo[2',1':2,3]thiazolo[4,5-*c*]pyridinyl, 1*H*-benzo[f]imidazo[4,5-b][1,4]thiazepinyl, and the like. The heteroaryl group can be substituted by a substituent disclosed herein.

The terms "-alkylene-cycloalkyl", "-alkylene-heterocyclyl", "-alkylene-aryl" and "-alkylene-heteroaryl" refer to cycloalkyl, heterocyclyl, aryl and heteroaryl groups linked through an alkylene group to the rest of the molecule, wherein the alkylene, cycloalkyl, heterocyclyl, aryl and heteroaryl groups each have the meanings as defined herein in the present invention. The alkylene, cycloalkyl, heterocyclyl, aryl and heteroaryl groups in the "-alkylene-cycloalkyl", "-alkylene-heterocyclyl", "-alkylene-aryl" and "-alkylene-heteroaryl" are each optionally substituted with one or more substituents as described herein in the present invention.

The term "pharmaceutically acceptable" means that it is physiologically tolerable and generally does not produce allergic or similar untoward reactions, such as gastrointestinal upset, dizziness, and the like, when administered to humans. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the compound is administered. Suitable pharmaceutical carriers are described in "Remington's Pharmaceutical Sciences" by E. W. Martin.

A "hydrate" refers to a compound disclosed herein or a salt thereof, which further includes a stoichiometric or non-stoichiometeric amount of water bound by non-covalent intermolecular forces, and also refers to the complex where the solvent molecule is water.

The term "solvate" refers to an association or complex of one or more solvent molecules and a compound disclosed herein. Some non-limiting examples of the solvent that form solvates include water, isopropanol, ethanol, MeOH, dimethylsulfoxide, ethyl acetate, acetic acid and ethanolamine.

An "ester" refers to an in vivo hydrolysable ester of a compound of the Formula (I), (II), (III) or (IV) containing hydroxy group. for example, a pharmaceutically acceptable ester which is hydrolysed in the human or animal body to produce the parent alcohol. The in vivo hydrolyzable ester groups of the compounds of formula (I), (II), (III) or (IV) containing a hydroxyl group include, but are not limited to, phosphate, acetoxymethoxy, 2,2-dimethylpropionyloxymethoxy, alkanoyl, benzoyl, phenylacetyl, alkoxycarbonyl, dialkylcarbamoyl, *N*-(dialkylaminoethyl)-N-alkylcarbamoyl, and the like.

An *"N-oxide"* refers to one or more than one nitrogen atoms oxidised to form an *N*-oxide, where a compound contains several amine functions. Particular examples of *N*-oxides are the N-oxides of a tertiary amine or a nitrogen atom of a nitrogen-containing heterocycle. *N*-oxides can be formed by treatment of the corresponding amine with an oxidizing agent such as hydrogen peroxide or a per-acid (e.g. a peroxycarboxylic acid) (See, Advanced Organic Chemistiy, by Jerry

March, 4th Edition, Wiley Interscience, pages). More particularly, N-oxides can be made by the procedure of L. W. Deady (Syn. Comm. 1977, 7, 509-514) in which the amine compound is reacted with m-chloroperoxybenzoic acid (MCPBA), for example, in an inert solvent such as dichloromethane.

A compound may exist in a number of different geometric isomeric, and tautomeric forms, and the compound of the Formula (I), (II), (III) or (IV) include all such forms. For the avoidance of doubt, where a compound can exist in one of several geometric isomeric or tautomeric forms and only one is specifically described or shown, all others are nevertheless embraced by Formula (I), (II), (III) or (IV).

The term "prodrug" refers to a compound that is transformed in vivo into a compound of Formula (I), (II), (III) or (IV). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic ($C_{1-24}$) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound. A thorough discussion of prodrugs is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345, all of which are incorporated herein by reference in their entireties.

Unless otherwise stated, all tautomeric forms of the compounds disclosed herein are within the scope of the invention.

Additionally, unless otherwise stated, structures depicted herein are also meant to include compounds that differ only in the presence of one or more isotopically enriched atoms. The present invention includes isotopically-labeled compounds, which are identical to those recited in Formula (I), (II), (III) or (IV), but that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine, and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labeled compounds of the present invention, for example those into which radioactive isotopes such as $^{3}H$ or $^{14}C$ are incorporated, are useful in drug and/or substrate tissue distribution assays. Isotopically labeled compound of Formula (I), (II), (III) or (IV) of this invention and prodrugs thereof can generally be prepared by carrying out the procedures disclosed in the Schemes and/or in the Examples below, by substituting a readily available isotopically labeled reagent for a non-isotopically labeled reagent.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

The compounds disclosed herein are useful in various pharmaceutically acceptable salt forms. The term "pharmaceutically acceptable salt" refers to those salt forms which would be apparent to the pharmaceutical chemist, i.e., those which are substantially nontoxic and which provide the desired pharmacokinetic properties, palatability, absorption, distribution, metabolism or excretion. Other factors, more practical in nature, which are also important in the selection, are cost of the raw materials, ease of crystallization, yield, stability, hygroscopicity and flowability of the resulting bulk drug.

A "pharmaceutically acceptable salts" refers to organic or inorganic salts of a compound disclosed herein. Pharmaceutically acceptable salts are well known in the art. For example, Berge et al., describe pharmaceutically acceptable salts in detail in J. Pharmacol Sci, 1977, 66:1-19, which is incorporated herein by reference. Examples of pharmaceutically acceptable, nontoxic salts include, but are not limited to, salts of an amino group formed with inorganic acids such as hydrochloric acid, hydrobromic acid, phosphoric acid, sulfuric acid, perchloric acid and nitric acid or with organic acids such as acetic acid, propionic acid, glycollic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid or salts obtained by using other methods used in the art such as ion exchange.

Other pharmaceutically acceptable salts include adipate, malate, 2-hydroxy propionate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, valerate, and the like. Salts derived from appropriate bases include alkali metal, alkaline earth metal, ammonium and $N^+(C_{1-4}\ alkyl)_4$ salts.

This invention also envisions the quaternization of any basic nitrogen-containing groups of the compounds disclosed herein. Water or oil soluble or dispersable products may be obtained by such quaternization. Representative alkali or alkaline earth metal salts include sodium, lithium, potassium, calcium, magnesium, ferric salt, zinc salt, copper salt, manganese salt, aluminium salt, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, $C_{1-8}$ sulfonate or aryl sulfonate. Amine salts include, but are not limited to, N,N-dibenzylethylenediamine, chloroprocaine, choline, ammonia, diethanolamine and other hydroxyalkylamine, ethylenediamine, N-methylglucamine, procaine, *N*-benzylphenethylamine, diethylamine and other alkylamine, piperazine and tris(hydroxymethyl)aminomethane. Alkali earth metal salts include, but are not limited to, barium, calcium and magnesium. Transition metal salts include, but are not limited to, zinc.

The term "protecting group" or "PG" refers to a substituent that is commonly employed to block or protect a particular functionality while reacting with other functional groups on the compound. For example, an "amino-protecting group" is a substituent attached to an amino group that blocks or protects the amino functionality in the compound. Suitable amino-protecting groups include acetyl, trifluoroacetyl, t-butoxy-carbonyl (BOC), benzyloxycarbonyl (CBZ) and 9-fluorenylmethylenoxy-carbonyl (Fmoc). Similarly, a "hydroxy-protecting group" refers to a substituent of a hydroxy group that blocks or protects the hydroxy functionality. Suitable protecting groups include acetyl and silyl. A "carboxy-protecting group" refers to a substituent of the carboxy group that blocks or protects the carboxy functionality. Common carboxy-protecting groups include $-CH_2CH_2SO_2Ph$, cyanoethyl, 2-(trimethylsilyl)ethyl, 2-(trimethylsilyl) ethoxy-methyl, 2-(p-toluenesulfonyl) ethyl, 2-(p-nitrophenylsulfonyl)-ethyl, 2-(diphenylphosphino)-ethyl, nitroethyl and the like. For a general description of protecting groups and their use, see T. W. Greene, Protective Groups in Organic Synthesis, John Wiley & Sons, New York, 1991**;** and P. J. Kocienski, Protecting Groups, Thieme, Stuttgart, 2005**.**

In the present invention, "room temperature" refers to the temperature from 10 °C to 40 °C. In some embodiments, "room temperature" refers to a temperature from 20 °C to 30 °C; in other embodiments, "room temperature" refers to 25 °C.

In the present invention, if the chemical name of the compound doesn't match the corresponding structure, the compound is characterized by the corresponding structure.

As used herein, the abbreviations for any protective groups, amino acids and other compounds are, unless otherwise indicated, in accord with their common usage, recognized abbreviations, or the IUPAC-IUB Commission on Biochemical Nomenclature (See, Biochem. 1972, 11: 942-944).

The term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both. In yet another embodiment, "treat", "treating" or "treatment" refers to preventing or delaying the onset or development or progression of the disease or disorder.

## DESCRIPTION OF COMPOUNDS OF THE INVENTION

The present invention provides a pyridopyrimidine derivative or a pharmaceutical composition thereof, which can be used as a PD-1/PD-L1 inhibitor. The present invention further relates to the use of the compound or pharmaceutical composition thereof for preparing a medicament for treating a disease and/or a disorder by inhibiting the activity of PD-1/PD-L1 with the compound. The present invention further describes a method for synthesizing the compound. The compounds of the present invention show good PD-1/PD-L1 inhibitory activity and pharmacokinetic properties.

In one aspect, the present invention provides a pyridopyrimidine derivative having the ability to inhibit PD-1/PD-L1 interaction, which is a structure as shown in formula (I) or a stereoisomer, tautomer, nitrogen oxide, hydrate, solvate, metabolite, ester, pharmaceutically acceptable salt or prodrug of the structure as shown in formula (I).

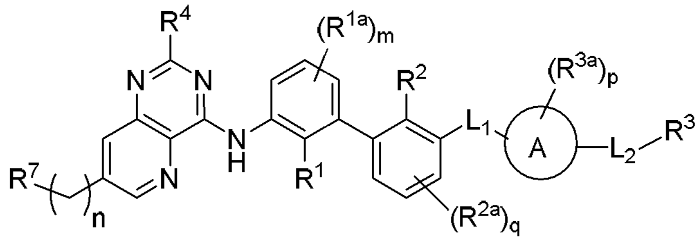

(I),

wherein: $R^1$, $R^2$, $R^{1a}$, $R^{2a}$, $R^3$, $R^{3a}$, $R^4$, $R^7$, $L_1$, $L_2$, A, m, n, q and p are as defined herein.

In some embodiments, m is 0, 1, 2 or 3.

In some embodiments, n is 0, 1, 2 or 3.

In some embodiments, q is 0, 1, 2 or 3.

In some embodiments, p is 0, 1, 2 or 3.

In some embodiments, t is 0, 1, 2 or 3.

In some embodiments, $L_1$ is selected from a bond, $-NR^z-$, -O-, $-(CH_2)_t-$, -HC=CH-, -S- or $-SO_2-$; wherein each of $R^2$ or t is as defined herein.

In some embodiments, $R^z$ is H, D, -OH, $C_{1-6}$ alkyl, $C_{2-6}$ alkenyl, $C_{2-6}$ alkynyl, $C_{1-6}$ haloalkyl, $C_{1-6}$ alkoxy or $C_{1-6}$ haloalkoxy.

In some embodiments, $R^z$ is H, D, -OH, $C_{1-4}$ alkyl, $C_{2-4}$ alkenyl, $C_{2-4}$ alkynyl, $C_{1-4}$ haloalkyl, $C_{1-4}$ alkoxy or $C_{1-4}$ haloalkoxy.

In other embodiments, $R^z$ is H, D, -OH, methyl, ethyl, *n*-propyl, isopropyl, *n*-butyl, isobutyl, *sec-butyl, tert-butyl,* vinyl, propenyl, allyl, ethynyl, propargyl, 1-propynyl, 1-butynyl, 2-butynyl, 3-butynyl, $-CH_2F$, $-CHF_2$, $-CF_3$, $-CH_2CHF_2$, $-CHFCH_2F$, $-CH_2CF_3$, $-OCH_3$, $-OCH_2CH_3$, $- OCH_2CH_2CH_3$, $-OCH(CH_3)_2$, $-OCHF_2$, $-OCF_3$, $-OCH_2CHF_2$ or $-OCH_2CF_3$.

In some embodiments, $R^1$, $R^2$, $R^{1a}$ and $R^{2a}$ are each independently H, D, F, Cl, Br, I, $- NO_2$, -CN, -OH, $-NH_2$, $C_{1-6}$ alkyl or $C_{1-6}$ haloalkyl.

In some embodiments, $R^1$, $R^2$, $R^{1a}$ and $R^{2a}$ are each independently H, D, F, Cl, Br, I, $- NO_2$, -CN, -OH, $-NH_2$, $C_{1-4}$ alkyl or $C_{1-4}$ haloalkyl.

In other embodiments, $R^1$, $R^2$, $R^{1a}$ and $R^{2a}$ are each independently H, D, F, Cl, Br, I, $- NO_2$, -CN, -OH, $-NH_2$, methyl, ethyl, *n*-propyl, isopropyl, *n*-butyl, isobutyl, *sec-butyl, tert-butyl,* $- CH_2F$, $-CHF_2$, $-CF_3$, $-CH_2CHF_2$, $-CHFCH_2F$, $-CH_2CF_3$, $-CH_2Cl$, $-CHCl_2$, $-CH_2CHCl_2$, $-CH_2Br$, $-CHBr_2$ or $-CH_2CHBr_2$.

In some embodiments, ring A is selected from $C_{3-8}$ cycloalkyl, heterocyclic consisting of 3-8 atoms, $C_{6-10}$ aryl, or heteroaryl consisting of 5-6 atoms.

In some embodiments, ring A is selected from cyclopropyl, cyclobutyl, cyclopentyl, cyclohexyl, oxetanyl, azetidinyl, pyrrolidinyl, tetrahydrofuranyl, piperidinyl, piperazinyl, morpholinyl, phenyl, naphthyl, pyrrolyl, pyrazolyl, imidazolyl, triazolyl, tetrazolyl, furyl, thienyl, thiazolyl, oxazolyl, pyridinyl, pyrimidyl, pyrazinyl or pyridazinyl.

In some embodiments, each $R^{3a}$ is independently H, D, F, Cl, Br, I, $-NO_2$, -CN, -OH, $- NH_2$, $C_{1-6}$ alkyl, $C_{2-6}$ alkenyl, $C_{2-6}$ alkynyl, $C_{1-6}$ haloalkyl, $C_{1-6}$ alkoxy or $C_{1-6}$ haloalkoxy, wherein the $C_{1-6}$ alkyl, $C_{2-6}$ alkenyl, $C_{2-6}$ alkynyl and $C_{1-6}$ alkoxy are independently and optionally substituted with 1, 2, 3 or 4 substituents selected from D, F, Cl, Br, I, $C_{1-6}$ alkyl, $C_{2-6}$ alkenyl, $C_{2-6}$ alkynyl, oxo, $-NO_2$, -CN, -OH, $-NH_2$, $-COOCH_3$ and -COOH.

In some embodiments, each $R^{3a}$ is independently H, D, F, Cl, Br, I, $-NO_2$, -CN, -OH, $- NH_2$, $C_{1-4}$ alkyl, $C_{2-4}$ alkenyl, $C_{2-4}$ alkynyl, $C_{1-4}$ haloalkyl, $C_{1-4}$ alkoxy or $C_{1-4}$ haloalkoxy, wherein the $C_{1-4}$ alkyl, $C_{2-4}$ alkenyl, $C_{2-4}$ alkynyl and $C_{1-4}$ alkoxy are independently and optionally substituted with 1, 2, 3 or 4 substituents selected from D, F, Cl, Br, I, $C_{1-4}$ alkyl, $C_{2-4}$ alkenyl, $C_{2-4}$ alkynyl, oxo, $-NO_2$, -CN, -OH, $-NH_2$, $-COOCH_3$ and -COOH.

In other embodiments, each of $R^{3a}$ is independently H, D, F, Cl, Br, I, $-NO_2$, -CN, -OH, $-NH_2$, methyl, ethyl, *n*-propyl, isopropyl, *n*-butyl, isobutyl, *sec*-butyl, *tert*-butyl, vinyl, propenyl, allyl, ethynyl, propargyl, 1-propynyl, 1-butynyl, 2-butynyl, 3-butynyl, $-CH_2F$, $-CHF_2$, $-CF_3$, $-CH_2CHF_2$, $- CHFCH_2F$, $-CH_2CF_3$, $-OCH_3$, $-OCH_2CH_3$, $-OCH_2CH_2CH_3$, $-OCH(CH_3)_2$, $-OCHF_2$, $-OCF_3$, $- OCH_2CHF_2$ or $-OCH_2CF_3$, wherein the methyl, ethyl, *n*-propyl, isopropyl, *n*-butyl, isobutyl, *sec*-butyl, *tert*-butyl, vinyl, propenyl, allyl, ethynyl, propargyl, 1-propynyl, 1-butynyl, 2-butynyl, 3-butynyl, $- OCH_3$, $-OCH_2CH_3$, $-OCH_2CH_2CH_3$ and $-OCH(CH_3)_2$ are independently and optionally substituted with 1, 2, 3 or 4 substituents selected from D, F, Cl, Br, I, methyl, ethyl, n-propyl, isopropyl, vinyl, propenyl, allyl, ethynyl, propargyl, 1-propynyl, oxo, $-NO_2$, -CN, -OH, $-NH_2$, $-COOCH_3$ and -COOH.

In some embodiments, $R^4$ is H, D, F, Cl, Br, I, $-NO_2$, -CN, -OH, $-NH_2$, $C_{1-6}$ alkyl, $C_{1-6}$ haloalkyl or $C_{3-8}$ cycloalkyl, wherein the $C_{1-6}$ alkyl and $C_{3-8}$ cycloalkyl are independently and optionally substituted with 1, 2, 3 or 4 substituents selected from D, F, Cl, Br, I, $C_{1-6}$ alkyl, $C_{1-6}$ haloalkyl, oxo, $-NO_2$, -CN, -OH, $-NH_2$, $-COOCH_3$ and -COOH.

In some embodiments, $R^4$ is H, D, F, Cl, Br, I, $-NO_2$, -CN, -OH, $-NH_2$, $C_{1-4}$ alkyl, $C_{1-4}$ haloalkyl or $C_{3-6}$ cycloalkyl, wherein the $C_{1-4}$ alkyl and $C_{3-6}$ cycloalkyl are independently and optionally substituted with 1, 2, 3 or 4 substituents selected from D, F, Cl, Br, I, $C_{1-4}$ alkyl, $C_{1-4}$ haloalkyl, oxo, $-NO_2$, -CN, -OH, $-NH_2$, $-COOCH_3$ and -COOH.

In other embodiments, $R^4$ is H, D, F, Cl, Br, I, $-NO_2$, -CN, -OH, $-NH_2$, methyl, ethyl, *n*-propyl, isopropyl, *n*-butyl, isobutyl, ***sec***-butyl, ***tert***-butyl, $-CH_2F$, $-CHF_2$, $-CF_3$, $-CH_2CHF_2$, $-CHFCH_2F$, $-CH_2CF_3$, cyclopropyl, cyclobutyl, cyclopentyl or cyclohexyl, wherein the methyl, ethyl, *n*-propyl, isopropyl, *n*-butyl, isobutyl, ***sec***-butyl, ***tert***-butyl, cyclopropyl, cyclobutyl, cyclopentyl and cyclohexyl are independently and optionally substituted with 1, 2, 3 or 4 substituents selected from D, F, Cl, Br, I, methyl, ethyl, *n*-propyl, isopropyl, *n*-butyl, isobutyl, ***sec***-butyl, ***tert***-butyl, $-CH_2F$, $-CHF_2$, $-CF_3$, $-CH_2CHF_2$, $-CHFCH_2F$, $-CH_2CF_3$, oxo, $-NO_2$, -CN, -OH, $-NH_2$, $-COOCH_3$ and -COOH.

In some embodiments, $L_2$ is a bond, $-C_{1-6}$ alkylene- or $-C_{1-6}$ alkylene-$NR^w$-$C_{1-6}$ alkylene-, wherein the each $-C_{1-6}$ alkylene is independently and optionally substituted with 1, 2, 3 or 4 substituents selected from D, F, Cl, Br, I, oxo, -OH, $C_{1-6}$ alkyl and $C_{1-6}$ haloalkyl; wherein, $R^w$ is as defined herein.

In some embodiments, $L_2$ is a bond, $-C_{1-3}$ alkylene- or $-C_{1-3}$ alkylene-$NR^w$-$C_{1-3}$ alkylene-, wherein the each $-C_{1-3}$ alkylene is independently and optionally substituted with 1, 2, 3 or 4 substituents selected from D, F, Cl, Br, I, oxo, -OH, $C_{1-4}$ alkyl and $C_{1-4}$ haloalkyl; wherein, $R^w$ is as defined herein.

In some embodiments, $L_2$ is a bond, -methylene-, -ethylene-, -methylene-$NR^w$-methylene-, or -ethylene-$NR^w$-ethylene-, wherein the -methylene- and -ethylene- are independently and optionally substituted with 1, 2, 3 or 4 substituents selected from D, F, Cl, Br, I, oxo, -OH, methyl, ethyl, *n*-propyl, isopropyl, *n*-butyl, isobutyl, ***sec***-butyl, ***tert***-butyl, $-CH_2F$, $-CHF_2$, $-CF_3$, $-CH_2CHF_2$, $-CHFCH_2F$ and $-CH_2CF_3$; wherein, $R^w$ is as defined herein.

In some embodiments, $R^w$ is H, D, -OH, $C_{1-6}$ alkyl, $C_{1-6}$ haloalkyl, $C_{2-6}$ alkenyl or $C_{2-6}$ alkynyl, wherein the $C_{1-6}$ alkyl, $C_{1-6}$ haloalkyl, $C_{2-6}$ alkenyl and $C_{2-6}$ alkynyl are independently and optionally substituted with 1, 2, 3 or 4 substituents selected from D, F, Cl, Br, I, oxo, -OH, $C_{1-6}$ alkyl and $C_{1-6}$ haloalkyl.

In some embodiments, $R^w$ is H, D, -OH, $C_{1-4}$ alkyl, $C_{1-4}$ haloalkyl, $C_{2-4}$ alkenyl or $C_{2-4}$ alkynyl, wherein the $C_{1-4}$ alkyl, $C_{1-4}$ haloalkyl, $C_{2-4}$ alkenyl and $C_{2-4}$ alkynyl are independently and optionally substituted with 1, 2, 3 or 4 substituents selected from D, F, Cl, Br, I, oxo, -OH, $C_{1-4}$ alkyl and $C_{1-4}$ haloalkyl.

In other embodiments, $R^w$ is H, D, -OH, methyl, ethyl, *n*-propyl, isopropyl, *n*-butyl, isobutyl, ***sec***-butyl, ***tert***-butyl, $-CH_2F$, $-CHF_2$, $-CF_3$, $-CH_2CHF_2$, $-CHFCH_2F$, $-CH_2CF_3$, vinyl, propenyl, allyl, ethynyl, propargyl, 1-propynyl, 1-butynyl, 2-butynyl or 3-butynyl, wherein the methyl, ethyl, *n*-propyl, isopropyl, *n*-butyl, isobutyl, ***sec***-butyl, ***tert***-butyl, $-CH_2F$, $-CHF_2$, $-CH_2CHF_2$, $-CHFCH_2F$, $-CH_2CF_3$, vinyl, propenyl, allyl, ethynyl, propargyl, 1-propynyl, 1-butynyl, 2-butynyl and 3-butynyl are independently and optionally substituted with 1, 2, 3 or 4 substituents selected from D, F, Cl, Br, I, oxo, -OH, methyl, ethyl, *n*-propyl, isopropyl, *n*-butyl, isobutyl, ***sec***-butyl, ***tert***-butyl, $-CH_2F$, $-CHF_2$, $-CF_3$, $-CH_2CHF_2$, $-CHFCH_2F$ and $-CH_2CF_3$.

In some embodiments, $R^3$ is $-NR^5R^6$, $C_{3-12}$ cycloalkyl or heterocyclic consisting of 3-12 atoms, wherein the $C_{3-12}$ cycloalkyl and heterocyclic consisting of 3-12 atoms are independently and optionally substituted with 1, 2, 3 or 4 substituents selected from D, halogen, $-NO_2$, -CN, -OH, $-NH_2$, oxo, $C_{1-6}$ alkyl, $-C(O)CH_3$, -C(O)OH, $-C_{1-4}$ alkylene C(O)OH, $-C(O)OCH_3$, -NHC(O)-$C_{1-4}$ alkyl, $-NHC(O)OCH_3$, $-C(O)NH_2$, $-S(O)_2CH_3$, $-S(O)_2NH_2$ and $-C(O)NHS(O)_2CH_3$; wherein, each of $R^5$ and $R^6$ is as defined herein.

In some embodiments, $R^3$ is $-NR^5R^6$, $C_{3-10}$ cycloalkyl or heterocyclic consisting of 3-10 atoms, wherein the $C_{3-10}$ cycloalkyl and heterocyclic consisting of 3-10 atoms are independently and optionally substituted with 1, 2, 3 or 4 substituents selected from D, halogen, $-NO_2$, -CN, -OH, $-NH_2$, oxo, $C_{1-4}$ alkyl, $-C(O)CH_3$, -C(O)OH, $-C_{1-4}$ alkylene C(O)OH, $-C(O)OCH_3$, -NHC(O)-$C_{1-4}$ alkyl, $-NHC(O)OCH_3$, $-C(O)NH_2$, $-S(O)_2CH_3$, $-S(O)_2NH_2$ and $-C(O)NHS(O)_2CH_3$; wherein, each of $R^5$ and $R^6$ is as defined herein.

In some embodiments, $R^3$ is $-NR^5R^6$, $C_{5-8}$ cycloalkyl or heterocyclic consisting of 4-9 atoms, wherein the $C_{5-8}$ cycloalkyl and heterocyclic consisting of 4-9 atoms are independently and optionally substituted with 1, 2, 3 or 4 substituents selected from D, halogen, $-NO_2$, -CN, -OH, $-NH_2$, oxo, $C_{1-4}$ alkyl, $-C(O)CH_3$, -C(O)OH, $-C_{1-4}$ alkylene C(O)OH, $-C(O)OCH_3$, -NHC(O)-$C_{1-4}$ alkyl, $-NHC(O)OCH_3$, $-C(O)NH_2$, $-S(O)_2CH_3$, $-S(O)_2NH_2$ and $-C(O)NHS(O)_2CH_3$; wherein, each of $R^5$ and $R^6$ is as defined herein.

In some embodiments, $R^3$ is $-NR^5R^6$, cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, 2-oxa-5-azabicyclo[2.2.1]heptan-5-yl, 2-azabicyclo[2.2.1]heptyl, 2-azabicyclo[2.2.2]octyl, 3-azabicyclo[3.1.0]hexyl, 2,6-diazaspiro[3.3]heptyl, 2-oxa-6-azaspiro[3.3]heptyl, 2,6-diazaspiro[3.4]octyl, 1,7-diazaspiro[4.4]nonyl, azetidinyl, pyrrolidyl, tetrahydrofuranyl, piperidinyl, piperazinyl or morpholinyl, wherein the cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, 2-oxa-5-azabicyclo[2.2.1]heptan-5-yl, 2-azabicyclo[2.2.1]heptyl, 2-azabicyclo[2.2.2]octyl, 3-azabicyclo[3.1.0]hexyl, 2,6-diazaspiro[3.3]heptyl, 2-oxa-6-azaspiro[3.3]heptyl, 2,6-diazaspiro[3.4]octyl, 1,7-diazaspiro[4.4]nonyl, azetidinyl, pyrrolidyl, tetrahydrofuranyl, piperidinyl, piperazinyl and morpholinyl are independently and optionally substituted with 1, 2, 3 or 4 substituents selected from D, F, Cl, Br, I, $-NO_2$, -CN, -OH, $-NH_2$, oxo, methyl, ethyl, *n*-propyl, isopropyl, *n*-butyl, isobutyl, ***sec***-butyl, ***tert***-butyl, $-C(O)CH_3$, -C(O)OH, -methylene C(O)OH, $-C(O)OCH_3$, $-NHC(O)CH_3$, $-NHC(O)OCH_3$, $-C(O)NH_2$, $-S(O)_2CH_3$, $-S(O)_2NH_2$ and $-C(O)NHS(O)_2CH_3$; wherein, each of $R^5$ and $R^6$ is as defined herein.

In some embodiments, $R^5$ and $R^6$ are each independently H, D, $C_{1-6}$ alkyl, $C_{3-10}$ cycloalkyl or heterocyclic consisting of 3-12 atoms, wherein the $C_{1-6}$ alkyl, $C_{3-10}$ cycloalkyl and heterocyclic consisting of 3-12 atoms are

independently and optionally substituted with 1, 2, 3 or 4 substituents selected from D, F, Cl, Br, I, $-NO_2$, -CN, -OH, $-NH_2$, oxo, $C_{1-6}$ alkyl, $C_{1-6}$ haloalkyl, hydroxy $C_{1-6}$ alkyl, amino $C_{1-6}$ alkyl, carboxy $C_{1-6}$ alkyl, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^6$; wherein, each of $R^a$, $R^b$, $R^c$ and $R^d$ is as defined herein.

In some embodiments, $R^5$ and $R^6$ are each independently H, D, $C_{1-4}$ alkyl, $C_{5-8}$ cycloalkyl or heterocyclic consisting of 4-9 atoms, wherein the $C_{1-4}$ alkyl, $C_{5-8}$ cycloalkyl and heterocyclic consisting of 4-9 atoms are independently and optionally substituted with 1, 2, 3 or 4 substituents selected from D, F, Cl, Br, I, $-NO_2$, -CN, -OH, $-NH_2$, oxo, $C_{1-4}$ alkyl, $C_{1-4}$ haloalkyl, hydroxy $C_{1-4}$ alkyl, amino $C_{1-4}$ alkyl, carboxy $C_{1-4}$ alkyl, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$; wherein, each of $R^a$, $R^b$, $R^c$ and $R^d$ is as defined herein.

In some embodiments, $R^5$ and $R^6$ are each independently H, D, $C_{1-4}$ alkyl, $C_{3-8}$ cycloalkyl or heterocyclic consisting of 3-10 atoms, wherein the $C_{1-4}$ alkyl, $C_{3-8}$ cycloalkyl and of heterocyclic consisting of 3-10 atoms are independently and optionally substituted with 1, 2, 3 or 4 substituents selected from D, F, Cl, Br, I, $-NO_2$, -CN, -OH, $-NH_2$, oxo, $C_{1-4}$ alkyl, $C_{1-4}$ haloalkyl, hydroxy $C_{1-4}$ alkyl, amino $C_{1-4}$ alkyl, carboxy $C_{1-4}$ alkyl, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$; wherein, each of $R^a$, $R^b$, $R^c$ and $R^d$ is as defined herein.

In some embodiments, $R^5$ and $R^6$ together with the atom to which they are attached form heterocyclic consisting of 4-9 atoms, wherein the heterocyclic consisting of 4-9 atoms optionally contains 1, 2 or 3 heteroatoms independently selected from oxygen, sulfur, or nitrogen, and is optionally substituted with 1, 2, 3 or 4 substituents independently selected from D, F, Cl, Br, I, $-NO_2$, -CN, oxo, $C_{1-4}$ alkyl, $C_{1-4}$ haloalkyl, hydroxy $C_{1-4}$ alkyl, amino $C_{1-4}$ alkyl, carboxy $C_{1-4}$ alkyl, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$; wherein, each of $R^a$, $R^b$, $R^c$ and $R^d$ is as defined herein.

In some embodiments, each of $R^5$ and $R^6$ is independently H, D, methyl, ethyl, *n*-propyl, isopropyl, *n*-butyl, isobutyl, *sec*-butyl, *tert*-butyl, cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, azetidinyl, pyrrolidyl, tetrahydrofuranyl, piperidinyl, piperazinyl or morpholinyl, wherein the methyl, ethyl, *n*-propyl, isopropyl, *n*-butyl, isobutyl, *sec*-butyl, *tert*-butyl, cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, azetidinyl, pyrrolidyl, tetrahydrofuranyl, piperidinyl, piperazinyl and morpholinyl are independently and optionally substituted with 1, 2, 3 or 4 substituents selected from D, F, Cl, Br, I, $-NO_2$, -CN, -OH, $-NH_2$, oxo, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, $-CH_2OH$, $-CH_2CH_2OH$, $-CH_2NH_2$, $-CH_2CH_2NH_2$, $-CH_2COOH$, $-CH_2CH_2COOH$, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$; wherein, each of $R^a$, $R^b$, $R^c$ and $R^d$ is as defined herein.

In some embodiments, $R^5$ and $R^6$ together with the atom to which they are attached form heterocyclic consisting of 3-12 atoms, wherein the heterocyclic consisting of 3-12 atoms optionally contains 1, 2 or 3 heteroatoms independently selected from oxygen, sulfur, or nitrogen, and is optionally substituted with 1, 2, 3 or 4 substituents independently selected from D, F, Cl, Br, I, $-NO_2$, -CN, oxo, $C_{1-6}$ alkyl, $C_{1-6}$ haloalkyl, hydroxy $C_{1-6}$ alkyl, amino $C_{1-6}$ alkyl, carboxy $C_{1-6}$ alkyl, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$; wherein, each of $R^a$, $R^b$, $R^c$ and $R^d$ is as defined herein.

In some embodiments, $R^5$ and $R^6$ together with the atom to which they are attached form heterocyclic consisting of 3-10 atoms, wherein the heterocyclic consisting of 3-10 atoms optionally contains 1, 2 or 3 heteroatoms independently selected from oxygen, sulfur, or nitrogen, and is optionally substituted with 1, 2, 3 or 4 substituents independently selected from D, F, Cl, Br, I, $-NO_2$, -CN, oxo, $C_{1-4}$ alkyl, $C_{1-4}$ haloalkyl, hydroxy $C_{1-4}$ alkyl, amino $C_{1-4}$ alkyl, carboxy $C_{1-4}$ alkyl, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$; wherein, each of $R^a$, $R^b$, $R^c$ and $R^d$ is as defined herein.

In other embodiments, $R^5$ and $R^6$ together with the atoms to which they are attached form a structure selected from the following:

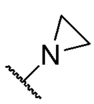 , 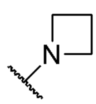 , 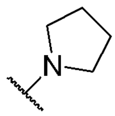 , 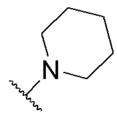 , 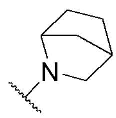 ,

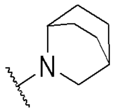, 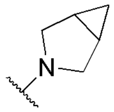, 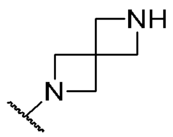, 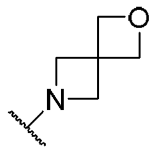, 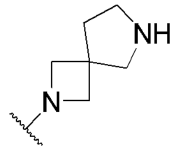 or 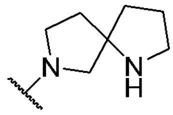,

wheiein the

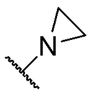, 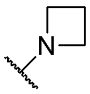, 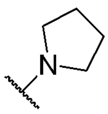, 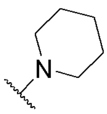, 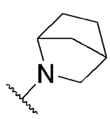, 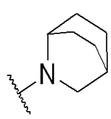, 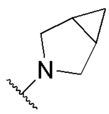, 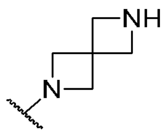, 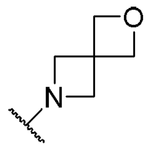,

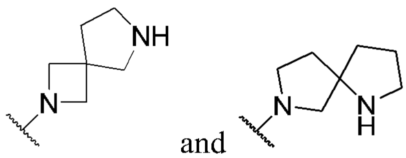 and

are independently and optionally substituted with 1, 2, 3 or 4 substituents selected from D, F, Cl, Br, I, $-NO_2$, -CN, oxo, methyl, ethyl, n-propyl, isopropyl, n-butyl, isobutyl, *sec-butyl, tert-butyl,* $-CH_2F$, $-CHF_2$, $-CF_3$, $-CH_2CHF_2$, $-CHFCH_2F$, $-CH_2CF_3$, $-CH_2OH$, $-CH_2CH_2OH$, $-CH_2NH_2$, $-CH_2CH_2NH_2$, $-CH_2COOH$, $-CH_2CH_2COOH$, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$; wherein, each of $R^a$, $R^b$, $R^c$ and $R^d$ is as defined herein.

In one embodiment, the $-NR^5R^6$ of the present invention is selected from the following structures:

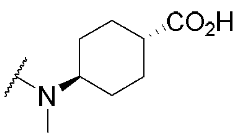, 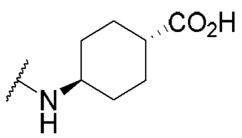, 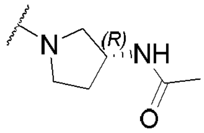, 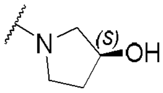, 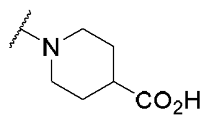,

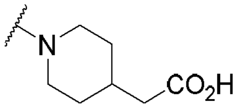, 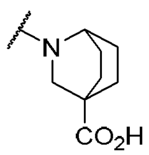, 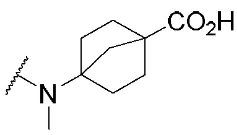, 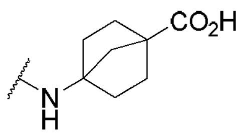,

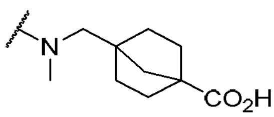, 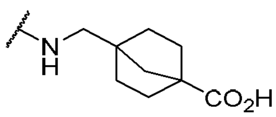, 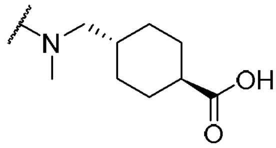, 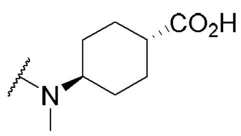,

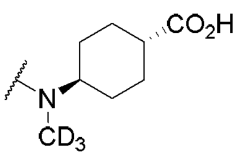, 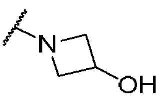, 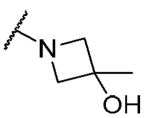, 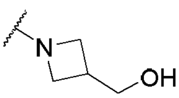, 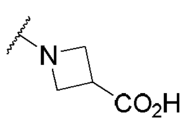, 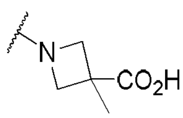,

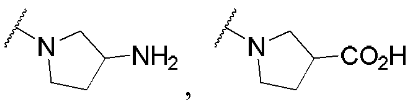, , 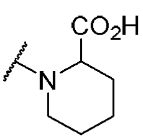, 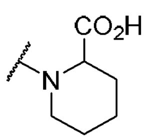, 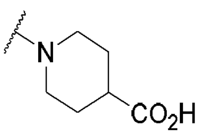, 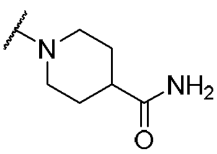,

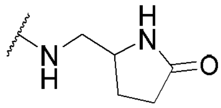, 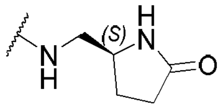, 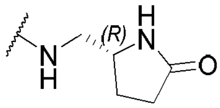, 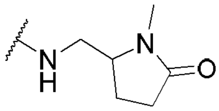, 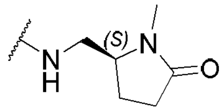,

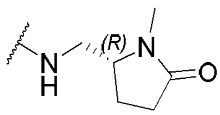, 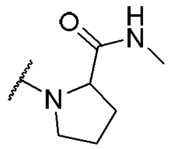, 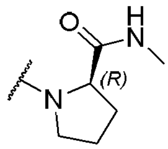, 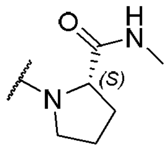, 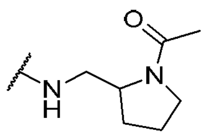,

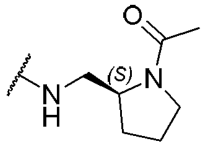, 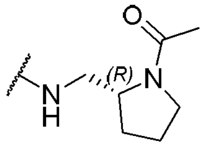, 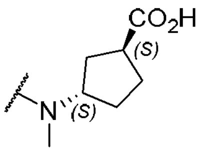, 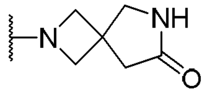, 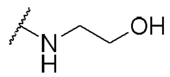,

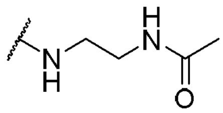, 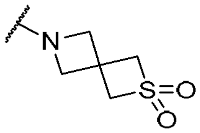, 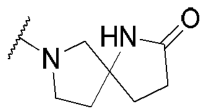, 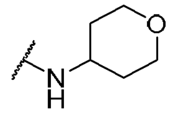, 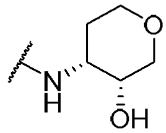,

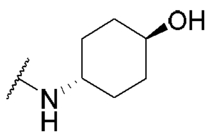, 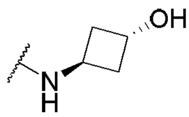, 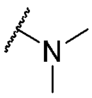, 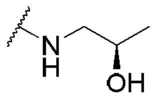, 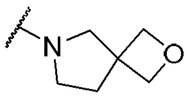 or 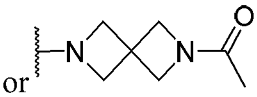.

In some embodiments, each of $R^a$, $R^b$, $R^c$ and $R^d$ is independently H, D, $C_{1-6}$ alkyl, $C_{3-8}$ cycloalkyl or heterocyclic consisting of 3-8 atoms, wherein the $C_{1-6}$ alkyl, $C_{3-8}$ cycloalkyl and heterocyclic consisting of 3-8 atoms are independently and optionally substituted with 1, 2, 3 or 4 substituents selected from D, F, Cl, Br, I, $C_{1-6}$ alkyl, $-NO_2$, -CN, -OH, $-NH_2$, -C(O)$CH_3$, -C(O)OH, - C(O)$OCH_3$, -NHC(O)$CH_3$, -NHC(O)$OCH_3$, -C(O)$NH_2$, $-S(O)_2CH_3$ and $-S(O)_2NH_2$.

In some embodiments, each of $R^a$, $R^b$, $R^c$ and $R^d$ is independently H, D, $C_{1-4}$ alkyl, $C_{3-6}$ cycloalkyl or heterocyclic consisting of 3-6 atoms, wherein the $C_{1-4}$ alkyl, $C_{3-6}$ cycloalkyl and heterocyclic consisting of 3-6 atoms are independently and optionally substituted with 1, 2, 3 or 4 substituents selected from D, F, Cl, Br, I, $C_{1-4}$ alkyl, $-NO_2$, -CN, -OH, $-NH_2$, -C(O)$CH_3$, -C(O)OH, - C(O)$OCH_3$, -NHC(O)$CH_3$, -NHC(O)$OCH_3$, -C(O)$NH_2$, $-S(O)_2CH_3$ and $-S(O)_2NH_2$.

In other embodiments, each of $R^a$, $R^b$, $R^c$ and $R^d$ is independently H, D, methyl, ethyl, *n*-propyl, isopropyl, *n*-butyl, isobutyl, *sec*-butyl, *tert*-butyl, cyclopropyl, cyclobutyl, cyclopentyl, cyclohexyl, azetidinyl, pyrrolidyl, tetrahydrofuranyl, piperidinyl, piperazinyl or morpholinyl, wherein the methyl, ethyl, *n*-propyl, isopropyl, *n*-butyl, isobutyl, *sec*-butyl, *tert*-butyl, cyclopropyl, cyclobutyl, cyclopentyl, cyclohexyl, azetidinyl, pyrrolidyl, tetrahydrofuranyl, piperidinyl, piperazinyl and morpholinyl are independently and optionally substituted with 1, 2, 3 or 4 substituents selected from D, F, Cl, Br, I, methyl, ethyl, *n*-propyl, isopropyl, *n*-butyl, isobutyl, *sec*-butyl, *tert*-butyl, $-NO_2$, -CN, -OH, $-NH_2$, -C(O)$CH_3$, -C(O)OH, -C(O)$OCH_3$, -NHC(O)$CH_3$, -NHC(O)$OCH_3$, - C(O)$NH_2$, $-S(O)_2CH_3$ and $-S(O)_2NH_2$.

In some embodiments, $R^7$ is $C_{3-10}$ cycloalkyl or heterocyclic consisting of 3-12 atoms, wherein the $C_{3-10}$ cycloalkyl and heterocyclic consisting of 3-12 atoms are independently and optionally substituted with 1, 2, 3 or 4 substituents selected from D, F, Cl, Br, I, $-NO_2$, -CN, -OH, - $NH_2$, oxo, $C_{1-6}$ alkyl, $C_{1-6}$ haloalkyl, $C_{1-6}$ hydroxy alkyl, $-OR^e$, $-C(O)R^e$, $-C(O)OR^e$, $-NR^eR^f$, - $NR^eC(O)R^f$, $-NR^eC(O)OR^f$ and $-C(O)NR^eR^f$; wherein, $R^e$ and $R^f$ are as defined herein.

In some embodiments, $R^7$ is $C_{3-8}$ cycloalkyl or heterocyclic consisting of 3-10 atoms, wherein the $C_{3-8}$ cycloalkyl and heterocyclic consisting of 3-10 atoms are independently and optionally substituted with 1, 2, 3 or 4 substituents selected from D, F, Cl, Br, I, $-NO_2$, -CN, -OH, - $NH_2$, oxo, $C_{1-4}$ alkyl, $C_{1-4}$ haloalkyl, $C_{1-4}$ hydroxy alkyl, $-OR^e$, $-C(O)R^e$, $-C(O)OR^e$, $-NR^eR^f$, - $NR^eC(O)R^f$, $-NR^eC(O)OR^f$ and $-C(O)NR^eR^f$; wherein, each of $R^e$ and $R^f$ is as defined herein.

In other embodiments, $R^7$ is cyclopropyl, cyclobutyl, cyclopentyl, cyclohexyl, cycloheptyl, cyclooctyl, bicyclo[1.1.1]pentyl, bicyclo[2.1.1]hexyl, bicyclo[2.2.1]heptyl, bicyclo[2.2.2]octyl, azetidinyl, oxetanyl, pyrrolidinyl, tetrahydrofuranyl, tetrahydrothiophenyl, thiazolidinyl, pyrazolidinyl, oxazolidinyl, imidazolidinyl, isoxazolidinyl, piperidinyl, piperazinyl or morpholinyl, wherein the cyclopropyl, cyclobutyl, cyclopentyl, cyclohexyl, cycloheptyl, cyclooctyl, bicyclo[1.1.1]pentyl, bicyclo[2.1.1]hexyl, bicyclo[2.2.1]heptyl, bicyclo[2.2.2]octyl, azetidinyl, oxetanyl, pyrrolidinyl, tetrahydrofuranyl, tetrahydrothiophenyl, thiazolidinyl, pyrazolidinyl, oxazolidinyl, imidazolidinyl, isoxazolidinyl, piperidinyl, piperazinyl and morpholinyl are independently and optionally substituted with 1, 2, 3 or 4 substituents selected from D, F, Cl, Br, I, - $NO_2$, -CN, -OH, $-NH_2$, oxo, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, $-CH_2OH$, $-CH_2CH_2OH$, $-CH(OH)CH_3$, $-OR^e$, $-C(O)R^e$, $-C(O)OR^e$, $-NR^eR^f$, $-NR^eC(O)R^f$, $-NR^eC(O)OR^f$ and $-C(O)NR^eR^f$; wherein, each of $R^e$ and $R^f$ is as defined herein.

In some embodiments, each of $R^e$ and $R^f$ is H, D, $C_{1-6}$ alkyl or $C_{3-8}$ cycloalkyl, wherein the $C_{1-6}$ alkyl and $C_{3-8}$ cycloalkyl are independently and optionally substituted with 1, 2, 3 or 4 substituents selected from D, F, Cl, Br, I, oxo, $-NO_2$, -CN, -OH, $-NH_2$, $-C(O)CH_3$, $-COOCH_3$ and - COOH.

In some embodiments, each of $R^e$ and $R^f$ is H, D, $C_{1-4}$ alkyl or $C_{3-6}$ cycloalkyl, wherein the $C_{1-4}$ alkyl and $C_{3-6}$ cycloalkyl are independently and optionally substituted with 1, 2, 3 or 4 substituents selected from D, F, Cl, Br, I, oxo, $-NO_2$, -CN, -OH, $-NH_2$, $-C(O)CH_3$, $-COOCH_3$ and - COOH.

In some embodiments, each of $R^e$ and $R^f$ is H, D, methyl, ethyl, *n*-propyl, isopropyl, *n*-butyl, isobutyl, *sec*-butyl, *tert*-butyl, cyclopropyl, cyclobutyl, cyclopentyl or cyclohexyl, wherein the methyl, ethyl, *n*-propyl, isopropyl, *n*-butyl, isobutyl, *sec*-butyl, *tert*-butyl, cyclopropyl, cyclobutyl, cyclopentyl and cyclohexyl are independently and optionally substituted with 1, 2, 3 or 4 substituents selected from D, F, Cl, Br, I, oxo, $-NO_2$, -CN, -OH, $-NH_2$, $-C(O)CH_3$, $-COOCH_3$ and -COOH.

In some embodiments, the present invention provides a compound of formula (II) or a stereoisomer, a geometric isomer, a tautomer, an N-oxide, a hydrate, a solvate, a metabolite, an eater, a pharmaceutically acceptable salt or a prodrug thereof,

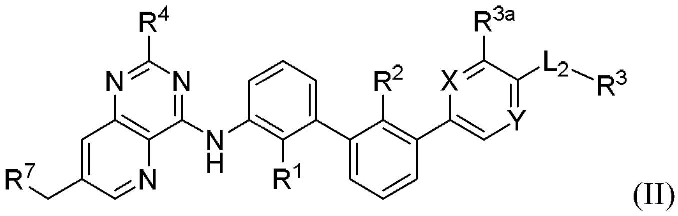

(II)

wherein, each of X, Y, $R^1$, $R^2$, $R^3$, $R^{3a}$, $R^4$, $R^7$ and $L_2$ is as defined herein.

In some embodiments, X is $CR^x$ or N; wherein $R^x$ is as defined herein.

In some embodiments, Y is $CR^y$ or N; wherein $R^y$ is as defined herein.

In some embodiments, $R^x$ and $R^y$ are each independently H, D, F, Cl, Br, I, $-NO_2$, -CN, -OH, $-NH_2$, $C_{1-6}$ alkyl, $C_{1-6}$ haloalkyl, $C_{1-6}$ alkoxy or $C_{1-6}$ haloalkoxy.

In some embodiments, $R^x$ and $R^y$ are each independently H, D, F, Cl, Br, I, $-NO_2$, -CN, -OH, $-NH_2$, $C_{1-4}$ alkyl, $C_{1-4}$ haloalkyl, $C_{1-4}$ alkoxy or $C_{1-4}$ haloalkoxy.

In other embodiments, $R^x$ and $R^y$ are each independently H, D, F, Cl, Br, I, $-NO_2$, -CN, -OH, $-NH_2$, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, $-OCH_3$, $-OCH_2CH_3$, $-OCH_2CH_2CH_3$, $-OCH(CH_3)_2$, $-OCHF_2$, $-OCF_3$, $-OCH_2CHF_2$ or $-OCH_2CF_3$.

In some embodiments, the present invention provides a compound of formula (III) or a stereoisomer, a tautomer, an N-oxide, a solvate, a metabolite, a pharmaceutically acceptable salt or a prodrug thereof,

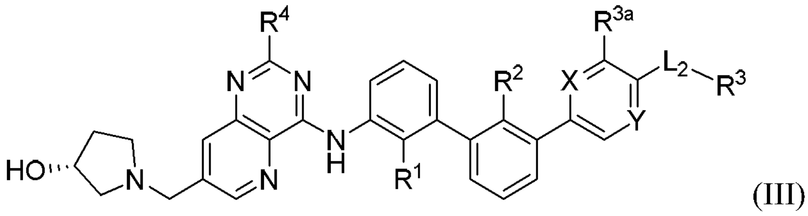

(III)

wherein, X, Y, $R^1$, $R^2$, $R^3$, $R^{3a}$ and $L_2$ are as defined herein.

In some embodiments, the present invention provides a compound of formula (IV) or a stereoisomer, a geometric isomer, a tautomer, an N-oxide, a solvate, a metabolite, a pharmaceutically acceptable salt or a prodrug thereof,

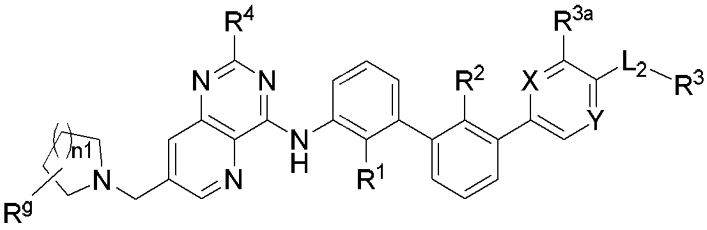

(IV)

wherein, n1 is 0, 1, 2, 3, 4 or 5;

$R^g$ is H, D, F, Cl, Br, I, $-NO_2$, -CN, -OH, $-NH_2$, oxo, $C_{1-6}$ alkyl, $C_{1-6}$ haloalkyl, $C_{1-6}$ hydroxy alkyl, $-OR^e$, $-C(O)R^e$, $-C(O)OR^e$, $-NR^eR^f$, $-NR^eC(O)R^f$, $-NR^eC(O)OR^f$ and $-C(O)NR^eR^f$;

wherein, each of X, Y, $R^1$, $R^2$, $R^3$, $R^{3a}$, $R^4$, $R^e$, $R^f$ and $L_2$ is as defined herein.

In some embodiments, $R^g$ is H, D, F, Cl, Br, I, $-NO_2$, -CN, -OH, $-NH_2$, oxo, $C_{1-4}$ alkyl, $C_{1-4}$ haloalkyl, $C_{1-4}$ hydroxy alkyl, $-OR^e$, $-C(O)R^e$, $-C(O)OR^e$, $-NR^eR^f$, $-NR^eC(O)R^f$, $-NR^eC(O)OR^f$ and $-C(O)NR^eR^f$;
wherein, each of $R^e$ and $R^f$ is as defined herein.

In some embodiments, $R^g$ is H, D, F, Cl, Br, I, $-NO_2$, -CN, -OH, $-NH_2$, oxo, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, $-CH_2OH$, $-CH_2CH_2OH$, $-CH(OH)CH_3$, $-OR^e$, $-C(O)R^e$, $-C(O)OR^e$, $-NR^eR^f$, $-NR^eC(O)R^f$, $-NR^eC(O)OR^f$ or $-C(O)NR^eR^f$;
wherein, each of $R^e$ and $R^f$ is as defined herein.

In another aspect, the present invention relates to one of the following compounds or a stereoisomer, tautomer, N-oxide, solvate, hydrate, metabolite, ester, pharmaceutically acceptable salt or prodrug thereof of one of the following compounds, but in no way limited thereto:

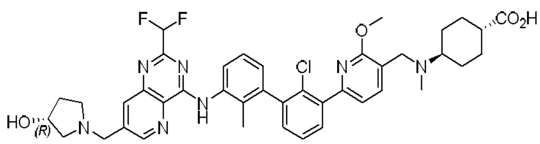

(1),

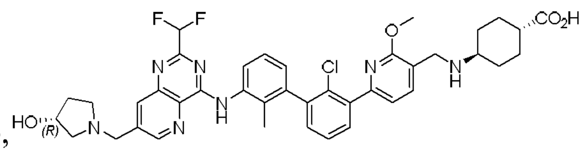

(2),

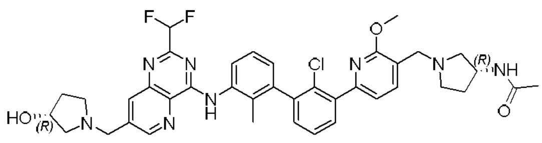

(3),

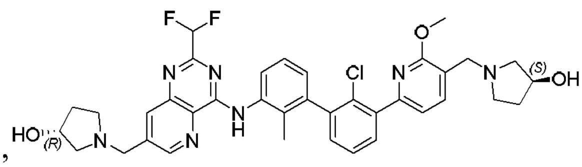

(4),

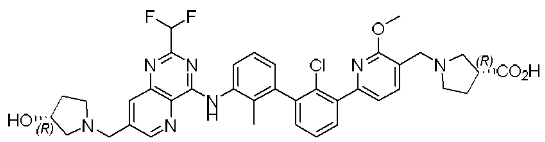

(5),

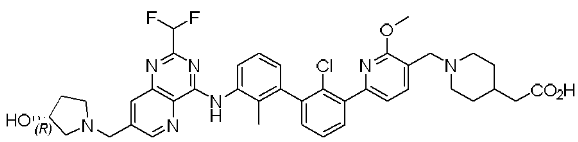

(6),

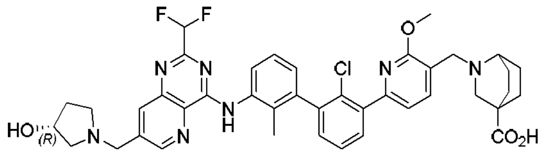

(7),

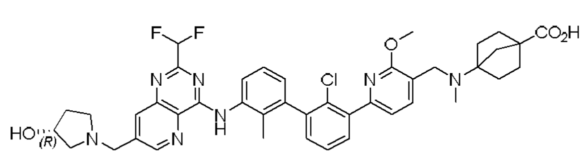

(8)

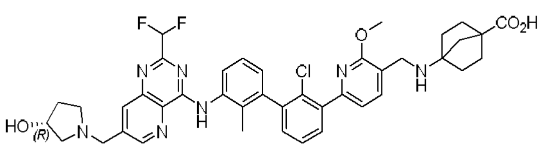

(9),

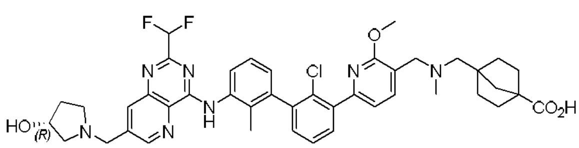

(10),

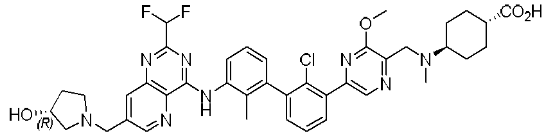
(11),
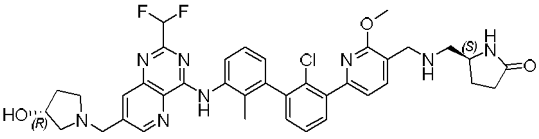
(12),

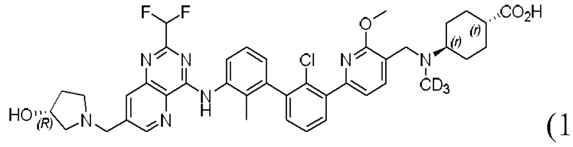
(13),
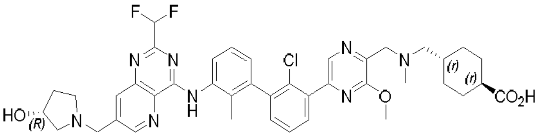
(14),

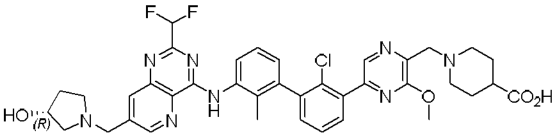
(15),
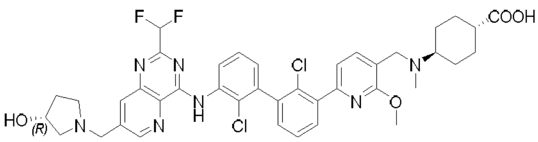
(16),

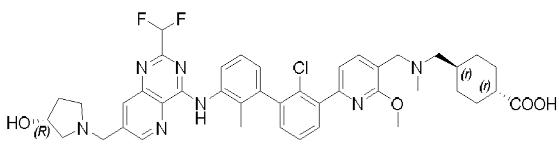
(17),
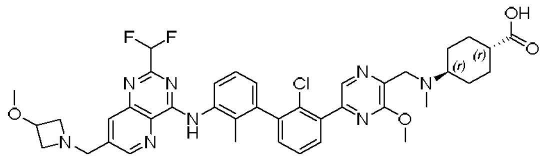
(18)

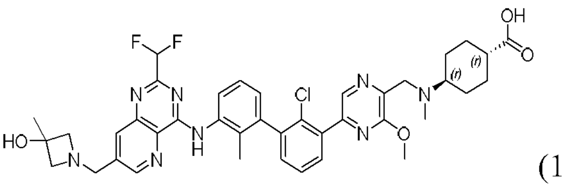
(19),
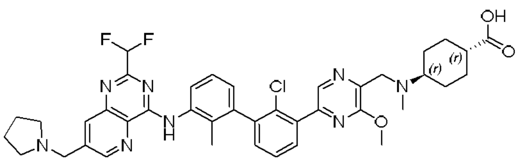
(20),

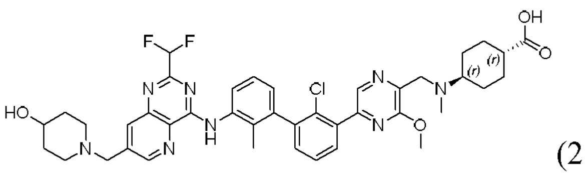
(21),
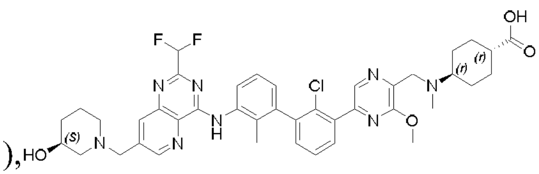
(22),

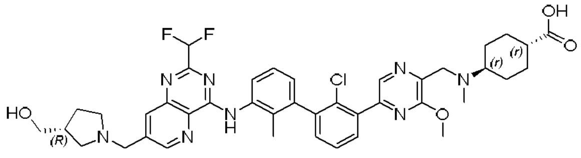
(23),
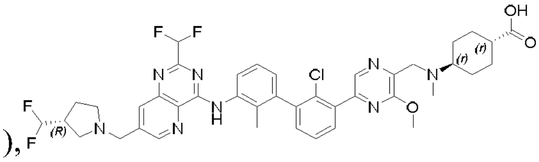
(24),

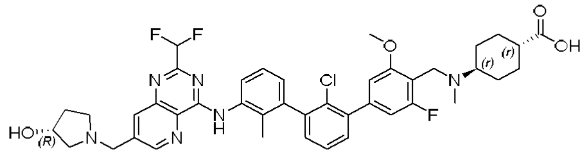
(25),
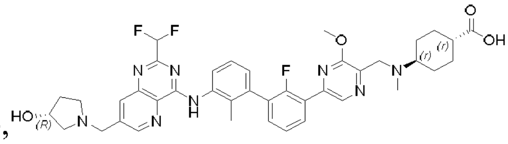
(26),

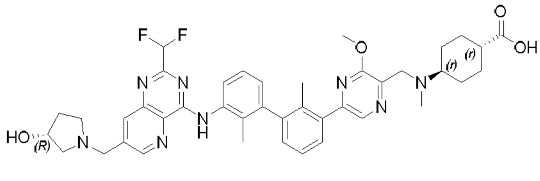 (27), 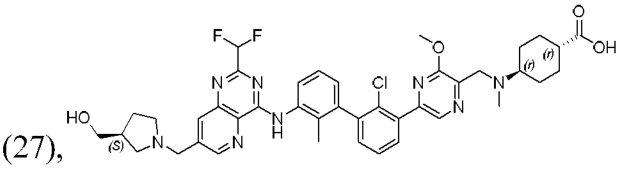 (28),

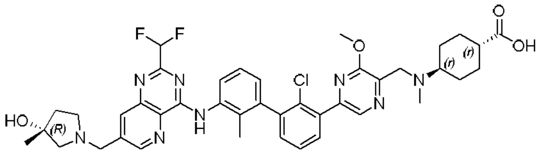 (29), 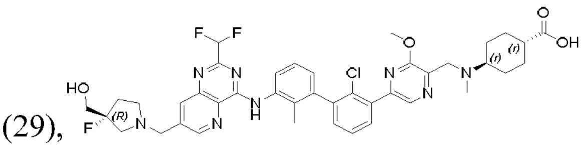 (30),

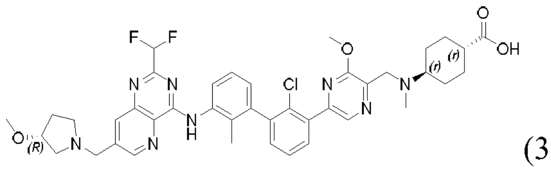 (31), 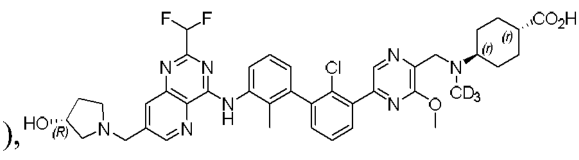 (32),

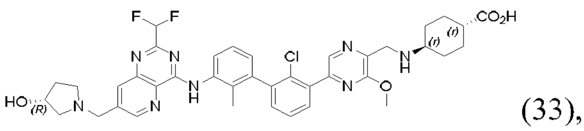 (33), 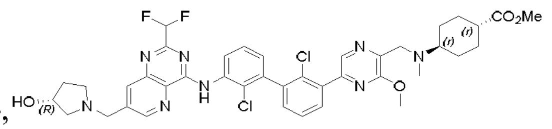 (34),

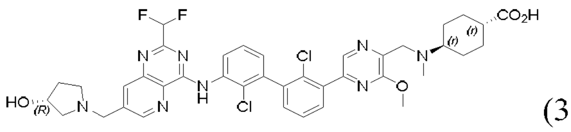 (35), 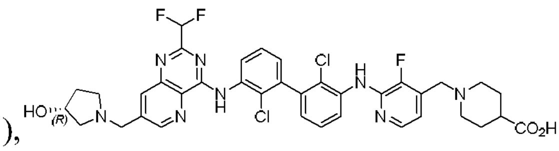 (36),

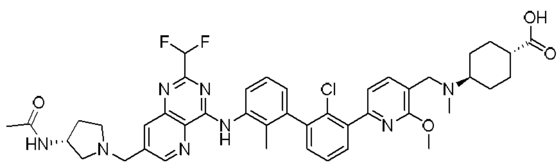 (37), 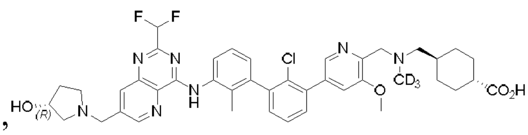 (38),

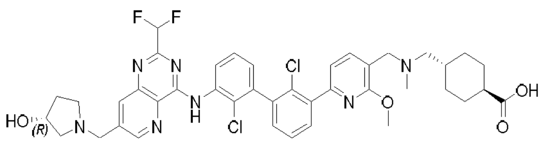 (39), 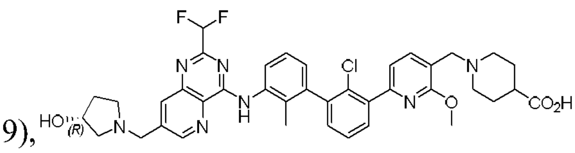 (40),

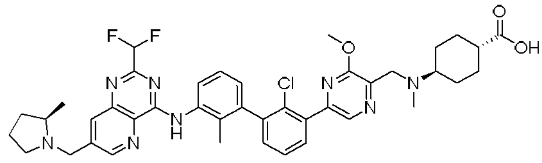 (41), 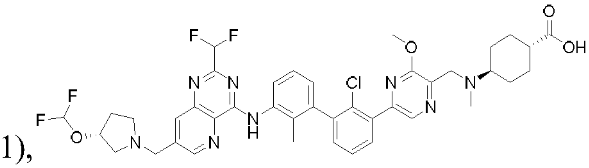 (42),

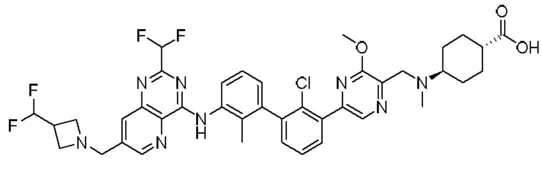 (43), 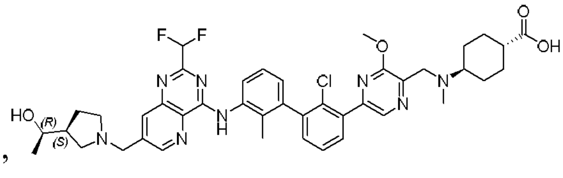 (44),

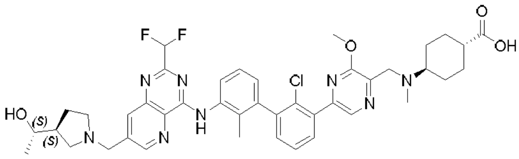 (45), 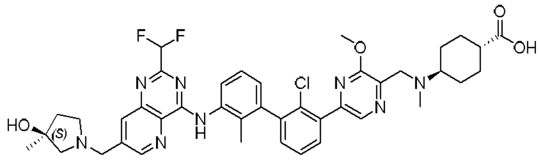 (46),

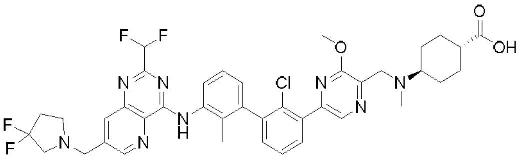 (47), 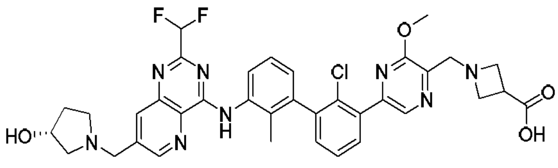 (48),

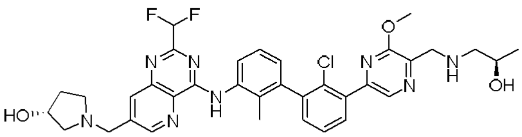 (49), 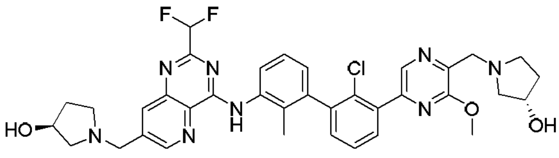 (50),

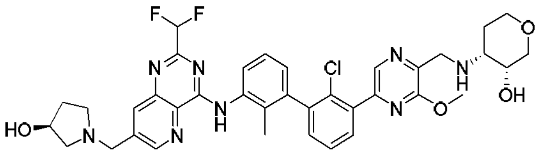 (51), 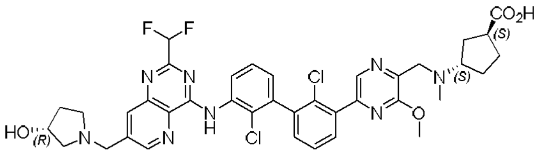 (52),

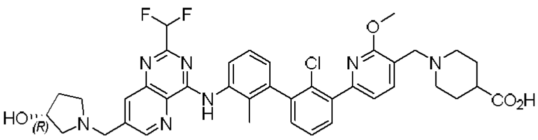 (53), 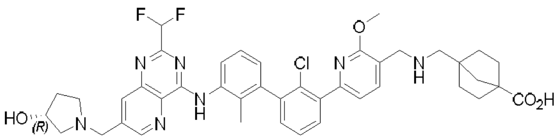 (54),

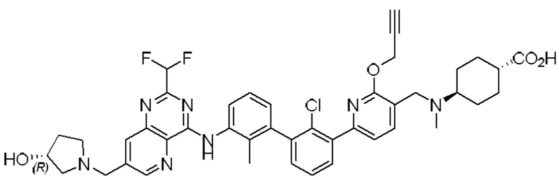 (55), 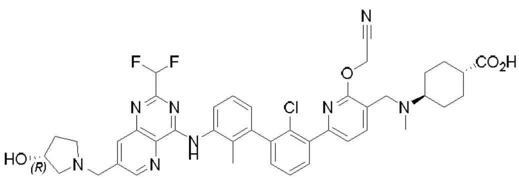 (56),

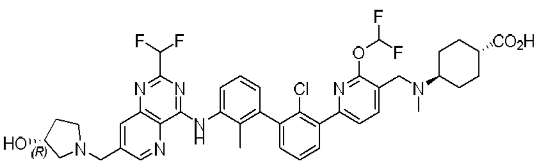 (57), 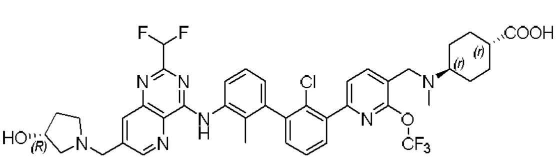 (58)

or 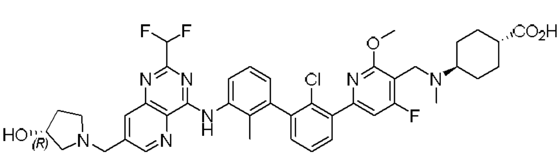 (59).

In one aspect, provided herein is a pharmaceutical composition comprising a compound of formula (I), (II), (III) or (IV), or a stereoisomer, a tautomer, an *N*-oxide, a hydrate, a solvate, a metabolite, a pharmaceutically acceptable salt or a prodrug thereof.

In some embodiments, the pharmaceutical composition disclosed herein further comprises pharmaceutically acceptable carriers, excipients, diluents, adjuvants, vehicles, or combination thereof.

In one aspect, the present invention relates to the use of a compound represented by formula (I), (II), (III) or (IV) or a pharmaceutical composition thereof in the preparation of a drug for treating a disease mediated by the PD-1/PD-L1 signaling pathway.

In some embodiments, the disease mediated by the PD-1/PD-L1 signaling pathway disclosed herein is cancer, an infectious disease, or an autoimmune disease.

In some embodiments, the cancer disclosed herein is selected from acute lymphoblastic leukemia, acute myeloid leukemia, chronic lymphocytic leukemia, chronic myelogenous leukemia, multiple myeloma, T-cell lymphoma, B-cell lymphoma, Waldenström's macroglobulinemia, pancreatic cancer, bladder cancer, colorectal cancer, breast cancer, prostate cancer, kidney cancer, liver cancer, lung cancer, ovarian cancer, cervical cancer, gastric cancer, esophageal cancer, head and neck cancer, melanoma, neuroendocrine cancer, CNS cancer, brain cancer, or bone cancer.

In some embodiments, the infectious disease disclosed herein is acquired immunodeficiency syndrome (HIV), hepatitis A, hepatitis B, hepatitis C, hepatitis D, herpes virus infection, papillomavirus infection, or influenza virus infection.

In some embodiments, the autoimmune disease according to the present invention is selected from Hashimoto's thyroiditis, hyperthyroidism, insulin-dependent diabetes mellitus, myasthenia gravis, ulcerative colitis, pernicious anemia with chronic atrophic gastritis, Goodpasture's syndrome, primary biliary cholangitis, multiple sclerosis, acute idiopathic polyneuritis, rheumatoid arthritis, systemic lupus erythematosus, systemic vasculitis, scleroderma, pemphigus, dermatomyositis, mixed connective tissue disease, or autoimmune hemolytic anemia.

In another aspect, the present invention provides a method for modulating an immune response mediated by the PD-1/PD-L1 signaling pathway in a patient, comprising administering to the subject a therapeutically effective amount of a compound of the present invention, thereby modulating the immune response in the patient.

In a further aspect, the present invention relates to methods for preparing, separating, and purifying the compounds of Formula (I), (II), (III) or (IV).

## PHARMACEUTICAL COMPOSITION OF THE COMPOUND OF THE INVENTION, PREPARATIONS AND ADMINISTRATION

As described in the present invention, the pharmaceutical composition of the present invention comprises any one of the compounds represented by formula (I), formula (II), formula (III) or formula (IV) of the present invention, and further comprises pharmaceutically acceptable excipients. These excipients, for example, as used in the present invention, include any solvent, solid excipient, diluent, binder, disintegrant, or other liquid excipient, dispersant, flavoring agent, suspending agent, surfactant, isotonic agent, thickener, emulsifier, preservative, solid binder or lubricant, etc., suitable for a specific target dosage form. As described in the following: In Remington: The Science and Practice of Pharmacy, 21st edition, 2005, ed. D.B. Troy, Lippincott Williams& Wilkins, Philadelphia, and Encyclopedia of Pharmaceutical Technology, eds. J. Swarbrick and J. C. Boylan, 1988-1999, Marcel Dekker, New York, both of which are herein incorporated by reference in their entireties, discloses various carriers used in formulating pharmaceutically acceptable compositions and known techniques for the preparation thereof. Except insofar as any conventional adjuvant incompatible with the compounds disclosed herein, such as by producing any undesirable biological effect or otherwise interacting in a deleterious manner with any other components of the pharmaceutically acceptable composition, its use is contemplated to be within the scope of this invention.

Some non-limiting examples of materials which can serve as pharmaceutically acceptable carriers include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants. When the compounds of the present invention are administered as pharmaceuticals to mammals, e.g., humans, they can be given per se or as a pharmaceutical composition containing, for example, 0.1 to 99.5% (more preferably, 0.5 to 90%) of active ingredient in combination with a pharmaceutically acceptable carrier.

"Therapeutically effective amount" or "effective amount" refers to a sufficient amount of one or more compounds of the present invention to treat, prevent, alleviate, ameliorate or eliminate one or more symptoms of a specific disease, disorder or syndrome, or to arrest or delay the onset of one or more symptoms of a specific disease, disorder or syndrome

described herein. In the case of treating cancer, a therapeutically effective amount of a drug can reduce the number of cancer cells; inhibit (i.e., slow to some extent or stop) the infiltration of cancer cells into surrounding organs; inhibit tumor metastasis; inhibit tumor growth to some extent; and/or alleviate to some extent one or more symptoms associated with cancer. In the case of infectious disease states, a therapeutically effective amount is an amount sufficient to reduce or alleviate the symptoms of infectious diseases (infections caused by bacteria, viruses and fungi).

The regimen of administration can affect what constitutes an effective amount. The compounds of the present invention may be administered to an individual before or after the onset of a disorder associated with the PD-1/PD-L1 signaling pathway. Further, several divided dosages, as well as staggered dosages, can be administered daily or sequentially, or the dose can be continuously infused, or can be a bolus injection. Further, the dosages of the compound(s) of the invention can be proportionally increased or decreased as indicated by the exigencies of the therapeutic or prophylactic situation.

Compounds of the invention may be used in the treatment of states, disorders or diseases as described herein, or for the manufacture of pharmaceutical compositions for use in the treatment of these diseases. Methods of use of compounds of the present invention in the treatment of these diseases, or pharmaceutical preparations having compounds of the present invention for the treatment of these diseases.

"Pharmaceutically acceptable carrier" is art recognized and includes a pharmaceutically acceptable material, composition or vehicle, suitable for administering compounds of the present invention to mammals. The carriers include liquid or solid filler, diluent, excipient, solvent or encapsulating material, involved in carrying the pharmaceutical active ingredient or transferring it from one organ or part of the body to another organ or part of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, α-tocopherol, and the like; and metal chelating agents, such as citric acid, ethylenediamine tetraacetic acid (EDTA), sorbitol, tartaric acid, phosphoric acid, and the like.

Formulations of the present invention include those suitable for oral, nasal, topical, buccal, sublingual, rectal, vaginal and/or parenteral administration. The formulations may conveniently be presented in unit dosage form and may be prepared by any methods well known in the art of pharmacy. The amount of active ingredient that can be combined with a carrier material to produce a single dosage form will generally be that amount of the compound that produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate,

potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluent commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants that may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the compound in the proper medium. Absorption enhancers can also be used to increase the flux of the compound across the skin. The rate of such flux can be

controlled by either providing a rate controlling membrane or dispersing the active compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of the drug in biodegradable polymers such as polylactide-polyglycolide. Depending on the ratio of drug to polymer, and the nature of the particular polymer employed, the rate of drug release can be controlled. Examples of other biodegradable polymers include poly (orthoesters) and poly (anhydrides). Depot injectable formulations are also prepared by entrapping the drug in liposomes or microemulsions that are compatible with body tissue.

The preparations of the present invention may be given orally, parenterally, topically, or rectally. They are of course given by forms suitable for each administration route. For example, they are administered in tablets or capsule form, by injection, inhalation, eye lotion, ointment, suppository, etc., administration by injection, infusion or inhalation; topical by lotion or ointment; and rectal by suppositories. Oral and/or IV administration is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracistemally and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the

compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound that is the lowest dose effective to produce a therapeutic effect. Such an effective dose will generally depend upon the factors described above. Generally, the dosage of the compounds of the present invention for patients is about 0.001 to about 100 mg/kg of body weight per day, more preferably from about 0.01 to about 80 mg/kg of body weight per day, and still more preferably from about 1.0 to about 50 mg/kg of body weight per day.

If desired, the effective daily dose of the active compound may be administered as two, three, four, five, six or more sub-doses separately at appropriate intervals throughout the day, optionally, in unit dosage forms.

The pharmaceutical composition or combination of the present invention can be in unit dosage of about 1-1000 mg of active ingredients for a subject of about 50-70 kg, preferably about 1-500 mg or about 1-250 mg or about 1-150 mg or about 1-100 mg or about 1-50 mg of active ingredients. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable in vitro and in vivo tests using advantageously mammals, e.g., mice, rats, dogs, monkeys or isolated organs, tissues and preparations thereof. The compounds of the present invention can be applied in vitro in the form of solutions, e.g., preferably aqueous solutions, and in vivo either enterally, parenterally, advantageously intravenously, e.g., as a suspension or in aqueous solution. A therapeutically effective amount in vivo may range depending on the route of administration, between about 0.1-500 mg/kg, or between about 1-100 mg/kg.

As used herein, the term "subject" refers to an animal. Typically, the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

Although the compounds of the present invention can be administered alone, but preferably administered in the form of a pharmaceutical composition of the compound.

## DRUG COMBINATIONS

Combination therapy using one or more compounds or compositions provided by the present invention, or pharmaceutically acceptable derivatives thereof, in combination with other drug activators such as ALK inhibitor ceritinib, NTRK inhibitor entrectinib, cisplatin, and carboplatin is used to treat the diseases and conditions described herein.

In practicing the methods, effective amounts of the compounds or compositions containing therapeutically effective concentrations of the compounds, which are formulated for oral, systemic, including parenteral or intravenous delivery, or for local or topical application are administered to an individual exhibiting the symptoms of the disease or disorder to be treated. The amounts are effective to treat, manage or ameliorate the disease or ameliorate or eliminate one or more symptoms of the disease or disorder.

Furthermore, it will be understood by those skilled in the art that the compounds, isomers, prodrugs and pharmaceutically acceptable derivatives provided herein, including pharmaceutical compositions and formulations containing these compounds, can be used in a wide variety of combination therapies to treat the conditions and diseases described above. Thus, also contemplated herein is the use of compounds, isomers, prodrugs and pharmaceutically acceptable derivatives provided herein in combination with other active pharmaceutical agents for the treatment of the disease/conditions described herein.

## GENERAL SYNTHETIC PROCEDURES

Generally, the compounds disclosed herein may be prepared by methods described herein, wherein the substituents are as defined for Formula (I), (II), (III) or (IV) above, except where further noted. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius

(°C). Reagents were purchased from commercial suppliers such as Aldrich Chemical Company, Arco Chemical Company and Alfa Chemical Company, and were used without further purification unless otherwise indicated. Common solvents were purchased from commercial suppliers such as Shantou XiLong Chemical Factory, Guangdong Guanghua Reagent Chemical Factory Co. Ltd., Guangzhou Reagent Chemical Factory, Tianjin YuYu Fine Chemical Ltd., Qingdao Tenglong Reagent Chemical Ltd., and Qingdao Ocean Chemical Factory.

Anhydrous THF, anhydrous dioxane, anhydrous toluene, and anhydrous ether were obtained by refluxing the solvent with sodium. Anhydrous $CH_2Cl_2$ and anhydrous $CHCl_3$ were obtained by refluxing the solvent with $CaH_2$. Anhydrous EtOAc, anhydrous PE, anhydrous hexane, anhydrous DMAC and anhydrous DMF were treated with anhydrous $Na_2SO_4$ prior to use.

The reactions set forth below were done generally under a positive pressure of nitrogen or argon or with a drying tube (unless otherwise stated) in anhydrous solvents, and the reaction flasks were typically fitted with rubber septa for the introduction of substrates and reagents via syringe. Glassware was oven dried and/or heat dried. Glassware was oven dried and/or heat dried.

Column chromatography was conducted using a silica gel column. Silica gel (300-400 mesh) was purchased from Qingdao Ocean Chemical Factory. The thin layer chromatography silica gel plate uses Yantai Huanghai HSGF254 or Qingdao GF254 silica gel plate, and the specification used for thin layer chromatography separation and purification products is 0.4mm~0.5mm.

1H NMR spectra were obtained by using $CDCl_3$, DMSO-$d_6$, $CD_3OD$ or acetone-$d_6$ solutions (reported in ppm), with TMS (0 ppm) or chloroform (7.25 ppm) as the reference standard. When peak multiplicities are reported, the following abbreviations are used: s (singlet), d (doublet), t (triplet), q (quartet), m (multiplet), br (broadened), dd (doublet of doublets), ddd (doublet doublet of doublets), dt (doublet of triplets), dq (doublet of quartets). Coupling constants *J*, when given, were reported in Hertz (*Hz*).

Low-resolution mass spectral (MS) data were also determined on an Agilent 6120 series LC-MS spectrometer equipped with G1311B binary pumps, a G1316A TCC (Temperature Control of Column, maintained at 30 °C), a G1329B autosampler and a G1315D DAD detector were used in the analysis. An ESI source was used on the LC-MS spectrometer.

Both LC-MS spectrometers were equipped with an Agilent Zorbax SB-C18, 2.1 × 30 mm, 5 μm column. Injection volume was decided by the sample concentration. The flow rate was 0.6 mL/min. The HPLC peaks were recorded by UV-Vis wavelength at 210 nm and 254 nm. The mobile phase was 0.1% formic acid in acetonitrile (phase A) and 0.1% formic acid in ultrapure water (phase B). The gradient elution conditions were showed in Table 1: The gradient elution conditions were showed in Table 1:

Table 1

| Time (min) | A ($CH_3CN$, 0.1% HCOOH) | B ($H_2O$, 0.1% HCOOH) |
|---|---|---|
| 0 - 3 | 5 - 100 | 95 - 0 |
| 3 - 6 | 100 | 0 |
| 6 - 6.1 | 100 - 5 | 0 - 95 |
| 6.1 - 8 | 5 | 95 |

Purities of compounds were assessed by Agilent 1100 Series high performance liquid chromatography (HPLC) with UV detection at 210 nm and 254 nm (Zorbax SB-C18, 2.1 × 30 mm, 4 micorn, 10 min, 0.6 mL/min flow rate, 5 to 95 % (0.1 % formic acid in $CH_3CN$) in (0.1 % formic acid in $H_2O$). Column was operated at 40 °C.

The following abbreviations are used throughout the specification:

| | | | |
|---|---|---|---|
| BOC, Boc | *tert*-butoxycarbonyl | mg | milligram |
| $CHCl_3$ | chloroform | mL, ml | milliliter |
| mM | millimole per liter | μL, μl | microlitre |
| M, N | mole per liter | g | gram |
| $CDCl_3$ | chloroform-d | PE | petroleum ether |
| DCM | dichloromethane | TFA | 2,2,2-trifluoroacetic acid |
| MeOH, $CH_3OH$ | methanol | h | hour, hours |
| DMF | *N,N*- dimethylformamide | $H_2O$ | water |
| DMSO | dimethylsulfoxide | $N_2$ | nitrogen |
| DMSO-$d_6$ | dimethyl sulfoxide-$d_6$ | $(o\text{-}tol)_3P$ | tris(2-methylphenyl)phosphine |
| EA, EtOAc | ethyl acetate | $K_2CO_3$ | potassium carbonate |

(continued)

| | | | |
|---|---|---|---|
| $Pd(OAc)_2$ | Palladium diacetate | ACN | acetonitrile |
| TEA | triethylamine | DMP | Dess-Martin periodinane |
| *t*-BuOH | *t*-butanol | $NaBH_3CN$ | sodium cyanoborohydride |
| AcOH | acetic acid | DAST | diethylaminosulphur trifluoride |
| X-Phos | 2-(Dicyclohexylphosphino)-2,4,6-Triisopropylbiphenyl | | |
| $Pd(dppf)Cl_2$ | [1,1'-bis(diphenylphosphino)ferrocene]dichloropalladium(II) | | |
| $Pd(dppf)Cl_2\cdot CH_2$ | $Cl_2$ 1,1'-bis(diphenylphosphino)ferrocene-palladium(II)dichloride dichloromethane complex | | |

The following synthetic schemes and synthetic intermediate schemes describe the steps for preparing the compounds disclosed in the present invention, unless otherwise stated, wherein, each of $R^1$, $R^2$, $R^{3a}$, $R^4$, $R^5$, $R^6$, $R^g$, n1, X and Y is as defined herein.

## Schenme 1

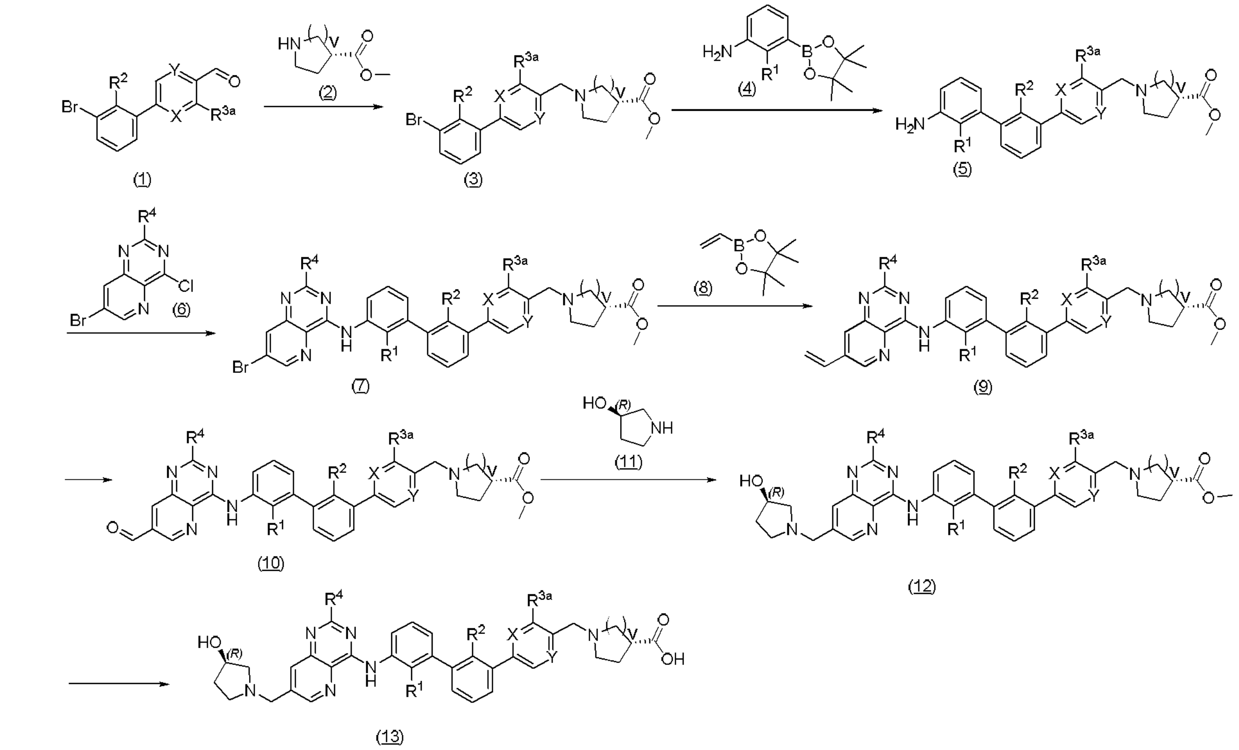
(1) (2) (3) (4) (5) (6) (7) (8) (9) (10) (11) (12) (13)

In the formula, v is 0, 1, 2 or 3.

The compound of formula (13) can be prepared by synthetic scheme 1: the compound of formula (1) and the compound of formula (2) undergo reductive amination reaction to obtain the compound of formula (3). The compound of formula (3) undergoes a coupling reaction with the compound of formula (4) to obtain the compound of formula (5). The compound of formula (5) and the compound of formula (6) undergo nucleophilic substitution reaction to obtain the compound of formula (7). The compound of formula (7) and the compound of formula (8) are reacted by coupling reaction to obtain the compound of formula (9). The compound of formula (9) is subjected to oxidation reaction to obtain the compound of formula (10). The compound of formula (10) undergoes reductive amination with the compound of formula (11) to obtain the compound of formula (12). The compound of formula (12) undergoes ester hydrolysis to obtain the compound of formula (13).

## Schenme 2

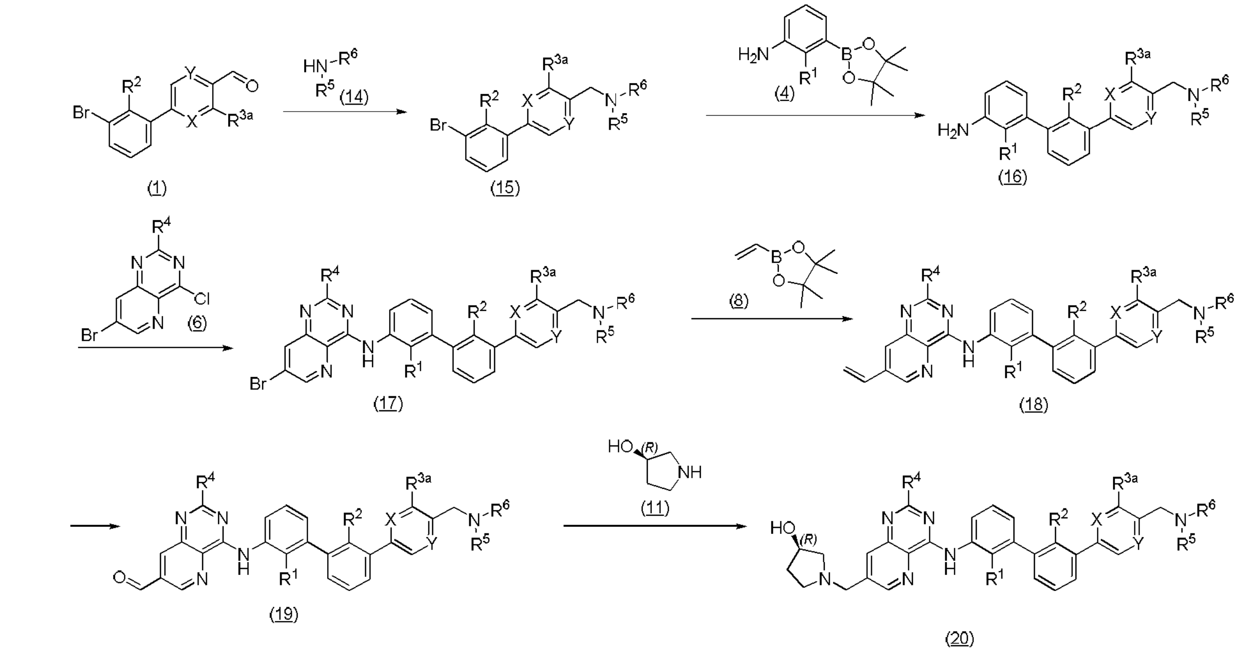


The compound of formula (20) can be prepared by synthetic scheme 2: the compound represented by formula (1) and the compound represented by formula (14) undergo reductive amination reaction to obtain the compound represented by formula (15). The compound of formula (15) undergoes a coupling reaction with the compound of formula (4) to obtain the compound of formula (16). The compound of formula (16) and the compound of formula (6) undergo nucleophilic substitution reaction to obtain the compound of formula (17). The compound of formula (17) and the compound of formula (8) are reacted by coupling reaction to obtain the compound of formula (18). The compound of formula (18) is subjected to oxidation reaction to obtain the compound of formula (19). The compound of formula (19) undergoes reductive amination with the compound of formula (11) to obtain the compound of formula (20).

## Schenme 3

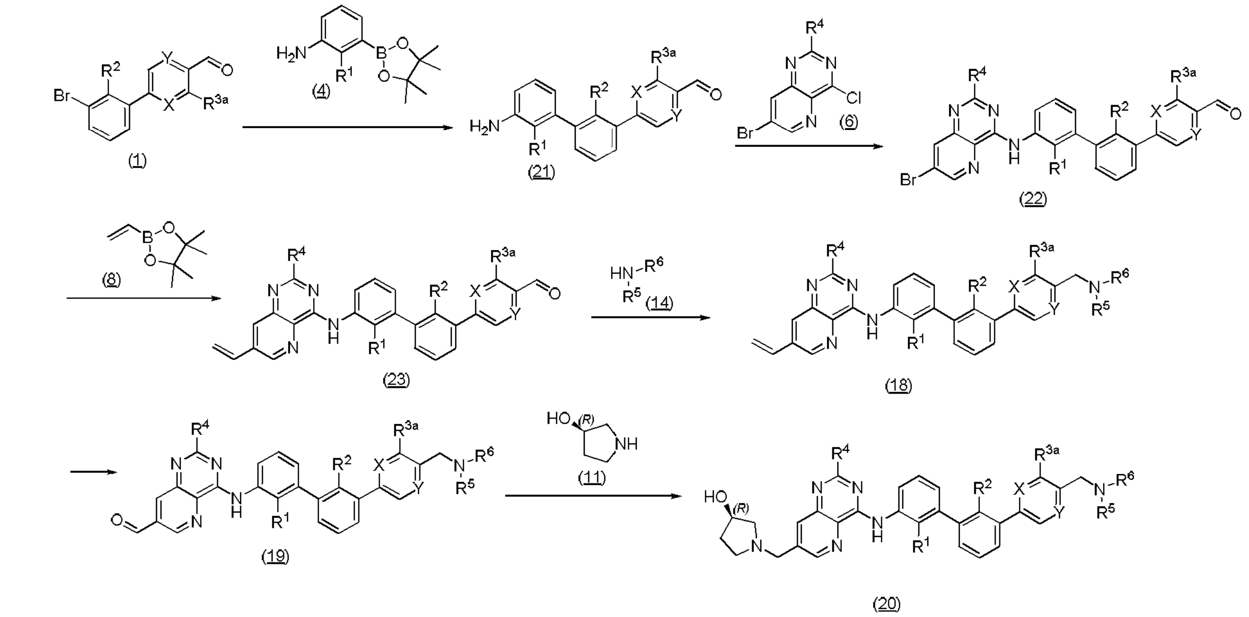


The compound of formula (20) can be prepared by synthetic scheme 3: the compound of formula (1) and the compound of formula (4) undergo coupling reaction to obtain the compound of formula (21). The compound of formula (21) and the compound of formula (6) undergo nucleophilic substitution reaction to obtain the compound of formula (22). The

compound of formula (22) and the compound of formula (8) are reacted by coupling reaction to obtain the compound of formula (23). The compound of formula (23) undergoes reductive amination with the compound of formula (14) to obtain the compound of formula (18). The compound of formula (18) is subjected to oxidation reaction to obtain the compound of formula (19). The compound of formula (19) undergoes reductive amination with the compound of formula (11) to obtain the compound of formula (20).

## Schenme 4

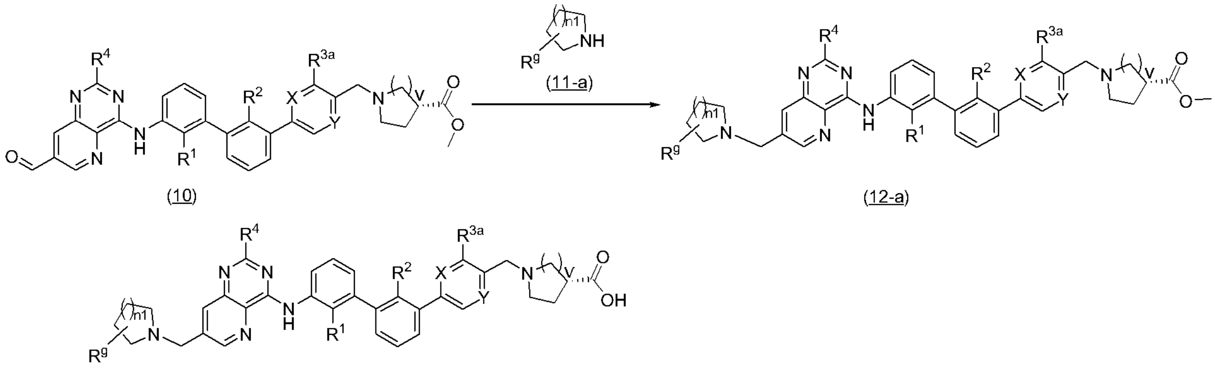

(10) (12-a)

(13-a)

The compound of formula (13-a) can be prepared by synthetic scheme 4: the compound of formula (10) and the compound of formula (11-a) undergo reductive amination reaction to obtain the compound of formula (12-a). The compound of formula (12-a) undergoes ester hydrolysis to obtain the compound of formula (13-a).

## Schenme 5

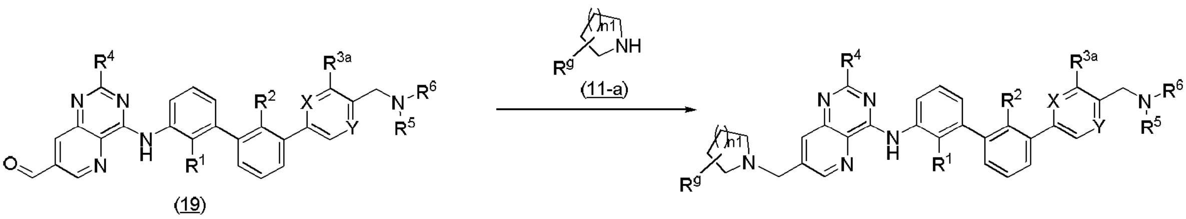

(19) (20-a)

The compound of formula (20-a) can be prepared by synthetic scheme 5: the compound of formula (19) and the compound of formula (11-a) undergo reductive amination reaction to obtain the compound of formula (20-a).

The following examples disclosed herein are presented to further describe the invention. However, these examples should not be used to limit the scope of the invention. When the structure and name of the compound of the present invention are inconsistent, the structure of the compound shall prevail.

### Example

### Intermediate fragment 1: 6-(3-bromo-2-chlorophenyl)-2-methoxynicotinaldehyde

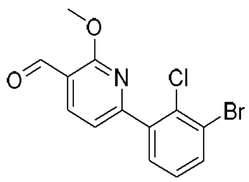

(3-Bromo-2-chlorophenyl)boronic acid (50 g, 212.5 mmol), 6-chloro-2-methoxynicotinaldehyde (36.5 g, 212.5 mmol), $Pd(PPh_3)_2Cl_2$ (7.46 g, 10.63 mmol), (*o*-tol)$_3$P (3.2 g, 10.6 mmol), $K_2CO_3$ (58.7 g, 425.0 mmol) were dissolved in 1,4-dioxane (1000 mL) and water (200 mL). The reaction mixture was stirred under $N_2$ atmosphere at 65 °C overnight. After the reaction solution was cooled, it was filtered through a Celite pad. The filtrate was concentrated under reduced pressure,

washed with an appropriate amount of ethyl acetate, and filtered to obtain 63 g of a white solid product with a yield of 62%.

MS (ESI, pos. ion) m/z: 326.1 $[M+H]^+$.

**Example 1 (1*r*,4*r*)-4-(((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)cyclohexane-1-carboxylic acid**

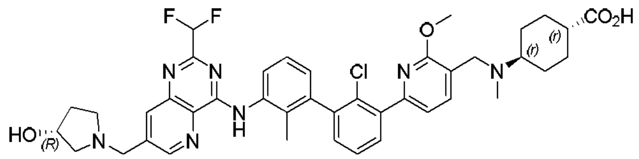

**Step 1: Synthesis of methyl (1*r*,4*r*)-4-(((6-(3-bromo-2-chlorophenyl)-2-methoxypyridin-3-yl)methyl)amino)cyclohexane-1-carboxylate**

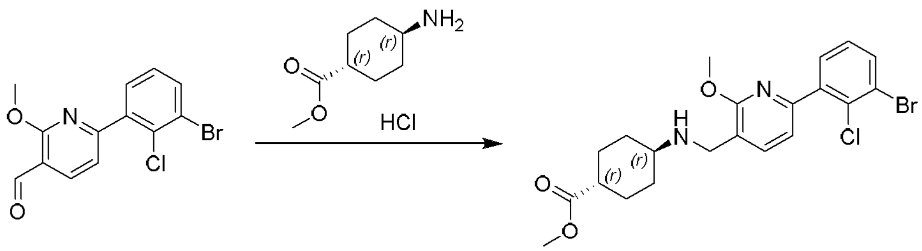


To a 500 mL single-necked bottle, 6-(3-bromo-2-chlorophenyl)-2-methoxy pyridin-3-aldehyde (7.5 g, 22.97 mmol), methyl (1r,4r)-4-aminocyclohexane-1-carboxylate hydrochloride (11.12 g, 57.42 mmol), DCM (100 mL), methanol (100 mL), triethylamine (3.49 g, 34.45 mmol) and acetic acid (2.07 g, 34.45 mmol) were added in sequence, and then stirred at 50 °C for 3 h. Sodium cyanoborohydride (2.89 g, 45.94 mmol) was added and the reaction was continued for 2 hours. After the reaction was complete, the pH was adjusted to 7-8 with saturated aqueous potassium carbonate solution, concentrated under reduced pressure, and then extracted with DCM (240 mL), dried over anhydrous sodium sulfate, filtered, and the filtrate was concentrated under reduced pressure to obtain 10.5 g of a light yellow oily liquid product with a yield of 97.7%.

MS (ESI, pos. ion) m/z: 467.1 $[M+H]^+$.

**Step 2: Synthesis of methyl (1*r*,4*r*)-4-(((6-(3-bromo-2-chlorophenyl)-2-methoxypyridin-3-yl)methyl)(methyl)amino)cyclohexane-1-carboxylate**

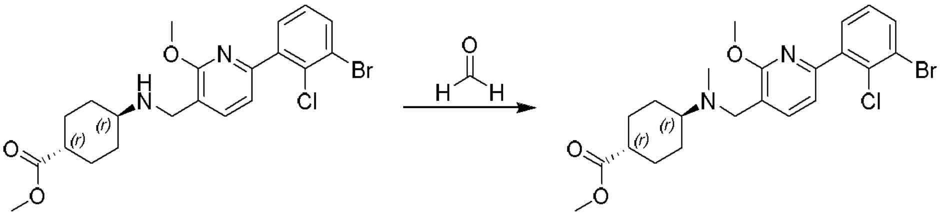

To a 500 mL single-necked bottle, methyl (1*r*,4*r*)-4-(((6-(3-bromo-2-chlorophenyl)-2-methoxypyridin-3-yl)methyl)amino)cyclohexane-1-carboxylate (10.5 g, 22.45 mmol), formaldehyde (5.06 g, 67.35 mmol), DCM (100 mL), methanol (100 mL), triethylamine (2.50 g, 24.70 mmol) and acetic acid (1.48 g, 24.70 mmol) were added in sequence, and then stirred at 50 °C for 3.5 h. Sodium cyanoborohydride (4.23 g, 67.35 mmol) was added and the reaction was continued for 2 hours. After the reaction was complete, the pH was adjusted to 7-8 with saturated aqueous potassium carbonate solution, concentrated under reduced pressure, and then extracted with DCM (240 mL), dried over anhydrous sodium

sulfate, filtered, and the filtrate was concentrated under reduced pressure. The residue was purified by silica gel column chromatography eluted with PE/EtOAc (v/v) = 3/1 to give a white solid product (10.10 g, yield 93.4%).

MS (ESI, pos. ion) m/z: 481.1 [M+H]+.

**Step 3: Synthesis of methyl (1*r*,4*r*)-4-(((6-(3'-amino-2-chloro-2'-methyl-[1,1'-biphenyl]-3-yl)-2-methoxypyridin-3-yl)methyl)(methyl)amino)cyclohexane-1-carboxylate**

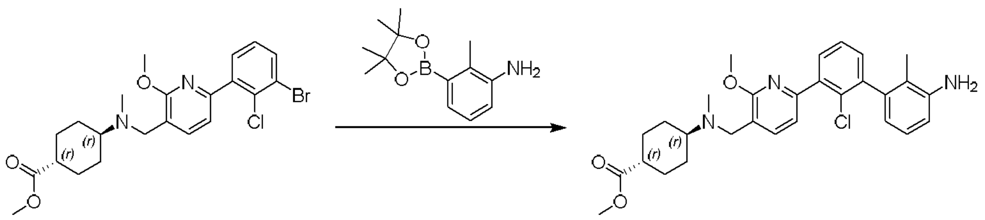

To a 30 mL microwave tube, methyl (1*r*,4*r*)-4-(((6-(3-bromo-2-chlorophenyl)-2-methoxypyridin-3-yl)methyl)(methyl)amino)cyclohexane-1-carboxylate (1.5 g, 3.11 mmol), 2-methyl-3-(4,4,5,5-tetramethyl-1,3,2-dioxaborolan-2-yl)aniline (0.94 g, 4.04 mmol), potassium carbonate (1.29 g, 9.33 mmol), Pd(dppf)$Cl_2$ $CH_2Cl_2$ (0.25 g, 0.31 mmol), 1,4-dioxane (15 mL) and water (3 mL) were added in sequence. The tube was sealed and reacted at 130 °C for 2 h. After the reaction was completed, the reaction solution was concentrated under reduced pressure, and the residue was separated and purified by silica gel column chromatography (eluent: PE/EtOAc (v/v) = 2/1) to obtain 1.32 g of a light yellow oily product with a yield of 83.5%.

MS (ESI, pos. ion) m/z: 508.5 [M+H]+.

**Step 4: Synthesis of methyl (1*r*,4*r*)-4-(((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)(methyl)amino)cyclohexane-1-carboxylate**

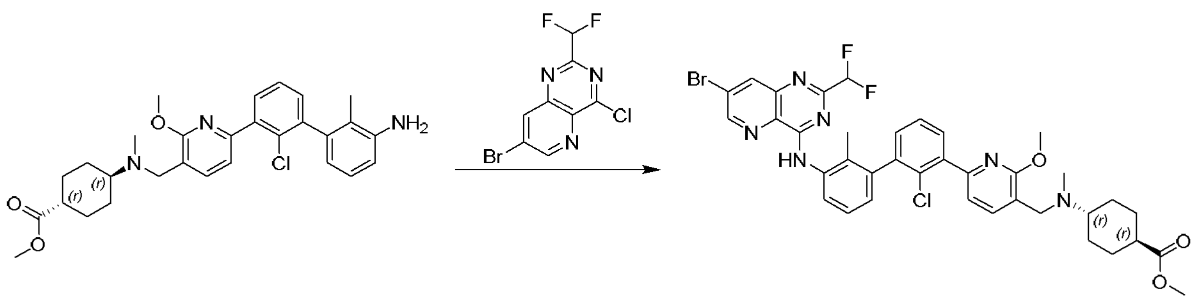

Into a 50 mL single-necked bottle, methyl (1*r*,4*r*)-4-(((6-(3'-amino-2-chloro-2'-methyl-[1,1'-biphenyl]-3-yl)-2-methoxypyridin-3-yl)methyl)(methyl)amino)cyclohexane-1-carboxylate (1.2 g, 2.36 mmol), 7-bromo-4-chloro-2-(difluoromethyl)pyrido[3,2-d]pyrimidine (0.69 g, 2.36 mmol) and tert-butanol (20 mL) were added in sequence and reacted at 130 °C for 3 h. After the reaction, the mixture was filtered. The filtrate was concentrated. The residue was purified by silica gel chromatography eluted with PE/EtOAc (v/v) = 2/1 to give the product as a light yellow oil (533.0 mg, yield 29.5%).

MS (ESI, pos. ion) m/z: 765.6 [M+H]+.

**Step 5: Synthesis of methyl (1*r*,4*r*)-4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)(methyl)amino)cyclohexane-1-carboxylate**

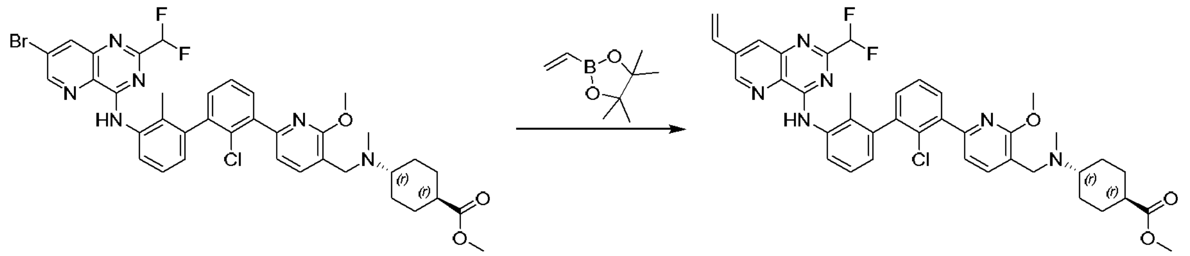

To a 30 mL microwave tube, methyl (1r,4r)-4-(((6-(3'-((7-bromo-2-(difluoromethyl)pyrido[3,2-*d*]pyrimidin-4-yl) amino)-2-chloro-2'-methyl-[1,1'-biphenyl]-3-yl)-2-methoxypyridin-3-yl)methyl)(methyl)amino)cyclohexane-1-carboxylate (0.48 g, 0.63 mmol), 4,4,5,5-tetramethyl-2-vinyl-1,3,2-dioxaborolane (0.29 g, 1.89 mmol), potassium phosphate (0.33 g, 1.57 mmol), Pd(dppf)$Cl_2$ $CH_2Cl_2$ (0.051 g, 0.063 mmol), 1,4-dioxane (10 mL) and water (2 mL) were added in sequence, the tube was sealed, and the reaction was carried out at 120 °C for 3 h. After the reaction was completed, the reaction solution was concentrated under reduced pressure, and the residue was separated and purified by silica gel column chromatography (eluent: PE/EtOAc = 1/1) to obtain 324.0 mg of a light yellow solid product with a yield of 72.5%.

MS (ESI, pos. ion) m/z: 713.2 $[M+H]^+$.

**Step 6: Synthesis of methyl (1*r*,4*r*)-4-(((6-(2-chloro-3'-((2-(difluoromethyl)-7-formylpyrido[3,2-*d*]pyrimidin-4-yl) amino)-2'-methyl-[1,1'-biphenyl]-3-yl)-2-methoxypyridin-3-yl)methyl)(methyl)amino)cyclohexane-1-carboxylate**

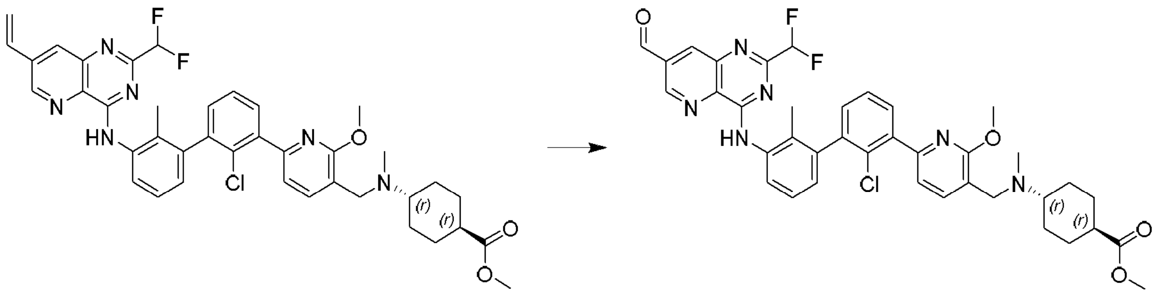

To a 50 mL single-necked bottle, methyl (1*r*,4*r*)-4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)(methyl)amino)cyclohexane-1-carboxylate (324 mg, 0.45 mmol), 1,4-dioxane (20 mL), water (8 mL), potassium osmate dihydrate (16.58 mg, 0.045 mmol) and sodium periodate (481.25 mg, 2.25 mmol) were added in sequence and reacted at room temperature for 1.5 h. After the reaction was complete, the mixture was concentrated under reduced pressure to remove dioxane, water (100 mL) was added, and then extracted with DCM (20 mL × 3). The organic phases were combined, washed with saturated brine (50 mL × 1), dried over anhydrous sodium sulfate, filtered, and the filtrate was concentrated under reduced pressure. The residue was separated and purified by silica gel column chromatography (eluent: DCM/MeOH (v/v) = 30/1) to obtain 255.0 mg of a light yellow solid product with a yield of 78.5%.

MS (ESI, pos. ion) m/z: 715.6 $[M+H]^+$.

**Step 7: Synthesis of methyl (1*r*,4*r*)-4-(((6-(2-chloro-3'-((2-(difluoromethyl)-7-(((*R*)-3-hydroxypyrrolidin-1-yl) methyl)pyrido[3,2-*d*]pyrimidin-4-yl)amino)-2'-methyl-[1,1'-biphenyl]-3-yl)-2-methoxypyridin-3-yl)methyl) (methyl)amino)cyclohexane-1-carboxylate**

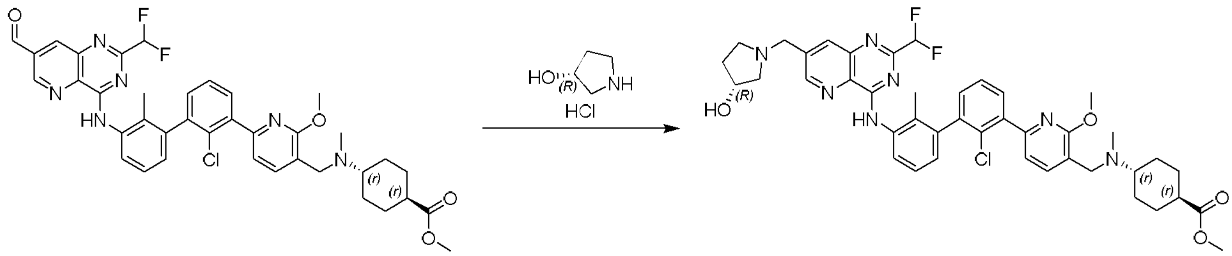


To a 100 mL single-necked bottle, methyl (1*r*,4*r*)-4-(((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)(methyl)amino)cyclohexane-1-carboxylate (255 mg, 0.36 mmol), (R)-pyrrolidin-3-ol hydrochloride (222.44 mg, 1.80 mmol), DCM (15 mL), DMF (15 mL), triethylamine (43.71 mg, 0.43 mmol) and acetic acid (25.94 mg, 0.43 mmol) were added in sequence and reacted at 50 °C for 2 h. Sodium cyanoborohydride (45.24 mg, 0.72 mmol) was added and the reaction was continued at room temperature. After the reaction was completed, the pH was adjusted to 7-8 with saturated potassium carbonate aqueous solution, then extracted with DCM (20 mL × 3), the organic phases were combined, washed with saturated brine (50 mL × 1), dried over anhydrous sodium sulfate, filtered, and the filtrate was concentrated under reduced pressure. The residue was separated and purified by silica gel column chromatography (eluent: DCM/MeOH (v/v) = 15/1) to obtain 30.0 mg of a light yellow solid product with a yield of 10.70%.

MS (ESI, pos. ion) m/z: 786.7 [M+H]+.

**Step 8: Synthesis of (1*r*,4*r*)-4-(((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)cyclohexane-1-carboxylic acid**

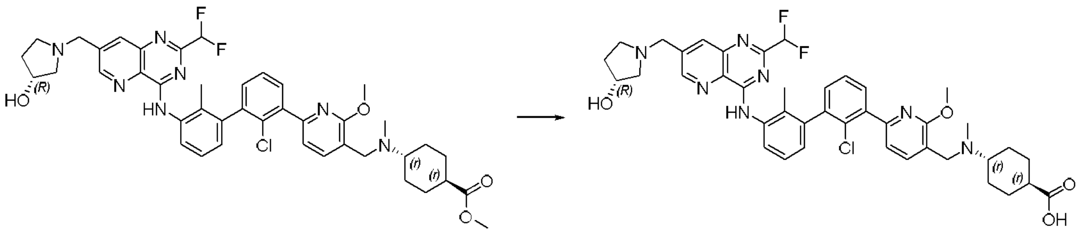

To a 25 mL single-necked bottle, methyl (1*r*,4*r*)-4-(((6-(2-chloro-3'-((2-(difluoromethyl)-7-(((*R*)-3-hydroxypyrrolidin-1-yl)methyl)pyrido[3,2-d]pyrimidin-4-yl)amino)-2'-methyl-[1,1'-biphenyl]-3-yl)-2-methoxypyridin-3-yl)methyl)(methyl)amino)cyclohexane-1-carboxylate (30 mg, 0.038 mmol), 1,4-dioxane (6 mL), water (2 mL) and lithium hydroxide monohydrate (7.97 mg, 0.19 mmol) were added in sequence and reacted at 50 °C. After the reaction was complete, the reaction mixture was concentrated under reduced pressure, a mixture of dichloromethane and methanol (DCM/MeOH (v/v) = 1:1) was added to dissolve the mixture, the pH was adjusted to 7-8 with 1 M HCl aqueous solution, and the mixture was concentrated under reduced pressure. The residue was separated and purified by silica gel column chromatography (eluent: DCM/MeOH (v/v) = 7.5/1) to obtain 13.3 mg of a white solid product with a yield of 45.2%.

MS (ESI, pos. ion) m/z: 772.31 [M+H]+.

$^1$H NMR (400 MHz, DMSO-*d*$_6$) δ (ppm) 10.33 (s, 1H), 8.94 (s, 1H), 8.16 (s, 1H), 7.78 (d, *J*=7.6 Hz, 1H), 7.74 (d, *J* = 8.3 Hz, 1H), 7.64 (d, *J* = 7.6 Hz, 1H), 7.52 (t, *J* = 7.5 Hz, 1H), 7.42 - 7.32 (m, 2H), 7.27 (d, *J* = 7.4 Hz, 1H), 7.15 (d, *J* = 7.4 Hz, 1H), 6.70 (t, *J* = 54.6 Hz, 1H), 4.26 - 4.19 (m, 1H), 3.90 (s, 3H), 3.88 - 3.80 (m, 1H), 3.54 (s, 4H), 2.76 - 2.64 (m, 2H), 2.43 - 2.38 (m, 2H), 2.17 (s, 3H), 2.04 (s, 3H), 1.99 - 1.76 (m, 6H), 1.65 - 1.52 (m, 1H), 1.40 - 1.25 (m, 6H).

**Example 2 (1*r*,4*r*)-4-(((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)amino)cyclohexane-1-carboxylic acid**

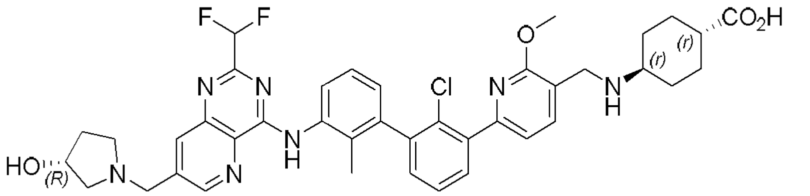

**Step 1: Synthesis of 6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-aldehyde**

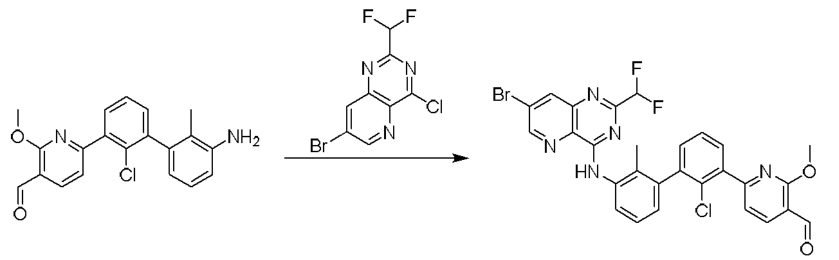

To a 100 mL single-necked bottle, 6-(3'-amino-2-chloro-2'-methyl-[1,1'-biphenyl]-3-yl)-2-methoxypyridin-3-aldehyde (1.0 g, 2.83 mmol), tert-butanol (50 mL) and 7-bromo-4-chloro-2-(difluoromethyl)pyrido[3,2-d]pyrimidine (1.25 g, 4.25 mmol) were added in sequence and reacted at 100 °C. After the reaction was complete, the mixture was concentrated under reduced pressure, diluted with water (100 mL), and then extracted with DCM (20 mL × 3). The organic phases were combined, washed with saturated brine (50 mL × 1), dried over anhydrous sodium sulfate, filtered, and the filtrate was concentrated under reduced pressure. The residue was separated and purified by silica gel column chromatography (eluent: PE/EtOAc (v/v) = 5/1) to obtain 1.68 g of a light yellow solid product with a yield of 97.03%.

MS (ESI, pos. ion) m/z: 610.4 $[M+H]^+$.

**Step 2: Synthesis of methyl (1*r*,4*r*)-4-(((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)amino)cyclohexane-1-carboxylate**

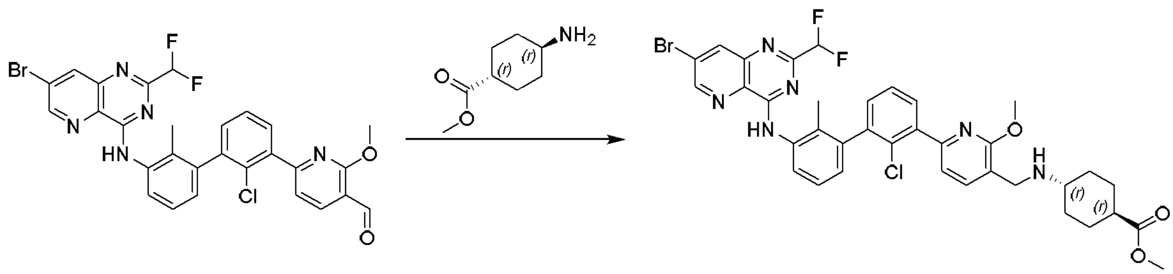

To a 100 mL single-necked bottle, 6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-aldehyde (1.68 g, 2.75 mmol), DCM (20 mL), methanol (20 mL), methyl (1r,4r)-4-aminocyclohexane-1-carboxylate hydrochloride (1.60 g, 8.25 mmol), triethylamine (0.42 g, 4.13 mmol) and acetic acid (0.25 g, 4.13 mmol) were added in sequence and reacted at 50 °C for 4 h. After the reaction, the pH was adjusted to 7-8 with saturated potassium carbonate aqueous solution, the mixture was concentrated. The residue was purified by silica gel chromatography eluted with DCM/MeOH (v/v) = 20/1 to give the product as a light yellow solid (1.7 g, 82.2%).

MS (ESI, pos. ion) m/z: 751.2 $[M+H]^+$.

**Step 3: Synthesis of methyl (1*r*,4*r*)-4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)amino)cyclohexane-1-carboxylate**

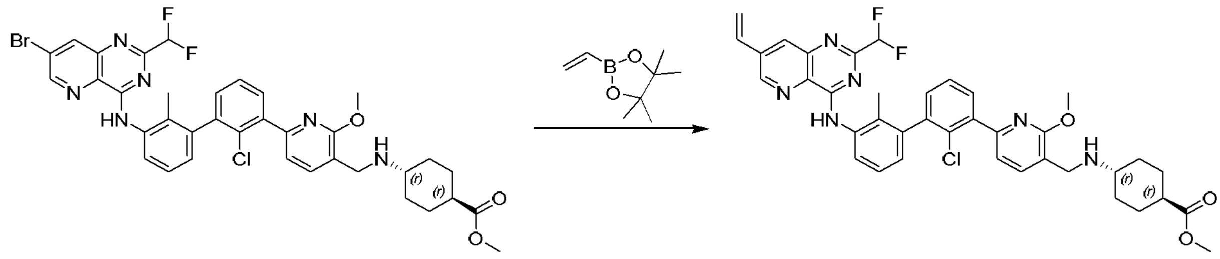

To a 25 mL single-necked bottle, methyl (1*r*,4*r*)-4-(((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)amino)cyclohexane-1-carboxylate (1.1 g, 1.46 mmol), vinylboronic acid pinacol ester (0.67 g, 4.38 mmol), potassium phosphate (0.77 g, 3.65 mmol), Pd(dppf)$Cl_2$ $CH_2Cl_2$ (0.12 g, 0.15 mmol), 1,4-dioxane (10 mL) and water (2 mL) were added and reacted at 130 °C under nitrogen atmosphere. After the reaction, the mixture was filtered. The filtrate was concentrated. The residue was purified by silica gel chromatography eluted with DCM/MeOH (v/v) = 20/1 to give the product as a light yellow solid (0.98 g, 95.8%).

MS (ESI, pos. ion) m/z: 699.3 $[M+H]^+$.

**Step 4: Synthesis of methyl (1*r*,4*r*)-4-(((6-(2-chloro-3'-((2-(difluoromethyl)-7-formylpyrido[3,2-*d*]pyrimidin-4-yl) amino)-2'-methyl-[1,1'-biphenyl]-3-yl)-2-methoxypyridin-3-yl)methyl)amino)cyclohexane-1-carboxylate**

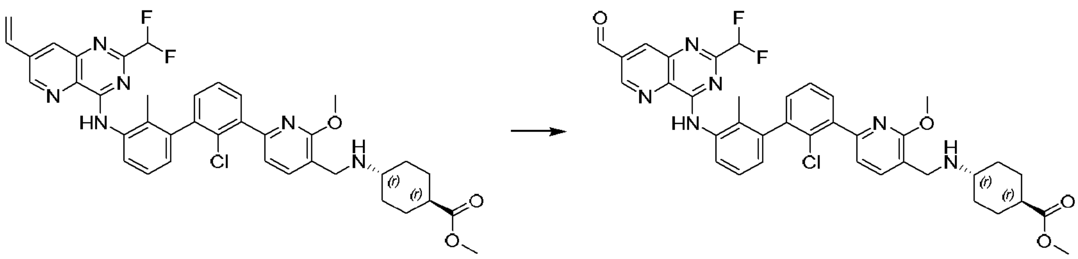

To a 100 mL single-necked bottle, methyl (1*r*,4*r*)-4-(((6-(2-chloro-3'-((2-(difluoromethyl)-7-vinylpyrido[3,2-*d*] pyrimidin-4-yl)amino)-2'-methyl-[1,1'-biphenyl]-3-yl)-2-methoxypyridin-3-yl)methyl)amino)cyclohexane-1-carboxylate (760 mg, 1.09 mmol), 1,4-dioxane (37.5 mL), water (15 mL), potassium osmate dihydrate (40.16 mg, 0.11 mmol) and sodium periodate (1165.70 mg, 5.45 mmol) were added in sequence and reacted at room temperature. After the reaction was completed, the reaction was quenched with saturated aqueous sodium sulfite solution (10 mL) and concentrated under reduced pressure. The residue was separated and purified by silica gel column chromatography (eluent: DCM/MeOH (v/v) = 30/1) to obtain 650.0 mg of a light yellow solid product with a yield of 85.29%.

MS (ESI, pos. ion) m/z: 701.6 $[M+H]^+$.

**Step 5: Synthesis of methyl (1*r*,4*r*)-4-(((6-(2-chloro-3'-((2-(difluoromethyl)-7-(((*R*)-3-hydroxypyrrolidin-1-yl) methyl)pyrido[3,2-*d*]pyrimidin-4-yl)amino)-2'-methyl-[1,1'-biphenyl]-3-yl)-2-methoxypyridin-3-yl)methyl)amino) cyclohexane-1-carboxylate**

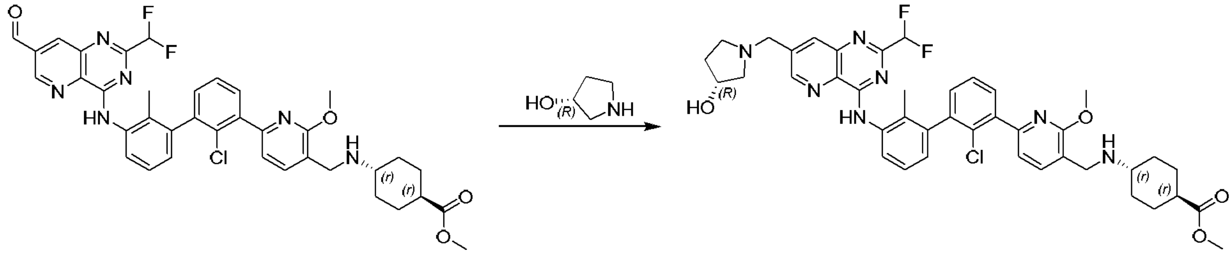

To a 100 mL single-necked bottle, methyl (1*r*,4*r*)-4-(((6-(2-chloro-3'-((2-(difluoromethyl)-7-vinylpyrido[3,2-*d*]

pyrimidin-4-yl)amino)-2'-methyl-[1,1'-biphenyl]-3-yl)-2-methoxypyridin-3-yl)methyl)amino)cyclohexane-1-carboxylate (450 mg, 0.64 mmol), (R)-pyrrolidin-3-ol (278.78 mg, 3.2 mmol), triethylamine (97.14 mg, 0.96 mmol), DCM (15 mL), methanol (15 mL) and acetic acid (57.65 mg, 0.96 mmol) were added in sequence and reacted at 50 °C for 5 h. Sodium cyanoborohydride (80.44 mg, 1.28 mmol) was added and the reaction was continued at room temperature. After the reaction was complete, the pH was adjusted to 7-8 with saturated aqueous potassium carbonate solution, the mixed solution was concentrated under reduced pressure, water (150 mL) was added, and then extracted with DCM (20 mL × 3), the organic phases were combined, washed with water (100 mL × 1), dried over anhydrous sodium sulfate, filtered, and the filtrate was concentrated under reduced pressure. The residue was separated and purified by silica gel column chromatography (eluent: DCM/MeOH (v/v) = 10/1) to obtain 398.0 mg of a light yellow solid product with a yield of 80.3%.

MS (ESI, pos. ion) m/z: 772.8 [M+H]$^+$.

**Step 6: Synthesis of (1*r*,4*r*)-4-(((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)amino)cyclohexane-1-carboxylic acid**

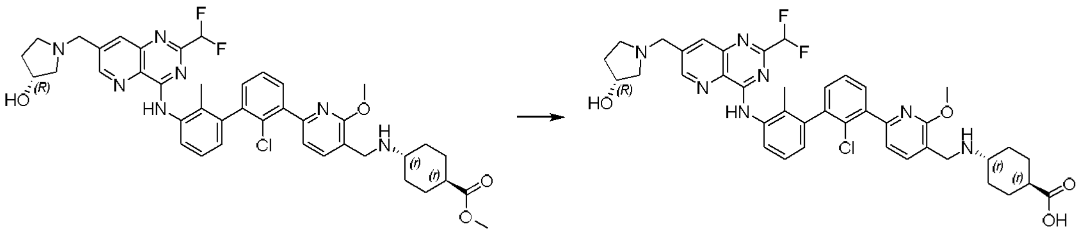

To a 5 mL single-necked bottle, methyl (1*r*,4*r*)-4-(((6-(2-chloro-3'-((2-(difluoromethyl)-7-(((*R*)-3-hydroxypyrrolidin-1-yl)methyl)pyrido[3,2-d]pyrimidin-4-yl)amino)-2'-methyl-[1,1'-biphenyl]-3-yl)-2-methoxypyridin-3-yl)methyl)amino) cyclohexane-1-carboxylate (53 mg, 0.069 mmol), 1,4-dioxane (6 mL), water (2 mL) and lithium hydroxide monohydrate (5.79 mg, 0.14 mmol) were added in sequence and reacted at 50 °C. After the reaction was completed, the mixture was concentrated under reduced pressure, and the residue was separated and purified by preparative silica gel thin layer chromatography (eluent: DCM/MeOH (v/v) = 4/1) to obtain 27.6 mg of a light yellow solid product with a yield of 53.04%.

MS (ESI, pos. ion) m/z: 758.30 [M+H]$^+$.

$^1$H NMR (400 MHz, DMSO-$d_6$) δ (ppm) 10.36 (s, 1H), 8.95 (s, 1H), 8.17 (s, 1H), 7.94 (d, *J* = 7.5 Hz, 1H), 7.73 (d, *J* = 7.8 Hz, 1H), 7.63 (dd, *J* = 7.6, 1.5 Hz, 1H), 7.55 (t, *J* = 7.6 Hz, 1H), 7.43 - 7.30 (m, 3H), 7.15 (d, *J* = 7.4 Hz, 1H), 6.70 (t, *J* = 54.5 Hz, 1H), 4.23 (s, 1H), 4.02 (s, 1H), 3.95 (s, 1H), 3.93 - 3.83 (m, 3H), 3.63 - 3.57 (m, 2H), 2.79 - 2.63 (m, 3H), 2.22 - 2.08 (m, 3H), 2.04 (s, 3H), 2.02 - 1.94 (m, 4H), 1.41 - 1.30 (m, 6H).

**Example 3 *N*-((*R*)-1-((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl-pyrrolidin-3-yl)acetamide**

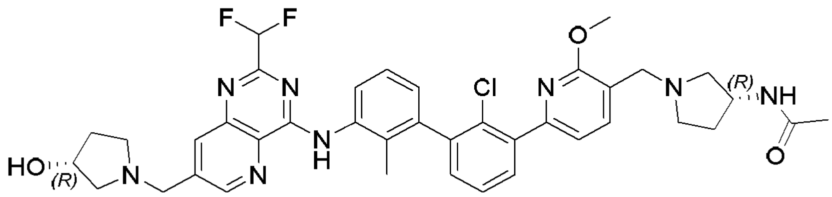

**Step 1: Synthesis of 6-(3'-amino-2-chloro-2'-methyl-[1,1'-biphenyl]-3-yl)-2-methoxynicotinaldehyde**

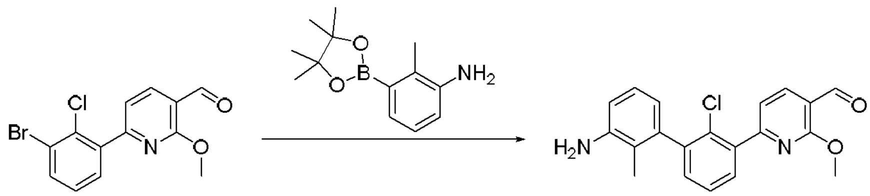

To a mixture of 6-(3-bromo-2-chlorophenyl)-2-methoxypyridine-3-aldehyde (1.52 g, 4.65 mmol), 2-methyl-3-(4,4,5,5-tetramethyl-1,3,2-dioxaborolan-2-yl)aniline (1.30 g, 5.58 mmol) in water (8 mL) and 1,4-dioxane (40 mL) was added potassium carbonate (1.29 g, 9.3 mmol) and Pd(dppf)$Cl_2$ $CH_2Cl_2$ (0.38 g, 0.47 mmol) in sequence, the mixture was protected by nitrogen and heated to 90 °C for 15 h. After the reaction was completed, the product was filtered and the filtrate was concentrated. The remaining liquid was extracted with ethyl acetate (60 mL × 3). The organic phases were combined, dried over anhydrous sodium sulfate, filtered, and the filtrate was concentrated. The residue was separated and purified by silica gel column chromatography (DCM/MeOH (v/v) = 4/1) to obtain 1.64 g of yellow powder with a yield of 99.9%.

MS (ESI, pos. ion) m/z: 353.4 $[M+H]^+$.

**Step 2: Synthesis of (*R*)-*N*-(1-((6-(3'-amino-2-chloro-2'-methyl-[1,1'-biphenyl]-3-yl)-2-methoxypyridin-3-yl) methyl)pyrrolidin-3-yl)acetamide**

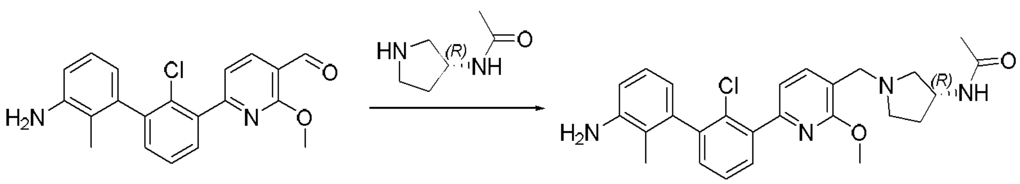

6-(3'-amino-2-chloro-2'-methyl-[1,1'-biphenyl]-3-yl)-2-methoxynicotinaldehyde (1.64 g, 4.65 mmol) and (*R*)-*N*-(pyrrolidin-3-yl)acetamide (2.38 g, 18.6 mmol) were dissolved in a mixed solvent of 2,2,2-trifluoroethanol (50 mL) and DCM (50 mL). After stirring at 50 °C for 30 min, sodium cyanoborohydride (146.10 mg, 2.33 mmol) was added to the system and the reaction was continued at room temperature for 10 min. After the reaction was complete, the system was concentrated under reduced pressure and the residue was purified by column chromatography (eluent: DCM/MeOH (v/v) = 15/1) to obtain 1.12 g of a yellow viscous substance with a yield of 51.8%.

MS (ESI, pos. ion) m/z: 465.5 $[M+H]^+$.

**Step 3: Synthesis of (*R*)-N-(1-((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)pyrrolidin-3-yl)acetamide**

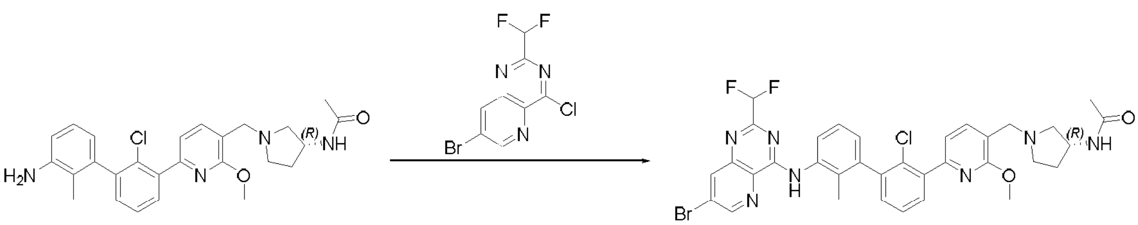

7-Bromo-4-chloro-2-(difluoromethyl)pyrido[3,2-d]pyrimidine (400 mg, 1.36 mmol) and (*R*)-*N*-(1-((6-(3'-amino-2-chloro-2'-methyl-[1,1'-biphenyl]-3-yl)-2-methoxypyridin-3-yl)methyl)pyrrolidin-3-yl)acetamide (758.86 mg, 1.63 mmol) were dissolved in tert-butanol (60 mL) and stirred at 130 °C for 3 h under nitrogen. After the reaction was complete, the system was concentrated under reduced pressure and the residue was purified by column chromatography (eluent: DCM/MeOH (v/v) = 20/1) to obtain 773 mg of a yellow powder with a yield of 78.7%.

MS (ESI, pos. ion) m/z: 722.1 $[M+H]^+$.

**Step 4: Synthesis of (*R*)-*N*-(1-((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)pyrrolidin-3-yl)acetamide**

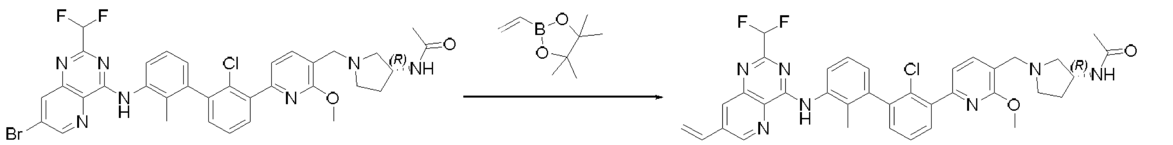

(*R*)-*N*-(1-((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)pyrrolidin-3-yl)acetamide (773 mg, 1.07 mmol) was dissolved in a mixed solvent of 1,4-dioxane (50 mL) and water (5 mL). vinylboronic acid pinacol ester (823.95 mg, 5.35 mmol), Pd(dppf)$Cl_2$ $CH_2Cl_2$ (305.83 mg, 0.37 mmol), and potassium phosphate (454.26 mg, 2.14 mmol) were added in sequence. The mixture was stirred at 100°C under nitrogen protection for 3 h. After the reaction was complete, the system was concentrated under reduced pressure and the residue was purified by column chromatography (eluent: DCM/MeOH (v/v) = 19/1) to obtain 556 mg of a tan solid with a yield of 77.6%.

MS (ESI, pos. ion) m/z: 670.2 $[M+H]^+$.

**Step 5: Synthesis of (*R*)-*N*-(1-((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)pyrrolidin-3-yl)acetamide**

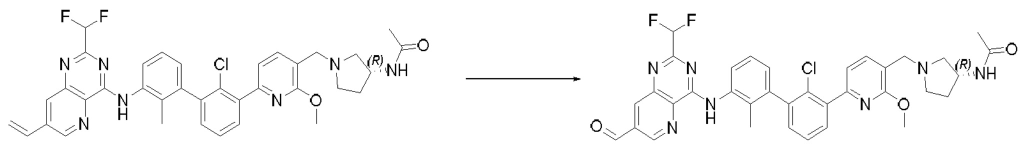

(*R*)-*N*-(1-((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)pyrrolidin-3-yl)acetamide (400 mg, 0.60 mmol) was dissolved in 1,4-dioxane (40 mL) and water (16 mL), potassium osmate dihydrate (22.11 mg, 0.060 mmol) and sodium periodate (641.67 mg, 3 mmol) were added, and the mixture was stirred at room temperature for 2 hours. After the reaction was complete, the system was concentrated under reduced pressure and the residue was purified by column chromatography (eluent: DCM/MeOH (v/v) = 10/1) to obtain 306 mg of a yellow powder with a yield of 76.3%.

MS (ESI, pos. ion) m/z: 672.6 $[M+H]^+$.

**Step 6: Synthesis of *N*-((*R*)-1-((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-**methoxypyridin-3-yl)methyl)pyrrolidin-3-yl)acetamide

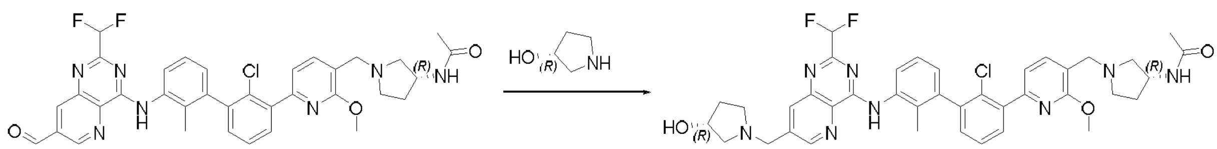

(*R*)-*N*-(1-((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)pyrrolidin-3-yl)acetamide (336 mg, 0.50 mmol) and (R)-pyrrolidin-3-ol (217.80 mg, 2.5 mmol) were dissolved in methanol (30 mL) and DCM (10 mL), and then acetic acid (150.13 mg, 2.5 mmol) was added dropwise. The reaction was stirred at room temperature for 2 h, and then sodium cyanoborohydride (94.26 mg, 1.5 mmol) was added and the reaction was stirred for 4 h. After the reaction was complete, the system was concentrated under reduced pressure and the residue was purified by column chromatography (eluent: DCM/MeOH (v/v) = 12/1) to obtain 10 mg of a yellow powder with a yield of 2.69%.

MS (ESI, pos. ion) m/z: 743.30 $[M+H]^+$.

$^1H$ NMR (400 MHz, DMSO-$d_6$) δ (ppm) 10.36 (s, 1H), 8.98 (s, 1H), 8.20 (s, 1H), 8.08 (s, 1H), 7.74 (d, *J* = 8.0 Hz, 1H), 7.65 (dd, *J* = 7.7, 1.8 Hz, 1H), 7.54 (t, *J* = 7.6 Hz, 1H), 7.37 (d, *J* = 7.6 Hz, 2H), 7.30 (d, *J* = 7.4 Hz, 1H), 7.19 - 7.12 (m, 2H), 6.71 (d, *J* = 2.5 Hz, 1H), 4.85 - 4.77 (m, 1H), 4.59 (t, *J* = 5.3 Hz, 1H), 4.27 - 4.22 (m, 1H), 4.20 - 4.15 (m, 1H), 3.92 (s, 3H), 3.69 - 3.57 (m, 3H), 2.84 - 2.66 (m, 6H), 2.15 - 2.07 (m, 2H), 2.04 (s, 3H), 1.78 (s, 3H), 1.65 - 1.58 (m, 2H), 1.36 (d, *J* = 8.8 Hz, 2H).

**Example 4 (*S*)-1-((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)pyrrolidin-3-ol**

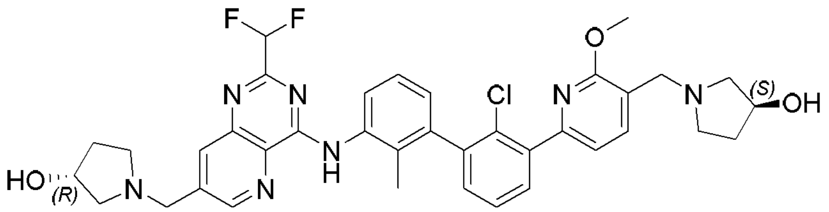

**Step 1: Synthesis of (*S*)-1-((6-(3-bromo-2-chlorophenyl)-2-methoxypyridin-3-yl)methyl)pyrrolidin-3-ol**

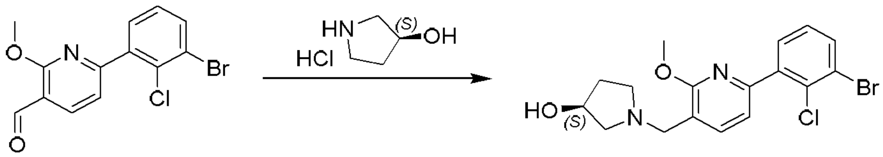

To a 250 mL single-necked bottle, 6-(3-bromo-2-chlorophenyl)-2-methoxynicotinaldehyde (4 g, 12.25 mmol), (S)-pyrrolidin-3-ol hydrochloride (3.03 g, 24.5 mmol), DCM (50 mL), DMF (50 mL), triethylamine (1.86 g, 18.38 mmol) and acetic acid (1.10 g, 18.38 mmol) were added in sequence, and the reaction was stirred at 50 °C overnight. Then, sodium cyanoborohydride (1.54 g, 24.5 mmol) was added and the reaction was continued at room temperature. After the reaction was complete, the pH was adjusted to 7-8 with saturated aqueous potassium carbonate solution, diluted with water (500 mL), and then extracted with DCM (80 mL × 3), the organic phases were combined, washed with water (500 mL × 2), dried over anhydrous sodium sulfate, filtered, and the filtrate was concentrated under reduced pressure. The residue was separated and purified by silica gel column chromatography (eluent: DCM/MeOH (v/v) = 40/1) to obtain 2.66 g of a white solid product with a yield of 54.61%.

MS (ESI, pos. ion) m/z: 397.3 [M+H]$^+$.

**Step 2: Synthesis of (*S*)-1-((6-(3'-amino-2-chloro-2'-methyl-[1,1'-biphenyl]-3-yl)-2-methoxypyridin-3-yl)methyl) pyrrolidin-3-ol**

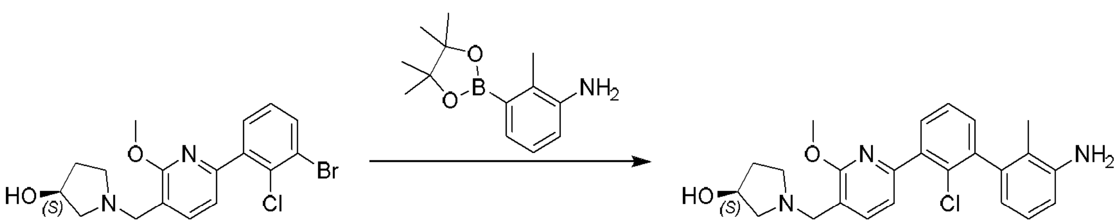

To a 20 mL microwave tube, (*S*)-1-((6-(3-bromo-2-chlorophenyl)-2-methoxypyridin-3-yl)methyl)pyrrolidin-3-ol (1.3 g, 3.27 mmol), 2-methyl-3-(4,4,5,5-tetramethyl-1,3,2-dioxaborolan-2-yl)aniline (0.99 g, 4.25 mmol), potassium carbonate (1.13 g, 8.18 mmol), Pd(dppf)$Cl_2$ $CH_2Cl_2$ (0.27 g, 0.33 mmol), 1,4-dioxane (10 mL) and water (2 mL) were added in sequence. The tube was sealed and reacted at 130 °C for 2 h. After the reaction was complete, the reaction solution was concentrated under reduced pressure to remove dioxane, water (150 mL) was added, and then extracted with

DCM (30 mL × 3). The organic phases were combined, washed with saturated brine (50 mL × 1), dried over anhydrous sodium sulfate, filtered, and the filtrate was concentrated under reduced pressure. The residue was separated and purified by silica gel column chromatography (eluent: DCM/MeOH (v/v) = 15/1) to obtain 813.0 mg of a brown solid product with a yield of 58.67%.

MS (ESI, pos. ion) m/z: 424.2 $[M+H]^+$.

**Step 3: Synthesis of (S)-1-((6-(3'-((7-bromo-2-(difluoromethyl)pyrido[3,2-d]pyrimidin-4-yl)amino)-2-chloro-2'-methyl-[1,1'-biphenyl]-3-yl)-2-methoxypyridin-3-yl)methyl)pyrrolidin-3-ol**

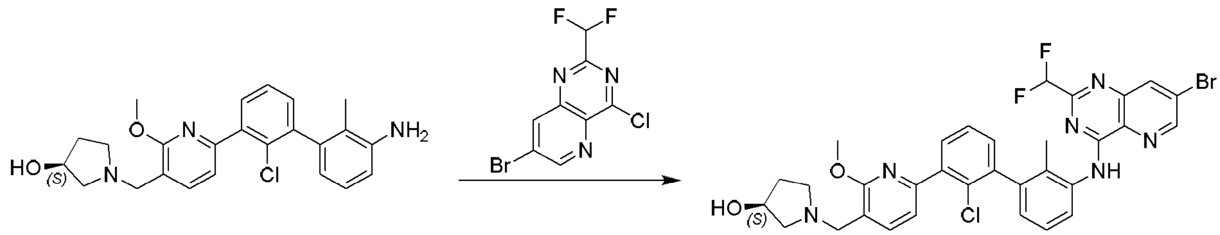

To a 100 mL single-necked bottle, (*S*)-1-((6-(3'-amino-2-chloro-2'-methyl-[1,1'-biphenyl]-3-yl)-2-methoxypyridin-3-yl)methyl)pyrrolidin-3-ol (500 mg, 1.18 mmol), 7-bromo-4-chloro-2-(difluoromethyl)pyrido[3,2-*d*]pyrimidine (416.98 mg, 1.42 mmol), *tert*-butanol (30 mL) and acetic acid (70.86 mg, 1.18 mmol) were added in sequence and the reaction was stirred at 120 °C. After the reaction was complete, the pH was adjusted to 7-8 with saturated aqueous potassium carbonate solution, the mixed solution was concentrated under reduced pressure, water (100 mL) was added, and then extracted with DCM (20 mL × 3), the organic phases were combined, washed with water (100 mL × 1), dried over anhydrous sodium sulfate, filtered, and the filtrate was concentrated under reduced pressure. The residue was separated and purified by silica gel column chromatography (eluent: DCM/MeOH (v/v) = 20/1) to obtain 605.0 mg of a light yellow solid product with a yield of 75.2%.

MS (ESI, pos. ion) m/z: 681.5 $[M+H]^+$.

**Step 4: Synthesis of (*S*)-1-((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)pyrrolidin-3-ol**

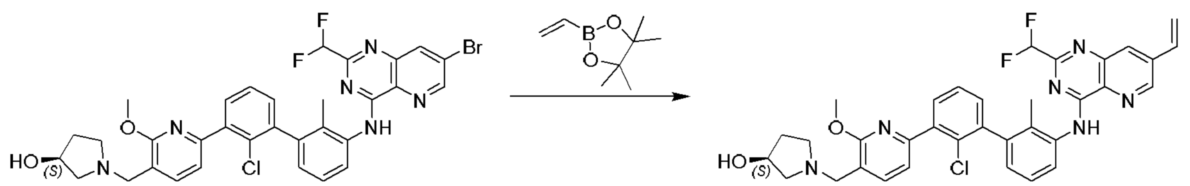

To a 20 mL microwave tube, (S)-1-((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)pyrrolidin-3-ol (400 mg, 0.59 mmol), 2-vinyl-4,4,5,5-tetramethyl-1,3,2-dioxaborolane (227.16 mg, 1.47 mmol), potassium phosphate (313.10 mg, 1.47 mmol), $Pd(dppf)Cl_2$ $CH_2Cl_2$ (48.18 mg, 0.059 mmol), 1,4-dioxane (7.5 mL) and water (1.5 mL) were added and reacted at 130 °C for 2 h under nitrogen atmosphere. After the reaction was complete, the reaction solution was concentrated under reduced pressure to remove dioxane, water (100 mL) was added, and then extracted with DCM (20 mL × 3). The organic phases were combined, washed with saturated brine (50 mL × 1), dried over anhydrous sodium sulfate, filtered, and the filtrate was concentrated under reduced pressure. The residue was separated and purified by silica gel column chromatography (eluent: DCM/MeOH (v/v) = 10/1) to obtain 340.0 mg of a light yellow solid product with a yield of 92.1%.

MS (ESI, pos. ion) m/z: 629.6 $[M+H]^+$.

**Step 5: Synthesis of (*S*)-4-((2'-chloro-3'-(5-((3-hydroxypyrrolidin-1-yl)methyl)-6-methoxypyridin-2-yl)-2-methyl-[1,1'-biphenyl]-3-yl)amino)-2-(difluoromethyl)pyrido[3,2-*d*]pyrimidine-7-carbaldehyde**

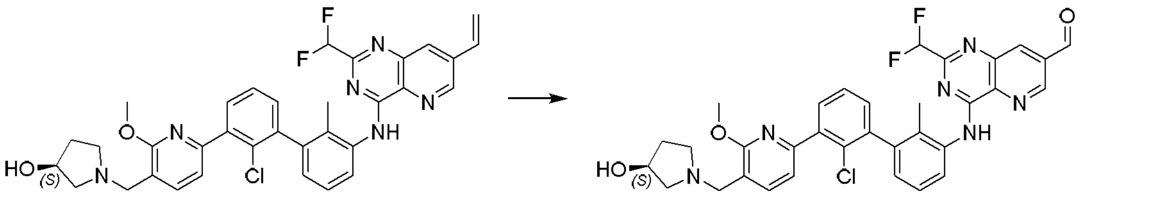

To a 50 mL single-necked bottle, (*S*)-1-((6-(2-chloro-3'-((2-(difluoromethyl)-7-vinylpyrido[3,2-*d*]pyrimidin-4-yl) amino)-2'-methyl-[1,1'-biphenyl]-3-yl)-2-methoxypyridin-3-yl)methyl)pyrrolidin-3-ol (244 mg, 0.39 mmol), 1,4-dioxane (15 mL), water (6 mL), potassium osmate dihydrate (14.37 mg, 0.039 mmol) and sodium periodate (417.09 mg, 1.95 mmol) were added in sequence and the reaction was stirred at room temperature for 1 h. After the reaction was complete, the reaction solution was concentrated under reduced pressure to remove dioxane, water (100 mL) was added, and then extracted with DCM (20 mL × 3). The organic phases were combined, washed with saturated brine (50 mL × 1), dried over anhydrous sodium sulfate, filtered, and the filtrate was concentrated under reduced pressure. The residue was separated and purified by silica gel column chromatography (eluent: DCM/MeOH (v/v) = 15/1) to obtain 215.0 mg of a light yellow solid product with a yield of 87.8%.

MS (ESI, pos. ion) m/z: 631.6 $[M+H]^+$.

**Step 6: Synthesis of (*S*)-1-((6-(2-chloro-3'-((2-(difluoromethyl)-7-(((*R*)-3-hydroxypyrrolidin-1-yl)methyl)pyrido [3,2-*d*]pyrimidin-4-yl)amino)-2'-methyl-[1,1'-biphenyl]-3-yl)-2-methoxypyridin-3-yl)methyl)pyrrolidin-3-ol**

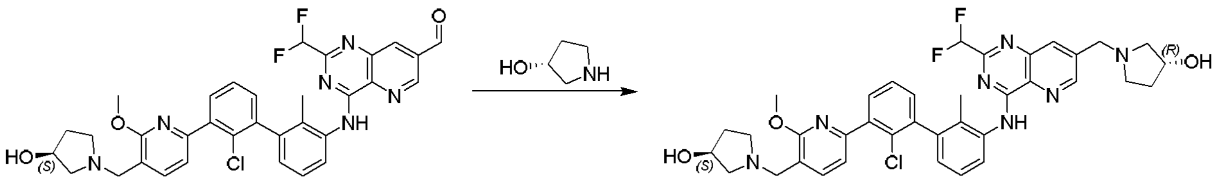

To a 25 mL single-necked bottle, (*S*)-4-((2'-chloro-3'-(5-((3-hydroxypyrrolidin-1-yl)methyl)-6-methoxypyridin-2-yl)-2-methyl-[1,1'-biphenyl]-3-yl)amino)-2-(difluoromethyl)pyrido[3,2-*d*]pyrimidine-7-carbaldehyde (142 mg, 0.23 mmol), (R)-pyrrolidin-3-ol (50.09 mg, 0.58 mmol), DCM (5 mL), DMF (5 mL), triethylamine (34.91 mg, 0.35 mmol) and acetic acid (20.72 mg, 0.35 mmol) were added in sequence, the mixture was reacted at 50 °C overnight, and then sodium cyanoborohydride (28.91 mg, 0.46 mmol) was added and the reaction was continued to stir at room temperature. After the reaction was complete, the pH was adjusted to 7-8 with saturated aqueous potassium carbonate solution, diluted with water (100 mL), and then extracted with DCM (15 mL × 3), the organic phases were combined, washed with water (100 mL × 2), dried over anhydrous sodium sulfate, filtered, and the filtrate was concentrated under reduced pressure. The residue is separated and purified by preparative silica gel thin layer chromatography (eluent: DCM/MeOH (v/v) = 7.5/1) to obtain 10.0 mg of a yellow solid product with a yield of 6.33%.

MS (ESI, pos. ion) m/z: 702.27 $[M+H]^+$.

$^1H$ NMR (400 MHz, DMSO-$d_6$) δ (ppm) 10.36 (s, 1H), 8.96 (s, 1H), 8.20 (s, 1H), 7.87 (d, *J* = 7.8 Hz, 1H), 7.73 (d, *J* = 7.7 Hz, 1H), 7.65 (d, *J* = 7.0 Hz, 1H), 7.54 (t, *J* = 7.7 Hz, 1H), 7.42 - 7.35 (m, 2H), 7.33 (d, *J* = 7.6 Hz, 1H), 7.16 (d, *J* = 7.9 Hz, 1H), 6.70 (t, *J* = 54.6 Hz, 1H), 5.10 - 4.98 (m, 1H), 4.86 (s, 1H), 3.93 (s, 3H), 3.63 - 3.52 (m, 6H), 3.04 - 2.67 (m, 8H), 2.03 (s, 3H), 1.76 - 1.40 (m, 4H).

**Example 5 (*R*)-1-((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)pyrrolidine-3-carboxylic acid**

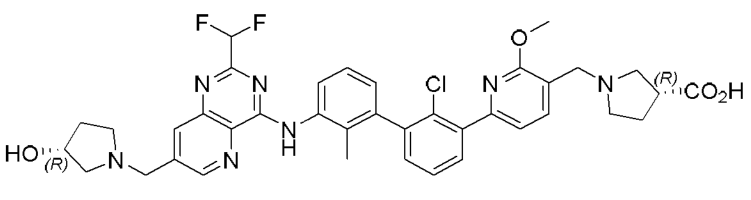

**Step 1: Synthesis of methyl (*R*)-1-((6-(3'-amino-2-chloro-2'-methyl-[1,1'-biphenyl]-3-yl)-2-methoxypyridin-3-yl) methyl)pyrrolidine-3-carboxylate**

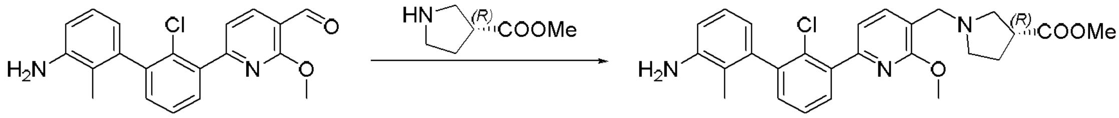

6-(3'-Amino-2-chloro-2'-methyl-[1,1-biphenyl]-3-yl)-2-methoxypyridine-3-aldehyde (0.2 g, 0.57 mmol) and methyl (*R*)-pyrrolidine-3-carboxylate hydrochloride (0.28 g, 1.71 mmol) were dissolved in 2,2,2-trifluoroethanol (15 mL) and DCM (15 mL). The mixture was stirred at 50 °C for 30 min. After the reaction was complete, the system was concentrated under reduced pressure and the residue was purified by column chromatography (eluent: DCM/MeOH (v/v) = 15/1) to obtain 0.26 g of a yellow viscous substance with a yield of 98.4%.
MS (ESI, pos. ion) m/z: 466.1 $[M+H]^+$.

**Step 2: Synthesis of methyl (*R*)-1-((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)pyrrolidine-3-carboxylate**

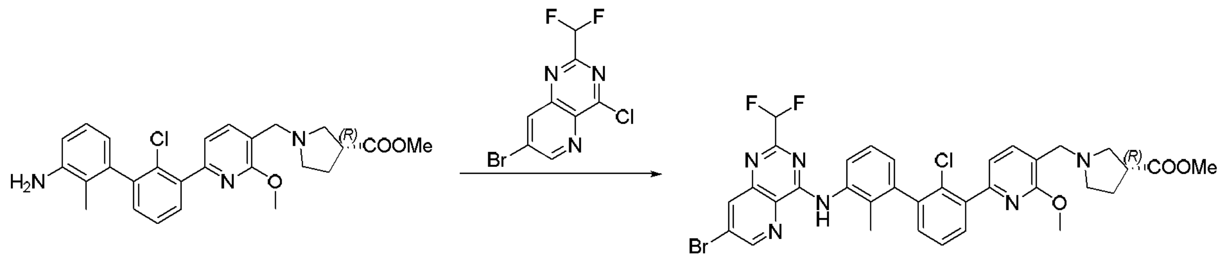

7-Bromo-4-chloro-2-(difluoromethyl)pyrido[3,2-d]pyrimidine (180 mg, 0.61 mmol) and methyl (*R*)-1-((6-(3'-amino-2-chloro-2'-methyl-[1,1'-biphenyl]-3-yl)-2-methoxypyridin-3-yl)methyl)pyrrolidine-3-carboxylate (0.26 g, 0.55 mmol) were dissolved in *tert*-butanol (15 mL) and stirred at 130 °C for 3 h. After the reaction was complete, the system was concentrated under reduced pressure and the residue was purified by column chromatography (eluent: DCM/MeOH (v/v) = 40/1) to obtain 186 mg of a yellow powder with a yield of 42.03%.
MS (ESI, pos. ion) m/z: 723.6 $[M+H]^+$.

**Step 3: Synthesis of methyl (*R*)-1-((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)pyrrolidine-3-carboxylate**

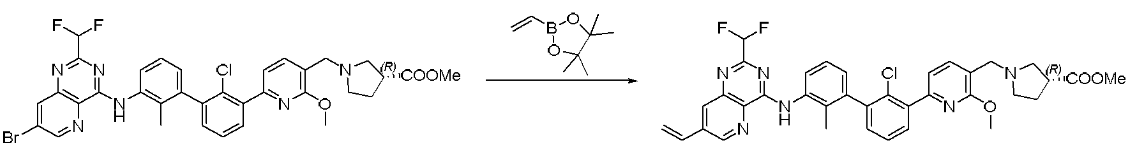

Methyl (*R*)-1-((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)pyrrolidine-3-carboxylate (186 mg, 0.26 mmol) was dissolved in a mixed solvent of 1,4-dioxane (15 mL) and water (3 mL). 2-vinyl-4,4,5,5-tetramethyl-1,3,2-dioxaborolane (200.21 mg, 1.3 mmol), $Pd(dppf)Cl_2$ $CH_2Cl_2$ (74.31 mg, 0.091 mmol) and potassium phosphate (110.38 mg, 0.52 mmol) were added in sequence. The mixture was stirred at 100°C for 3 h. After the reaction was complete, the system was concentrated under reduced pressure and the residue was purified by column chromatography (eluent: DCM/MeOH (v/v) = 30/1) to obtain 172 mg of a tan solid with a yield of 99.76%.
MS (ESI, pos. ion) m/z: 671.2 $[M+H]^+$.

**Step 4: Synthesis of methyl (*R*)-1-((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)pyrrolidine-3-carboxylate**

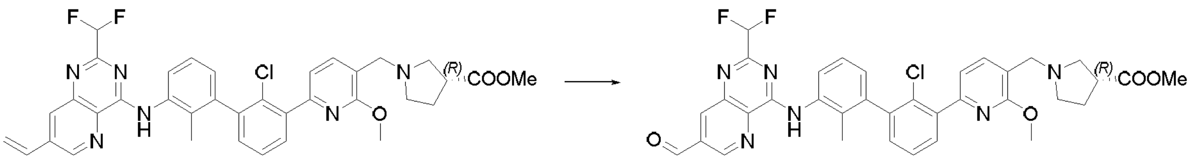

Methyl (*R*)-1-((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)pyrrolidine-3-carboxylate (152 mg, 0.23 mmol) was dissolved in 1,4-dioxane (30 mL) and water (12 mL), sodium periodate (245.97 mg, 1.15 mmol) and potassium osmate dihydrate (8.47 mg, 0.023 mmol) were added, and the reaction was stirred at room temperature for 4 h. After the reaction was complete, the system was concentrated under reduced pressure and the residue was purified by column chromatography (eluent: DCM/MeOH (v/v) = 10/1) to obtain 48 mg of a yellow powder with a yield of 31.5%.

MS (ESI, pos. ion) m/z: 675.0 $[M+H]^+$.

**Step 5: Synthesis of methyl (*R*)-1-((6-(2-chloro-3'-((2-(difluoromethyl)-7-(((*R*)-3-hydroxypyrrolidin-1-yl)methyl) pyrido[3,2-*d*]pyrimidin-4-yl)amino)-2'-methyl-[1,1'-biphenyl]-3-yl)-2-methoxypyridin-3-yl)methyl)pyrrolidine-3-carboxylate**

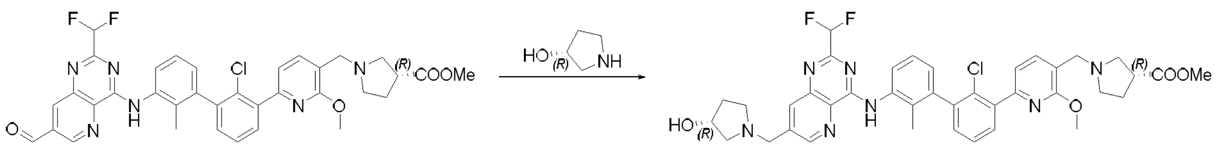

Methyl (*R*)-1-((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)pyrrolidine-3-carboxylate (56 mg, 0.083 mmol) and (R)-pyrrolidin-3-ol (36.15 mg, 0.42 mmol) were dissolved in methanol (10 mL) and DCM (3 mL). The mixture was stirred at room temperature for 3 h. Sodium cyanoborohydride (15.65 mg, 0.25 mmol) was added and the reaction was continued at room temperature for 1.5 h. After the reaction was complete, the system was concentrated under reduced pressure and the residue was purified by column chromatography (eluent: DCM/MeOH (v/v) = 12/1) to obtain 22 mg of a yellow powder with a yield of 35.5%.

MS (ESI, pos. ion) m/z: 644.6 $[M+H]^+$.

**Step 6: Synthesis of (*R*)-1-((6-(2-chloro-3'-((2-(difluoromethyl)-7-(((*R*)-3-hydroxypyrrolidin-1-yl)methyl)pyrido [3,2-*d*]pyrimidin-4-yl)amino)-2'-methyl-[1,1'-biphenyl]-3-yl)-2-methoxypyridin-3-yl)methyl)pyrrolidine-3-carboxylic acid**

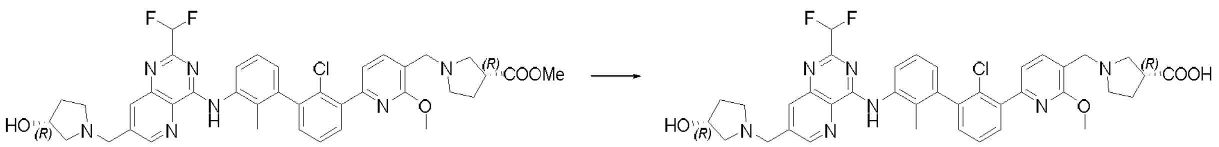

Methyl (*R*)-1-((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)pyrrolidine-3-carboxylate (22 mg, 0.030 mmol) was dissolved in a mixed solution of 1,4-dioxane (3 mL) and water (3 mL), lithium hydroxide monohydrate (15.11 mg, 0.36 mmol) was added, and the reaction was stirred at room temperature for 3.5 h. After the reaction was complete, the system was concentrated under reduced pressure and the residue was purified by column chromatography (eluent: DCM/MeOH (v/v) = 6/1) to obtain 10 mg of a yellow powder with a yield of 46.3%.

MS (ESI, pos. ion) m/z: 730.26 $[M+H]^+$.

$^1$H NMR (400 MHz, DMSO-$d_6$) δ (ppm) 10.34 (s, 1H), 8.95 (s, 1H), 8.17 (s, 1H), 7.76 (dd, *J* = 13.5, 7.7 Hz, 2H), 7.64 (d, *J* = 7.7 Hz, 1H), 7.53 (t, *J* = 7.6 Hz, 1H), 7.36 (d, *J* = 7.8 Hz, 2H), 7.28 (d, *J* = 7.5 Hz, 1H), 7.15 (d, *J* = 7.6 Hz, 1H), 6.74 - 6.65 (m, 1H), 4.24 - 4.20 (m, 1H), 3.91 (s, 3H), 3.60 (dd, *J* = 9.3, 4.9 Hz, 4H), 2.99 - 2.92 (m, 2H), 2.78 - 2.67 (m, 5H), 2.62 - 2.55 (m, 3H), 2.04 (s, 3H), 1.99 - 1.96 (m, 2H), 1.62 - 1.55 (m, 1H), 1.49 - 1.41 (m, 1H).

**Example 6 (*R*)-2-(1-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)piperidin-4-yl)acetic acid**

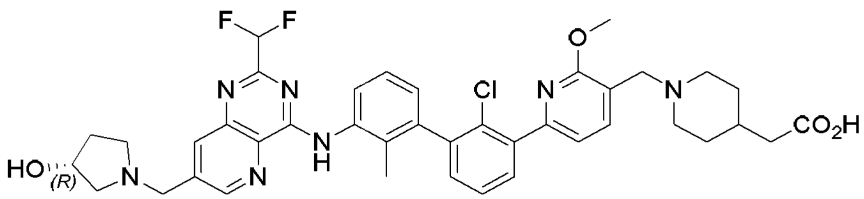

**Step 1: Synthesis of methyl 2-(1-((6-(3-bromo-2-chlorophenyl)-2-methoxypyridin-3-yl)methyl)piperidin-4-yl) acetate**

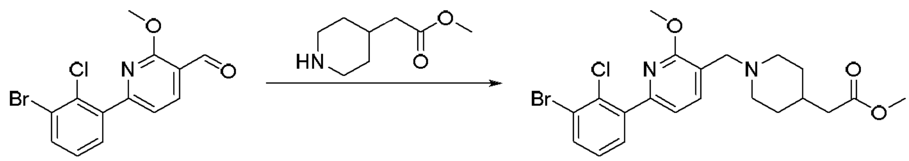

6-(3-Bromo-2-chlorophenyl)-2-methoxynicotinaldehyde (1.5 g, 4.59 mmol) was dissolved in a mixed solvent of DCM (15 mL) and trifluoroethanol (15 mL), and methyl 2-(piperidin-4-yl)acetate (2.67 g, 13.77 mmol) and triethylamine (2.32 g, 22.95 mmol) were added, and the mixture was heated and stirred at 60 °C overnight. Sodium cyanoborohydride (0.58 g, 9.18 mmol) was added and stirred at room temperature for 40 min. After the reaction of the raw materials was complete, the mixture was concentrated under reduced pressure, and the residue was separated and purified by silica gel column chromatography (eluent: PE/EtOAc (v/v) = 7/1) to obtain 1.38 g of yellow solid product with a yield of 64.2%.

MS (ESI, pos. ion) m/z: 467.1 $[M+H]^+$.

**Step 2: Synthesis of methyl 2-(1-((6-(3'-amino-2-chloro-2'-methyl-[1,1'-biphenyl]-3-yl)-2-methoxypyridin-3-yl) methyl)piperidin-4-yl)acetate**

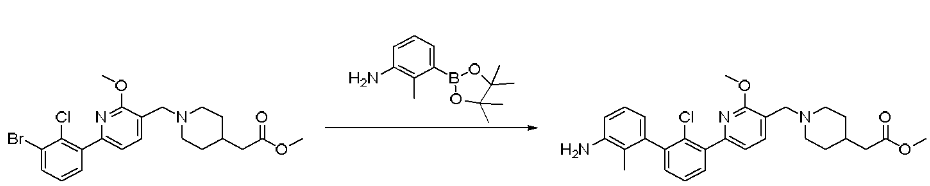

Methyl 2-(1-((6-(3-bromo-2-chlorophenyl)-2-methoxypyridin-3-yl)methyl)piperidin-4-yl)acetate (1.38 g, 2.95 mmol), 2-methyl-3-(4,4,5,5-tetramethyl-1,3,2-dioxaborolan-2-yl)aniline (2.06 g, 8.85 mmol), Pd(dppf)$Cl_2$ $CH_2Cl_2$ (0.60 g, 0.74 mmol), and potassium carbonate (1.43 g, 10.33 mmol) were dissolved in a mixed solvent of 1,4-dioxane (20 mL) and water (4 mL) and heated at 90 °C under nitrogen protection for overnight reaction. After the reaction of the raw materials was complete, the reaction solution was concentrated under reduced pressure, and the residue was separated and purified by silica gel column chromatography (eluent: PE/EtOAc (v/v) = 3/2) to obtain 1.38 g of yellow liquid product with a yield of 94.7%.

MS (ESI, pos. ion) m/z: 594.5 $[M+H]^+$.

**Step 3: Synthesis of methyl 2-(1-((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)piperidin-4-yl)acetate**

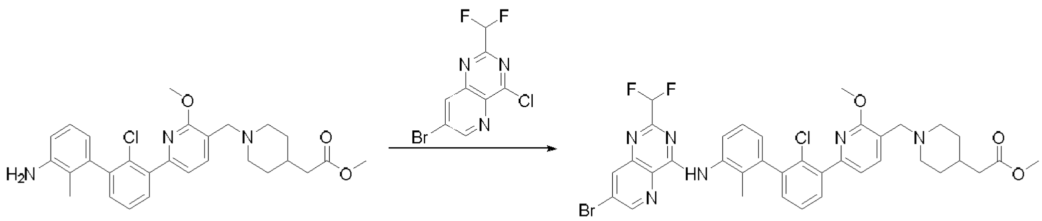

Methyl 2-(1-((6-(3'-amino-2-chloro-2'-methyl-[1,1'-biphenyl]-3-yl)-2-methoxypyridin-3-yl)methyl)piperidin-4-yl) acetate (1.38 g, 2.79 mmol) and 7-bromo-4-chloro-2-(difluoromethyl)pyrido[3,2-*d*]pyrimidine (0.99 g, 3.35 mmol) were dissolved in *tert*-butanol (20 mL), acetic acid (0.17 g, 2.79 mmol) was added, and the mixture was protected by $N_2$ and stirred at 100 °C for 5 h. After the reaction was complete, the reaction solution was concentrated under reduced pressure, and the residue was separated and purified by silica gel column chromatography (eluent: PE/EtOAc (v/v) = 3/2) to obtain 1.34 g of yellow solid product with a yield of 63.8%.

MS (ESI, pos. ion) m/z: 751.2 $[M+H]^+$.

**Step 4: Synthesis of methyl 2-(1-((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)piperidin-4-yl)acetate**

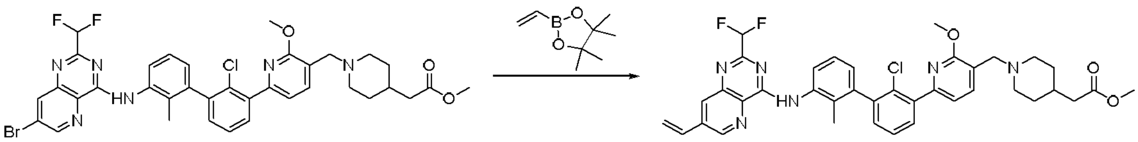

Methyl 2-(1-((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)piperidin-4-yl)acetate (1.34 g, 1.78 mmol), 4,4,5,5-tetramethyl-2-vinyl-1,3,2-dioxaborolane (1.37 g, 8.90 mmol), Pd(dppf)$Cl_2$ $CH_2Cl_2$ (0.51 g, 0.62 mmol), potassium phosphate (0.76 g, 3.56 mmol) were dissolved in a mixed solvent of 1,4-dioxane (40 mL) and water (8 mL) and heated at 100 °C for 5 h. After the reaction was complete, the reaction solution was concentrated under reduced pressure, and the residue was separated and purified by silica gel column chromatography (eluent: PE/EtOAc (v/v) = 3/2) to obtain 1.24 g of yellow solid product with a yield of 99.5%.

MS (ESI, pos. ion) m/z: 699.3 $[M+H]^+$.

**Step 5: Synthesis of methyl 2-(1-((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)piperidin-4-yl)acetate**

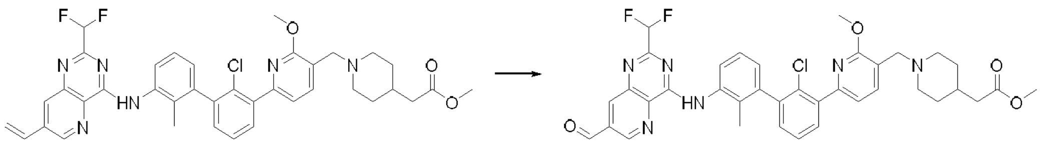

Methyl 2-(1-((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)piperidin-4-yl)acetate (1.24 g, 1.77 mmol) was dissolved in a mixed solvent of 1,4-dioxane (62.5 mL) and water (25 mL), then potassium osmate dihydrate (0.065 g, 0.18 mmol) was added, followed by sodium periodate (1.89 g, 8.85 mmol), and the mixture was reacted at room temperature for 1.5 h. After the reaction of the raw materials was complete, the reaction solution was concentrated under reduced pressure, and the residue was separated and purified by silica gel column chromatography (eluent: DCM/MeOH (v/v) = 33/1) to obtain 0.67 g of yellow liquid product with a yield of 53.9%.

MS (ESI, pos. ion) m/z: 701.3 [M+H]$^+$.

**Step 6: Synthesis of methyl (*R*)-2-(1-((6-(2-chloro-3'-((2-(difluoromethyl)-7-((3-hydroxypyrrolidin-1-yl)methyl) pyrido[3,2-*d*]pyrimidin-4-yl)amino)-2'-methyl-[1,1'-biphenyl]-3-yl)-2-methoxypyridin-3-yl)methyl)piperidin-4-yl) acetate**

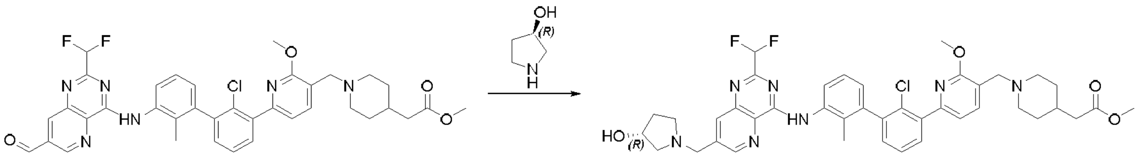

Methyl 2-(1-((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)piperidin-4-yl)acetate (0.147 g, 0.21 mmol) was dissolved in a mixed solvent of DCM (10 mL) and MeOH (10 mL), (*R*)-pyrrolidin-3-ol (0.091 g, 1.05 mmol) and acetic acid (0.1 mL) were added, and the mixture was stirred at room temperature overnight. Sodium cyanoborohydride (0.026 g, 0.42 mmol) was added and stirred at room temperature for 1 h. After the reaction was completed, the reaction solution was concentrated under reduced pressure, and the residue was separated and purified by silica gel column chromatography (eluent: DCM/MeOH (v/v) = 20/1) to obtain 0.16 g of a yellow oily product with a yield of 98.8%.
MS (ESI, pos. ion) m/z: 732.3 [M+H]$^+$.

**Step 7: Synthesis of (*R*)-2-(1-((6-(2-chloro-3'-((2-(difluoromethyl)-7-((3-hydroxypyrrolidin-1-yl)methyl)pyrido [3,2-*d*]pyrimidin-4-yl)amino)-2'-methyl-[1,1'-biphenyl]-3-yl)-2-methoxypyridin-3-yl)methyl)piperidin-4-yl)acetic acid**

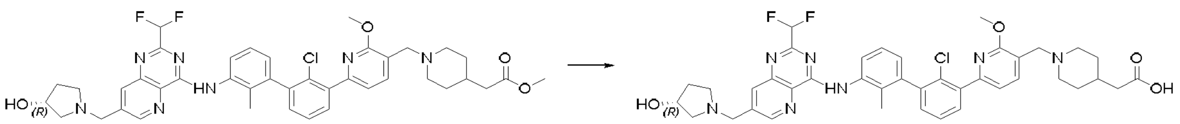

Methyl (*R*)-2-(1-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)piperidin-4-yl)acetate (0.16 g, 0.21 mmol) was dissolved in 1,4-dioxane (12.5 mL) and water (2.5 mL), lithium hydroxide monohydrate (0.088 g, 2.1 mmol) was added, and the reaction was stirred at room temperature overnight. After the reaction of the raw materials was complete, the pH of the system was adjusted to about 5 with acetic acid, the reaction solution was concentrated under reduced pressure, and the residue was separated and purified by preparative thin layer chromatography (eluent: DCM/MeOH (v/v) = 4/1) to obtain 37 mg of a yellow solid product with a yield of 21.7%.
MS (ESI, pos. ion) m/z: 758.3024 [M+H]$^-$.
$^1$H NMR (400 MHz, DMSO-$d_6$) δ (ppm) 10.36 (s, 1H), 8.98 (s, 1H), 8.21 (d, *J* = 6.2 Hz, 1H), 7.82 (d, *J* = 6.8 Hz, 1H), 7.74 (d, *J* = 8.0 Hz, 1H), 7.65 (dd, *J* = 7.7, 1.7 Hz, 1H), 7.54 (t, *J* = 7.6 Hz, 1H), 7.40 - 7.36 (m, 2H), 7.30 (d, *J* = 7.4 Hz, 1H), 7.16 (d, *J* = 7.6 Hz, 1H), 6.71 (s, 1H), 4.27 - 4.24 (m, 1H), 3.91 (s, 3H), 3.57 (s, 4H), 2.89 (d, *J* = 7.7 Hz, 1H), 2.75 - 2.73 (m, 1H), 2.63 - 2.61 (m, 1H), 2.53 (d, *J* = 2.2 Hz, 5H), 2.41 - 2.38 (m, 1H), 2.16 (d, *J* = 6.6 Hz, 2H), 2.04 (s, 3H), 1.69 - 1.66 (m, 2H), 1.35 - 1.33 (m, 5H).

**Example 7 (*R*)-2-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)-2-azabicyclo[2.2.2]octane-4-carboxylic acid**

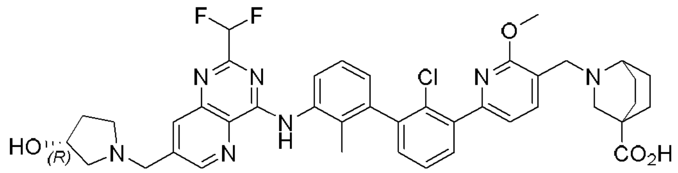

**Step 1: Synthesis of methyl 2-azabicyclo[2.2.2]octane-4-carboxylate 2,2,2-trifluoroacetate**

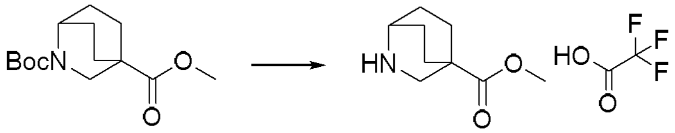

2-(*Tert*-butyl) 4-methyl-2-azabicyclo[2.2.2]octane-2,4-dicarboxylate (0.9 g, 3.34 mmol) was dissolved in DCM (20 mL), TFA (11.42 g, 100.20 mmol) was added, and the mixture was reacted at room temperature for 5 h. After the reaction of the raw materials was complete, the reaction solution was concentrated under reduced pressure to obtain 0.94 g of a yellow liquid product with a yield of 99.3%.

MS (ESI, pos. ion) m/z: 170.4 $[M+H]^+$.

**Step 2: Synthesis of methyl 2-((6-(3-bromo-2-chlorophenyl)-2-methoxypyridin-3-yl)methyl)-2-azabicyclo [2.2.2] octane-4-carboxylate**

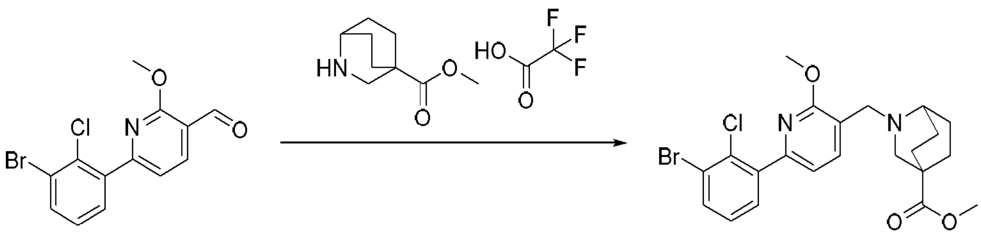

The compound of 6-(3-bromo-2-chlorophenyl)-2-methoxynicotinaldehyde (0.5 g, 1.53 mmol) was dissolved in a mixed solvent of DCM (10 mL) and trifluoroethanol (10 mL), and methyl 2-azabicyclo[2.2.2]octane-4-carboxylate 2,2,2-trifluoroacetate (0.93 g, 3.29 mmol) and triethylamine (0.77 g, 7.65 mmol) were added, and the mixture was heated and stirred at 60 °C overnight. After cooling to room temperature, sodium cyanoborohydride (0.19 g, 3.06 mmol) was added, and the mixture was stirred at room temperature for 1.5 h. After the reaction was complete, the mixture was concentrated under reduced pressure, and the residue was separated and purified by silica gel column chromatography (eluent: PE/EtOAc (v/v) = 2/1) to obtain 0.73 g of yellow solid product with a yield of 99.4%.

MS (ESI, pos. ion) m/z: 479.1 $[M+H]^+$.

**Step 3: Synthesis of methyl 2-((6-(3'-amino-2-chloro-2'-methyl-[1,1'-biphenyl]-3-yl)-2-methoxypyridin-3-yl) methyl)-2-azabicyclo [2.2.2] octane-4-carboxylate**

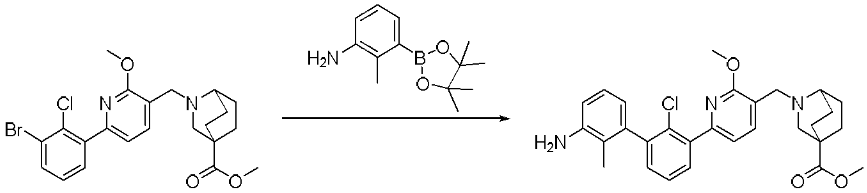

Methyl 2-((6-(3-bromo-2-chlorophenyl)-2-methoxypyridin-3-yl)methyl)-2-azabicyclo[2.2.2]octane-4-carboxylate (0.73 g, 1.52 mmol), 2-methyl-3-(4,4,5,5-tetramethyl-1,3,2-dioxaborolan-2-yl)aniline (1.06 g, 4.56 mmol), Pd(dppf)$Cl_2$ $CH_2Cl_2$ (0.31 g, 0.38 mmol), and potassium carbonate (0.74 g, 5.32 mmol) were dissolved in a mixed solvent of 1,4-dioxane (30 mL) and water (6 mL) and heated at 90 °C under nitrogen protection for overnight reaction. After the reaction of the raw materials was complete, the reaction solution was concentrated under reduced pressure, and the residue was separated and purified by silica gel column chromatography (eluent: PE/EtOAc (v/v) = 2/1) to obtain 0.76 g of yellow liquid product with a yield of 98.7%.

MS (ESI, pos. ion) m/z: 506.5 $[M+H]^+$.

**Step 4: Synthesis of methyl 2-((6-(3'-((7-bromo-2-(difluoromethyl)pyrido[3,2-d]pyrimidin-4-yl)amino)-2'-methyl-[1,1'-biphenyl]-3-yl)-2-methoxypyridin-3-yl)methyl)-2-azabicyclo [2.2.2]octane-4-carboxylate**

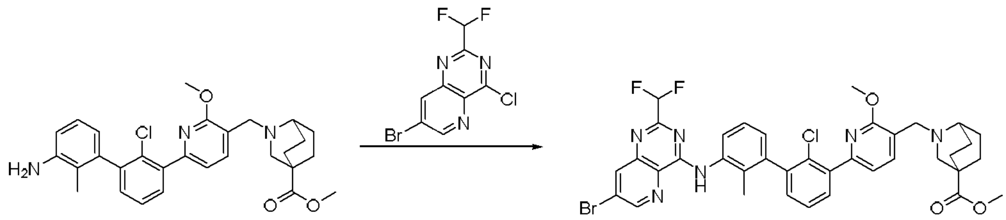

Methyl 2-((6-(3'-amino-2-chloro-2'-methyl-[1,1'-biphenyl]-3-yl)-2-methoxypyridin-3-yl)methyl)-2-azabicyclo [2.2.2]octane-4-carboxylate (0.76 g, 1.50 mmol) and 7-bromo-4-chloro-2-(difluoromethyl)pyrido[3,2-*d*]pyrimidine (0.53 g, 1.80 mmol) were dissolved in *tert*-butanol (20 mL), acetic acid (0.09 g, 1.50 mmol) was added, and the mixture was reacted at 100 °C for 5 h. After the reaction was complete, the mixture was concentrated under reduced pressure, and the residue was separated and purified by silica gel column chromatography (eluent: PE/EtOAc (v/v) = 3/1) to obtain 0.88 g of yellow solid product with a yield of 76.7%.

MS (ESI, pos. ion) m/z: 763.6 $[M+H]^+$.

**Step 5: Synthesis of methyl 2-((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)-2-azabicyclo [2.2.2]octane-4-carboxylate**

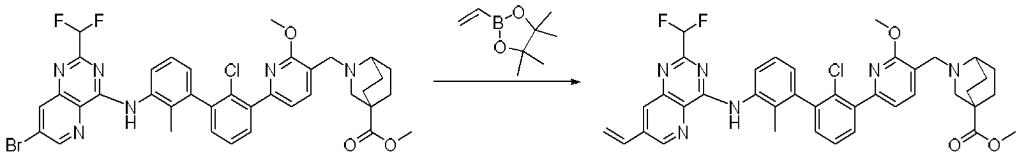

Methyl 2-((6-(3'-((7-bromo-2-(difluoromethyl)pyrido[3,2-d]pyrimidin-4-yl)amino)-2'-methyl-[1,1'-biphenyl]-3-yl)-2-methoxypyridin-3-yl)methyl)-2-azabicyclo[2.2.2]octane-4-carboxylate (0.88 g, 1.15 mmol), 4,4,5,5-tetramethyl-2-vinyl-1,3,2-dioxaborolane (0.89 g, 5.75 mmol), Pd(dppf)$Cl_2$ $CH_2Cl_2$ (0.33 g, 0.40 mmol), potassium phosphate (0.49 g, 2.3 mmol) were dissolved in a mixed solvent of 1,4-dioxane (35 mL) and water (7 mL) and heated at 100 °C for 5 h under nitrogen protection. After the reaction was complete, the reaction solution was concentrated under reduced pressure, and the residue was separated and purified by silica gel column chromatography (eluent: PE/EtOAc (v/v) = 3/2) to obtain 0.719 g of yellow solid product with a yield of 87.78%.

MS (ESI, pos. ion) m/z: 711.7 $[M+H]^+$.

**Step 6: Synthesis of methyl 2-((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)-2-azabicyclo[2.2.2] octane-4-carboxylate**

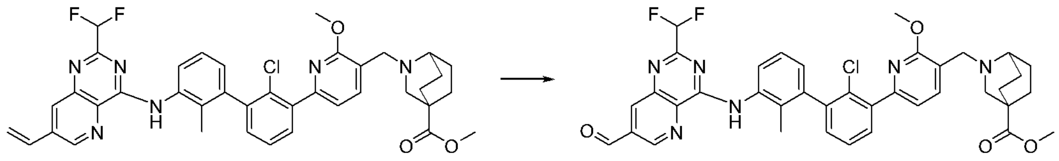

Methyl 2-((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)-2-azabicyclo[2.2.2]octane-4-carboxylate (0.72 g, 1.01 mmol) was dissolved in a mixed solvent of 1,4-dioxane (30 mL) and water (12 mL), then potassium osmate dihydrate (0.037 g, 0.1 mmol) was added, followed by sodium periodate (1.08 g, 5.05 mmol), and the mixture was reacted at room temperature for 3.5 h. After the reaction of the raw materials was complete, the mixture was filtered through celite, and the filtrate was concentrated under reduced pressure, and the residue was separated and purified by silica gel column chromatography (eluent: DCM/MeOH (v/v) = 33/1) to obtain 0.11 g of yellow liquid product with a yield of 15.2%.

MS (ESI, pos. ion) m/z: 713.7 $[M+H]^+$.

**Step 7: Synthesis of methyl (*R*)-2-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)-2-azabicyclo [2.2.2] octane-4-carboxylate**

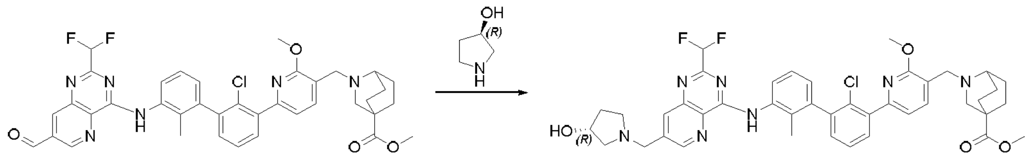

Methyl 2-((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)-2-azabicyclo[2.2.2]octane-4-carboxylate (0.108 g, 0.15 mmol) was dissolved in a mixed solvent of DCM (5 mL) and MeOH (5 mL), (*R*)-pyrrolidin-3-ol (0.065 g, 0.75 mmol) and 2 drops of acetic acid were added, and the mixture was stirred at room temperature overnight. Sodium cyanoborohydride (0.019 g, 0.3 mmol) was added and stirred at room temperature for 1 h. After the reaction of the raw materials was complete, the reaction solution was concentrated under reduced pressure, and the residue was separated and purified by silica gel column chromatography (eluent: DCM/MeOH (v/v) = 20/1) to obtain 0.077 g of yellow liquid product with a yield of 64.8%.

MS (ESI, pos. ion) m/z: 784.3 $[M+H]^+$.

**Step 8: Synthesis of (*R*)-2-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)-2-azabicyclo[2.2.2]octane-4-carboxylic acid**

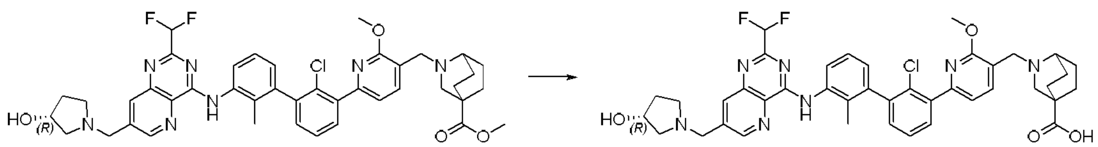

Methyl (*R*)-2-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)-2-azabicyclo[2.2.2]octane-4-carboxylate (0.077 g, 0.098 mmol) was dissolved in 1,4-dioxane (10 mL) and water (2 mL), lithium hydroxide monohydrate (0.041 g, 0.98 mmol) was added, and the reaction was stirred at room temperature overnight. After the reaction of the raw materials was complete, the pH of the system was adjusted to about 5 with acetic acid, the reaction solution was concentrated under reduced pressure, and the residue was separated and purified by preparative thin layer chromatography (eluent: DCM/MeOH (v/v) = 2/1) to obtain 44 mg of a yellow solid product with a yield of 58.18%.

MS (ESI, pos. ion) m/z: 770.3060 $[M+H]^-$.

$^1H$ NMR (400 MHz, DMSO-$d_6$) δ (ppm) 10.34 (s, 1H), 8.95 (s, 1H), 8.16 (s, 1H), 7.82 (d, *J* = 7.6 Hz, 1H), 7.74 (d, *J* = 8.0 Hz, 1H), 7.64 (d, *J* = 7.7 Hz, 1H), 7.53 (t, *J* = 7.7 Hz, 1H), 7.40 - 7.33 (m, 2H), 7.28 (d, *J* = 7.4 Hz, 1H), 7.15 (d, *J* = 7.5 Hz,

1H), 6.71 (s, 1H), 4.24 - 4.20 (m, 1H), 3.91 (s, 3H), 3.88 (s, 1H), 3.85 (s, 1H), 3.66 (s, 2H), 2.75 - 2.71 (m, 3H), 2.68 (d, *J* = 7.3 Hz, 1H), 2.62 - 2.59 (m, 1H), 2.41 (dd, *J* = 9.7, 3.6 Hz, 2H), 2.03 (s, 3H), 1.71 - 1.67 (m, 3H), 1.61 - 1.55 (m, 1H), 1.50 - 1.45 (m, 2H), 1.35 - 1.32 (m, 5H).

**Example 8 (*R*)-4-(((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)bicyclo[2.2.1]heptane-1-carboxylic acid**

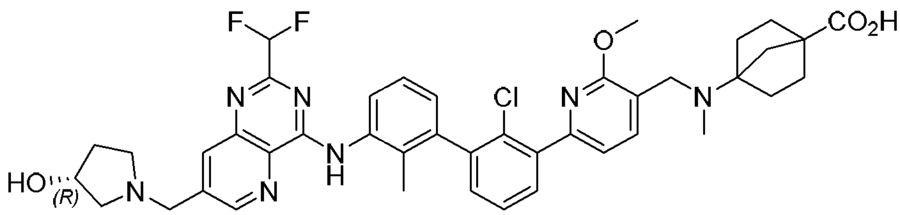

**Step 1: Synthesis of methyl 4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)amino)bicyclo[2.2.1]heptane-1-carboxylate**

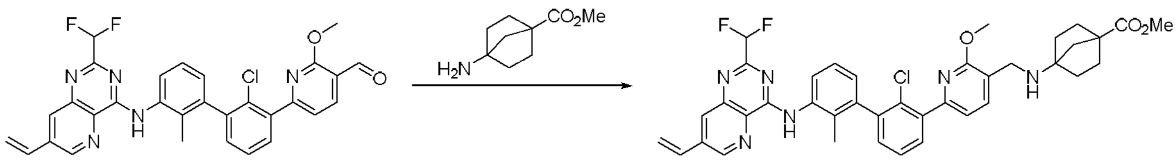

At room temperature, 6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-aldehyde (1.2 g, 2.15 mmol) and methyl 4-aminobicyclo[2.2.1]heptane-1-carboxylate (1.09 g, 6.45 mmol) were added to 2,2,2-trifluoroethanol (8 mL) and DCM (8 mL). The mixture was stirred at 45 °C for 0.5 h, and then sodium cyanoborohydride (0.14 g, 2.15 mmol) was slowly added and the reaction was continued to stir. After the reaction was completed, the reaction solution was concentrated under reduced pressure to remove the solvent, and the residue was separated and purified by silica gel column chromatography (eluent: $CH_2Cl_2$/MeOH (v/v) = 25/1), and 1.50 g of yellow solid product was obtained with a yield of 98.07%.
MS (ESI, pos. ion) m/z: 711.3 $[M+H]^+$.

**Step 2: Synthesis of methyl 4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)(methyl)amino)bicyclo [2.2.1] heptane-1-carboxylate**

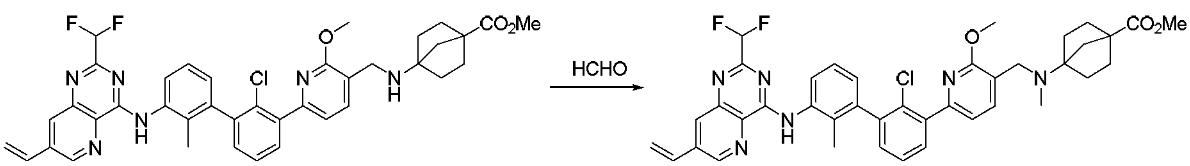


At room temperature, methyl 4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)amino)bicyclo[2.2.1]heptane-1-carboxylate (0.90 g, 1.27 mmol) and aqueous formaldehyde solution (0.11 g, 3.81 mmol) were added to DCM (5 mL) and 2,2,2-trifluoroethanol (5 mL), and the reaction was stirred at 45 °C for 30 minutes. Then, sodium cyanoborohydride (0.020 g, 0.32 mmol) was slowly added to continue the reaction. After the reaction was completed, the reaction solution was concentrated under reduced pressure to remove the solvent, and the residue was separated and purified by silica gel column chromatography (eluent: $CH_2Cl_2$/MeOH (v/v) = 20/1), and 0.80 g of yellow solid product was obtained with a yield of 87.17%.
MS (ESI, pos. ion) m/z: 725.3 $[M+H]^+$.

**Step 3: Synthesis of methyl 4-(((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)(methyl)amino)bicyclo[2.2.1]heptane-1-carboxylate**

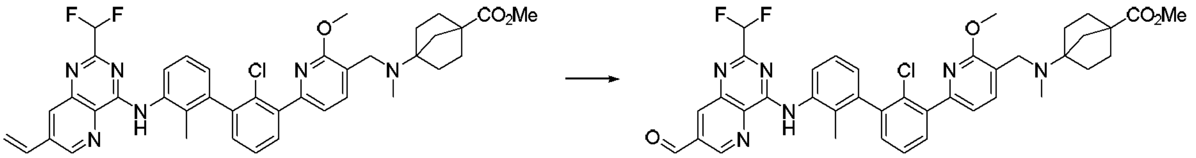

At room temperature, methyl 4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)(methyl)amino)bicyclo[2.2.1]heptane-1-carboxylate (0.75 g, 1.03 mmol), potassium osmate dihydrate (0.038 g, 0.10 mmol), sodium periodate (1.10 g, 5.15 mmol) were added to 1,4-dioxane (33 mL) and water (13 mL), and the reaction was stirred at room temperature. After the raw material was consumed, the reaction solution was concentrated under reduced pressure to remove the solvent, and the residue was separated and purified by silica gel column chromatography (eluent: $CH_2Cl_2$/MeOH (v/v) = 30/1), and 0.30 g of yellow oily product was obtained with a yield of 39.89%.

MS (ESI, pos. ion) m/z: 727.0 $[M+H]^+$.

**Step 4: Synthesis of methyl (*R*)-4-(((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)bicyclo[2.2.1]heptane-1-carboxylate**

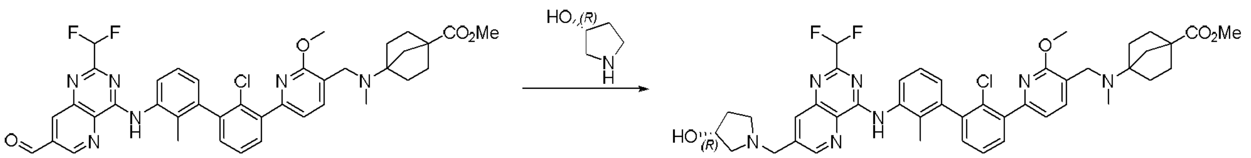

At room temperature, methyl 4-(((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)(methyl)amino)bicyclo[2.2.1]heptane-1-carboxylate (0.29 g, 0.40 mmol) and (3R)-pyrrolidin-3-ol (0.070 g, 0.80 mmol) were added to methanol (5 mL) and DCM (5 mL). The reaction was stirred at room temperature overnight. Then, sodium cyanoborohydride (0.025 g, 0.40 mmol) was slowly added and the reaction was continued to stir. After the raw material was consumed, the reaction solution was concentrated under reduced pressure to remove the solvent, and the residue was separated and purified by silica gel column chromatography (eluent: DCM/MeOH (v/v) = 10/1), and 0.20 g of yellow solid product was obtained with a yield of 62.82%.

MS (ESI, pos. ion) m/z: 798.0 $[M+H]^+$.

**Step 5: Synthesis of (*R*)-4-(((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)bicyclo [2.2.1]heptane-1-carboxylic acid**

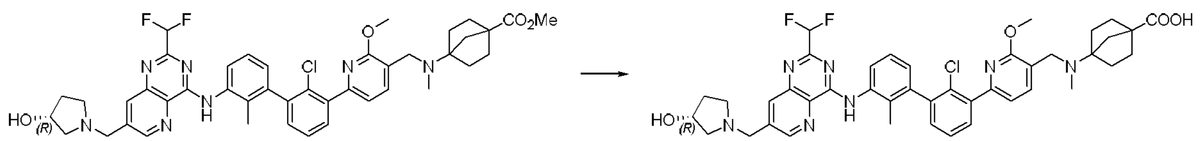

At room temperature, methyl (*R*)-4-(((6-(2-chloro-3'-((2-(difluoromethyl)-7-((3-hydroxypyrrolidin-1-yl)methyl) pyrido[3,2-*d*]pyrimidin-4-yl)amino)-2'-methyl-[1,1'-biphenyl]-3-yl)-2-methoxypyridin-3-yl)methyl)(methyl)amino)bicyclo [2.2.1]heptane-1-carboxylate (0.2 g, 0.25 mmol) and lithium hydroxide monohydrate (0.031 g, 0.75 mmol) were added to 1,4-dioxane (6 mL) and water (1.5 mL), and the reaction was stirred at 45 °C. After the raw material was consumed, the reaction solution was concentrated under reduced pressure to remove the solvent, and the residue was separated and

purified by silica gel column chromatography (eluent: DCM/MeOH (v/v) = 8/1), and 0.15 g of yellow solid product was obtained with a yield of 76.34%.

MS (ESI, pos. ion) m/z: 784.3290 $[M+H]^-$.

$^1H$ NMR (400 MHz, DMSO-$d_6$) δ 10.33 (s, 1H), 8.95 (d, *J* = 2.0 Hz, 1H), 8.17 (d, *J* = 1.9 Hz, 1H), 7.82 (d, *J* = 7.5 Hz, 1H), 7.76 (d, *J* = 8.0 Hz, 1H), 7.64 (dd, *J* = 7.7, 1.8 Hz, 1H), 7.53 (t, *J* = 7.6 Hz, 1H), 7.41 - 7.34 (m, 2H), 7.28 (d, *J* = 7.4 Hz, 1H), 7.16 (d, *J* = 7.6 Hz, 1H), 6.71 (t, *J* = 54.5 Hz, 1H), 4.76 (s, 1H), 4.23 (dq, *J* = 7.0, 3.3 Hz, 1H), 3.91 (s, 3H), 3.90 - 3.80 (m, 2H), 2.78 - 2.64 (m, 2H), 2.47 (d, *J* = 11.6 Hz, 1H), 2.42 (dd, *J* = 9.7, 3.5 Hz, 1H), 2.16 (s, 3H), 2.05 (s, 3H), 1.99 (q, *J* = 12.7, 9.8 Hz, 3H), 1.82 - 1.72 (m, 2H), 1.67 (s, 4H), 1.58 (q, *J* = 8.3, 7.4 Hz, 3H), 1.22 (s, 2H).

**Example 9 (*R*)-4-(((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)amino)bicyclo[2.2.1]heptane-1-carboxylic acid**

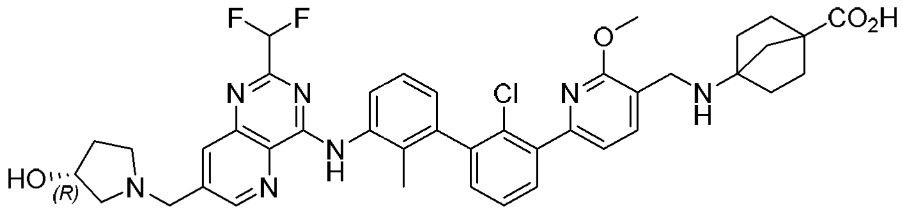

Step 1: Synthesis of ethyl 4-(((6-(3'-((7-bromo-2-(difluoromethyl)pyrido[3,2-d]pyrimidin-4-yl)amino)-2-chloro-2'-methyl-[1,1'-biphenyl]-3-yl)-2-methoxypyridin-3-**yl)methyl)amino)bicyclo[2.2.1]heptane-1-carboxylate**

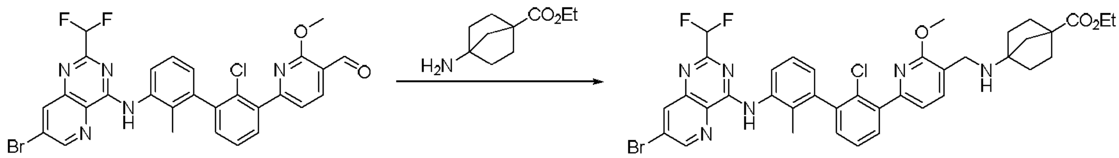

At room temperature, 6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxynicotinaldehyde (2.6 g, 4.26 mmol) and ethyl 4-aminobicyclo[2.2.1]heptane-1-carboxylate (1.56 g, 8.52 mmol) were added to DCM (10 mL) and 2,2,2-trifluoroethanol (10 mL). The mixture was stirred at 45 °C for 1 h, and then sodium cyanoborohydride (0.27 g, 4.26 mmol) was slowly added and the reaction was continued to stir. After the raw material was consumed, the reaction solution was concentrated under reduced pressure to remove the solvent, and the residue was separated and purified by silica gel column chromatography (eluent: DCM/MeOH (v/v) = 25/1), and 0.80 g of yellow solid product was obtained with a yield of 24.16%.

MS (ESI, pos. ion) m/z: 772.2 $[M+H]^+$.

**Step 2: Synthesis of ethyl 4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)amino)bicyclo[2.2.1]heptane-1-carboxylate**

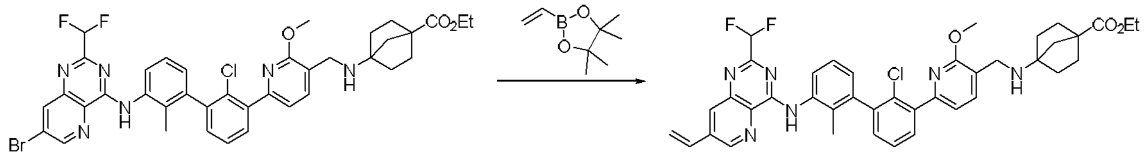

At room temperature, ethyl 4-(((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)amino)bicyclo[2.2.1]heptane-1-carboxylate (0.78 g, 1.00 mmol), vinyl boronic acid pinacol ester (0.77 g, 5 mmol), Pd(dppf)$Cl_2$·DCM (0.12 g, 0.15 mmol) and potassium phosphate (0.42 g, 2 mmol) were added to 1,4-dioxane (10 mL) and water (2.5 mL) and the reaction was stirred at 90 °C.

After the raw material was consumed, the reaction solution was concentrated under reduced pressure to remove the solvent, and the residue was separated and purified by silica gel column chromatography (eluent: DCM/MeOH (v/v) = 10/1), and 0.65 g of yellow solid product was obtained with a yield of 89.41%.

MS (ESI, pos. ion) m/z: 725.0 [M+H]+.

**Step 3: Synthesis of ethyl 4-(((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)amino)bicyclo[2.2.1]heptane-1-carboxylate**

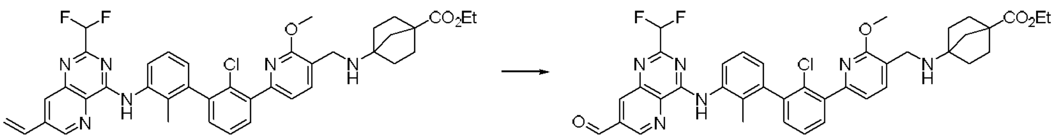

At room temperature, ethyl 4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)amino)bicyclo[2.2.1]heptane-1-carboxylate (0.65 g, 0.90 mmol) was added to 1,4-dioxane (33 mL) and water (13 mL), and then sodium periodate (0.96 g, 4.5 mmol) and potassium osmate dihydrate (0.033 g, 0.090 mmol) were slowly added, and the reaction was stirred at room temperature for 30 min. After the raw material was consumed, the reaction solution was concentrated under reduced pressure to remove the solvent, and the residue was separated and purified by silica gel column chromatography (eluent: PE/EtOAc (v/v) = 1/1), and 0.36 g of yellow solid product was obtained with a yield of 55.23%.

MS (ESI, pos. ion) m/z: 727.7 [M+H]+.

**Step 4: Synthesis of ethyl (*R*)-4-(((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)amino)bicyclo [2.2.1]heptane-1-carboxylate**

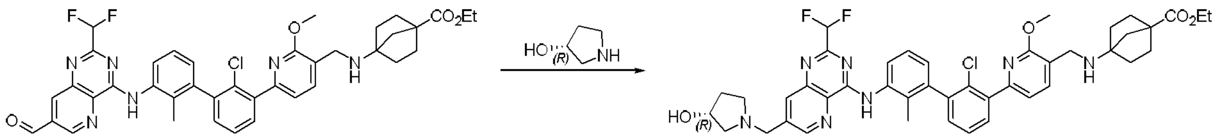

At room temperature, ethyl 4-(((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)amino)bicyclo[2.2.1]heptane-1-carboxylate (0.35 g, 0.48 mmol) and (3*R*)-pyrrolidin-3-ol (0.13 g, 1.44 mmol) were added to DCM (5 mL) and 2,2,2-trifluoroethanol (5 mL). The reaction was stirred at 45 °C for 30 min. Then, sodium cyanoborohydride (0.030 g, 0.48 mmol) was slowly added and the reaction was continued to stir. After the raw material was consumed, the reaction solution was concentrated under reduced pressure to remove the solvent, and the residue was separated and purified by silica gel column chromatography (eluent: DCM/MeOH (v/v) = 10/1), and 0.35 g of yellow solid product was obtained with a yield of 91.09%.

MS (ESI, pos. ion) m/z: 798.8 [M+H]+.

**Step 5: Synthesis of (*R*)-4-(((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)amino)bicyclo[2.2.1]heptane-1-carboxylic acid**

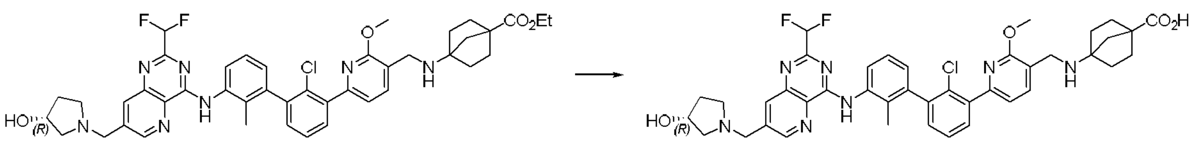

At room temperature, ethyl (*R*)-4-(((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)bicyclo

[2.2.1]heptane-1-carboxylate (0.2 g, 0.25 mmol) and lithium hydroxide monohydrate (0.031 g, 0.75 mmol) were added to 1,4-dioxane (6 mL) and water (1.5 mL), and the reaction was stirred at 45 °C. After the raw material was consumed, the reaction solution was concentrated under reduced pressure to remove the solvent, and the residue was separated and purified by silica gel column chromatography (eluent: DCM/MeOH (v/v) = 10/1), and 0.080 g of yellow solid product was obtained with a yield of 40.73%.

MS (ESI, pos. ion) m/z: 770.3 $[M+H]^+$.

$^1$H NMR (400 MHz, DMSO-$d_6$) δ 10.34 (s, 1H), 8.95 (d, *J* = 1.9 Hz, 1H), 8.17 (d, *J* = 2.0 Hz, 1H), 7.86 (d, *J* = 7.5 Hz, 1H), 7.75 (d, *J* = 8.0 Hz, 1H), 7.63 (dd, *J* = 7.8, 1.8 Hz, 1H), 7.53 (t, *J* = 7.6 Hz, 1H), 7.38 (dd, *J* = 9.3, 6.9 Hz, 2H), 7.26 (d, *J* = 7.5 Hz, 1H), 7.16 (d, *J* = 7.5 Hz, 1H), 6.71 (t, *J* = 54.5 Hz, 1H), 4.23 (dq, *J* = 6.8, 3.4 Hz, 1H), 3.92 (s, 3H), 3.90 - 3.81 (m, 2H), 3.73 (s, 3H), 2.77 - 2.65 (m, 2H), 2.47 (d, *J* = 9.6 Hz, 1H), 2.42 (dd, *J* = 9.7, 3.5 Hz, 1H), 2.05 (s, 3H), 1.97 - 1.86 (m, 2H), 1.76 - 1.66 (m, 2H), 1.64 - 1.59 (m, 2H), 1.56 (s, 3H), 1.49 (dd, *J* = 10.5, 3.7 Hz, 2H).

**Example 10 (*R*)-4-((((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)methyl)bicyclo [2.2.1]heptane-1-carboxylic acid**

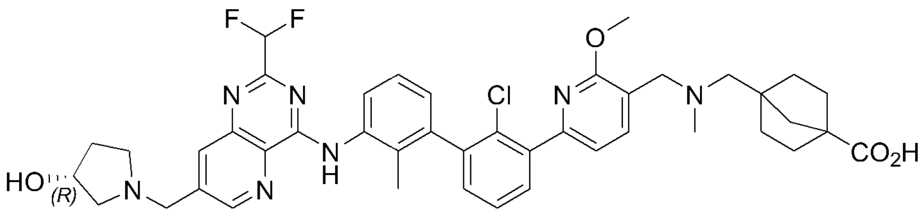

**Step 1: Synthesis of methyl 4-((((6-(3-bromo-2-chlorophenyl)-2-methoxypyridin-3-yl)methyl)amino)methyl)bicyclo[2.2.1]heptane-1-carboxylate**

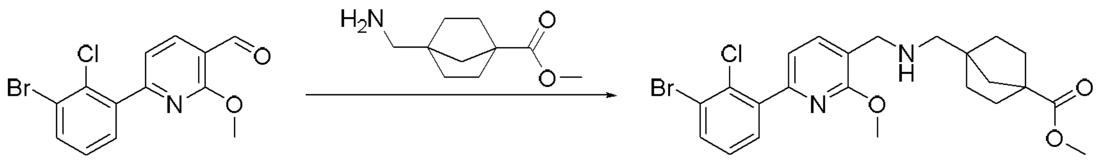

6-(3-Bromo-2-chlorophenyl)-2-methoxynicotinaldehyde (3 g, 9.19 mmol) and methyl 4-(aminomethyl)bicyclo [2.2.1]heptane-1-carboxylate (3.03 g, 16.54 mmol) were dissolved in 2,2,2-trifluoroethanol (30 mL) and DCM (30 mL), and triethylamine (2 mL) was added dropwise. The mixture was stirred at 50 °C for 15 h. Sodium cyanoborohydride (866.3 mg, 13.8 mmol) was added and the reaction was continued at room temperature. After the raw material was consumed, the reaction solution was concentrated under reduced pressure to remove the solvent, and the residue was separated and purified by silica gel column chromatography (DCM/MeOH (v/v) = 20/1), and 4.53 g of a light yellow viscous product was obtained with a yield of 99.86%.

MS (ESI, pos. ion) m/z: 493.1 $[M+H]^+$.

**Step 2: Synthesis of methyl 4-((((6-(3-bromo-2-chlorophenyl)-2-methoxypyridin-3-yl)methyl)(methyl)amino) methyl)bicyclo[2.2.1]heptane-1-carboxylate**

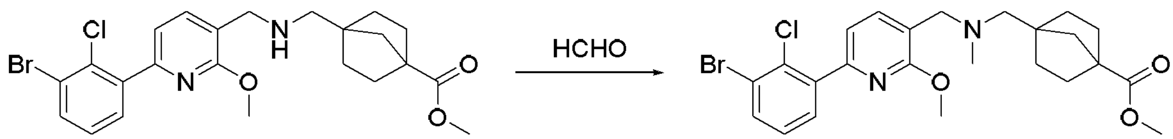


Methyl 4-((((6-(3-bromo-2-chlorophenyl)-2-methoxypyridin-3-yl)methyl)amino)methyl)bicyclo[2.2.1]heptane-1-carboxylate (4.69 g, 9.50 mmol) and aqueous formaldehyde solution (3.08 g, 38 mmol) were dissolved in methanol (120 ml), and then acetic acid (713.09 mg, 11.88 mmol) was added and stirred at room temperature for 20 h. Sodium cyanoborohydride (2984.9 mg, 47.5 mmol) was slowly added and stirred at room temperature for 1 h. After the raw material was consumed, the reaction solution was concentrated under reduced pressure to remove the solvent, and the residue was separated and purified by silica gel column chromatography (DCM/MeOH (v/v) = 40/1), and 3.16 g of a light yellow viscous product was obtained with a yield of 65.52%.

MS (ESI, pos. ion) m/z: 507.4 $[M+H]^+$.

**Step 3: Synthesis of methyl 4-((((6-(3'-amino-2-chloro-2'-methyl-[1,1'-biphenyl]-3-yl)-2-methoxypyridin-3-yl) methyl)(methyl)amino)methyl)bicyclo[2.2.1]heptane-1-carboxylate**

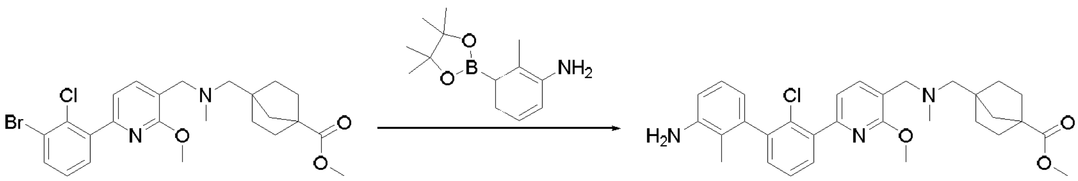

Methyl 4-((((6-(3-bromo-2-chlorophenyl)-2-methoxypyridin-3-yl)methyl)(methyl)amino)methyl)bicyclo[2.2.1] heptane-1-carboxylate (0.5 g, 0.98 mmol), 2-methyl-3-(tetramethyl-1,3,2-dioxaborolan-2-yl)aniline (0.46 g, 1.96 mmol) were dissolved in a mixed solvent of water (4 mL) and 1,4-dioxane (20 mL), then potassium carbonate (0.31 g, 2.94 mmol) and $Pd(dppf)Cl_2 \cdot DCM$ (0.074 g, 0.098 mmol) was added in sequence, the mixture was protected by nitrogen and heated to 90 °C for 15 h. After the raw material was consumed, the reaction solution was concentrated under reduced pressure to remove the solvent, and the residue was separated and purified by silica gel column chromatography (PE/EtOAc (v/v) = 6/1), and 0.525 g of a light yellow viscous product was obtained with a yield of 99.84%.

MS (ESI, pos. ion) m/z: 534.5 $[M+H]^+$.

**Step 4: Synthesis of methyl 4-((((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)(methyl)amino)methyl)bicyclo[2.2.1]heptane-1-carboxylate**

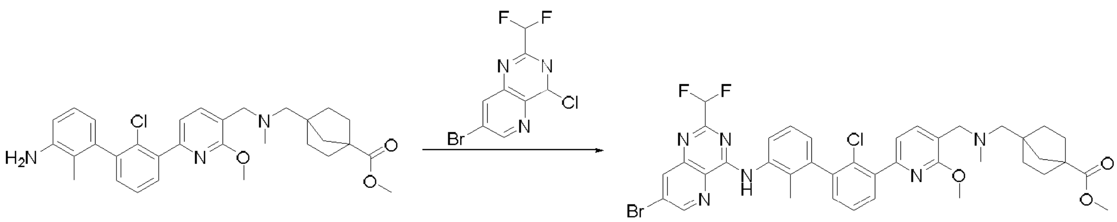

7-Bromo-4-chloro-2-(difluoromethyl)pyrido[3,2-*d*]pyrimidine (400 mg, 1.36 mmol) and methyl 4-((((6-(3'-amino-2-chloro-2'-methyl-[1,1'-biphenyl]-3-yl)-2-methoxypyridin-3-yl)methyl)(methyl)amino)methyl)bicyclo[2.2.1]heptane-1-carboxylate (0.52 g, 0.98 mmol) were dissolved in n-butanol (30 mL) and stirred at 130 °C for 3 h under $N_2$. After the raw material was consumed, the reaction solution was concentrated under reduced pressure to remove the solvent, and the residue was separated and purified by silica gel column chromatography (DCM/MeOH (v/v) = 20/1), and 0.433 g of a brown yellow powder product was obtained with a yield of 40.24%.

MS (ESI, pos. ion) m/z: 791.2 $[M+H]^+$.

**Step 5: Synthesis of methyl 4-((((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)(methyl)amino)methyl)bicyclo[2.2.1]heptane-1-carboxylate**

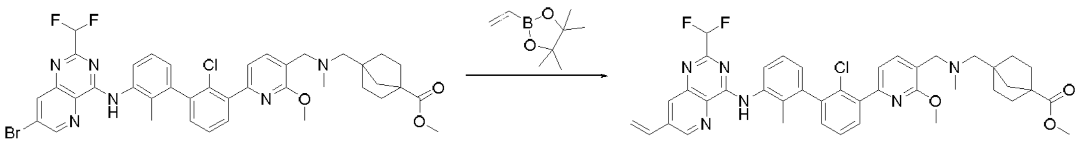

Methyl 4-((((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)(methyl)amino)methyl)bicyclo[2.2.1]heptane-1-carboxylate (0.433 g, 0.55 mmol) was dissolved in a mixed solvent of 1,4-dioxane (25 mL) and water (5 mL). 2-vinyl-4,4,5,5-tetramethyl-1,3,2-dioxaborolane (423.53 mg, 2.75 mmol), Pd(dppf)$Cl_2$·DCM (157.20 mg, 0.19 mmol) and potassium phosphate (233.50 mg, 1.1 mmol) were added in sequence. The mixture was stirred at 100 °C for 3 h under $N_2$. After the raw material was consumed, the reaction solution was concentrated under reduced pressure to remove the solvent, and the residue was separated and purified by silica gel column chromatography (DCM/MeOH (v/v) = 20/1), and 0.4 g of brown-yellow viscous product was obtained with a yield of 98.98%.

MS (ESI, pos. ion) m/z: 739.3 $[M+H]^+$.

**Step 6: Synthesis of methyl 4-((((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)(methyl)amino)methyl)bicyclo[2.2.1]heptane-1-carboxylate**

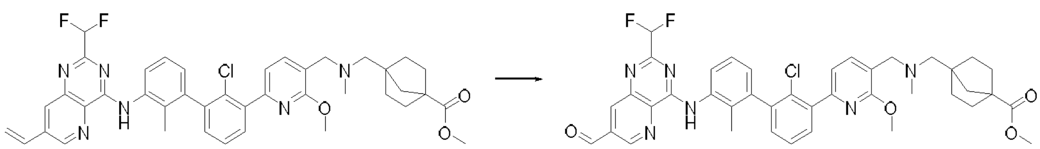

Methyl 4-((((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)(methyl)amino)methyl)bicyclo[2.2.1]heptane-1-carboxylate (0.4 g, 0.54 mmol) was dissolved in 1,4-dioxane (18 mL) and water (6 mL), potassium osmate dihydrate (9.95 mg, 0.027 mmol) and sodium periodate (0.29 g, 1.35 mmol) were added in sequence, and the system was stirred at room temperature for 2 h. After the raw material was consumed, the reaction solution was concentrated under reduced pressure to remove the solvent, and the residue was separated and purified by silica gel column chromatography (DCM/MeOH (v/v) = 20/1), and 0.253 g of brown-yellow viscous product was obtained with a yield of 63.08%.

MS (ESI, pos. ion) m/z: 741.3 $[M+H]^+$.

**Step 7: Synthesis of methyl (*R*)-4-((((6-(2-chloro-3'-((2-(difluoromethyl)-7-((3-hydroxypyrrolidin-1-yl)methyl) pyrido[3,2-*d*]pyrimidin-4-yl)amino)-2'-methyl-[1,1'-biphenyl]-3-yl)-2-methoxypyridin-3-yl)methyl)(methyl)amino)methyl)bicyclo[2.2.1] heptane-1-carboxylate**

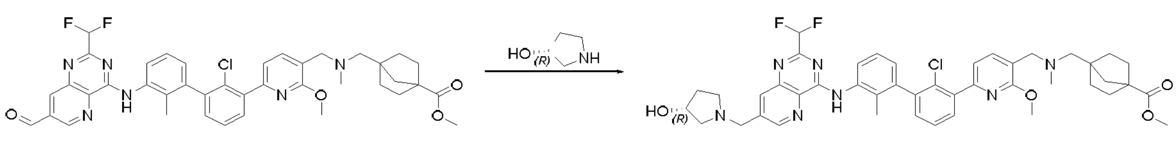

Methyl 4-((((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)(methyl)amino)methyl)bicyclo[2.2.1]heptane-1-carboxylate (0.253 g, 0.34 mmol) and (3*R*)-pyrrolidin-3-ol (148.10 mg, 1.70 mmol) were dissolved in methanol (30 mL) and DCM (10 mL), and acetic acid (102.09 mg, 1.70 mmol) was added dropwise. The reaction was stirred at room temperature for 2 h. Sodium cyanoborohydride (64.10 mg, 1.02 mmol) was added and the reaction was continued at room temperature for 1.5 h. After the raw material was consumed, the reaction solution was concentrated under reduced pressure to remove the solvent, and the residue was separated and purified by silica gel column chromatography (DCM/MeOH (v/v) = 12/1), and 0.15 g of a light yellow powder product was obtained with a yield of 54.10%.

MS (ESI, pos. ion) m/z: 812.6 $[M+H]^+$.

**Step 8: Synthesis of (*R*)-4-((((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)methyl)bicyclo[2.2.1]heptane-1-carboxylic acid**

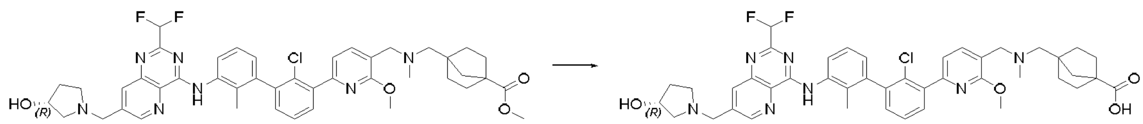

Methyl (*R*)-4-((((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)methyl)bicyclo[2.2.1]heptane-1-carboxylate (0.15 g, 0.18 mmol) was dissolved in a mixed solvent of 1,4-dioxane (15 ml) and water (3 ml), lithium hydroxide monohydrate (90.63 mg, 2.16 mmol) was added, and the mixture was heated to 60 °C and stirred for 4 h. After the raw material was consumed, the reaction solution was concentrated under reduced pressure to remove the solvent, and the residue was separated and purified by silica gel column chromatography (DCM/MeOH = 12/1), and 70 mg of a light yellow powder product was obtained with a yield of 47.49%.

MS (ESI, pos. ion) m/z: 798.3 $[M+H]^+$.

$^1H$ NMR (400 MHz, DMSO-$d_6$) δ (ppm) 8.94 (s, 1H), 8.15 (s, 1H), 7.80 (d, *J* = 7.5 Hz, 1H), 7.73 (d, *J* = 8.1 Hz, 1H), 7.63 (d, *J* = 7.6 Hz, 1H), 7.52 (t, *J* = 7.9 Hz, 1H), 7.44 - 7.19 (m, 4H), 7.14 (d, *J* = 7.7 Hz, 1H), 6.69 (t, *J* = 54.5 Hz, 1H), 4.23 (dd, *J* = 10.7, 6.7 Hz, 1H), 3.88 (s, 3H), 3.86 - 3.77 (m, 4H), 2.76 - 2.63 (m, 3H), 2.53 (s, 2H), 2.44 - 2.36 (m, 2H), 2.21 (s, 3H), 2.03 (s, 4H), 1.78 (s, 3H), 1.65 - 1.57 (m, 3H), 1.52 - 1.43 (m, 3H), 1.41 - 1.37 (m, 2H).

**Example 11 (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

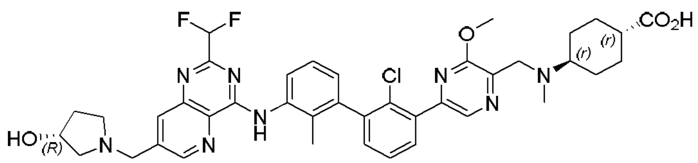

**Step 1: Synthesis of methyl (1*r*,4*r*)-4-(((5-(3-bromo-2-chlorophenyl)-3-methoxypyrazin-2-yl)methyl)amino)cyclohexane-1-carboxylate**

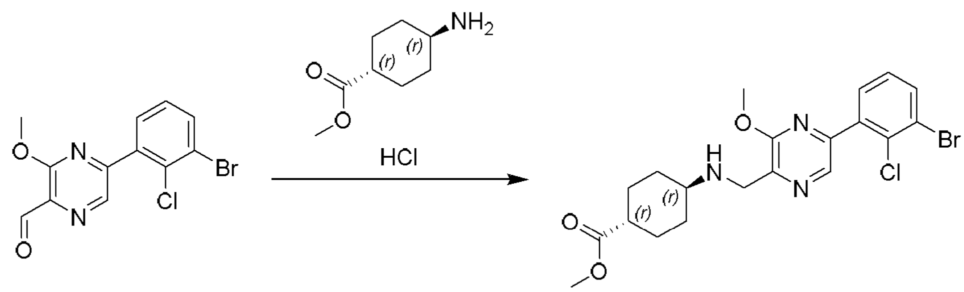


5-(3-Bromo-2-chlorophenyl)-3-methoxypyrazine-2-carboxaldehyde (0.66 g, 2.01 mmol), methyl (1*r*,4*r*)-4-aminocyclohexane-1-carboxylate hydrochloride (1.17 g, 6.03 mmol), DCM (20 mL), methanol (20 mL), triethylamine (0.31 g, 3.01 mmol) and acetic acid (0.12 g, 2.01 mmol) were added to a 100 mL single-necked bottle in sequence, and the mixture was reacted at 50 °C overnight. Sodium cyanoborohydride (0.25 g, 4.02 mmol) was added, and the reaction was continued at room temperature. After the reaction was complete, the filtrate was concentrated under reduced pressure to remove the solvent, and 830.0 mg of a white solid product was obtained with a yield of 87.87%.

MS (ESI, pos. ion) m/z: 467.9 $[M+H]^+$.

**Step 2: Synthesis of methyl (1*r*,4*r*)-4-(((5-(3-bromo-2-chlorophenyl)-3-methoxypyrazin-2-yl)methyl(methyl)) amino)cyclohexane-1-carboxylate**

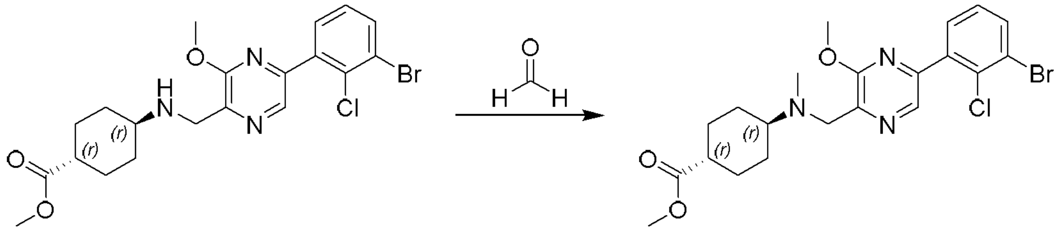

Methyl (1*r*,4*r*)-4-(((5-(3-bromo-2-chlorophenyl)-3-methoxypyrazin-2-yl)methyl)(methyl)amino)cyclohexane-1-carboxylate (800 mg, 1.71 mmol), formaldehyde (385.13 mg, 5.13 mmol), DCM (15 mL), methanol (15 mL), triethylamine (259.55 mg, 2.56 mmol) and acetic acid (123.22 mg, 2.05 mmol) were added to a 100 mL single-necked bottle in sequence, and the mixture was reacted at 50 °C for 8 h. Sodium cyanoborohydride (429.83 mg, 6.84 mmol) was added and the reaction was continued at room temperature. After the reaction was completed, the filtrate was concentrated under reduced pressure to remove the solvent, and the residue was separated and purified by silica gel column chromatography (eluent: PE/EtOAc (v/v) = 1/1) to obtain 750.0 mg of a light yellow solid product with a yield of 91.03%.

MS (ESI, pos. ion) m/z: 481.9 $[M+H]^+$.

**Step 3: Synthesis of methyl (1*r*,4*r*)-4-(((5-(3'-amino-2-chloro-2'-methyl-[1,1'-biphenyl]-3-yl)-3-methoxypyrazin-2-yl)methyl)(methyl)amino)cyclohexane-1-carboxylate**

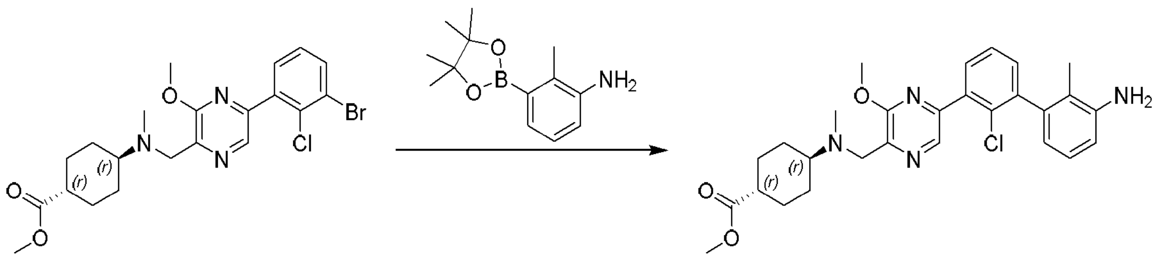

To a 30 mL microwave tube, methyl (1*r*,4*r*)-4-(((5-(3-bromo-2-chlorophenyl)-3-methoxypyrazin-2-yl)methyl)(methyl)amino)cyclohexane-1-carboxylate (790 mg, 1.64 mmol), 2-methyl-3-(tetramethyl-1,3,2-dioxaborolan-2-yl)aniline (477.88 mg, 2.05 mmol), potassium carbonate (566.66 mg, 4.1 mmol), Pd(dppf)$Cl_2$·DCM (133.93 mg, 0.16 mmol), 1,4-dioxane (10 mL) and water (2 mL) were added in sequence. The tube was sealed and reacted at 130 °C for 2 h. After the reaction was completed, the reaction solution was concentrated under reduced pressure to remove the solvent, and the residue was separated and purified by silica gel column chromatography (eluent: DCM/MeOH (v/v) = 20/1) to obtain 750.0 mg of a light yellow solid product with a yield of 90.04%.

MS (ESI, pos. ion) m/z: 509.2 $[M+H]^+$.

**Step 4: Synthesis of methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclohexane-1-carboxylate**

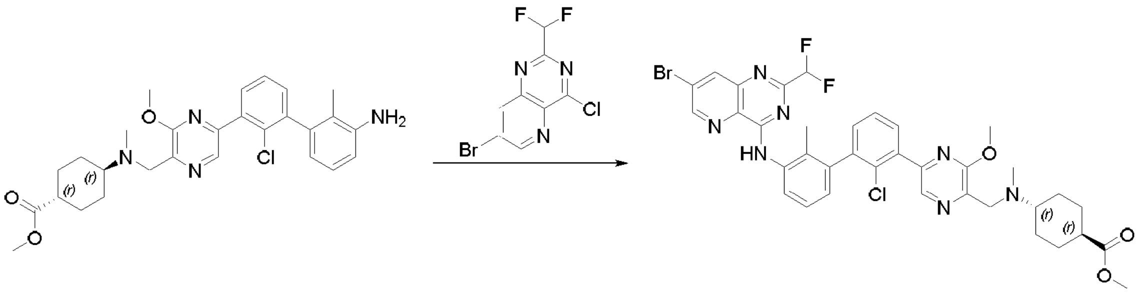

To a 100 mL single-necked bottle, methyl (1*r*,4*r*)-4-(((5-(3'-amino-2-chloro-2'-methyl-[1,1'-biphenyl]-3-yl)-3-methoxypyrazin-2-yl)methyl)(methyl)amino)cyclohexane-1-carboxylate (790 mg, 1.55 mmol), *tert*-butanol (50 mL) and 7-bromo-4-chloro-2-(difluoromethyl)pyrido[3,2-*d*]pyrimidine (593.38 mg, 2.02 mmol)were added in sequence and reacted at 100 °C under $N_2$. After the reaction was complete, the solvent was removed by concentration under reduced pressure to obtain 1.13 g of a light yellow solid product with a yield of 94.92%.

MS (ESI, pos. ion) m/z: 765.9 $[M+H]^+$.

**Step 5: Synthesis of methyl (1*r*,4*r*)-4-(((5-(2-chloro-3'-((2-(difluoromethyl)-7-vinylpyrido[3,2-*d*]pyrimidin-4-yl) amino)-2'-methyl-[1,1'-biphenyl]-3-yl)-3-methoxypyrazin-2-yl)methyl)(methyl)amino)cyclohexane-1-carboxylate**

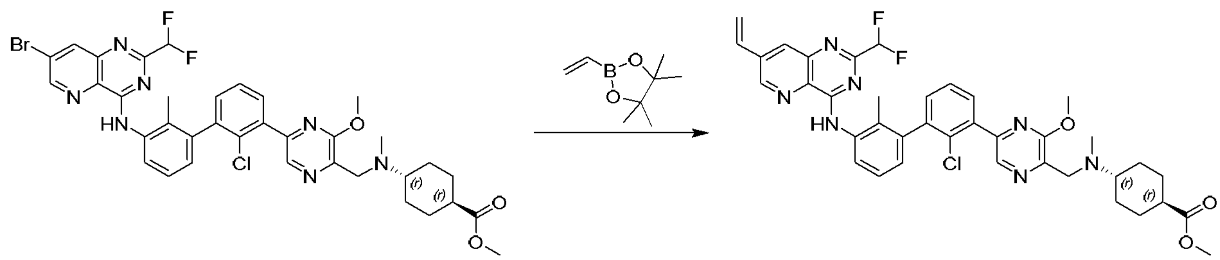

To a 30 mL microwave tube, methyl (1*r*,4*r*)-4-(((5-(3'-((7-bromo-2-(difluoromethyl)pyrido[3,2-*d*]pyrimidin-4-yl) amino)-2-chloro-2'-methyl-[1,1'-biphenyl]-3-yl)-3-methoxypyrazin-2-yl)methyl)(methyl)amino)cyclohexane-1-carboxylate (1.13 g, 1.47 mmol), 2-vinyl-4,4,5,5-tetramethyl-1,3,2-dioxaborolane (0.68 g, 4.41 mmol), potassium phosphate (0.78 g, 3.67 mmol), Pd(dppf)$Cl_2$·DCM (0.12 g, 0.15 mmol), 1,4-dioxane (15 mL) and water (3 mL) were added in sequence, the tube was sealed, and the reaction was carried out at 130 °C for 1.5 h. After the reaction was completed, the reaction solution was concentrated under reduced pressure to remove the solvent, and the residue was separated and purified by silica gel column chromatography (eluent: DCM/MeOH (v/v) = 40/1) to obtain 935.0 mg of a white solid product with a yield of 88.87%.

MS (ESI, pos. ion) m/z: 714.3 $[M+H]^+$.

**Step 6: Synthesis of methyl (1*r*,4*r*)-4-(((5-(2-chloro-3'-((2-(difluoromethyl)-7-formylpyrido[3,2-*d*]pyrimidin-4-yl) amino)-2'-methyl-[1,1'-biphenyl]-3-yl)-3-methoxypyrazin-2-yl)methyl)(methyl)amino)cyclohexane-1-carboxylate**

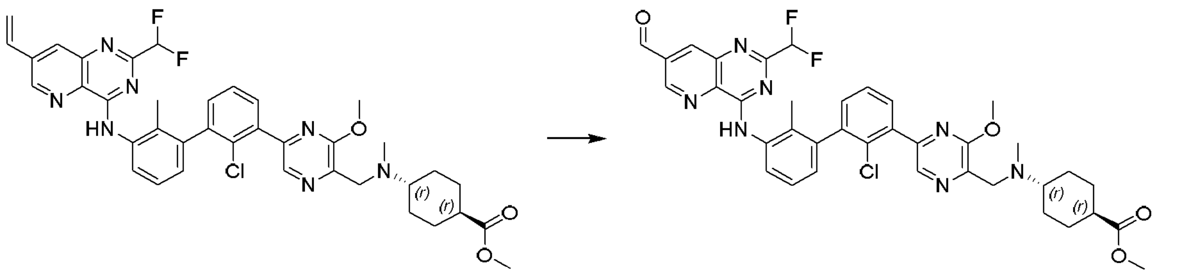

To a 250 mL single-necked bottle, methyl (1*r*,4*r*)-4-(((5-(2-chloro-3'-((2-(difluoromethyl)-7-vinylpyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (935 mg, 1.31 mmol), 1,4-dioxane (75 mL), water (30 mL), potassium osmate dihydrate (48 mg, 0.13 mmol) and sodium periodate (1400 mg, 6.55 mmol) were added in sequence and reacted at room temperature. After the reaction was completed, the filtrate was concentrated under reduced pressure to remove the solvent, and the residue was separated and purified by silica gel column chromatography (eluent: DCM/MeOH (v/v) = 30/1) to obtain 270.0 mg of a light yellow solid product with a yield of 28.80%.

MS (ESI, pos. ion) m/z: 716.2 $[M+H]^+$.

**Step 7: Synthesis of methyl (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

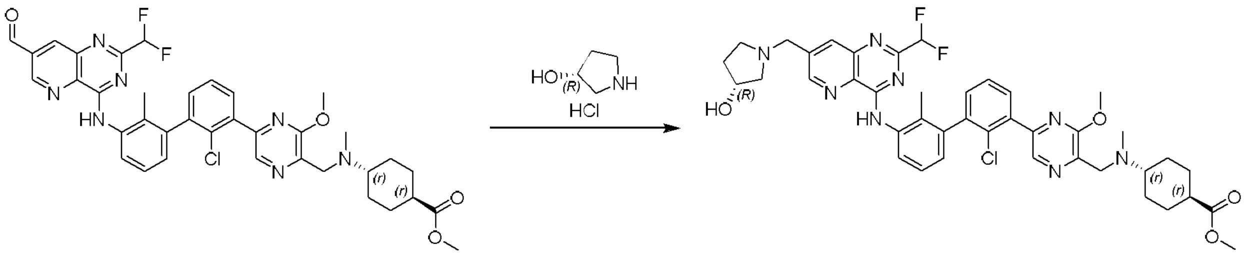


To a 100 mL single-necked bottle, methyl (1*r*,4*r*)-4-(((5-(2-chloro-3'-((2-(difluoromethyl)-7-formylpyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (210 mg, 0.29 mmol), (3R)-pyrrolidin-3-ol (179.19 mg, 1.45 mmol), DCM (15 mL), methanol (15 mL), triethylamine (44 mg, 0.43 mmol) and acetic acid (26 mg, 0.43 mmol) were added in sequence and reacted at 50 °C overnight. Sodium cyanoborohydride (36 mg, 0.57 mmol) was added and the reaction was continued at room temperature. After the reaction was completed, the filtrate was concentrated under reduced pressure to remove the solvent, and the residue was separated and purified by silica gel column chromatography (eluent: DCM/MeOH (v/v) = 10/1) to obtain 200.0 mg of a light yellow solid product with a yield of 86.63%.

MS (ESI, pos. ion) m/z: 787.4 $[M+H]^+$.

**Step 8: Synthesis of (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

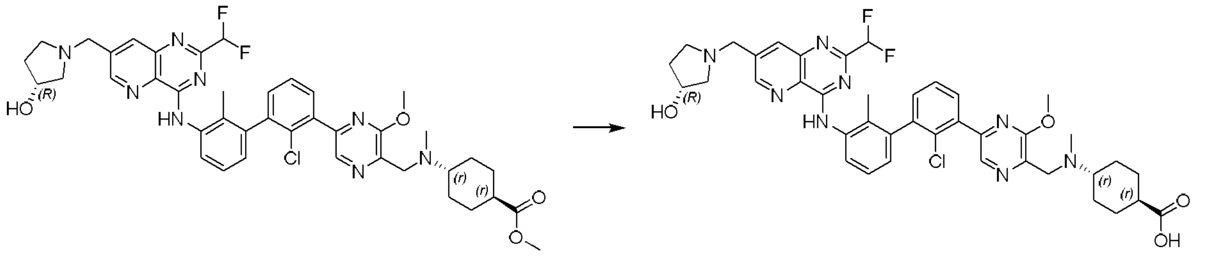

To a 50 mL single-necked bottle, methyl (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (170 mg, 0.22 mmol), 1,4-dioxane (15 mL), water (5 mL) and lithium hydroxide monohydrate (18 mg, 0.43 mmol) were added in sequence and reacted at 50 °C. After the reaction was complete, the solvent was removed by concentration under reduced pressure, and the mixture was dissolved with DCM (15 mL) and MeOH (15 mL). Acetic acid (1 mL) was added and the solvent was removed by concentration under reduced pressure. The residue was separated and purified by preparative silica gel thin layer chromatography. (Eluent: DCM/MeOH (v/v) = 5/1) to obtain 66.0 mg of a light yellow solid product with a yield of 39.53%.

MS (ESI, pos. ion) m/z: 773.3 $[M+H]^+$.

$^1$H NMR (400 MHz, DMSO-$d_6$) δ 10.35 (s, 1H), 8.95 (d, *J* = 1.9 Hz, 1H), 8.42 (s, 1H), 8.18 (d, *J* = 1.9 Hz, 1H), 7.76 (d, *J* = 8.0 Hz, 1H), 7.71 (dd, *J* = 7.7, 1.7 Hz, 1H), 7.59 (t, *J* = 7.6 Hz, 1H), 7.44 (dd, *J* = 7.6, 1.7 Hz, 1H), 7.39 (t, *J* = 7.8 Hz, 1H), 7.17 (d, *J* = 7.5 Hz, 1H), 6.71 (t, *J* = 54.5 Hz, 1H), 4.74 (s, 1H), 4.23 (dp, *J* = 7.0, 3.4 Hz, 1H), 3.96 (s, 3H), 3.87 (q, *J* = 14.0 Hz, 2H), 3.71 (s, 2H), 2.74 (dd, *J* = 9.7, 6.1 Hz, 2H), 2.48 (d, *J* = 3.3 Hz, 2H), 2.42 (dd, *J* = 9.6, 3.6 Hz, 1H), 2.22 (s, 3H), 2.13 (d, *J* = 8.3 Hz, 1H), 2.05 (s, 3H), 2.04 - 1.99 (m, 1H), 1.96 (d, *J* = 7.7 Hz, 2H), 1.87 (d, *J* = 7.6 Hz, 2H), 1.59 (dddd, *J* = 13.1, 8.1, 5.4, 3.1 Hz, 1H), 1.41 - 1.27 (m, 4H).

**Example 12 (*S*)-5-((((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)amino)methyl)pyrrolidin-2-one**

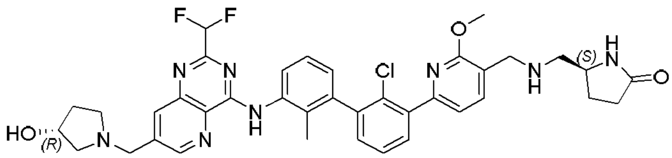

**Step 1: Synthesis of 6-(3'-amino-2-chloro-2'-methyl-[1,1'-biphenyl]-3-yl)-2-methoxynicotinaldehyde**

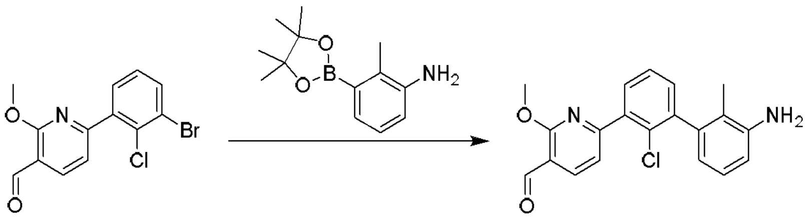

To a 50 mL microwave tube, 6-(3-bromo-2-chlorophenyl)-2-methoxypyridin-3-aldehyde (2 g, 6.12 mmol), 2-methyl-3-(tetramethyl-1,3,2-dioxaborolan-2-yl)aniline (1.71 g, 7.34 mmol), potassium carbonate (2.11 g, 15.3 mmol), Pd(dppf)$Cl_2$·DCM (0.50 g, 0.61 mmol), 1,4-dioxane (20 mL) and water (4 mL) were added in sequence. The mixture was reacted at 100 °C overnight. After the reaction was completed, the filtrate was concentrated under reduced pressure to remove the solvent, and the residue was separated and purified by silica gel column chromatography (eluent: PE/EtOAc (v/v) = 3/1) to obtain 1.95 g of a yellow solid product with a yield of 90.25%.

MS (ESI, pos. ion) m/z: 353.1 $[M+H]^+$.

**Step 2: Synthesis of 6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxynicotinaldehyde**

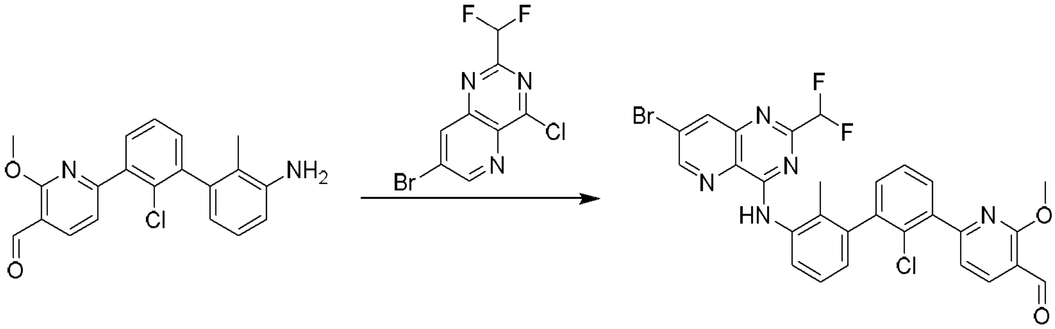

To a 100 mL single-necked bottle, 6-(3'-amino-2-chloro-2'-methyl-[1,1'-biphenyl]-3-yl)-2-methoxynicotinaldehyde (800 mg, 2.27 mmol), *tert*-butanol (50 mL) and 7-bromo-4-chloro-2-(difluoromethyl)pyrido[3,2-*d*]pyrimidine (802.16

mg, 2.72 mmol) were added in sequence and reacted at 100 °C overnight under $N_2$. After the reaction was complete, the solvent was removed by concentration under reduced pressure to obtain 1.30 g of a light yellow solid product with a yield of 93.86%.

MS (ESI, pos. ion) m/z: 609.8 [M+H]$^+$.

**Step 3: Synthesis of (*S*)-5-((((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)amino)methyl)pyrrolidin-2-one**

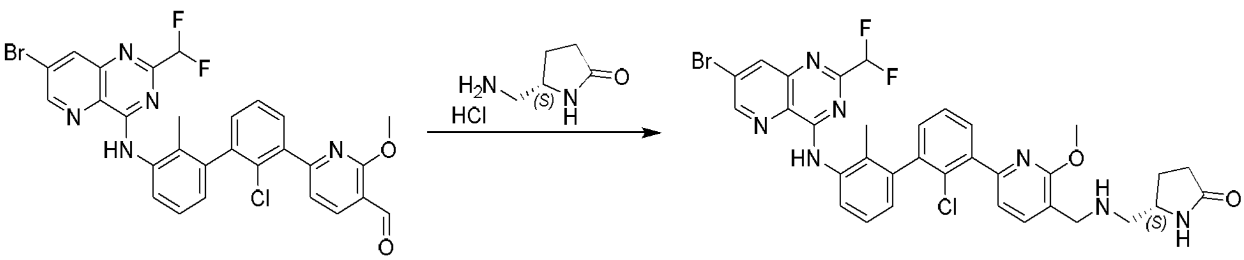

6-(3'-((7-Bromo-2-(difluoromethyl)pyrido[3,2-*d*]pyrimidin-4-yl)amino)-2-chloro-2'-methyl-[1,1'-biphenyl]-3-yl)-2-methoxynicotinaldehyde (800 mg, 1.31 mmol), (5*S*)-5-(aminomethyl)pyrrolidin-2-one hydrochloride (986.50 mg, 6.55 mmol), DCM (15 mL), methanol (15 mL), triethylamine (198.84 mg, 1.97 mmol) and acetic acid (94.40 mg, 1.57 mmol) were added to a 100 mL single-necked bottle in sequence and reacted at 50 °C for 9 h. Sodium cyanoborohydride (164.64 mg, 2.62 mmol) was added and the reaction was continued at room temperature. After the reaction was completed, the mixture was concentrated under reduced pressure to remove the solvent, and the residue was separated and purified by silica gel column chromatography (eluent: DCM/MeOH (v/v) = 30/1) to obtain 850.0 mg of a light yellow solid product with a yield of 91.54%.

MS (ESI, pos. ion) m/z: 708.6 [M+H]$^+$.

**Step 4: Synthesis of (*S*)-5-((((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)amino)methyl)pyrrolidin-2-one**

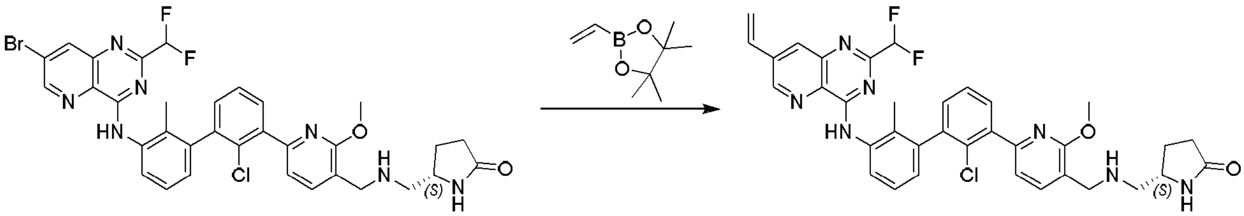

To a 50 mL single-necked bottle, (*S*)-5-((((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)amino)methyl)pyrrolidin-2-one (523 mg, 0.74 mmol), 2-vinyl-4,4,5,5-tetramethyl-1,3,2-dioxaborolane (341.90 mg, 2.22 mmol), potassium phosphate (392.70 mg, 1.85 mmol), Pd(dppf)$Cl_2$·DCM (60.43 mg, 0.074 mmol), 1,4-dioxane (15 mL) and water (3 mL) were added and reacted at 130 °C. After the reaction was completed, the reaction solution was concentrated under reduced pressure to remove the solvent, and the residue was separated and purified by silica gel column chromatography (eluent: DCM/MeOH (v/v) = 20/1) to obtain 460.0 mg of a light yellow solid product with a yield of 95.04%.

MS (ESI, pos. ion) m/z: 656.0 [M+H]$^+$.

**Step 5: Synthesis of (*S*)-4-((2'-chloro-3'-(6-methoxy-5-((((5-oxopyrrolidin-2-yl)methyl)amino)methyl)pyridin-2-yl)-2-methyl-[1,1'-biphenyl]-3-yl)amino)-2-(difluoromethyl)pyrido[3,2-*d*]pyrimidine-7-carbaldehyde**

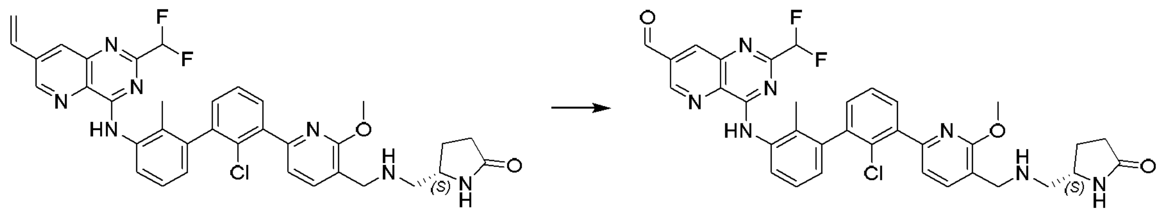

To a 250 mL single-necked bottle, (*S*)-5-((((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)amino)methyl)pyrrolidin-2-one (443.0 mg, 0.68 mmol), 1,4-dioxane (50 mL), water (20 mL), potassium osmate dihydrate (25.05 mg, 0.068 mmol) and sodium periodate (727.23 mg, 3.40 mmol) were added in sequence and the reaction was stirred at room temperature for 2.5 h. After the reaction was completed, the reaction was quenched with saturated aqueous sodium sulfite solution (5 mL) and concentrated under reduced pressure. The residue was separated and purified by silica gel column chromatography (eluent: DCM/MeOH (v/v) = 20/1) to obtain 155.0 mg of a light yellow solid product with a yield of 34.88%.

MS (ESI, pos. ion) m/z: 658.0 $[M+H]^+$.

**Step 6: Synthesis of (*S*)-5-((((6-(2-chloro-3'-((2-(difluoromethyl)-7-(((*R*)-3-hydroxypyrrolidin-l-yl)methyl)pyrido[3,2-*d*]pyrimidin-4-yl)amino)-2'-methyl-[1,1'-biphenyl]-3-yl)-2-methoxypyridin-3-yl)methyl)amino)methyl)pyrrolidin-2-one**

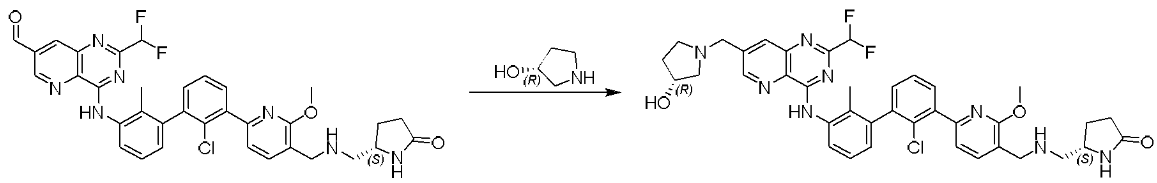

To a 50 mL single-necked bottle, (*S*)-4-((2'-chloro-3'-(6-methoxy-5-((((5-oxopyrrolidin-2-yl)methyl)amino)methyl)pyridin-2-yl)-2-methyl-[1,1'-biphenyl]-3-yl)amino)-2-(difluoromethyl)pyrido[3,2-*d*]pyrimidine-7-carbaldehyde (100 mg, 0.15 mmol), (*R*)-pyrrolidin-3-ol (65.34 mg, 0.75 mmol), DCM (7.5 mL) and 2,2,2-trifluoroethanol (7.5 mL) were added in sequence and reacted at 50 °C overnight. Sodium cyanoborohydride (18.85 mg, 0.30 mmol) was added and the reaction was continued at room temperature. After the reaction was completed, the filtrate was concentrated under reduced pressure to remove the solvent, and the residue was separated and purified by silica gel column chromatography (eluent: DCM/MeOH (v/v) = 15/1) to obtain 10.1 mg of a light yellow solid product with a yield of 9.11 %.

MS (ESI, pos. ion) m/z: 729.3 $[M+H]^+$.

$^1H$ NMR (400 MHz, DMSO-$d_6$) δ (ppm) 10.36 (s, 1H), 8.96 (s, 1H), 8.19 (s, 1H), 7.86 (d, *J* = 7.1 Hz, 1H), 7.73 (d, *J* = 7.5 Hz, 1H), 7.69 (s, 1H), 7.64 (d, *J* = 7.6 Hz, 1H), 7.54 (t, *J* = 7.4 Hz, 1H), 7.44 - 7.34 (m, 2H), 7.30 (d, *J* = 6.8 Hz, 1H), 7.16 (d, *J* = 7.9 Hz, 1H), 6.71 (t, *J* = 54.4 Hz, 1H), 4.80 (s, 1H), 4.24 (s, 1H), 3.93 (s, 3H), 3.90 - 3.50 (m, 6H), 2.85 - 2.63 (m, 4H), 2.21 - 2.08 (m, 3H), 2.04 (s, 3H), 2.01 - 1.95 (m, 1H), 1.77 - 1.42 (m, 4H).

**Example 13 (1*r*,4*r*)-4-(((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl-$d_3$)amino)cyclohexane-1-carboxylic acid**

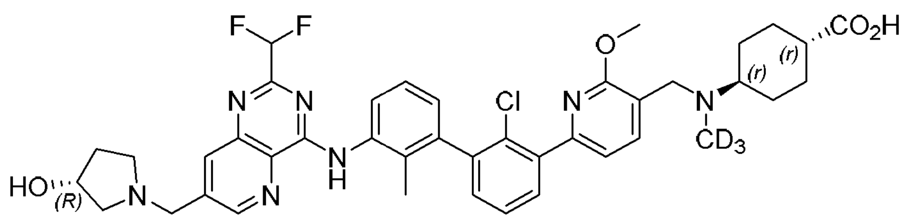

**Step 1: Synthesis of methyl (1*r*,4*r*)-4-((tert-butoxycarbonyl)amino)cyclohexane-1-carboxylate**

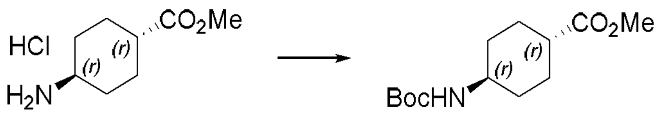

Methyl (1*r*,4*r*)-4-aminocyclohexane-1-carboxylate hydrochloride (10 g, 51.62 mmol) was dissolved in dichloromethane (300 mL). Di-*tert*-butyl dicarbonate (14.23 mL, 61.94 mmol) and triethylamine (14.35 mL, 103.24 mmol) were added in sequence, and the system was stirred at room temperature for 6.5 h. The system was stopped from stirring, and water (100 mL) was added to dilute the system. The liquids were separated, and the aqueous phase was extracted with dichloromethane (60 mL × 3). The organic phases were combined and dried over anhydrous sodium sulfate, and the filtrate was concentrated under reduced pressure to obtain 13.28 g of a white solid with a yield of 99.95%.
MS (ESI, pos. ion) m/z: 202.2 $[M+H]^+$.

**Step 2: Synthesis of methyl (1*r*,4*r*)-4-((*tert*-butoxycarbonyl)(methyl-*d*$_3$)amino)cyclohexane-1-carboxylate**

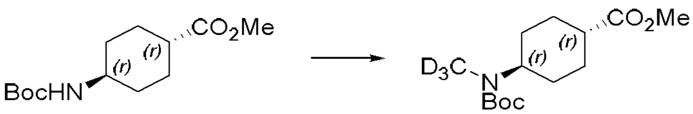

Methyl (1*r*,4*r*)-4-((*tert*-butoxycarbonyl)amino)cyclohexane-1-carboxylate (5 g, 19.43 mmol) was dissolved in DMF (30 mL), deuterated iodomethane (1.81 mL, 29.14 mmol) was added, sodium hydride (2.33 g, 58.29 mmol) was slowly added in an ice bath and stirred for 0.5 h. The mixture was stirred at room temperature for 12 h. The system was stopped stirring, saturated aqueous ammonium chloride solution (40 mL) was added to the system to quench the reaction, ethyl acetate (40 mL × 3) was added for extraction, the organic phases were combined and washed with water (30 mL) and saturated brine (30 mL) in sequence, the organic phases were collected and dried over anhydrous sodium sulfate, filtered under reduced pressure and washed with ethyl acetate (20 mL), the filtrate was concentrated under reduced pressure, and the residue was directly used in the next step.
MS (ESI, pos. ion) m/z: 219.4 $[M+H]^+$.

**Step 3: Synthesis of methyl (1*r*,4*r*)-4-((methyl-*d*$_3$)amino)cyclohexane-1-carboxylate**

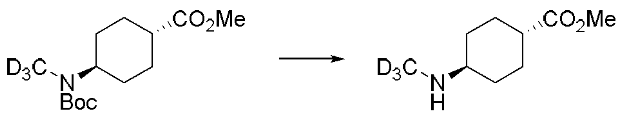

Methyl (1*r*,4*r*)-4-((*tert*-butoxycarbonyl)(methyl-*d*$_3$)amino)cyclohexane-1-carboxylate (5.3 g, 19.32 mmol) was dissolved in dichloromethane (100 mL), trifluoroacetic acid (20.09 mL, 270.48 mmol) was slowly added, and the system was stirred at room temperature for 4.5 h. The reaction was stopped and stirred, and the system was concentrated under reduced pressure. Water (20 mL) and dichloromethane (20 mL) were added to dilute the residue. Saturated potassium carbonate aqueous solution (10 mL) was added to adjust the pH of the system to 8-9. Dichloromethane (20 mL × 3) was used for extraction. The organic phases were combined and dried over anhydrous sodium sulfate, filtered under reduced pressure and washed with dichloromethane (10 mL). The filtrate was concentrated under reduced pressure, and the residue was used directly in the next step.
MS (ESI, pos. ion) m/z: 175.2 $[M+H]^+$.

**Step 4: Synthesis of methyl (1*r*,4*r*)-4-(((6-(3-bromo-2-chlorophenyl)-2-methoxypyridin-3-yl)methyl)(methyl-*d*$_3$)amino)cyclohexane-1-carboxylate**

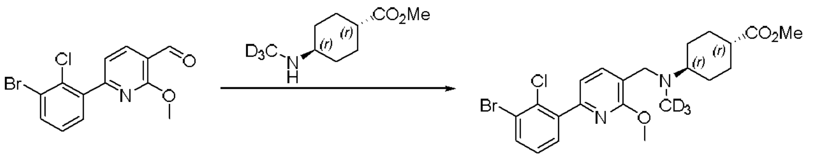

6-(3-Bromo-2-chlorophenyl)-2-methoxynicotinaldehyde (3 g, 9.19 mmol) and methyl (1*r*,4*r*)-4-((methyl-$d_3$)amino)cyclohexane-1-carboxylate (2.08 g, 11.95 mmol) were dissolved in a mixed solvent of dichloromethane (50 mL) and 2,2,2-trifluoroethanol (100 mL), acetic acid (1.05 mL, 18.38 mmol) and triethylamine (5.11 mL, 36.76 mmol) were added, and the system was stirred at 60 °C for 7 h. The system was cooled to room temperature, sodium cyanoborohydride (1.73 g, 27.57 mmol) was added, and the system was stirred at room temperature overnight. The reaction was stopped and stirred, the system was concentrated under reduced pressure, water (25 mL) and dichloromethane (25 mL) were added to dilute the residue, and saturated potassium carbonate aqueous solution (15 mL) was added to adjust the pH of the system to 8-9. Dichloromethane (20 mL × 3) was added for extraction, the organic phases were combined and dried over anhydrous sodium sulfate, filtered under reduced pressure, washed with dichloromethane (10 mL), and the filtrate was concentrated under reduced pressure. The residue was separated and purified by silica gel column chromatography (eluent: PE/EtOAc (v/v) = 4/1) to obtain 1.33 g of a light yellow solid with a yield of 30%.

MS (ESI, pos. ion) m/z: 484.4 $[M+H]^+$.

**Step 5: Synthesis of methyl (1*r*,4*r*)-4-(((6-(3'-amino-2-chloro-2'-methyl-[1,1'-biphenyl]-3-yl)-2-methoxypyridin-3-yl)methyl)(methyl-$d_3$)amino)cyclohexane-1-carboxylate**

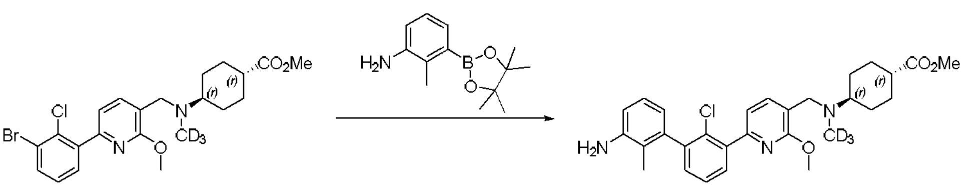

Methyl (1*r*,4*r*)-4-(((6-(3-bromo-2-chlorophenyl)-2-methoxypyridin-3-yl)methyl)(methyl-$d_3$)amino)cyclohexane-1-carboxylate (1.4 g, 2.89 mmol) and 2-methyl-3-(4,4,4,4-tetramethyl-1,3,2-dioxaborolan-2-yl)aniline (1.01 g, 4.33 mmol) were dissolved in a mixed solvent of 1,4-dioxane (50 mL) and water (10 mL). [1,1'-Bis(diphenylphosphino)ferrocene]palladium dichloride dichloromethane adduct (0.24 g, 0.29 mmol) and potassium carbonate (0.80 g, 5.78 mmol) were added. The system was stirred at 90 °C for 5.5 h under $N_2$. The reaction was stopped and stirred. Water (20 mL) was added to dilute the system, and the mixture was extracted with ethyl acetate (20 mL × 3). The organic phases were combined and dried over anhydrous sodium sulfate. The filtrate was concentrated under reduced pressure. The residue was separated and purified by silica gel column chromatography (eluent: PE/EtOAc (v/v) = 2/1) to obtain 1.25 g of a yellow solid with a yield of 85%.

MS (ESI, pos. ion) m/z: 511.5 $[M+H]^+$.

**Step 6: Synthesis of methyl (1*r*,4*r*)-4-(((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)(methyl-$d_3$)amino)cyclohexane-1-carboxylate**

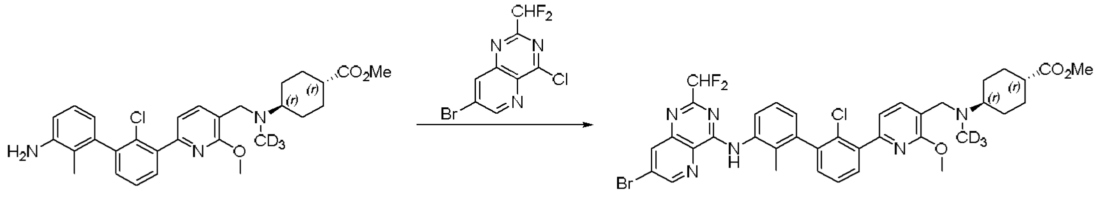

Methyl (1*r*,4*r*)-4-(((6-(3'-amino-2-chloro-2'-methyl-[1,1'-biphenyl]-3-yl)-2-methoxypyridin-3-yl)methyl)(methyl-$d_3$)amino)cyclohexane-1-carboxylate (1.4 g, 2.74 mmol) and 7-bromo-4-chloro-2-(difluoromethyl)pyrido[3,2-d]pyrimidine (1.05 g, 3.56 mmol) were dissolved in *tert*-butanol (80 mL), and hydrochloric acid (1.03 mL, 4.11 mmol) was added. The

system was stirred at 130 °C overnight under $N_2$. The reaction was stopped and stirred, and the system was concentrated under reduced pressure. Water (25 mL) and dichloromethane (25 mL) were added to dilute the system, and then saturated potassium carbonate solution (15 mL) was added. The mixture was extracted with dichloromethane (25 mL × 3). The organic phases were combined and dried over anhydrous sodium sulfate. The filtrate was concentrated under reduced pressure, and the residue was separated and purified by silica gel column chromatography (eluent: PE/EtOAc (v/v) = 2/1) to obtain 1.97 g of a yellow solid with a yield of 94%.

MS (ESI, pos. ion) m/z: 768.5 $[M+H]^+$.

**Step 7: Synthesis of methyl (1*r*,4*r*)-4-(((6-(2-chloro-3'-((2-(difluoromethyl)-7-vinylpyrido[3,2-*d*]pyrimidin-4-yl) amino)-2'-methyl-[1,1'-biphenyl]-3-yl)-2-methoxypyridin-3-yl)methyl)(methyl-*d*$_3$)amino)cyclohexane-1-carboxylate**

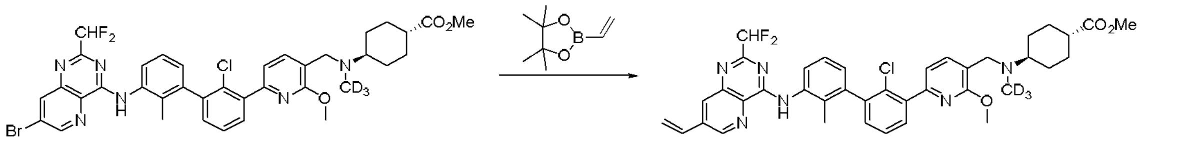

Methyl (1*r*,4*r*)-4-(((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)(methyl-*d*$_3$)amino)cyclohexane-1-carboxylate (1.97 g, 2.56 mmol) was dissolved in a mixed solvent of 1,4-dioxane (70 mL) and water (10 mL), 2-vinyl-4,4,5,5-tetramethyl-1,3,2-dioxaborolane (0.56 mL, 3.33 mmol), [1,1'-Bis(diphenylphosphino)ferrocene]palladium dichloride dichloromethane adduct (0.21 g, 0.26 mmol) and potassium phosphate (1.36 g, 6.4 mmol) were added in sequence, and the system was stirred at 100 °C overnight. The reaction was stopped and stirred. The system was cooled to room temperature and diluted with water (20 mL) and ethyl acetate (20 mL). The liquids were separated, the aqueous phase was extracted with ethyl acetate (20 mL × 3), the organic phases were combined and dried over anhydrous sodium sulfate, filtered under reduced pressure, washed with ethyl acetate (10 mL), and the filtrate was concentrated under reduced pressure. The residue was separated and purified by silica gel column chromatography (eluent: PE/EtOAc (v/v) = 1/1) to obtain 1.57 g of a yellow solid with a yield of 86%.

MS (ESI, pos. ion) m/z: 716.6 $[M+H]^+$.

**Step 8: Synthesis of methyl (1*r*,4*r*)-4-(((6-(2-chloro-3'-((2-(difluoromethyl)-7-formylpyrido[3,2-*d*]pyrimidin-4-yl) amino)-2'-methyl-[1,1'-biphenyl]-3-yl)-2-methoxypyridin-3-yl)methyl)(methyl-*d*$_3$)amino)cyclohexane-1-carboxylate**

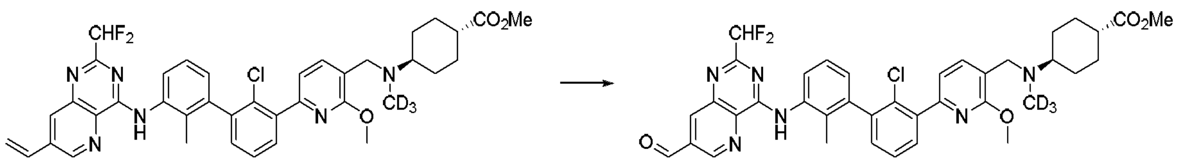

Methyl (1*r*,4*r*)-4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)(methyl-*d*$_3$)amino)cyclohexane-1-carboxylate (1.57 g, 2.19 mmol) was dissolved in 1,4-dioxane (90 mL) and water (30 mL), potassium osmate dihydrate (40.35 mg, 0.11 mmol) and sodium periodate (1.17 g, 5.47 mmol) were added in sequence, and the system was stirred at room temperature for 2 h. The reaction was stopped and the system was filtered under reduced pressure. The filtrate was separated and the aqueous phase was extracted with ethyl acetate (10 mL × 3). The organic phases were combined and dried over anhydrous sodium sulfate. The filtrate was concentrated under reduced pressure and the residue was separated and purified by silica gel column chromatography (eluent: PE/EtOAc (v/v) = 1/2) to obtain 0.64 g of a green solid with a yield of 41%.

MS (ESI, pos. ion) m/z: 718.3 $[M+H]^+$.

**Step 9: Synthesis of methyl (1*r*,4*r*)-4-(((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl-$d_3$)amino)cyclohexane-1-carboxylate**

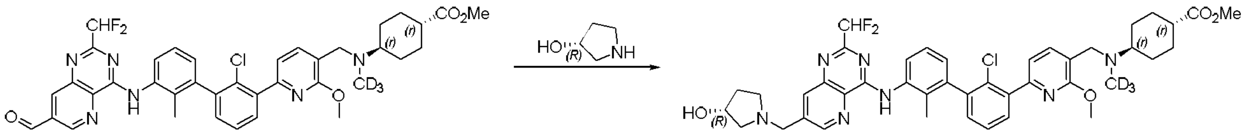

Methyl (1*r*,4*r*)-4-(((6-(2-chloro-3'-((2-(difluoromethyl)-7-formylpyrido[3,2-*d*]pyrimidin-4-yl)amino)-2'-methyl-[1,l'-biphenyl]-3-yl)-2-methoxypyridin-3-yl)methyl)(methyl-$d_3$)amino)cyclohexane-1-carboxylate (0.64 g, 0.89 mmol) and (*R*)-pyrrolidin-3-ol (0.36 mL, 4.45 mmol) were dissolved in a mixed solvent of methanol (42 mL) and dichloromethane (14 mL), acetic acid (0.15 mL, 2.67 mmol) was added, and the system was stirred at room temperature for 7 h. Sodium cyanoborohydride (0.17 g, 2.67 mmol) was added, and the system was stirred at room temperature overnight. The reaction was stopped and stirred. The system was concentrated under reduced pressure and diluted with water (15 mL) and DCM (15 mL). Saturated potassium carbonate solution (5 mL) was added to adjust the pH of the system to 9-10. The layers were separated and the aqueous phase was extracted with DCM (15 mL × 3). The organic phases were combined and dried over anhydrous sodium sulfate. The mixture was filtered under reduced pressure and washed with DCM (10 mL). The filtrate was concentrated under reduced pressure and the residue was separated and purified by silica gel column chromatography (eluent: DCM/MeOH (v/v) = 10/1) to obtain 0.52 g of orange-red oil with a yield of 74%.
MS (ESI, pos. ion) m/z: 789.4 $[M+H]^+$.

**Step 10: Synthesis of (1*r*,4*r*)-4-(((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl-$d_3$)amino)cyclohexane-1-carboxylic acid**

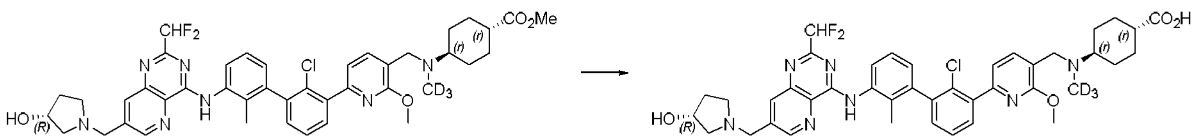

Methyl (1*r*,4*r*)-4-(((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl-$d_3$)amino)cyclohexane-1-carboxylate (0.52 g, 0.66 mmol) was dissolved in 1,4-dioxane (40 mL), and a solution of lithium hydroxide monohydrate (553.87 mg, 13.20 mmol) in water (10 mL) was added, and the system was stirred at room temperature for 9 h. The reaction was stopped and stirring was stopped. Acetic acid was added to the system to adjust the pH to 4-5. The system was concentrated under reduced pressure. The residue was separated and purified by silica gel column chromatography (eluent: DCM/MeOH (v/v) = 2/1) to obtain 125 mg of a yellow solid with a yield of 24%.
MS (ESI, pos. ion) m/z: 775.0 $[M+H]^+$.
$^1H$ NMR (400 MHz, DMSO-$d_6$) δ (ppm) 10.33 (s, 1H), 8.95 (s, 1H), 8.17 (s, 1H), 7.77 (t, *J* = 8.3 Hz, 2H), 7.64 (d, *J* = 7.8 Hz, 1H), 7.53 (t, *J* = 7.5 Hz, 1H), 7.40 - 7.35 (m, 2H), 7.27 (d, *J* = 7.3 Hz, 1H), 7.16 (d, *J* = 7.1 Hz, 1H), 6.71 (t, *J* = 54.5 Hz, 1H), 4.26 - 4.20 (m, 2H), 3.91 (s, 3H), 3.89 (s, 1H), 3.85 (s, 1H), 3.81 (s, 1H), 2.76 - 2.72 (m, 1H), 2.69 - 2.66 (m, 1H), 2.42 (d, *J* = 6.9 Hz, 2H), 2.08 (s, 1H), 2.05 (s, 3H), 2.02 - 1.98 (m, 2H), 1.93 (s, 2H), 1.83 (s, 2H), 1.63 - 1.55 (m, 2H), 1.39 - 1.28 (m, 4H).

**Example 14 (1*r*,4*r*)-4-((((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)methyl)cyclohexane-1-carboxylic acid**

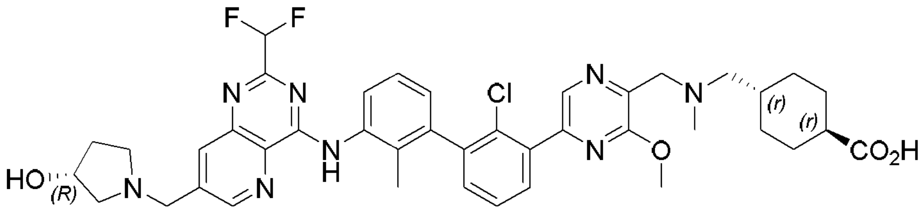

**Step 1: Synthesis of 5-(3-bromo-2-chlorophenyl)-3-methoxypyrazine-2-carbaldehyde**

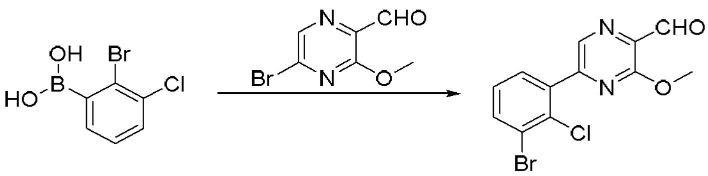

(2-Bromo-3-chlorophenyl)boronic acid (17.3 g, 73.53 mmol), 5-bromo-3-methoxypyrazine-2-carbaldehyde (19.15 g, 88.24 mmol), $Pd(PPh_3)Cl_2$ (1.55 g, 2.21 mmol), tri(o-methylphenyl)phosphine (0.67 g, 2.21 mmol), potassium carbonate (10.16 g, 73.53 mmol) were dissolved in a mixed solvent of 1,4-dioxane (200 mL) and water (50 mL), and the resulting solution was protected with nitrogen and heated to 65 °C. After the reaction was completed, a large amount of water was added to the reaction system, and a large amount of solid was precipitated. It was filtered and dried to obtain 22.01 g of a white solid product with a yield of 91.38%.

MS (ESI, pos. ion) m/z: 327.0 $[M+H]^+$.

**Step 2: Synthesis of methyl (1*r*,4*r*)-4-((((5-(3-bromo-2-chlorophenyl)-3-methoxypyrazin-2-yl)methyl)amino) methyl)cyclohexane-1-carboxylate**

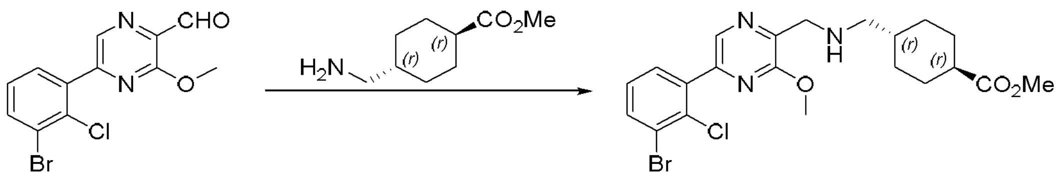

At room temperature, 5-(3-bromo-2-chlorophenyl)-3-methoxypyrazine-2-carbaldehyde (5 g, 15.26 mmol) and methyl (1*r*,4*r*)-4-(aminomethyl)cyclohexane-1-carboxylate (3.92 g, 22.89 mmol) were added to 2,2,2-trifluoroethanol (20 mL) and DCM (20 mL), and the mixture was stirred at 45 °C for 30 minutes, and then sodium cyanoborohydride (0.96 g, 15.26 mmol) was slowly added and the reaction was continued under stirring. After the reaction was completed, the reaction solution was concentrated under reduced pressure to remove the solvent, and the residue was separated and purified by silica gel column chromatography (eluent: PE/EtOAc (v/v) = 10/1) to obtain 6.5 g of a yellow oily product with a yield of 88.2%.

MS (ESI, pos. ion) m/z: 482.0 $[M+H]^+$.

**Step 3: Synthesis of methyl (1*r*,4*r*)-4-((((5-(3-bromo-2-chlorophenyl)-3-methoxypyrazin-2-yl)methyl)(methyl) amino)methyl)cyclohexane-1-carboxylate**

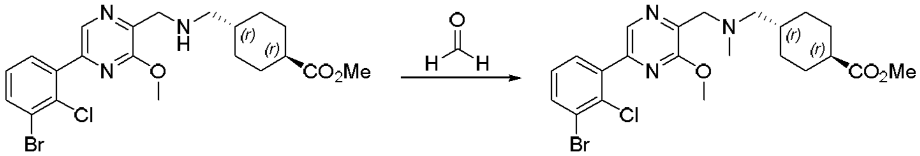

At room temperature, methyl (1*r*,4*r*)-4-((((5-(3-bromo-2-chlorophenyl)-3-methoxypyrazin-2-yl)methyl)amino) methyl)cyclohexane-1-carboxylate (12.9 g, 26.72 mmol) and formaldehyde (0.80 g, 26.72 mmol) were added to 2,2,2-trifluoroethanol (20 mL) and DCM (20 mL), and the mixture was stirred at 45 °C for 30 minutes, and then sodium cyanoborohydride (1.68 g, 26.72 mmol) was slowly added and the reaction was continued under stirring. After the reaction was completed, the reaction solution was concentrated under reduced pressure to remove the solvent, and the residue was separated and purified by silica gel column chromatography (eluent: PE/EtOAc (v/v) = 10/1) to obtain 8.2 g of a yellow oily product with a yield of 61.77%.

MS (ESI, pos. ion) m/z: 496.4 $[M+H]^+$.

**Step 4: Synthesis of methyl (1*r*,4*r*)-4-((((5-(3'-amino-2-chloro-2'-methyl-[1,1'-biphenyl]-3-yl)-3-methoxypyrazin-2-yl)methyl)(methyl)amino)methyl)cyclohexane-1-carboxylate**

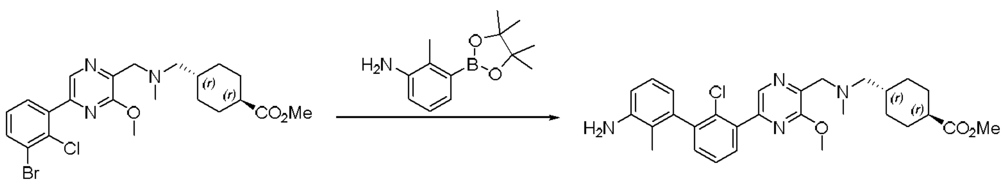

At room temperature, methyl (1*r*,4*r*)-4-((((5-(3-bromo-2-chlorophenyl)-3-methoxypyrazin-2-yl)methyl)(methyl) amino)methyl)cyclohexane-1-carboxylate (8.2 g, 16.50 mmol), 2-methyl-3-(tetramethyl-1,3,2-dioxaborolan-2-yl)aniline (7.69 g, 33 mmol), $Pd(dppf)Cl_2\cdot DCM\cdot$ ( 0.62 g, 0.83 mmol), potassium carbonate (4.56 g, 33 mmol) were added to 1,4-dioxane (100 mL) and water (25 mL), and the reaction was stirred at 90 °C. After the reaction was completed, the reaction solution was concentrated under reduced pressure to remove the solvent, and the residue was separated and purified by silica gel column chromatography (eluent: PE/EtOAc (v/v) = 3/1) to obtain 8.4 g of a yellow oily product with a yield of 97.30%.

MS (ESI, pos. ion) m/z: 523.3 $[M+H]^+$.

**Step 5: Synthesis of methyl (1*r*,4*r*)-4-((((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)methyl)cyclohexane-1-carboxylate**

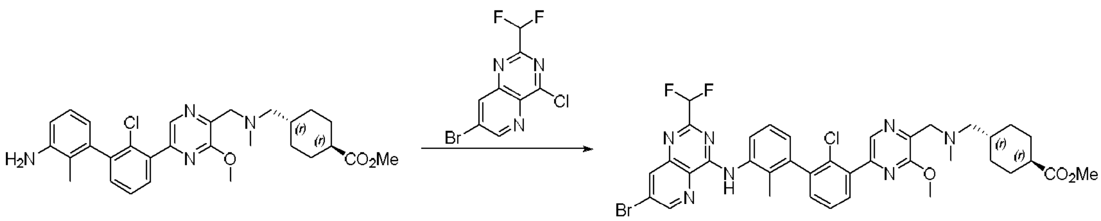

At room temperature, methyl (1*r*,4*r*)-4-((((5-(3'-amino-2-chloro-2'-methyl-[1,1'-biphenyl]-3-yl)-3-methoxypyrazin-2-yl)methyl)(methyl)amino)methyl)cyclohexane-1-carboxylate (8.3 g, 15.87 mmol) and 7-bromo-4-chloro-2-(difluoromethyl)pyrido[3,2-*d*]pyrimidine (6.08 g, 20.63 mmol) were added to tert-butanol (100 mL) and stirred at 100 °C for reaction. After the reaction was completed, the reaction solution was concentrated under reduced pressure to remove the solvent, and the residue was separated and purified by silica gel column chromatography (eluent: $CH_2Cl_2$/MeOH (v/v) = 10/1), and 11.95 g of yellow solid product was obtained with a yield of 96.42%.

MS (ESI, pos. ion) m/z: 780.5 $[M+H]^+$.

**Step 6: Synthesis of methyl (1*r*,4*r*)-4-((((5-(2-chloro-3'-((2-(difluoromethyl)-7-vinylpyrido|3,2-*d*]pyrimidin-4-yl) amino)-2'-methyl-[1,1'-biphenyl]-3-yl)-3-methoxypyrazin-2-yl)methyl)(methyl)amino)methyl)cyclohexane-1-carboxylate**

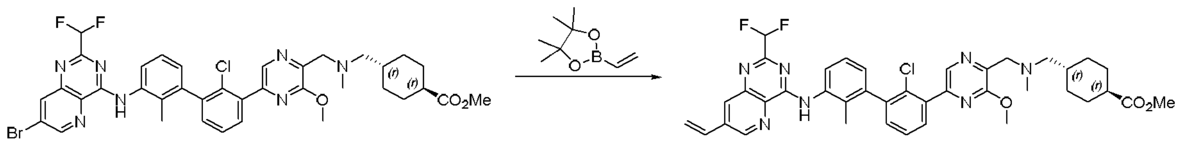

Methyl (1*r*,4*r*)-4-((((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)methyl)cyclohexane-1-carboxylate (8.1 g, 10.37 mmol), 2-vinyl-4,4,5,5-tetramethyl-1,3,2-dioxaborolane (4.79 g, 31.1 mmol), Pd(dppf)$Cl_2$·DCM· (1.27 g, 1.56 mmol) and potassium phosphate (4.40 g, 20.74 mmol) were added to 1,4-dioxane (150 mL) and water (30 mL) at room temperature. The reaction was stirred at 100 °C. After the reaction was completed, the reaction solution was concentrated under reduced pressure to remove the solvent, and the residue was separated and purified by silica gel column chromatography (eluent: $CH_2Cl_2$/MeOH (v/v) = 10/1), and 5.5 g of yellow solid product was obtained with a yield of 72.83%.

MS (ESI, pos. ion) m/z: 728.1 $[M+H]^+$.

**Step 7: Synthesis of methyl (1*r*,4*r*)-4-((((5-(2-chloro-3'-((2-(difluoromethyl)-7-formylpyrido[3,2-*d*]pyrimidin-4-yl) amino)-2'-methyl-[1,1'-biphenyl]-3-yl)-3-methoxypyrazin-2-yl)methyl)(methyl)amino)methyl)cyclohexane-1-carboxylate**

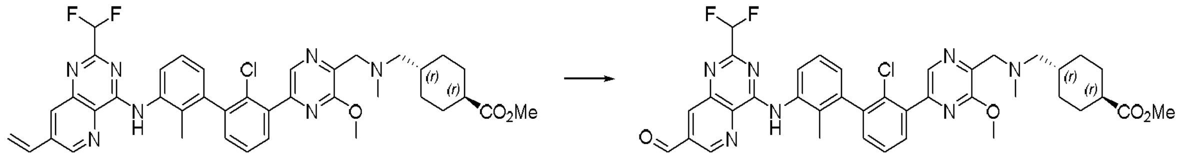

At room temperature, methyl (1*r*,4*r*)-4-((((5-(2-chloro-3'-((2-(difluoromethyl)-7-vinylpyrido[3,2-*d*]pyrimidin-4-yl) amino)-2'-methyl-[1,1'-biphenyl]-3-yl)-3-methoxypyrazin-2-yl)methyl)(methyl)amino)methyl)cyclohexane-1-carboxylate (3.0 g, 4.12 mmol) was added to 1,4-dioxane (200 mL) and water (75 mL), and then potassium osmate dihydrate (0.15 g, 0.41 mmol) and sodium periodate (4.41 g, 20.6 mmol) were slowly added and the reaction was continued to stir. After the reaction was completed, the reaction solution was concentrated under reduced pressure to remove the solvent, and the residue was separated and purified by silica gel column chromatography (eluent: $CH_2Cl_2$/MeOH (v/v) = 20/1), and 1.80 g of yellow oily product was obtained with a yield of 59.84%.

MS (ESI, pos. ion) m/z: 729.9 $[M+H]^+$.

**Step 8: Synthesis of methyl (1*r*,4*r*)-4-((((5-(2-chloro-3'-((2-(difluoromethyl)-7-(((*R*)-3-hydroxypyrrolidin-1-yl) methyl)pyrido[3,2-*d*]pyrimidin-4-yl)amino)-2'-methyl-[1,1'-biphenyl]-3-yl)-3-methoxypyrazin-2-yl)methyl) (methyl)amino)methyl)cyclohexane-1-carboxylate**

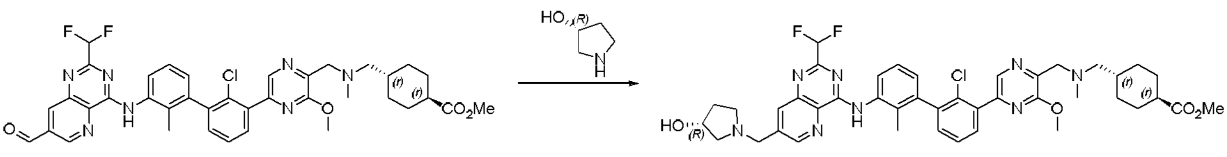

At room temperature, methyl (1*r*,4*r*)-4-((((5-(2-chloro-3'-((2-(difluoromethyl)-7-formylpyrido[3,2-*d*]pyrimidin-4-yl)amino)-2'-methyl-[1,1'-biphenyl]-3-yl)-3-methoxypyrazin-2-yl)methyl)(methyl)amino)methyl)cyclohexane-1-carboxylate (2.9 g, 3.97 mmol) and (3R)-pyrrolidin-3-ol (1.04 g, 11.91 mmol) were added to dichloromethane (20 mL) and 2,2,2-trifluoroethanol (20 mL), and the reaction was stirred at 45 °C. After the reaction was completed, the reaction solution was concentrated under reduced pressure to remove the solvent, and the residue was separated and purified by silica gel column chromatography (eluent: $CH_2Cl_2$/MeOH (v/v) = 20/1), and 2.50 g of yellow oily product was obtained with a yield of 78.56%.

MS (ESI, pos. ion) m/z: 801.8 $[M+H]^+$.

**Step 9: Synthesis of (1*r*,4*r*)-4-((((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)methyl)cyclohexane-1-carboxylic acid**

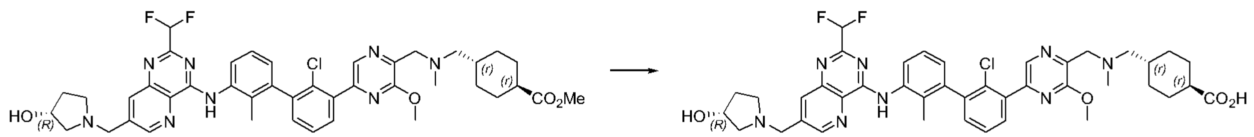

At room temperature, methyl (1*r*,4*r*)-4-((((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)methyl)cyclohexane-1-carboxylate (2.49 g, 3.11 mmol) was added to 1,4-dioxane (30 mL) and water (5 mL), and then lithium hydroxide monohydrate (0.65 g, 15.55 mmol) was slowly added, and the reaction was stirred at 50 °C. After the reaction was completed, the reaction solution was concentrated under reduced pressure to remove the solvent, and the residue was separated and purified by silica gel column chromatography (eluent: $CH_2Cl_2$/MeOH (v/v) = 10/1), and 1.0 g of white solid product was obtained with a yield of 40.88%.

MS (ESI, pos. ion) m/z: 787.3 $[M+H]^+$.

$^1$H NMR (400 MHz, DMSO-$d_6$) δ 10.31 (s, 1H), 8.93 (d, *J* = 1.9 Hz, 1H), 8.42 (s, 1H), 8.15 (d, *J* = 1.9 Hz, 1H), 7.77 (d, *J* = 8.0 Hz, 1H), 7.68 (dd, *J* = 7.7, 1.7 Hz, 1H), 7.56 (td, *J* = 7.5, 1.5 Hz, 1H), 7.42 (dd, *J* = 7.6, 1.7 Hz, 1H), 7.37 (t, *J* = 7.8 Hz, 1H), 7.18 - 7.11 (m, 1H), 6.69 (t, *J* = 54.6 Hz, 1H), 4.21 (qd, *J* = 6.7, 3.3 Hz, 1H), 3.94 (s, 3H), 3.85 (q, *J* = 14.0 Hz, 2H), 3.59 (s, 2H), 3.16 (s, 3H), 2.78 - 2.62 (m, 2H), 2.46 (d, *J* = 8.3 Hz, 1H), 2.41 (dd, *J* = 9.7, 3.5 Hz, 1H), 2.21 (d, *J* = 7.0 Hz, 2H), 2.17 (s, 3H), 2.04 (s, 3H), 1.80 (d, *J* = 19.3 Hz, 3H), 1.59 (ddt, *J* = 12.5, 8.8, 3.5 Hz, 1H), 1.45 (s, 1H), 1.33 - 1.19 (m, 2H), 0.76 (q, *J* = 12.8, 12.4 Hz, 2H).

**Example 15 (*R*)-1-((5-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)piperidine-4-carboxylic acid**

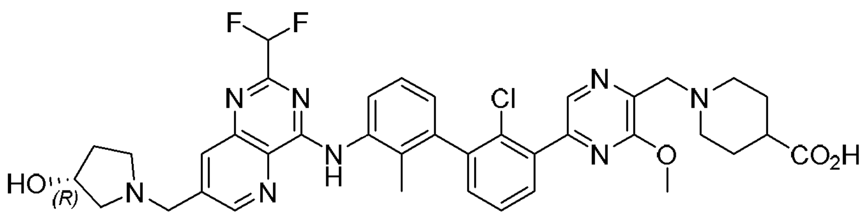

**Step 1: Synthesis of methyl 1-((5-(3-bromo-2-chlorophenyl)-3-methoxypyrazin-2-yl)methyl)piperidine-4-carboxylate**

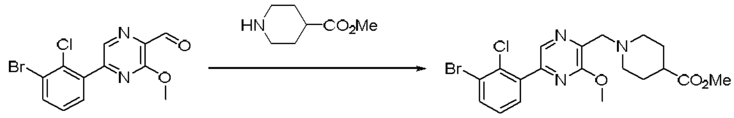

5-(3-Bromo-2-chlorophenyl)-3-methoxypyrazine-2-carbaldehyde (5 g, 15.26 mmol) and methyl piperidine-4-carboxylate (6.55 g, 45.78 mmol) were dissolved in a mixed solvent of dichloromethane (40 mL) and methanol (120 mL), acetic acid (1.75 mL, 30.52 mmol) was added, and the mixture was stirred at room temperature for 8 h. Sodium cyanoborohydride (2.88 g, 45.78 mmol) was added, and the system was stirred at room temperature overnight. After the reaction was complete, the mixture was concentrated under reduced pressure, and the residue was separated and purified by silica gel column chromatography (eluent: PE/EtOAc (v/v) = 4/1) to obtain 6.9 g of orange-yellow oil with a yield of 99%.

MS (ESI, pos. ion) m/z: 454.1 $[M+H]^+$.

**Step 2: Synthesis of methyl 1-((5-(3'-amino-2-chloro-2'-methyl-[1,1'-biphenyl]-3-yl)-3-methoxypyrazin-2-yl) methyl)piperidine-4-carboxylate**

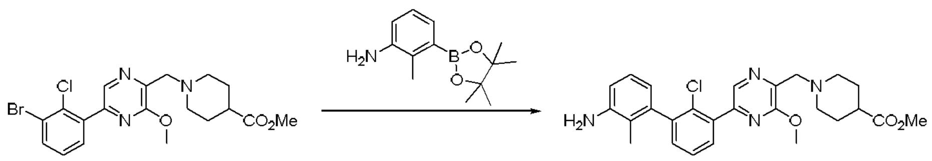

Methyl 1-((5-(3-bromo-2-chlorophenyl)-3-methoxypyrazin-2-yl)methyl)piperidine-4-carboxylate (5.3 g, 11.65 mmol) and 2-methyl-3-(4,4,5,5-tetramethyl-1,3,2-dioxaborolan-2-yl)aniline (4.07 g, 17.48 mmol) were dissolved in a mixed solvent of 1,4-dioxane (150 mL) and water (30 mL). [1,1'-Bis(diphenylphosphino)ferrocene]palladium dichloride dichloromethane adduct (0.95 g, 1.17 mmol) and potassium carbonate (3.22 g, 23.3 mmol) were added. The system was stirred at 90 °C for 10.5 h under $N_2$. After the reaction was complete, the reaction solution was concentrated under reduced pressure, and the residue was separated and purified by silica gel column chromatography (eluent: PE/EtOAc (v/v) = 1/1) to obtain 4.5 g of red oil with a yield of 80%.

MS (ESI, pos. ion) m/z: 481.5 $[M+H]^+$.

**Step 3: Synthesis of methyl 1-((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)piperidine-4-carboxylate**

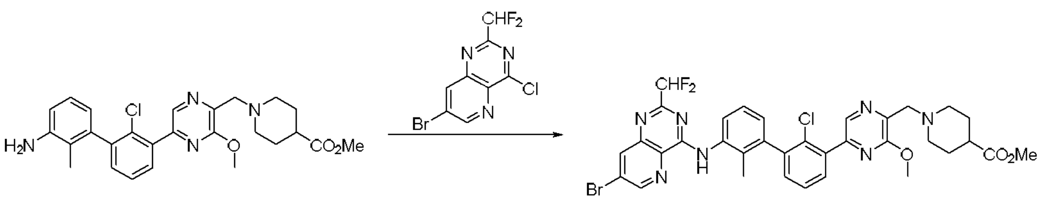

Methyl 1-((5-(3'-amino-2-chloro-2'-methyl-[1,1'-biphenyl]-3-yl)-3-methoxypyrazin-2-yl)methyl)piperidine-4-carboxylate (4.5 g, 9.36 mmol) and 7-bromo-4-chloro-2-(difluoromethyl)pyrido[3,2-d]pyrimidine (3.58 g, 12.17 mmol) were dissolved in tert-butanol (160 mL), and a solution of hydrochloric acid in 1,4-dioxane (3.51 mL, 14.04 mmol) was added. The system was stirred at 130 °C overnight under $N_2$. After the reaction was completed, the filtrate was concentrated under reduced pressure, and the residue was separated and purified by silica gel column chromatography (eluent: PE/EtOAc (v/v) = 1/2) to obtain 6 g of yellow solid with a yield of 87%.

MS (ESI, pos. ion) m/z: 738.0 $[M+H]^+$.

**Step 4: Synthesis of methyl 1-((5-(2-chloro-3'-((2-(difluoromethyl)-7-vinylpyrido[3,2-*d*]pyrimidin-4-yl)amino)-2'-methyl-[1,1'-biphenyl]-3-yl)-3-methoxypyrazin-2-yl)methyl)piperidine-4-carboxylate**

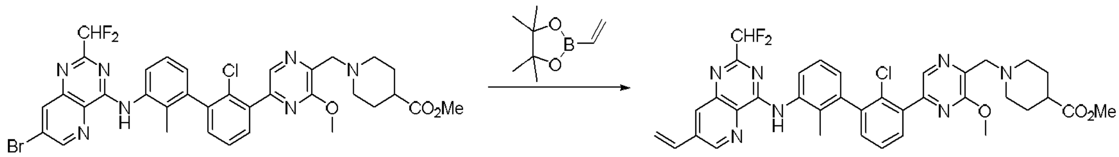

Methyl 1-((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)piperidine-4-carboxylate (6 g, 8.12 mmol) was dissolved in a mixed solvent of 1,4-dioxane (300 mL) and water (30 mL), 2-vinyl-4,4,5,5-tetramethyl-1,3,2-dioxaborolane (1.79 mL, 10.56 mmol), [1,1"-Bis(diphenylphosphino)ferrocene]palladium dichloride dichloromethane adduct (0.66 g, 0.81 mmol) and potassium phosphate (4.31 g, 20.30 mmol) were added in sequence, and the system was stirred at 100 °C overnight. After the reaction was completed, the filtrate was concentrated under reduced pressure, and the residue was separated and purified by silica gel column chromatography (eluent: PE/EtOAc (v/v) = 1/2) to obtain 4.4 g of yellow solid with a yield of

81%.

MS (ESI, pos. ion) m/z: 686.6 $[M+H]^+$.

**Step 5: Synthesis of methyl 1-((5-(2-chloro-3'-((2-(difluoromethyl)-7-formylpyrido[3,2-*d*]pyrimidin-4-yl)amino)-2'-methyl-[1,1'-biphenyl]-3-yl)-3-methoxypyrazin-2-yl)methyl)piperidine-4-carboxylate**

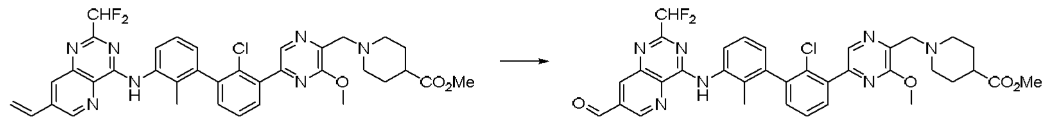

Methyl 1-((5-(2-chloro-3'-((2-(difluoromethyl)-7-vinylpyrido[3,2-*d*]pyrimidin-4-yl)amino)-2'-methyl-[1,1'-biphenyl]-3-yl)-3-methoxypyrazin-2-yl)methyl)piperidine-4-carboxylate (4.4 g, 6.41 mmol) was dissolved in 1,4-dioxane (270 mL) and water (90 mL), potassium osmate dihydrate (118.09 mg, 0.32 mmol) and sodium periodate (3.43 g, 16.02 mmol) were added in sequence, and the system was stirred at room temperature for 7.5 h. After the reaction was completed, the filtrate was concentrated under reduced pressure, and the residue was separated and purified by silica gel column chromatography (eluent: PE/EtOAc (v/v) = 1/4) to obtain 1.63 g of brown-yellow solid with a yield of 37%.

MS (ESI, pos. ion) m/z: 688.9 $[M+H]^+$.

**Step 6: Synthesis of methyl (*R*)-1-((5-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)piperidine-4-carboxylate**

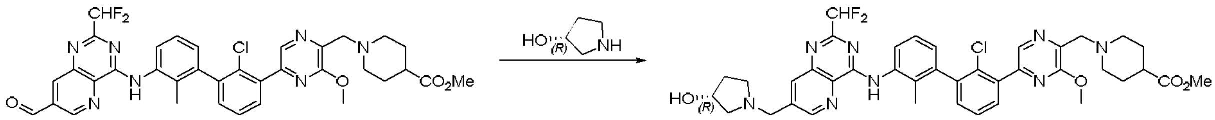

Methyl 1-((5-(2-chloro-3'-((2-(difluoromethyl)-7-formylpyrido[3,2-*d*]pyrimidin-4-yl)amino)-2'-methyl-[1,1'-biphenyl]-3-yl)-3-methoxypyrazin-2-yl)methyl)piperidine-4-carboxylate (1.6 g, 2.33 mmol) and (3*R*)-pyrrolidin-3-ol (0.94 mL, 11.65 mmol) were dissolved in a mixed solvent of methanol (90 mL) and dichloromethane (30 mL), acetic acid (0.40 mL, 6.99 mmol) was added, and the system was stirred at room temperature for 6 h. Sodium cyanoborohydride (0.44 g, 6.99 mmol) was added, and the system was stirred at room temperature overnight. After the reaction was complete, the filtrate was concentrated under reduced pressure, and the residue was separated and purified by silica gel column chromatography (eluent: DCM/MeOH (v/v) = 15/1) to obtain 0.83 g of yellow oil with a yield of 47%.

MS (ESI, pos. ion) m/z: 759.3 $[M+H]^+$.

**Step 7: Synthesis of (*R*)-1-((5-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)piperidine-4-carboxylic acid**

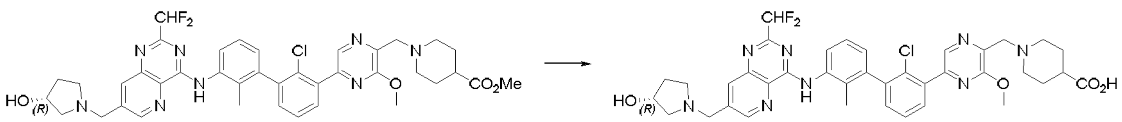

Methyl (*R*)-1-((5-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)piperidine-4-carboxylate (0.83 g, 1.09 mmol) was dissolved in 1,4-dioxane (60 mL), and a solution of lithium hydroxide monohydrate (914.73 mg, 21.8 mmol) in water (15 mL) was added, and the system was stirred overnight at room temperature. The reaction was stopped and stirring was stopped. Acetic acid was added to the system to adjust the pH to 4-5. The system was concentrated under reduced pressure. The residue was separated and purified by silica gel column chromatography (eluent: DCM/MeOH (v/v) = 10/1)

to obtain 337.2 mg of a yellow solid with a yield of 41%.

MS (ESI, pos. ion) m/z: 745.3 $[M+H]^+$.

$^1H$ NMR (400 MHz, DMSO-$d_6$) δ 10.35 (s, 1H), 8.95 (s, 1H), 8.46 (s, 1H), 8.17 (s, 1H), 7.76 (d, *J* = 7.9 Hz, 1H), 7.71 (d, *J* = 7.3 Hz, 1H), 7.58 (t, *J* = 7.5 Hz, 1H), 7.44 (d, *J* = 7.1 Hz, 1H), 7.39 (t, *J* = 7.7 Hz, 1H), 7.17 (d, *J* = 7.5 Hz, 1H), 6.71 (t, *J* = 54.5 Hz, 1H), 4.25 - 4.21 (m, 1H), 3.97 (s, 3H), 3.90 (d, *J* = 13.9 Hz, 1H), 3.83 (d, *J* = 13.9 Hz, 1H), 3.62 (s, 4H), 2.86 (d, *J* = 10.7 Hz, 2H), 2.74 (dd, *J* = *9.4,* 6.1 Hz, 1H), 2.68 (q, *J* = 7.7 Hz, 1H), 2.49 - 2.47 (m, 1H), 2.41 (dd, *J* = 9.4, 2.9 Hz, 1H), 2.13 (t, *J* = 10.8 Hz, 2H), 2.04 (s, 3H), 2.01 (dd, *J* = 14.9, 7.5 Hz, 1H), 1.74 (d, *J* = 11.4 Hz, 2H), 1.61 - 1.51 (m, 1H), 1.51 (dt, *J* = 12.5, 6.3 Hz, 2H).

**Example 16 (1*r*,4*r*)-4-(((6-(2,2'-dichloro-3'-((2-(difluoromethyl)-7-(((*R*)-3-hydroxypyrrolidin-1-yl)methyl)pyrido [3,2-*d*]pyrimidin-4-yl)amino)-[1,1'-biphenyl]-3-yl)-2-methoxypyridin-3-yl)methyl)(methyl)amino)cyclohexane-1-carboxylic acid**

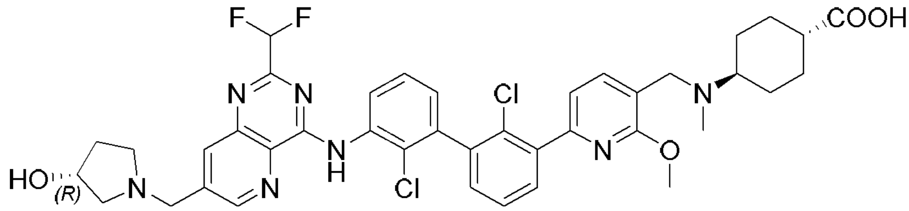

**Step 1: Synthesis of methyl (1*r*,4*r*)-4-(((6-(3-bromo-2-chlorophenyl)-2-methoxypyridin-3-yl)methyl)amino)cyclohexane-1-carboxylate**

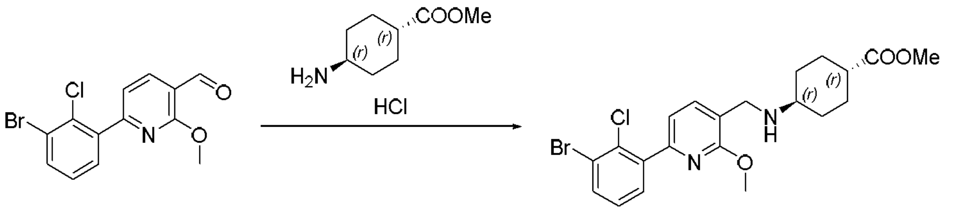


6-(3-Bromo-2-chlorophenyl)-2-methoxynicotinaldehyde (1.1 g, 3.37 mmol) and methyl (1*r*,4*r*)-4-aminocyclohexane-1-carboxylate (0.98 g, 5.05 mmol) were dissolved in 2,2,2-trifluoroethanol (15 mL) and dichloromethane (15 mL). The mixture was stirred at 50 °C for 16 h. Sodium cyanoborohydride (317.66 mg, 5.05 mmol) was added to the system and the mixture was stirred at room temperature for 3 h. After the reaction was completed, the system was concentrated and extracted with ethyl acetate (10 mL × 2). The organic phases were combined and concentrated under reduced pressure. The mixture was separated and purified by silica gel column chromatography (eluent: DCM/MeOH (v/v) = 15/1) to obtain 1.57 g of a light yellow viscous product with a yield of 99.64%.

MS (ESI, pos. ion) m/z: 467.1 $[M+H]^+$.

**Step 2: Synthesis of methyl (1*r*,4*r*)-4-(((6-(3-bromo-2-chlorophenyl)-2-methoxypyridin-3-yl)methyl)(methyl)amino)cyclohexane-1-carboxylate**

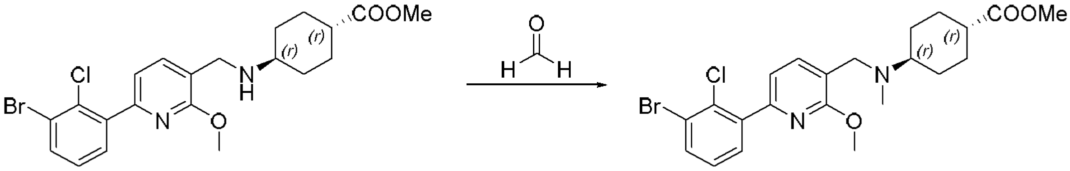

Methyl (1*r*,4*r*)-4-(((6-(3-bromo-2-chlorophenyl)-2-methoxypyridin-3-yl)methyl)amino)cyclohexane-1-carboxylate (0.44 g, 0.94 mmol) and formaldehyde (0.31 g, 3.76 mmol) were dissolved in methanol (40 mL), and then acetic

acid (70.56 mg, 1.17 mmol) was added and stirred at room temperature for 18 h. Sodium cyanoborohydride (295.35 mg, 4.70 mmol) was slowly added and stirred at room temperature for 2.5 h. After the reaction was complete, saturated sodium carbonate solution (10 ml) was added to quench the reaction, extracted with ethyl acetate (10 mL × 3), and the organic phases were combined and dried over anhydrous sodium sulfate. Filtered, the filtrate was concentrated, and the reduced pressure concentration was carried out and purified by silica gel column chromatography (eluent: dichloromethane/-methanol (v/v) = 40/1) to obtain 0.45 g of a light yellow viscous product with a yield of 99.29%.

MS (ESI, pos. ion) m/z: 482.9 $[M+H]^+$.

**Step 3: Synthesis of methyl (1*r*,4*r*)-4-(((6-(3'-amino-2,2'-dichloro-[1,1'-biphenyl]-3-yl)-2-methoxypyridin-3-yl)methyl)(methyl)amino)cyclohexane-1-carboxylate**

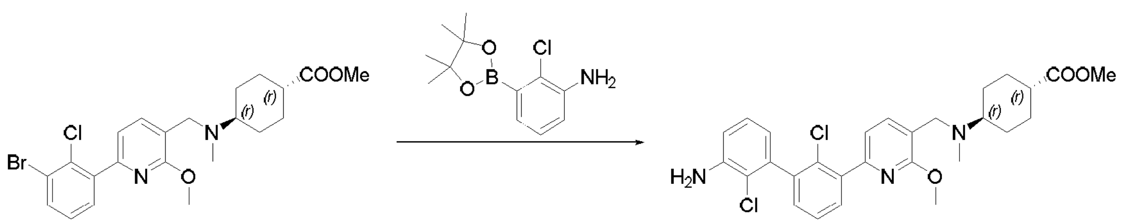

Methyl (1*r*,4*r*)-4-(((6-(3-bromo-2-chlorophenyl)-2-methoxypyridin-3-yl)methyl)(methyl)amino)cyclohexane-1-carboxylate (0.66 g, 1.37 mmol) and 2-chloro-3-(tetramethyl-1,3,2-dioxaborolan-2-yl)aniline (0.69 g, 2.74 mmol) were dissolved in a mixed solvent of water (4 mL) and 1,4-dioxane (20 mL). Sodium carbonate (0.44 g, 4.11 mmol) and [1,1'-bis(dicyclohexylphosphino)ferrocene]dichloropalladium(II) (0.10 g, 0.14 mmol) were added in sequence. The mixture was heated to 90 °C for 6 h under $N_2$. After the reaction was completed, the product was filtered and the filtrate was concentrated. The residue was separated and purified by silica gel column chromatography (eluent: dichloromethane/-methanol (v/v) = 20/1) to obtain 0.72 g of a brown-black viscous product with a yield of 99.46%.

MS (ESI, pos. ion) m/z: 528.5 $[M+H]^+$.

**Step 4: Synthesis of methyl (1*r*,4*r*)-4-(((6-(3'-((7-bromo-2-(difluoromethyl)pyrido[3,2-*d*]pyrimidin-4-yl)amino)-2,2'-dichloro-[1,1'-biphenyl]-3-yl)-2-methoxypyridin-3-yl)methyl)(methyl)amino)cyclohexane-1-carboxylate**

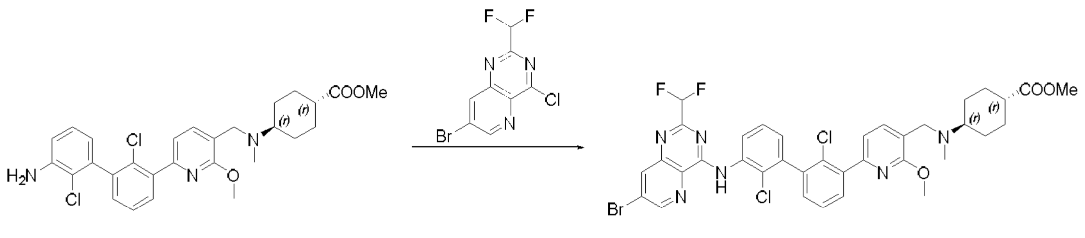

7-Bromo-4-chloro-2-(difluoromethyl)pyrido[3,2-*d*]pyrimidine (500 mg, 1.70 mmol) and methyl (1*r*,4*r*)-4-(((6-(3'-amino-2,2'-dichloro-[1,1'-biphenyl]-3-yl)-2-methoxypyridin-3-yl)methyl)(methyl)amino)cyclohexane-1-carboxylate (0.72 g, 1.36 mmol) were dissolved in *tert*-butanol (50 mL) and stirred at 130 °C for 3 h under $N_2$. After the reaction was complete, the mixture was concentrated under reduced pressure, and the residue was separated and purified by silica gel column chromatography (eluent: DCM/MeOH (v/v) = 20/1) to obtain 0.5 g of brown yellow powder with a yield of 46.66%.

MS (ESI, pos. ion) m/z: 785.5 $[M+H]^+$.

**Step 5: Synthesis of methyl (1*r*,4*r*)-4-(((6-(2,2'-dichloro-3'-((2-(difluoromethyl)-7-vinylpyrido[3,2-*d*]pyrimidin-4-yl)amino)-[1,1'-biphenyl]-3-yl)-2-methoxypyridin-3-yl)methyl)(methyl)amino)cyclohexane-1-carboxylate**

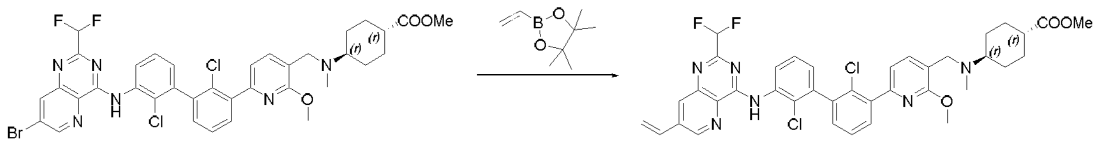

Methyl (1*r*,4*r*)-4-(((6-(3'-((7-bromo-2-(difluoromethyl)pyrido[3,2-*d*]pyrimidin-4-yl)amino)-2,2'-dichloro-[1,1'-biphenyl]-3-yl)-2-methoxypyridin-3-yl)methyl)(methyl)amino)cyclohexane-1-carboxylate (0.315 g, 0.40 mmol) was dissolved in a mixed solvent of 1,4-dioxane (30 mL) and water (6 mL), 2-vinyl-4,4,5,5-tetramethyl-1,3,2-dioxaborolane (616.04 mg, 4 mmol), [1,1'-Bis(diphenylphosphino)ferrocene]palladium dichloride dichloromethane adduct (32.67 mg, 0.040 mmol) and potassium phosphate (169.82 mg, 0.80 mmol) were added in sequence, and the system was stirred at 100 °C for 4 h under $N_2$. After the reaction was completed, the product was filtered and concentrated, and the residue was purified by column chromatography (eluent: DCM/MeOH (v/v) = 20/1) to obtain 0.29 g of a yellow solid with a yield of 98.70%.

MS (ESI, pos. ion) m/z: 733.2 $[M+H]^+$.

**Step 6: Synthesis of methyl (1*r*,4*r*)-4-(((6-(2,2'-dichloro-3'-((2-(difluoromethyl)-7-formylpyrido[3,2-*d*]pyrimidin-4-yl)amino)-[1,1'-biphenyl]-3-yl)-2-methoxypyridin-3-yl)methyl)(methyl)amino)cyclohexane-1-carboxylate**

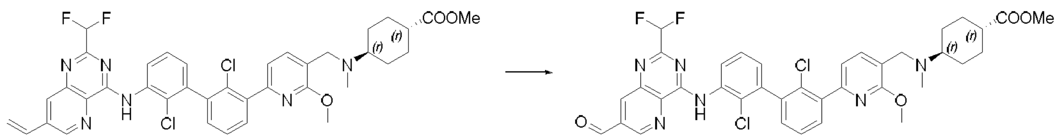

Methyl (1*r*,4*r*)-4-(((6-(2,2'-dichloro-3'-((2-(difluoromethyl)-7-vinylpyrido[3,2-*d*]pyrimidin-4-yl)amino)-[1,1'-biphenyl]-3-yl)-2-methoxypyridin-3-yl)methyl)(methyl)amino)cyclohexane-1-carboxylate (0.29 g, 0.40 mmol) was dissolved in 1,4-dioxane (18 mL) and water (6 mL), potassium osmate dihydrate (7.37 mg, 0.020 mmol) and sodium periodate (0.21 g, 1 mmol) were added in sequence, and the system was stirred at room temperature for 2 h. After the reaction was completed, the mixture was filtered and separated. The aqueous phase was extracted with ethyl acetate (20 mL × 3). The organic phases were combined and dried over anhydrous sodium sulfate. The filtrate was concentrated under reduced pressure. The residue was separated and purified by silica gel column chromatography (eluent: DCM/MeOH (v/v) = 20/1) to obtain 0.188 g of a brown-yellow viscous product with a yield of 64.65%.

MS (ESI, pos. ion) m/z: 735.5 $[M+H]^+$.

**Step 7: Synthesis of methyl (1*r*,4*r*)-4-(((6-(2,2'-dichloro-3'-((2-(difluoromethyl)-7-(((*R*)-3-hydroxypyrrolidin-1-yl)methyl)pyrido[3,2-*d*]pyrimidin-4-yl)amino)-[1,1'-biphenyl]-3-yl)-2-methoxypyridin-3-yl)methyl)(methyl)amino)cyclohexane-1-carboxylate**

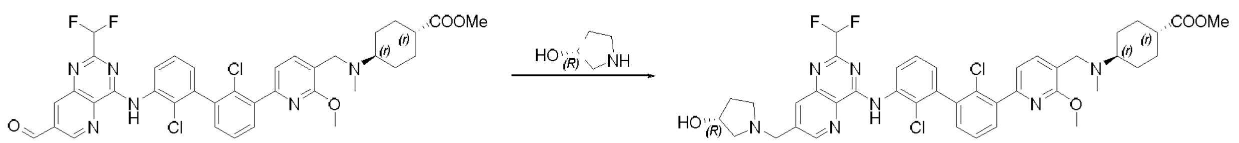

Methyl (1*r*,4*r*)-4-(((6-(2,2'-dichloro-3'-((2-(difluoromethyl)-7-formylpyrido[3,2-*d*]pyrimidin-4-yl)amino)-[1,1'-biphenyl]-3-yl)-2-methoxypyridin-3-yl)methyl)(methyl)amino)cyclohexane-1-carboxylate (0.188 g, 0.26 mmol) and (3R)-pyrrolidin-3-ol (113.26 mg, 1.3 mmol) were dissolved in methanol (24 mL) and DCM (7 mL), the reaction was stirred at room temperature for 21 h. Sodium cyanoborohydride (49.02 mg, 0.78 mmol) was added and the reaction was continued at room temperature for 3 h. After the reaction was complete, the system was concentrated and the residue was separated and purified by silica gel column chromatography (eluent: DCM/MeOH (v/v) = 12/1) to obtain 0.14 g of brown yellow powder with a yield of 67.90%.

MS (ESI, pos. ion) m/z: 806.0 $[M+H]^+$.

**Step 8: Synthesis of (1*r*,4*r*)-4-(((6-(2,2'-dichloro-3'-((2-(difluoromethyl)-7-(((*R*)-3-hydroxypyrrolidin-1-yl)methyl) pyrido[3,2-*d*]pyrimidin-4-yl)amino)-[1,1'-biphenyl]-3-yl)-2-methoxypyridin-3-yl)methyl)(methyl)amino)cyclohexane-1-carboxylic acid**

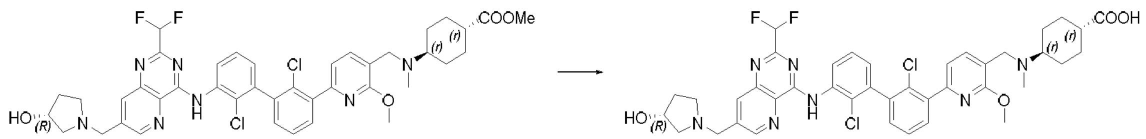

Methyl (1*r*,4*r*)-4-(((6-(2,2'-dichloro-3'-((2-(difluoromethyl)-7-(((*R*)-3-hydroxypyrrolidin-1-yl)methyl)pyrido[3,2-*d*] pyrimidin-4-yl)amino)-[1,1'-biphenyl]-3-yl)-2-methoxypyridin-3-yl)methyl)(methyl)amino)cyclohexane-1-carboxylate (0.14 g, 0.17 mmol) was dissolved in a mixed solution of 1,4-dioxane (15 mL) and water (3 mL), lithium hydroxide monohydrate (85.60 mg, 2.04 mmol) was added, and the reaction was stirred at room temperature for 15 h. After the reaction was completed, water (5 mL) was added for dilution, dilute hydrochloric acid (5 mL) was used to adjust the pH to 5, and the filtrate was concentrated. The residue was separated and purified by silica gel column chromatography (eluent: dichloromethane/methanol (v/v) = 8/1) to obtain 60 mg of light orange powder with a yield of 43.62%.

MS (ESI, pos. ion) m/z: 791.9 $[M+H]^+$.

$^1H$ NMR (400 MHz, DMSO-$d_6$) δ 8.98 (s, 1H), 8.56 (d, *J* = 8.3 Hz, 1H), 8.23 (s, 1H), 7.77 (d, *J* = 7.7 Hz, 1H), 7.68 (d, *J* = 7.7 Hz, 1H), 7.56 (dt, *J* = 14.1, 8.1 Hz, 3H), 7.41 (d, *J* = 7.5 Hz, 1H), 7.27 (d, *J* = 7.6 Hz, 3H), 4.23 - 4.19 (m, 1H), 3.89 (s, 3H), 3.86 - 3.79 (m, 4H), 3.01 - 2.90 (m, 1H), 2.72 (d, *J* = 7.6 Hz, 2H), 2.67 (d, *J* = 5.3 Hz, 2H), 2.37 - 2.28 (m, 1H), 2.16 (s, 3H), 2.06 - 1.98 (m, 3H), 1.91 (s, 3H), 1.86 - 1.75 (m, 4H), 1.61 - 1.55 (m, 1H).

**Example 17 (1*r*,4*r*)-4-((((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)methyl) cyclohexane-1-carboxylic acid**

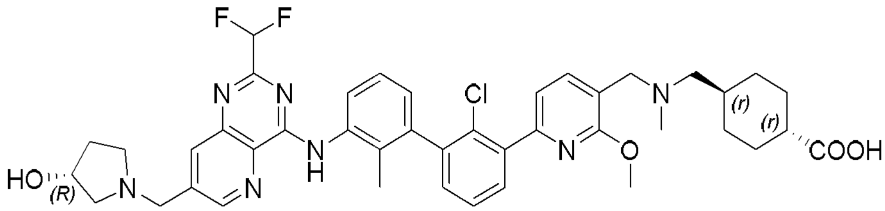

**Step 1: Synthesis of methyl (1*r*,4*r*)-4-((((6-(3-bromo-2-chlorophenyl)-2-methoxypyridin-3-yl)methyl)amino) methyl)cyclohexane-1-carboxylate**

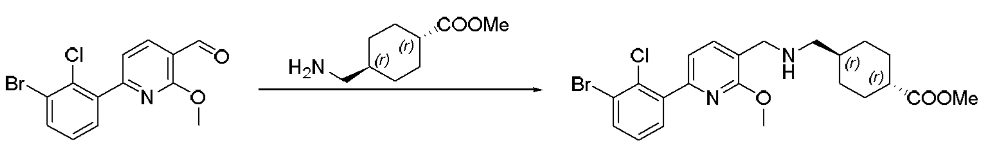

6-(3-Bromo-2-chlorophenyl)-2-methoxynicotinaldehyde (2.0 g, 6.12 mmol) and methyl (1*r*,4*r*)-4-(aminomethyl) cyclohexane-1-carboxylate (1.36 g, 7.96 mmol) were dissolved in 2,2,2-trifluoroethanol (10 mL) and dichloromethane (20 mL). The mixture was stirred at 50 °C for 2 h. Sodium cyanoborohydride (380 mg, 6.12 mmol) was added to the system and the mixture was stirred at room temperature for 3 h. After the reaction was completed, the system was concentrated and extracted with ethyl acetate (10 mL × 2). The organic phases were combined and concentrated under reduced pressure. The residue was separated and purified by silica gel column chromatography (eluent: PE/EtOAc (v/v) = 1/1) to obtain 2.60 g of a light yellow viscous product with a yield of 88.11%.

MS (ESI, pos. ion) m/z: 481.4 $[M+H]^+$.

**Step 2: Synthesis of methyl (1*r*,4*r*)-4-((((6-(3-bromo-2-chlorophenyl)-2-methoxypyridin-3-yl)methyl)(methyl)amino)methyl)cyclohexane-1-carboxylate**

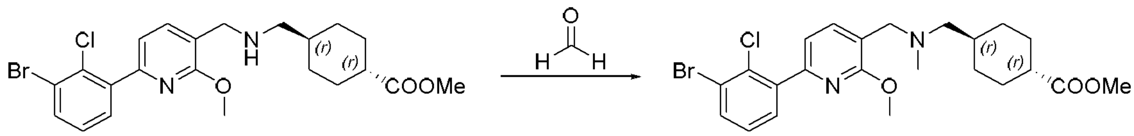

Methyl (1*r*,4*r*)-4-((((6-(3-bromo-2-chlorophenyl)-2-methoxypyridin-3-yl)methyl)amino)methyl)cyclohexane-1-carboxylate (3.8 g, 7.89 mmol) and formaldehyde (0.71 g, 23.67 mmol) were dissolved in 2,2,2-trifluoroethanol (20 mL) and dichloromethane (20 mL). The mixture was stirred at 50 °C for 2 h. Sodium cyanoborohydride (500 mg, 7.89 mmol) was added to the system and the mixture was stirred at room temperature for 3 h. After the reaction was completed, the product was concentrated under reduced pressure and the residue was separated and purified by silica gel column chromatography (eluent: PE/EtOAc (v/v) = 1/1) to obtain 3.65 g of a light yellow viscous product with a yield of 93.33%.

MS (ESI, pos. ion) m/z: 495.4 $[M+H]^+$.

**Step 3: Synthesis of methyl (1*r*,4*r*)-4-((((6-(3'-amino-2-chloro-2'-methyl-[1,1'-biphenyl]-3-yl)-2-methoxypyridin-3-yl)methyl)(methyl)amino)methyl)cyclohexane-1-carboxylate**

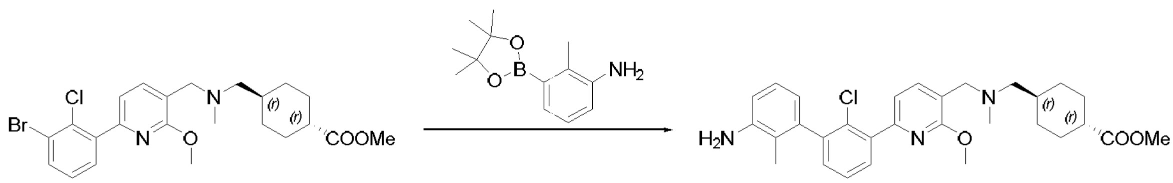

Methyl (1*r*,4*r*)-4-((((6-(3-bromo-2-chlorophenyl)-2-methoxypyridin-3-yl)methyl)(methyl)amino)methyl)cyclohexane-1-carboxylate (1.70 g, 3.43 mmol) and 2-chloro-3-(tetramethyl-1,3,2-dioxaborin-2-yl)aniline (1.2 g, 5.15 mmol) were dissolved in a mixed solvent of water (5 mL) and 1,4-dioxane (20 mL). Potassium carbonate (0.71 g, 5.15 mmol) and [1,1'-bis(dicyclohexylphosphino)ferrocene]dichloropalladium(II) (0.14 g, 0.17 mmol) were added in sequence. The mixture was heated to 100 °C for overnight. After the reaction was completed, the product was filtered, the filtrate was concentrated, and the residue was purified by silica gel column chromatography (eluent: PE/EtOAc (v/v) = 1/1) to obtain 1.70 g of a brown-black viscous substance with a yield of 94.97%.

MS (ESI, pos. ion) m/z: 522.3 $[M+H]^+$.

**Step 4: Synthesis of methyl (1*r*,4*r*)-4-((((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl) methyl)(methyl)amino)methyl)cyclohexane-1-carboxylate**

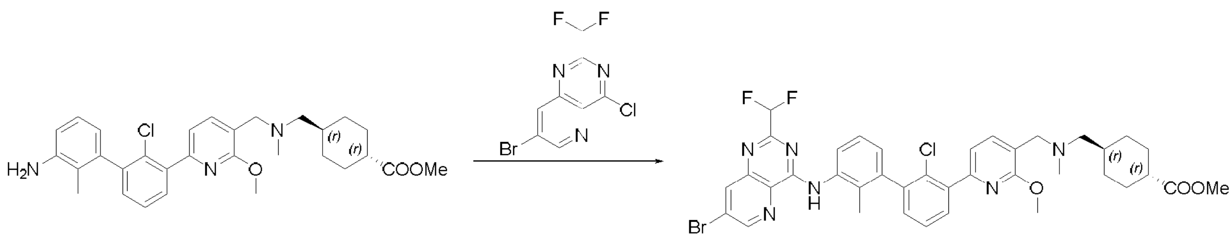

7-Bromo-4-chloro-2-(difluoromethyl)pyrido[3,2-d]pyrimidine (1.18 g, 4.02 mmol) and methyl (1*r*,4*r*)-4-((((6-(3'-amino-2-chloro-2'-methyl-[1,1'-biphenyl]-3-yl)-2-methoxypyridin-3-yl)methyl)(methyl)amino)methyl)cyclohexane-1-carboxylate (1.75 g, 3.35 mmol) were dissolved in *tert*-butanol (50 mL) and stirred at 120 °C for 5 h under $N_2$. After the reaction was complete, the mixture was concentrated under reduced pressure, and the residue was separated and purified by silica

gel column chromatography (eluent: DCM/MeOH (v/v) = 20/1) to obtain 2.1 g of brown yellow powder with a yield of 80.31%.

MS (ESI, pos. ion) m/z: 779.5 $[M+H]^+$.

**Step 5: Synthesis of methyl (1*r*,4*r*)-4-((((6-(2-chloro-3'-((2-(difluoromethyl)-7-vinylpyrido[3,2-*d*]pyrimidin-4-yl) amino)-2'-methyl-[1,1'-biphenyl]-3-yl)-2-methoxypyridin-3-yl)methyl) (methyl)amino)methyl)cyclohexane-1-carboxylate**

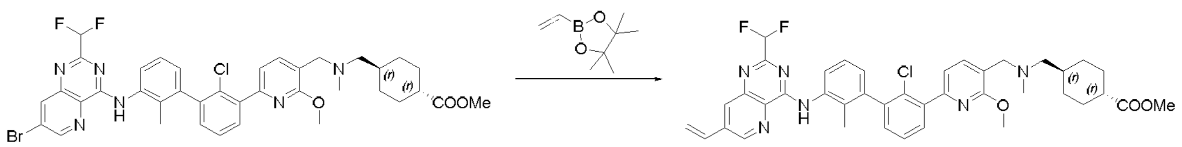

Methyl (1*r*,4*r*)-4-((((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)(methyl)amino)methyl)cyclohexane-1-carboxylate (2.0 g, 2.56 mmol) was dissolved in a mixed solvent of 1,4-dioxane (30 mL) and water (6 mL), 2-vinyl-4,4,5,5-tetramethyl-1,3,2-dioxaborolane (0.99 g, 6.4 mmol), [1,1'-Bis(diphenylphosphino)ferrocene]palladium dichloride dichloromethane adduct (0.21 g, 0.26 mmol) and potassium phosphate (1.36 g, 6.4 mmol) were added in sequence, and the system was stirred at 100 °C for 4 h under $N_2$. After the reaction was completed, the mixture was filtered and concentrated, and the residue was purified by column chromatography (eluent: DCM/MeOH (v/v) = 20/1) to obtain 1.6 g of a yellow solid with a yield of 85.81%.

MS (ESI, pos. ion) m/z: 727.3 $[M+H]^+$.

**Step 6: Synthesis of methyl (1*r*,4*r*)-4-((((6-(2-chloro-3'-((2-(difluoromethyl)-7-formylpyrido[3,2-*d*]pyrimidin-4-yl) amino)-2'-methyl-[1,1'-biphenyl]-3-yl)-2-methoxypyridin-3-yl)methyl) (methyl)amino)methyl)cyclohexane-1-carboxylate**

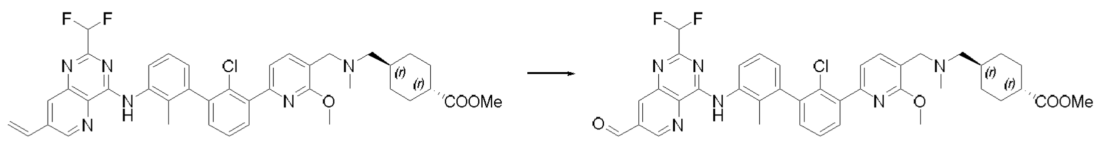

Methyl (1*r*,4*r*)-4-((((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)(methyl) amino)methyl)cyclohexane-1-carboxylate (2.56 g, 3.52 mmol) was dissolved in 1,4-dioxane (36 mL) and water (12 mL), potassium osmate dihydrate (0.065 mg, 0.18 mmol) and sodium periodate (1.51 g, 7.04 mmol) were added in sequence, and the system was stirred at room temperature for 2 h. After the reaction was completed, the mixture was filtered and separated. The aqueous phase was extracted with ethyl acetate (20 mL × 3). The organic phases were combined and dried over anhydrous sodium sulfate. The filtrate was concentrated under reduced pressure. The residue was separated and purified by silica gel column chromatography (eluent: DCM/MeOH (v/v) = 20/1) to obtain 0.87 g of a brown-yellow viscous product with a yield of 33.89%.

MS (ESI, pos. ion) m/z: 729.6 $[M+H]^+$.

**Step 7: Synthesis of methyl (1*r*,4*r*)-4-(((6-(2-chloro-3'-((2-(difluoromethyl)-7-(((*R*)-3-hydroxypyrrolidin-1-yl) methyl)pyrido[3,2-*d*]pyrimidin-4-yl)amino)-2'-methyl-[1,1'-biphenyl]-3-yl)-2-methoxypyridin-3-yl)methyl) (methyl)amino)methyl)cyclohexane-1-carboxylate**

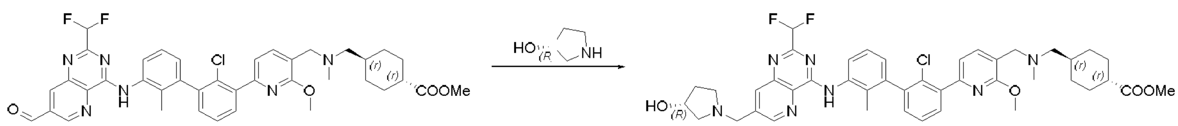

Methyl (1*r*,4*r*)-4-((((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)(methyl) amino)methyl)cyclohexane-1-carboxylate (1.4 g, 1.92 mmol) and (3R)-pyrrolidin-3-ol (836.35 mg , 9.6 mmol) were dissolved in methanol (45 mL) and DCM (15 mL), and acetic acid (576.48 mg, 9.6 mmol) was added dropwise. The reaction was stirred at room temperature for 13 h. Sodium cyanoborohydride (361.96 mg, 5.76 mmol) was added and the reaction was continued at room temperature for 1 hours. After the reaction was complete, the mixture was concentrated under reduced pressure, and the residue was separated and purified by silica gel column chromatography (eluent: DCM/MeOH (v/v) = 10/1) to obtain 873 mg of light yellow powder with a yield of 56.82%.

MS (ESI, pos. ion) m/z: 800.8 $[M+H]^+$.

**Step 8: Synthesis of (1*r*,4*r*)-4-(((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)methyl)cyclohexane-1-carboxylic acid**

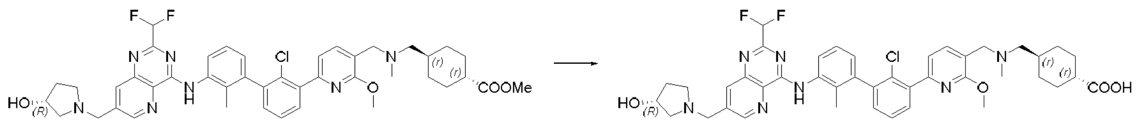

Methyl (1*r*,4*r*)-4-((((6-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)(methyl)amino)methyl)cyclohexane-1-carboxylate (0.773 g, 0.97 mmol) was dissolved in a mixed solution of 1,4-dioxane (50 mL) and water (10 mL), lithium hydroxide monohydrate (488.41 mg, 11.64 mmol) was added, and the reaction was stirred at room temperature for 20 h. After the reaction was completed, the product was concentrated under reduced pressure and the residue was separated and purified by silica gel column chromatography (eluent: dichloromethane/methanol (v/v) = 9/1) to obtain 293.8 mg of yellow powder with a yield of 36.89%.

MS (ESI, pos. ion) m/z: 786.3 $[M+H]^+$.

$^1$H NMR (400 MHz, DMSO-$d_6$) δ 10.42 (s, 1H), 9.03 (d, *J* = 27.1 Hz, 1H), 8.30 (s, 1H), 7.86 (s, 1H), 7.71 (d, *J* = 7.9 Hz, 1H), 7.65 (d, *J* = 7.6 Hz, 1H), 7.54 (q, *J* = 10.4, 9.2 Hz, 1H), 7.36 (d, *J* = 24.6 Hz, 2H), 7.23 - 7.09 (m, 1H), 7.03 - 6.39 (m, 2H), 5.08 (s, 1H), 4.36 (d, *J* = 42.9 Hz, 2H), 4.19 (d, *J* = 22.7 Hz, 2H), 3.92 (s, 3H), 3.22 (d, *J* = 7.4 Hz, 1H), 3.11 (d, *J* = 9.9 Hz, 1H), 3.06 - 2.93 (m, 3H), 2.85 - 2.71 (m, 2H), 2.41 - 2.36 (m, 1H), 2.18 - 2.08 (m, 2H), 2.06 (s, 1H), 2.03 (s, 2H), 1.90 (s, 3H), 1.87 (s, 3H), 1.76 - 1.69 (m, 1H), 1.68 - 1.57 (m, 1H), 1.32 (d, *J* = 11.7 Hz, 2H), 1.27 - 1.12 (m, 2H).

**Example 18 (1*r*,4*r*)-4-(((5-(2-chloro-3'-((2-(difluoromethyl)-7-((3-methoxyazetidin-1-yl)methyl)pyrido[3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylic acid**

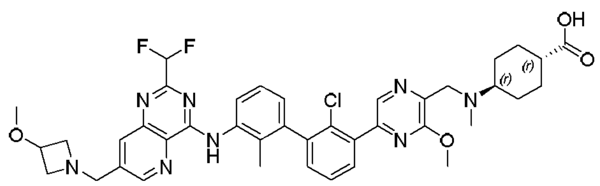

**Step 1: Synthesis of methyl (1*r*,4*r*)-4-(((5-(2-chloro-3'-((2-(difluoromethyl)-7-((3-methoxy azetidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

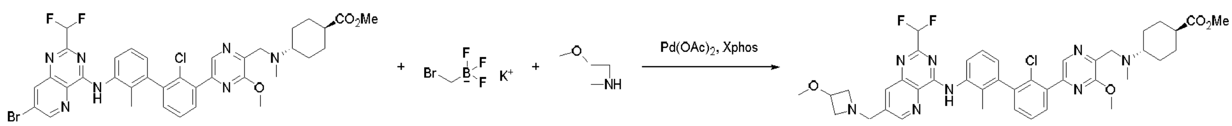

At room temperature, 3-methoxyazetidine (0.34 g, 3.90 mmol), potassium bromomethyl trifluoroborate (0.78 g, 3.90 mmol) and sodium carbonate (0.83 g, 7.80 mmol) were added to acetonitrile (10 mL), and stirred at 80 °C for 5 hours, concentrated under reduced pressure, and then $Pd(OAc)_2$ (0.0029 g, 0.013 mmol), X-Phos (0.012 g, 0.026 mmol), potassium carbonate (0.54 g, 3.90 mmol), methyl (1r,4r)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino) cyclohexane-1-carboxylate (1.0 g, 1.30 mmol), 1,4-dioxane (30 mL) and water (6 mL), the reaction mixture was stirred at 110 °C. After the reaction was completed, the reaction solution was concentrated under reduced pressure, and the residue was separated and purified by silica gel column chromatography (eluent: DCM/MeOH (v/v) = 20/1) to obtain 0.52 g of a yellow solid product with a yield of 50.66%.

MS (ESI, pos. ion) m/z: 787.3 $[M+H]^+$.

**Step 2: Synthesis of (1*r*,4*r*)-4-(((5-(2-chloro-3'-((2-(difluoromethyl)-7-((3-methoxyazetidin-1-yl)methyl)pyrido [3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylic acid**

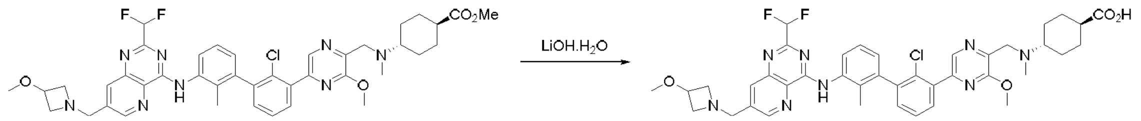


At room temperature, methyl (1*r*,4*r*)-4-(((5-(2-chloro-3'-((2-(difluoromethyl)-7-((3-methoxyazetidin-1-yl)methyl) pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (0.50 g, 0.64 mmol) and lithium hydroxide monohydrate (0.04 g, 0.96 mmol) were added to 1,4-dioxane (15 mL) and water (5 mL), and the reaction was stirred at 50 °C. After the reaction was completed, the reaction solution was concentrated under reduced pressure, and the residue was separated and purified by silica gel column chromatography (eluent: DCM/MeOH (v/v) = 5/1) to obtain 0.38 g of a white solid product with a yield of 77.4%.

MS (ESI, pos. ion) m/z: 773.31 $[M+H]^+$.

$^1H$ NMR (400 MHz, DMSO-$d_6$) δ 10.32 (s, 1H), 8.88 (s, 1H), 8.39 (s, 1H), 8.11 (s, 1H), 7.74 (d, *J* = 8.0 Hz, 1H), 7.68 (d, *J* = 7.6 Hz, 1H), 7.56 (t, *J* = 7.6 Hz, 1H), 7.44 - 7.34 (m, 2H), 7.15 (d, *J* = 7.6 Hz, 1H), 6.68 (t, *J* = 54.5 Hz, 1H), 4.01 (q, *J* = 5.9 Hz, 1H), 3.94 (s, 3H), 3.85 (s, 3H), 3.57 - 3.51 (m, 2H), 3.17 (s, 3H), 3.14 (s, 2H), 2.97 (t, *J* = 6.6 Hz, 1H), 2.45 (d, *J* = 11.7 Hz, 1H), 2.20 (s, 3H), 2.12 (s, 1H), 2.02 (s, 3H), 1.94 (d, *J* = 7.4 Hz, 1H), 1.90 (s, 1H), 1.85 (d, *J* = 8.1 Hz, 2H), 1.32 (d, *J* = 9.7 Hz, 4H)..

**Example 19 (1*r*,4*r*)-4-(((5-(2-chloro-3'-((2-(difluoromethyl)-7-((3-hydroxy-3-methylazetidin-1-yl)methyl)pyrido [3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylic acid**

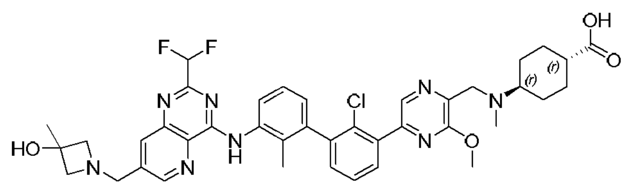

**Step 1: Synthesis of methyl (1*r*,4*r*)-4-(((5-(2-chloro-3'-((2-(difluoromethyl)-7-((3-hydroxy-3-methylazetidin-1-yl) methyl)pyrido[3,2-*d*]pyrimidin-4-yl)amino)-2'-methyl-[1,1'-biphenyl]-3-yl)-3-methoxypyrazin-2-yl)methyl) (methyl)amino)cyclohexane-1-carboxylate**

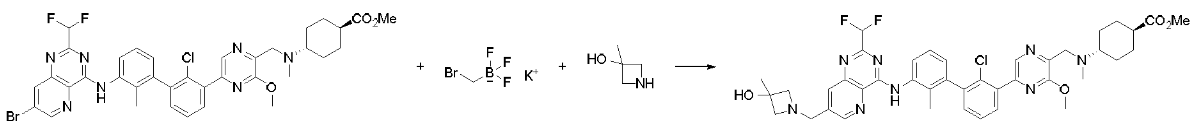

At room temperature, 3-methylazetidin-3-ol (0.34 g, 3.90 mmol), potassium bromomethyl trifluoroborate (0.78 g, 3.90 mmol) and sodium carbonate (0.83 g, 7.80 mmol) were added to acetonitrile (10 mL), and stirred at 80 °C for 5 hours, concentrated under reduced pressure, and then $Pd(OAc)_2$ (0.0029 g, 0.013 mmol), X-Phos (0.012 g, 0.026 mmol), potassium carbonate (0.54 g, 3.90 mmol), methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl) amino)cyclohexane-1-carboxylate (1.0 g, 1.30 mmol), 1,4-dioxane (30 mL) and water (6 mL), the reaction mixture was stirred at 110 °C. After the reaction, the mixture was filtered. The filtrate was concentrated. The residue was purified by silica gel chromatography eluted with DCM/MeOH (v/v) = 20/1 to give the product as a yellow solid (0.26 g, 25.33%).
MS (ESI, pos. ion) m/z: 787.4 $[M+H]^+$.

**Step 2: Synthesis of (1*r*,4*r*)-4-(((5-(2-chloro-3'-((2-(difluoromethyl)-7-((3-hydroxy-3-methylazetidin-1-yl)methyl) pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

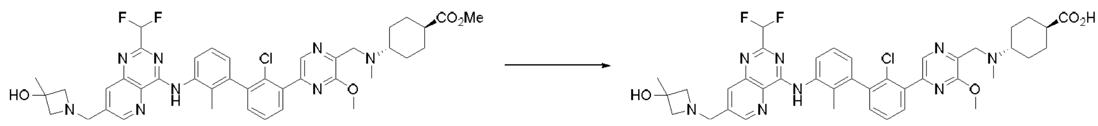

At room temperature, methyl (1*r*,4*r*)-4-(((5-(2-chloro-3'-((2-(difluoromethyl)-7-((3-hydroxy-3-methylazetidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (0.25 g, 0.32 mmol) and lithium hydroxide monohydrate (0.02 g, 0.48 mmol) were added to 1,4-dioxane (10 mL) and water (3 mL), and the reaction was stirred at 50 °C. After the reaction was completed, the reaction solution was concentrated under reduced pressure, and the residue was separated and purified by silica gel column chromatography (eluent: DCM/MeOH (v/v) = 5/1) to obtain 0.13 g of a yellow solid product with a yield of 52.9%.
MS (ESI, pos. ion) m/z: 773.31 $[M+H]^+$.
$^1$H NMR (400 MHz, DMSO-$d_6$) δ 10.29 (s, 1H), 8.88 (d, *J* = 1.8 Hz, 1H), 8.38 (s, 1H), 8.09 (s, 1H), 7.75 (d, *J* = 7.9 Hz, 1H), 7.67 (d, *J* = 7.6 Hz, 1H), 7.55 (t, *J* = 7.6 Hz, 1H), 7.44 - 7.33 (m, 2H), 7.14 (d, *J*= 7.5 Hz, 1H), 6.68 (t, *J*= 54.5 Hz, 1H), 3.93 (s, 3H), 3.85 (s, 2H), 3.24 (d, *J* = 6.9 Hz, 2H), 3.17 (s, 4H), 3.01 (d, *J*= 6.9 Hz, 2H), 2.47 - 2.38 (m, 1H), 2.18 (s, 3H), 2.02 (s, 3H), 1.91 (d, *J*= 7.9 Hz, 2H), 1.85 - 1.79 (m, 2H), 1.38 (s, 3H), 1.31 - 1.25 (m, 4H).

**Example 20 (1*r*,4*r*)-4-(((5-(2-chloro-3'-((2-(difluoromethyl)-7-(pyrrolidin-1-ylmethyl)pyrido[3,2-*d*]pyrimidin-4-yl) amino)-2'-methyl-[1,1'-biphenyl]-3-yl)-3-methoxy pyrazin-2-yl)methyl)(methyl)amino)cyclohexane-1-carboxylic acid**

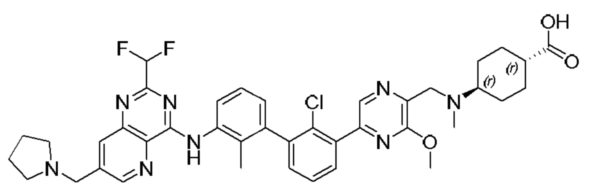

**Step 1: Synthesis of methyl (1*r*,4*r*)-4-(((5-(2-chloro-3'-((2-(difluoromethyl)-7-(pyrrolidin-1-ylmethyl)pyrido[3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylate**

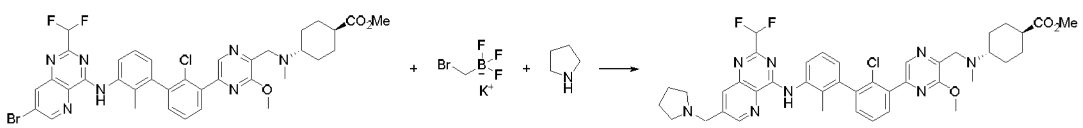

At room temperature, pyrrolidine (0.28 g, 3.90 mmol), potassium bromomethyl trifluoroborate (0.78 g, 3.90 mmol)

and sodium carbonate (0.41 g, 3.90 mmol) were added to acetonitrile (6 mL), and stirred at 80 °C for 5 hours, concentrated under reduced pressure, and then $Pd(OAc)_2$ (15.0 mg, 0.06 mmol), X-Phos (62.0 mg, 0.13 mmol), potassium carbonate (0.54 g, 3.90 mmol), methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino) cyclohexane-1-carboxylate (1.0 g, 1.30 mmol), 1,4-dioxane (20 mL) and water (5 mL), the reaction mixture was stirred at 110 °C. After the reaction was completed, the reaction solution was concentrated under reduced pressure, and the residue was separated and purified by silica gel column chromatography (eluent: DCM/MeOH (v/v) = 10/1) to obtain 0.31 g of a yellow solid product with a yield of 30.8%.

MS (ESI, pos. ion) m/z: 771.3 $[M+H]^+$.

**Step 2: Synthesis of (1*r*,4*r*)-4-(((5-(2-chloro-3'-((2-(difluoromethyl)-7-(pyrrolidin-1-ylmethyl)pyrido[3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylic acid**

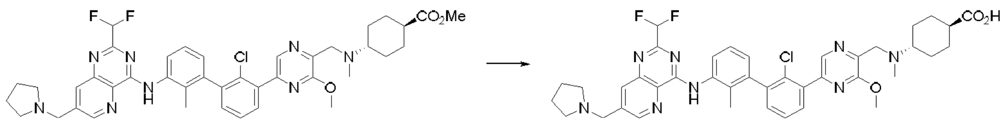

At room temperature, methyl (1r,4r)-4-(((5-(2-chloro-3'-((2-(difluoromethyl)-7-(pyrrolidin-1-ylmethyl)pyrido [3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (280 mg, 0.36 mmol) and lithium hydroxide monohydrate (60 mg, 1.44 mmol) were added to methanol (2.0 mL), tetrahydrofuran (1.0 mL) and water (1.0 mL), and the reaction was stirred at 60 °C for 2 h. After the reaction was completed, the reaction solution was concentrated under reduced pressure, and the residue was separated and purified by silica gel column chromatography (eluent: DCM/MeOH (v/v) = 10/1) to obtain 70 mg of a yellow solid product with a yield of 25.5%.

MS (ESI, pos. ion) m/z: 773.31 $[M+H]^+$.

$^1$H NMR (400 MHz, DMSO-$d_6$) δ 10.37 (s, 1H), 8.97 (s, 1H), 8.43 (s, 1H), 8.18 (s, 1H), 7.77 - 7.67 (m, 2H), 7.59 (t, *J* = 7.6 Hz, 1H), 7.46 - 7.34 (m, 2H), 7.17 (d, *J* = 7.5 Hz, 1H), 6.71 (t, *J* = 54.5 Hz, 1H), 3.96 (s, 3H), 3.89 (s, 2H), 3.71 (s, 2H), 2.59 - 2.51 (m, 7H), 2.28 - 2.09 (m, 4H), 2.00 - 1.82 (m, 5H), 1.79 - 1.70 (m, 4H), 1.39 - 1.28 (m, 4H).

**Example 21 (1*r*,4*r*)-4-(((5-(2-chloro-3'-((2-(difluoromethyl)-7-((4-hydroxypiperidin-1-yl)methyl)pyrido[3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylic acid**

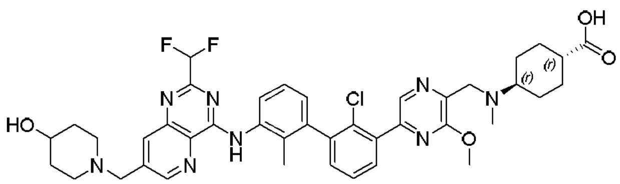

**Step 1: Synthesis of methyl (1*r*,4*r*)-4-(((5-(2-chloro-3'-((2-(difluoromethyl)-7-(hydroxymethyl) pyrido[3,2-*d*]pyrimidin-4-yl)amino)-2'-methyl-[1,1'-biphenyl]-3-yl)-3-methoxypyrazin-2-yl) methyl)(methyl)amino)cyclohexane-1-carboxylate**

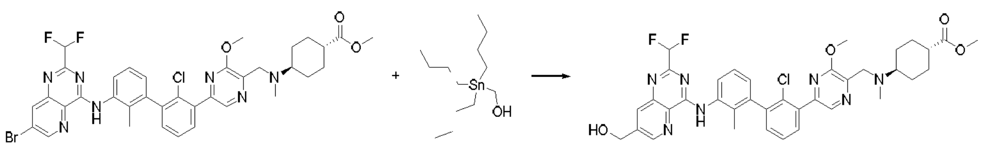

To the reaction flask, methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl) (methyl)amino)cyclohexane-1-carboxylate (3.00 g, 3.91 mmol), 1,4-dioxane (40.0 mL), (tributylstannyl)methanol (2.01 g, 6.26 mmol), chloro(2-dicyclohexylphosphino-2',4',6'-tri-i-propyl-1,1'-biphenyl)[2-(2-aminoethyl)phenyl] palladium(II) (0.15 g, 0.20 mmol), after nitrogen replacement, the mixture was heated to 90 °C and stirred overnight. After the reaction was completed, the reaction solution was concentrated under reduced pressure, and the residue was separated and purified by silica gel column chromatography (eluent: DCM/MeOH (v/v) = 10/1) to obtain 2.50 g of a yellow solid product with a yield of 89.0%.

MS (ESI, pos. ion) m/z: 718.2 [M+H]$^+$.

**Step 2: Synthesis of methyl (1*r*,4*r*)-4-(((5-(2-chloro-3'-((2-(difluoromethyl)-7-(hydroxymethyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

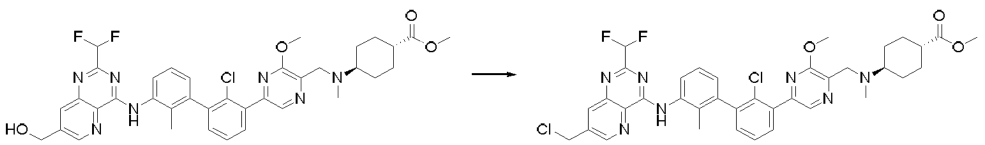

To the reaction flask, methyl (1*r*,4*r*)-4-(((5-(2-chloro-3'-((7-(chloromethyl)-2-(difluoromethyl)pyrido[3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylate (1.30 g, 1.81 mmol), dichloromethane (30.0 mL) and thionyl chloride (0.43 g, 3.62 mmol) were added in sequence, and DMF (0.2 mL) was added dropwise, and the mixture was stirred at room temperature overnight. After the reaction was completed, methanol (3 mL) was added to the above mixture to quench the reaction, and the reaction solution was concentrated under reduced pressure. The residue was separated and purified by silica gel column chromatography (eluent: DCM/MeOH (v/v) = 50/1) to obtain 1.2 g of a yellow solid product with a yield of 90%.

MS (ESI, pos. ion) m/z: 736.2 [M+H]$^+$.

**Step 3: Synthesis of methyl (1*r*,4*r*)-4-(((5-(2-chloro-3'-((2-(difluoromethyl)-7-((4-hydroxypiperidin-1-yl)methyl) pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

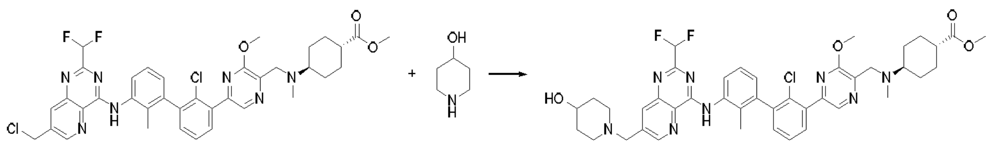

To the reaction bottle, methyl (1*r*,4*r*)-4-(((5-(2-chloro-3'-((7-(chloromethyl)-2-(difluoromethyl)pyrido[3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylate (200 mg, 0.27 mmol), acetonitrile (3.0 mL), piperidin-4-ol (33 mg, 0.32 mmol), potassium carbonate (75 mg, 0.54 mmol) and tetrabutylammonium bromide (4 mg, 0.01 mmol) were added in sequence, and the mixture was heated to 80 °C and stirred for 1 h. After the reaction, water (30 mL) was added to the mixture, and the resulting mixture was extracted with a mixed solvent (DCM/MeOH (v/v) = 10/1, 30 mL × 3). The organic phases were combined, washed with saturated brine (60 mL), dried over anhydrous sodium sulfate, filtered, and concentrated under reduced pressure. The residue was separated and purified by silica gel column chromatography (eluent: DCM/MeOH (v/v) = 10/1) to obtain 200 mg of a light yellow oily product with a yield of 91.9%.

MS (ESI, pos. ion) m/z: 801.4 [M+H]$^+$.

**Step 4: Synthesis of (1*r*,4*r*)-4-(((5-(2-chloro-3'-((2-(difluoromethyl)-7-((4-hydroxypiperidin-1-yl)methyl)pyrido [3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylic acid**

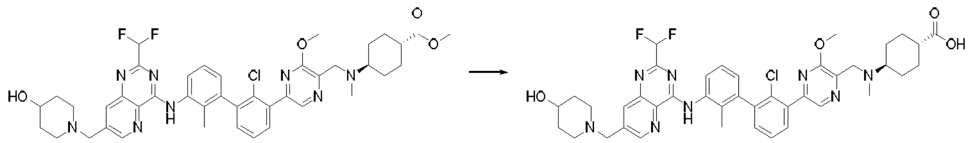

To the reaction bottle, methyl (1*r*,4*r*)-4-(((5-(2-chloro-3'-((2-(difluoromethyl)-7-((4-hydroxypiperidin-1-yl)methyl) pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (200 mg, 0.25 mmol), lithium hydroxide (24 mg, 1.00 mmol), tetrahydrofuran (2.0 mL), methanol (1.0 mL) and water (1.0 mL) were added in sequence, and the mixture was heated to 50 °C and stirred for 1 h. After the reaction was completed, glacial acetic acid (0.5 mL) was added to the mixture, and the mixture was concentrated under reduced pressure. The residue was separated and purified by silica gel column chromatography (eluent: DCM/MeOH (v/v) = 4/1) to obtain 80 mg of a yellow-green solid product with a yield of 40.7%.

MS (ESI, pos. ion) m/z: 787.33 $[M+H]^+$.

$^1H$ NMR (400 MHz, DMSO-$d_6$) δ 10.35 (s, 1H), 8.93 (s, 1H), 8.42 (s, 1H), 8.14 (s, 1H), 7.75 (d, *J* = 7.8 Hz, 1H), 7.70 (d, *J* = 6.8 Hz, 1H), 7.61 - 7.54 (m, 1H), 7.44 (d, *J*= 6.6 Hz, 1H), 7.38 (t, *J* = 7.8 Hz, 1H), 7.17 (d, *J* = 7.4 Hz, 1H), 6.71 (t, *J* = 54.5 Hz, 1H), 3.96 (s, 3H), 3.74 (s, 2H), 3.70 (s, 2H), 3.52 - 3.44 (m, 1H), 2.77 - 2.67 (m, 2H), 2.21 (s, 3H), 2.19 - 2.09 (m, 4H), 2.04 (s, 3H), 1.99 - 1.91 (m, 2H), 1.89 - 1.80 (m, 2H), 1.78 - 1.67 (m, 2H), 1.48 - 1.37 (m, 2H), 1.37 - 1.29 (m, 4H).

**Example 22 (1*r*,4*r*)-4-(((5-(2-chloro-3'-((2-(difluoromethyl)-7-(((*S*)-3-hydroxypiperidin-1-yl)methyl)pyrido[3,2-*d*] pyrimidin-4-yl)amino)-2'-methyl-[1,1'-biphenyl]-3-yl)-3-methoxy pyrazin-2-yl)methyl)(methyl)amino)cyclohexane-1-carboxylic acid**

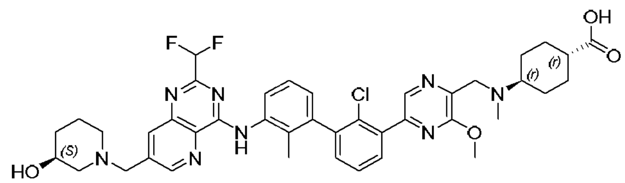

**Step 1: Synthesis of methyl (1*r*,4*r*)-4-(((5-(2-chloro-3'-((2-(difluoromethyl)-7-(((*S*)-3-hydroxypiperidin-1-yl) methyl)pyrido[3,2-*d*]pyrimidin-4-yl)amino)-2'-methyl-[1,1'-biphenyl]-3-yl)-3-methoxypyrazin-2-yl)methyl) (methyl)amino)cyclohexane-1-carboxylate**

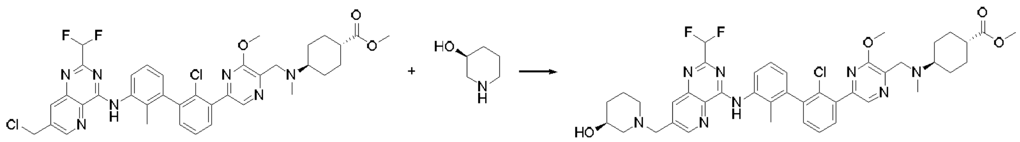

To the reaction bottle, methyl (1*r*,4*r*)-4-(((5-(2-chloro-3'-((7-(chloromethyl)-2-(difluoromethyl)pyrido[3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylate (270 mg, 0.37 mmol), acetonitrile (8.0 mL), (S)-piperidin-3-ol (75 mg, 0.74 mmol), potassium carbonate (0.10 g, 0.74 mmol) and *tert*-butylammonium bromide (12 mg, 0.04 mmol) were added in sequence, and the mixture was heated to 80 °C and stirred for 2 h. After the reaction, water (30 mL) was added to the mixture, and the resulting mixture was extracted with a mixed solvent (DCM/MeOH (v/v) = 10/1, 30 mL × 3). The organic phases were combined, washed with saturated brine (60 mL), dried over anhydrous sodium sulfate, filtered, and concentrated under reduced pressure. The residue was separated and purified by silica gel column chromatography (eluent: DCM/MeOH (v/v) = 4/1) to obtain 170 mg of a light yellow solid product with a yield of 57.9%.

MS (ESI, pos. ion) m/z: 801.4 $[M+H]^+$.

**Step 2: Synthesis of (1*r*,4*r*)-4-(((5-(2-chloro-3'-((2-(difluoromethyl)-7-(((*S*)-3-hydroxypiperidin-1-yl)methyl)pyrido[3,2-*d*]pyrimidin-4-yl)amino)-2'-methyl-[1,1'-biphenyl]-3-yl)-3-methoxy pyrazin-2-yl)methyl)(methyl)amino) cyclohexane-1-carboxylic acid**

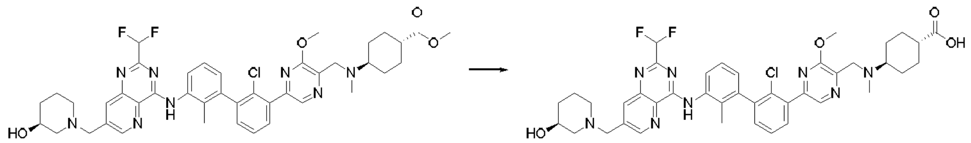

To the reaction bottle, methyl (1*r*,4*r*)-4-(((5-(2-chloro-3'-((2-(difluoromethyl)-7-(((*S*)-3-hydroxypiperidin-1-yl) methyl)pyrido[3,2-*d*]pyrimidin-4-yl)amino)-2'-methyl-[1,1'-biphenyl]-3-yl)-3-methoxypyrazin-2-yl)methyl)(methyl)amino) cyclohexane-1-carboxylate (170 mg, 0.21 mmol), methanol (1.0 mL), tetrahydrofuran (1.5 mL), water (1.0 mL) and lithium hydroxide monohydrate (35 mg, 0.84 mmol) were added in sequence, and the mixture was heated to 50 °C and stirred for 1 h. After the reaction was completed, glacial acetic acid (0.5 mL) was added to the mixture, and the mixture was concentrated under reduced pressure. The residue was separated and purified by silica gel column chromatography (eluent: DCM/MeOH (v/v) = 4/1) to obtain 90 mg of a light yellow solid product with a yield of 53.88%.

MS (ESI, pos. ion) m/z: 787.33 $[M+H]^+$.

$^1$H NMR (400 MHz, DMSO-$d_6$) δ 9.37 (s, 1H), 8.84 (s, 1H), 8.53 (d, *J*= 8.2 Hz, 1H), 8.49 (s, 1H), 8.17 (s, 1H), 7.62 (dd, *J* = 7.6, 1.4 Hz, 1H), 7.48 - 7.38 (m, 2H), 7.35 (dd, *J* = 7.5, 1.5 Hz, 1H), 7.10 (d, *J* = 7.4 Hz, 1H), 6.59 (t, *J* = 55.0 Hz, 1H), 4.04 (s, 3H), 3.93 (s, 2H), 3.88 - 3.82 (m, 1H), 3.75 (s, 2H), 2.82 - 2.70 (m, 1H), 2.67 - 2.60 (m, 1H), 2.55 - 2.45 (m, 5H), 2.44 - 2.35 (m, 1H), 2.29 - 2.23 (m, 4H), 2.17 - 2.05 (m, 4H), 1.86 - 1.75 (m, 1H), 1.74 - 1.63 (m, 1H), 1.61 - 1.52 (m, 2H), 1.52 - 1.42 (m, 4H).

**Example 23 (1*r*,4*r*)-4-(((5-(2-chloro-3'-((2-(difluoromethyl)-7-(((*R*)-3-(hydroxymethyl) pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino) cyclohexane-1-carboxylic acid**

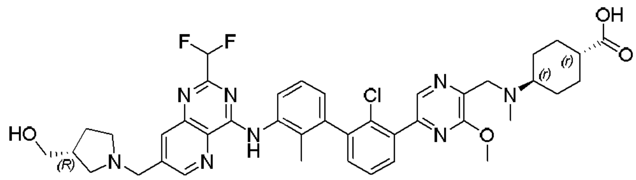

**Step 1: Synthesis of (1*r*,4*r*)-4-(((5-(2-chloro-3'-((2-(difluoromethyl)-7-(((*R*)-3-(hydroxymethyl)pyrrolidin-1-yl) methyl)pyrido[3,2-*d*]pyrimidin-4-yl)amino)-2'-methyl-[1,1'-biphenyl]-3-yl)-3-methoxypyrazin-2-yl)methyl) (methyl)amino)cyclohexane-1-carboxylic acid**

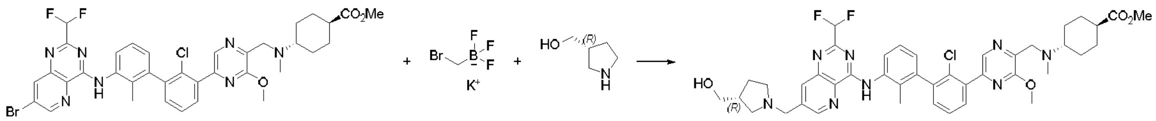

To a 50 mL single-necked bottle, (R)-pyrrolidin-3-ylmethanol (0.39 g, 3.90 mmol), potassium (bromomethyl) trifluoroborate (0.78 g, 3.90 mmol), sodium carbonate (0.21 g, 1.95 mmol) and ACN (10 mL) were added in sequence and reacted at 80 °C for 5 h. The mixture was concentrated under reduced pressure, and methyl (1*r*,4*r*)-4-(((5-(3'-((7-bromo-2-(difluoromethyl)pyrido[3,2-d]pyrimidin-4-yl)amino)-2-chloro-2'-methyl-[1,1'-biphenyl]-3-yl)-3-methoxypyrazin-2-yl)methyl) (methyl)amino)cyclohexane-1-carboxylate (1.0 g, 1.30 mmol), potassium carbonate (0.54 g, 3.90 mmol), $Pd(OAc)_2$ (0.029 g, 0.13 mmol), X-Phos (0.12 g, 0.26 mmol), 1,4-dioxane (30 mL) and water (6 mL) were added, and the reaction mixture was reacted at 110 °C under nitrogen overnight. After the reaction was complete, the reaction solution was concentrated under reduced pressure to remove dioxane, water (100 mL) was added, and then extracted with DCM (20 mL × 3). The organic phases were combined, washed with saturated brine (60 mL), dried over anhydrous sodium sulfate,

filtered, and the filtrate was concentrated under reduced pressure. The residue was separated and purified by silica gel column chromatography (eluent: DCM/MeOH (v/v) = 40/1) to obtain 0.63 g of a brown solid product with a yield of 60.3%.

MS (ESI, pos. ion) m/z: 801.4 $[M+H]^+$.

**Step 2: Synthesis of (1*r*,4*r*)-4-(((5-(2-chloro-3'-((2-(difluoromethyl)-7-(((*R*)-3-(hydroxymethyl)pyrrolidin-1-yl) methyl)pyrido[3,2-*d*]pyrimidin-4-yl)amino)-2'-methyl-[1,1'-biphenyl]-3-yl)-3-methoxypyrazin-2-yl)methyl) (methyl)amino)cyclohexane-1-carboxylic acid**

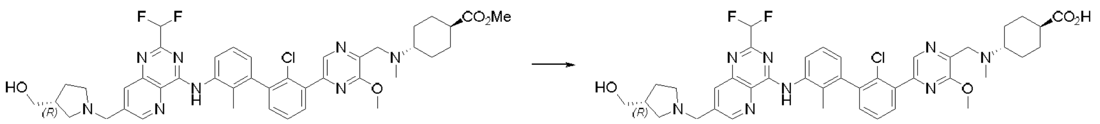

To a 50 mL single-necked bottle, methyl (1*r*,4*r*)-4-(((5-(2-chloro-3'-((2-(difluoromethyl)-7-(((R)-3-(hydroxymethyl)pyrrolidin-1-yl)methyl)pyrido[3,2-*d*]pyrimidin-4-yl) amino)-2'-methyl-[1,1'-biphenyl]-3-yl)-3-methoxypyrazin-2-yl) methyl)(methyl)amino) cyclohexane-1-carboxylate (523 mg, 0.65 mmol), 1,4-dioxane (15 mL), water (5 mL) and lithium hydroxide monohydrate (40.91 mg, 0.98 mmol) were added in sequence and reacted at 50 °C overnight. After the reaction was completed, an appropriate amount of acetic acid was added and the mixture was concentrated under reduced pressure. The residue was separated and purified by preparative silica gel thin layer chromatography (eluent: DCM/MeOH (v/v) = 6/1) to obtain 280.0 mg of a yellow solid product with a yield of 54.5%.

MS (ESI, pos. ion) m/z: 787.32 $[M+H]^+$.

$^1$H NMR (400 MHz, DMSO-$d_6$) δ 10.33 (s, 1H), 8.93 (d, *J* = 1.3 Hz, 1H), 8.40 (s, 1H), 8.14 (s, 1H), 7.73 (d, *J* = 7.9 Hz, 1H), 7.68 (d, *J* = 6.3 Hz, 1H), 7.57 (t, *J* = 7.6 Hz, 1H), 7.42 (d, *J* = 6.2 Hz, 1H), 7.38 (t, *J* = 7.8 Hz, 1H), 7.15 (d, *J* = 7.4 Hz, 1H), 6.68 (t, *J* = 54.5 Hz, 1H), 3.94 (s, 3H), 3.89 - 3.77 (m, 3H), 3.69 (s, 2H), 3.33 - 3.24 (m, 3H), 2.61 - 2.53 (m, 2H), 2.40 - 2.29 (m, 2H), 2.27 - 2.22 (m, 1H), 2.20 (s, 3H), 2.14 - 2.08 (m, 1H), 2.02 (s, 3H), 1.98 - 1.80 (m, 7H), 1.47 - 1.32 (m, 4H).

**Example 24 (1*r*,4*r*)-4-(((5-(2-chloro-3'-((2-(difluoromethyl)-7-(((*R*)-3-(difluoromethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino) cyclohexane-1-carboxylic acid**

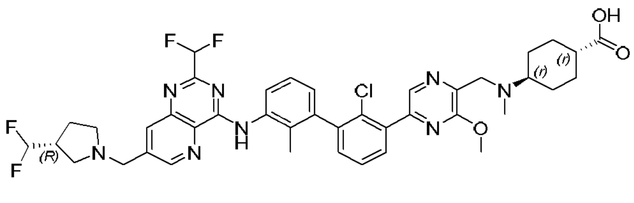

**Step 1: *Tert*-butyl (*R*)-3-(hydroxymethyl)pyrrolidine-1-carboxylate**

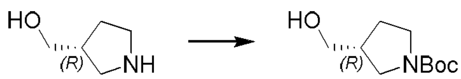

(*R*)-Pyrrolidin-3-ylmethanol (3.5 g, 34.60 mmol), DCM (100 mL), TEA (7.00 g, 69.2 mmol) and di-*tert*-butyl dicarbonate (9.82 g, 44.98 mmol) were added to a 250 mL single-necked bottle in sequence and reacted at room temperature. After the reaction was completed, the mixture was concentrated under reduced pressure to obtain 6.93 g of a yellow oily product with a yield of 99.5%.

MS (ESI, pos. ion) m/z: 146.2 $[M+H-56]^+$.

**Step 2: Synthesis of *tert*-butyl (*R*)-3-formylpyrrolidine-1-carboxylate**

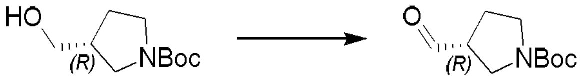

To a 250 mL single-necked bottle, *tert*-butyl (*R*)-3-(hydroxymethyl)pyrrolidine-1-carboxylate (6.65 g, 33.04 mmol), DCM (120 mL) and DMP (21.02 g, 49.56 mmol) were added in sequence and reacted at room temperature. After the reaction, the residue was removed by filtration, the pH was adjusted to 7-8 with saturated potassium carbonate solution, dried over anhydrous sodium sulfate, filtered, and the filtrate was concentrated under reduced pressure. The residue was separated and purified by silica gel column chromatography (eluent: PE/EtOAc (v/v) = 5/1) to obtain 6.35 g of colorless oily product with a yield of 96.5%.

MS (ESI, pos. ion) m/z: 144.2 $[M+H-56]^+$.

**Step 3: Synthesis of *tert*-butyl (*R*)-3-(difluoromethyl)pyrrolidine-1-carboxylate**

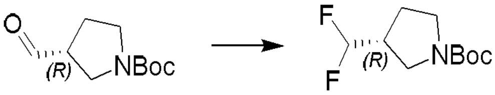

To a 250 mL single-necked bottle, *tert*-butyl (*R*)-3-(hydroxymethyl)pyrrolidine-1-carboxylate (6.35 g, 31.87 mmol) and DCM (100 mL) were added. The reaction mixture was cooled to 0 °C and DAST (12.84 g, 79.67 mmol) was added and reacted at room temperature. After the reaction was completed, the pH was adjusted to 7~8 with a saturated potassium carbonate solution to quench the reaction, water (200 mL) was added, and then extracted with DCM (40 mL × 3). The organic phases were combined, washed with water (200 mL × 1), dried over anhydrous sodium sulfate, filtered, and the filtrate was concentrated under reduced pressure. The residue was separated and purified by silica gel column chromatography (eluent: PE/EtOAc (v/v) = 10/1) to obtain 5.50 g of a colorless oily product with a yield of 78.0%.

MS (ESI, pos. ion) m/z: 166.2 $[M+H-56]^+$.

**Step 4: Synthesis of (*R*)-3-(difluoromethyl)pyrrolidine**

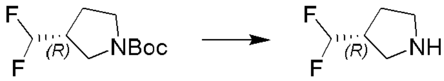

To a 250 mL single-necked bottle, *tert*-butyl (*R*)-3-(difluoromethyl)pyrrolidine-1-carboxylate (4.64 g, 20.97 mmol), DCM (50 mL) and hydrochloric acid (7.65 g, 209.7 mmol) were added in sequence and reacted at room temperature. After the reaction was completed, the mixture was concentrated under reduced pressure, dissolved with MeOH (50 mL), adjusted to pH 7~8 with saturated potassium carbonate solution, dried over anhydrous sodium sulfate, filtered, and the filtrate was concentrated under reduced pressure. The residue was separated and purified by silica gel column chromatography (eluent: DCM/MeOH (v/v) = 10/1) to obtain 1.15 g of a brown oily product with a yield of 45.27%.

MS (ESI, pos. ion) m/z: 122.2 $[M+H]^+$.

**Step 5: Synthesis of methyl (1*r*,4*r*)-4-(((5-(2-chloro-3'-((2-(difluoromethyl)-7-(((R)-3-(difluoromethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

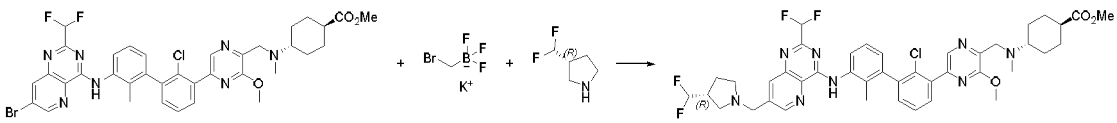

To a 100 mL single-necked bottle, (*R*)-3-(difluoromethyl)pyrrolidine (0.47 g, 3.90 mmol), potassium (bromomethyl)trifluoroborate (0.78 g, 3.90 mmol), sodium carbonate (0.41 g, 3.90 mmol) and ACN (20 mL) were added in sequence and reacted at 80 °C overnight. Then, methyl (1*r*,4*r*)-4-(((5-(3'-((7-bromo-2-(difluoromethyl)pyrido[3,2-*d*]

pyrimidin-4-yl)amino)-2-chloro-2'-methyl-[1,1'-biphenyl]-3-yl)-3-methoxypyrazin-2-yl)methyl)(methyl)amino)cyclohexane-1-carboxylate (1 g, 1.30 mmol), potassium carbonate (0.54 g, 3.90 mmol), $Pd(OAc)_2$ (0.029 g, 0.13 mmol), X-Phos (0.12 g, 0.26 mmol), 1,4-dioxane (30 mL) and water (6 mL) were added, and the reaction mixture was reacted at 130 °C for 8 h under $N_2$. After the reaction was completed, the reaction solution was concentrated under reduced pressure, and the residue was separated and purified by silica gel column chromatography (eluent: DCM/MeOH (v/v) = 50/1) to obtain 595.0 mg of a yellow solid product with a yield of 55.6%.

MS (ESI, pos. ion) m/z: 821.4 $[M+H]^+$.

**Step 6: Synthesis of (1*r*,4*r*)-4-(((5-(2-chloro-3'-((2-(difluoromethyl)-7-(((*R*)-3-(difluoromethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

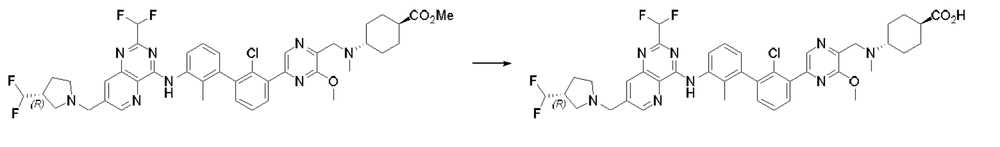

To a 100 mL single-necked bottle, methyl (1*r*,4*r*)-4-(((5-(2-chloro-3'-((2-(difluoromethyl)-7-(((*R*)-3-(difluoromethyl)pyrrolidin-1-yl)methyl)pyrido[3,2-*d*]pyrimidin-4-yl) amino)-2'-methyl-[1,1'-biphenyl]-3-yl)-3-methoxypyrazin-2-yl)methyl)(methyl)amino) cyclohexane-1-carboxylate (579 mg, 0.70 mmol), 1,4-dioxane (21 mL), water (7 mL) and lithium hydroxide monohydrate (58.74 mg, 1.4 mmol) were added in sequence and reacted at 50 °C. After the reaction was completed, the reaction mixture was concentrated under reduced pressure, DCM (10 mL) and MeOH (10 mL) were added, the pH was adjusted to 6~7 with dioxane hydrochloride solution (4.0 M), and the reaction mixture was concentrated under reduced pressure. The residue was separated and purified by silica gel column chromatography (eluent: DCM/MeOH (v/v) = 10/1) to obtain 490.0 mg of a yellow solid product with a yield of 86.1%.

MS (ESI, pos. ion) m/z: 807.31 $[M+H]^+$.

$^1$H NMR (400 MHz, DMSO-$d_6$) δ 10.36 (s, 1H), 8.94 (s, 1H), 8.47 (s, 1H), 8.17 (s, 1H), 7.73 - 7.67 (m, 2H), 7.59 (t, *J* = 7.0 Hz, 1H), 7.44 (d, *J* = 6.0 Hz, 1H), 7.38 (t, *J* = 7.3 Hz, 1H), 7.15 (d, *J* = 7.1 Hz, 1H), 6.68 (t, *J* = 54.5 Hz, 1H), 5.98 (t, *J* = 57.5 Hz, 1H), 3.97 (s, 3H), 3.90 (s, 2H), 3.87 (s, 2H), 2.89 - 2.78 (m, 1H), 2.70 - 2.59 (m, 2H), 2.24 - 2.14 (m, 2H), 2.08 - 1.88 (m, 10H), 1.77 - 1.65 (m, 1H), 1.50 - 1.27 (m, 8H).

**Example 25 (1*r*,4*r*)-4-(((2'-chloro-3''-((2-(difluoromethyl)-7-(((*R*)-3-hydroxypyrrolidin-1-yl)methyl)pyrido[3,2-*d*]pyrimidin-4-yl)amino)-3-fluoro-5-methoxy-2'-methyl-[1,1':3',1''-terphenyl]-4-yl)methyl)(methyl)amino)cyclohexane-1-carboxylic acid**

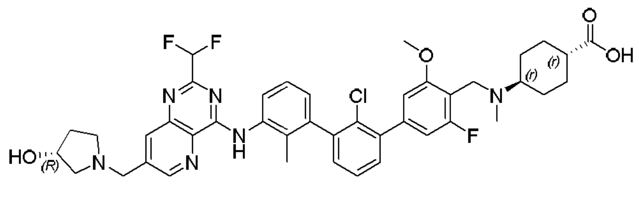

**Step 1: Synthesis of methyl (1*r*,4*r*)-4-(((3'-bromo-2'-chloro-3-fluoro-5-methoxy-[1,1'-biphenyl]-4-yl)methyl)amino)cyclohexane-1-carboxylate**

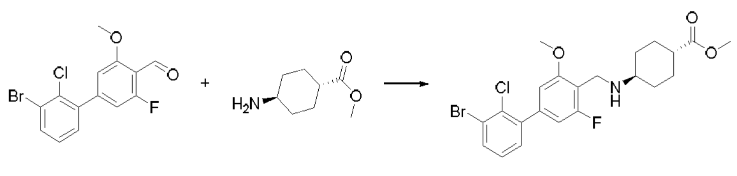

3'-Bromo-2'-chloro-3-fluoro-5-methoxy-[1,1'-biphenyl]-4-carbaldehyde (6.0 g, 17.46 mmol) was dissolved in a

mixed solvent of DCM (60 mL) and trifluoroethanol (60 mL), and methyl (1r,4r)-4-aminocyclohexane-1-carboxylate (4.73 g, 24.44 mmol) and TEA (0.88 g, 8.73 mmol) were added, and the mixture was heated and stirred at 60 °C for 2.5 h. After cooling to room temperature, sodium cyanoborohydride (1.10 g, 17.46 mmol) was added, and the mixture was stirred at room temperature for 50 min. After the reaction of the raw materials was complete, $K_2CO_3$ (3 g) was added to the system to quench the reaction, the reaction solution was concentrated under reduced pressure, and the residue was separated and purified by silica gel column chromatography (eluent: PE/EtOAc (v/v) = 4/1) to obtain 8.23 g of yellow liquid product with a yield of 97.2%.

MS (ESI, pos. ion) m/z: 484.3 $[M+H]^+$.

**Step 2: Synthesis of methyl (1*r*,4*r*)-4-(((3'-bromo-2'-chloro-3-fluoro-5-methoxy-[1,1'-biphenyl]-4-yl)methyl)(methyl)amino)cyclohexane-1-carboxylate**

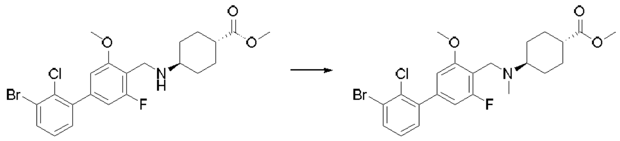

Methyl (1*r*,4*r*)-4-(((3'-bromo-2'-chloro-3-fluoro-5-methoxy-[1,1'-biphenyl]-4-yl)methyl)amino)cyclohexane-1-carboxylate (9.61 g, 19.82 mmol) was dissolved in methanol (100 mL), and then formaldehyde (16.09 g, 198.20 mmol) and acetic acid (0.60 g, 9.91 mmol) was added and stirred at room temperature overnight. Sodium cyanoborohydride (1.25 g, 19.82 mmol) was added and stirred at room temperature for 20 min. After the reaction of the raw materials was complete, $K_2CO_3$ (3 g) was added to the system to quench the reaction, the reaction solution was concentrated under reduced pressure, and the residue was separated and purified by silica gel column chromatography (eluent: PE/EtOAc (v/v) = 3/2) to obtain 9.82 g of yellow liquid product with a yield of 99.3%.

MS (ESI, pos. ion) m/z: 498.30 $[M+H]^+$.

**Step 3: Synthesis of methyl (1*r*,4*r*)-4-(((3"-amino-2'-chloro-3-fluoro-5-methoxy-2'-methyl-[1,1':3',1"-terphenyl]-4-yl)methyl)(methyl)amino)cyclohexane-1-carboxylate**

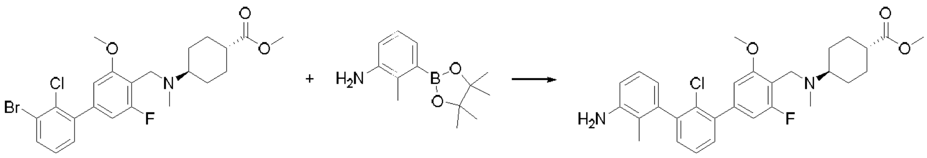

Methyl (1*r*,4*r*)-4-(((3'-bromo-2'-chloro-3-fluoro-5-methoxy-[1,1'-biphenyl]-4-yl)methyl)(methyl)amino)cyclohexane-1-carboxylate (4.90 g, 9.82 mmol), 2-methyl-3-(4,4,5,5-tetramethyl-1,3,2-dioxaborolan-2-yl)aniline (6.87 g, 29.46 mmol), [1,1'-bis(diphenylphosphino)ferrocene]palladium dichloride dichloromethane adduct (1.48 g, 1.96 mmol), and potassium carbonate (4.07 g, 29.46 mmol) were dissolved in a mixed solvent of 1,4-dioxane (120 mL) and water (24 mL) and heated at 90 °C under nitrogen protection for overnight reaction. After the reaction of the raw materials was complete, the reaction solution was concentrated under reduced pressure, and the residue was separated and purified by silica gel column chromatography (eluent: PE/EtOAc (v/v) = 3/2) to obtain 3.11 g of yellow liquid product with a yield of 60.3%.

MS (ESI, pos. ion) m/z: 525.5 $[M+H]^+$.

**Step 4: Synthesis of methyl (1*r*,4*r*)-4-(((3"-((7-bromo-2-(difluoromethyl)pyrido[3,2-*d*]pyrimidin-4-yl)amino)-2'-chloro-3-fluoro-5-methoxy-2"-methyl-[1,1':3',1"-terphenyl]-4-yl)methyl)(methyl)amino)cyclohexane-1-carboxylate**

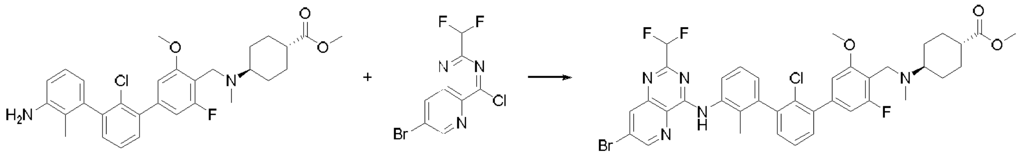

Methyl (1*r*,4*r*)-4-(((3"-amino-2'-chloro-3-fluoro-5-methoxy-2'-methyl-[1,1':3',1"-terphenyl]-4-yl)methyl)(methyl)amino)cyclohexane-1-carboxylate (2.85 g, 5.43 mmol) and 7-bromo-4-chloro-2-(difluoromethyl)pyrido[3,2-*d*]pyrimidine (1.92 g, 6.52 mmol) were added to *t*-BuOH (100 mL) and stirred at 100 °C for 5 h. After the reaction of the raw materials was complete, the reaction solution was concentrated under reduced pressure, and the residue was separated and purified by silica gel column chromatography (eluent: DCM/MeOH (v/v) = 30/1) to obtain 4.16 g of a yellow solid product with a yield of 97.9%.

MS (ESI, pos. ion) m/z: 782.0 $[M+H]^+$.

**Step 5: Synthesis of methyl (1*r*,4*r*)-4-(((2'-chloro-3"-((2-(difluoromethyl)-7-vinylpyrido[3,2-*d*]pyrimidin-4-yl)amino)-3-fluoro-5-methoxy-2"-methyl-[1,1':3',1"-terphenyl]-4-yl)methyl) (methyl)amino)cyclohexane-1-carboxylate**

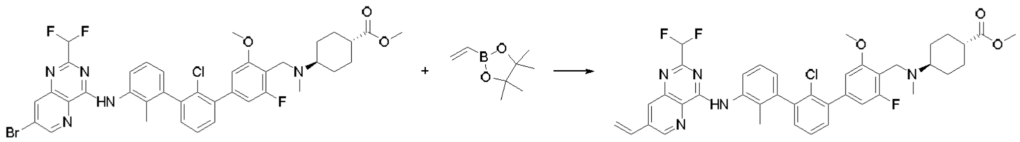

Methyl (1*r*,4*r*)-4-(((3"-((7-bromo-2-(difluoromethyl)pyrido[3,2-*d*]pyrimidin-4-yl)amino)-2'-chloro-3-fluoro-5-methoxy-2"-methyl-[1,1':3',1"-terphenyl]-4-yl)methyl)(methyl) amino)cyclohexane-1-carboxylate (4.60 g, 5.87 mmol), 2-vinyl-4,4,5,5-tetramethyl-1,3,2-dioxaborolane (4.52 g, 29.35 mmol), [1,1'-bis(diphenylphosphino)ferrocene]palladium dichloride dichloromethane adduct (1.68 g, 2.05 mmol), and potassium phosphate (3.74 g, 17.61 mmol) were dissolved in a mixed solvent of 1,4-dioxane (100 mL) and water (20 mL) and heated at 100 °C under nitrogen protection for 5 h. After the reaction of the raw materials was complete, the reaction solution was concentrated under reduced pressure, and the residue was separated and purified by silica gel column chromatography (eluent: PE/EtOAc (v/v) = 1/1) to obtain 3.2 g of yellow solid product with a yield of 74.6%.

MS (ESI, pos. ion) m/z: 730.6 $[M+H]^+$.

**Step 6: Synthesis of methyl (1*r*,4*r*)-4-(((2'-chloro-3'-((2-(difluoromethyl)-7-formylpyrido[3,2-*d*]pyrimidin-4-yl) amino)-3-fluoro-5-methoxy-2'-methyl-[1,1':3',1"-terphenyl]-4-yl)methyl) (methyl)amino)cyclohexane-1-carboxylate**

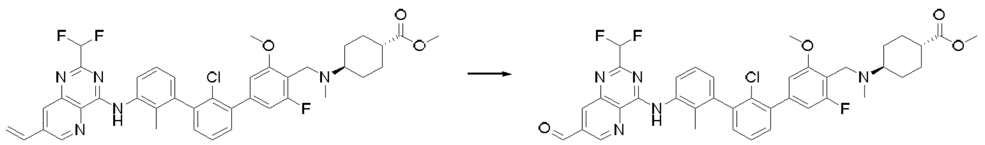

Methyl (1*r*,4*r*)-4-(((2'-chloro-3"-((2-(difluoromethyl)-7-vinylpyrido[3,2-*d*]pyrimidin-4-yl)amino)-3-fluoro-5-methoxy-2"-methyl-[1,1':3',1"-terphenyl]-4-yl)methyl)(methyl)amino) cyclohexane-1-carboxylate (3.00 g, 4.11 mmol) was dissolved in a mixed solvent of 1,4-dioxane (100 mL) and water (40 mL), then potassium osmate dihydrate (0.15 g, 0.41 mmol) was added, followed by sodium periodate (4.40 g, 20.55 mmol), and the mixture was reacted at room temperature for 5 h. After the reaction of the raw materials was complete, the reaction was quenched with saturated aqueous sodium sulfite solution (20 mL), concentrated under reduced pressure to remove the organic solvent, added with water (300 mL), and then extracted with DCM (70 mL × 3). The organic phases were combined, washed with water (200 mL), dried over anhydrous sodium sulfate, filtered, and the filtrate was concentrated under reduced pressure. The residue was separated and purified by silica gel column chromatography (eluent: PE/EtOAc (v/v) = 2/3) to obtain 0.33 g of a yellow solid product with a yield of 10.97%.

MS (ESI, pos. ion) m/z: 732.3 $[M+H]^+$.

**Step 7: Synthesis of methyl (1*r*,4*r*)-4-(((2'-chloro-3'-((2-(difluoromethyl)-7-(((*R*)-3-hydroxypyrrolidin-1-yl) methyl)pyrido[3,2-*d*]pyrimidin-4-yl)amino)-3-fluoro-5-methoxy-2'-methyl-[1,1':3',1"-terphenyl]-4-yl)methyl) (methyl)amino)cyclohexane-1-carboxylate**

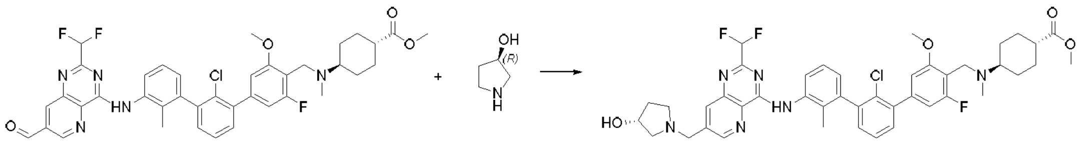

Methyl (1*r*,4*r*)-4-(((2'-chloro-3"-((2-(difluoromethyl)-7-formylpyrido[3,2-*d*]pyrimidin-4-yl)amino)-3-fluoro-5-methoxy-2"-methyl-[1,1':3',1"-terphenyl]-4-yl)methyl)(methyl)amino) cyclohexane-1-carboxylate (0.33 g, 0.45 mmol) was dissolved in a mixed solvent of DCM (15 mL) and MeOH (15 mL), (*R*)-pyrrolidin-3-ol (0.2 g, 2.25 mmol) was added, and the mixture was stirred at room temperature overnight. Sodium cyanoborohydride (0.057 g, 0.9 mmol) was added and stirred at room temperature for 35 min. After the reaction of the raw materials was complete, $K_2CO_3$ (0.3 g) was added to the system to quench the reaction, the reaction solution was concentrated under reduced pressure, and the residue was separated and purified by silica gel column chromatography (eluent: DCM/MeOH (v/v) = 20/1) to obtain 0.35 g of yellow liquid product with a yield of 96.67%.

MS (ESI, pos. ion) m/z: 803.3 $[M+H]^+$.

**Step 8: Synthesis of (1*r*,4*r*)-4-(((2'-chloro-3"-((2-(difluoromethyl)-7-(((*R*)-3-hydroxypyrrolidin-1-yl)methyl)pyrido [3,2-*d*]pyrimidin-4-yl)amino)-3-fluoro-5-methoxy-2"-methyl-[1,1':3',1"-terphenyl]-4-yl)methyl)(methyl)amino) cyclohexane-1-carboxylic acid**

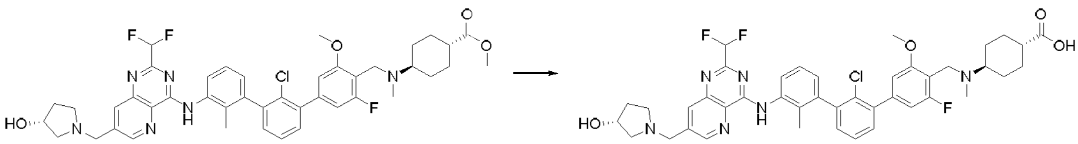

Methyl (1*r*,4*r*)-4-(((2'-chloro-3"-((2-(difluoromethyl)-7-(((*R*)-3-hydroxypyrrolidin-1-yl)methyl)pyrido[3,2-*d*]pyrimidin-4-yl)amino)-3-fluoro-5-methoxy-2"-methyl-[1,1':3',1"-terphenyl]-4-yl)methyl)(methyl)amino)cyclohexane-1-carboxylate (0.35 g, 0.44 mmol) was dissolved in a mixed solution of 1,4-dioxane (15 mL) and water (3 mL), lithium hydroxide monohydrate (0.18 g, 4.4 mmol) was added, and the reaction was stirred at room temperature overnight. After the reaction of the raw materials was complete, the pH of the system was adjusted to about 5 with acetic acid, the reaction solution was concentrated under reduced pressure, and the residue was separated and purified by column chromatography (eluent: DCM/MeOH (v/v) = 7/1) to obtain 85 mg of a yellow solid product with a yield of 24.72%.

MS (ESI, pos. ion) m/z: 789.31 $[M+H]^+$.

$^1H$ NMR (400 MHz, DMSO-$d_6$) δ 10.32 (s, 1H), 8.94 (s, 1H), 8.16 (s, 1H), 7.74 (d, *J* = 8.0 Hz, 1H), 7.54 - 7.47 (m, 2H), 7.40 - 7.31 (m, 2H), 7.14 (d, *J* = 7.6 Hz, 1H), 6.92 - 6.85 (m, 2H), 6.70 (t, *J* = 54.5 Hz, 1H), 4.26 - 4.19 (m, 1H), 3.93 - 3.79 (m, 7H), 3.57 - 3.56 (m, 3H), 2.76 - 2.71 (m, 1H), 2.68 (d, *J* = 7.8 Hz, 1H), 2.43 - 2.37 (m, 2H), 2.12 (s, 3H), 2.04 (s, 3H), 1.96 - 1.90 (m, 2H), 1.85 - 1.80 (m, 2H), 1.62 - 1.54 (m, 1H), 1.35 - 1.25 (m, 5H).

**Example 26 (1*r*,4*r*)-4-(((5-(3'-((2-(difluoromethyl)-7-(((*R*)-3-hydroxypyrrolidin-1-yl)methyl) pyrido[3,2-*d*]pyrimidin-4-yl)amino)-2-fluoro-2'-methyl-[1,1'-biphenyl]-3-yl)-3-methoxy pyrazin-2-yl)methyl)(methyl)amino)cyclohexane-1-carboxylic acid**

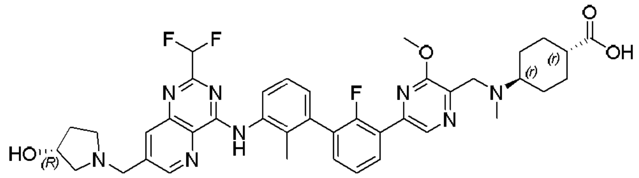

**Step 1: Synthesis of methyl (1*r*,4*r*)-4-(((5-(3-bromo-2-fluorophenyl)-3-methoxypyrazin-2-yl)methyl)amino)cyclohexane-1-carboxylate**

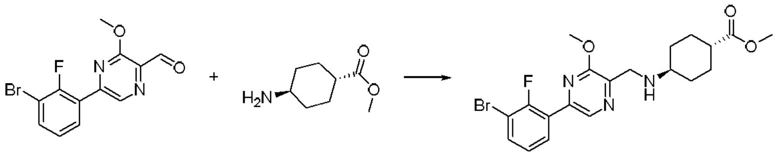

5-(3-Bromo-2-fluorophenyl)-3-methoxypyrazine-2-carbaldehyde (8.00 g, 25.71 mmol) was dissolved in a mixed solvent of DCM (60 mL) and trifluoroethanol (60 mL), and methyl (1*r*,4*r*)-4-aminocyclohexane-1-carboxylate (7.47 g, 38.56 mmol) and TEA (1.30 g, 12.86 mmol) were added, and the mixture was heated and stirred at 60 °C for 1 h. After cooling to room temperature, sodium cyanoborohydride (1.62 g, 25.71 mmol) was added, and the mixture was stirred at room temperature for 1 h. After the reaction of the raw materials was complete, $K_2CO_3$ (3 g) was added to the system to quench the reaction, the reaction solution was concentrated under reduced pressure, and the residue was separated and purified by silica gel column chromatography (eluent: PE/EtOAc (v/v) = 2/1) to obtain 11.63 g of yellow liquid product with a yield of 99.99%.

MS (ESI, pos. ion) m/z: 452.4 $[M+H]^+$.

**Step 2: Synthesis of methyl (1*r*,4*r*)-4-(((5-(3-bromo-2-fluorophenyl)-3-methoxypyrazin-2-yl)methyl)(methyl)amino)cyclohexane-1-carboxylate**

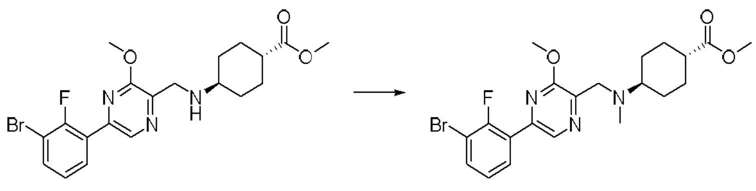

Methyl (1*r*,4*r*)-4-(((5-(3-bromo-2-fluorophenyl)-3-methoxypyrazin-2-yl)methyl)amino)cyclohexane-1-carboxylate (11.63 g, 25.71 mmol) was dissolved in methanol (100 mL), and then formaldehyde (20.87 g, 257.10 mmol) and acetic acid (0.77 g, 12.86 mmol) was added and stirred at room temperature overnight. Sodium cyanoborohydride (1.62 g, 25.71 mmol) was added and stirred at room temperature for 20 min. After the reaction of the raw materials was complete, $K_2CO_3$ (4 g) was added to the system to quench the reaction, the reaction solution was concentrated under reduced pressure, and the residue was separated and purified by silica gel column chromatography (eluent: PE/EtOAc (v/v) = 3/2) to obtain 11.98 g of yellow liquid product with a yield of 99.9%.

MS (ESI, pos. ion) m/z: 466.2 $[M+H]^+$.

**Step 3: Synthesis of methyl (1*r*,4*r*)-4-(((5-(3'-amino-2-fluoro-2'-methyl-[1,1'-biphenyl]-3-yl)-3-methoxypyrazin-2-yl)methyl)(methyl)amino)cyclohexane-1-carboxylate**

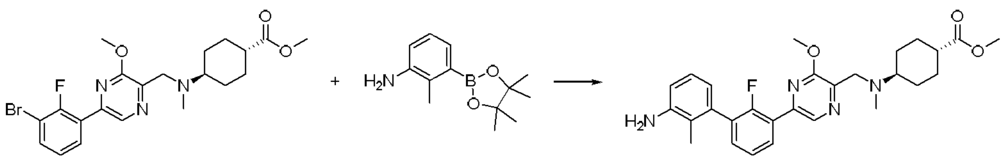

Methyl (1*r*,4*r*)-4-(((5-(3-bromo-2-fluorophenyl)-3-methoxypyrazin-2-yl)methyl)(methyl)amino)cyclohexane-1-carboxylate (7.00 g, 15.01 mmol), 2-methyl-3-(4,4,5,5-tetramethyl-1,3,2-dioxaborolan-2-yl)aniline (7.00 g, 30.02 mmol), [1,1'-bis(diphenylphosphino)ferrocene]palladium dichloride dichloromethane adduct (2.27 g, 3.00 mmol), and potassium carbonate (6.22 g, 45.03 mmol) were dissolved in a mixed solvent of 1,4-dioxane (120 mL) and water (24 mL) and heated at 90 °C under nitrogen protection for overnight reaction. After the reaction of the raw materials was complete, the reaction

solution was concentrated under reduced pressure, and the residue was separated and purified by silica gel column chromatography (eluent: PE/EtOAc (v/v) = 3/2) to obtain 5.33 g of yellow liquid product with a yield of 72.09%.

MS (ESI, pos. ion) m/z: 493.3 $[M+H]^+$.

**Step 4: Synthesis of methyl (1*r*,4*r*)-4-(((5-(3'-((7-bromo-2-(difluoromethyl)pyrido[3,2-*d*]pyrimidin-4-yl)amino)-2-fluoro-2'-methyl-[1,1'-biphenyl]-3-yl)-3-methoxypyrazin-2-yl) methyl)(methyl)amino)cyclohexane-1-carboxylate**

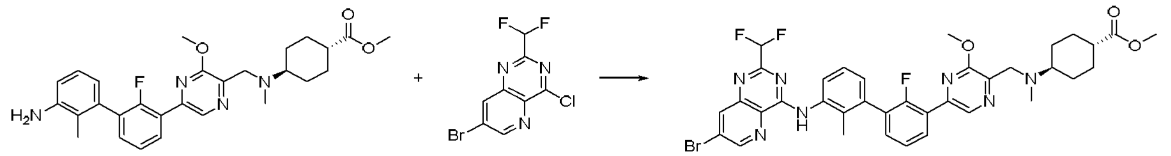

Methyl (1r,4r)-4-(((5-(3'-amino-2-fluoro-2'-methyl-[1,1'-biphenyl]-3-yl)-3-methoxypyrazin-2-yl)methyl)(methyl) amino)cyclohexane-1-carboxylate (2.00 g, 4.06 mmol) and 7-bromo-4-chloro-2-(difluoromethyl)pyrido[3,2-d]pyrimidine (1.43 g, 4.87 mmol) were added to *t*-BuOH (80 mL) and stirred at 100 °C for 6 h. After the reaction of the raw materials was complete, the reaction solution was concentrated under reduced pressure, and the residue was separated and purified by silica gel column chromatography (eluent: DCM/MeOH (v/v) = 30/1) to obtain 2.86 g of a yellow solid product with a yield of 93.9%.

MS (ESI, pos. ion) m/z: 750.1 $[M+H]^+$.

**Step 5: Synthesis of methyl (1*r*,4*r*)-4-(((5-(3'-((2-(difluoromethyl)-7-vinylpyrido[3,2-*d*]pyrimidin-4-yl)amino)-2-fluoro-2'-methyl-[1,1'-biphenyl]-3-yl)-3-methoxypyrazin-2-yl) methyl)(methyl)amino)cyclohexane-1-carboxylate**

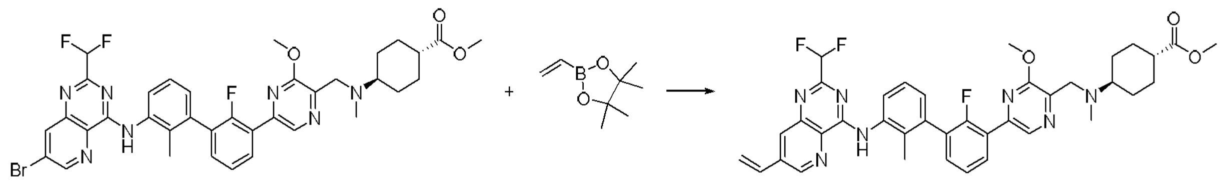

Methyl (1*r*,4*r*)-4-(((5-(3'-((7-bromo-2-(difluoromethyl)pyrido[3,2-*d*]pyrimidin-4-yl)amino)-2-fluoro-2'-methyl-[1,1'-biphenyl]-3-yl)-3-methoxypyrazin-2-yl)methyl)(methyl)amino) cyclohexane-1-carboxylate (2.86 g, 3.81 mmol), 4,4,5,5-tetramethyl-2-vinyl-1,3,2-dioxaborolane (2.93 g, 19.05 mmol), [1,1'-bis(diphenylphosphino)ferrocene] palladium dichloride dichloromethane adduct (1.09 g, 1.33 mmol), and potassium phosphate (1.62 g, 7.62 mmol) were dissolved in a mixed solvent of 1,4-dioxane (90 mL) and water (18 mL) and heated at 100 °C under nitrogen protection for 5 h. After the reaction of the raw materials was complete, the reaction solution was concentrated under reduced pressure, and the residue was separated and purified by silica gel column chromatography (eluent: PE/EtOAc (v/v) = 1/1) to obtain 2.42 g of yellow solid product with a yield of 91.03%.

MS (ESI, pos. ion) m/z: 698.3 $[M+H]^+$.

**Step 6: Synthesis of methyl (1*r*,4*r*)-4-(((5-(3'-((2-(difluoromethyl)-7-formylpyrido[3,2-*d*]pyrimidin-4-yl)amino)-2-fluoro-2'-methyl-[1,1'-biphenyl]-3-yl)-3-methoxypyrazin-2-yl) methyl)(methyl)amino)cyclohexane-1-carboxylate**

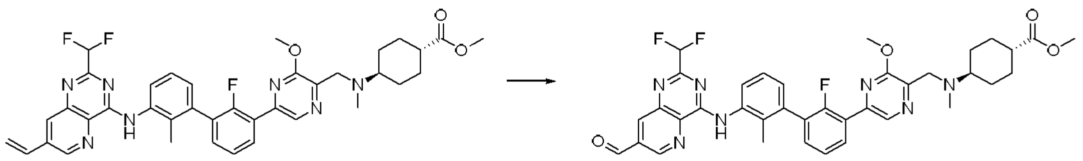

Methyl (1*r*,4*r*)-4-(((5-(3'-((2-(difluoromethyl)-7-vinylpyrido[3,2-*d*]pyrimidin-4-yl)amino)-2-fluoro-2'-methyl-[1,1'-biphenyl]-3-yl)-3-methoxypyrazin-2-yl)methyl)(methyl)amino) cyclohexane-1-carboxylate (2.46 g, 3.53 mmol) was dissolved in a mixed solvent of 1,4-dioxane (100 mL) and water (40 mL), then potassium osmate dihydrate (0.13 g, 0.35 mmol) was added, followed by sodium periodate (3.78 g, 17.65 mmol), and the mixture was reacted at room temperature for 5 h. After the reaction of the raw materials was complete, the reaction was quenched with saturated aqueous sodium sulfite solution (20 mL), concentrated under reduced pressure to remove the organic solvent, added with water (300 mL), and then extracted with DCM (70 mL × 3). The organic phases were combined, washed with water (200 mL × 1), dried over anhydrous sodium sulfate, filtered, and the filtrate was concentrated under reduced pressure. The residue was separated and purified by silica gel column chromatography (eluent: PE/EtOAc (v/v) = 2/3) to obtain 0.27 g of a yellow solid product with a yield of 10.94%.

MS (ESI, pos. ion) m/z: 700.3 $[M+H]^+$.

Step 7: **Synthesis of methyl (1*r*,4*r*)-4-(((5-(3'-((2-(difluoromethyl)-7-(((R)-3-hydroxypyrrolidin-1-yl)methyl)pyrido [3,2-*d*]pyrimidin-4-yl)amino)-2-fluoro-2'-methyl-[1,1'-biphenyl]-3-yl)-3-methoxypyrazin-2-yl)methyl)(methyl) amino)cyclohexane-1-carboxylate**

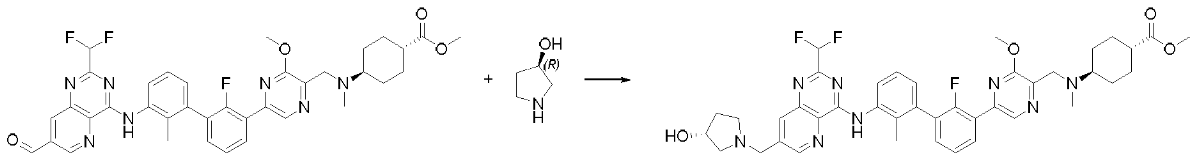

Methyl (1*r*,4*r*)-4-(((5-(3'-((2-(difluoromethyl)-7-formylpyrido[3,2-*d*]pyrimidin-4-yl)amino)-2-fluoro-2'-methyl-[1,1'-biphenyl]-3-yl)-3-methoxypyrazin-2-yl)methyl)(methyl)amino) cyclohexane-1-carboxylate (0.27 g, 0.39 mmol) was dissolved in a mixed solvent of DCM (15 mL) and MeOH (15 mL), (R)-pyrrolidin-3-ol (0.17 g, 1.95 mmol) was added, and the mixture was stirred at room temperature overnight. Sodium cyanoborohydride (0.049 g, 0.78 mmol) was added and stirred at room temperature for 35 min. After the reaction of the raw materials was complete, $K_2CO_3$ (0.3 g) was added to the system to quench the reaction, the reaction solution was concentrated under reduced pressure, and the residue was separated and purified by silica gel column chromatography (eluent: DCM/MeOH (v/v) = 20/1) to obtain 0.29 g of yellow liquid product with a yield of 97.5%.

MS (ESI, pos. ion) m/z: 386.4 (1/2$[M+H]^-$).

**Step 8: Synthesis of (1*r*,4*r*)-4-(((5-(3'-((2-(difluoromethyl)-7-(((*R*)-3-hydroxypyrrolidin-1-yl)methyl)pyrido[3,2-*d*] pyrimidin-4-yl)amino)-2-fluoro-2'-methyl-[1,1'-biphenyl]-3-yl)-3-methoxypyrazin-2-yl)methyl)(methyl)amino)cyclohexane-1-carboxylic acid**

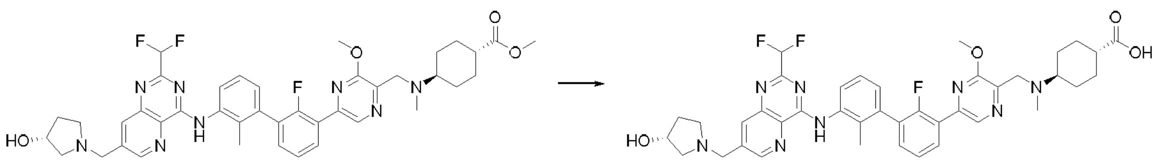

Methyl (1*r*,4*r*)-4-(((5-(3'-((2-(difluoromethyl)-7-(((*R*)-3-hydroxypyrrolidin-1-yl)methyl)pyrido[3,2-*d*]pyrimidin-4-yl)amino)-2-fluoro-2'-methyl-[1,1'-biphenyl]-3-yl)-3-methoxy pyrazin-2-yl)methyl)(methyl)amino)cyclohexane-1-carboxylate (0.29 g, 0.38 mmol) was dissolved in a mixed solution of 1,4-dioxane (12.5 mL) and water (2.5 mL), lithium hydroxide monohydrate (0.16 g, 3.8 mmol) was added, and the reaction was stirred at room temperature overnight. After the reaction of the raw materials was complete, the pH of the system was adjusted to about 5 with acetic acid, the reaction solution was concentrated under reduced pressure, and the residue was separated and purified by column chromatography (eluent: DCM/MeOH (v/v) = 7/1) to obtain 45.6 mg of a yellow solid product with a yield of 16.02%.

MS (ESI, pos. ion) m/z: 757.34 $[M+H]^+$.

$^1$H NMR (400 MHz, DMSO-$d_6$) δ 10.37 (s, 1H), 8.95 (s, 1H), 8.55 (s, 1H), 8.17 (s, 1H), 8.07 (t, *J* = 7.3 Hz, 1H), 7.74 (d, *J* = 8.0 Hz, 1H), 7.50 - 7.44 (m, 2H), 7.41 (t, *J* = 7.8 Hz, 1H), 7.26 (d, *J* = 7.6 Hz, 1H), 6.72 (t, *J* = 109.0 Hz, 1H), 4.24 - 4.21 (m, 1H), 4.01 (s, 3H), 3.90 (d, *J* = 13.8 Hz, 1H), 3.83 (d, *J* = 13.9 Hz, 1H), 3.69 (s, 2H), 2.75 - 2.72 (m, 1H), 2.68 (d, *J* = 7.7 Hz, 1H), 2.63 - 2.60 (m, 1H), 2.45 - 2.38 (m, 4H), 2.19 (s, 3H), 2.11 (s, 3H), 2.09 - 2.07 (m, 1H), 2.06 - 1.99 (m, 2H), 1.96 - 1.93 (m,

2H), 1.87 - 1.82 (m, 2H), 1.61 - 1.56 (m, 1H), 1.32 - 1.30 (m, 2H).

**Example 27 (1*r*,4*r*)-4-(((5-(3'-((2-(difluoromethyl)-7-(((*R*)-3-hydroxypyrrolidin-1-yl)methyl)pyrido[3,2-*d*]pyrimidin-4-yl)amino)-2,2'-dimethyl-[1,1'-biphenyl]-3-yl)-3-methoxy pyrazin-2-yl)methyl)(methyl)amino)cyclohexane-1-carboxylic acid**

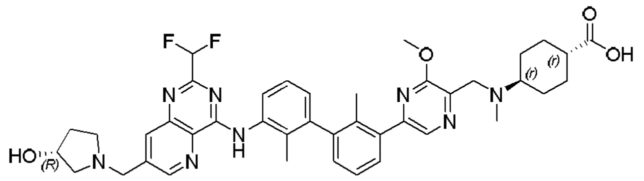

**Step 1: Synthesis of 5-(3-bromo-2-methylphenyl)-3-methoxypyrazine-2-carbaldehyde**

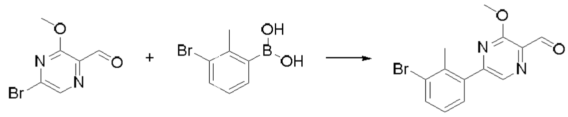

5-Bromo-3-methoxypyrazine-2-carbaldehyde (10.00 g, 46.08 mmol), (3-bromo-2-methylphenyl)boronic acid (10.89 g, 50.69 mmol), $Pd(PPh_5)Cl_2$ (0.97 g, 1.38 mmol), tri-*o*-tolylphosphine (0.42 g, 1.38 mmol), potassium carbonate (9.55 g, 69.12 mmol) were dissolved in a mixed solvent of 1,4-dioxane (80 mL) and water (20 mL), and the resulting solution was protected with nitrogen and heated to 65 °C for overnight. Water (300 mL) was added to the system to precipitate a large amount of solid, which was stirred at room temperature for 2 h, filtered, and the filter cake was washed with ethanol (30 mL × 3) to obtain 11.66 g of a yellow solid product with a yield of 82.4%.
MS (ESI, pos. ion) m/z: 307.1 $[M+H]^+$.

**Step 2: Synthesis of methyl (1*r*,4*r*)-4-(((5-(3-bromo-2-methylphenyl)-3-methoxypyrazin-2-yl)methyl)amino)cyclohexane-1-carboxylate**

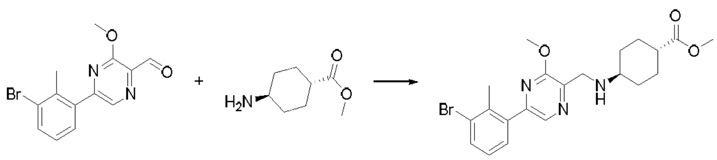

5-(3-Bromo-2-methylphenyl)-3-methoxypyrazine-2-carbaldehyde (3.00 g, 9.77 mmol) was dissolved in a mixed solvent of DCM (30 mL) and trifluoroethanol (30 mL), and methyl (1*r*,4*r*)-4-aminocyclohexane-1-carboxylate (2.84 g, 14.65 mmol) and TEA (0.49 g, 4.88 mmol) were added, and the mixture was heated and stirred at 60 °C for 2 h. After cooling to room temperature, sodium cyanoborohydride (0.61 g, 9.77 mmol) was added, and the mixture was stirred at room temperature for 0.5 hours. After the reaction of the raw materials was complete, $K_2CO_3$ (2 g) was added to the system to quench the reaction, the reaction solution was concentrated under reduced pressure, and the residue was separated and purified by silica gel column chromatography (eluent: PE/EtOAc (v/v) = 4/1) to obtain 4.37 g of yellow solid product with a yield of 99.79%.
MS (ESI, pos. ion) m/z: 448.2 $[M+H]^+$.

**Step 3: Synthesis of methyl (1*r*,4*r*)-4-(((5-(3-bromo-2-methylphenyl)-3-methoxypyrazin-2-yl)methyl)(methyl)amino)cyclohexane-1-carboxylate**

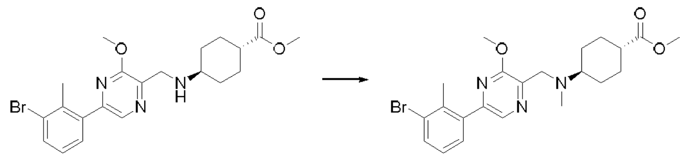

Methyl (1*r*,4*r*)-4-(((5-(3-bromo-2-methylphenyl)-3-methoxypyrazin-2-yl)methyl)amino)cyclohexane-1-carboxylate (4.37 g, 9.75 mmol) was dissolved in methanol (80 mL), and then formaldehyde (3.96 g, 48.75 mmol) and acetic acid (0.29 g, 4.88 mmol) was added and stirred at room temperature overnight. Sodium cyanoborohydride (0.61 g, 9.75 mmol) was added and stirred at room temperature for 40 min. After the reaction of the raw materials was complete, $K_2CO_3$ (2 g) was added to the system to quench the reaction, the reaction solution was concentrated under reduced pressure, and the residue was separated and purified by silica gel column chromatography (eluent: PE/EtOAc (v/v) = 3/2) to obtain 3.23 g of yellow liquid product with a yield of 71.7%.

MS (ESI, pos. ion) m/z: 462.1 $[M+H]^+$.

**Step 4: Synthesis of methyl (1*r*,4*r*)-4-(((5-(3'-amino-2,2'-dimethyl-[1,1'-biphenyl]-3-yl)-3-methoxypyrazin-2-yl)methyl)(methyl)amino)cyclohexane-1-carboxylate**

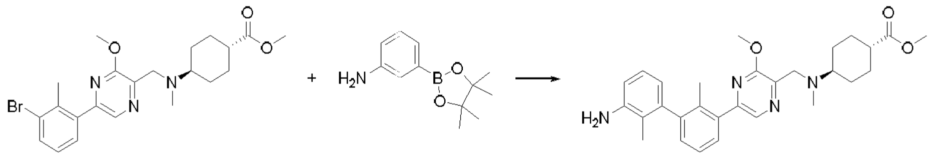

Methyl (1*r*,4*r*)-4-(((5-(3-bromo-2-methylphenyl)-3-methoxypyrazin-2-yl)methyl)(methyl)amino)cyclohexane-1-carboxylate (3.23 g, 6.99 mmol), 2-methyl-3-(4,4,5,5-tetramethyl-1,2-dioxaborolan-2-yl)aniline (3.26 g, 13.98 mmol), [1,1'-bis(diphenylphosphino)ferrocene]palladium dichloride dichloromethane adduct (0.53 g, 0.70 mmol), and potassium carbonate (2.90 g, 20.97 mmol) were dissolved in a mixed solvent of 1,4-dioxane (90 mL) and water (18 mL) and heated at 90 °C under nitrogen protection for overnight reaction. After the reaction of the raw materials was complete, the reaction solution was concentrated under reduced pressure, and the residue was separated and purified by silica gel column chromatography (eluent: PE/EtOAc (v/v) = 3/2) to obtain 2.64 g of yellow liquid product with a yield of 77.34%.

MS (ESI, pos. ion) m/z: 489.3 $[M+H]^+$.

**Step 5: Synthesis of methyl (1*r*,4*r*)-4-(((5-(3'-((7-bromo-2-(difluoromethyl)pyrido[3,2-*d*]pyrimidin-4-yl)amino)-2,2'-dimethyl-[1,1'-biphenyl]-3-yl)-3-methoxypyrazin-2-yl)methyl) (methyl)amino)cyclohexane-1-carboxylate**

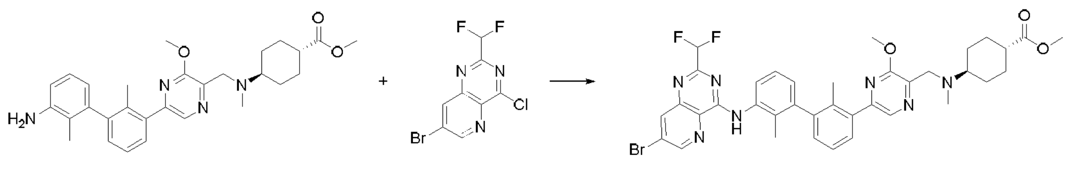

Methyl (1*r*,4*r*)-4-(((5-(3'-amino-2,2'-dimethyl-[1,1'-biphenyl]-3-yl)-3-methoxypyrazin-2-yl)methyl)(methyl)amino)cyclohexane-1-carboxylate (2.64 g, 5.40 mmol) and 7-bromo-4-chloro-2-(difluoromethyl)pyrido[3,2-*d*]pyrimidine (1.91 g, 6.48 mmol) were added to *t*-BuOH (80 mL) and stirred at 100 °C overnight. After the reaction of the raw materials was complete, the reaction solution was concentrated under reduced pressure, and the residue was separated and purified by silica gel column chromatography (eluent: DCM/MeOH (v/v) = 30/1) to obtain 2.90 g of a yellow solid product with a yield of 71.89%.

MS (ESI, pos. ion) m/z: 746.3 $[M+H]^+$.

**Step 6: Synthesis of methyl (1*r*,4*r*)-4-(((5-(3'-((2-(difluoromethyl)-7-(((*R*)-3-hydroxypyrrolidin-1-yl)methyl)pyrido [3,2-*d*]pyrimidin-4-yl)amino)-2,2'-dimethyl-[1,1'-biphenyl]-3-yl)-3-methoxy pyrazin-2-yl)methyl)(methyl)amino) cyclohexane-1-carboxylate**

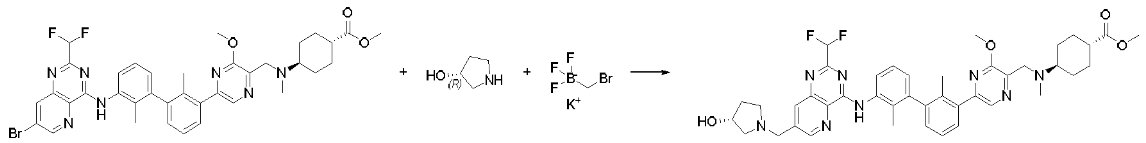

(*R*)-Pyrrolidin-3-ol (0.35 g, 4.02 mmol), potassium (bromomethyl)trifluoroborate (0.81 g, 4.02 mmol), and sodium carbonate (0.21 g, 2.01 mmol) were dissolved in acetonitrile (10 mL), heated at 80 °C for 5.5 h, and the system was concentrated. Then, methyl (1*r*,4*r*)-4-(((5-(3'-((7-bromo-2-(difluoromethyl)pyrido[3,2-*d*]pyrimidin-4-yl)amino)-2,2'-dimethyl-[1,1'-biphenyl]-3-yl)-3-methoxypyrazin-2-yl)methyl)(methyl)amino)cyclohexane-1-carboxylate (1.00 g, 1.34 mmol), palladium acetate (0.03 g, 0.13 mmol), X-Phos (0.13 g, 0.27 mmol), potassium carbonate (0.56 g, 4.02 mmol), 1,4-dioxane (25 mL) and water (5 mL) were added, the mixture was heated to 110 °C overnight under $N_2$. After the reaction of the raw materials was complete, the reaction solution was concentrated under reduced pressure, and the residue was separated and purified by silica gel column chromatography (eluent: DCM/MeOH (v/v) = 20/1) to obtain 0.50 g of a yellow solid product with a yield of 48.68%.

MS (ESI, pos. ion) m/z: 767.4 $[M+H]^+$.

**Step 7: Synthesis of (1*r*,4*r*)-4-(((5-(3'-((2-(difluoromethyl)-7-(((*R*)-3-hydroxypyrrolidin-1-yl)methyl)pyrido[3,2-*d*] pyrimidin-4-yl)amino)-2,2'-dimethyl-[1,1'-biphenyl]-3-yl)-3-methoxy pyrazin-2-yl)methyl)(methyl)amino)cyclohexane-1-carboxylic acid**

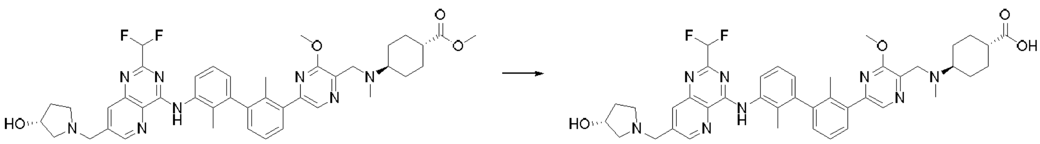

Methyl (1*r*,4*r*)-4-(((5-(3'-((2-(difluoromethyl)-7-(((*R*)-3-hydroxypyrrolidin-1-yl)methyl)pyrido[3,2-*d*]pyrimidin-4-yl)amino)-2,2'-dimethyl-[1,1'-biphenyl]-3-yl)-3-methoxy pyrazin-2-yl)methyl)(methyl)amino)cyclohexane-1-carboxylate (0.50 g, 0.65 mmol) was dissolved in a mixed solution of 1,4-dioxane (20 mL) and water (4 mL), lithium hydroxide monohydrate (0.14 g, 3.25 mmol) was added, and the reaction was stirred at room temperature overnight. After the reaction of the raw materials was complete, the pH of the system was adjusted to about 5 with acetic acid, the reaction solution was concentrated under reduced pressure, and the residue was separated and purified by column chromatography (eluent: DCM/MeOH (v/v) = 7/1) to obtain 480 mg of a yellow solid product with a yield of 97.79%.

MS (ESI, pos. ion) m/z: 753.36 $[M+H]^+$.

$^1$H NMR (400 MHz, DMSO-$d_6$) δ 10.41 (s, 1H), 8.95 (d, *J*= 2.0 Hz, 1H), 8.31 (s, 1H), 8.17 (d, *J*= *1.9* Hz, 1H), 7.68 (dd, *J* = 7.9, 1.4 Hz, 1H), 7.51 (dd, *J*= 7.8, 1.5 Hz, 1H), 7.39 (dt, *J*= 16.2, 7.7 Hz, 2H), 7.25 (dd, *J* = 7.6, 1.5 Hz, 1H), 7.12 (dd, *J* = 7.6, 1.4 Hz, 1H), 6.70 (t, *J*= 54.6 Hz, 1H), 4.24 - 4.22 (m, 1H), 3.94 (s, 3H), 3.89 (s, 1H), 3.85 (s, 1H), 3.69 (s, 2H), 3.18 - 3.17 (m, 1H), 2.77 - 2.65 (m, 2H), 2.48 - 2.39 (m, 3H), 2.21 (s, 3H), 2.10 (s, 3H), 2.08 - 2.00 (m, 1H), 1.98 (s, 3H), 1.95 - 1.89 (m, 3H), 1.86 - 1.80 (m, 2H), 1.63 - 1.55 (m, 1H), 1.35 - 1.26 (m, 4H).

**Example 28 (1*r*,4*r*)-4-(((5-(2-chloro-3'-((2-(difluoromethyl)-7-(((*S*)-3-(hydroxymethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino) cyclohexane-1-carboxylic acid**

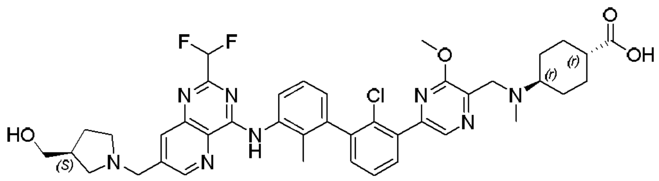

**Step 1: Synthesis of (1*r*,4*r*)-4-(((5-(2-chloro-3'-((2-(difluoromethyl)-7-(((*S*)-3-(hydroxymethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

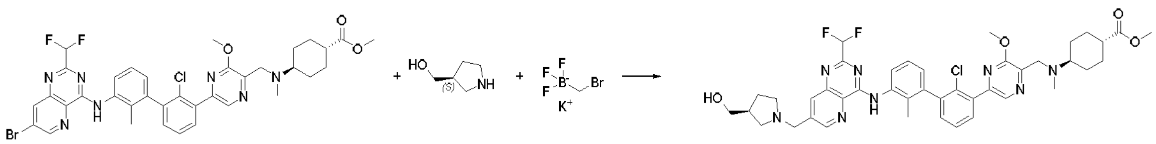

(*S*)-Pyrrolidin-3-ol (0.39 g, 3.90 mmol), potassium (bromomethyl)trifluoroborate (0.78 g, 3.90 mmol), and sodium carbonate (0.21 g, 1.95 mmol) were dissolved in acetonitrile (10 mL), heated at 80 °C for 5 h, and the system was concentrated. Then, methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclohexane-1-carboxylate (1.00 g, 1.30 mmol), palladium acetate (0.029 g, 0.13 mmol), X-Phos (0.12 g, 0.26 mmol), potassium carbonate (0.54 g, 3.90 mmol), 1,4-dioxane (25 mL) and water (5 mL) were added, the mixture was heated to 110 °C overnight under $N_2$. The reaction solution was concentrated under reduced pressure, and the residue was separated and purified by column chromatography (eluent: DCM/MeOH (v/v) = 20/1) to obtain 0.23 g of a yellow solid product with a yield of 22.02%.

MS (ESI, pos. ion) m/z: 401.3 (1/2[M+H]$^-$).

**Step 2: Synthesis of (1*r*,4*r*)-4-(((5-(2-chloro-3'-((2-(difluoromethyl)-7-(((*S*)-3-(hydroxymethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

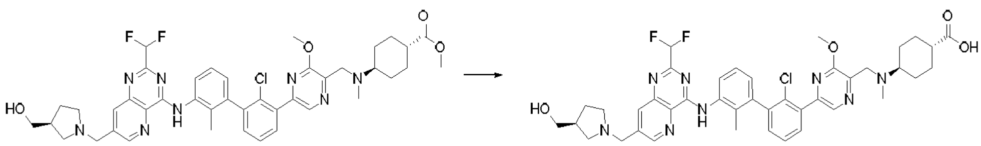

(1*r*,4*r*)-4-(((5-(2-Chloro-3'-((2-(difluoromethyl)-7-(((*S*)-3-(hydroxymethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid (0.23 g, 0.29 mmol) was dissolved in a mixed solution of 1,4-dioxane (12.5 mL) and water (2.5 mL), lithium hydroxide monohydrate (0.061 g, 1.45 mmol) was added, and the reaction was stirred at room temperature overnight. After the reaction of the raw materials was complete, the pH of the system was adjusted to about 5 with acetic acid, the reaction solution was concentrated under reduced pressure, and the residue was separated and purified by column chromatography (eluent: DCM/MeOH (v/v) = 7/1) to obtain 0.17 g of a yellow solid product with a yield of 75.23%.

MS (ESI, pos. ion) m/z: 787.32 [M+H]$^+$.

$^1$H NMR (400 MHz, DMSO-$d_6$) δ 10.35 (s, 1H), 8.94 (s, 1H), 8.42 (s, 1H), 8.15 (s, 1H), 7.75 (d, *J* = 8.0 Hz, 1H), 7.70 (d, *J* = 7.6 Hz, 1H), 7.58 (t, *J*= 7.6 Hz, 1H), 7.43 (d, *J* = 7.5 Hz, 1H), 7.38 (t, *J* = 7.8 Hz, 1H), 7.16 (d, *J* = 7.6 Hz, 1H), 6.71 (t, *J* = 54.5 Hz, 1H), 3.95 (s, 3H), 3.88 (d, *J* = 13.8 Hz, 1H), 3.81 (d, *J* = 13.8 Hz, 1H), 3.70 (s, 2H), 3.32 - 3.31 (m, 1H), 3.30 (s, 2H), 3.29 - 3.27 (m, 2H), 2.59 - 2.54 (m, 2H), 2.38 - 2.33 (m, 1H), 2.21 (s, 3H), 2.15 - 2.09 (m, 1H), 2.04 (s, 3H), 1.99 - 1.80 (m, 7H), 1.36 - 1.29 (m, 4H).

**Example 29 (1*r*,4*r*)-4-(((5-(2-chloro-3'-((2-(difluoromethyl)-7-(((*R*)-3-hydroxy-3-methyl pyrrolidin-1-yl)methyl) pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

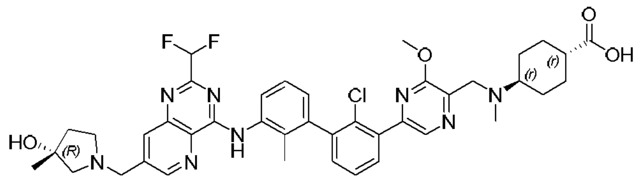

**Step 1: Synthesis of methyl (1*r*,4*r*)-4-(((5-(2-chloro-3'-((2-(difluoromethyl)-7-(((*R*)-3-hydroxy-3-methylpyrrolidin-1-yl)methyl)pyrido[3,2-*d*]pyrimidin-4-yl)amino)-2'-methyl-[1,1'-biphenyl]-3-yl)-3-methoxypyrazin-2-yl) methyl)(methyl)amino)cyclohexane-1-carboxylate**

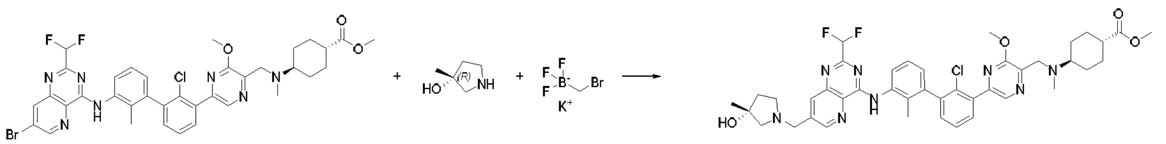

(*R*)-3-Methylpyrrolidin-3-ol (0.79 g, 7.83 mmol) was dissloved in acetonitrile (20 mL), potassium (bromomethyl) trifluoroborate (1.57 g, 7.83 mmol), and sodium carbonate (0.55 g, 5.22 mmol), heated at 80 °C for 5 h, and the system was concentrated. Then, methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclohexane-1-carboxylate (2.00 g, 2.61 mmol), palladium acetate (0.088 g, 0.39 mmol), X-Phos (0.37 g, 0.78 mmol), potassium carbonate (1.08 g, 7.83 mmol), 1,4-dioxane (50 mL) and water (10 mL) were added, the mixture was heated to 110 °C overnight under $N_2$. After the reaction of the raw materials was complete, the reaction solution was concentrated under reduced pressure, and the residue was separated and purified by silica gel column chromatography (eluent: DCM/MeOH (v/v) = 20/1) to obtain 1.35 g of a yellow solid product with a yield of 64.61%.

MS (ESI, pos. ion) m/z: 801.3 $[M+H]^+$.

**Step 2: Synthesis of (1*r*,4*r*)-4-(((5-(2-chloro-3'-((2-(difluoromethyl)-7-(((*R*)-3-hydroxy-3-methylpyrrolidin-1-yl) methyl)pyrido[3,2-*d*]pyrimidin-4-yl)amino)-2'-methyl-[1,1'-biphenyl]-3-yl)-3-methoxypyrazin-2-yl)methyl) (methyl)amino)cyclohexane-1-carboxylic acid**

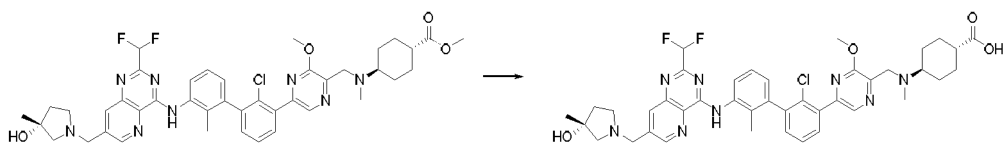

Methyl (1*r*,4*r*)-4-(((5-(2-chloro-3'-((2-(difluoromethyl)-7-(((*R*)-3-hydroxy-3-methylpyrrolidin-1-yl)methyl)pyrido [3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (0.81 g, 1.01 mmol) was dissolved in a mixed solution of 1,4-dioxane (20 mL) and water (4 mL), lithium hydroxide monohydrate (0.13 g, 3.03 mmol) was added, and the reaction was stirred at room temperature overnight. After the reaction of the raw materials was complete, the pH of the system was adjusted to about 5 with acetic acid, the reaction solution was concentrated under reduced pressure, and the residue was separated and purified by column chromatography (eluent: DCM/MeOH (v/v) = 7/1) to obtain 0.77 g of a yellow solid product with a yield of 96.8%.

MS (ESI, pos. ion) m/z: 787.33 $[M+H]^+$.

$^1H$ NMR (400 MHz, DMSO-$d_6$) δ 10.33 (s, 1H), 8.96 (d, *J* = 1.9 Hz, 1H), 8.42 (s, 1H), 8.18 (d, *J* = 1.9 Hz, 1H), 7.77 (d, *J* = 8.0 Hz, 1H), 7.70 (dd, *J* = 7.7, 1.7 Hz, 1H), 7.58 (t, *J* = 7.6 Hz, 1H), 7.44 (dd, *J* = 7.6, 1.7 Hz, 1H), 7.39 (t, *J* = 7.8 Hz, 1H), 7.17 (d, *J* = 7.5 Hz, 1H), 6.71 (t, *J* = 54.5 Hz, 1H), 4.61 (s, 1H), 3.96 (s, 3H), 3.87 (d, *J* = 3.0 Hz, 2H), 3.70 (s, 2H), 2.77 - 2.70 (m, 1H),

2.64 - 2.57 (m, 1H), 2.56 - 2.52 (m, 1H), 2.48 - 2.46 (m, 1H), 2.22 (s, 3H), 2.17 - 2.10 (m, 1H), 2.05 (s, 3H), 1.99 - 1.93 (m, 2H), 1.91 - 1.85 (m, 3H), 1.83 - 1.71 (m, 2H), 1.40 - 1.30 (m, 4H), 1.26 (s, 3H).

**Example 30 (1*r*,4*r*)-4-(((5-(2-chloro-3'-((2-(difluoromethyl)-7-(((*R*)-3-fluoro-3-(hydroxymethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

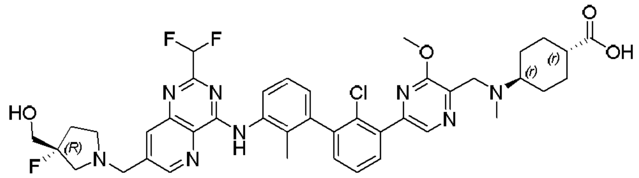

**Step 1: Synthesis of methyl (1*r*,4*r*)-4-(((5-(2-chloro-3'-((2-(difluoromethyl)-7-(((*R*)-3-fluoro-3-(hydroxymethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

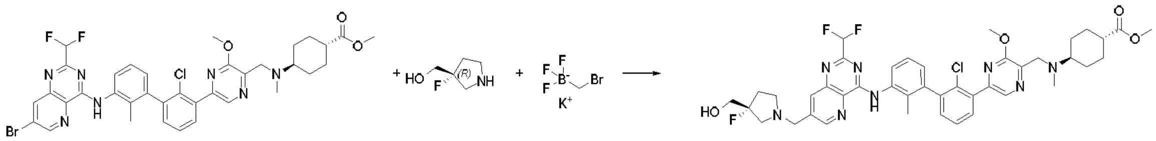

(*R*)-(3-Fluoropyrrolidin-3-yl)methanol (0.61 g, 3.90 mmol) was dissloved in acetonitrile (10 mL), potassium (bromomethyl)trifluoroborate (0.78 g, 3.90 mmol), and sodium carbonate (0.83 g, 7.80 mmol), heated at 80 °C for 5 h, and the system was concentrated. Then, methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclohexane-1-carboxylate (1.00 g, 1.30 mmol), palladium acetate (0.058 g, 0.26 mmol), X-Phos (0.25 g, 0.52 mmol), potassium carbonate (0.54 g, 3.90 mmol), 1,4-dioxane (25 mL) and water (5 mL) were added, the mixture was heated to 110 °C overnight under $N_2$. The reaction solution was concentrated under reduced pressure, and the residue was separated and purified by column chromatography (eluent: DCM/MeOH (v/v) = 20/1) to obtain 0.50 g of a yellow solid product with a yield of 46.81%.
MS (ESI, pos. ion) m/z: 819.3 $[M+H]^+$.

**Step 2: Synthesis of (1*r*,4*r*)-4-(((5-(2-chloro-3'-((2-(difluoromethyl)-7-(((*R*)-3-fluoro-3-(hydroxymethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

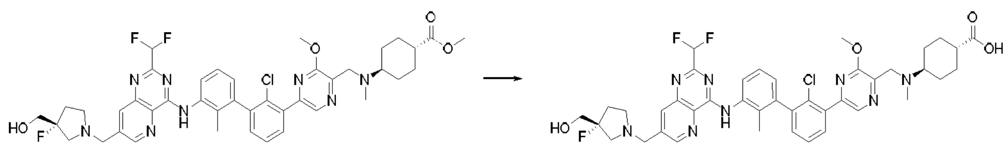

Methyl (1*r*,4*r*)-4-(((5-(2-chloro-3'-((2-(difluoromethyl)-7-(((*R*)-3-fluoro-3-(hydroxymethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (0.50 g, 0.61 mmol) was dissolved in a mixed solution of 1,4-dioxane (15 mL) and water (3 mL), lithium hydroxide monohydrate (0.10 g, 2.44 mmol) was added, and the reaction was stirred at room temperature overnight. After the reaction of the raw materials was complete, the pH of the system was adjusted to about 5 with acetic acid, the reaction solution was concentrated under reduced pressure, and the residue was separated and purified by column chromatography (eluent: DCM/MeOH (v/v) = 7/1) to obtain 0.45 g of a yellow solid product with a yield of 91.57%.
MS (ESI, pos. ion) m/z: 805.32 $[M+H]^+$.
$^1H$ NMR (400 MHz, DMSO-$d_6$) δ 10.35 (s, 1H), 8.95 (d, *J* = 1.9 Hz, 1H), 8.42 (s, 1H), 8.17 (d, *J* = 1.9 Hz, 1H), 7.75

(dd, $J$ = 8.1,1.3 Hz, 1H), 7.70 (dd, $J$ = 7.6, 1.7 Hz, 1H), 7.58 (t, $J$ = 7.6 Hz, 1H), 7.43 (dd, $J$ = 7.6, 1.7 Hz, 1H), 7.39 (t, $J$ = 7.8 Hz, 1H), 7.17 (d, $J$ = 7.5 Hz, 1H), 6.71 (t, $J$ = 54.5 Hz, 1H), 5.11 (s, 1H), 3.96 (s, 3H), 3.90 (s, 2H), 3.70 (s, 2H), 3.53 (d, $J$ = 6.6 Hz, 1H), 3.48 (d, $J$= 10.2 Hz, 1H), 2.86 - 2.79 (m, 1H), 2.77 (s, 1H), 2.71 (s, 1H), 2.59 - 2.53 (m, 1H), 2.48 - 2.43 (m, 1H), 2.21 (s, 3H), 2.17 - 2.09 (m, 1H), 2.04 (s, 3H), 1.98 - 1.84 (m, 6H), 1.37 - 1.29 (m, 4H).

**Example 31 (1*r*,4*r*)-4-(((5-(2-chloro-3'-((2-(difluoromethyl)-7-(((*R*)-3-methoxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

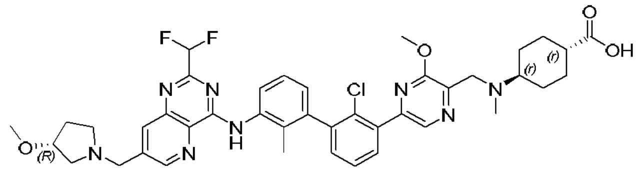

**Step 1: Synthesis of methyl (1*r*,4*r*)-4-(((5-(2-chloro-3'-((2-(difluoromethyl)-7-(((*R*)-3-methoxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

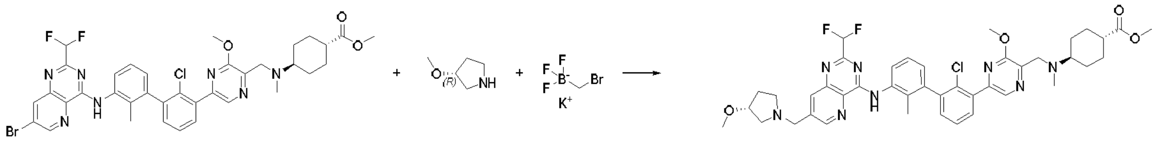

(*R*)-3-Methoxypyrrolidine (0.39 g, 3.90 mmol) was dissloved in acetonitrile (10 mL), potassium (bromomethyl) trifluoroborate (0.78 g, 3.90 mmol), and sodium carbonate (0.28 g, 2.60 mmol), heated at 80 °C for 5 h, and the system was concentrated. Then, methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclohexane-1-carboxylate (1.00 g, 1.30 mmol), palladium acetate (0.044 g, 0.20 mmol), X-Phos (0.19 g, 0.39 mmol), potassium carbonate (0.54 g, 3.90 mmol), 1,4-dioxane (25 mL) and water (5 mL) were added, the mixture was heated to 110 °C overnight under $N_2$. After the reaction of the raw materials was complete, the reaction solution was concentrated under reduced pressure, and the residue was separated and purified by silica gel column chromatography (eluent: DCM/MeOH (v/v) = 20/1) to obtain 0.68 g of a yellow solid product with a yield of 65.09%.
MS (ESI, pos. ion) m/z: 801.3 $[M+H]^+$.

**Step 2: Synthesis of (1*r*,4*r*)-4-(((5-(2-chloro-3'-((2-(difluoromethyl)-7-(((*R*)-3-methoxypyrrolidin-1-yl)methyl)pyrido [3,2-*d*] pyrimidin-4-yl)amino)-2'-methyl-[1,1'-biphenyl]-3-yl)-3-methoxypyrazin-2-yl)methyl)(methyl)amino) cyclohexane-1-carboxylic acid**

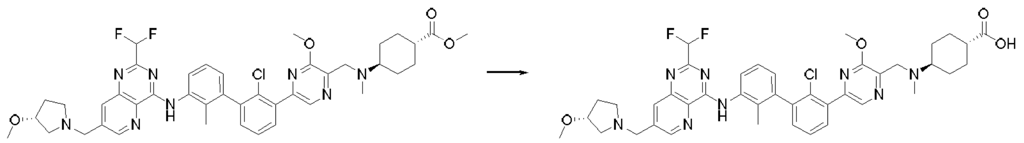

Methyl (1*r*,4*r*)-4-(((5-(2-chloro-3'-((2-(difluoromethyl)-7-(((*R*)-3-methoxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (0.68 g, 0.85 mmol) was dissolved in a mixed solution of 1,4-dioxane (20 mL) and water (4 mL), lithium hydroxide monohydrate (0.11 g, 2.55 mmol) was added, and the reaction was stirred at room temperature overnight. After the reaction of the raw materials was complete, the pH of the system was adjusted to about 5 with acetic acid, the reaction solution was concentrated under reduced pressure, and the residue was separated and purified by column chromatography (eluent: DCM/MeOH (v/v) = 7/1) to obtain 0.63 g of a yellow solid product with a yield of 94.30%.

MS (ESI, pos. ion) m/z: 787.33 $[M+H]^+$.

$^1H$ NMR (400 MHz, DMSO-*d*$_6$) δ 10.36 (s, 1H), 8.95 (d, *J* = 1.9 Hz, 1H), 8.43 (s, 1H), 8.16 (s, 1H), 7.75 (d, *J* = 8.0 Hz, 1H), 7.70 (d, *J* = 7.4 Hz, 1H), 7.59 (t, *J* = 7.6 Hz, 1H), 7.44 (d, *J* = 6.8 Hz, 1H), 7.39 (t, *J* = 7.8 Hz, 1H), 7.17 (d, *J* = 7.6 Hz, 1H), 6.71 (t, *J* = 54.5 Hz, 1H), 3.97 (s, 3H), 3.93 - 3.90 (m, 1H), 3.87 (d, *J* = 4.3 Hz, 2H), 3.74 (s, 2H), 3.17 (s, 3H), 2.76 (dd, *J* = 10.1, 6.2 Hz, 1H), 2.68 - 2.62 (m, 1H), 2.56 - 2.53 (m, 2H), 2.24 (s, 3H), 2.17 - 2.12 (m, 1H), 2.05 (s, 3H), 1.99 - 1.95 (m, 2H), 1.92 - 1.90 (m, 3H), 1.88 - 1.86 (m, 1H), 1.74 - 1.66 (m, 1H), 1.38 - 1.30 (m, 4H).

**Example 32 (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl-*d*$_3$)amino)cyclohexane-1-carboxylic acid**

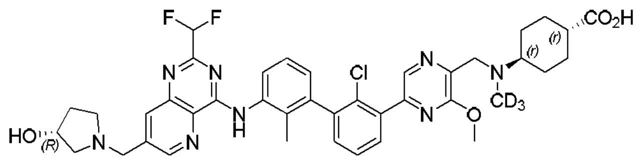

**Step 1: Synthesis of methyl (1*r*,4*r*)-4-(((5-(3-bromo-2-chlorophenyl)-3-methoxypyrazin-2-yl)methyl)(methyl-*d*$_3$)amino)cyclohexane-1-carboxylate**

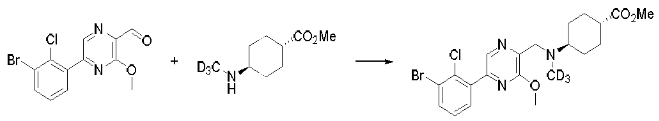

5-(3-Bromo-2-chlorophenyl)-3-methoxypyrazine-2-carbaldehyde (3.76 g, 11.48 mmol) and methyl (1*r*,4*r*)-4-((methyl-*d*$_3$)amino)cyclohexane-1-carboxylate (1.0 g, 5.74 mmol) were dissolved in a mixed solvent of dichloromethane (50 mL) and 2,2,2-trifluoroethanol (50 mL), triethylamine (3.99 mL, 28.70 mmol) was added, and the system was stirred at room temperature overnight. The system was cooled to room temperature, sodium cyanoborohydride (1.08 g, 17.22 mmol) was added, and the system was stirred at room temperature for 5.5 h. After the reaction was completed, the system was concentrated under reduced pressure, and the residue was diluted with water (30 mL) and dichloromethane (30 mL), and extracted with dichloromethane (30 mL × 3). The organic phases were combined and dried over anhydrous sodium sulfate, filtered under reduced pressure and washed with dichloromethane (15 mL). The filtrate was concentrated under reduced pressure, and the residue was separated and purified by column chromatography (PE/EtOAc (v/v) = 1/2) to obtain 1.7 g of a yellow solid with a yield of 61%.

MS (ESI, pos. ion) m/z: 485.3 $[M+H]^+$.

**Step 2: Synthesis of methyl (1*r*,4*r*)-4-(((5-(3'-amino-2-chloro-2'-methyl-[1,1'-biphenyl]-3-yl)-3-methoxypyrazin-2-yl)methyl)(methyl-*d*$_3$)amino)cyclohexane-1-carboxylate**

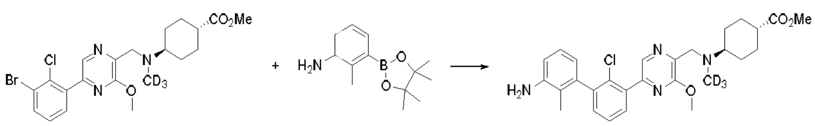

Methyl (1*r*,4*r*)-4-(((5-(3-bromo-2-chlorophenyl)-3-methoxypyrazin-2-yl)methyl)(methyl-ds)amino)cyclohexane-1-carboxylate (2.36 g, 4.86 mmol) and 2-methyl-3-(4,4,5,5-tetramethyl-1,3,2-dioxaborolan-2-yl)aniline (1.70 g, 7.29 mmol) were dissolved in a mixed solvent of 1,4-dioxane (200 mL) and water (40 mL). [1,1'-Bis(diphenylphosphino)ferrocene]palladium dichloride dichloromethane adduct (0.40 g, 0.49 mmol) and potassium carbonate (1.34 g, 9.72 mmol) were added. The system was stirred at 90 °C for 11 h under $N_2$. The stirring was stopped, and the system was diluted with water (20 mL), extracted with EtOAc (20 mL × 3), the organic phases were combined and dried over anhydrous

sodium sulfate, filtered under reduced pressure and washed with EtOAc (10 mL), the filtrate was concentrated under reduced pressure, and the residue was separated and purified by column chromatography (PE/EtOAc (v/v) = 1/4) to obtain 1.59 g of a reddish-brown solid with a yield of 64%.

MS (ESI, pos. ion) m/z: 512.2 $[M+H]^+$.

**Step 3: Synthesis of methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl-*d*$_3$)amino)cyclohexane-1-carboxylate**

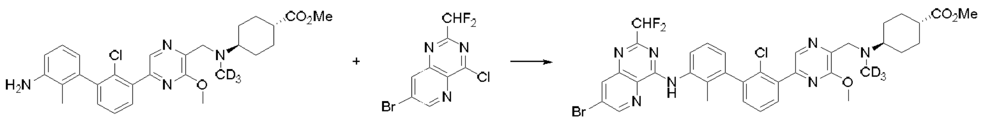

Methyl (1*r*,4*r*)-4-(((5-(3'-amino-2-chloro-2'-methyl-[1,1'-biphenyl]-3-yl)-3-methoxypyrazin-2-yl)methyl)(methyl-*d*$_3$)amino)cyclohexane-1-carboxylate (1.59 g, 3.11 mmol) and 7-bromo-4-chloro-2-(difluoromethyl)pyrido[3,2-*d*]pyrimidine (1.19 g, 4.04 mmol) were dissolved in *tert*-butanol (100 mL), and hydrochloric acid (1.17 mL, 4.67 mmol) was added. The system was stirred at 100 °C overnight under $N_2$. The reaction was stopped and stirred, the system was concentrated under reduced pressure, water (25 mL) and dichloromethane (25 mL) were added to dilute the system, saturated potassium carbonate solution (15 mL) was added, and the mixture was extracted with dichloromethane (25 mL × 3), the organic phases were combined and dried over anhydrous sodium sulfate. The mixture was filtered under reduced pressure and washed with dichloromethane (10 mL), the filtrate was concentrated under reduced pressure, and the residue was separated and purified by column chromatography (PE/EtOAc (v/v) = 1/4) to obtain 1.86 g of a reddish brown solid with a yield of 78%.

MS (ESI, pos. ion) m/z: 769.0 $[M+H]^+$.

**Step 4: Synthesis of methyl (1*r*,4*r*)-4-(((5-(2-chloro-3'-((2-(difluoromethyl)-7-vinylpyrido[3,2-*d*]pyrimidin-4-yl) amino)-2'-methyl-[1,1'-biphenyl]-3-yl)-3-methoxypyrazin-2-yl)methyl)(methyl-*d*$_3$)amino)cyclohexane-1-carboxylate**

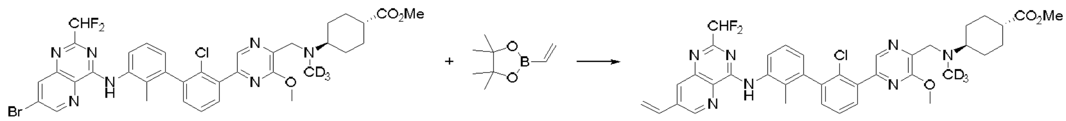

Methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl-*d*$_3$)amino)cyclohexane-1-carboxylate (1.86 g, 2.42 mmol) was dissolved in a mixed solvent of 1,4-dioxane (120 mL) and water (12 mL), 4,4,5,5-tetramethyl-2-vinyl-1,3,2-dioxaborolane (0.49 mL, 3.18 mmol), [1,1'-Bis(diphenylphosphino)ferrocene]palladium dichloride dichloromethane adduct (0.20 g, 0.24 mmol) and potassium phosphate (1.28 g, 6.05 mmol) were added in sequence, and the system was stirred at 100 °C overnight. The stirring was stopped, the system was cooled to room temperature and diluted with water (20 mL) and EtOAc (20 mL), the liquids were separated, the aqueous phase was extracted with EtOAc (20 mL × 3), the organic phases were combined and dried over anhydrous sodium sulfate, filtered under reduced pressure and washed with EtOAc (10 mL), the filtrate was concentrated under reduced pressure, and the residue was separated and purified by column chromatography (PE/EtOAc (v/v) = 1/5) to obtain 1.2 g of a reddish brown solid with a yield of 69%.
MS (ESI, pos. ion) m/z: 717.6 $[M+H]^+$.

**Step 5: Synthesis of methyl (1*r*,4*r*)-4-(((5-(2-chloro-3'-((2-(difluoromethyl)-7-formylpyrido[3,2-*d*]pyrimidin-4-yl) amino)-2'-methyl-[1,1'-biphenyl]-3-yl)-3-methoxypyrazin-2-yl)methyl)(methyl-*d*$_3$)amino)cyclohexane-1-carboxylate**

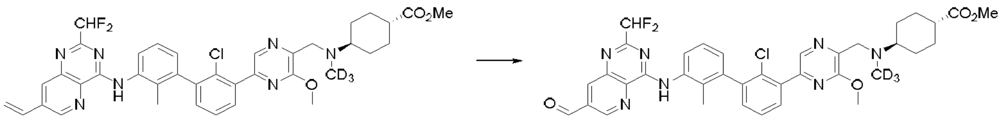

Methyl (1r,4r)-4-(((5-(2-chloro-3'-((2-(difluoromethyl)-7-vinylpyrido[3,2-*d*]pyrimidin-4-yl)amino)-2'-methyl-[1,1'-biphenyl]-3-yl)-3-methoxypyrazin-2-yl)methyl)(methyl-$d_3$)amino)cyclohexane-1-carboxylate (1.2 g, 1.67 mmol) was dissolved in 1,4-dioxane (90 mL) and water (30 mL), potassium osmate dihydrate (30.77 mg, 0.084 mmol) and sodium periodate (0.89 g, 4.17 mmol) were added in sequence, and the system was stirred at room temperature for 6.5 h. The stirring was stopped, the system was filtered under reduced pressure, the filtrate was separated, the aqueous phase was extracted with EtOAc (10 mL × 3), the organic phases were combined and dried over anhydrous sodium sulfate, filtered under reduced pressure and washed with EtOAc (10 mL), the filtrate was concentrated under reduced pressure, and the residue was separated and purified by column chromatography (PE/EtOAc (v/v) = 1/5) to obtain 0.46 g of a red viscous product with a yield of 38%.

MS (ESI, pos. ion) m/z: 719.5 $[M+H]^+$.

**Step 6: Synthesis of methyl (1r,4r)-4-(((5-(2-chloro-3'-((2-(difluoromethyl)-7-(((*R*)-3-hydroxypyrrolidin-1-yl) methyl)pyrido[3,2-*d*]pyrimidin-4-yl)amino)-2'-methyl-[1,1'-biphenyl]-3-yl)-3-methoxypyrazin-2-yl)methyl) (methyl-$d_3$)amino)cyclohexane-1-carboxylate**

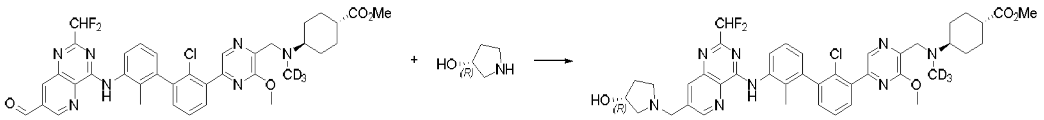

Methyl (1*r*,4*r*)-4-(((5-(2-chloro-3'-((2-(difluoromethyl)-7-formylpyrido[3,2-*d*]pyrimidin-4-yl)amino)-2'-methyl-[1,1'-biphenyl]-3-yl)-3-methoxypyrazin-2-yl)methyl)(methyl-$d_3$)amino)cyclohexane-1-carboxylate (0.2 g, 0.28 mmol) and (R)-pyrrolidin-3-ol (0.11 mL, 1.40 mmol) were dissolved in a mixed solvent of methanol (30 mL) and dichloromethane (10 mL), acetic acid (0.048 mL, 0.84 mmol) was added, and the system was stirred at room temperature overnight. Sodium cyanoborohydride (0.053 g, 0.84 mmol) was added, and the system was stirred at room temperature for 5.5 h. The reaction was stopped and stirred, and the system was concentrated under reduced pressure. Water (10 mL) and DCM (10 mL) were added to dilute the system. Saturated potassium carbonate solution (5 mL) was added to adjust the pH of the system to 9-10. The liquids were separated, and the aqueous phase was extracted with DCM (10 mL × 3). The organic phases were combined and dried over anhydrous sodium sulfate, filtered under reduced pressure and washed with DCM (10 mL). The filtrate was concentrated under reduced pressure, and the residue was separated by column chromatography (eluent: DCM/MeOH (v/v) = 20/1) and then sent to preparative chromatography for separation to obtain 90 mg of a yellow viscous solid with a yield of 40.9%.

MS (ESI, pos. ion) m/z: 790.7 $[M+H]^+$.

**Step 7: Synthesis of (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl-$d_3$)amino) cyclohexane-1-carboxylic acid**

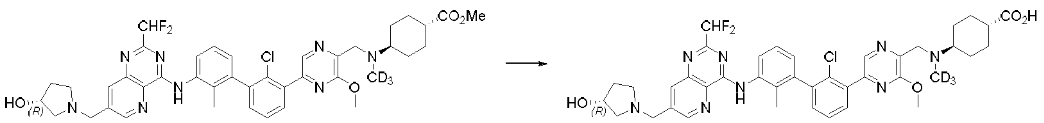

Methyl (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl-ds)amino)cyclohexane-1-carboxylate (0.1 g, 0.13 mmol) was dissolved in 1,4-dioxane (20 mL), and a solution of lithium hydroxide monohydrate (109.10 mg, 2.6 mmol) in water (5 mL) was added, and the system was stirred at room temperature for 9.5 h. The stirring was stopped, acetic acid was added to the system to adjust the pH to 4-5, and the system was concentrated under reduced pressure. The residue was separated by column chromatography (eluent: DCM/MeOH (v/v) = 7/1) and sent for preparative

separation to obtain 40.2 mg of a yellow solid with a yield of 40.9%.

MS (ESI, pos. ion) m/z: 776.33 $[M+H]^+$.

$^1H$ NMR (400 MHz, DMSO-$d_6$) δ 10.38 (s, 1H), 9.03 (s, 1H), 8.47 (s, 1H), 8.30 (s, 1H), 7.74 (d, *J* = 7.1 Hz, 1H), 7.69 (d, *J* = 7.6 Hz, 1H), 7.59 (t, *J* = 7.4 Hz, 1H), 7.44 (d, *J* = 7.3 Hz, 1H), 7.39 - 7.37 (m, 1H), 7.16 (d, *J* = 7.4 Hz, 1H), 6.68 (t, *J* = 54.6 Hz, 1H), 5.56 (s, 1H), 3.98 (s, 3H), 3.05 (s, 1H), 2.95 (s, 2H), 2.33 - 2.26 (m, 1H), 2.04 (s, 5H), 2.02 - 1.95 (m, 4H), 1.90 (s, 3H), 1.71 (s, 1H), 1.53 - 1.45 (m, 2H), 1.41 - 1.32 (m, 3H), 1.28 - 1.24 (m, 2H).

**Example 33 (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)amino)cyclohexane-1-carboxylic acid**

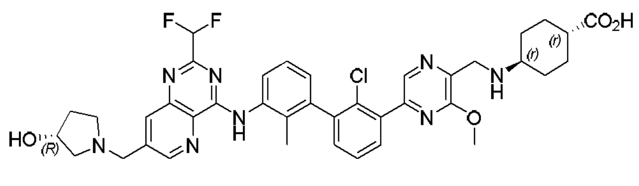

**Step 1: Synthesis of 5-(3'-amino-2-chloro-2'-methyl-[1,1'-biphenyl]-3-yl)-3-methoxypyrazine-2-carbaldehyde**

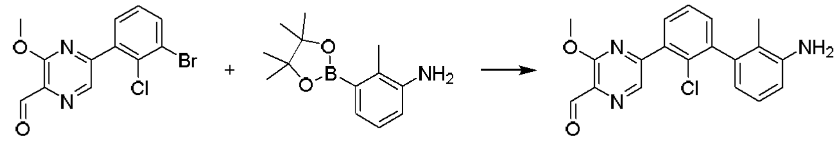

To a 500 mL microwave tube, 5-(3-bromo-2-chlorophenyl)-3-methoxypyrazine-2-carbaldehyde (12 g, 36.63 mmol), 2-methyl-2-(tetramethyl-1,3,2-dioxaborolan-2-yl)aniline (11.10 g, 47.62 mmol), potassium carbonate (12.66 g, 91.58 mmol), [1,1'-Bis(diphenylphosphino)ferrocene]palladium dichloride dichloromethane adduct (7.48 g, 9.16 mmol), 1,4-dioxane (250 mL) and water (50 mL) were added in sequence. The mixture was reacted at 120 °C under $N_2$. After the reaction was complete, the reaction solution was concentrated under reduced pressure, water (500 mL) was added, and then extracted with DCM (100 mL × 3). The organic phases were combined, washed with saturated brine (300 mL × 1), dried over anhydrous sodium sulfate, filtered, and the filtrate was concentrated under reduced pressure. The residue was separated and purified by silica gel column chromatography (eluent: DCM/MeOH (v/v) = 60/1) to obtain 10.50 g of a brown solid product with a yield of 81.01%.

MS (ESI, pos. ion) m/z: 354.1 $[M+H]^+$.

**Step 2: Synthesis of 5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazine-2-carbaldehyde**

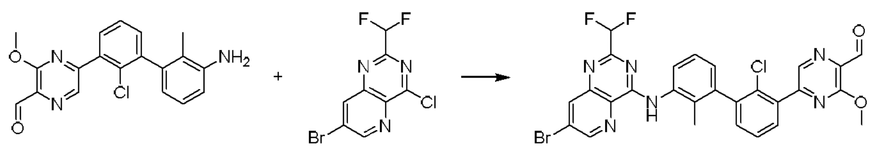

To a 500 mL single-necked bottle, 5-(3'-amino-2-chloro-2'-methyl-[1,1'-biphenyl]-3-yl)-3-methoxypyrazine-2-carbaldehyde (6.00 g, 16.96 mmol), *tert*-butanol (200 mL) and 7-bromo-4-chloro-2-(difluoromethyl)pyrido[3,2-*d*]pyrimidine (5.49 g, 18.66 mmol) were added in sequence and reacted at 100 °C overnight under $N_2$. After the reaction was complete, the reaction solution was concentrated under reduced pressure, water (300 mL) was added, and then extracted with DCM (60 mL × 3). The organic phases were combined, washed with saturated brine (150 mL × 1), dried over anhydrous sodium sulfate, filtered, and the filtrate was concentrated under reduced pressure. The residue was separated and purified by silica gel column chromatography (eluent: DCM/MeOH (v/v) = 40/1) to obtain 8.95 g of a brown solid product

with a yield of 86.26%.

MS (ESI, pos. ion) m/z: 612.0 $[M+H]^+$.

**Step 4: Synthesis of methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)amino)cyclohexane-1-carboxylate**

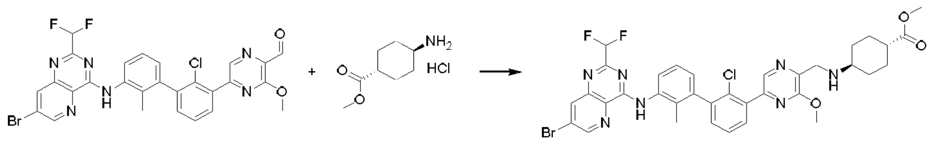

5-(3'-((7-Bromo-2-(difluoromethyl)pyrido[3,2-d]pyrimidin-4-yl)amino)-2-chloro-2'-methyl-[1,1'-biphenyl]-3-yl)-3-methoxypyrazine-2-carbaldehyde (10.20 g, 16.67 mmol), methyl (1r,4r)-4-aminocyclohexane-1-carboxylate (9.69 g, 50.01 mmol), DCM (100 mL), methanol (100 mL), TEA (3.37 g, 33.34 mmol) and acetic acid (1.00 g, 16.67 mmol) were added to a 500 mL single-necked bottle in sequence and reacted at 50 °C overnight. $NaBH_3CN$ (2.10 g, 33.34 mmol) was added, and the reaction was continued at room temperature for 0.5 h. After the reaction was completed, the pH was adjusted to 7~8 with 1N $K_2CO_3$ aqueous solution to quench the reaction, and the organic solvent was removed by concentration under reduced pressure. Water (500 mL) was added, and then extracted with DCM (70 mL × 3). The organic phases were combined, washed with water (200 mL × 1), dried over anhydrous sodium sulfate, filtered, and the filtrate was concentrated under reduced pressure. The residue was separated and purified by silica gel column chromatography (eluent: DCM/MeOH (v/v) = 60/1) to obtain 9.80 g of a light yellow solid product with a yield of 78.06%.

MS (ESI, pos. ion) m/z: 752.2 $[M+H]^+$.

**Step 4: Synthesis of methyl (1*r*,4*r*)-4-(((5-(2-chloro-3'-((2-(difluoromethyl)-7-vinylpyrido[3,2-*d*]pyrimidin-4-yl) amino)-2'-methyl-[1,1'-biphenyl]-3-yl)-3-methoxypyrazin-2-yl)methyl)amino)cyclohexane-1-carboxylate**

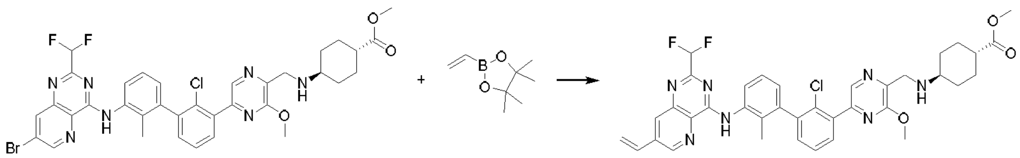

To a 50 mL single-necked bottle, methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)amino)cyclohexane-1-carboxylate (700 mg, 0.93 mmol), 2-vinyl-4,4,5,5-tetramethyl-1,3,2-dioxaborolane (429.69 mg, 2.79 mmol), tripotassium phosphate (493.53 mg, 2.33 mmol), [1,1'-Bis(diphenylphosphino)ferrocene]palladium dichloride dichloromethane adduct (75.95 mg, 0.093 mmol), 1,4-dioxane (15 mL) and water (3 mL) were added in sequence, and the reaction was carried out at 130 °C under $N_2$. After the reaction was complete, the reaction solution was concentrated under reduced pressure, water (150 mL) was added, and then extracted with DCM (20 mL × 3). The organic phases were combined, washed with saturated brine (50 mL × 1), dried over anhydrous sodium sulfate, filtered, and the filtrate was concentrated under reduced pressure. The residue was separated and purified by silica gel column chromatography (eluent: DCM/MeOH (v/v) = 30/1) to obtain 635.0 mg of a yellow solid product with a yield of 97.56%.

MS (ESI, pos. ion) m/z: 700.3 $[M+H]^+$.

**Step 5: Synthesis of methyl (1*r*,4*r*)-4-(((5-(2-chloro-3'-((2-(difluoromethyl)-7-formylpyrido[3,2-*d*]pyrimidin-4-yl) amino)-2'-methyl-[1,1'-biphenyl]-3-yl)-3-methoxypyrazin-2-yl)methyl)amino)cyclohexane-1-carboxylate**

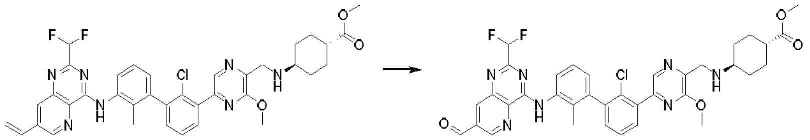

To a 100 mL single-necked bottle, methyl (1*r*,4*r*)-4-(((5-(2-chloro-3'-((2-(difluoromethyl)-7-vinylpyrido[3,2-*d*]pyrimidin-4-yl)amino)-2'-methyl-[1,1'-biphenyl]-3-yl)-3-methoxypyrazin-2-yl)methyl)amino)cyclohexane-1-carboxylate (500 mg, 0.71 mmol), 1,4-dioxane (25 mL), water (10 mL), potassium osmate dihydrate (26.16 mg, 0.071 mmol) and sodium periodate (759.31 mg, 3.55 mmol) were added in sequence and reacted at room temperature. After the reaction was complete, the reaction was quenched with saturated aqueous sodium sulfite solution (5 mL), concentrated under reduced pressure, water (100 mL) was added, and then extracted with DCM (15 mL × 3). The organic phases were combined, washed with water (40 mL × 1), dried over anhydrous sodium sulfate, filtered, and the filtrate was concentrated under reduced pressure. The residue was separated and purified by silica gel column chromatography (eluent: DCM/MeOH (v/v) = 30/1) to obtain 350.0 mg of a light yellow solid product with a yield of 69.80%.

MS (ESI, pos. ion) m/z: 702.5 $[M+H]^+$.

**Step 6: Synthesis of methyl (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)amino)cyclohexane-1-carboxylate**

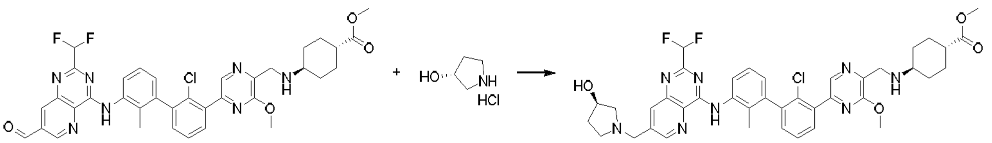

To a 50 mL single-necked bottle, methyl (1*r*,4*r*)-4-(((5-(2-chloro-3'-((2-(difluoromethyl)-7-formylpyrido[3,2-*d*]pyrimidin-4-yl)amino)-2'-methyl-[1,1'-biphenyl]-3-yl)-3-methoxypyrazin-2-yl)methyl)amino)cyclohexane-1-carboxylate (200 mg, 0.28 mmol), (R)-pyrrolidin-3-ol hydrochloride (173.01 mg, 1.40 mmol), DCM (10 mL), MeOH (10 mL), TEA (42.50 mg, 0.42 mmol) and AcOH (25.22 mg, 0.42 mmol) were added in sequence and reacted at room temperature overnight. $NaBH_3CN$ (35.19 mg, 0.56 mmol) was added, and the reaction was continued at room temperature for 1 h. After the reaction was completed, the pH was adjusted to 7~8 with 1N $K_2CO_3$ aqueous solution to quench the reaction, and the mixture was concentrated under reduced pressure. Water (100 mL) was added, and then extracted with DCM (15 mL × 3). The organic phases were combined, washed with water (40 mL × 1), dried over anhydrous sodium sulfate, filtered, and the filtrate was concentrated under reduced pressure. The residue was separated and purified by silica gel column chromatography (eluent: DCM/MeOH (v/v) = 10/1) to obtain 150.0 mg of a light yellow solid product with a yield of 68.10%. MS (ESI, pos. ion) m/z: 773.4 $[M+H]^+$.

**Step 7: Synthesis of (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)amino)cyclohexane-1-carboxylic acid**

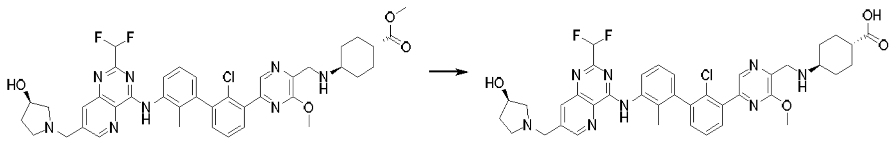

To a 25 mL single-necked bottle, methyl (1*r*,4*r*)-4-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)amino)cyclohexane-1-carboxylate (63 mg, 0.081 mmol), 1,4-dioxane (6 mL), water (2 mL) and lithium hydroxide monohydrate (6.80 mg, 0.16 mmol) were added in sequence and reacted at 50 °C. After the reaction was complete, the mixture was concentrated under reduced pressure, dissolved with DCM (5 mL) and MeOH (5 mL), the pH was adjusted to 6~7 with a hydrogen chloride-dioxane solution (4.0 M), and concentrated under reduced pressure. The residue was separated and

purified by silica gel column chromatography (eluent: DCM/MeOH (v/v) = 3/1) to obtain 38.7 mg of a yellow solid product with a yield of 62.56%.

MS (ESI, pos. ion) m/z: 759.30 [M+H]+.

$^1$H NMR (400 MHz, DMSO-*d*$_6$) δ 10.36 (s, 1H), 8.95 (d, *J* = 1.6 Hz, 1H), 8.45 (s, 1H), 8.17 (s, 1H), 7.74 (d, *J* = 7.9 Hz, 1H), 7.69 (dd, *J* = 7.7, 1.5 Hz, 1H), 7.59 (t, *J* = 7.6 Hz, 1H), 7.44 (dd, *J* = 7.5, 1.5 Hz, 1H), 7.38 (t, *J* = 7.8 Hz, 1H), 7.16 (d, *J* = 7.4 Hz, 1H), 6.70 (t, *J* = 54.5 Hz, 1H), 4.77 (s, 1H), 4.28 - 4.18 (m, 1H), 3.98 (s, 3H), 3.96 (s, 2H), 3.92 - 3.81 (m, 3H), 2.77 - 2.63 (m, 3H), 2.41 (dd, *J* = 9.7, 3.4 Hz, 1H), 2.17 - 2.10 (m, 1H), 2.04 (s, 3H), 2.02 - 1.86 (m, 5H), 1.62 - 1.54 (m, 1H), 1.41 - 1.25 (m, 4H), 1.17 - 1.08 (m, 2H).

**Example 34 methyl (1*r*,4*r*)-4-(((5-(2,2'-dichloro-3'-((2-(difluoromethyl)-7-(((*R*)-3-hydroxypyrrolidin-1-yl)methyl) pyrido[3,2-*d*]pyrimidin-4-yl)amino)-[1,1'-biphenyl]-3-yl)-3-methoxypyrazin-2-yl)methyl)(methyl)amino)cyclohexane-1-carboxylate**

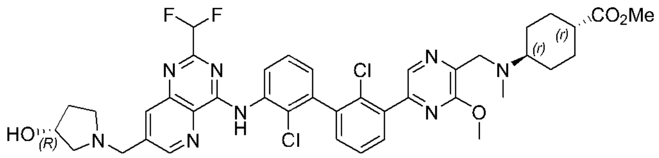

**Step 1: Synthesis of methyl (1*r*,4*r*)-4-(((5-(3-bromo-2-chlorophenyl)-3-methoxypyrazin-2-yl)methyl)amino)cyclohexane-1-carboxylate**

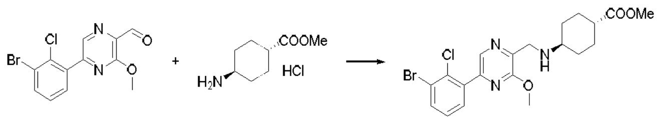

5-(3-Bromo-2-chlorophenyl)-3-methoxypyrazine-2-carbaldehyde (6 g, 18.32 mmol) and methyl (1*r*,4*r*)-4-aminocyclohexane-1-carboxylate (5.32 g, 27.48 mmol) and $Et_3N$ (3 mL) were dissolved in 2,2,2-trifluoroethanol (70 mL) and DCM (70 mL) and stirred at 50 °C for 0.5 h. $NaBH_3CN$ (1726.84 mg, 27.48 mmol) was added, and the reaction was continued at room temperature for 0.5 h. After the reaction was complete, the system was concentrated and the residue was separated and purified by silica gel column chromatography (DCM/MeOH (v/v) = 15/1) to obtain 7.46 g of a light yellow viscous product with a yield of 87.06%.

MS (ESI, pos. ion) m/z: 468.1 [M+H]+.

**Step 2: Synthesis of methyl (1*r*,4*r*)-4-(((5-(3-bromo-2-chlorophenyl)-3-methoxypyrazin-2-yl)methyl)(methyl) amino)cyclohexane-1-carboxylate**

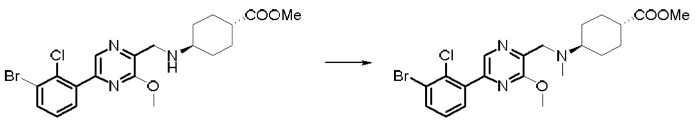

Methyl (1*r*,4*r*)-4-(((5-(3-bromo-2-chlorophenyl)-3-methoxypyrazin-2-yl)methyl)amino)cyclohexane-1-carboxylate (7.46 g, 15.95 mmol), formaldehyde (1.94 g, 23.92 mmol) and $Et_3N$ (3 mL) were dissolved in 2,2,2-trifluoroethanol (70 mL) and DCM (70 mL) and stirred at 50 °C for 2 h. $NaBH_3CN$ (1503.45 mg, 23.92 mmol) was added, and the reaction was continued at room temperature for 0.2 h. After the reaction was complete, the system was concentrated and the residue was separated and purified by silica gel column chromatography (DCM/MeOH (v/v) = 20/1) to obtain 6.75 g of brown-yellow viscous product with a yield of 87.67%.

MS (ESI, pos. ion) m/z: 482.2 [M+H]+.

**Step 3: Synthesis of methyl (1*r*,4*r*)-4-(((5-(3'-amino-2,2'-dichloro-[1,1'-biphenyl]-3-yl)-3-methoxypyrazin-2-yl) methyl)(methyl)amino)cyclohexane-1-carboxylate**

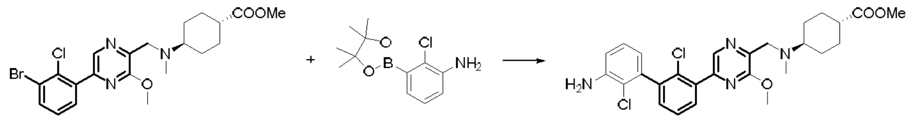

Methyl (1*r*,4*r*)-4-(((5-(3-bromo-2-chlorophenyl)-3-methoxypyrazin-2-yl)methyl)(methyl)amino)cyclohexane-1-carboxylate (5.45 g, 11.29 mmol) and 2-chloro-3-(4,4,5,5-tetramethyl-1,3,2-dioxaborolan-2-yl)aniline (5.72 g, 22.58 mmol) were dissolved in a mixed solvent of water (30 mL) and 1,4-dioxane (150 mL). Sodium carbonate (3.59 g, 33.87 mmol) and dichloro[1,1'-bis(dicyclohexylphosphino)ferrocene]dichloropalladium(II) (0.85 g, 1.13 mmol) were added in sequence. The mixture was heated to 90 °C for 8 h under $N_2$. After the reaction was complete, the system was concentrated and the residue was separated and purified by silica gel column chromatography (DCM/MeOH (v/v) = 20/1) to obtain 4.06 g of a yellow viscous product with a yield of 67.93%.

MS (ESI, pos. ion) m/z: 529.3 $[M+H]^+$.

**Step 4: Synthesis of methyl (1*r*,4*r*)-4-(((5-(3'-((7-bromo-2-(difluoromethyl)pyrido[3,2-*d*]pyrimidin-4-yl)amino)-2,2'-dichloro-[1,1'-biphenyl]-3-yl)-3-methoxypyrazin-2-yl)methyl)(methyl)amino)cyclohexane-1-carboxylate**

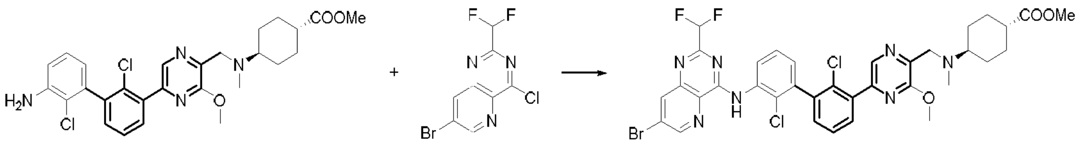

Methyl (1*r*,4*r*)-4-(((5-(3'-amino-2,2'-dichloro-[1,1'-biphenyl]-3-yl)-3-methoxypyrazin-2-yl)methyl)(methyl)amino)cyclohexane-1-carboxylate (4.22 g, 7.97 mmol) and 7-bromo-4-chloro-2-(difluoromethyl)pyrido[3,2-*d*]pyrimidine (2.82 g, 9.56 mmol) were added to tert-butanol (150 mL) and stirred at 100 °C for 18 h under $N_2$. After the reaction was complete, the system was concentrated and the residue was separated and purified by silica gel column chromatography (EtOAc/PE (v/v) = 2/1) to obtain 6 g of a yellow viscous product with a yield of 95.59%.

MS (ESI, pos. ion) m/z: 786.0 $[M+H]^+$.

**Step 5: Synthesis of methyl (1*r*,4*r*)-4-(((5-(2,2'-dichloro-3'-((2-(difluoromethyl)-7-vinylpyrido[3,2-*d*]pyrimidin-4-yl)amino)-[1,1'-biphenyl]-3-yl)-3-methoxypyrazin-2-yl)methyl)(methyl)amino)cyclohexane-1-carboxylate**

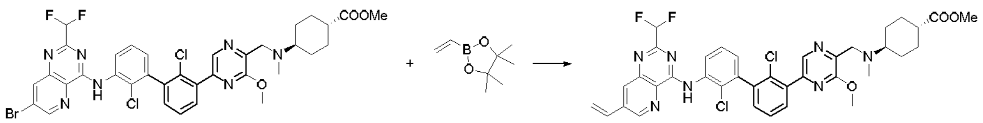

Methyl (1*r*,4*r*)-4-(((5-(3'-((7-bromo-2-(difluoromethyl)pyrido[3,2-*d*]pyrimidin-4-yl)amino)-2,2'-dichloro-[1,1'-biphenyl]-3-yl)-3-methoxypyrazin-2-yl)methyl)(methyl)amino)cyclohexane-1-carboxylate (6 g, 7.62 mmol) was dissolved in a mixed solvent of 1,4-dioxane (150 mL) and water (30 mL), 4,4,5,5-tetramethyl-2-vinyl-1,3,2-dioxaborolane (11.74 g, 76.2 mmol), [1,1'-bis(diphenylphosphino)ferrocene]palladium dichloride dichloromethane adduct (622.28 mg, 0.76 mmol) and potassium phosphate (3.23 g, 15.24 mmol) were added in sequence, and the system was stirred at 100 °C for 6 h under $N_2$. After the reaction was complete, the system was concentrated and the residue was separated and purified by silica gel column chromatography (EtOAc/PE (v/v) = 2/1) to obtain 4.73 g of a yellow viscous product with a yield of 84.51%.

MS (ESI, pos. ion) m/z: 734.4 $[M+H]^+$.

**Step 6: Synthesis of methyl (1*r*,4*r*)-4-(((5-(2,2'-dichloro-3'-((2-(difluoromethyl)-7-formylpyrido[3,2-*d*]pyrimidin-4-yl)amino)-[1,1'-biphenyl]-3-yl)-3-methoxypyrazin-2-yl)methyl)(methyl)amino)cyclohexane-1-carboxylate**

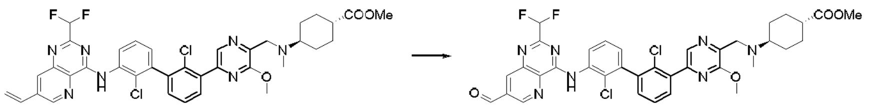

Methyl (1*r*,4*r*)-4-(((5-(2,2'-dichloro-3'-((2-(difluoromethyl)-7-vinylpyrido[3,2-*d*]pyrimidin-4-yl)amino)-[1,1'-biphenyl]-3-yl)-3-methoxypyrazin-2-yl)methyl)(methyl)amino)cyclo hexane-1-carboxylate (4.73 g, 6.44 mmol) was dissolved in 1,4-dioxane (120 mL) and water (40 mL), potassium osmate dihydrate (118.64 mg, 0.32 mmol) and sodium periodate (3.44 g, 16.1 mmol) were added in sequence, and the system was stirred at room temperature for 3 h. After the reaction was completed, the mixture was filtered and separated. The aqueous phase was extracted with EtOAc (30 mL × 3). The organic phases were combined and dried over anhydrous sodium sulfate. The filtrate was concentrated under reduced pressure. The residue was separated and purified by silica gel column chromatography (DCM/MeOH (v/v) = 20/1) to obtain 2.42 g of a brown-yellow viscous product with a yield of 51.03%.
MS (ESI, pos. ion) m/z: 736.2 $[M+H]^+$.

**Step 7: Synthesis of methyl (1*r*,4*r*)-4-(((5-(2,2'-dichloro-3'-((2-(difluoromethyl)-7-(((*R*)-3-hydroxypyrrolidin-1-yl)methyl)pyrido[3,2-*d*]pyrimidin-4-yl)amino)-[1,1'-biphenyl]-3-yl)-3-methoxypyrazin-2-yl)methyl)(methyl)amino)cyclohexane-1-carboxylate**

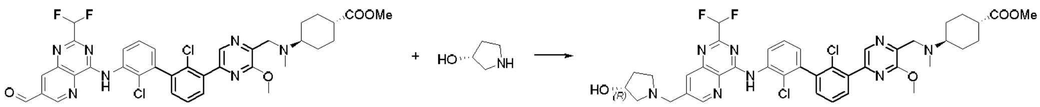

Methyl (1*r*,4*r*)-4-(((5-(2,2'-dichloro-3'-((2-(difluoromethyl)-7-formylpyrido[3,2-*d*]pyrimidin-4-yl)amino)-[1,1'-biphenyl]-3-yl)-3-methoxypyrazin-2-yl)methyl)(methyl)amino)cyclo hexane-1-carboxylate (2.42 g, 3.29 mmol), (*R*)-pyrrolidin-3-ol (1.43 g, 16.45 mmol) and TEA (3 mL) were dissolved in 2,2,2-trifluoroethanol (50 mL) and DCM (50 mL), the reaction was stirred at 50 °C for 0.5 h. $NaBH_3CN$ (310.12 mg, 4.94 mmol) was added, and the reaction was continued at room temperature. After the reaction was complete, the system was concentrated and the residue was separated and purified by silica gel column chromatography (DCM/MeOH (v/v) = 20/1) to obtain 1.26 g of brown yellow powder with a yield of 47.48%.
MS (ESI, pos. ion) m/z: 807.0 $[M+H]^+$.
$^1H$ NMR (400 MHz, DMSO-$d_6$) δ 10.26 (s, 1H), 8.99 (s, 1H), 8.53 (d, *J* = 8.1 Hz, 1H), 8.45 (s, 1H), 8.27 (s, 1H), 7.74 (d, *J* = 7.7 Hz, 1H), 7.59 (dt, *J* = 14.3, 7.9 Hz, 2H), 7.49 (d, *J* = 7.6 Hz, 1H), 7.27 (d, *J* = 7.6 Hz, 1H), 6.83 (t, *J* = 54.4 Hz, 1H), 4.25 (s, 1H), 4.01 (s, 1H), 3.99 (s, 1H), 3.96 (s, 3H), 3.56 (s, 2H), 3.54 (s, 3H), 3.16 (s, 1H), 2.90 - 2.73 (m, 4H), 2.62 (d, *J* = 7.8 Hz, 1H), 2.54 (s, 1H), 2.44 (s, 3H), 2.31 - 2.22 (m, 1H), 2.08 - 2.02 (m, 1H), 1.89 (s, 2H), 1.67 - 1.59 (m, 1H), 1.48 - 1.30 (m, 5H).

**Example 35 (1*r*,4*r*)-4-(((5-(2,2'-dichloro-3'-((2-(difluoromethyl)-7-(((*R*)-3-hydroxypyrrolidin-1-yl)methyl)pyrido[3,2-*d*]pyrimidin-4-yl)amino)-[1,1'-biphenyl]-3-yl)-3-methoxypyrazin-2-yl)methyl)(methyl)amino)cyclohexane-1-carboxylic acid**

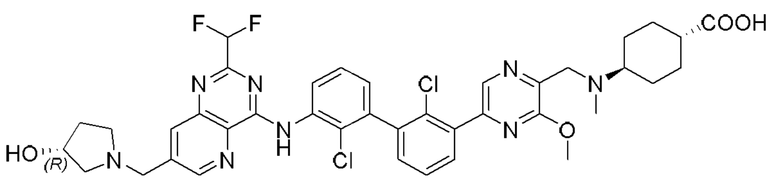

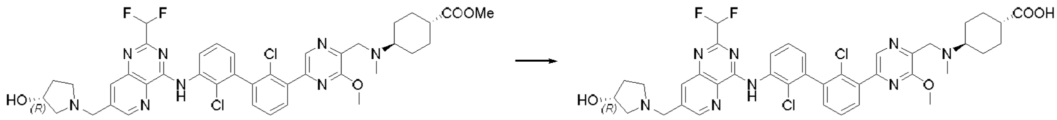

(1*r*,4*r*)-4-(((5-(2,2'-Dichloro-3'-((2-(difluoromethyl)-7-(((*R*)-3-hydroxypyrrolidin-1-yl) methyl)pyrido[3,2-*d*]pyrimidin-4-yl)amino)-[1,1'-biphenyl]-3-yl)-3-methoxypyrazin-2-yl)methyl) (methyl)amino)cyclohexane-1-carboxylic acid (0.86 g, 1.06 mmol) was dissolved in a mixed solution of 1,4-dioxane (80 mL) and water (16 mL), lithium hydroxide monohydrate (533.73 mg, 12.72 mmol) was added, and the reaction was stirred at room temperature for 22 h. After the reaction was complete, the system was concentrated and the residue was separated and purified by silica gel column chromatography (DCM/MeOH (v/v) = 8/1) to obtain 0.336 g of light orange powder with a yield of 39.76%.

MS (ESI, pos. ion) m/z: 793.25 $[M+H]^+$.

$^1H$ NMR (400 MHz, DMSO-$d_6$) δ 10.25 (s, 1H), 8.98 (s, 1H), 8.54 (d, *J* = 8.2 Hz, 1H), 8.43 (s, 1H), 8.24 (s, 1H), 7.74 (d, *J* = 7.6 Hz, 1H), 7.59 (q, *J* = 8.6 Hz, 2H), 7.49 (d, *J* = 7.6 Hz, 1H), 7.27 (d, *J* = 7.5 Hz, 1H), 6.83 (t, *J* = 54.4 Hz, 1H), 4.22 (s, 1H), 3.96 (s, 3H), 3.92 (s, 1H), 3.89 (s, 1H), 3.85 (s, 1H), 3.16 (s, 1H), 2.73 (dd, *J* = 15.2, 7.7 Hz, 3H), 2.64 (s, 1H), 2.45 (s, 1H), 2.43 (s, 1H), 2.33 (s, 3H), 2.13 (d, *J* = 11.3 Hz, 1H), 2.02 (dd, *J* = 13.5, 7.1 Hz, 1H), 1.97 (s, 1H), 1.93 (s, 1H), 1.90 (s, 1H), 1.59 (d, *J* = 10.0 Hz, 1H), 1.40 - 1.30 (m, 4H).

**Example 36 (*R*)-1-((2-((2,2'-dichloro-3'-((2-(difluoromethyl)-7-((3-hydroxypyrrolidin-1-yl)methyl)pyrido[3,2-*d*] pyrimidin-4-yl)amino)-[1,1'-biphenyl]-3-yl)amino)-3-fluoropyridin-4-yl)methyl)piperidine-4-carboxylic acid**

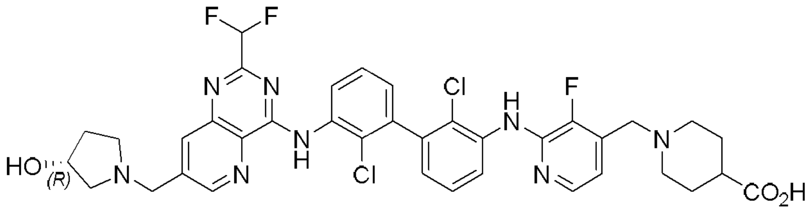

**Step 1: Synthesis of methyl 1-((2-((3'-amino-2,2'-dichloro-[1,1'-biphenyl]-3-yl)amino)-3-fluoropyridin-4-yl) methyl)piperidine-4-carboxylate**

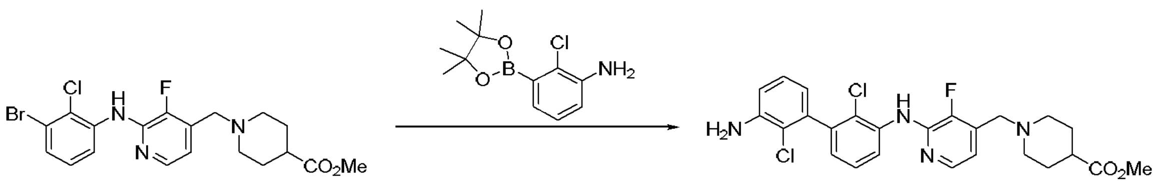

1

Methyl 1-((2-((3-bromo-2-chlorophenyl)amino)-3-fluoropyridin-4-yl)methyl)piperidine-4-carboxylate (0.60 g, 1.31 mmol) and 2-chloro-3-(4,4,5,5-tetramethyl-1,3,2-dioxaborolan-2-yl)aniline (0.43 g, 1.70 mmol) were dissolved in a mixed solvent of water (6 mL) and 1,4-dioxane (30 mL), then potassium carbonate (470.74 mg, 3.41 mmol) and Pd(dppf) $Cl_2$ (95.85 mg, 0.13 mmol) were added in sequence, the mixture was protected by nitrogen and heated to 90 °C for 16 h. The reaction was stopped and heated, and the mixture was filtered and concentrated. The remaining liquid was extracted with ethyl acetate three times (10 ml × 3). The organic phases were combined, dried over anhydrous sodium sulfate, filtered, and the filtrate was concentrated. Silica gel column chromatography was used for separation and purification (DCM/MeOH (v/v) = 40/1) to obtain 0.66 g of brown-yellow solid with a yield of 99.80%.
MS (ESI, pos. ion) m/z: 503.1 $[M+H]^+$.

**Step 2: Synthesis of methyl 1-((2-((3'-((7-bromo-2-(difluoromethyl)pyrido[3,2-*d*]pyrimidin-4-yl)amino)-2,2'-dichloro-[1,1'-biphenyl]-3-yl)amino)-3-fluoropyridin-4-yl)methyl)piperidine-4-carboxylate**

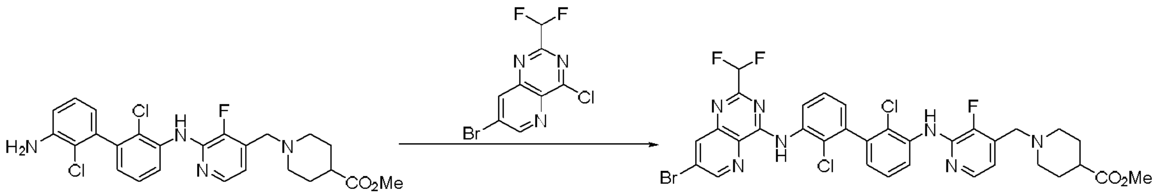

7-Bromo-4-chloro-2-(difluoromethyl)pyrido[3,2-*d*]pyrimidine (380 mg, 1.29 mmol), methyl 1-((2-((3'-amino-2,2'-dichloro-[1,1'-biphenyl]-3-yl)amino)-3-fluoropyridin-4-yl)methyl)piperidine-4-carboxylate (0.66 g, 1.30 mmol) were dissolved in *tert*-butanol (120 mL), 4M hydrochloric acid (1.29 mL, 5.16 mmol) was added, and the system was stirred at 130 °C for 10 h under $N_2$. The reaction was stopped and stirred, and the system was concentrated under reduced pressure. Water (10 mL) and DCM (10 mL) were added to dilute the system, and then saturated potassium carbonate solution (10 mL) was added. The mixture was extracted with DCM (10 mL × 3). The organic phases were combined and dried over anhydrous sodium sulfate. The mixture was filtered off under reduced pressure and washed with DCM (10 mL), concentrated under reduced pressure, and purified by column chromatography (DCM/MeOH (v/v) = 40/1) to obtain 770 mg of a light yellow powder with a yield of 78.37%.
MS (ESI, pos. ion) m/z: 760.07 $[M+H]^+$.

**Step 3: Synthesis of methyl 1-((2-((2,2'-dichloro-3'-((2-(difluoromethyl)-7-vinylpyrido[3,2-*d*]pyrimidin-4-yl)amino)-[1,1'-biphenyl]-3-yl)amino)-3-fluoropyridin-4-yl)methyl)piperidine-4-carboxylate**

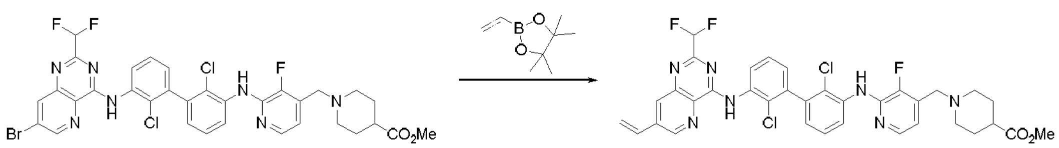

Methyl 1-((2-((3'-((7-bromo-2-(difluoromethyl)pyrido[3,2-d]pyrimidin-4-yl)amino)-2,2'-dichloro-[1,1'-biphenyl]-3-yl)amino)-3-fluoropyridin-4-yl)methyl)piperidine-4-carboxylate (855 mg, 1.12 mmol) was dissolved in a mixed solvent of 1,4-dioxane (100 mL) and water (20 mL), 2-vinyl-4,4,5,5-tetramethyl-1,3,2-dioxaborolane (862.46 mg, 5.60 mmol), Pd(dppf)$Cl_2$ $CH_2Cl_2$ (320.12 mg, 0.39 mmol) and potassium phosphate (475.48 mg, 2.24 mmol) were added in sequence. The mixture was stirred at 100 °C for 3 h under $N_2$. The system was cooled to room temperature and diluted with water (15 mL) and EA (15 mL). The liquids were separated, the aqueous phase was extracted with EA (15 mL × 3), the organic phases were combined and dried over anhydrous sodium sulfate, filtered under reduced pressure and washed with EA (15 mL), the filtrate was concentrated under reduced pressure, and the residue was separated and purified by column chromatography (DCM/MeOH (v/v) = 30/1) to obtain 795 mg of a brown-yellow viscous product with a yield of 99.92%.
MS (ESI, pos. ion) m/z: 708.18 $[M+H]^+$.

**Step 4: Synthesis of methyl 1-((2-((2,2'-dichloro-3'-((2-(difluoromethyl)-7-formylpyrido[3,2-*d*]pyrimidin-4-yl)amino)-[1,1'-biphenyl]-3-yl)amino)-3-fluoropyridin-4-yl)methyl)piperidine-4-carboxylate**

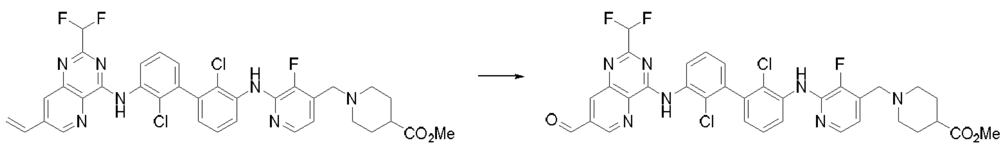

Methyl 1-((2-((2,2'-dichloro-3'-((2-(difluoromethyl)-7-vinylpyrido[3,2-*d*]pyrimidin-4-yl)amino)-[1,1'-biphenyl]-3-yl)amino)-3-fluoropyridin-4-yl)methyl)piperidine-4-carboxylate (701.47 mg, 0.99 mmol) was dissolved in 1,4-dioxane (70 mL) and water (28 mL), sodium periodate (1058.76 mg, 4.95 mmol) and potassium osmate dihydrate (36.48 mg, 0.099 mmol) were added, and the reaction was stirred at room temperature for 1.5 h. The reaction was quenched with saturated sodium bicarbonate solution (4 ml). The layers were separated and the aqueous phase was extracted with EA (40 mL×3). The organic phases were combined and dried over anhydrous sodium sulfate, filtered under reduced pressure and

washed with EtOAc (10 mL). The filtrate was concentrated under reduced pressure and the residue was separated and purified by silica gel column chromatography (DCM/MeOH (v/v) = 10/1) to give 216 mg of a yellow powder with a yield of 30.71%.
MS (ESI, pos. ion) m/z: 710.15 $[M+H]^+$.

**Step 5: Synthesis of methyl (*R*)-1-((2-((2,2'-dichloro-3'-((2-(difluoromethyl)-7-((3-hydroxypyrrolidin-1-yl)methyl) pyrido[3,2-*d*]pyrimidin-4-yl)amino)-[1,1'-biphenyl]-3-yl)amino)-3-fluoropyridin-4-yl)methyl)piperidine-4-carboxylate**

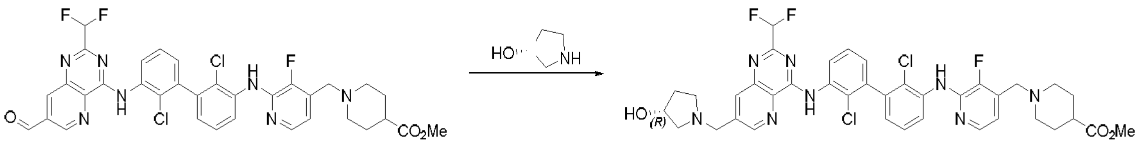

Methyl 1-((2-((2,2'-dichloro-3'-((2-(difluoromethyl)-7-formylpyrido[3,2-*d*]pyrimidin-4-yl)amino)-[1,1'-biphenyl]-3-yl)amino)-3-fluoropyridin-4-yl)methyl)piperidine-4-carboxylate (0.266 g, 0.37 mmol) and (*R*)-pyrrolidin-3-ol (161.17 mg, 1.85 mmol) were dissolved in methanol (30 mL) and dichloromethane (10 mL), acetic acid (111.09 mg, 1.85 mmol) was added, and the system was stirred at room temperature for 2 h. Sodium cyanoborohydride (69.75 mg, 1.11 mmol) was added and the reaction was continued at room temperature for 1.5 h. The reaction was stopped, the system was concentrated, and then extracted with EtOAc (10 ml $\times$ 2). The organic phases were combined and washed with saturated sodium bicarbonate (20 ml), water (20 ml), and saturated brine (20 ml) in sequence. The organic phases were collected and dried over anhydrous sodium sulfate. The mixture was filtered and the filtrate was evaporated again, and then separated and purified by silica gel column chromatography (DCM/MeOH (v/v) = 12/1) to obtain 0.116 g of light yellow powder with a yield of 39.64%.
MS (ESI, pos. ion) m/z: 781.23 $[M+H]^+$.

**Step 6: Synthesis of (*R*)-1-((2-((2,2'-dichloro-3'-((2-(difluoromethyl)-7-((3-hydroxypyrrolidin-1-yl)methyl)pyrido [3,2-*d*]pyrimidin-4-yl)amino)-[1,1'-biphenyl]-3-yl)amino)-3-fluoropyridin-4-yl)methyl)piperidine-4-carboxylic acid**

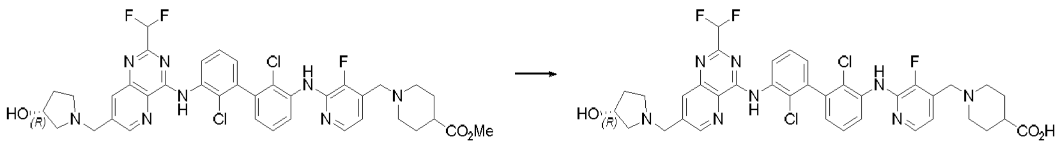

Methyl (*R*)-1-((2-((2,2'-dichloro-3'-((2-(difluoromethyl)-7-((3-hydroxypyrrolidin-1-yl)methyl)pyrido[3,2-*d*]pyrimidin-4-yl)amino)-[1,1'-biphenyl]-3-yl)amino)-3-fluoropyridin-4-yl)methyl)piperidine-4-carboxylate (116 mg, 0.15 mmol) was dissolved in a mixed solvent of 1,4-dioxane (15 mL) and water (3 mL), and a solution of lithium hydroxide monohydrate (75.53 mg, 1.80 mmol) was added, and the system was stirred at room temperature for 13 h. The mixture was diluted with water (5 ml), the pH was adjusted to 5 with dilute hydrochloric acid (5 ml), and the filtrate was concentrated. The product was separated and purified by silica gel column chromatography (dichloromethane/methanol (v/v) = 6/1) to obtain 0.1 g of light yellow powder with a yield of 87.78%.

MS (ESI, pos. ion) m/z: 767.2 $[M+H]^+$.
$^1H$ NMR (400 MHz, DMSO-$d_6$) $\delta$ 10.26 (s, 1H), 9.00 (d, *J* = 1.9 Hz, 1H), 8.58 (dd, *J* = 8.2, 1.6 Hz, 1H), 8.24 (d, *J* = 1.9 Hz, 1H), 8.17 - 8.06 (m, 2H), 7.92 (d, *J* = 5.1 Hz, 1H), 7.58 (t, *J* = 7.9 Hz, 1H), 7.44 (t, *J* = 7.9 Hz, 1H), 7.23 (dd, *J* = 7.6, 1.6 Hz, 1H), 7.09 (dd, *J* = 7.6, 1.6 Hz, 1H), 6.95 - 6.81 (m, 2H), 4.22 (dq, *J* = 6.7, 3.6 Hz, 1H), 3.92 (d, *J* = 14.2 Hz, 1H), 3.85 (d, *J* = 14.1 Hz, 1H), 3.55 (s, 2H), 2.76 (ddd, *J* = 16.1, 11.1, 4.9 Hz, 3H), 2.68 (t, *J* = 7.6 Hz, 1H), 2.47 (d, *J* = 8.3 Hz, 1H), 2.42 (dd, *J* = 9.7, 3.6 Hz, 1H), 2.20 (tt, *J* = 11.3, 4.0 Hz, 1H), 2.05 (ddd, *J* = 16.8, 13.0, 8.3 Hz, 3H), 1.79 (dd, *J* = 13.2, 3.7 Hz, 2H), 1.63 - 1.51 (m, 3H), 1.34 (d, *J* = 5.9 Hz, 1H).

**Example 37 (1*r*,4*r*)-4-(((6-(3'-((7-(((*R*)-3-acetamidopyrrolidin-1-yl)methyl)-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)(methyl)amino)cyclohexane-1-carboxylic acid**

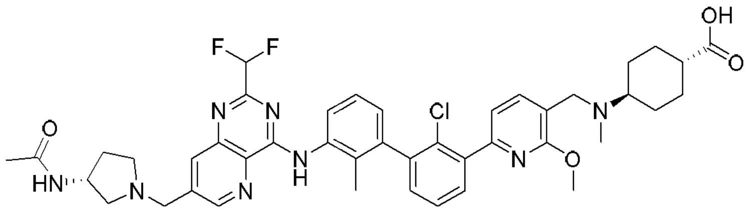

**Step 1: Synthesis of *tert*-butyl (*R*)-3-acetamidopyrrolidine-1-carboxylate**

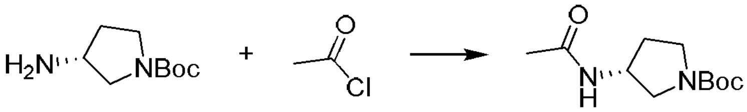

(*R*)-1-Boc-3-aminopyrrolidine (2 g, 10.74 mmol), DCM (50 mL) and TEA (1.63 g, 16.11 mmol) were added into a 100 mL two-necked flask in sequence. The temperature was lowered to 0 °C under nitrogen protection, and acetyl chloride (0.84 g, 10.74 mmol) was slowly added dropwise and reacted at room temperature. After the reaction was complete, the reaction solution was slowly added dropwise to water (100 mL) to quench the reaction, and then extracted with DCM (20 mL×3). The organic phases were combined, washed with water (60 mL×1), dried over anhydrous sodium sulfate, filtered, and the filtrate was concentrated under reduced pressure to remove the solvent to obtain 2.40 g of a colorless transparent liquid product with a yield of 97.90%.

**Step 2: Synthesis of (*R*)-*N*-(pyrrolidin-3-yl)acetamide hydrochloride**

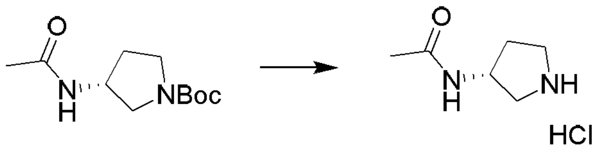

*Tert*-butyl (*R*)-3-acetamidopyrrolidine-1-carboxylate (2.35 g, 10.29 mmol) and DCM (15 mL) were added into a 100 mL single-necked flask in sequence. The temperature was lowered to 0 °C under nitrogen protection, and hydrochloric acid (3.75 g, 102.90 mmol) was slowly added dropwise and reacted at room temperature under $N_2$. After the reaction was complete, the mixture was concentrated under reduced pressure to obtain 1.60 g of a brown oily product with a yield of 94.41%.
MS (ESI, pos. ion) m/z: 129.10 $[M+H]^+$.

**Step 3: Synthesis of methyl (1*r*,4*r*)-4-(((6-(3'-((7-(((*R*)-3-acetamidopyrrolidin-1-yl)methyl)-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)(methyl)amino)cyclohexane-1-carboxylate**

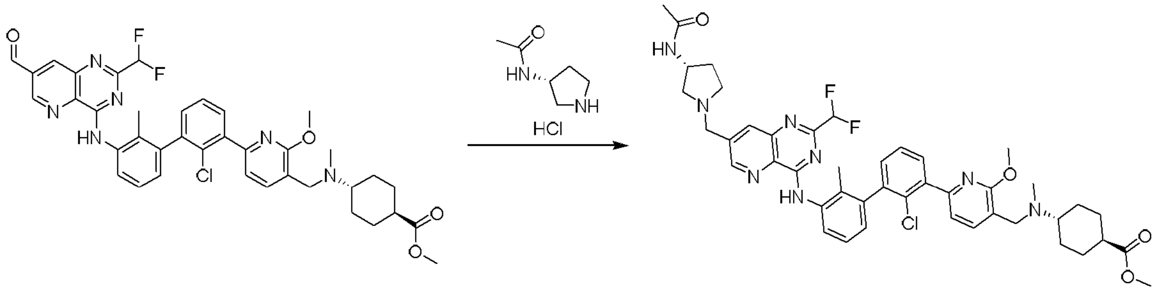


To a 50 mL single-necked bottle, methyl (1*r*,4*r*)-4-(((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)(methyl)amino)cyclohexane-1-carboxylate (150 mg, 0.21 mmol), (R)-*N*-(pyrrolidin-3-yl)acetamide hydrochloride (138.29 mg, 0.84 mmol), DCM (8 mL), DMF (8 mL), TEA (31.87 mg, 0.32 mmol) and acetic acid (18.92 mg, 0.32 mmol) were added in sequence and reacted at room temperature for 1.5 h. Sodium cyanoborohydride (26.39 mg, 0.42 mmol) was added and the reaction was continued at room temperature. After the reaction was completed, the pH was adjusted to 7~8 with 1N $K_2CO_3$ aqueous solution to quench the reaction, and the mixture was diluted with water (200 mL), and then extracted with DCM (20 mL × 3). The organic phases were combined, washed with water (200 mL × 2), dried over anhydrous sodium sulfate, filtered, and the filtrate was concentrated under reduced pressure to remove the solvent. The residue was separated and purified by silica gel column chromatography (eluent: DCM/MeOH (v/v) = 10/1) to obtain 130.0 mg of a light yellow solid product with a yield of 74.92%.

MS (ESI, pos. ion) m/z: 827.36 $[M+H]^+$.

**Step 10: Synthesis of (1*r*,4*r*)-4-(((6-(3'-((7-(((*R*)-3-acetamidopyrrolidin-1-yl)methyl)-2-(difluoromethyl)pyrido[3,2-d]pyrimidin-4-yl)amino)-2-chloro-2'-methyl-[1,1'-biphenyl]-3-yl)-2-methoxypyridin-3-yl)methyl)(methyl)amino)cyclohexane-1-carboxylic acid**

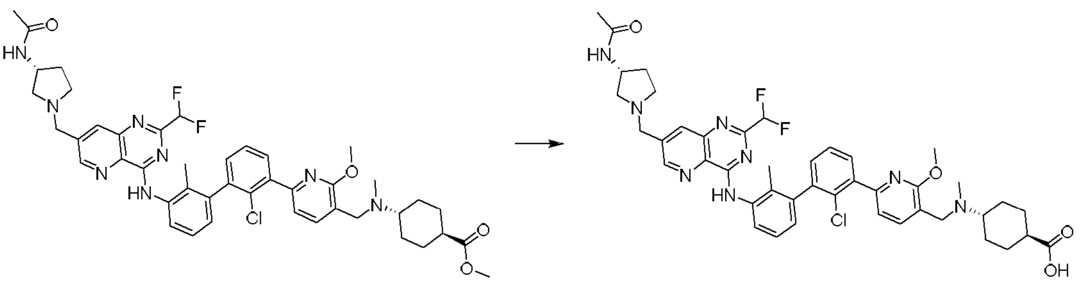

To a 25 mL single-necked bottle, methyl (1*r*,4*r*)-4-(((6-(3'-((7-(((*R*)-3-acetamidopyrrolidin-1-yl)methyl)-2-(difluoromethyl)pyrido[3,2-d]pyrimidin-4-yl)amino)-2-chloro-2'-methyl-[1,1'-biphenyl]-3-yl)-2-methoxypyridin-3-yl)methyl)(methyl)amino)cyclohexane-1-carboxylate (66 mg, 0.080 mmol), 1,4-dioxane (6 mL), water (2 mL) and lithium hydroxide monohydrate (6.71 mg, 0.16 mmol) were added in sequence and reacted at 50 °C. After the reaction was completed, the solvent was removed by concentration under reduced pressure, and the product was dissolved with DCM (6 mL) and MeOH (6 mL), and washed with hydrogen chloride dioxane solution (4.0 M) to adjust the pH to 5-6, concentrate under reduced pressure to remove the solvent, and the residue was separated and purified by preparative silica gel thin layer chromatography (developing solvent: DCM / MeOH (v/v) = 3/1) to obtain 20.1 mg of a light yellow solid product in a yield of 30.09%.

MS (ESI, pos. ion) m/z: 813.34 $[M+H]^+$.

$^1$H NMR (400 MHz, DMSO-$d_6$) δ 10.34 (s, 1H), 8.96 (s, 1H), 8.20 (s, 1H), 8.04 (d, *J* = 6.8 Hz, 1H), 7.81 (s, 1H), 7.74 (d, *J* = 8.0 Hz, 1H), 7.64 (d, *J* = 6.5 Hz, 1H), 7.54 (t, *J* = 7.6 Hz, 1H), 7.40-7.35 (m, 2H), 7.31 (s, 1H), 7.16 (d, *J* = 7.5 Hz, 1H), 6.71 (t, *J* = 54.5 Hz, 1H), 4.17 (s, 1H), 3.92 (s, 3H), 3.91 - 3.77 (m, 3H), 3.56 (s, 2H), 2.79-2.64 (m, 3H), 2.50 (s, 3H), 2.21-2.10 (m, 3H), 2.04 (s, 3H), 2.01-1.90 (m, 4H), 1.77 (s, 3H), 1.65 - 1.33 (m, 6H).

**Example 38 (1*r*,4*r*)-4-((((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyridin-2-yl)methyl)(methyl-$d_3$)amino)methyl)cyclohexane-1-carboxylic acid**

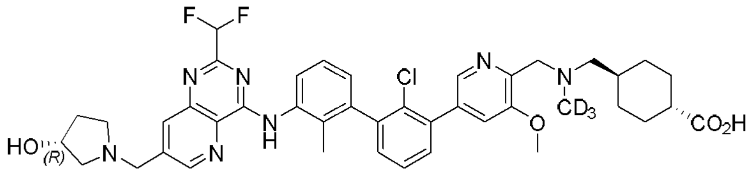

**Step 1: Synthesis of methyl (1*r*,4*r*)-4-((((5-(3-bromo-2-chlorophenyl)-3-methoxypyridin-2-yl)methyl)(methyl-$d_3$)amino)methyl)cyclohexane-1-carboxylate**

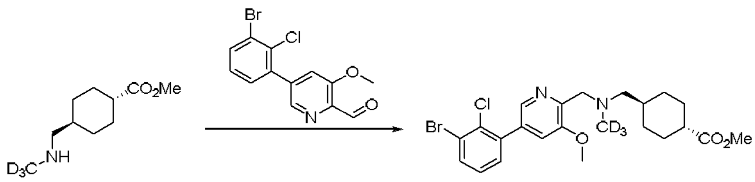

5-(3-Bromo-2-chlorophenyl)-3-methoxypyrazine-2-carbaldehyde (3.5 g, 10.7 mmol) and methyl (1*r*,4*r*)-4-(((methyl-$d_3$)amino)methyl)cyclohexane-1-carboxylate (1.61 g, 8.58 mmol) were dissolved in a mixed solvent of dichloromethane (30 mL) and 2,2,2-trifluoroethanol (30 mL), triethylamine (4.5 mL, 32.2 mmol) was added, and the system was stirred at room temperature overnight. The system was cooled to room temperature, sodium cyanoborohydride (0.67 g, 10.72 mmol) was added, and the system was stirred at room temperature for 5.5 h. After the stirring was stopped, the system was concentrated under reduced pressure, and the residue was diluted with water (30 mL) and dichloromethane (30 mL), and extracted with dichloromethane (30 mL × 3). The organic phases were combined and dried over anhydrous sodium sulfate, filtered under reduced pressure and washed with dichloromethane (15 mL). The filtrate was concentrated under reduced pressure, and the residue was separated and purified by column chromatography (PE/EA (v/v) = 1/2) to obtain 3.03 g of a yellow solid with a yield of 70.8%.
MS (ESI, pos. ion) m/z: 498.3 $[M+H]^+$.

**Step 2: Synthesis of methyl (1*r*,4*r*)-4-((((5-(3'-amino-2-chloro-2'-methyl-[1,1'-biphenyl]-3-yl)-3-methoxypyridin-2-yl)methyl)(methyl-$d_3$)amino)methyl)cyclohexane-1-carboxylate**

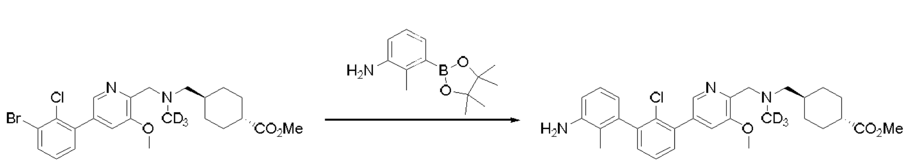

Methyl (1*r*,4*r*)-4-((((5-(3-bromo-2-chlorophenyl)-3-methoxypyridin-2-yl)methyl)(methyl-$d_3$)amino)methyl)cyclohexane-1-carboxylate (3.0 g, 6.0 mmol) and 2-methyl-3-(4,4,4,4-tetramethyl-1,3,2-dioxaborolan-2-yl)aniline (1.96 g, 8.41 mmol) were dissolved in a mixed solvent of 1,4-dioxane (40 mL) and water (5 mL). [1,1'-Bis(diphenylphosphino)ferrocene] palladium dichloride dichloromethane adduct (0.25 g, 0.30 mmol) and potassium carbonate (1.66 g, 12.02 mmol) were added. The system was stirred at 90 °C for 12 h under $N_2$. The stirring was stopped, and the system was diluted with water (20 mL), extracted with EA (20 mL × 3), the organic phases were combined and dried over anhydrous sodium sulfate, filtered under reduced pressure and washed with EA (10 mL), the filtrate was concentrated under reduced pressure, and the residue was separated and purified by column chromatography (PE/EA (v/v) = 1/4) to obtain 3.15 g of a reddish-brown solid with a yield of 99.8%.
MS (ESI, pos. ion) m/z: 525.2 $[M+H]^+$.

**Step 3: Synthesis of methyl (1*r*,4*r*)-4-((((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyridin-2-yl)methyl)(methyl-$d_3$)amino)methyl)cyclohexane-1-carboxylate**

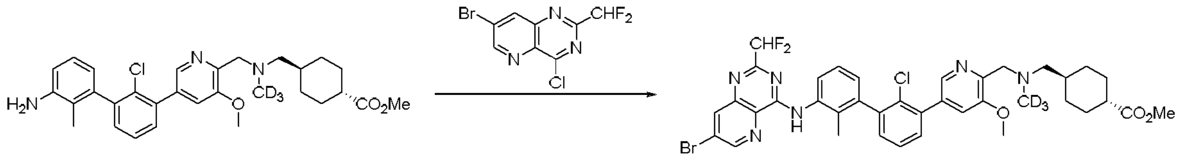

Methyl (1*r*,4*r*)-4-((((5-(3'-amino-2-chloro-2'-methyl-[1,1'-biphenyl]-3-yl)-3-methoxypyridin-2-yl)methyl)(methyl-$d_3$)amino)methyl)cyclohexane-1-carboxylate (3.15 g, 6.0 mmol) and 7-bromo-4-chloro-2-(difluoromethyl)pyrido[3,2-*d*]pyrimidine (2.12 g, 7.2 mmol) were added to *tert*-butanol (80 mL) and stirred at 100 °C for 3 h under $N_2$. The stirring was stopped, and the product was filtered off under reduced pressure and washed with ethanol (20 mL) to obtain 3.7 g of a yellow solid with a yield of 78.8%.
MS (ESI, pos. ion) m/z: 782.0 $[M+H]^+$.

**Step 4: Synthesis of methyl (1*r*,4*r*)-4-((((5-(2-chloro-3'-((2-(difluoromethyl)-7-vinylpyrido[3,2-*d*]pyrimidin-4-yl)amino)-2'-methyl-[1,1'-biphenyl]-3-yl)-3-methoxypyridin-2-yl)methyl)(methyl-$d_3$)amino)methyl)cyclohexane-1-carboxylate**

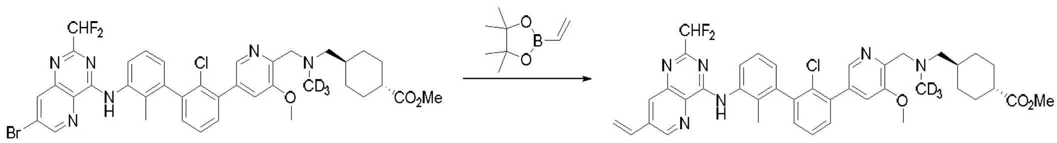

Methyl (1*r*,4*r*)-4-((((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyridin-2-yl)methyl)(methyl-$d_3$)amino)methyl)cyclohexane-1-carboxylate (3.7 g, 4.72 mmol) was dissolved in a mixed solvent of 1,4-dioxane (80 mL) and water (8 mL), 4,4,5,5-tetramethyl-2-vinyl-1,3,2-dioxaborolane (1.09 g, 7.08 mmol), [1,1'-Bis(diphenylphosphino)ferrocene]palladium dichloride dichloromethane adduct (0.37 g, 0.47 mmol) and potassium phosphate (2.17 g, 9.44 mmol) were added in sequence, and the system was stirred at 100 °C overnight. The stirring was stopped, the system was cooled to room temperature and diluted with water (20 mL) and EA (20 mL). The liquids were separated, the aqueous phase was extracted with EA (20 mL×3), the organic phases were combined and dried over anhydrous sodium sulfate, filtered under reduced pressure and washed with EA (10 mL), the filtrate was concentrated under reduced pressure, and the residue was separated and purified by column chromatography (DCM/MeOH (v/v) =30/1) to obtain 3.4 g of a yellow solid with a yield of 98.5%.
MS (ESI, pos. ion) m/z: 730.6 $[M+H]^+$.

**Step 5: Synthesis of methyl (1*r*,4*r*)-4-((((5-(2-chloro-3'-((2-(difluoromethyl)-7-formylpyrido[3,2-*d*]pyrimidin-4-yl)amino)-2'-methyl-[1,1'-biphenyl]-3-yl)-3-methoxypyridin-2-yl)methyl)(methyl-$d_3$)amino)methyl)cyclohexane-1-carboxylate**

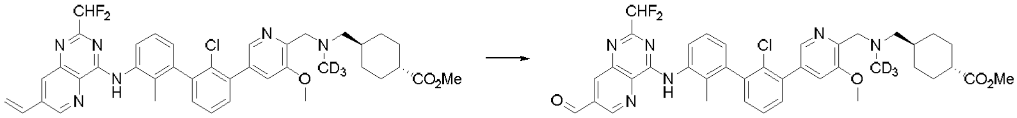

Methyl (1*r*,4*r*)-4-((((5-(2-chloro-3'-((2-(difluoromethyl)-7-vinylpyrido[3,2-*d*]pyrimidin-4-yl)amino)-2'-methyl-[1,1'-biphenyl]-3-yl)-3-methoxypyridin-2-yl)methyl)(methyl-$d_3$)amino)methyl)cyclohexane-1-carboxylate (3.4 g, 4.66 mmol) was dissolved in 1,4-dioxane (40 mL) and water (20 mL), potassium osmate dihydrate (34 mg, 0.093 mmol) and sodium periodate (2.49 g, 11.65 mmol) were added in sequence, and the system was stirred at room temperature for

6.5 h. The stirring was stopped, and the system was filtered under reduced pressure. The filtrate was separated, the aqueous phase was extracted with EA (10 mL×3), the organic phases were combined and dried over anhydrous sodium sulfate, filtered under reduced pressure and washed with EA (10 mL), the filtrate was concentrated under reduced pressure, and the residue was separated and purified by column chromatography (DCM/MeOH (v/v) =50/1) to obtain 2.0 g of a yellow solid with a yield of 58.7%.
MS (ESI, pos. ion) m/z: 732.5 $[M+H]^+$.

**Step 6: Synthesis of methyl (1*r*,4*r*)-4-((((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyridin-2-yl)methyl)(methyl-*d*$_3$)amino)methyl)cyclohexane-1-carboxylate**

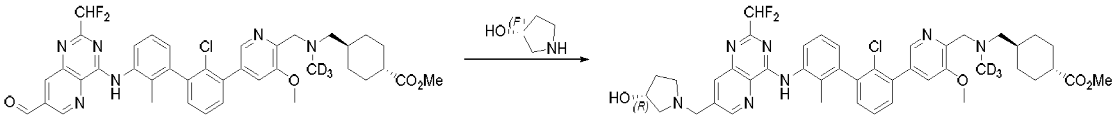

Methyl (1*r*,4*r*)-4-((((5-(2-chloro-3'-((2-(difluoromethyl)-7-formylpyrido[3,2-*d*]pyrimidin-4-yl)amino)-2'-methyl-[1,1'-biphenyl]-3-yl)-3-methoxypyridin-2-yl)methyl)(methyl-*d*$_3$)amino)methyl)cyclohexane-1-carboxylate (2.0 g, 2.73 mmol) and (3*R*)-pyrrolidin-3-ol (0.48 mL, 5.46 mmol) were dissolved in a mixed solvent of methanol (20 mL) and dichloromethane (20 mL), acetic acid (0.10 mL, 1.7 mmol) was added, and the system was stirred at room temperature overnight. Sodium cyanoborohydride (0.17 g, 2.73 mmol) was added, and the system was stirred at room temperature for 5.5 h. The reaction was stopped and stirred. The system was concentrated under reduced pressure and diluted with water (10 mL) and DCM (10 mL). Saturated potassium carbonate solution (5 mL) was added to adjust the pH of the system to 9-10. The layers were separated and the aqueous phase was extracted with DCM (10 mL × 3). The organic phases were combined and dried over anhydrous sodium sulfate. The mixture was filtered under reduced pressure and washed with DCM (10 mL). The filtrate was concentrated under reduced pressure and the residue was separated and purified by silica gel column chromatography (DCM/MeOH (v/v) = 50/1) to obtain 1.0 g of yellow solid with a yield of 45.6%.
MS (ESI, pos. ion) m/z: 803.7 $[M+H]^+$.

**Step 7: Synthesis of (1*r*,4*r*)-4-((((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyridin-2-yl)methyl)(methyl-*d*$_3$)amino)methyl)cyclohexane-1-carboxylic acid**

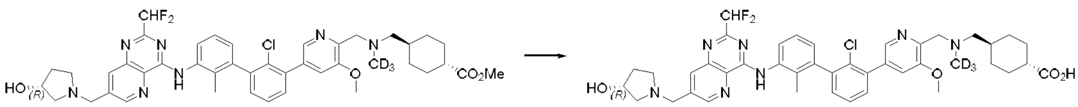

Methyl (1*r*,4*r*)-4-((((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyridin-2-yl)methyl)(methyl-*d*$_3$)amino)methyl)cyclohexane-1-carboxylate (1.0 g, 1.24 mmol) was dissolved in 1,4-dioxane (20 mL) and water (5 mL), and a solution of lithium hydroxide monohydrate (0.52 g, 12.4 mmol) was added, and the system was stirred at room temperature for 5 h. The stirring was stopped, and a dilute hydrochloric acid solution was added to adjust the pH of the system to 5-6. The liquids were separated, the aqueous phase was extracted with DCM (10 mL×3), the organic phases were combined and dried over anhydrous sodium sulfate, filtered under reduced pressure and washed with DCM (10 mL), the filtrate was concentrated under reduced pressure, and the residue was separated and purified by column chromatography (DCM/MeOH (v/v) =7/1) to obtain 0.85 g of a yellow solid with a yield of 86.5%.

MS (ESI, pos. ion) m/z: 789.7 $[M+H]^+$.

$^1$H NMR (400 MHz, DMSO-*d*$_6$) δ 10.31 (s, 1H), 8.93 (s, 1H), 8.15 (s, 1H), 7.89 - 7.68 (m, 2H), 7.62 (d, *J* = 7.4 Hz, 1H), 7.50 (t, *J* = 7.7 Hz, 1H), 7.35 (s, 2H), 7.31 - 7.23 (m, 1H), 7.19 - 7.08 (m, 1H), 6.69 (t, *J* = 54.4 Hz, 1H), 5.73 (s, 1H), 4.21 (s, 1H), 3.85 (d, *J* = 21.2 Hz, 5H), 3.42 (s, 2H), 2.79 - 2.59 (m, 3H), 2.22 - 2.11 (m, 2H), 2.00 (d, *J* = 19.0 Hz, 5H), 1.80 (d, *J* = 27.8 Hz, 10H), 1.26 (dd, *J* = 28.6, 15.2 Hz, 2H).

**Example 39 (1*r*,4*r*)-4-((((6-(2,2'-dichloro-3'-((2-(difluoromethyl)-7-(((*R*)-3-hydroxypyrrolidin-1-yl)methyl)pyrido [3,2-*d*]pyrimidin-4-yl)amino)-[1,1'-biphenyl]-3-yl)-2-methoxypyridin-3-yl)methyl)(methyl)amino)methyl)cyclohexane-1-carboxylic acid**

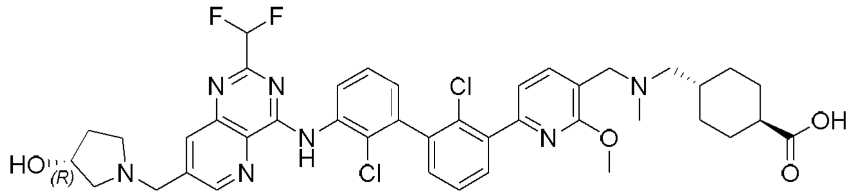

**Step 1: Synthesis of methyl (1*r*,4*r*)-4-((((6-(3-bromo-2-chlorophenyl)-2-methoxypyridin-3-yl)methyl)amino) methyl)cyclohexane-1-carboxylate**

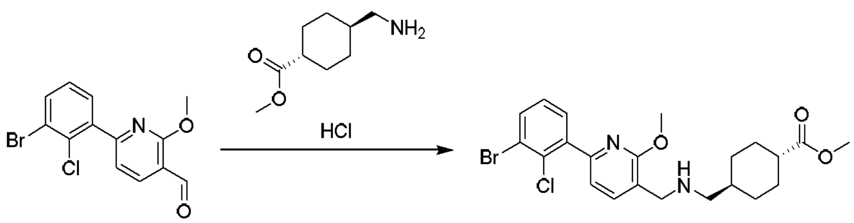


6-(3-Bromo-2-chlorophenyl)-2-methoxynicotinaldehyde (3.5 g, 10.72mmol), methyl (1*s*,4*s*)-4-(aminomethyl) cyclohexane-1-carboxylate hydrochloride (4.45 g, 21.44 mmol), DCM (60 mL), MeOH (60 mL), TEA (1.63 g, 16.08 mmol) and acetic acid (0.97 g, 16.08 mmol) were added to a 250 mL single-necked bottle in sequence, and the mixture was reacted at room temperature overnight. Sodium cyanoborohydride (1.35 g, 21.44 mmol) was added and the reaction was continued at room temperature. After the reaction was completed, the pH was adjusted to 7~8 with 1N $K_2CO_3$ aqueous solution, and the organic solvent was removed by concentration under reduced pressure. Water (300 mL) was added, and then extracted with DCM (50 mL × 3). The organic phases were combined, washed with water (200 mL × 1), dried over anhydrous sodium sulfate, filtered, and the filtrate was concentrated under reduced pressure to remove solution. The residue was separated and purified by silica gel column chromatography (eluent: DCM/MeOH (v/v) = 40/1) to obtain 5.05 g of a yellow oily product with a yield of 97.80%.
MS (ESI, pos. ion) m/z: 480.08 $[M+H]^+$.

**Step 2: Synthesis of methyl (1*r*,4*r*)-4-((((6-(3-bromo-2-chlorophenyl)-2-methoxypyridin-3-yl)methyl)(methyl) amino)methyl)cyclohexane-1-carboxylate**

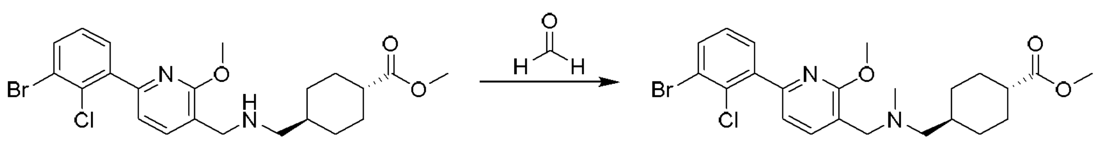

To a 250 mL single-necked bottle, methyl (1*r*,4*r*)-4-((((6-(3-bromo-2-chlorophenyl)-2-methoxypyridin-3-yl) methyl)amino)methyl)cyclohexane-1-carboxylate (5.01 g, 10.40 mmol), formaldehyde (2.34 g, 31.20 mmol, wt 40%), methanol (60 mL), DCM (60 mL), TEA(1.58 g, 15.60 mmol) and acetic acid (0.75 g, 12.48 mmol) were added in sequence, and then stirred at room temperature for 5 h. Sodium cyanoborohydride (2.61 g, 41.6 mmol) was added and the reaction was continued at room temperature. After the reaction was completed, the pH was adjusted to 7~8 with 1N $K_2CO_3$ aqueous solution, and the organic solvent was removed by concentration under reduced pressure. Water (300 mL) was added, and then extracted with DCM (50 mL × 3). The organic phases were combined, washed with water (150 mL × 1), dried over anhydrous sodium sulfate, filtered, and the filtrate was concentrated under reduced pressure to remove solution. The residue was separated and purified by silica gel column chromatography (eluent: DCM/MeOH (v/v) = 50/1) to

obtain 4.95 g of a yellow solid product with a yield of 96.01%.
MS (ESI, pos. ion) m/z: 494.10 [M+H]$^+$.

**Step 3: Synthesis of methyl (1*r*,4*r*)-4-((((6-(3'-amino-2,2'-dichloro-[1,1'-biphenyl]-3-yl)-2-methoxypyridin-3-yl) methyl)(methyl)amino)methyl)cyclohexane-1-carboxylate**

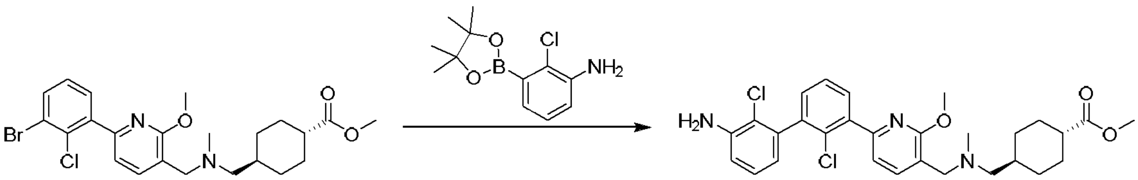

To a 250 mL microwave tube, methyl (1*r*,4*r*)-4-((((6-(3-bromo-2-chlorophenyl)-2-methoxypyridin-3-yl)methyl) (methyl)amino)methyl)cyclohexane-1-carboxylate (4.5 g, 9.08 mmol), 2-chloro-3-(4,4,5,5-tetramethyl-1,3,2-dioxaborolan-2-yl)aniline (2.76 g, 10.90 mmol), potassium carbonate (3.14 g, 22.7 mmol), [1,1'-Bis(diphenylphosphino)ferrocene] palladium dichloride dichloromethane adduct (0.74 g, 0.91 mmol), 1,4-dioxane (100 mL) and water (20 mL) were added in sequence. The mixture was reacted at 100 °C under $N_2$. After the reaction was complete, the reaction solution was concentrated under reduced pressure to remove dioxane, water (300 mL) was added, and then extracted with DCM (50 mL × 3). The organic phases were combined, washed with saturated brine (150 mL × 1), dried over anhydrous sodium sulfate, filtered, and the filtrate was concentrated under reduced pressure. The residue was separated and purified by silica gel column chromatography (eluent: DCM/MeOH (v/v) = 40/1) to obtain 4.75 g of a light yellow oily product with a yield of 96.48%.
MS (ESI, pos. ion) m/z: 542.20 [M+H]$^+$.

**Step 4: Synthesis of methyl (1*r*,4*r*)-4-((((6-(3'-((7-bromo-2-(difluoromethyl)pyrido[3,2-*d*]pyrimidin-4-yl)amino)-2,2'-dichloro-[1,1'-biphenyl]-3-yl)-2-methoxypyridin-3-yl)methyl)(methyl)amino)methyl)cyclohexane-1-carboxylate**

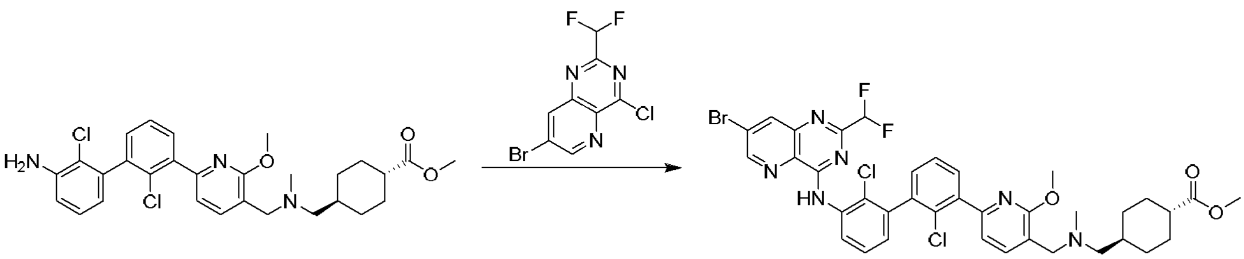

To a 250 mL single-necked bottle, methyl (1r,4r)-4-((((6-(3'-amino-2,2'-dichloro-[1,1'-biphenyl]-3-yl)-2-methoxypyridin-3-yl)methyl)(methyl)amino)methyl)cyclohexane-1-carboxylate (4.79 g, 8.83 mmol), *tert*-butanol (100 mL) and 7-bromo-4-chloro-2-(difluoromethyl)pyrido[3,2-d]pyrimidine (2.86 g, 9.71 mmol)were added in sequence and reacted at 100 °C. After the reaction was complete, the reaction solution was concentrated under reduced pressure to remove the organic solvent, water (300 mL) was added, and then extracted with DCM (50 mL × 3). The organic phases were combined, washed with saturated brine (150 mL × 1), dried over anhydrous sodium sulfate, filtered, and the filtrate was concentrated under reduced pressure to remove solution. The residue was separated and purified by silica gel column chromatography (eluent: DCM/MeOH (v/v) = 60/1) to obtain 7.07 g of a yellow solid product with a yield of 92.24%.
MS (ESI, pos. ion) m/z: 798.13 [M+H]$^+$.

**Step 5: Synthesis of methyl (1*r*,4*r*)-4-(((6-(2,2'-dichloro-3'-((2-(difluoromethyl)-7-vinylpyrido[3,2-*d*]pyrimidin-4-yl)amino)-[1,1'-biphenyl]-3-yl)-2-methoxypyridin-3-yl)methyl)(methyl)amino)methyl)cyclohexane-1-carboxylate**

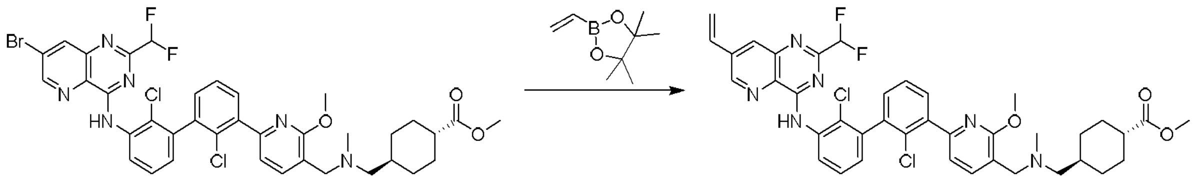

To a 500 mL single-necked bottle, methyl (1*r*,4*r*)-4-((((6-(3'-((7-bromo-2-(difluoromethyl)pyrido[3,2-d]pyrimidin-4-yl)amino)-2,2'-dichloro-[1,1'-biphenyl]-3-yl)-2-methoxypyridin-3-yl)methyl)(methyl)amino)methyl)cyclohexane-1-carboxylate (6.00 g, 7.50 mmol), 4,4,5,5-tetramethyl-2-vinyl-1,3,2-dioxaborolane (3.47 g, 22.5 mmol), potassium phosphate (3.98 g, 18.75 mmol), [1,1'-Bis(diphenylphosphino)ferrocene]palladium dichloride dichloromethane adduct (0.61 g, 0.75 mmol), 1,4-dioxane (200 mL) and water (40 mL) were added in sequence, and the reaction was carried out at 100 °C. After the reaction was complete, the reaction solution was concentrated under reduced pressure, water (500 mL) was added, and then extracted with DCM (100 mL × 3). The organic phases were combined, washed with saturated brine (300 mL × 1), dried over anhydrous sodium sulfate, filtered, and the filtrate was concentrated under reduced pressure. The residue was separated and purified by silica gel column chromatography (eluent: DCM/MeOH (v/v) = 50/1) to obtain 5.05 g of a white solid product with a yield of 90.12%.
MS (ESI, pos. ion) m/z: 747.24 $[M+H]^+$.

**Step 6: Synthesis of methyl (1*r*,4*r*)-4-((((6-(2,2'-dichloro-3'-((2-(difluoromethyl)-7-formylpyrido[3,2-*d*]pyrimidin-4-yl)amino)-[1,1'-biphenyl]-3-yl)-2-methoxypyridin-3-yl)methyl)(methyl)amino)methyl)cyclohexane-1-carboxylate**

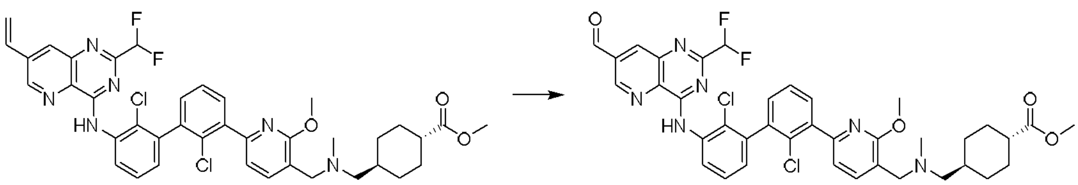

To a 250 mL single-necked bottle, methyl (1*r*,4*r*)-4-((((6-(2,2'-dichloro-3'-((2-(difluoromethyl)-7-vinylpyrido[3,2-d]pyrimidin-4-yl)amino)-[1,1'-biphenyl]-3-yl)-2-methoxypyridin-3-yl)methyl)(methyl)amino)methyl)cyclohexane-1-carboxylate (2.85 g, 3.81 mmol), 1,4-dioxane (75 mL), water (30 mL), potassium osmate dihydrate (0.14 g, 0.38 mmol) and sodium periodate (4.07 g, 19.05 mmol) were added in sequence and reacted at room temperature. After the reaction was complete, the reaction was quenched with saturated aqueous sodium sulfite solution (20 mL), concentrated under reduced pressure, water (300 mL) was added, and then extracted with DCM (70 mL × 3). The organic phases were combined, washed with water (200 mL × 1), dried over anhydrous sodium sulfate, filtered, and the filtrate was concentrated under reduced pressure. The residue was separated and purified by silica gel column chromatography (eluent: DCM/MeOH (v/v) = 30/1) to obtain 1.56 g of a yellow solid product with a yield of 54.59%.
MS (ESI, pos. ion) m/z: 749.22 $[M+H]^+$.

**Step 7: Synthesis of methyl (1*r*,4*r*)-4-(((6-(2,2'-dichloro-3'-((2-(difluoromethyl)-7-(((*R*)-3-hydroxypyrrolidin-1-yl)methyl)pyrido[3,2-d]pyrimidin-4-yl)amino)-[1,1'-biphenyl]-3-yl)-2-methoxypyridin-3-yl)methyl)(methyl)amino)methyl)cyclohexane-1-carboxylate**

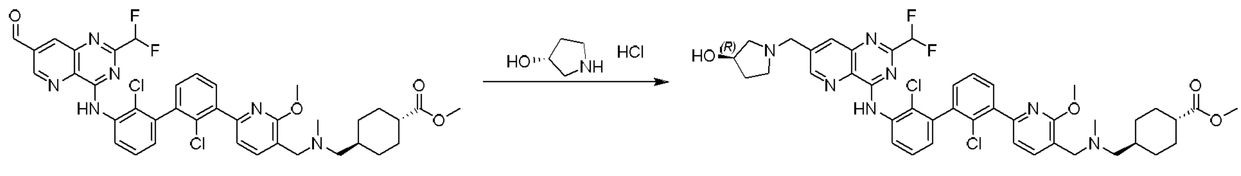


To a 100 mL single-necked bottle, methyl (1*r*,4*r*)-4-((((6-(2,2'-dichloro-3'-((2-(difluoromethyl)-7-formylpyrido[3,2-d]pyrimidin-4-yl)amino)-[1,1'-biphenyl]-3-yl)-2-methoxypyridin-3-yl)methyl)(methyl)amino)methyl)cyclohexane-1-carboxylate (1.2 g, 1.60 mmol), (R)-pyrrolidin-3-ol hydrochloride (0.99 g, 8 mmol), DCM (15 mL), MeOH (15 mL), TEA

(0.32 g, 3.2 mmol) and acetic acid (0.14 g, 2.40 mmol) were added in sequence and reacted at 50 °C for 1 h. Sodium cyanoborohydride (0.20 g, 3.2 mmol) was added and the reaction was continued at room temperature. After the reaction was completed, the pH was adjusted to 7~8 with 1N $K_2CO_3$ aqueous solution, and the mixture was concentrated under reduced pressure. Water (150 mL) was added, and then extracted with DCM (15 mL × 3). The organic phases were combined, washed with water (50 mL × 1), dried over anhydrous sodium sulfate, filtered, and the filtrate was concentrated under reduced pressure. The residue was separated and purified by silica gel column chromatography (eluent: DCM/MeOH (v/v) = 30/1) to obtain 0.75 g of a yellow solid product with a yield of 57.08%. MS (ESI, pos. ion) m/z: 820.30 $[M+H]^+$.

**Step 8: Synthesis of (1*r*,4*r*)-4-((((6-(2,2'-dichloro-3'-((2-(difluoromethyl)-7-(((*R*)-3-hydroxypyrrolidin-1-yl)methyl) pyrido[3,2-d]pyrimidin-4-yl)amino)-[1,1'-biphenyl]-3-yl)-2-methoxypyridin-3-yl)methyl)(methyl)amino)methyl) cyclohexane-1-carboxylic acid**

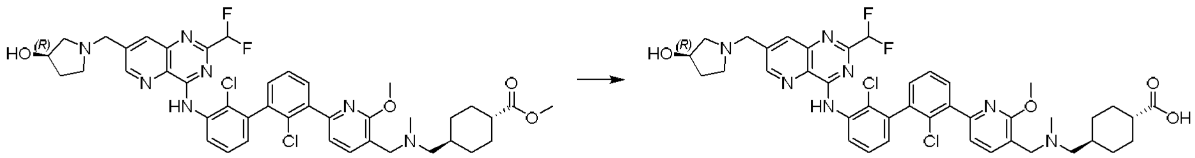

To a 100 mL single-necked bottle, methyl (1*r*,4*r*)-4-((((6-(2,2'-dichloro-3'-((2-(difluoromethyl)-7-(((*R*)-3-hydroxypyrrolidin-1-yl)methyl)pyrido[3,2-d]pyrimidin-4-yl)amino)-[1,1'-biphenyl]-3-yl)-2-methoxypyridin-3-yl)methyl)(methyl) amino)methyl)cyclohexane-1-carboxylate (500 mg, 0.61 mmol), 1,4-dioxane (21 mL), water (7 mL) and lithium hydroxide monohydrate (33.27 mg, 0.79 mmol) were added in sequence and reacted at 50 °C. After the reaction was completed, the solvent was removed by concentration under reduced pressure, and the residue was washed with DCM (10 mL) and MeOH (10 mL). The residue was separated and purified by silica gel column chromatography (eluent: DCM/MeOH (v/v) = 3/1) to obtain 255.0 mg of a light yellow solid product with a yield of 51.89%.

MS (ESI, pos. ion) m/z: 806.28 $[M+H]^+$.
$^1$H NMR (400 MHz, DMSO-$d_6$) δ 10.23 (s, 1H), 8.98 (s, 1H), 8.60 (d, *J* = 8.1 Hz, 1H), 8.23 (s, 1H), 7.76 (d, *J* = 7.5 Hz, 1H), 7.68 (d, *J* = 7.3 Hz, 1H), 7.60 - 7.52 (m, 2H), 7.42 (d, *J* = 6.9 Hz, 1H), 7.31 - 7.22 (m, 2H), 6.86 (t, *J* = 54.4 Hz, 1H), 4.79 (s, 1H), 4.22 (s, 1H), 3.94 - 3.80 (m, 6H), 3.43 (s, 2H), 2.77 - 2.63 (m, 2H), 2.48 - 2.38 (m, 2H), 2.16 (d, *J* = 6.9 Hz, 2H), 2.13 (s, 3H), 2.10 - 1.96 (m, 2H), 1.89 - 1.81 (m, 4H), 1.64 - 1.42 (m, 2H), 1.35 - 1.17 (m, 4H).

**Example 40 (*R*)-1-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)piperidine-4-carboxylic acid**

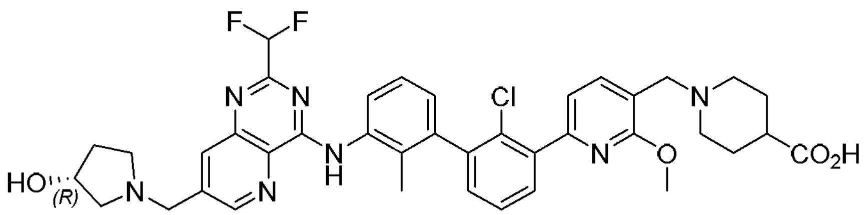

**Step 1: Synthesis of 6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxynicotinaldehyde**

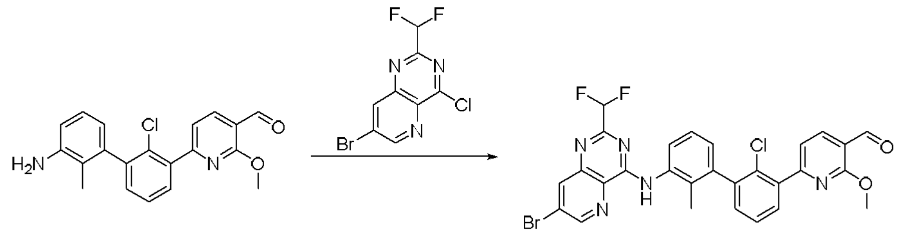

6-(3'-Amino-2-chloro-2'-methyl-[1,1'-biphenyl]-3-yl)-2-methoxynicotinaldehyde (3.83 g, 10.86 mmol) and 7-bromo-4-chloro-2-(difluoromethyl)pyrido[3,2-d]pyrimidine (3.84 g, 13.03 mmol) were dissolved in tert-butyl alcohol (120 mL), and the system was stirred at 100 °C for 5 h under $N_2$. The system was concentrated under reduced pressure, and the residue was separated and purified by column chromatography (EA/PE (v/v) = 2/1) to obtain 5.08 g of a yellow solid with a yield of 76.61%.
MS (ESI, pos. ion) m/z: 610.03 $[M+H]^+$.

**Step 2: Synthesis of methyl 1-((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)piperidine-4-carboxylate**

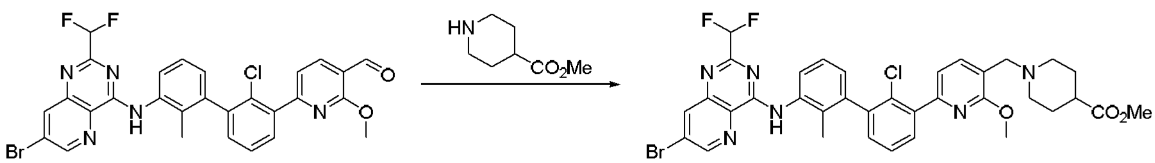

6-(3'-((7-Bromo-2-(difluoromethyl)pyrido[3,2-d]pyrimidin-4-yl)amino)-2-chloro-2'-methyl-[1,1'-biphenyl]-3-yl)-2-methoxynicotinaldehyde (5.08 g, 8.32 mmol) and methyl piperidine-4-carboxylate15 (5.96 g, 41.6 mmol) were dissolved in 2,2,2-trifluoroethanol (70 mL) and DCM (70 mL), and TEA(6 ml) was added. The mixture was stirred at 50 °C for 0.5 h. Sodium cyanoborohydride (784.24 mg, 12.48 mmol) was added and the reaction was continued at room temperature for 1 h. The reaction was quenched by adding water, extracted with dichloromethane (20 ml × 3), and the organic phases were combined, dried over anhydrous sodium sulfate, filtered and concentrated. The residue was separated and purified by silica gel column chromatography (DCM/MeOH (v/v) = 30/1) to obtain 2.017 g of a yellow solid with a yield of 32.86%.
MS (ESI, pos. ion) m/z: 737.1 $[M+H]^+$.

**Step 7: Synthesis of methyl 1-((6-(2-chloro-3'-((2-(difluoromethyl)-7-vinylpyrido[3,2-*d*]pyrimidin-4-yl)amino)-2'-methyl-[1,1'-biphenyl]-3-yl)-2-methoxypyridin-3-yl)methyl)piperidine-4-carboxylate**

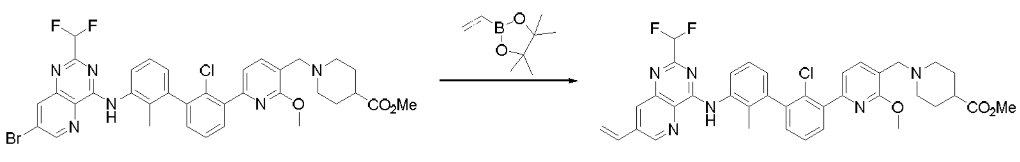

Methyl 1-((6-(3'-((7-bromo-2-(difluoromethyl)pyrido[3,2-*d*]pyrimidin-4-yl)amino)-2-chloro-2'-methyl-[1,1'-biphenyl]-3-yl)-2-methoxypyridin-3-yl)methyl)piperidine-4-carboxylate (2.017 g, 2.73 mmol) was dissolved in a mixed solvent of 1,4-dioxane (50 mL) and water (10 mL), 4,4,5,5-tetramethyl-2-vinyl-1,3,2-dioxaborolane (4.20 g, 27.3 mmol), [1,1'-Bis(diphenylphosphino)ferrocene]palladium dichloride dichloromethane adduct (222.94 mg, 0.27 mmol) and potassium phosphate (1.16 g, 5.46 mmol) were added in sequence, and the system was stirred at 100 °C for 4 h under $N_2$. The reaction was stopped. The mixture was filtered and concentrated, and the residue was separated and purified by column chromatography (DCM/MeOH (v/v) = 30/1) to obtain 1.428 g of a brown-yellow viscous substance with a yield of 76.26%.
MS (ESI, pos. ion) m/z: 685.2 $[M+H]^+$.

**Step 8: Synthesis of methyl 1-((6-(2-chloro-3'-((2-(difluoromethyl)-7-formylpyrido[3,2-d]pyrimidin-4-yl)amino)-2-chloro-2'-methyl-[1,1'-biphenyl]-3-yl)-2-methoxypyridin-3-yl)methyl)piperidine-4-carboxylate**

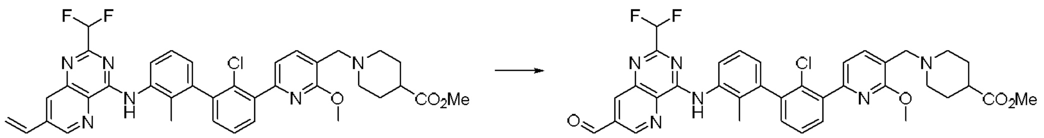

Methyl 1-((6-(2-chloro-3'-((2-(difluoromethyl)-7-vinylpyrido[3,2-*d*]pyrimidin-4-yl)amino)-2-chloro-2'-methyl-[1,1'-biphenyl]-3-yl)-2-methoxypyridin-3-yl)methyl)piperidine-4-carboxylate (1.428 g, 2.08 mmol) was dissolved in 1,4-dioxane (45 mL) and water (15 mL), potassium osmate dihydrate (38.32 mg, 0.10 mmol) and sodium periodate (1.11 g, 5.2 mmol) were added in sequence, and the system was stirred at room temperature for 20 h. The mixture was filtered and separated. The aqueous phase was extracted with EA (20 mL×3). The organic phases were combined and dried over anhydrous sodium sulfate. The filtrate was concentrated under reduced pressure to obtain 0.541 g of a yellow solid with a yield of 37.78%.
MS (ESI, pos. ion) m/z: 687.2 $[M+H]^+$.

**Step 9: Synthesis of methyl (*R*)-1-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)piperidine-4-carboxylate**

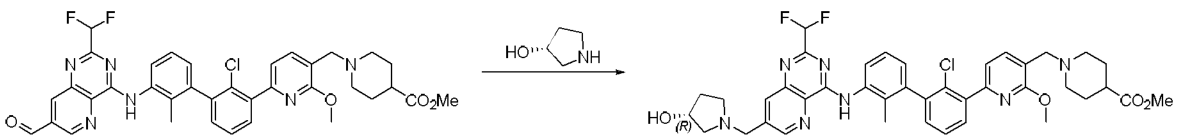

Methyl 1-((6-(2-chloro-3'-((2-(difluoromethyl)-7-formylpyrido[3,2-*d*]pyrimidin-4-yl)amino)-2'-methyl-[1,1'-biphenyl]-3-yl)-2-methoxypyridin-3-yl)methyl)piperidine-4-carboxylate (0.541 g, 0.79 mmol) and (*R*)-pyrrolidin-3-ol (0.34 g, 3.95 mmol) were dissolved in 2,2,2-trifluoroethanol (20 mL) and DCM (20 mL), and TEA (1.5 ml) was added. The mixture was stirred at 50 °C for 2 h. Sodium cyanoborohydride (74.47 mg, 1.19 mmol) was added and the reaction was continued at room temperature for 0.5 h. After the reaction was complete, the system was concentrated and the residue was separated and purified by silica gel column chromatography (DCM/MeOH (v/v) = 20/1) to obtain 0.444 g of yellow viscous product with a yield of 74.37%.
MS (ESI, pos. ion) m/z: 758.3 $[M+H]^+$.

**Step 10: Synthesis of (*R*)-1-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)piperidine-4-carboxylic acid**

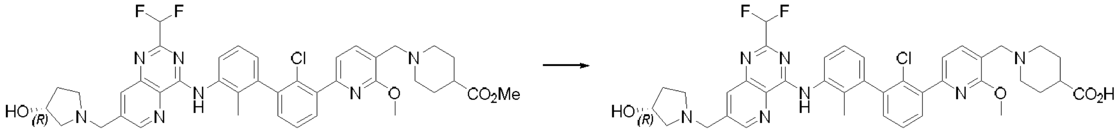

Methyl (*R*)-1-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-2-methoxypyridin-3-yl)methyl)piperidine-4-carboxylate (0.444 g, 0.59 mmol) was dissolved in a mixed solvent of 1,4-dioxane (40 mL) and water (8 mL), and a solution of lithium hydroxide monohydrate (297.08 mg, 7.08 mmol) was added, and the system was stirred at room temperature for 8 h. The stirring was stopped, water (5 ml) was added for dilution, acetic acid (5 ml) was used to adjust the pH to 5, and the filtrate was concentrated. The residue was separated and purified by silica gel column chromatography (dichloromethane/methanol (v/v) = 2/1) to obtain 71 mg of light orange powder with a yield of 16.29%.

MS (ESI, pos. ion) m/z: 744.2 [M+H]$^+$.
$^1$H NMR (400 MHz, DMSO-$d_6$) δ 10.51 (s, 1H), 9.09 (s, 1H), 8.46 (s, 1H), 7.97 (d, *J* = 7.5 Hz, 1H), 7.67 (s, 1H), 7.65 (s, 1H), 7.57 (d, *J* = 7.8 Hz, 1H), 7.41 (s, 1H), 7.39 (s, 2H), 7.17 (d, *J* = 7.6 Hz, 1H), 6.70 (t, *J* = 54.5 Hz, 1H), 5.52 (s, 1H), 4.58 (s, 4H), 4.43 (s, 3H), 3.16 - 3.08 (m, 7H), 3.03 (dd, *J* = 3.2, 1.8 Hz, 2H), 2.27 - 2.10 (m, 4H), 1.91 - 1.78 (m, 6H).

**Example 41 (1*s*,4*s*)-4-(((5-(2-chloro-3-(3-((2-(difluoromethyl)-7-(((*R*)-2-methylpyrrolidin-1-yl)methyl)pyrido[3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylic acid**

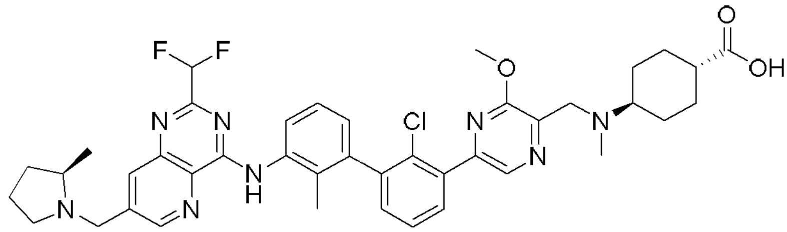

**Step 1: Synthesis of methyl (1s,4s)-4-(((5-(2-chloro-3-(3-((2-(difluoromethyl)-7-(((*R*)-2-methylpyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

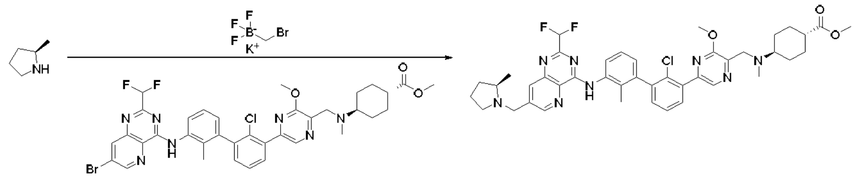

(*R*)-2-Methylpyrrolidine (0.47 g, 3.90 mmol) was dissolved in acetonitrile (10 ml), lithium hydroxide monohydrate (0.18 g, 4.29 mmol) was added, and the mixture was heated at 80 °C for 0.5 h. Potassium (bromomethyl)trifluoroborate (0.78 g, 3.90 mmol), sodium carbonate (0.34 g, 3.25 mmol) and acetonitrile (10 ml) were added, and the mixture was heated at 80 °C for 4.5 h. The system was concentrated, and then methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclohexane-1-carboxylate (1.00 g, 1.30 mmol), palladium acetate (0.044 g, 0.20 mmol), X-Phos (0.19 g, 0.39 mmol), potassium carbonate (0.54 g, 3.90 mmol), 1,4-dioxane (25 ml) and water (5 ml) were added, and the reaction was heated to 110 °C overnight under $N_2$. The reaction solution was concentrated under reduced pressureto remove solution, and the residue was separated and purified by column chromatography (eluent: DCM/MeOH (v/v) = 20/1) to obtain 0.46 g of a yellow solid product with a yield of 44.93%.
MS (ESI, pos. ion) m/z: 785.35 [M+H]$^+$.

**Step 2: Synthesis of (1*s*,4*s*)-4-(((5-(2-chloro-3-(3-((2-(difluoromethyl)-7-(((*R*)-2-methylpyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

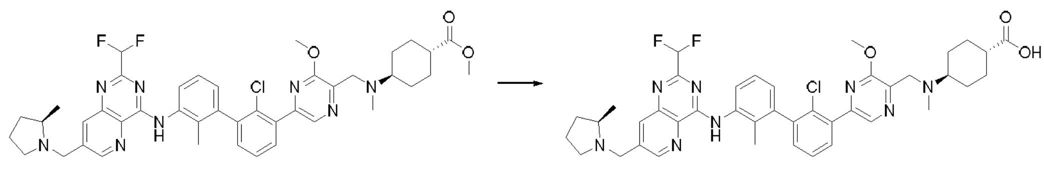

Methyl (1*s*,4*s*)-4-(((5-(2-chloro-3-(3-((2-(difluoromethyl)-7-(((*R*)-2-methylpyrrolidin-1-yl)methyl)pyrido[3,2-*d*]

pyrimidin-4-yl)amino)-2'-methyl-[1,1'-biphenyl]-3-yl)-3-methoxypyrazin-2-yl)methyl)(methyl)amino)cyclohexane-1-carboxylate (0.46 g, 0.59 mmol) was dissolved in a mixed solution of 1,4-dioxane (20 mL) and water (4 mL), lithium hydroxide monohydrate (0.12 g, 2.95 mmol) was added, and the reaction was stirred at room temperature overnight. After the reaction of the raw materials was complete, the pH of the system was adjusted to about 5 with acetic acid, the reaction solution was concentrated under reduced pressure to remove solution, and the residue was separated and purified by column chromatography (eluent: DCM/MeOH (v/v) = 7/1) to obtain 0.43 g of a yellow solid product with a yield of 95.18%.

MS (ESI, pos. ion) m/z: 771.33 $[M+H]^+$.
$^1$H NMR (400 MHz, DMSO-$d_6$) δ 10.31 (s, 1H), 8.92 (s, 1H), 8.40 (s, 1H), 8.14 (s, 1H), 7.75 (d, *J* = 8.0 Hz, 1H), 7.69 (d, *J* = 7.7 Hz, 1H), 7.57 (t, *J* = 7.6 Hz, 1H), 7.45 - 7.36 (m, 2H), 7.15 (d, *J* = 7.6 Hz, 1H), 6.69 (t, *J* = 54.5 Hz, 1H), 3.94 (s, 3H), 3.50 (s, 2H), 3.46 (s, 2H), 2.83 - 2.79 (m, 1H), 2.48 - 2.45 (m, 1H), 2.20 (s, 3H), 2.03 (s, 3H), 1.96 - 1.93 (m, 2H), 1.91 - 1.88 (m, 5H), 1.86 - 1.83 (m, 1H), 1.66 - 1.60 (m, 2H), 1.37 - 1.29 (m, 5H), 1.13 (d, *J* = 5.9 Hz, 3H).
$^{13}$C NMR (151 MHz, DMSO-$d_6$) δ 177.11, 158.94, 157.77, 157.06, 151.64, 147.37, 144.21, 143.40, 141.77, 141.28, 140.32, 136.64, 136.35, 135.42, 134.78, 131.82, 131.12, 130.08, 127.53, 127.35, 126.09, 125.64, 112.77, 62.23, 59.34, 54.51, 53.83, 53.58, 42.64, 37.97, 32.58, 28.47, 27.26, 21.44, 19.27, 15.50.

**Example 42 (1*r*,4*r*)-4-(((5-(2-chloro-3'-((7-(((*R*)-3-(difluoromethoxy)pyrrolidin-1-yl)methyl)-2-(difluoromethyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

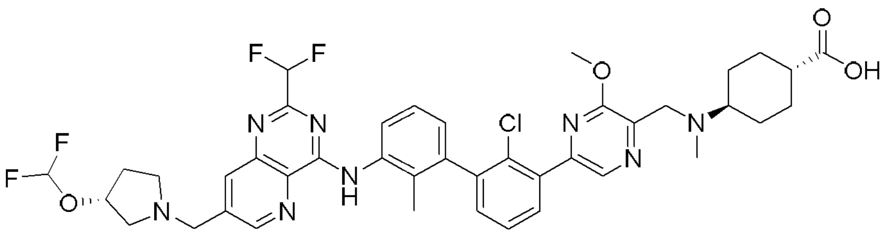

**Step 1: Synthesis of *tert*-butyl (*R*)-3-hydroxypyrrolidine-1-carboxylate**

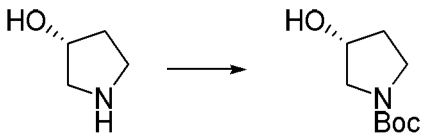

(*R*)-Pyrrolidin-3-ol (1.00 g, 11.48 mmol), dichloromethane (20.0 mL) and triethylamine (2.32 g, 22.96 mmol) were added to the reaction flask in sequence. Di-*tert*-butyl dicarbonate (3.01 g, 13.78 mmol) was then added dropwise to the mixture, and bubbles were generated in the system. After the addition was completed, the mixture was stirred at room temperature for 2 h. Water (30 mL) and ethyl acetate (30 mL) were added to the mixture in sequence. After the mixture was separated, the aqueous phase was extracted with ethyl acetate (30 mL). The organic phases were combined, washed with saturated brine (60 mL), dried over anhydrous sodium sulfate, filtered, and concentrated. The residue was separated and purified by silica gel column chromatography (PE:EA (v/v) = 2:1) to obtain the target product as a colorless transparent oil (2.00 g, yield 93.06%).
$^1$H NMR (400 MHz, $CDCl_3$) δ 4.48 - 4.40 (m, 1H), 3.52 - 3.41 (m, 3H), 3.40 - 3.27 (m, 1H), 1.94 - 1.77 (m, 2H), 1.46 (s, 9H).

**Step 2: Synthesis of *tert*-butyl (*R*)-3-(difluoromethoxy)pyrrolidine-1-carboxylate**

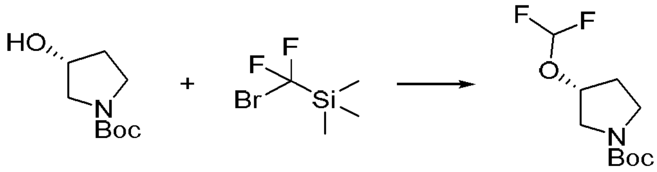

To the reaction flask, *tert*-butyl (*R*)-3-hydroxypyrrolidine-1-carboxylate (2.00 g, 10.68 mmol), dichloromethane (20.0 mL), water (20.0 mL), potassium acetate (6.29 g, 64.08 mmol) and (bromodifluoromethyl)trimethylsilane (8.68 g, 42.72 mmol) were added in sequence. The mixture was stirred at room temperature overnight. (bromodifluoromethyl) trimethylsilane (2.00 g, 9.84 mmol) was added to the above mixture, and the mixture was stirred at room temperature overnight. Water (30 mL) and dichloromethane (30 mL) were added to the above mixture in sequence. The aqueous phase after separation of the obtained mixture was extracted with dichloromethane (30 mL), the organic phases were combined, and saturated brine (60 mL) was added. The residue was purified by silica gel column chromatography (PE:EA (v/v) = 10:1) to obtain the target product as a colorless transparent liquid (1.30 g, yield 51.30%).

$^1$H NMR (400 MHz, $CDCl_3$) $\delta$ 6.23 (t, *J* = 73.9 Hz, 1H), 4.84 - 4.76 (m, 1H), 3.60 - 3.38 (m, 4H), 2.14 - 1.99 (m, 2H), 1.46 (s, 9H).
$^{19}$F NMR (376 MHz, $CDCl_3$) $\delta$ -82.49 (d, *J* = 14.7 Hz).

**Step 3: Synthesis of (*R*)-3-(difluoromethoxy)pyrrolidine**

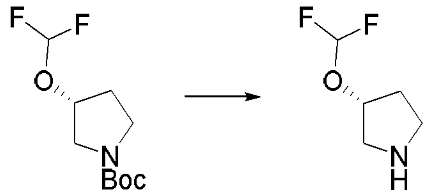

To the reaction flask, *tert*-butyl (*R*)-3-(difluoromethoxy)pyrrolidine-1-carboxylate (1.30 g, 5.48 mmol), ethyl acetate (10.0 mL), and hydrochloric acid (6.0 mL, 36 mmol, 6.00 mol/L) were added in sequence, and the mixture was stirred at room temperature overnight. The above mixture was concentrated to dryness, and the residue was diluted with water (5 mL) and the pH was adjusted to 8~9 with solid sodium carbonate. After the residue was concentrated, the residue was separated and purified by silica gel column. (DCM:MeOH (v/v) = 9:1) to obtain the target product as a light yellow oil (300 mg, yield 39.92%).

$^1$H NMR (400 MHz, $CDCl_3$) $\delta$ 6.25 (t, *J* = 73.0 Hz, 1H), 4.99 - 4.91 (m, 1H), 3.46 - 3.26 (m, 4H), 2.22 - 2.13 (m, 2H).
$^{19}$F NMR (376 MHz, $CDCl_3$) $\delta$ -83.09, -83.10.

**Step 4: Synthesis of methyl (1*r*,4*r*)-4-(((5-(2-chloro-3'-((7-(((*R*)-3-(difluoromethoxy)pyrrolidin-1-yl)methyl)-2-(difluoromethyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

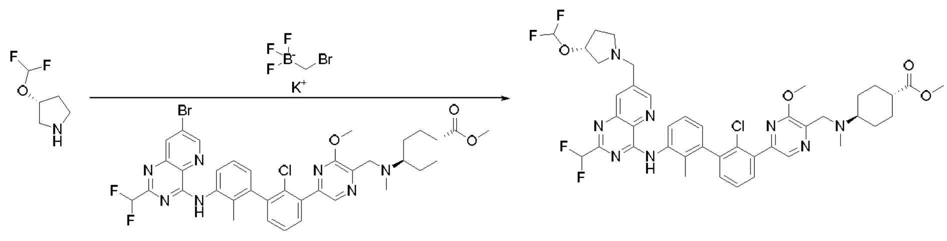

To the reaction flask, (R)-3-(difluoromethoxy)pyrrolidine (0.30 g, 3.19 mmol), sodium carbonate (0.23 g, 2.18 mmol), acetonitrile (10.0 mL), and potassium(bromomethyl)trifluoroborate (0.44 g, 2.18 mmol) were added in sequence. After nitrogen substitution, the mixture was heated to 80 °C and stirred for 5 h. After the solvent was dried, potassium

carbonate (0.43 g, 3.12 mmol), palladium acetate (23.0 mg, 0.10 mmol), 2-(dicyclohexylphosphino)-2,4,6-triisopropylbiphenyl (99.0 mg, 0.21 mmol), methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino) cyclohexane-1-carboxylate (0.80 g, 1.04 mmol), 1,4-dioxane (15.0 mL) and water (5.0 mL), after nitrogen replacement, the mixture was heated to reflux and stirred overnight. The reaction system was concentrated and purified by silica gel column (DCM: MeOH (v/v) = 10:1) to obtain the target product as a light brown solid (250 mg, yield 28.63%).

MS (ESI, pos. ion) m/z: 837.3 $[M+H]^+$.
$^1$H NMR (400 MHz, $CDCl_3$) δ 9.38 (s, 1H), 8.87 (d, *J* = 1.7 Hz, 1H), 8.55 (d, *J* = 8.1 Hz, 1H), 8.49 (s, 1H), 8.19 (s, 1H), 7.62 (dd, *J* = 7.6, 1.6 Hz, 1H), 7.48 - 7.39 (m, 2H), 7.38 - 7.33 (m, 1H), 7.10 (d, *J* = 7.4 Hz, 1H), 6.60 (t, *J* = 55.0 Hz, 2H), 6.20 (t, *J* = 74.1 Hz, 2H), 4.83 - 4.75 (m, 1H), 4.05 (s, 3H), 3.88 (s, 2H), 3.79 (s, 2H), 3.67 (s, 3H), 2.94 - 2.87 (m, 1H), 2.83 - 2.71 (m, 2H), 2.63 - 2.53 (m, 2H), 2.40 (s, 3H), 2.31 - 2.20 (m, 5H), 2.13 - 2.03 (m, 4H), 2.03 - 1.94 (m, 1H), 1.56 - 1.38 (m, 4H).

**Step 5: Synthesis of (1*r*,4*r*)-4-(((5-(2-chloro-3'-((7-(((*R*)-3-(difluoromethoxy)pyrrolidin-1-yl)methyl)-2-(difluoromethyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

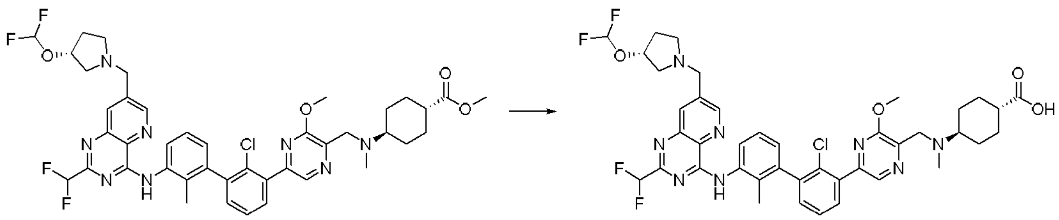

To the reaction bottle, methyl (1*r*,4*r*)-4-(((5-(2-chloro-3'-((7-(((*R*)-3-(difluoromethoxy)pyrrolidin-1-yl)methyl)-2-(difluoromethyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (250 mg, 0.30 mmol), methanol (2.0 mL), tetrahydrofuran (1.0 mL), water (1.0 mL) and lithium hydroxide monohydrate (50.0 mg, 1.20 mmol) were added in sequence, and the mixture was heated to 50 °C and stirred for 1 h. The system was concentrated under reduced pressure, glacial acetic acid (0.5 mL) was added, and the mixture was diluted with water (2 mL) and toluene (20 mL) in sequence and concentrated under reduced pressure. The residue was separated and purified by silica gel column chromatography (DCM:MeOH (v/v) = 10:1) to obtain the target product as a yellow solid (170 mg, yield 69.16%).

MS (ESI, pos. ion) m/z: 823.2 $[M+H]^+$.
$^1$H NMR (400 MHz, DMSO-$d_6$) δ 12.00 (s, 1H), 10.38 (s, 1H), 8.95 (d, *J* = 1.6 Hz, 1H), 8.46 (s, 1H), 8.17 (s, 1H), 7.73 (dd, *J* = 11.9, 7.8 Hz, 2H), 7.59 (t, *J* = 7.6 Hz, 1H), 7.45 (d, *J* = 6.6 Hz, 1H), 7.39 (t, *J* = 7.8 Hz, 1H), 7.17 (d, *J* = 7.2 Hz, 1H), 6.87 - 6.45 (m, 2H), 4.73 (s, 1H), 3.98 (s, 3H), 3.90 (q, *J* = 14.1 Hz, 4H), 2.84 - 2.73 (m, 2H), 2.72 - 2.64 (m, 1H), 2.33 - 2.12 (m, 4H), 2.04 (s, 3H), 2.01 - 1.94 (m, 3H), 1.93 - 1.88 (m, 4H), 1.86 - 1.78 (m, 1H), 1.45 - 1.28 (m, 4H).
$^{19}$F NMR (376 MHz, DMSO-$d_6$) δ -80.49, -118.55, -118.56.

**Example 43 (1*r*,4*r*)-4-(((5-(2-chloro-3'-((2-(difluoromethyl)-7-((3-(difluoromethyl)azetidin-1-yl)methyl)pyrido[3,2-*d*]pyrimidin-4-yl)amino)-2'-methyl-[1,1'-biphenyl]-3-yl)-3-methoxy pyrazin-2-yl)methyl)(methyl)amino)cyclohexane-1-carboxylic acid**

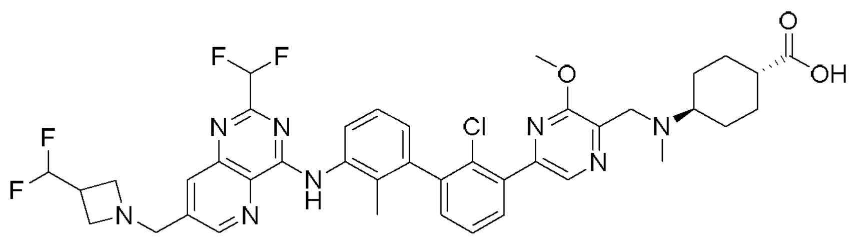

**Step 1: Synthesis of *tert*-butyl 3-formylazetidine-1-carboxylate**

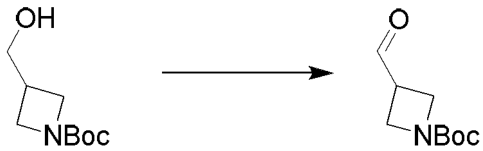

To a 100 mL single-necked bottle, *tert*-butyl 3-(hydroxymethyl)azetidine-1-carboxylate (3 g, 16.02 mmol), DCM (30 mL) and Dess-Martin periodinane (10.19 g, 24.03 mmol) were added in sequence and reacted at room temperature. After the reaction was completed, the reaction solution was filtered through celite, sodium sulfite aqueous solution (150 mL) was added, and then extracted with DCM (20 mL × 3), the organic phases were combined, washed with saturated brine (50 mL × 1), dried over anhydrous sodium sulfate, filtered, and the filtrate was concentrated under reduced pressure to remove the solvent. The residue was separated and purified by silica gel column chromatography (eluent: PE/EA (v/v) = 3/1) to obtain 2.85 g of colorless oily product with a yield of 96.04%.
MS (ESI, pos. ion) m/z: 130.05 $[M-56+H]^+$.

**Step 2: Synthesis of *tert*-butyl 3-(difluoromethyl)azetidine-1-carboxylate**

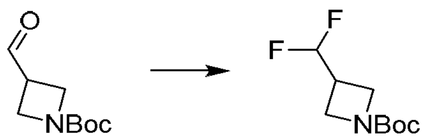

*Tert*-butyl 3-formylazetidine-1-carboxylate (2.85 g, 15.39 mmol) and DCM (50 mL) were added to a 100 mL single-mouth bottle in sequence, and diethylaminosulfur trifluoride (6.20 g, 38.48 mmol) was slowly added dropwise at 0 °C and reacted at room temperature. After the reaction was completed, 1N $K_2CO_3$ aqueous solution was slowly added dropwise to quench the reaction, water (100 mL) was added, and then extracted with DCM (20 mL × 3), the organic phases were combined, washed with water (50 mL × 1), dried over anhydrous sodium sulfate, filtered, and the filtrate was concentrated under reduced pressure to remove the solvent. The residue was separated and purified by silica gel column chromatography (eluent: PE/EA (v/v) = 20/1) to obtain 0.65 g of colorless oily product with a yield of 20.39%.
MS (ESI, pos. ion) m/z: 152.05 $[M-56+H]^+$.

**Step 3: Synthesis of 3-(difluoromethyl)azetidine**

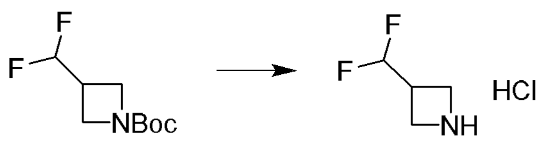

To a 50 mL single-necked bottle, *tert-butyl* 3-(difluoromethyl)azetidine-1-carboxylate (548 mg, 2.64 mmol), DCM (10 mL) and hydrochloric acid (962.54 mg, 26.40 mmol) were added in sequence and reacted at room temperature. After the reaction was complete, the mixture was concentrated under reduced pressure to obtain 365.0 mg of a brown solid product with a yield of 96.14%.

MS (ESI, pos. ion) m/z: 107.05 $[M-56+H]^+$.

**Step 4: Synthesis of methyl (1*r*,4*r*)-4-(((5-(2-chloro-3'-((2-(difluoromethyl)-7-((3-(difluoromethyl)azetidin-1-yl) methyl)pyrido[3,2-*d*]pyrimidin-4-yl)amino)-2'-methyl-[1,1'-biphenyl]-3-yl)-3-methoxypyrazin-2-yl)methyl) (methyl)amino)cyclohexane-1-carboxylate**

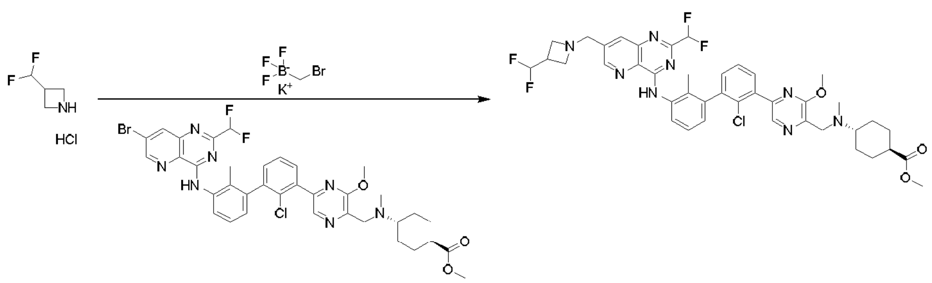


To a 50 mL single-necked bottle, 3-(difluoromethyl)azetidine (0.21 g, 1.95 mmol), potassium(bromomethyl) trifluoroborate (0.39 g, 1.95 mmol), sodium carbonate (0.31 g, 2.93 mmol) and acetonitrile (15 mL) were added in sequence and reacted at 80 °C for 5 h. The mixture was concentrated under reduced pressure and methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclohexane-1-carboxylate (0.5 g, 0.65 mmol), palladium acetate (0.036 g, 0.16 mmol), X-Phos (0.15 g, 0.33 mmol), potassium carbonate (0.27 g, 1.95 mmol), 1,4-dioxane (15 mL) and water (3 mL) were added and reacted at 110 °C under $N_2$. After the reaction was complete, the reaction solution was concentrated under reduced pressure, water (100 mL) was added, and then extracted with DCM (20 mL × 3). The organic phases were combined, washed with saturated brine (50 mL × 1), dried over anhydrous sodium sulfate, filtered, and the filtrate was concentrated under reduced pressure. The residue was separated and purified by silica gel column chromatography (eluent: DCM/MeOH (v/v) = 20/1) to obtain 210.0 mg of a yellow solid product with a yield of 39.91%. MS (ESI, pos. ion) m/z: 807.32 $[M+H]^+$.

**Step 5: Synthesis of (1*r*,4*r*)-4-(((5-(2-chloro-3'-((2-(difluoromethyl)-7-((3-(difluoromethyl)azetidin-1-yl)methyl) pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

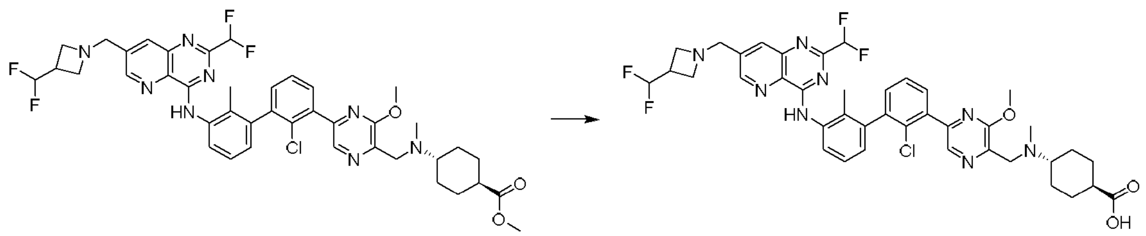

To a 50 mL single-necked bottle, methyl (1*r*,4*r*)-4-(((5-(2-chloro-3'-((2-(difluoromethyl)-7-((3-(difluoromethyl) azetidin-1-yl)methyl)pyrido[3,2-*d*]pyrimidin-4-yl)amino)-2'-methyl-[1,1'-biphenyl]-3-yl)-3-methoxypyrazin-2-yl)methyl) (methyl)amino)cyclohexane-1-carboxylate (210 mg, 0.26 mmol), 1,4-dioxane (12 mL), water (4 mL) and lithium hydroxide monohydrate (21.82 mg, 0.52 mmol) were added in sequence and reacted at 50 °C. After the reaction was completed, the solvent was removed by concentration under reduced pressure, and the mixture was dissolved with DCM (8 mL) and MeOH (8 mL). The pH was adjusted to 6-7 with a hydrogen chloride-dioxane solution (4.0 M), and the solvent was removed by concentration under reduced pressure. The residue was separated and purified by silica gel column chromatography (eluent: DCM /MeOH (v/v) = 5/1) to obtain a yellow solid crude product (141.0 mg, yield 68.33%). The obtained crude product was further purified by preparative liquid chromatography to obtain 24.0 mg of a pure product.

MS (ESI, pos. ion) m/z: 793.30 $[M+H]^+$.
$^1$H NMR (400 MHz, DMSO-$d_6$) δ 10.34 (s, 1H), 8.89 (d, *J* = 1.8 Hz, 1H), 8.39 (s, 1H), 8.12 (d, *J* = 1.7 Hz, 1H), 7.77 - 7.65 (m, 2H), 7.57 (t, *J* = 7.6 Hz, 1H), 7.42 (dd, *J* = 7.5, 1.6 Hz, 1H), 7.38 (t, *J* = 7.8 Hz, 1H), 7.16 (d, *J* = 7.5 Hz, 1H), 6.68 (t, *J* = 54.5 Hz, 1H), 6.26 (td, *J* = 56.7, 4.9 Hz, 1H), 3.94 (s, 3H), 3.86 (s, 2H), 3.69 (s, 2H), 3.67 - 3.65 (m, 1H), 3.38 - 3.36 (m, 2H), 3.24 - 3.15 (m, 2H), 2.19 (s, 3H), 2.02 (s, 3H), 1.96 - 1.78 (m, 4H), 1.35 - 1.21 (m, 7H).

**Example 44 (1*r*,4*r*)-4-(((5-(2-chloro-3'-((2-(difluoromethyl)-7-(((*S*)-3-((*R*)-1-hydroxyethyl)pyrrolidin-1-yl)methyl) pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

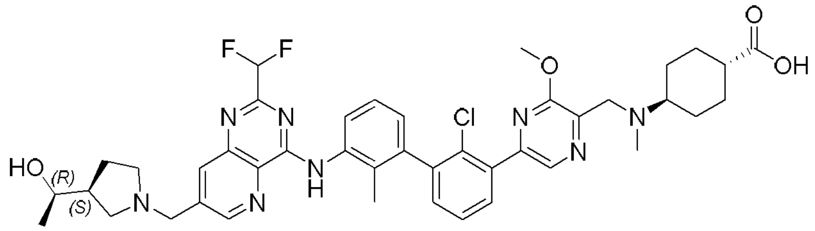

**Step 1: Synthesis of (*R*)-1-((*S*)-pyrrolidin-3-yl)ethan-1-ol**

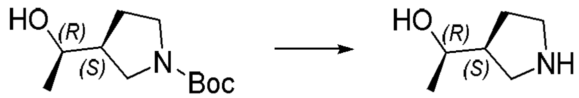

*Tert*-butyl (*S*)-3-((*R*)-1-hydroxyethyl)pyrrolidine-1-carboxylate (1.15 g, 5.34 mmol) was dissolved in DCM (30 ml), 4N HCl dioxane solution (3.50 g, 96.12 mmol) was added and reacted at room temperature for 4.5 h. After the reaction of the raw materials was complete, the reaction solution was concentrated under reduced pressure to remove the solvent, DCM (10 ml) and methanol (4 ml) were added to dilute the system, potassium carbonate was added to adjust the pH to about 8, and the solution was filtered. The filtrate was concentrated under reduced pressure to obtain 0.61 g of a yellow liquid product with a yield of 99.16%.
MS (ESI, pos. ion) m/z: 116.11 $[M+H]^+$.

**Step 2: Synthesis of methyl (1*r*,4*r*)-4-(((5-(2-chloro-3'-((2-(difluoromethyl)-7-(((*S*)-3-((*R*)-1-hydroxyethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

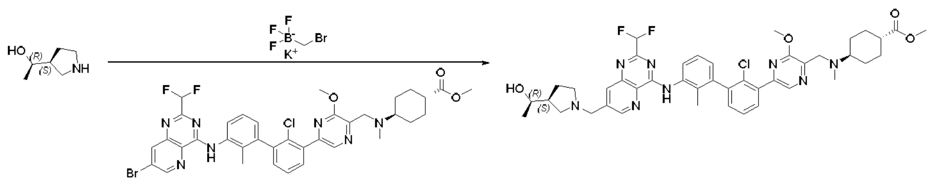

(*R*)-1-((*S*)-Pyrrolidin-3-yl)ethan-1-ol (0.61 g, 5.33 mmol) was dissloved in acetonitrile (10 mL), potassium(bromomethyl)trifluoroborate (1.07 g, 5.33 mmol), and sodium carbonate (0.62 g, 5.85 mmol), heated at 80 °C for 5 h, and the system was concentrated. Then, methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclohexane-1-carboxylate (1.00 g, 1.30 mmol), palladium acetate (0.044 g, 0.20 mmol), X-Phos (0.19 g, 0.39 mmol), potassium carbonate (0.54 g, 3.90 mmol), 1,4-dioxane (25 mL) and water (5 mL) were added, the mixture was heated to 110 °C overnight under $N_2$. After the reaction was completed, the reaction solution was concentrated under reduced pressure to remove the solvent, and the residue was separated and purified by silica gel column chromatography (eluent: DCM/MeOH (v/v) = 20/1) to obtain 0.58 g of a yellow solid product with a yield of 54.56%.
MS (ESI, pos. ion) m/z: 815.36 $[M+H]^+$.

**Step 3: Synthesis of (1*r*,4*r*)-4-(((5-(2-chloro-3'-((2-(difluoromethyl)-7-(((*S*)-3-((*R*)-1-hydroxyethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

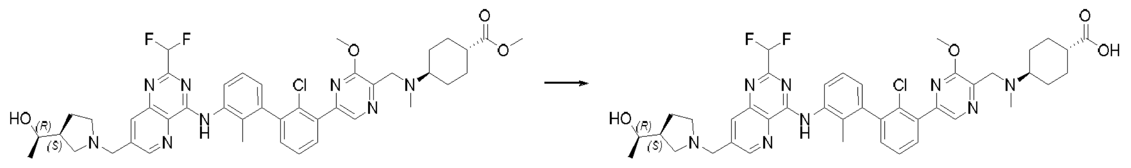

Methyl (1*r*,4*r*)-4-(((5-(2-chloro-3'-((2-(difluoromethyl)-7-(((*S*)-3-((*R*)-1-hydroxyethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (0.58 g, 0.71 mmol) was dissolved in a mixed solution of 1,4-dioxane (15 mL) and water (3 mL), lithium hydroxide monohydrate (0.12 g, 2.84 mmol) was added, and the reaction was stirred at room temperature overnight. After the reaction of the raw materials was complete, the pH of the system was adjusted to about 5 with acetic acid, the reaction solution was concentrated under reduced pressure to remove solution, and the residue was separated and purified by column chromatography (eluent: DCM/MeOH (v/v) = 7/1) to obtain 0.53 g of a yellow solid product with a yield of 92.98%. MS (ESI, pos. ion) m/z: 801.35 $[M+H]^+$.

$^1$H NMR (400 MHz, DMSO-$d_6$) δ 10.33 (s, 1H), 8.94 (d, *J* = 1.9 Hz, 1H), 8.42 (s, 1H), 8.15 (d, *J* = 1.9 Hz, 1H), 7.76 (d, *J* = 7.9 Hz, 1H), 7.70 (dd, *J* = 7.7, 1.7 Hz, 1H), 7.58 (t, *J* = 7.6 Hz, 1H), 7.43 (dd, *J* = 7.6, 1.7 Hz, 1H), 7.38 (t, *J* = 7.8 Hz, 1H), 7.16 (d, *J* = 7.5 Hz, 1H), 6.71 (t, *J* = 54.5 Hz, 1H), 3.96 (s, 3H), 3.88 (d, *J* = 13.8 Hz, 1H), 3.80 (d, *J* = 13.8 Hz, 1H), 3.70 (s, 2H), 3.45 - 3.38 (m, 1H), 3.17 (s, 1H), 2.63 - 2.54 (m, 2H), 2.48 - 2.42 (m, 2H), 2.21 (s, 3H), 2.13 -2.06 (m, 1H), 2.04 (s, 3H), 1.97 - 1.90 (m, 2H), 1.87 - 1.74 (m, 6H), 1.36 - 1.28 (m, 4H), 1.02 (d, *J* = 6.1 Hz, 3H).
$^{13}$C NMR (151 MHz, DMSO-$d_6$) δ 177.62, 159.21, 158.01, 157.36, 151.73, 147.59, 144.56, 143.78, 141.57, 140.57, 136.97, 136.68, 135.73, 135.04, 132.11, 131.43, 130.45, 127.73, 126.26, 125.95, 113.07, 69.71, 62.63, 57.08, 54.32, 54.08, 53.85, 45.90, 43.38, 38.22, 28.92, 27.69, 27.59, 22.50, 15.82.

**Example 45 (1*r*,4*r*)-4-(((5-(2-chloro-3'-((2-(difluoromethyl)-7-(((*S*)-3-((*S*)-1-hydroxyethyl)pyrrolidin-1-yl)methyl) pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

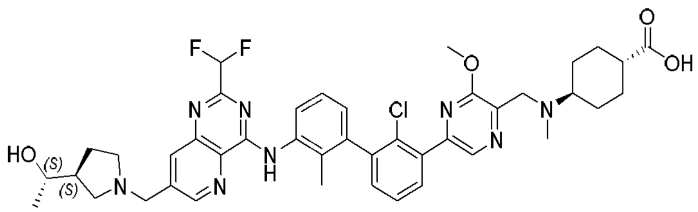

**Step 1: Synthesis of (*S*)-1-((*S*)-pyrrolidin-3-yl)ethan-1-ol**

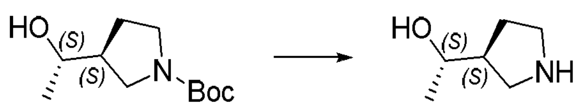

*Tert*-butyl (*S*)-3-((*S*)-1-hydroxyethyl)pyrrolidine-1-carboxylate (0.94 g, 4.37 mmol) was dissolved in DCM (20 ml), 4N HCl dioxane solution (1.59 g, 43.70 mmol) was added and reacted at room temperature for 2 h. After the reaction of the raw materials was complete, the reaction solution was concentrated under reduced pressure to remove the solvent, DCM (10 ml) and methanol (4 ml) were added to dilute the system, potassium carbonate was added to adjust the pH to about 8, and the solution was filtered. The filtrate was concentrated under reduced pressure to obtain 0.50 g of a yellow liquid product with a yield of 99.43%.
MS (ESI, pos. ion) m/z: 116.11 $[M+H]^+$.

**Step 2: Synthesis of methyl (1*r*,4*r*)-4-(((5-(2-chloro-3'-((2-(difluoromethyl)-7-(((*S*)-3-((*S*)-1-hydroxyethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

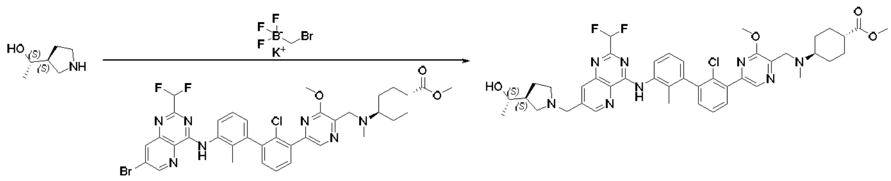

(*S*)-1-((*S*)-Pyrrolidin-3-yl)ethan-1-ol (0.50 g, 4.36 mmol) was dissloved in acetonitrile (10 mL), potassium(bromomethyl)trifluoroborate (0.87 g, 4.36 mmol), and sodium carbonate (0.62 g, 5.85 mmol), heated at 80 °C for 5 h, and the system was concentrated. Then, methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclohexane-1-carboxylate (1.00 g, 1.30 mmol), palladium acetate (0.044 g, 0.20 mmol), X-Phos (0.19 g, 0.39 mmol), potassium carbonate (0.54 g, 3.90 mmol), 1,4-dioxane (25 mL) and water (5 mL) were added, the mixture was heated to 110 °C overnight under $N_2$. The reaction solution was concentrated under reduced pressure, and the residue was separated and purified by column chromatography (eluent: DCM/MeOH (v/v) = 20/1) to obtain 0.39 g of a yellow solid product with a yield of 36.69%.
MS (ESI, pos. ion) m/z: 815.36 $[M+H]^+$.

**Step 3: Synthesis of (1*r*,4*r*)-4-(((5-(2-chloro-3'-((2-(difluoromethyl)-7-(((*S*)-3-((*S*)-1-hydroxyethyl)pyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

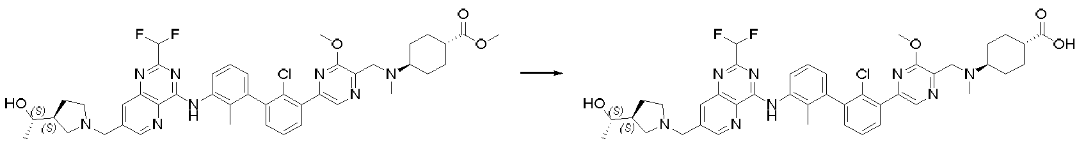

Methyl (1*r*,4*r*)-4-(((5-(2-chloro-3'-((2-(difluoromethyl)-7-(((*S*)-3-((*S*)-1-hydroxyethyl)pyrrolidin-l-yl)methyl)pyrido[3,2-*d*]pyrimidin-4-yl)amino)-2'-methyl-[1,1'-biphenyl] -3-yl)-3-methoxypyrazin-2-yl)methyl)(methyl)amino)cyclohexane-1-carboxylate (0.39 g, 0.48 mmol) was dissolved in a mixed solution of 1,4-dioxane (15 mL) and water (3 mL), lithium hydroxide monohydrate (0.081 g, 1.92 mmol) was added, and the reaction was stirred at room temperature overnight. After the reaction of the raw materials was complete, the pH of the system was adjusted to about 5 with acetic acid, the reaction solution was concentrated under reduced pressure to remove solution, and the residue was separated and purified by column chromatography (eluent: DCM/MeOH (v/v) = 7/1) to obtain 0.313 g of a yellow solid product with a yield of 81.66%.

MS (ESI, pos. ion) m/z: 801.35 $[M+H]^+$.
$^1$H NMR (400 MHz, DMSO-$d_6$) δ 10.35 (s, 1H), 8.95 *(d, J* = 1.9 Hz, 1H), 8.42 (s, 1H), 8.15 (d, *J* = 1.9 Hz, 1H), 7.76 (d, *J* = 7.9 Hz, 1H), 7.70 (dd, *J* = 7.7, 1.7 Hz, 1H), 7.58 (t, *J* = 7.6 Hz, 1H), 7.44 (dd, *J* = 7.5, 1.8 Hz, 1H), 7.39 (t, *J* = 7.8 Hz, 1H), 7.17 (d, *J* = 7.6 Hz, 1H), 6.71 (t, *J* = 54.5 Hz, 1H), 3.96 (s, 3H), 3.87 (d, *J* = 13.9 Hz, 1H), 3.82 (d, *J* = 13.9 Hz, 1H), 3.71 (s, 2H), 3.47 - 3.44 (m, 2H), 2.68 -2.60 (m, 2H), 2.49 - 2.42 (m, 2H), 2.22 (s, 3H), 2.16 - 2.10 (m, 1H), 2.05 (s, 3H), 2.00 - 1.63 (m, 8H), 1.37 - 1.30 (m, 4H), 1.00 (d, *J* = 6.1 Hz, 3H).
$^{13}$C NMR (151 MHz, DMSO-$d_6$) δ 177.15, 159.22, 158.00, 157.36, 151.71, 147.62, 144.55, 143.64, 141.56, 140.57, 136.97, 136.66, 135.74, 135.06, 132.13, 131.42, 130.47, 127.73, 126.27, 125.96, 113.06, 68.99, 62.53, 57.07, 56.72, 54.09, 53.81, 45.58, 42.78, 38.23, 28.70, 27.54, 27.08, 22.44, 15.82.

**Example 46 (1*r*,4*r*)-4-(((5-(2-chloro-3'-((2-(difluoromethyl)-7-(((*S*)-3-hydroxy-3-methylpyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

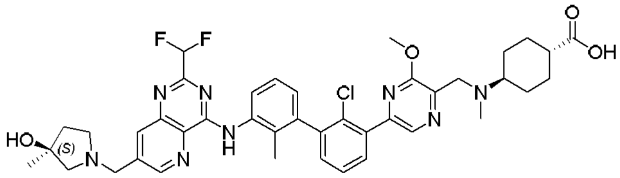

**Step 1: Synthesis of methyl (1*r*,4*r*)-4-(((5-(2-chloro-3'-((2-(difluoromethyl)-7-(((*S*)-3-hydroxy-3-methylpyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

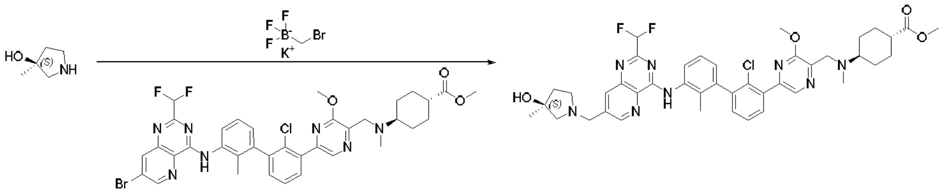


At room temperature, (*S*)-3-methylpyrrolidin-3-ol (0.90 g, 7.83 mmol) was dissolved in ACN (20 mL), and potassium(bromomethyl)trifluoroborate (1.57 g, 7.83 mmol) and sodium carbonate (0.55 g, 5.22 mmol) were added. The reaction was heated at 80 °C for 5 h, and concentrated to proceed to the next step. Then, methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl) (methyl)amino)cyclohexane-1-carboxylate (2 g, 2.61 mmol), potassium carbonate (1.08 g, 7.83 mmol), palladium acetate (0.088 g, 0.39 mmol), 2-dicyclohexylphosphino-2',4',6'-triisopropylbiphenyl (0.25 g, 0.52 mmol), 1,4-dioxane (50 mL) and water (10 mL) were added, and the reaction was stirred at 110 °C under $N_2$. The reaction solution was concentrated under reduced pressure, and the residue was separated and purified by silica gel column chromatography (eluent: $CH_2Cl_2$/MeOH (v/v) = 20/1) to obtain a yellow solid product (0.73 g, yield 34.94%).

**Step 2: Synthesis of (1*r*,4*r*)-4-(((5-(2-chloro-3'-((2-(difluoromethyl)-7-(((S)-3-hydroxy-3-methylpyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

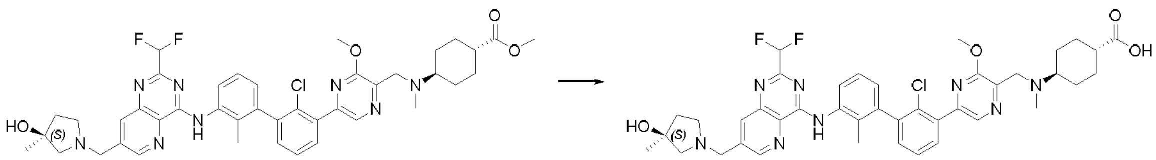

At room temperature, methyl (1*r*,4*r*)-4-(((5-(2-chloro-3'-((2-(difluoromethyl)-7-(((*S*)-3-hydroxy-3-methylpyrrolidin-l-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (0.70 g, 0.87 mmol) was dissolved in a mixed solution of 1,4-dioxane (5 mL) and water (2 mL), lithium hydroxide monohydrate (0.055 g, 1.30 mmol) was added, and the reaction was stirred at 50 °C for 2 h. The reaction solution was concentrated under reduced pressure to remove solution, and the residue was separated and purified by silica gel column chromatography (eluent: $CH_2Cl_2$/MeOH (v/v) = 5/1) to obtain 0.43 g of a yellow solid product with a yield of 62.5 %.

MS (ESI, pos. ion) m/z: 787.3 $[M+H]^+$.

$^1$H NMR (400 MHz, DMSO-$d_6$) δ 10.36 (s, 1H), 8.97 (d, *J* = 1.9 Hz, 1H), 8.44 (s, 1H), 8.20 (d, *J* = 1.9 Hz, 1H), 7.76 (d, *J* = 8.0 Hz, 1H), 7.71 (dd, *J* = 7.7, 1.8 Hz, 1H), 7.59 (t, *J* = 7.6 Hz, 1H), 7.44 (dd, *J* = 7.6, 1.7 Hz, 1H), 7.39 (t, *J* = 7.8 Hz, 1H), 7.17 (d, *J* = 7.5 Hz, 1H), 6.71 (t, *J* = 54.5 Hz, 1H), 4.66 (s, 1H), 3.97 (s, 3H), 3.91 (d, *J* = 3.3 Hz, 2H), 3.77 (s, 2H), 3.57 (s, 2H), 2.76 (t, *J* = 8.0 Hz, 1H), 2.66 (dd, *J* = 10.9, 4.4 Hz, 1H), 2.55 (d, *J* = 7.5 Hz, 2H), 2.27 (s, 3H), 2.05 (s, 3H), 1.99 - 1.94 (m, 2H), 1.90 (d, *J* = 12.2 Hz, 2H), 1.78 (q, *J* = 7.2, 6.3 Hz, 2H), 1.40 - 1.30 (m, 2H), 1.26 (s, 3H), 1.23 (d, *J* = 2.6 Hz, 2H).

**Example 47 (1*r*,4*r*)-4-(((5-(2-chloro-3'-((2-(difluoromethyl)-7-((3,3-difluoropyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

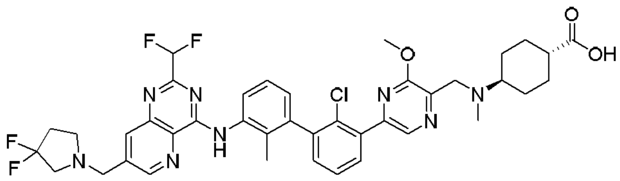

**Step 1: Synthesis of methyl (1*r*,4*r*)-4-(((5-(2-chloro-3'-((2-(difluoromethyl)-7-((3,3-difluoropyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate**

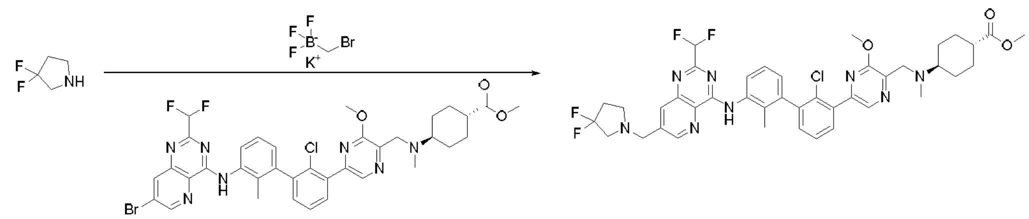

At room temperature, 3,3-difluoropyrrolidine (0.95 g, 7.83 mmol) was dissolved in ACN (20 mL), and potassium(bromomethyl)trifluoroborate (1.57 g, 7.83 mmol) and potassium carbonate (0.55 g, 5.22 mmol) were added. The reaction was heated at 80 °C for 5 h, and concentrated to proceed to the next step. Then, methyl (1*r*,4*r*)-4-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl) (methyl)amino)cyclohexane-1-carboxylate (2 g, 2.61 mmol), potassium carbonate (1.08 g, 7.83 mmol), palladium acetate (0.088 g, 0.39 mmol), 2-dicyclohexylphosphino-2',4',6'-triisopropylbiphenyl (0.37 g, 0.78 mmol), 1,4-dioxane (50 mL) and water (10 mL) were added, and the reaction was stirred at 110 °C under $N_2$. The reaction solution was concentrated under reduced pressure, and the residue was separated and purified by silica gel column chromatography (eluent: $CH_2Cl_2$/MeOH (v/v) = 20/1) to obtain a yellow solid product (1.55 g, yield 73.64%).

**Step 2: Synthesis of (1*r*,4*r*)-4-(((5-(2-chloro-3'-((2-(difluoromethyl)-7-((3,3-difluoropyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylic acid**

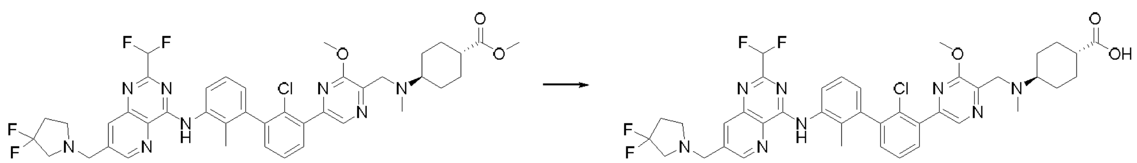

At room temperature, methyl (1*r*,4*r*)-4-(((5-(2-chloro-3'-((2-(difluoromethyl)-7-((3,3-difluoropyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclohexane-1-carboxylate (1.55 g, 1.92 mmol) was dissolved in a mixed solution of 1,4-dioxane (10 mL) and water (3 mL), lithium hydroxide monohydrate (0.12 g, 2.88 mmol) was added, and the reaction was stirred at 50 °C for 2 h. The reaction solution was concentrated under reduced pressure, and the residue was separated and purified by silica gel column chromatography (eluent: $CH_2Cl_2$/MeOH (v/v) = 5/1) to obtain a yellow solid product (1.41 g, yield 92.6%).

MS (ESI, pos. ion) m/z: 793.3 $[M+H]^+$.

$^1H$ NMR (400 MHz, DMSO-$d_6$) δ 12.01 (s, 1H), 10.38 (s, 1H), 8.96 (d, *J* = 1.9 Hz, 1H), 8.44 (s, 1H), 8.19 (d, *J* = 1.9 Hz,

1H), 7.77 - 7.67 (m, 2H), 7.59 (t, *J* = 7.6 Hz, 1H), 7.44 (dd, *J* = 7.6, 1.7 Hz, 1H), 7.39 (t, *J* = 7.8 Hz, 1H), 7.17 (d, *J* = 7.5 Hz, 1H), 6.71 (t, *J* = 54.5 Hz, 1H), 4.11 (s, 1H), 3.95 (d, *J* = 12.4 Hz, 5H), 3.76 (s, 1H), 3.17 (s, 3H), 2.98 (t, *J* = 13.2 Hz, 2H), 2.80 (t, *J* = 6.9 Hz, 2H), 2.35 - 2.24 (m, 4H), 2.18 - 2.12 (m, 1H), 2.05 (s, 3H), 1.97 (d, *J*= 9.7 Hz, 2H), 1.90 - 1.86 (m, 1H), 1.40 - 1.30 (m, 4H).

**Example 48 (*R*)-1-((6-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)azetidine-3-carboxylic acid**

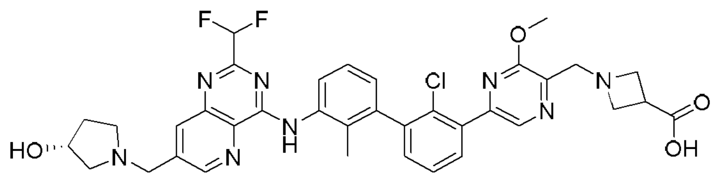

**Step 1: Synthesis of methyl (*R*)-1-((5-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)azetidine-3-carboxylate**

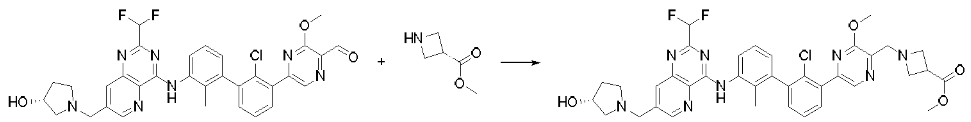

(*R*)-5-(2-Chloro-3'-((2-(difluoromethyl)-7-((3-hydroxypyrrolidin-1-yl)methyl)pyrido [3,2-*d*]pyrimidin-4-yl)amino)-2'-methyl-[1,1'-biphenyl]-3-yl)-3-methoxypyrazine-2-carbaldehyde (0.20 g, 0.32 mmol) was dissolved in a mixed solvent of DCM (5 ml) and methanol (5 ml), and methyl azetidine-3-carboxylate (0.073 g, 0.48 mmol) and TEA (0.032 g, 0.32 mmol) were added, and the mixture was reacted at room temperature for 4.5 h. Sodium cyanoborohydride (0.02 g, 0.32 mmol) was added and stirred at room temperature for 25 min. After the reaction of the raw materials was complete, saturated potassium carbonate solution was added to the reaction solution to adjust the pH to about 8, water (15 ml) was added, and then extracted with DCM (10 mL × 3). The organic phases were combined, washed with saturated brine (10 mL × 1), dried over anhydrous sodium sulfate, filtered, and the filtrate was concentrated under reduced pressure. The residue was separated and purified by silica gel column chromatography (eluent: DCM/MeOH (v/v) = 25/1) to obtain 0.066 g of a yellow solid product with a yield of 28.53%.
MS (ESI, pos. ion) m/z: 731.27 $[M+H]^+$.

**Step 2: Synthesis of (*R*)-1-((5-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)azetidine-3-carboxylic acid**

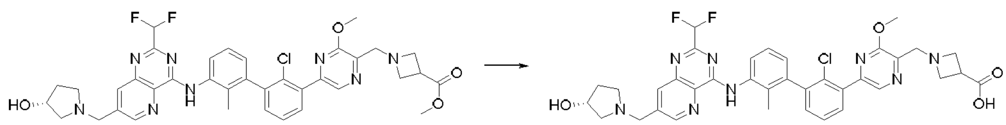

Methyl (*R*)-1-((5-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)azetidine-3-carboxylate (0.066 g, 0.09 mmol) was dissolved in a mixed solution of 1,4-dioxane (5 mL) and water (1 mL), lithium hydroxide monohydrate (0.019 g, 0.45 mmol) was added, and the reaction was stirred at room temperature overnight. After the reaction of the raw materials was complete, acetic acid (0.5 ml) was added to the system for neutralization, and the reaction solution was concentrated under reduced pressure. The residue was separated and purified by column chromatography (eluent: DCM/MeOH (v/v) = 5/1) to obtain 0.033 g of a yellow solid product with a yield of 50.98%.

MS (ESI, pos. ion) m/z: 717.25 [M+H]+.

$^1$H NMR (400 MHz, DMSO-$d_6$) δ 10.35 (s, 1H), 8.95 (s, 1H), 8.42 (s, 1H), 8.17 (s, 1H), 7.75 (d, *J* = 8.0 Hz, 1H), 7.69 (d, *J* = 7.7 Hz, 1H), 7.58 (t, *J* = 7.6 Hz, 1H), 7.44 *(d, J* = 7.5 Hz, 1H), 7.39 (t, *J* = 7.8 Hz, 1H), 7.17 (d, *J* = 7.6 Hz, 1H), 6.71 (t, *J* = 54.5 Hz, 1H), 4.25 - 4.21 (m, 1H), 3.96 (s, 3H), 3.91 (d, *J* = 13.9 Hz, 1H), 3.84 (d, *J* = 14.0 Hz, 1H), 3.72 (s, 2H), 3.57 - 3.56 (m, 2H), 3.37 (s, 2H), 3.24 - 3.17 (m, 2H), 2.77 - 2.67 (m, 3H), 2.42 (dd, *J* = 9.8, 3.5 Hz, 1H), 2.04 (s, 3H), 1.63 -1.55 (m, 1H), 1.34 - 1.33 (m, 1H).

Example 49 **(*R*)-1-((4-((2'-chloro-3'-(5-((((*R*)-2-hydroxypropyl)amino)methyl)-6-methoxypyrazin-2-yl)-2-methyl-[1,1'-biphenyl]-3-yl)amino)-2-(difluoromethyl)pyrido[3,2-*d*]pyrimidin-7-yl)methyl)pyrrolidin-3-ol**

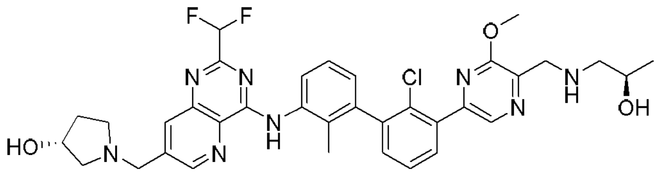

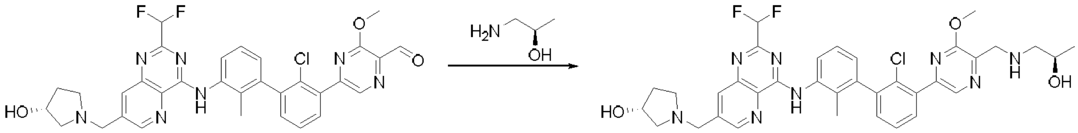

(*R*)-5-(2-Chloro-3'-((2-(difluoromethyl)-7-((3-hydroxypyrrolidin-1-yl)methyl)pyrido [3,2-*d*]pyrimidin-4-yl)amino)-2'-methyl-[1,1'-biphenyl]-3-yl)-3-methoxypyrazine-2-carbaldehyde (0.20 g, 0.32 mmol) was dissolved in a mixed solvent of DCM (5 ml) and methanol (5 ml), and D-aminopropanol (0.036 g, 0.48 mmol) and acetic acid (0.01 ml) were added, and the mixture was reacted at room temperature for 4.5 h. Sodium cyanoborohydride (0.02 g, 0.32 mmol) was added and stirred at room temperature for 25 min. After the reaction of the raw materials was complete, saturated potassium carbonate solution was added to the reaction solution to adjust the pH to about 8, water (15 ml) was added, and then extracted with DCM (10 mL × 3), the organic phases were combined, washed with saturated brine (15 mL × 1), dried over anhydrous sodium sulfate, filtered, and the filtrate was concentrated under reduced pressure to remove the solvent. The residue was separated and purified by silica gel column chromatography (eluent: $CH_2Cl_2$/MeOH (v/v) = 7/1) to obtain 45 mg of a yellow solid product with a yield of 53.95%.

MS (ESI, pos. ion) m/z: 691.27 [M+H]+.

$^1$H NMR (400 MHz, DMSO-$d_6$) δ 10.34 (s, 1H), 8.94 (s, 1H), 8.45 (s, 1H), 8.16 (s, 1H), 7.74 (d, *J* = 8.0 Hz, 1H), 7.69 (d, *J* = 7.8 Hz, 1H), 7.59 (t, *J* = 7.6 Hz, 1H), 7.44 (d, *J* = 7.6 Hz, 1H), 7.38 (t, *J* = 7.7 Hz, 1H), 7.16 (d, *J* = 7.6 Hz, 1H), 6.70 (t, *J* = 54.5 Hz, 1H), 4.24 - 4.20 (m, 1H), 3.97 (s, 3H), 3.93 *(d, J* = 9.0 Hz, 2H), 3.88 - 3.84 (m, 2H), 3.77 - 3.71 (m, 3H), 2.76 - 2.66 (m, 3H), 2.61 - 2.56 (m, 2H), 2.40 (d, *J* = 9.7 Hz, 2H), 2.03 (s, 3H), 1.62 - 1.55 (m, 1H), 1.36 - 1.30 (m, 1H), 1.05 (d, *J* = 6.1 Hz, 3H).

$^{13}$C NMR (151 MHz, DMSO-$d_6$) δ 159.23, 157.34, 157.18, 151.77, 147.43, 144.55, 143.63, 141.57, 141.39, 140.55, 136.96, 136.61, 135.85, 135.07, 132.17, 131.43, 130.44, 127.79, 127.60, 126.30, 126.00, 113.06, 69.88, 65.58, 62.87, 57.10, 56.96, 54.21, 52.80, 49.39, 34.92, 21.96, 15.83.

**Example 50 (*S*)-1-((5-(2-chloro-3'-((2-(difluoromethyl)-7-(((*S*)-3-hydroxypyrrolidin-1-yl)methyl)pyrido[3,2-*d*]pyrimidin-4-yl)amino)-2'-methyl-[1,1'-biphenyl]-3-yl)-3-methoxy pyrazin-2-yl)methyl)pyrrolidin-3-ol**

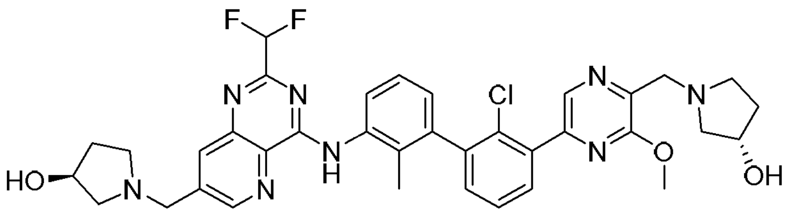

**Step 1: Synthesis of 5-bromo-2-(1,3-dioxolan-2-yl)-3-methoxypyrazine**

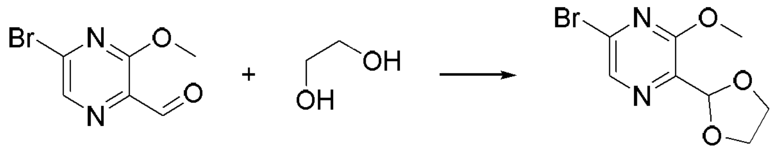

5-Bromo-3-methoxypyrazine-2-carboxaldehyde (8.00 g, 36.86 mmol), toluene (100.0 mL), ethylene glycol (4.58 g, 73.72 mmol) and p-toluenesulfonic acid monohydrate (0.35 g, 1.84 mmol) were added to the reaction flask in sequence. After installing a water separator, the mixture was heated to 130 °C and stirred for 3 h. The mixture was concentrated to dryness. The residue was diluted with ethyl acetate (60 mL) and water (100 mL) in sequence. After separation, the aqueous phase was extracted with ethyl acetate (100 mL). The organic phases were combined, washed with saturated brine (200 mL), dried over anhydrous sodium sulfate, filtered and concentrated to obtain 9.26 g of a brown oily product with a yield of 96.2 %.
MS (ESI, pos. ion) m/z: 261.1 $[M+H]^+$;

**Step 2: Synthesis of 3'-(5-(1,3-dioxolan-2-yl)-6-methoxypyrazin-2-yl)-2'-chloro-2-methyl-[1,1'-biphenyl]-3-amine**

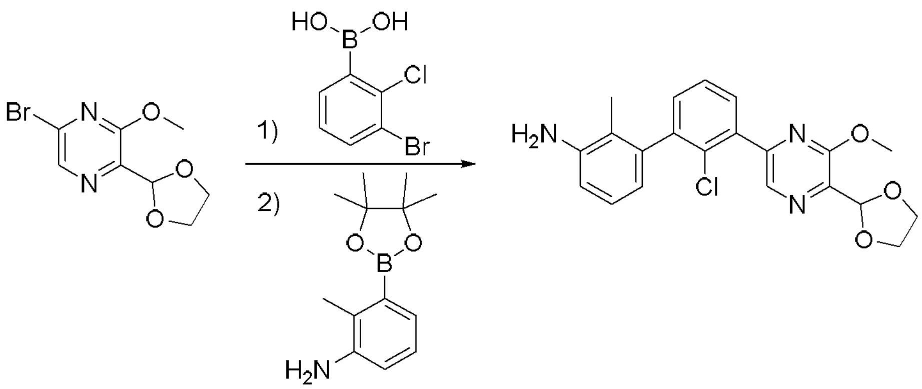


5-bromo-2-(1,3-dioxolan-2-yl)-3-methoxypyrazine (9.62 g, 36.85 mmol), (3-bromo-2-chlorophenyl)boric acid (9.10 g, 38.69 mmol), bis(triphenylphosphine)palladium dichloride (1.29 g, 1.84 mmol), tri(o-methylphenyl)phosphine (1.12 g, 3.69 mmol), potassium carbonate (12.73 g, 92.13 mmol), 1,4-dioxane (100 mL) and water (20 mL) were added in sequence to the reaction flask. After nitrogen substitution, the mixture was heated to 90 °C and stirred for 45 min. The temperature was lowered to room temperature, and 2-methyl-3-(4,4,5,5-tetramethyl-1,3,2-dioxaborolan-2-yl)aniline (8.59 g, 36.84 mmol) was added to the above system. After nitrogen replacement, the mixture was heated to reflux and stirred for 8 h. After the reaction was completed, the reaction mixture was separated with water (100 mL), and the aqueous phase was extracted with ethyl acetate (100 mL). The organic phases were combined, washed with saturated brine (200 mL), dried over anhydrous sodium sulfate, filtered, concentrated, and the residue was separated and purified by silica gel column chromatography (PE:EA (v/v) = 2:1) to obtain the target product as a white solid (9.40 g, yield 64.13%).

MS (ESI, pos. ion) m/z: 398.2 $[M+H]^+$;
$^1$H NMR (400 MHz, $CDCl_3$) δ 8.53 (s, 1H), 7.56 (dd, *J* = 7.6, 1.7 Hz, 1H), 7.41 (t, *J* = 7.6 Hz, 1H), 7.33 (dd, *J* = 7.5, 1.7 Hz, 1H), 7.10 (t, *J* = 7.7 Hz, 1H), 6.75 (d, *J* = 7.6 Hz, 1H), 6.66 (d, *J* = 7.5 Hz, 1H), 6.28 (s, 1H), 4.30 (dd, *J* = 8.5, 5.1 Hz, 2H), 4.12 (dd, *J* = 8.4, 5.1 Hz, 2H), 4.08 (s, 3H), 1.96 (s, 3H).

**Step 3: Synthesis of *N*-(3'-(5-(1,3-dioxolan-2-yl)-6-methoxypyrazin-2-yl)-2'-chloro-2-methyl-[1,1'-biphenyl]-3-yl)-7-bromo-2-(difluoromethyl)pyrido[3,2-*d*]pyrimidin-4-amine**

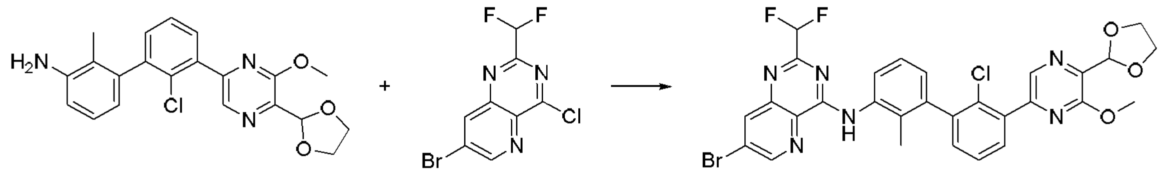

3'-(5-(1,3-Dioxolan-2-yl)-6-methoxypyrazin-2-yl)-2'-chloro-2-methyl-[1,1'-biphenyl]-3-amine (7.50 g, 18.85 mmol), 7-bromo-4-chloro-2-(difluoromethyl)pyrido[3,2-*d*]pyrimidine (6.11 g, 20.74 mmol) and isopropanol (75.0 mL) were added to the reaction bottle in sequence, and the mixture was heated to 70 °C and stirred for 1 h. TLC showed that the raw materials were basically reacted. The reaction solution was filtered, and the obtained solid was diluted with isopropanol (60 mL) and heated to 80 °C and stirred for 30 min, then transferred to room temperature and stirred for 1 h. The reaction solution was filtered to obtain the target product as a yellow solid (4.25 g, yield 34.37%).
MS (ESI, pos. ion) m/z: 655.1 $[M+H]^+$;

**Step 4: Synthesis of (4-((3'-(5-(1,3-dioxolan-2-yl)-6-methoxypyrazin-2-yl)-2'-chloro-2-methyl-[1,1'-biphenyl]-3-yl)amino)-2-(difluoromethyl)pyrido[3,2-*d*]pyrimidin-7-yl)methanol**

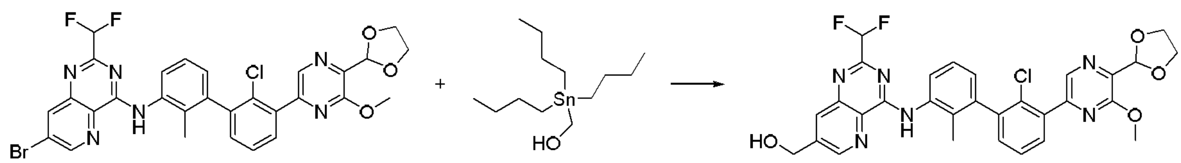

*N*-(3'-(5-(1,3-Dioxolan-2-yl)-6-methoxypyrazin-2-yl)-2'-chloro-2-methyl-[1,1'-biphenyl]-3-yl)-7-bromo-2-(difluoromethyl)pyrido[3,2-*d*]pyrimidin-4-amine (1.94 g, 2.96 mmol), (tributylstannyl)methanol (1.24 g, 3.85 mmol), chloro [(di(1-adamantyl)-*N*-butylphosphine)-2-(2-aminobiphenyl)]palladium(II) (0.10 g, 0.15 mmol) and 1,4-dioxane (40.0 mL) were added to the reaction flask in sequence. After nitrogen substitution, the mixture was heated to 105 °C and stirred for 5 h. The residue was concentrated under reduced pressure and purified by silica gel column chromatography (DCM:MeOH (v/v) = 100:1) to obtain the target product as a light yellow solid (1.20 g, yield 66.84%).
MS (ESI, pos. ion) m/z: 607.2 $[M+H]^+$;

**Step 5: Synthesis of *N*-(3'-(5-(1,3-dioxolan-2-yl)-6-methoxypyrazin-2-yl)-2'-chloro-2-methyl-[1,1'-biphenyl]-3-yl)-7-(chloromethyl)-2-(difluoromethyl)pyrido[3,2-*d*]pyrimidin-4-amine**

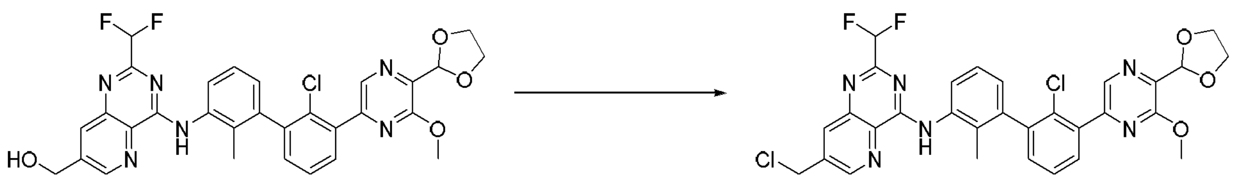

To the reaction flask, (4-((3'-(5-(1,3-dioxolan-2-yl)-6-methoxypyrazin-2-yl)-2'-chloro-2-methyl-[1,1'-biphenyl]-3-yl)amino)-2-(difluoromethyl)pyrido[3,2-*d*]pyrimidin-7-yl)methanol (2.00 g, 3.29 mmol), dichloromethane (25.0 mL), triethylamine (0.67 g, 6.58 mmol) and thionyl chloride (0.59 g, 4.94 mmol) were added in sequence, and the mixture was stirred at room temperature for 1 h. The above mixture was concentrated under reduced pressure to obtain 2.06 g of a yellow-green solid with a yield of 100 %.
MS (ESI, pos. ion) m/z: 625.2 $[M+H]^+$;

**Step 6: Synthesis of (*S*)-1-((4-((3'-(5-(1,3-dioxolan-2-yl)-6-methoxypyrazin-2-yl)-2'-chloro-2-methyl-[1,1'-biphenyl]-3-yl)amino)-2-(difluoromethyl)pyrido[3,2-*d*]pyrimidin-7-yl)methyl)pyrrolidin-3-ol**

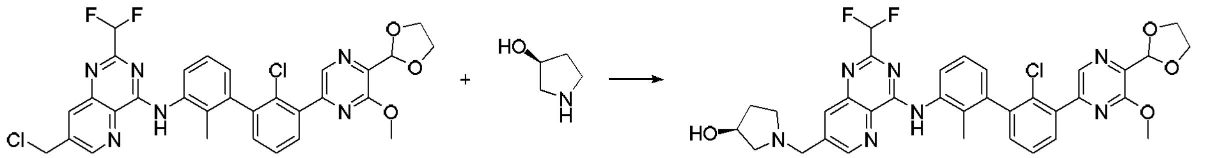

*N*-(3'-(5-(1,3-Dioxolan-2-yl)-6-methoxypyrazin-2-yl)-2'-chloro-2-methyl-[1,1'-biphenyl]-3-yl)-7-(chloromethyl)-2-(difluoromethyl)pyrido[3,2-*d*]pyrimidin-4-amine (100.0 mg, 0.16 mmol), potassium carbonate (40.0 mg, 0.32 mmol), potassium iodide (27.0 mg, 0.16 mmol), (S)-pyrrolidin-3-ol (15.0 mg, 0.17 mmol) and *N,N*-dimethylformamide (2.0 mL) were added sequentially to the reaction bottle, and the mixture was heated to 80 °C and stirred for 2 h. Ethyl acetate (20 mL) and water (20 mL) were added to the above mixture in sequence for dilution. After the obtained mixture was separated, the aqueous phase was extracted with ethyl acetate (20 mL). The organic phases were combined, washed with saturated brine (50 mL), dried over anhydrous sodium sulfate, filtered, and concentrated. The residue was separated and purified by silica gel column chromatography (DCM: MeOH (v/v) = 50:1) to obtain the target product as a light yellow solid (72.8 mg, yield 67.35%).

MS (ESI, pos. ion) m/z: 676.2 $[M+H]^+$;

**Step 7: Synthesis of (*S*)-5-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxypyrazine-2-carbaldehyde**

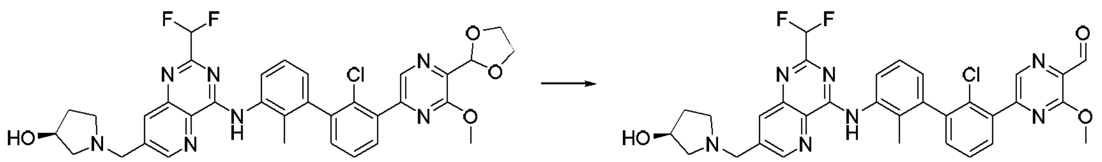

(*S*)-1-((4-((3'-(5-(1,3-Dioxolan-2-yl)-6-methoxypyrazin-2-yl)-2'-chloro-2-methyl-[1,1'-biphenyl]-3-yl)amino)-2-(difluoromethyl)pyrido[3,2-*d*]pyrimidin-7-yl)methyl)pyrrolidin-3-ol (490.0 mg, 0.72 mmol), tetrahydrofuran (8.0 mL) and hydrochloric acid (3.0 mL, 18.00 mmol, 6 mol/L) were added to the reaction bottle in sequence, and the mixture was stirred at room temperature overnight. The above mixture was concentrated to dryness, and the residue was diluted with ethyl acetate (30 mL) and saturated sodium bicarbonate aqueous solution (30 mL). After the mixture was separated, the aqueous phase was extracted with ethyl acetate (30 mL), and the organic phases were combined, washed with saturated brine (60 mL), dried over anhydrous sodium sulfate, filtered, and concentrated. The residue was separated and purified by silica gel column (PE:EA (v/v) = 3:1) to obtain the crude target product as a yellow solid (380.0 mg, yield 82.96%).
MS (ESI, pos. ion) m/z: 632.3 $[M+H]^+$;

**Step 8: Synthesis of (*S*)-1-((5-(2-chloro-3'-((2-(difluoromethyl)-7-(((*S*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)pyrrolidin-3-ol**

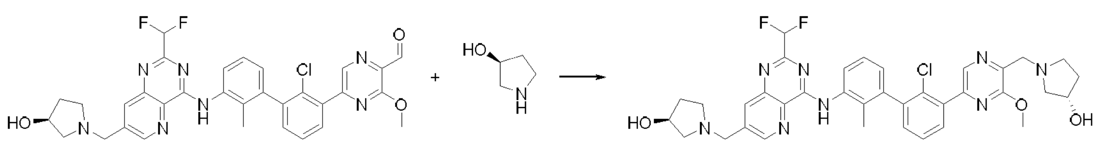

To the reaction bottle, (*S*)-5-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxypyrazine-2-carbaldehyde (183.0 mg, 0.29 mmol), dichloromethane (3.0 mL), (S)-pyrrol-3-ol (25.0 mg, 0.29 mmol), methanol (3.0 mL) and acetic acid (0.05 mL) were added in sequence. The mixture was stirred at room temperature for 1 h and then concentrated. The residue was separated and purified by silica gel column (DCM:MeOH (v/v) = 30:1) to obtain the target product as a yellow-green solid (70.0 mg, yield 34.38%).

MS (ESI, pos. ion) m/z: 703.3 $[M+H]^+$.

$^1H$ NMR (400 MHz, $CDCl_3$) δ 9.37 (s, 1H), 8.87 (s, 1H), 8.53 (d, *J* = 8.1 Hz, 1H), 8.48 (s, 1H), 8.19 (s, 1H), 7.62 (d, *J* = 7.3 Hz, 1H), 7.50 - 7.39 (m, 2H), 7.36 (d, *J* = 7.1 Hz, 1H), 7.09 (d, *J* = 7.4 Hz, 1H), 6.59 (t, *J* = 55.0 Hz, 1H), 4.44 - 4.35 (m, 2H), 4.08 - 3.98 (m, 5H), 3.93 - 3.83 (m, 2H), 3.21 (dd, *J* = 15.3, 7.6 Hz, 1H), 3.05 - 2.99 (m, 1H), 2.98 - 2.82 (m, 4H), 2.72 (t, *J* = 12.1 Hz, 3H), 2.41 (dd, *J*= 15.1, 8.5 Hz, 1H), 2.30 - 2.17 (m, 5H), 1.93 - 1.74 (m, 2H).

**Example 51 (*S*)-1-((4-((2'-chloro-3'-(5-((((3*R*,4*R*)-3-hydroxytetrahydro-2*H*-pyran-4-yl)amino)methyl)-6-methoxypyrazin-2-yl)-2-methyl-[1,1'-biphenyl]-3-yl)amino)-2-(difluoromethyl)pyrido[3,2-*d*]pyrimidin-7-yl)methyl)pyrrolidin-3-ol**

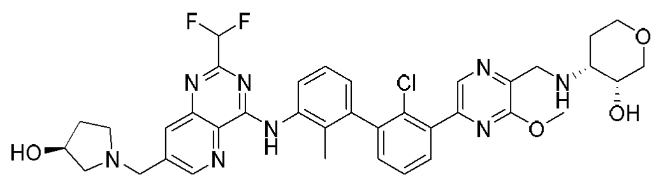

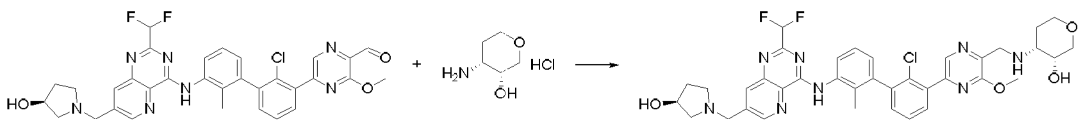

To the reaction bottle, (*S*)-5-(2-chloro-3'-((2-(difluoromethyl)-7-((3-hydroxypyrrolidin-1-yl)methyl)pyrido[3,2-*d*]pyrimidin-4-yl)amino)-2'-methyl-[1,1'-biphenyl]-3-yl)-3-methoxy pyrazine-2-carbaldehyde (90.0 mg, 0.14 mmol), triethylamine (16.0 mg, 0.15 mmol), dichloromethane (0.5 mL), methanol (0.5 mL), (3*R*,4*R*)-4-aminotetrahydro-2*H*-pyran-3-ol hydrochloride (24.0 mg, 0.15 mmol) and acetic acid (0.05 mL) were added in sequence. After the addition was complete, the mixture was stirred at room temperature for 30 min. The mixture was placed in an ice bath and stirred for 10 min. Sodium cyanoborohydride (9.7 mg, 0.15 mmol) was added thereto. After the addition, the mixture was slowly heated to room temperature and stirred for 4 h. The mixture was diluted with dichloromethane (10 mL) and water (20 mL) and then separated. The aqueous phase was extracted with a mixed solvent (DCM:MeOH (v/v) = 10:1, 20 mL). The organic phases were combined, washed with saturated brine (30 mL), dried over anhydrous sodium sulfate, filtered, and concentrated. The residue was separated and purified by silica gel column (DCM:MeOH (v/v) = 20:1). After concentration, it was separated and purified by thick preparative plate (DCM:MeOH(v/v) = 15:1) to obtain the target product as a light yellow solid (10.0 mg, yield 9.58%).

MS (ESI, pos. ion) m/z: 733.5 $[M+H]^+$.

$^1H$ NMR (400 MHz, $CDCl_3$) δ 9.37 (s, 1H), 8.87 (s, 1H), 8.51 (d, *J* = 8.2 Hz, 1H), 8.45 (s, 1H), 8.19 (s, 1H), 7.62 (d, *J* = 7.5 Hz, 1H), 7.48 - 7.34 (m, 3H), 7.09 (d, *J* = 7.5 Hz, 1H), 6.58 (t, *J* = 55.0 Hz, 1H), 4.42 - 4.36 (m, 1H), 4.11 - 4.00 (m, 6H), 3.99 - 3.93 (m, 1H), 3.90 (s, 2H), 3.81 (s, 1H), 3.49 - 3.37 (m, 2H), 3.01 - 2.82 (m, 4H), 2.76 (d, *J* = 10.0 Hz, 1H), 2.72 - 2.65 (m, 1H), 2.47 - 2.39 (m, 1H), 2.27 - 2.24 (m, 3H), 2.24 - 2.15 (m, 1H), 1.98 - 1.85 (m, 1H), 1.84 - 1.75 (m, 1H), 1.64 (d, *J* = 12.5 Hz, 1H).

**Example 52 (1*S*,3*S*)-3-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxy pyrazin-2-yl)methyl)(methyl)amino)cyclopentane-1-carboxylic acid**

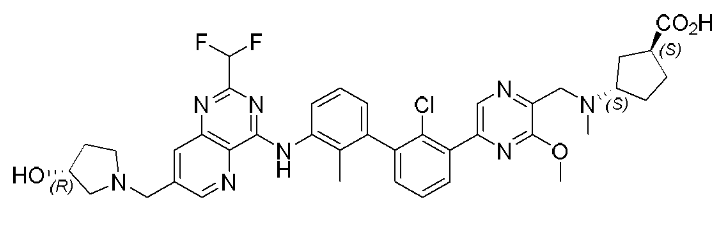

**Step 1: Synthesis of methyl (1*S*,3*S*)-3-(((5-(3-bromo-2-chlorophenyl)-3-methoxypyrazin-2-yl)methyl)amino)cyclopentane-1-carboxylate**

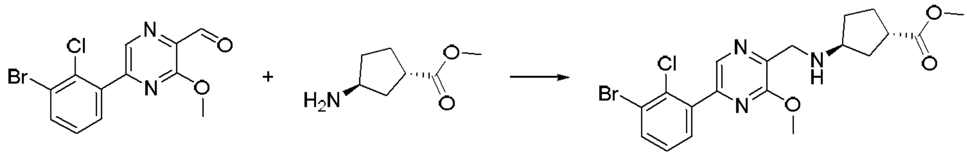

At room temperature, 5-(3-bromo-2-chlorophenyl)-3-methoxypyrazine-2-carbaldehyde (4 g, 12.21 mmol) and methyl (1S,3S)-3-aminocyclopentane-1-carboxylate (2.27 g, 15.87 mmol) were added to 2,2,2-trifluoroethanol (20 mL) and dichloromethane (20 mL). The mixture was heated to 45 °C and stirred for reaction. Then, sodium cyanoborohydride (0.77 g, 12.21 mmol) was slowly added and the reaction was stirred for 1 h. The reaction solution was concentrated under reduced pressure to remove the solvent, and the residue was separated and purified by silica gel column chromatography (eluent: PE/EA (v/v) = 3/1) to obtain 3.7 g of a yellow oily product with a yield of 66.6%.
MS (ESI, pos. ion) m/z: 454.0 $[M+H]^+$;

**Step 2: Synthesis of methyl (1*S*,3*S*)-3-(((5-(3-bromo-2-chlorophenyl)-3-methoxypyrazin-2-yl)methyl)amino)cyclopentane-1-carboxylate**

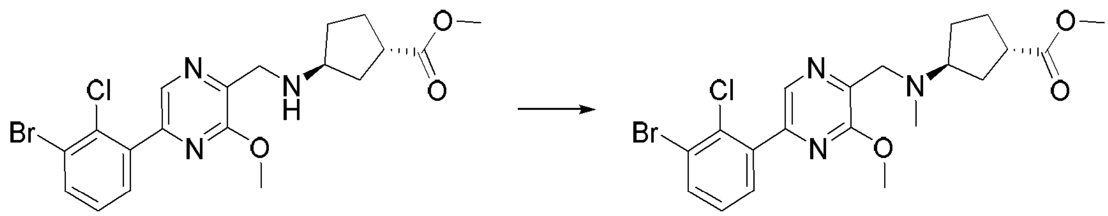

methyl (1*S*,3*S*)-3-(((5-(3-bromo-2-chlorophenyl)-3-methoxypyrazin-2-yl)methyl)(methyl)amino)cyclopentane-1-carboxylate

At room temperature, methyl (1*S*,3*S*)-3-(((5-(3-bromo-2-chlorophenyl)-3-methoxypyrazin-2-yl)methyl)amino) cyclopentane-1-carboxylate (3.6 g, 7.92 mmol) and formaldehyde (0.24 g, 7.92 mmol) were added to DCM (20 mL) and 2,2,2-trifluoroethanol (20 mL), and the mixture was stirred at 45 °C for 2 h, and then sodium cyanoborohydride (0.50 g, 7.92 mmol) was slowly added and the reaction was continued for 2 h. The reaction solution was concentrated under reduced pressure to remove the solvent, and the residue was separated and purified by silica gel column chromatography (eluent: PE/EA (v/v) = 1/1) to obtain 3.2 g of a yellow oily product with a yield of 86.2%.
MS (ESI, pos. ion) m/z: 468.0 $[M+H]^+$;

**Step 3: Synthesis of methyl (1*S*,3*S*)-3-(((5-(3'-amino-2-chloro-2'-methyl-[1,1'-biphenyl]-3-yl)-3-methoxypyrazin-2-yl)methyl)(methyl)amino)cyclopentane-1-carboxylate**

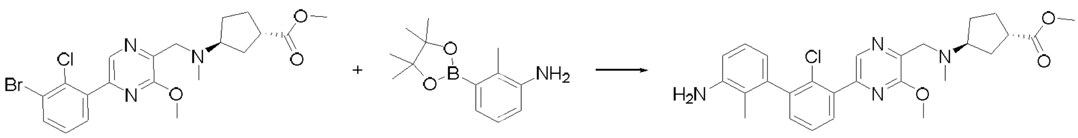

At room temperature, methyl (1*S*,3*S*)-3-(((5-(3-bromo-2-chlorophenyl)-3-methoxypyrazin-2-yl)methyl)(methyl) amino)cyclopentane-1-carboxylate (3.0 g, 6.40 mmol), 3-amino-2-methylphenylboronic acid pinacol ester (2.98 g, 12.8 mmol), 1,1'-bis(di-cyclohexylphosphino)ferrocene palladium dichloride (0.24 g, 0.32 mmol) and potassium carbonate

(1.77 g, 12.8 mmol) were added to 1,4-dioxane (30 mL) and water (6 mL), the nitrogen was replaced, and the mixture was heated to 90 °C and stirred for 6 h. The reaction solution was concentrated under reduced pressure to remove the solvent, and the residue was separated and purified by silica gel column chromatography (eluent: PE/EA (v/v) = 1/1) to obtain 2.0 g of a yellow oily product with a yield of 63.1%.
MS (ESI, pos. ion) m/z: 495.4 $[M+H]^+$;

**Step 4: Synthesis of methyl (1*S*,3*S*)-3-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclopentane-1-carboxylate**

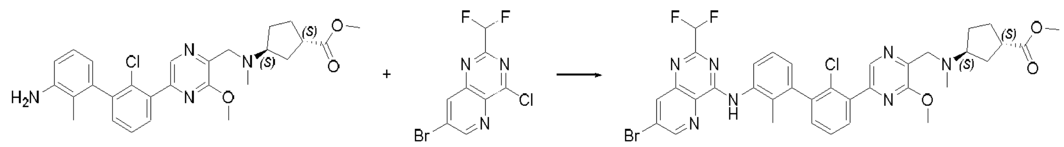

At room temperature, methyl (1*S*,3*S*)-3-(((5-(3'-amino-2-chloro-2'-methyl-[1,1'-biphenyl]-3-yl)-3-methoxypyrazin-2-yl)methyl)(methyl)amino)cyclopentane-1-carboxylate (1.9 g, 3.84 mmol) and 7-bromo-4-chloro-2-(difluoromethyl) pyrido[3,2-*d*]pyrimidine (1.36 g, 4.61 mmol) were dissolved in *tert*-butanol (30 mL) and stirred at 100 °C for 10 h. The reaction solution was concentrated under reduced pressureto remove solution, and the residue was separated and purified by column chromatography (eluent: DCM/MeOH (v/v) = 20/1) to obtain 2.80 g of a yellow solid product with a yield of 96.9%.
MS (ESI, pos. ion) m/z: 752.2 $[M+H]^+$;

**Step 5: Synthesis of methyl (1*S*,3*S*)-3-(((5-(2-chloro-3'-((2-(difluoromethyl)-7-vinylpyrido[3,2-*d*]pyrimidin-4-yl) amino)-2'-methyl-[1,1'-biphenyl]-3-yl)-3-methoxypyrazin-2-yl)methyl)(methyl)amino)cyclopentane-1-carboxylate**

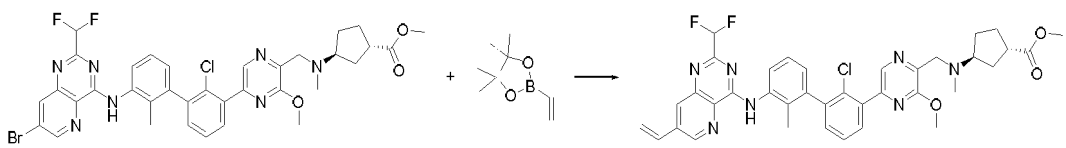

At room temperature, methyl (1*S*,3*S*)-3-(((5-(3'-((7-bromo-2-(difluoromethyl)pyrido[3,2-*d*]pyrimidin-4-yl)amino)-2-chloro-2'-methyl-[1,1'-biphenyl]-3-yl)-3-methoxypyrazin-2-yl)methyl)(methyl)amino)cyclopentane-1-carboxylate ( 2.7 g, 3.59 mmol), 2-vinylboronic acid pinacol ester (2.76 g, 17.95 mmol), 1,1'-bis(di-cyclohexylphosphino)ferrocene palladium dichloride (0.15 g, 0.18 mmol) and potassium carbonate (1.52 g, 7.18 mmol) were added to 1,4-dioxane (30 mL) and water (7.5 mL), the nitrogen was replaced, and the mixture was heated to 100 °C and stirred for 10 h. The reaction solution was concentrated under reduced pressure to remove solution, and the residue was separated and purified by silica gel column chromatography (eluent: $CH_2Cl_2$/MeOH (v/v) = 20/1) to obtain 2.0 g of a yellow solid product with a yield of 79.7 %.

MS (ESI, pos. ion) m/z: 700.2 $[M+H]^+$.

**Step 6: Synthesis of methyl (1*S*,3*S*)-3-(((5-(2-chloro-3'-((2-(difluoromethyl)-7-formylpyrido[3,2-*d*]pyrimidin-4-yl) amino)-2'-methyl-[1,1'-biphenyl]-3-yl)-3-methoxypyrazin-2-yl)methyl)(methyl)amino)cyclopentane-1-carboxylate**

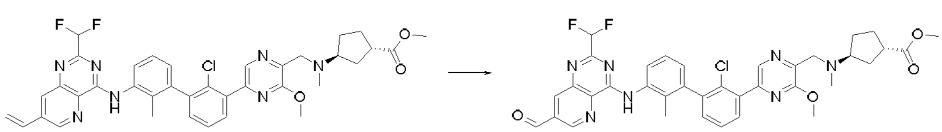

At room temperature, methyl (1*S*,3*S*)-3-(((5-(2-chloro-3'-((2-(difluoromethyl)-7-vinylpyrido[3,2-*d*]pyrimidin-4-yl)amino)-2'-methyl-[1,1'-biphenyl]-3-yl)-3-methoxypyrazin-2-yl) methyl)(methyl)amino)cyclopentane-1-carboxylate (2.0 g, 2.86 mmol) was dissolved in 1,4-dioxane (50 mL) and water (10 mL), and then potassium osmate dihydrate (0.053 g, 0.14 mmol) and sodium periodate (2.14 g, 10.01 mmol) were slowly added and the reaction was continued with stirring for 5 h. The reaction solution was concentrated under reduced pressureto remove solution, and the residue was separated and purified by column chromatography (eluent: DCM/MeOH (v/v) = 10/1) to obtain 0.51 g of a yellow oily product with a yield of 25.3%.

MS (ESI, pos. ion) m/z: 702.5 $[M+H]^+$.

**Step 7: Synthesis of methyl (1*S*,3*S*)-3-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclopentane-1-carboxylate**

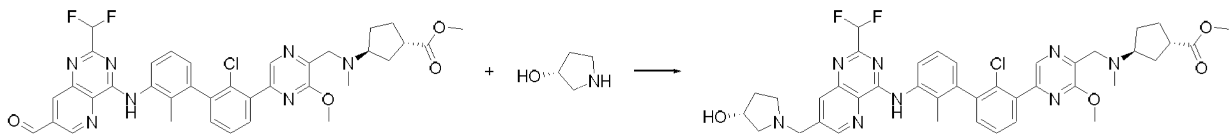

At room temperature, methyl (1*S*,3*S*)-3-(((5-(2-chloro-3'-((2-(difluoromethyl)-7-formylpyrido[3,2-*d*]pyrimidin-4-yl)amino)-2'-methyl-[1,1'-biphenyl]-3-yl)-3-methoxypyrazin-2-yl) methyl)(methyl)amino)cyclopentane-1-carboxylate (0.50 g, 0.71 mmol) and (3R)-pyrrolidin-3-ol (0.12 g, 1.42 mmol) were added to 2,2,2-trifluoroethanol (10 mL) and DCM (10 mL). The reaction was stirred at 45 °C for 30 min. Then, sodium cyanoborohydride (0.045 g, 0.71 mmol) was slowly added and the reaction was continued for 1 h. The reaction solution was concentrated under reduced pressureto remove solution, and the residue was separated and purified by column chromatography (eluent: DCM/MeOH (v/v) = 10/1) to obtain 0.34 g of a yellow oily product with a yield of 61.8%.

MS (ESI, pos. ion) m/z: 773.6 $[M+H]^+$.

**Step 8: Synthesis of (1*S*,3*S*)-3-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclopentane-1-carboxylic acid**

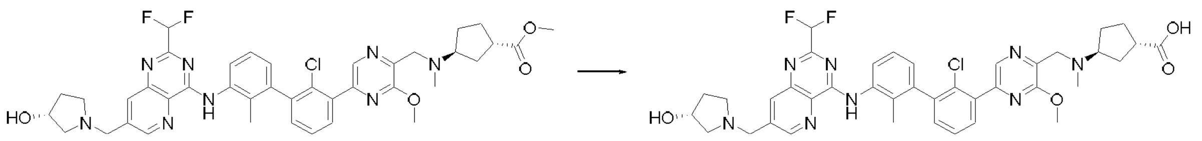

At room temperature, methyl (1*S*,3*S*)-3-(((5-(2-chloro-3'-((2-(difluoromethyl)-7-(((*R*)-3-hydroxypyrrolidin-1-yl)methyl)pyrido[3,2-*d*]pyrimidin-4-yl)amino)-2'-methyl-[1,1'-biphenyl]-3-yl)-3-methoxypyrazin-2-yl)methyl)(methyl)amino)cyclopentane-1-carboxylate (0.34 g, 0.44 mmol) and lithium hydroxide monohydrate (0.092 g, 2.2 mmol) were added to 1,4-dioxane (8 mL) and water (2 mL), and the reaction was stirred at 45 °C for 10 h. The reaction solution was concentrated under reduced pressureto remove solution, and the residue was separated and purified by column chromatography (eluent: DCM/MeOH (v/v) = 8/1) to obtain 0.20 g of a yellow solid product with a yield of 59.9%.

MS (ESI, pos. ion) m/z: 759.2 $[M+H]^+$;

$^1$H NMR (400 MHz, DMSO-$d_6$) δ 10.55 (s, 1H), 9.17 - 9.08 (m, 1H), 8.60 (s, 1H), 8.49 (s, 1H), 7.77 - 7.66 (m, 2H), 7.62 (t, *J*=7.6 Hz, 1H), 7.52 - 7.46 (m, 1H), 7.40 (t, *J* = 7.8 Hz, 1H), 7.19 (d, *J* = 7.6 Hz, 1H), 6.72 (t, *J* = 54.5 Hz, 1H), 4.65 (s, 1H), 4.56 - 4.40 (m, 1H), 4.03 (s, 3H), 4.00 (d, *J* = 7.3 Hz, 1H), 3.86 (d, *J* = 14.1 Hz, 2H), 3.56 (s, 3H), 3.17 (s, 2H), 2.96 (p, *J* = 7.8 Hz, 1H), 2.83 (s, 2H), 2.29 (s, 1H), 2.22 - 2.05 (m, 4H), 2.04 (s, 3H), 1.98 (s, 1H), 1.89 (q, *J* = 9.8, 8.8 Hz, 2H), 1.78 - 1.65 (m, 2H).

**Biological Assay**

**Experiment 1: Detecting the inhibitory activity of compounds on the PD1/PD-L1 pathway at the cellular level using the PD1/PD-L1 Blockade Bioassay method**

1. Experimental materials:

| Reagents and consumables | Manufacturer | Part Number |
|---|---|---|
| Hygromycin B | Gibco | 10687-010 |
| Puromycin | Gibco | A11138-03 |
| G418 | Gibco | GB10131-027 |
| 1640 medium | Gibco | A10491-01 |
| 1640 medium (without phenol red) | Gibco | 11835055 |
| F12K medium | Gibco | 21127-002 |
| One-GloTM Luciferase Assay System | Promega | E6120 |
| 384-well plate | Corning | 3570 |

2. Specific experimental steps:

1) Cell recovery and culture
PD-1 Jukat T cells were revived and cultured with 1640 medium containing 100 μg/mL Hygromycin B, 0.5 μg/mL Puromycin, 10% FBS and 1% dual antibody; PD-L1 aAPC/CHO-K1 cells were revived and cultured with F12K medium containing 100 μg/mL Hygromycin B, 200 μg/mL G418, 10% FBS and 1% dual antibody.
2) Cell plating
The revived cells need to be passaged 2-3 times. When the cells enter the exponential growth phase with good growth status, the PD-L1 aAPC/CHO-K1 was digested with trypsin, and the cells were resuspended in F12K containing 5% FBS and adjusted to a density of $5 \times 10^5$ cells/mL. 20 μl/well were plated in a 384-well plate (10,000 cells/well) and incubated in an incubator overnight for 16-20 h.
3) Cellular drug delivery

a Preparation of compound stock solution: Dissolve the compound to be tested in DMSO to prepare a 20 mM stock solution.
b Compound gradient dilution: dilute the compound in 1640 medium containing 5% FBS without antibiotics or phenol red, and make 3-fold gradient dilutions to prepare 10 concentration gradients (2X).
c Adding drugs: Take out the cell culture plate, discard the supernatant, add 20 μl/well of the prepared compound, and incubate in the incubator for 1.5 h.

4) Cell co-culture
PD-1 Jukat T cells were collected, washed once with PBS, and resuspended in 1640 medium containing 5% FBS without antibiotics or phenol red. The density was adjusted to $1 \times 10^6$ cells/mL. 20 μl PD-1 Jukat T cells (20,000 cells/well) were added to each well and incubated in the incubator for 6 h.
5) Plate reading test
ONE-Glo™ Luciferase Assay reagent was thawed to room temperature in advance and then added to a 384-well plate at 40 μl/well. Incubate in the dark for 15 minutes and read the fluorescence signal on a plate reader.
6) Data Analysis

① %Activity calculation formula:

$$\%Activity = (\overline{RLU_{cmpd}} - \overline{RLU_{vehicle}}) / (\overline{RLU_{positive}} - \overline{RLU_{vehicle}}) \times 100$$

Among them, %Activity: % Activity
RLU: Resulting Luminescence unit
$\overline{RLU}_{vehicle}$: Average RLU value of negative control wells on each plate

$\overline{RLU}_{positive}$: Average RLU value of positive control wells on each plate
Vehicle control: DMSO (0.1%) corresponding to the test value was used as negative control (its % Activity was 0%);
Positive control: The highest detection value at the atezolizumab administration concentration was used as the positive control (its % Activity was 100%).
② $EC_{50}$ value calculation method: The $EC_{50}$ value was obtained by the nonlinear fitting formula of Graph Pad Prism 8.0 software. The calculation formula is as follows:

$$Y=Bottom + (Top-Bottom)/(1+10^{\wedge}((LogEC_{50}-X) \times HillSlope))$$

X: log of activator concentration ( logarithm of compound concentration ); Y: % Activity
③ $E_{max}$ definition: The maximum % Activity value corresponding to the maximum activation value of the compound relative to atezolizumab (the activation value is set to 100%).

Experiments have shown that the compounds of the present invention have high inhibitory activity on the protein interaction of PD-1/PD-L1 at the molecular level. Specifically, the inhibitory activity of the compounds of the present invention on the protein interaction of PD-1/PD-L1 is less than 500 nM; most of the compounds have an $IC_{50}$ of less than 100 nM, and some of the compounds have an $IC_{50}$ of less than 50 nM. Among them, the results of the inhibition test of the compounds of the present invention on the PD1/PD-L1 pathway at the cellular level are shown in Table 2.

Table 2 Experimental results of the activity of the compounds provided in the examples of the present invention in inhibiting the PD1/PD-L1 pathway at the cellular level

| Example No. | $EC_{50}$ (nM) | $E_{max}$ (%) |
|---|---|---|
| Example 1 | 44.0 | 98.2 |
| Example 2 | 98.3 | 117.9 |
| Example 9 | 90.7 | 117.3 |
| Example 10 | 70.7 | 79.3 |
| Example 11 | 20.4 | 98.5 |
| Example 13 | 38.2 | 103.5 |
| Example 14 | 31.3 | 103.3 |
| Example 15 | 41.8 | 97.0 |
| Example 16 | 28.1 | 107 |
| Example 17 | 109 | 94.1 |
| Example 18 | 71.1 | 68 |
| Example 19 | 43.2 | 80 |
| Example 20 | 63.5 | 108 |
| Example 21 | 104.7 | 65.0 |
| Example 23 | 44.4 | 74 |
| Example 27 | 111.7 | 63.0 |
| Example 28 | 49.5 | 95 |
| Example 29 | 42.9 | 89.0 |
| Example 32 | 76.3 | 84.1 |
| Example 33 | 58.6 | 95.3 |
| Example 35 | 46.1 | 101.7 |
| Example 36 | 55.5 | 85.0 |
| Example 40 | 57.7 | 121.0 |
| Example 41 | 90.6 | 79 |

(continued)

| Example No. | $EC_{50}$ (nM) | $E_{max}$ (%) |
|---|---|---|
| Example 42 | 90.9 | 74 |
| Example 44 | 84.5 | 69 |
| Example 45 | 94.7 | 87.3 |
| Example 46 | 60.7 | 86.7 |
| Example 50 | 99.1 | 96 |
| Example 51 | 99.1 | 96 |
| Example 52 | 75.8 | 74.3 |

**Experiment 2: Pharmacokinetics experiment**

1. Experimental Methods

1) Preparation of the compound solutions of Example 1 and Example 17: The test compound was prepared into a solution using 10% dimethyl sulfoxide, 10% Kolliphor HS15 and 80% physiological saline;
Preparation of the compound solution of Example 11: The compound to be tested is prepared into a solution with 5% dimethyl sulfoxide, 5% Kolliphor HS15 and 90% saline for oral administration or intravenous injection.

2) Male SD rats weighing 250-345 g were randomly divided into two groups. One group was intravenously injected with the test compound at a dose of 1.0 mg/kg, and the other group was orally administered with the test compound at a dose of 5.0 mg/kg. Blood was collected at 0.083, 0.25, 0.5, 1, 2, 5, 7 and 24 hours after administration.

3) A standard curve of an appropriate range was established according to the sample concentration, and the concentration of the test compound in the plasma sample was determined in the MRM mode using an AB SCIEX Q TRAP 4000 LC-MS/MS. The pharmacokinetic parameters were calculated using the non-compartmental model method using WinNonLin 6.3 software.

The experimental results are shown in Table 3.

Table 3 Pharmacokinetic parameters of the compounds of the present invention in SD rats

| Example No. | Route of administration | Dose (mg/kg) | $T_{1/2}$ (h) | $C_{max}$ (ng/mL) | $AUC_{last}$ (h*ng/mL) | Cl (mL/min*kg) | Vss (L/kg) | F(%) |
|---|---|---|---|---|---|---|---|---|
| Example 1 | iv | 1 | 5.4 | 877 | 7710 | 2.1 | 0.82 | N/A |
| | ig | 5 | 4.9 | 935 | 12300 | N/A | N/A | 32 |
| Example 11 | iv | 1 | 3.9 | 1100 | 6370 | 2.6 | 0.75 | N/A |
| | ig | 5 | 4.3 | 1630 | 16700 | N/A | N/A | 52.9 |
| Example 17 | iv | 1 | 7.5 | 675 | 9760 | 1.6 | 0.95 | N/A |
| | ig | 5 | N/A | 1980 | 27000 | N/A | N/A | 55 |
| Example 38 | iv | 1 | 6.6 | 621 | 8890 | 1.7 | 0.91 | N/A |
| | ig | 5 | 6.1 | 1850 | 25900 | N/A | N/A | 57.8 |
| Example 40 | iv | 1 | 4.5 | 1870 | 12900 | 1.3 | 0.43 | N/A |
| | ig | 5 | 4.1 | 1260 | 15100 | N/A | N/A | 23 |
| Note: N/A means blank or not detected. | | | | | | | | |

**Experimental conclusion:** As can be seen from Table 3, the compounds of the present invention exhibited good pharmacokinetic properties in SD rats.

In the description of the present specification, the reference terms to "one embodiment", "some embodiments", "an example", "a specific example", or "some examples", and the like means that a particular feature, structure, material, or characteristic described in connection with the embodiment or example is included in at least one embodiment or example of the present disclosure. In the present specification, the schematic representation of the above terms is not necessarily directed to the same embodiment or example. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

**1.** A compound of Formula (I) or a stereoisomer, a tautomer, an *N*-oxide, a hydrate, a solvate, a metabolite, an eater, a pharmaceutically acceptable salt or a prodrug thereof,

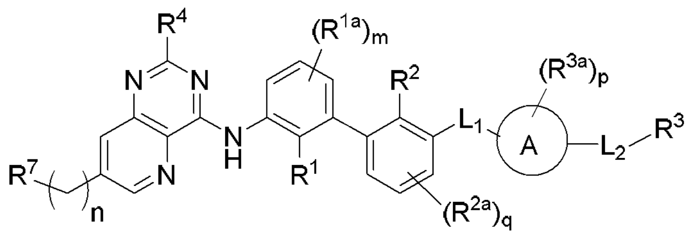

(I)

wherein:

$L_1$ is selected from a bond, $-NR^z-$, -O-, $-(CH_2)_t-$, -HC=CH-, -S- or $-SO_2-$;
$R^Z$ is H, D, -OH, $C_{1-6}$ alkyl, $C_{2-6}$ alkenyl, $C_{2-6}$ alkynyl, $C_{1-6}$ haloalkyl, $C_{1-6}$ alkoxy or $C_{1-6}$ haloalkoxy;
$R^1$, $R^2$, $R^{1a}$ and $R^{2a}$ are each independently H, D, F, Cl, Br, I, $-NO_2$, -CN, -OH, $-NH_2$, $C_{1-6}$ alkyl or $C_{1-6}$ haloalkyl;
Ring A is selected from $C_{3-8}$ cycloalkyl, heterocyclic consisting of 3-8 atoms, $C_{6-10}$ aryl, or heteroaryl consisting of 5-6 atoms;
each $R^{3a}$ is independently H, D, F, Cl, Br, I, $-NO_2$, -CN, -OH, $-NH_2$, $C_{1-6}$ alkyl, $C_{2-6}$ alkenyl, $C_{2-6}$ alkynyl, $C_{1-6}$haloalkyl, $C_{1-6}$ alkoxy or $C_{1-6}$ haloalkoxy, wherein the $C_{1-6}$ alkyl, $C_{2-6}$ alkenyl, $C_{2-6}$ alkynyl and $C_{1-6}$ alkoxy are independently and optionally substituted with 1, 2, 3 or 4 substituents selected from D, F, Cl, Br, I, $C_{1-6}$ alkyl, $C_{2-6}$ alkenyl, $C_{2-6}$ alkynyl, oxo, $-NO_2$, -CN, -OH, $-NH_2$, $-COOCH_3$ and -COOH;
$R^4$ is H, D, F, Cl, Br, I, $-NO_2$, -CN, -OH, $-NH_2$, $C_{1-6}$ alkyl, $C_{1-6}$ haloalkyl or $C_{3-8}$ cycloalkyl, wherein the $C_{1-6}$ alkyl and $C_{3-8}$ cycloalkyl are independently and optionally substituted with 1, 2, 3 or 4 substituents selected from D, F, Cl, Br, I, $C_{1-6}$alkyl, $C_{1-6}$ haloalkyl, oxo, $-NO_2$, -CN, -OH, $-NH_2$, $-COOCH_3$ and -COOH;
$L_2$ is a bond, $-C_{1-6}$ alkylene- or $-C_{1-6}$ alkylene-$NR^w$-$C_{1-6}$ alkylene-, wherein the each $C_{1-6}$ alkylene is independently and optionally substituted with 1, 2, 3 or 4 substituents selected from D, F, Cl, Br, I, oxo, -OH, $C_{1-6}$ alkyl and $C_{1-6}$ haloalkyl;
$R^w$ is H, D, -OH, $C_{1-6}$ alkyl, $C_{1-6}$ haloalkyl, $C_{2-6}$ alkenyl or $C_{2-6}$ alkynyl, wherein the $C_{1-6}$ alkyl, $C_{1-6}$ haloalkyl, $C_{2-6}$ alkenyl and $C_{2-6}$ alkynyl are independently and optionally substituted with 1, 2, 3 or 4 substituents selected from D, F, Cl, Br, I, oxo, -OH, $C_{1-6}$ alkyl and $C_{1-6}$ haloalkyl;
$R^3$ is $-NR^5R^6$, $C_{3-12}$ cycloalkyl or heterocyclic consisting of 3-12 atoms, wherein the $C_{3-12}$ cycloalkyl and of heterocyclic consisting of 3-12 atoms are independently and optionally substituted with 1, 2, 3 or 4 substituents selected from D, halogen, $-NO_2$, -CN, -OH, $-NH_2$, oxo, $C_{1-6}$ alkyl, $-C(O)CH_3$, -C(O)OH, $-C_{1-4}$ alkylene C(O)OH, $-C(O)OCH_3$, -NHC(O)-$C_{1-4}$ alkyl, $-NHC(O)OCH_3$, $-C(O)NH_2$, $-S(O)_2CH_3$, $-S(O)_2NH_2$ and $-C(O)NHS(O)_2CH_3$;
$R^5$ and $R^6$ are each independently H, D, $C_{1-6}$ alkyl, $C_{3-10}$ cycloalkyl or heterocyclic consisting of 3-12 atoms, wherein the $C_{1-6}$ alkyl, $C_{3-10}$ cycloalkyl and heterocyclic consisting of 3-12 atoms, are independently and optionally substituted with 1, 2, 3 or 4 substituents selected from D, F, Cl, Br, I, $-NO_2$, -CN, -OH, $-NH_2$, oxo, $C_{1-6}$ alkyl, $C_{1-6}$ haloalkyl, hydroxy $C_{1-6}$ alkyl, amino $C_{1-6}$ alkyl, carboxy $C_{1-6}$ alkyl, $-OR^a$, $-C(O)R^a$, $-C(O)OR^a$,

$-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$; or

$R^5$ and $R^6$ together with the atom to which they are attached form heterocyclic consisting of 3-12 atoms, wherein heterocyclic consisting of 3-12 atoms optionally contains 1, 2 or 3 heteroatoms independently selected from oxygen, sulfur, or nitrogen, and is optionally substituted with 1, 2, 3 or 4 substituents independently selected from D, F, Cl, Br, I, $-NO_2$, -CN, oxo, $C_{1-6}$ alkyl, $C_{1-6}$haloalkyl, hydroxy $C_{1-6}$ alkyl, amino $C_{1-6}$ alkyl, carboxy $C_{1-6}$ alkyl, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$;

each of $R^a$, $R^b$, $R^c$ and $R^d$ is independently H, D, $C_{1-6}$ alkyl, $C_{3-8}$ cycloalkyl or heterocyclic consisting of 3-8 atoms, wherein the $C_{1-6}$ alkyl, $C_{3-8}$ cycloalkyl and heterocyclic consisting of 3-8 atoms are independently and optionally substituted with 1, 2, 3 or 4 substituents selected from D, F, Cl, Br, I, $C_{1-6}$ alkyl, $-NO_2$, -CN, -OH, $-NH_2$, $-C(O)CH_3$, -C(O)OH, $-C(O)OCH_3$, $-NHC(O)CH_3$, $-NHC(O)OCH_3$, $-C(O)NH_2$, $-S(O)_2CH_3$ and $-S(O)_2NH_2$;

$R^7$ is $C_{3-10}$ cycloalkyl or heterocyclic consisting of 3-12 atoms, wherein the $C_{3-10}$ cycloalkyl and of heterocyclic consisting of 3-12 atoms are independently and optionally substituted with 1, 2, 3 or 4 substituents selected from D, F, Cl, Br, I, $-NO_2$, -CN, -OH, $-NH_2$, oxo, $C_{1-6}$ alkyl, $C_{1-6}$ haloalkyl, $C_{1-6}$hydroxy alkyl, $-OR^e$, $-C(O)R^e$, $-C(O)OR^e$, $-NR^eR^f$, $-NR^eC(O)R^f$, $-NR^eC(O)OR^f$ and $-C(O)NR^eR^f$;

each of $R^e$ and $R^f$ is H, D, $C_{1-6}$ alkyl or $C_{3-8}$ cycloalkyl, wherein the $C_{1-6}$ alkyl and $C_{3-8}$ cycloalkyl are independently and optionally substituted with 1, 2, 3 or 4 substituents selected from D, F, Cl, Br, I, oxo, $-NO_2$, -CN, -OH, $-NH_2$, $-C(O)CH_3$, $-COOCH_3$ and -COOH;

each of m, n, q, p and t is independently 0, 1, 2 or 3.

**2.** The compound of claim 1, wherein:

$R^1$, $R^2$, $R^{1a}$ and $R^{2a}$ are each independently H, D, F, Cl, Br, I, $-NO_2$, -CN, -OH, $-NH_2$, $C_{1-4}$ alkyl or $C_{1-4}$ haloalkyl;

each $R^{3a}$ is independently H, D, F, Cl, Br, I, $-NO_2$, -CN, -OH, $-NH_2$, $C_{1-4}$ alkyl, $C_{2-4}$ alkenyl, $C_{2-4}$ alkynyl, $C_{1-4}$ haloalkyl, $C_{1-4}$ alkoxy or $C_{1-4}$ haloalkoxy, wherein the $C_{1-4}$ alkyl, $C_{2-4}$ alkenyl, $C_{2-4}$ alkynyl and $C_{1-4}$ alkoxy are independently and optionally substituted with 1, 2, 3 or 4 substituents selected from D, F, Cl, Br, I, $C_{1-4}$ alkyl, $C_{2-4}$ alkenyl, $C_{2-4}$ alkynyl, oxo, $-NO_2$, -CN, -OH, $-NH_2$, $-COOCH_3$ and -COOH;

$R^4$ is H, D, F, Cl, Br, I, $-NO_2$, -CN, -OH, $-NH_2$, $C_{1-4}$ alkyl, $C_{1-4}$ haloalkyl or $C_{3-6}$ cycloalkyl, wherein the $C_{1-4}$ alkyl and $C_{3-6}$ cycloalkyl are independently and optionally substituted with 1, 2, 3 or 4 substituents selected from D, F, Cl, Br, I, $C_{1-4}$ alkyl, $C_{1-4}$ haloalkyl, oxo, $-NO_2$, -CN, -OH, $-NH_2$, $-COOCH_3$ and -COOH.

**3.** The compound of claim 1 or 2, wherein:

$R^1$, $R^2$, $R^{1a}$ and $R^{2a}$ are each independently H, D, F, Cl, Br, I, $-NO_2$, -CN, -OH, $-NH_2$, methyl, ethyl, *n*-propyl, isopropyl, *n*-butyl, isobutyl, *sec-butyl, tert-butyl,* $-CH_2F$, $-CHF_2$, $-CF_3$, $-CH_2CHF_2$, $-CHFCH_2F$, $-CH_2CF_3$, $-CH_2Cl$, $-CHCl_2$, $-CH_2CHCl_2$, $-CH_2Br$, $-CHBr_2$ or $-CH_2CHBr_2$;

each of $R^{3a}$ is independently H, D, F, Cl, Br, I, $-NO_2$, -CN, -OH, $-NH_2$, methyl, ethyl, *n*-propyl, isopropyl, *n*-butyl, isobutyl, *sec-butyl, tert-butyl,* vinyl, propenyl, allyl, ethynyl, propargyl, 1-propynyl, 1-butynyl, 2-butynyl, 3-butynyl, $-CH_2F$, $-CHF_2$, $-CF_3$, $-CH_2CHF_2$, $-CHFCH_2F$, $-CH_2CF_3$, $-OCH_3$, $-OCH_2CH_3$, $-OCH_2CH_2CH_3$, $-OCH(CH_3)_2$, $-OCHF_2$, $-OCF_3$, $-OCH_2CHF_2$ or $-OCH_2CF_3$, wherein the methyl, ethyl, *n*-propyl, isopropyl, *n*-butyl, isobutyl, *sec*-butyl, *tert*-butyl, vinyl, propenyl, allyl, ethynyl, propargyl, 1-propynyl, 1-butynyl, 2-butynyl, 3-butynyl, $-OCH_3$, $-OCH_2CH_3$, $-OCH_2CH_2CH_3$ and $-OCH(CH_3)_2$ are independently and optionally substituted with 1, 2, 3 or 4 substituents selected from D, F, Cl, Br, I, methyl, ethyl, *n*-propyl, isopropyl, vinyl, propenyl, allyl, ethynyl, propargyl, 1-propynyl, oxo, $-NO_2$, -CN, -OH, $-NH_2$, $-COOCH_3$ and -COOH;

$R^4$ is H, D, F, Cl, Br, I, $-NO_2$, -CN, -OH, $-NH_2$, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, cyclopropyl, cyclobutyl, cyclopentyl or cyclohexyl, wherein the methyl, ethyl, *n*-propyl, isopropyl, *n*-butyl, isobutyl, *sec*-butyl, *tert*-butyl, cyclopropyl, cyclobutyl, cyclopentyl and cyclohexyl are independently and optionally substituted with 1, 2, 3 or 4 substituents selected from D, F, Cl, Br, I, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, oxo, $-NO_2$, -CN, -OH, $-NH_2$, $-COOCH_3$ and -COOH.

**4.** The compound of any one of claims 1-3, wherein:
ring A is selected from cyclopropyl, cyclobutyl, cyclopentyl, cyclohexyl, oxetanyl, azetidinyl, pyrrolidinyl, tetrahydrofuranyl, piperidinyl, piperazinyl, morpholinyl, phenyl, naphthyl, pyrrolyl, pyrazolyl, imidazolyl, triazolyl, tetrazolyl, furyl, thienyl, thiazolyl, oxazolyl, pyridinyl, pyrimidyl, pyrazinyl or pyridazinyl.

**5.** The compound of any one of claims 1-4, wherein:

$R^3$ is $-NR^5R^6$, $C_{3-10}$ cycloalkyl or heterocyclic consisting of 3-10 atoms, wherein the $C_{3-10}$ cycloalkyl and heterocyclic consisting of 3-10 atoms are independently optionally substituted with 1, 2, 3 or 4 substituents selected from D, halogen, $-NO_2$, -CN, -OH, $-NH_2$, oxo, $C_{1-4}$ alkyl, $-C(O)CH_3$, -C(O)OH, $-C_{1-4}$ alkylene C(O)OH, $-C(O)OCH_3$, -NHC(O)-$C_{1-4}$ alkyl, $-NHC(O)OCH_3$, $-C(O)NH_2$, $-S(O)_2CH_3$, $-S(O)_2NH_2$ and $-C(O)NHS(O)_2CH_3$;
$R^5$ and $R^6$ are each independently H, D, $C_{1-4}$ alkyl, $C_{3-8}$ cycloalkyl or heterocyclic consisting of 3-10 atoms, wherein the $C_{1-4}$ alkyl, $C_{3-8}$ cycloalkyl and of heterocyclic consisting of 3-10 atoms are independently and optionally substituted with 1, 2, 3 or 4 substituents selected from D, F, Cl, Br, I, $-NO_2$, -CN, -OH, $-NH_2$, oxo, $C_{1-4}$ alkyl, $C_{1-4}$ haloalkyl, hydroxy $C_{1-4}$ alkyl, amino $C_{1-4}$ alkyl, carboxy $C_{1-4}$ alkyl, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$; or
$R^5$ and $R^6$ together with the atom to which they are attached form heterocyclic consisting of 3-10 atoms, wherein the heterocyclic consisting of 3-10 atoms optionally contains 1, 2 or 3 heteroatoms independently selected from oxygen, sulfur, or nitrogen, and is optionally substituted with 1, 2, 3 or 4 substituents independently selected from D, F, Cl, Br, I, $-NO_2$, -CN, oxo, $C_{1-4}$ alkyl, $C_{1-4}$ haloalkyl, hydroxy $C_{1-4}$ alkyl, amino $C_{1-4}$ alkyl, carboxy $C_{1-4}$ alkyl, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$;
each of $R^a$, $R^b$, $R^c$ and $R^d$ is independently H, D, $C_{1-4}$ alkyl, $C_{3-6}$ cycloalkyl or heterocyclic consisting of 3-6 atoms, wherein the $C_{1-4}$ alkyl, $C_{3-6}$ cycloalkyl and heterocyclic consisting of 3-6 atoms are independently optionally substituted with 1, 2, 3 or 4 substituents selected from D, F, Cl, Br, I, $C_{1-4}$ alkyl, $-NO_2$, -CN, -OH, $-NH_2$, $-C(O)CH_3$, -C(O)OH, $-C(O)OCH_3$, $-NHC(O)CH_3$, $-NHC(O)OCH_3$, $-C(O)NH_2$, $-S(O)_2CH_3$ and $-S(O)_2NH_2$.

**6.** The compound of any one of claims 1-5, wherein:

$R^3$ is $-NR^5R^6$, cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, 2-oxa-5-azabicyclo[2.2.1]heptan-5-yl, 2-azabicyclo[2.2.1]heptyl, 2-azabicyclo[2.2.2]octyl, 3-azabicyclo[3.1.0]hexyl, 2,6-diazaspiro[3.3]heptyl, 2-oxa-6-azaspiro[3.3]heptyl, 2,6-diazaspiro[3.4]octyl, 1,7-diazaspiro[4.4]nonyl, azetidinyl, pyrrolidyl, tetrahydrofuranyl, piperidinyl, piperazinyl or morpholinyl, wherein the cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, 2-oxa-5-azabicyclo[2.2.1]heptan-5-yl, 2-azabicyclo[2.2.1]heptyl, 2-azabicyclo[2.2.2]octyl, 3-azabicyclo[3.1.0]hexyl, 2,6-diazaspiro[3.3]heptyl, 2-oxa-6-azaspiro[3.3]heptyl, 2,6-diazaspiro[3.4]octyl, 1,7-diazaspiro[4.4]nonyl, azetidinyl, pyrrolidyl, tetrahydrofuranyl, piperidinyl, piperazinyl and morpholinyl are independently and optionally substituted with 1, 2, 3 or 4 substituents selected from D, F, Cl, Br, I, $-NO_2$, -CN, -OH, $-NH_2$, oxo, methyl, ethyl, *n*-propyl, isopropyl, *n*-butyl, isobutyl, *sec*-butyl, *tert*-butyl, $-C(O)CH_3$, -C(O)OH, -methylene C(O)OH, $-C(O)OCH_3$, $-NHC(O)CH_3$, $-NHC(O)OCH_3$, $-C(O)NH_2$, $-S(O)_2CH_3$, $-S(O)_2NH_2$ and $-C(O)NHS(O)_2CH_3$;
each of $R^5$ and $R^6$ is independently H, D, methyl, ethyl, *n*-propyl, isopropyl, *n*-butyl, isobutyl, *sec*-butyl, *tert*-butyl, cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, azetidinyl, pyrrolidyl, tetrahydrofuranyl, piperidinyl, piperazinyl or morpholinyl, wherein the methyl, ethyl, *n*-propyl, isopropyl, *n*-butyl, isobutyl, *sec-butyl, tert-butyl,* cyclopropyl, cyclobutyl, cyclopentyl, cyclohexyl, bicyclo[1.1.1]pentyl, bicyclo[2.1.1]hexyl, bicyclo[2.2.1]heptyl, bicyclo[2.2.2]octyl, azetidinyl, pyrrolidyl, tetrahydrofuranyl, piperidinyl, piperazinyl and morpholinyl are independently and optionally substituted with 1, 2, 3 or 4 substituents selected from D, F, Cl, Br, I, $-NO_2$, -CN, -OH, $-NH_2$, oxo, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, $-CH_2OH$, $-CH_2CH_2OH$, $-CH_2NH_2$, $-CH_2CH_2NH_2$, $-CH_2COOH$, $-CH_2CH_2COOH$, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^6$ and $-C(O)NR^aS(O)_2R^b$; or
$R^5$ and $R^6$ together with the atoms to which they are attached form a structure selected from the following:

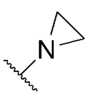, 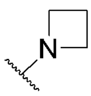, 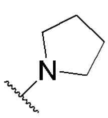, 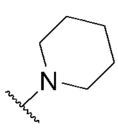, 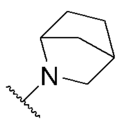, 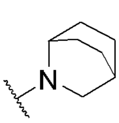, 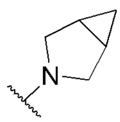, 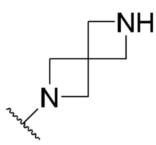,

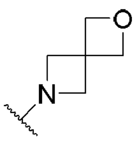, 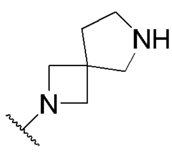 or 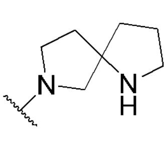,

wheiein the

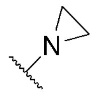, 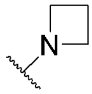, 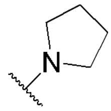, 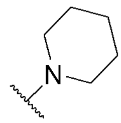,

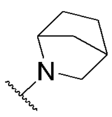, 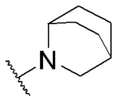, 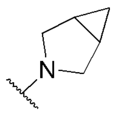, 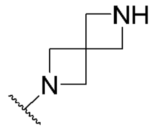, 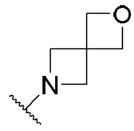, 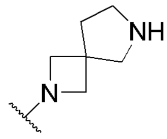 and 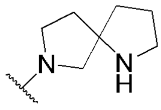

are independently and optionally substituted with 1, 2, 3 or 4 substituents selected from D, F, Cl, Br, I, $-NO_2$, -CN, oxo, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, $-CH_2OH$, $-CH_2CH_2OH$, $-CH_2NH_2$, $-CH_2CH_2NH_2$, $-CH_2COOH$, $-CH_2CH_2COOH$, $-OR^a$, $-C(O)R^a$, $-C(O)OR^a$, $-NR^aR^b$, $-NR^cC(O)R^d$, $-NR^aC(O)OR^b$, $-C(O)NR^aR^b$, $-S(O)_2R^a$, $-S(O)_2NR^aR^b$, $-NR^aS(O)_2R^b$ and $-C(O)NR^aS(O)_2R^b$;

each of $R^a$, $R^b$, $R^c$ and $R^d$ is independently H, D, methyl, ethyl, *n*-propyl, isopropyl, *n*-butyl, isobutyl, *sec*-butyl, *tert*-butyl, cyclopropyl, cyclobutyl, cyclopentyl, cyclohexyl, azetidinyl, pyrrolidyl, tetrahydrofuranyl, piperidinyl, piperazinyl or morpholinyl, wherein the methyl, ethyl, *n*-propyl, isopropyl, *n*-butyl, isobutyl, *sec*-butyl, *tert*-butyl, cyclopropyl, cyclobutyl, cyclopentyl, cyclohexyl, azetidinyl, pyrrolidyl, tetrahydrofuranyl, piperidinyl, piperazinyl and morpholinyl are independently and optionally substituted with 1, 2, 3 or 4 substituents selected from D, F, Cl, Br, I, methyl, ethyl, *n*-propyl, isopropyl, *n*-butyl, isobutyl, *sec*-butyl, *tert*-butyl, $-NO_2$, -CN, -OH, $-NH_2$, $-C(O)CH_3$, -C(O)OH, $-C(O)OCH_3$, $-NHC(O)CH_3$, $-NHC(O)OCH_3$, $-C(O)NH_2$, $-S(O)_2CH_3$ and $-S(O)_2NH_2$.

**7.** The compound of any one of claims 1-6, wherein:

$L_2$ is a bond, $-C_{1-3}$ alkylene- or $-C_{1-3}$ alkylene-$NR^w$-$C_{1-3}$ alkylene-, wherein the each $-C_{1-3}$ alkylene is independently and optionally substituted with 1, 2, 3 or 4 substituents selected from D, F, Cl, Br, I, oxo, -OH, $C_{1-4}$ alkyl and $C_{1-4}$ haloalkyl;

$R^w$ is H, D, -OH, $C_{1-4}$ alkyl, $C_{1-4}$ haloalkyl, $C_{2-4}$ alkenyl or $C_{2-4}$ alkynyl, wherein the $C_{1-4}$ alkyl, $C_{1-4}$ haloalkyl, $C_{2-4}$ alkenyl and $C_{2-4}$ alkynyl are independently and optionally substituted with 1, 2, 3 or 4 substituents selected from D, F, Cl, Br, I, oxo, -OH, $C_{1-4}$ alkyl and $C_{1-4}$ haloalkyl;

$R^z$ is H, D, -OH, $C_{1-4}$ alkyl, $C_{2-4}$ alkenyl, $C_{2-4}$ alkynyl, $C_{1-4}$ haloalkyl, $C_{1-4}$ alkoxy or $C_{1-4}$ haloalkoxy;

$R^7$ is $C_{3-8}$ cycloalkyl or heterocyclic consisting of 3-10 atoms, wherein the $C_{3-8}$ cycloalkyl and of heterocyclic consisting of 3-10 atoms are independently and optionally substituted with 1, 2, 3 or 4 substituents selected from D, F, Cl, Br, I, $-NO_2$, -CN, -OH, $-NH_2$, oxo, $C_{1-4}$ alkyl, $C_{1-4}$ haloalkyl, $C_{1-4}$ hydroxy alkyl, $-OR^e$, $-C(O)R^e$, $-C(O)OR^e$, $-NR^eR^f$, $-NR^eC(O)R^f$, $-NR^eC(O)OR^f$ and $-C(O)NR^eR^f$;

each of $R^e$ and $R^f$ is H, D, $C_{1-4}$ alkyl or $C_{3-6}$ cycloalkyl, wherein the $C_{1-4}$ alkyl and $C_{3-6}$ cycloalkyl are independently and optionally substituted with 1, 2, 3 or 4 substituents selected from D, F, Cl, Br, I, oxo, $-NO_2$, -CN, -OH, $-NH_2$, $-C(O)CH_3$, $-COOCH_3$ and -COOH.

**8.** The compound of any one of claims 1-7, wherein:

$L_2$ is a bond, -methylene-, -ethylene-, -methylene-$NR^w$-methylene-, or -ethylene-$NR^w$-ethylene-, wherein the -methylene- and -ethylene- are independently and optionally substituted with 1, 2, 3 or 4 substituents selected from D, F, Cl, Br, I, oxo, -OH, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$ and $-CH_2CF_3$;

$R^w$ is H, D, -OH, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, vinyl, propenyl, allyl, ethynyl, propargyl, 1-propynyl, 1-butynyl, 2-butynyl or 3-butynyl, wherein the methyl, ethyl, *n*-propyl, isopropyl, *n*-butyl, isobutyl, *sec*-butyl, *tert*-butyl, $-CH_2F$, $-CHF_2$, $-CH_2CHF_2$, $-CHFCH_2F$, $-CH_2CF_3$, vinyl, propenyl, allyl, ethynyl, propargyl, 1-propynyl, 1-butynyl, 2-butynyl and 3-butynyl are independently and optionally substituted with 1, 2, 3 or 4 substituents selected from D, F, Cl, Br, I, oxo, -OH, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$ and $-CH_2CF_3$;

$R^z$ is H, D, -OH, methyl, ethyl, *n*-propyl, isopropyl, *n*-butyl, isobutyl, *sec*-butyl, *tert*-butyl, vinyl, propenyl, allyl,

ethynyl, propargyl, 1-propynyl, 1-butynyl, 2-butynyl, 3-butynyl, $-CH_2F$, $-CHF_2$, $-CF_3$, $-CH_2CHF_2$, $-CHFCH_2F$, $-CH_2CF_3$, $-OCH_3$, $-OCH_2CH_3$, $-OCH_2CH_2CH_3$, $-OCH(CH_3)_2$, $-OCHF_2$, $-OCF_3$, $-OCH_2CHF_2$ or $-OCH_2CF_3$;
$R^7$ is cyclopropyl, cyclobutyl, cyclopentyl, cyclohexyl, cycloheptyl, cyclooctyl, bicyclo[1.1.1]pentyl, bicyclo[2.1.1]hexyl, bicyclo[2.2.1]heptyl, bicyclo[2.2.2]octyl, azetidinyl, oxetanyl, pyrrolidinyl, tetrahydrofuranyl, tetrahydrothiophenyl, thiazolidinyl, pyrazolidinyl, oxazolidinyl, imidazolidinyl, isoxazolidinyl, piperidinyl, piperazinyl or morpholinyl, wherein the cyclopropyl, cyclobutyl, cyclopentyl, cyclohexyl, cycloheptyl, cyclooctyl, bicyclo[1.1.1]pentyl, bicyclo[2.1.1]hexyl, bicyclo[2.2.1]heptyl, bicyclo[2.2.2]octyl, azetidinyl, oxetanyl, pyrrolidinyl, tetrahydrofuranyl, tetrahydrothiophenyl, thiazolidinyl, pyrazolidinyl, oxazolidinyl, imidazolidinyl, isoxazolidinyl, piperidinyl, piperazinyl and morpholinyl are independently and optionally substituted with 1, 2, 3 or 4 substituents selected from D, F, Cl, Br, I, $-NO_2$, -CN, -OH, $-NH_2$, oxo, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, $-CH_2OH$, $-CH_2CH_2OH$, $-CH(OH)CH_3$, $-OR^e$, $-C(O)R^e$, $-C(O)OR^e$, $-NR^eR^f$, $-NR^eC(O)R^f$, $-NR^eC(O)OR^f$ and $-C(O)NR^eR^f$;
each of $R^e$ and $R^f$ is H, D, methyl, ethyl, *n*-propyl, isopropyl, *n*-butyl, isobutyl, *sec*-butyl, *tert*-butyl, cyclopropyl, cyclobutyl, cyclopentyl or cyclohexyl, wherein the methyl, ethyl, *n*-propyl, isopropyl, *n*-butyl, isobutyl, *sec*-butyl, *tert*-butyl, cyclopropyl, cyclobutyl, cyclopentyl and cyclohexyl are independently and optionally substituted with 1, 2, 3 or 4 substituents selected from D, F, Cl, Br, I, oxo, $-NO_2$, -CN, -OH, $-NH_2$, $-C(O)CH_3$, $-COOCH_3$ and -COOH.

**9.** The compound of any one of claims 1-8, which is a compound of Formula (II) or a stereoisomer, a geometric isomer, a tautomer, an *N*-oxide, a solvate, a metabolite, a pharmaceutically acceptable salt or a prodrug thereof,

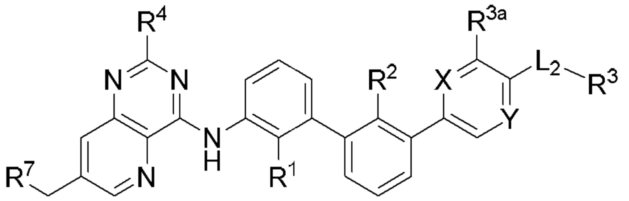
(II)

wherein, X is $CR^x$ or N;
Y is $CR^y$ or N;
$R^x$ and $R^y$ are independently H, D, F, Cl, Br, I, $-NO_2$, -CN, -OH, $-NH_2$, $C_{1-6}$ alkyl, $C_{1-6}$ haloalkyl, $C_{1-6}$ alkoxy or $C_{1-6}$ haloalkoxy.

**10.** The compound of claim 9, wherein:
$R^x$ and $R^y$ are each independently H, D, F, Cl, Br, I, $-NO_2$, -CN, -OH, $-NH_2$, methyl, ethyl, *n*-propyl, isopropyl, *n*-butyl, isobutyl, *sec*-butyl, *tert*-butyl, $-CH_2F$, $-CHF_2$, $-CF_3$, $-CH_2CHF_2$, $-CHFCH_2F$, $-CH_2CF_3$, $-OCH_3$, $-OCH_2CH_3$, $-OCH_2CH_2CH_3$, $-OCH(CH_3)_2$, $-OCHF_2$, $-OCF_3$, $-OCH_2CHF_2$ or $-OCH_2CF_3$.

**11.** The compound of any one of claims 1-10, which is a stereoisomer, a tautomer, an N-oxide, a hydrate, a solvate, a metabolite, a pharmaceutically acceptable salt, or a prodrug having one of the following structures:

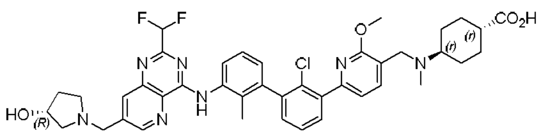
(1),
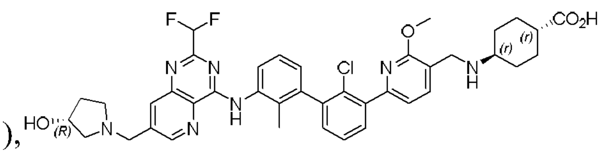
(2),
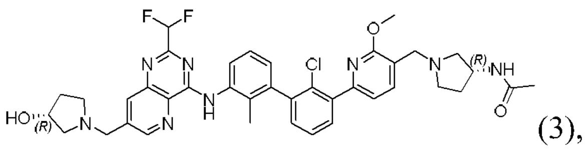
(3),
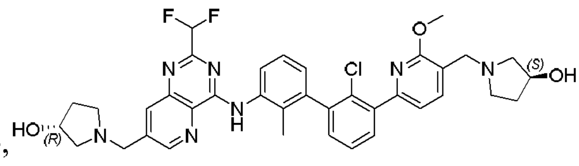
(4),

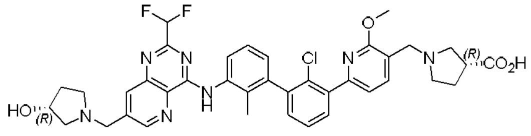 (5), 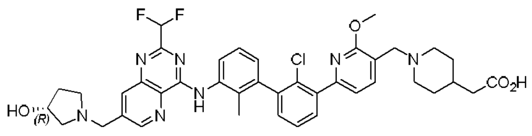 (6),

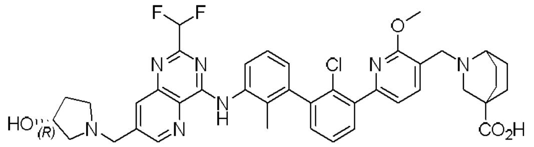 (7), 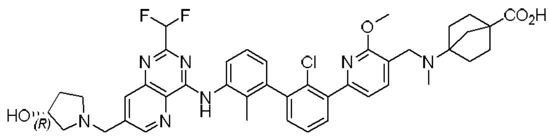 (8),

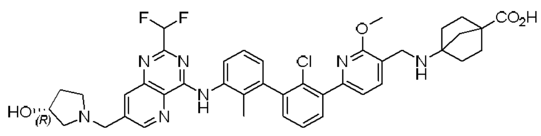 (9), 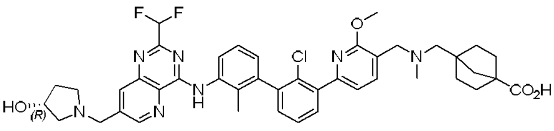 (10),

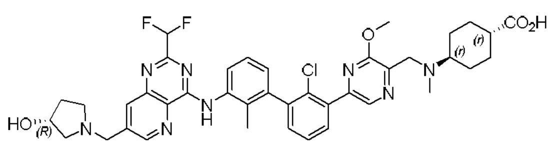 (11), 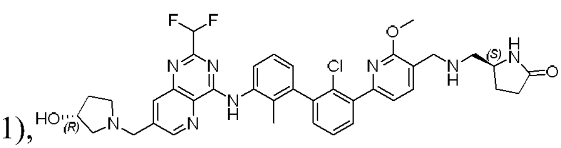 (12),

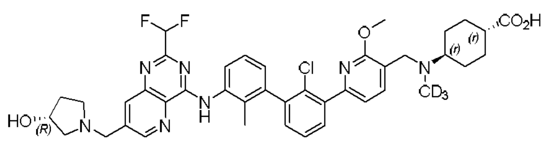 (13), 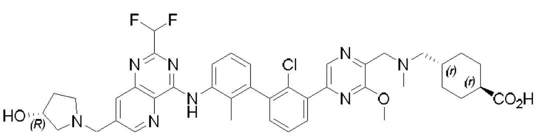 (14),

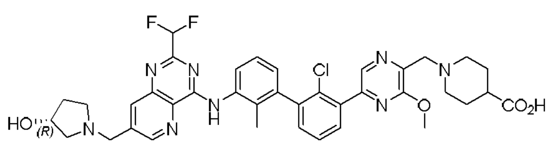 (15), 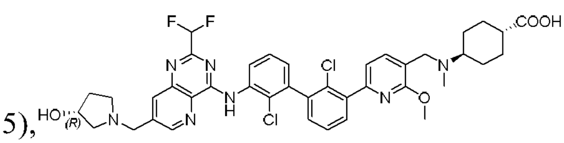 (16),

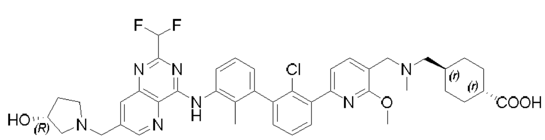 (17), 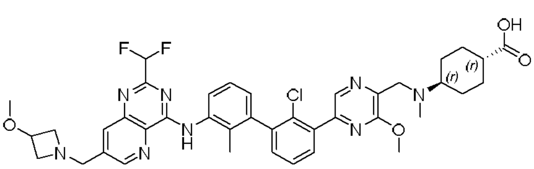 (18),

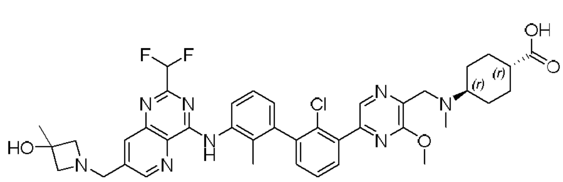 (19), 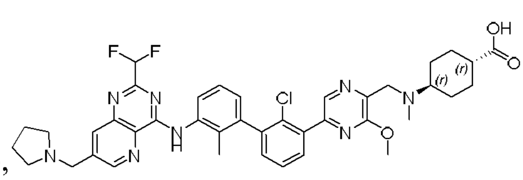 (20),

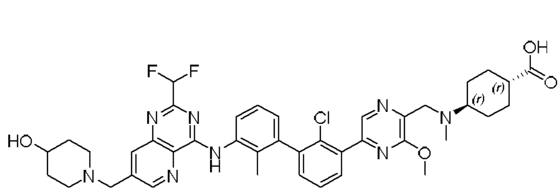 (21), 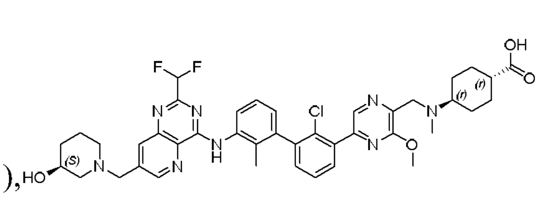 (22),

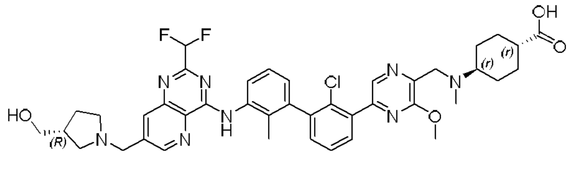
(23),
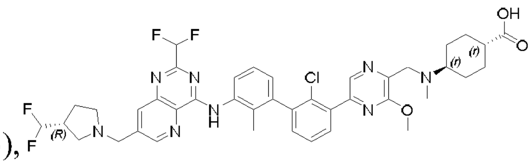
(24),

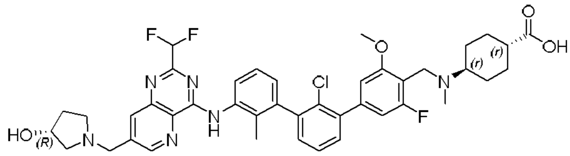
(25),
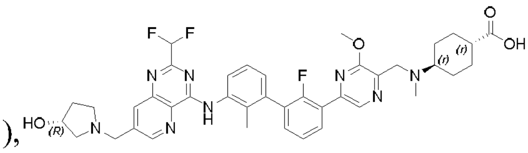
(26),

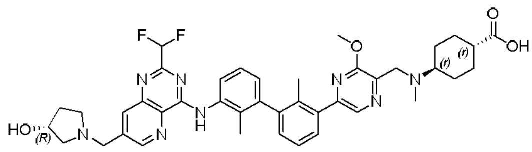
(27),
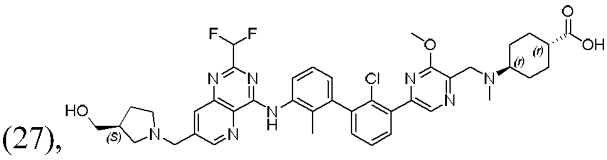
(28),

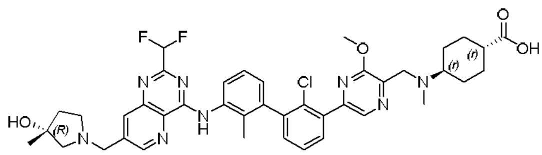
(29),
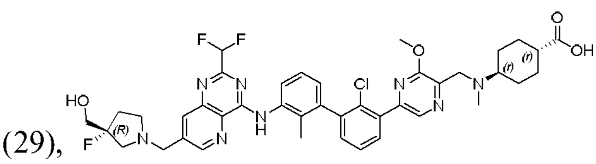
(30),

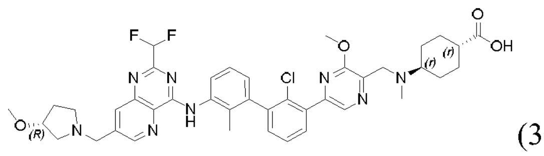
(31),
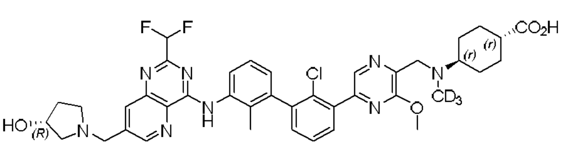
(32),

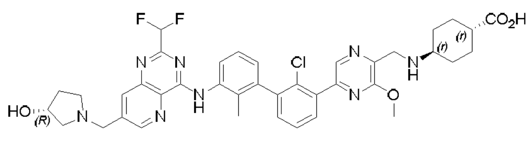
(33),
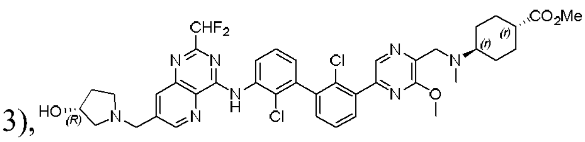
(34),

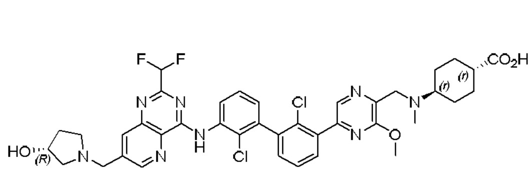
(35),
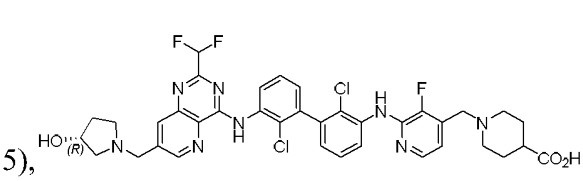
(36),

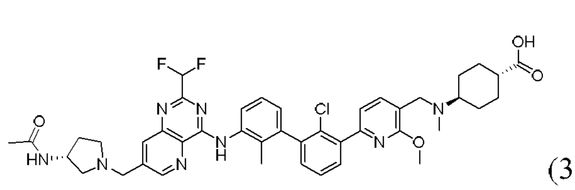
(37),
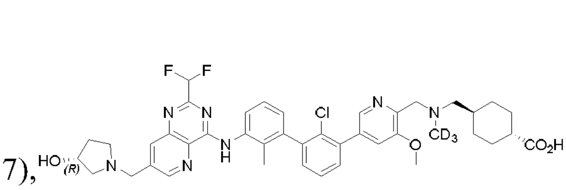
(38),

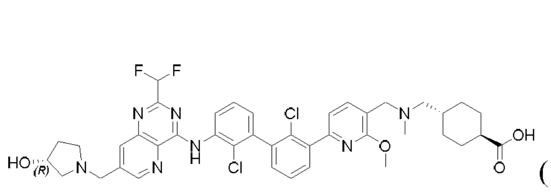
(39),
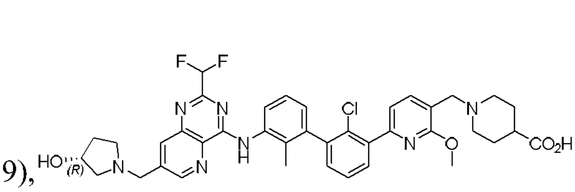
(40),

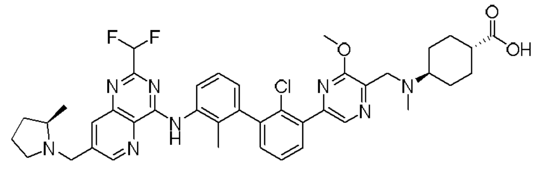
(41),
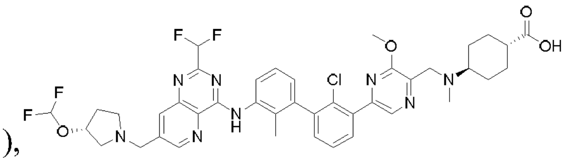
(42),

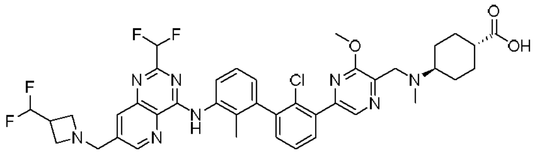
(43),
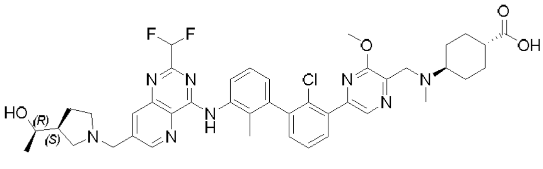
(44),

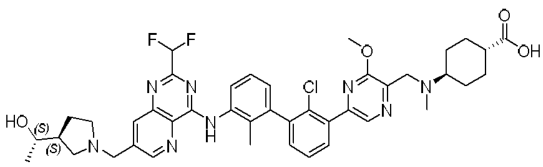
(45),
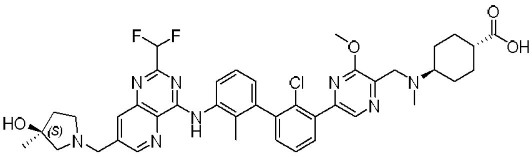
(46),

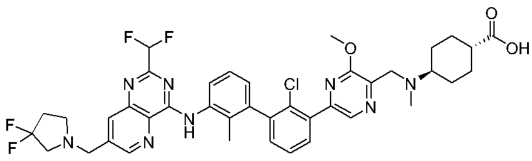
(47),
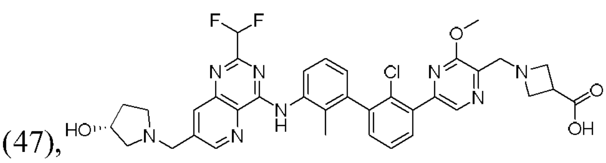
(48),

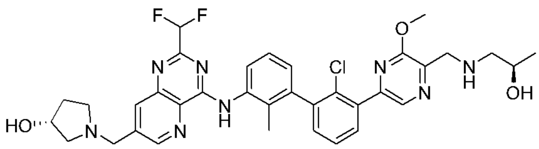
(49),
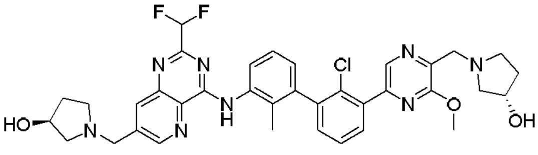
(50),

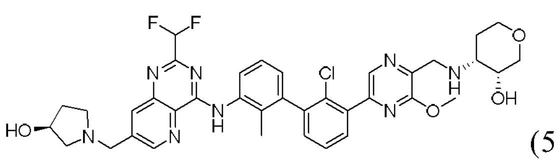
(51),
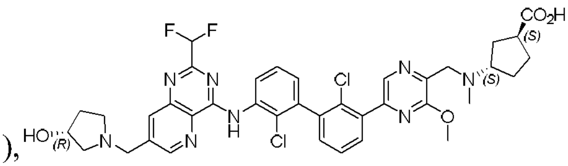
(52),

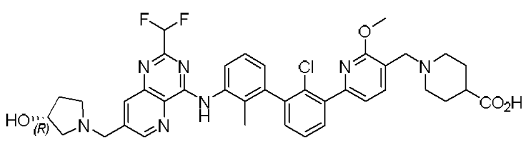
(53),
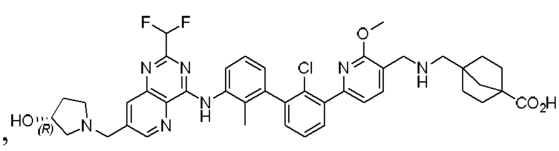
(54),

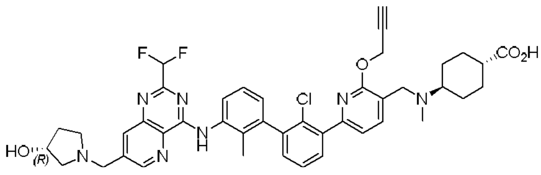
(55),
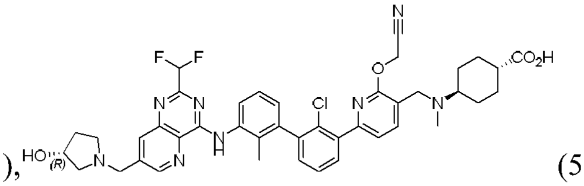
(56),

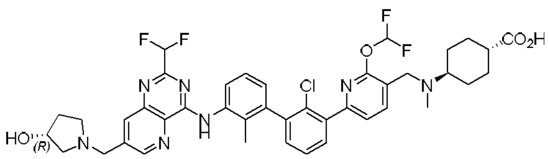 (57), 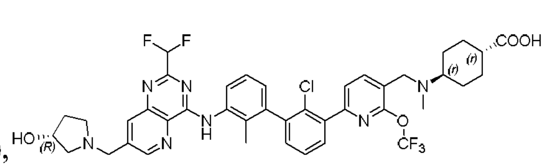 (58)

or 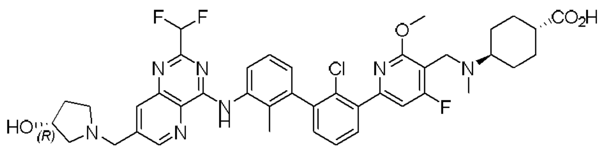 (59).

**12.** A pharmaceutical composition comprising the compound of any one of claims 1 to 11; the pharmaceutical composition further comprising pharmaceutically acceptable carriers, excipients, diluents, adjuvant, vehicles and combinations thereof.

**13.** Use of the compound of any one of claims 1-11 or the pharmaceutical composition of claim 12 in the manufacture a medicament for treating a disease mediated by the PD-1/PD-L1 signaling pathway.

**14.** The use of claim 13, wherein the disease mediated by the PD-1/PD-L1 signaling pathway disclosed herein is cancer, infectious disease, or autoimmune disease.

**15.** The use of claim 13, wherein the cancer is selected from acute lymphoblastic leukemia, acute myeloid leukemia, chronic lymphocytic leukemia, chronic myelogenous leukemia, multiple myeloma, T-cell lymphoma, B-cell lymphoma, Waldenström's macroglobulinemia, pancreatic cancer, bladder cancer, colorectal cancer, breast cancer, prostate cancer, kidney cancer, liver cancer, lung cancer, ovarian cancer, cervical cancer, gastric cancer, esophageal cancer, head and neck cancer, melanoma, neuroendocrine cancer, CNS cancer, brain cancer, or bone cancer;

the infectious disease is selected from HIV, hepatitis A, hepatitis B, hepatitis C, hepatitis D, herpes virus infection, papillomavirus infection, or influenza virus infection;
the autoimmune disease is selected from Chronic lymphocytic thyroiditis, hyperthyroidism, insulin-dependent diabetes mellitus, myasthenia gravis, ulcerative colitis, pernicious anemia with chronic atrophic gastritis, Goodpasture's syndrome, primary biliary cholangitis, multiple sclerosis, acute idiopathic polyneuritis, rheumatoid arthritis, systemic lupus erythematosus, systemic vasculitis, scleroderma, pemphigus, dermatomyositis, mixed connective tissue disease, or autoimmune hemolytic anemia.

**INTERNATIONAL SEARCH REPORT**

International application No. **PCT/CN2024/077994**

**A. CLASSIFICATION OF SUBJECT MATTER**

C07D 471/04(2006.01)i; A61K 31/519(2006.01)i; A61P 35/00(2006.01)i; A61P 35/02(2006.01)i; A61P 31/14(2006.01)i; A61P 31/16(2006.01)i; A61P 31/18(2006.01)i; A61P 31/20(2006.01)i; A61P 31/22(2006.01)i; A61P 19/00(2006.01)i; A61P 37/00(2006.01)i; A61P 29/00(2006.01)i

According to International Patent Classification (IPC) or to both national classification and IPC

**B. FIELDS SEARCHED**

Minimum documentation searched (classification system followed by classification symbols)

IPC:C07D 471/-: A61K 31/-; A61P 35/-; A61P 31/-; A61P 19/-; A61P 37/-; A61P 29/-

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; GBTXT; CATXT; CNKI; 万方; STN; WEB OF SCIENCE; 程序性细胞死亡受体-1; 吡啶并嘧啶; 癌症; 肿瘤; 感染; 自身免疫性疾病; 广东东阳光药业; 刘兵; 管鸣宇; 刘璐; 张英俊; 张航; 喻性龙; 潘小光; 席云龙; 李家灿; 赵欣; programmed death receptor-1; PD-1; PD-L1; CD279; pyrid+; pyrimidin+; cancer+; tumor+; tumour+; infect+; autoimmune disease; 式(I), (II)结构, structures of formulas (I) and (11); 具体化合物结构, structures of specific compounds

**C. DOCUMENTS CONSIDERED TO BE RELEVANT**

| Category* | Citation of document, with indication, where appropriate, of the relevant passages | Relevant to claim No. |
|---|---|---|
| X | CN 115611924 A (SHANGHAI HUAHUITUO PHARMACEUTICAL TECHNOLOGY CO., LTD. et al.) 17 January 2023 (2023-01-17) description, page 91 embodiment 63, and claims 1 and 11-14 | 1-3, 5-8, 12-15 |
| A | CN 115677689 A (HANGZHOU HERTZ PHARMACEUTICAL CO., LTD.) 03 February 2023 (2023-02-03) claim 6, compound 002, and claims 11-15 | 1-15 |
| A | CN 110582493 A (INCYTE CORP.) 17 December 2019 (2019-12-17) entire document | 1-15 |
| A | CN 112587666 A (GUANGZHOU MAXINOVEL PHARMACEUTICALS CO., LTD.) 02 April 2021 (2021-04-02) entire document | 1-15 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08.04月2024（08.04.2024）** | **23 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/077994**

**C. DOCUMENTS CONSIDERED TO BE RELEVANT**

| Category* | Citation of document, with indication, where appropriate, of the relevant passages | Relevant to claim No. |
|---|---|---|
| A | CN 114644628 A (GUANGDONG HEC PHARMACEUTICAL CO., LTD.) 21 June 2022 (2022-06-21)<br>entire document | 1-15 |
| A | WO 2022040002 A1 (ALIGOS THERAPEUTICS INC.) 24 February 2022 (2022-02-24)<br>entire document | 1-15 |
| A | CN 112457331 A (LONGWOOD BIOPHARMACEUTICALS CO., LTD.) 09 March 2021 (2021-03-09)<br>entire document | 1-15 |
| A | CN 113248492 A (SHANGHAI HAIYAN PHARMACEUTICAL TECHNOLOGY CO., LTD. et al.) 13 August 2021 (2021-08-13)<br>entire document | 1-15 |

Form PCT/ISA/210 (second sheet) (July 2022)

**INTERNATIONAL SEARCH REPORT**
**Information on patent family members**

International application No.
**PCT/CN2024/077994**

| Patent document cited in search report | Publication date (day/month/year) | Patent family member(s) | Publication date (day/month/year) |
|---|---|---|---|
| CN 115611924 A | 17 January 2023 | None | |
| CN 115677689 A | 03 February 2023 | WO 2023006019 A1 | 02 February 2023 |
| CN 110582493 A | 17 December 2019 | IL 267461 A | 29 August 2019 |
| | | IL 267461 B | 01 December 2022 |
| | | IL 267461 B2 | 01 April 2023 |
| | | AU 2022204591 A1 | 14 July 2022 |
| | | US 2019225601 A1 | 25 July 2019 |
| | | US 10800768 B2 | 13 October 2020 |
| | | AU 2017382870 A1 | 04 July 2019 |
| | | AU 2017382870 B2 | 24 March 2022 |
| | | ECSP 19052302 A | 30 September 2019 |
| | | NI 201900070 A | 11 March 2020 |
| | | US 2022194931 A1 | 23 June 2022 |
| | | US 11787793 B2 | 17 October 2023 |
| | | ES 2934230 T3 | 20 February 2023 |
| | | US 2018179197 A1 | 28 June 2018 |
| | | US 10308644 B2 | 04 June 2019 |
| | | UA 126394 C2 | 28 September 2022 |
| | | CL 2019001744 A1 | 04 October 2019 |
| | | MA 47123 A | 17 March 2021 |
| | | GEP 20227428 B | 25 October 2022 |
| | | IL 295660 A | 01 October 2022 |
| | | MX 2019007416 A | 11 December 2019 |
| | | IL 289511 A | 01 March 2022 |
| | | IL 289511 B1 | 01 March 2023 |
| | | IL 289511 B2 | 01 July 2023 |
| | | CR 20190318 A | 21 October 2019 |
| | | PH 12019501443 A1 | 28 October 2019 |
| | | CO 2019007863 A2 | 01 April 2020 |
| | | MX 2022005225 A | 08 June 2022 |
| | | US 2021107900 A1 | 15 April 2021 |
| | | US 11339149 B2 | 24 May 2022 |
| | | CA 3047986 A1 | 28 June 2018 |
| | | EP 3558985 A1 | 30 October 2019 |
| | | EP 3558985 B1 | 07 September 2022 |
| | | WO 2018119266 A1 | 28 June 2018 |
| | | KR 20190111025 A | 01 October 2019 |
| | | JP 2020504737 A | 13 February 2020 |
| | | JP 7101678 B2 | 15 July 2022 |
| | | TW 201835073 A | 01 October 2018 |
| | | TWI 808955 B | 21 July 2023 |
| | | MY 197635 A | 29 June 2023 |
| | | JP 2022130701 A | 06 September 2022 |
| | | BR 112019012993 A2 | 03 December 2019 |
| | | PE 20191532 A1 | 23 October 2019 |
| CN 112587666 A | 02 April 2021 | None | |
| CN 114644628 A | 21 June 2022 | WO 2022127847 A1 | 23 June 2022 |
| WO 2022040002 A1 | 24 February 2022 | TW 202227451 A | 16 July 2022 |
| | | US 2023065527 A1 | 02 March 2023 |

Form PCT/ISA/210 (patent family annex) (July 2022)

**INTERNATIONAL SEARCH REPORT**
**Information on patent family members**

International application No.
**PCT/CN2024/077994**

| Patent document cited in search report | Publication date (day/month/year) | Patent family member(s) | Publication date (day/month/year) |
|---|---|---|---|
| | | US 11760761 B2 | 19 September 2023 |
| | | AU 2021329259 A1 | 02 March 2023 |
| | | CA 3190154 A1 | 24 February 2022 |
| | | JP 2023539463 A | 14 September 2023 |
| | | KR 20230059801 A | 03 May 2023 |
| | | EP 4185569 A1 | 31 May 2023 |
| CN 112457331 A | 09 March 2021 | None | |
| CN 113248492 A | 13 August 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017066227 A
- WO 2018044963 A


### Non-patent literature cited in the description


- **GREENWALD**. *Annu.Rev. Immunol*, 2005, vol. 23, 515-548
- **OKAZAKI** ; **HONJO**. *Trends Immunol*, 2006 (4), 195-201
- **PARRY et al.** *Mol Cell Biol*, 2005, 9543-9553
- **SHARPE et al.** *Nat Immunol*, 2007, vol. 8, 239-245
- **POSTOW et al.** *J. Clinical Oncol*, 2015, vol. 33, 1-9
- Handbook of Chemistry and Physics,. 1994
- **JACQUES et al.** Enantiomers, Racemates and Resolutions. Wiley Interscience, 1981
- **ROBERT E. GAWLEY** ; **JEFFREY AUBÉ**. Principles of Asymmetric Synthesis. Elsevier, 2012
- **ELIEL, E.L**. Stereochemistry of Carbon Compounds. McGraw-Hill, 1962
- **WILEN, S.H**. Tables of Resolving Agents and Optical Resolutions. Univ. of Notre Dame Press, 1972, 268
- Chiral Separation Techniques: A Practical Approach. Wiley-VCH Verlag GmbH & Co. KGaA, 2007
- **JERRY MARCH**. Advanced Organic Chemistiy. Wiley Interscience
- **L. W. DEADY**. *Syn. Comm.*, 1977, vol. 7, 509-514
- Pro-drugs as Novel Delivery Systems. **T. HIGUCHI** ; **V. STELLA**. Bioreversible Carriers in Drug Design. American Pharmaceutical Association and Pergamon Press, 1987, vol. 14
- **J. RAUTIO et al.** Prodrugs: Design and Clinical Applications. *Nature Review Drug Discovery*, 2008, vol. 7, 255-270
- **S. J. HECKER et al.** Prodrugs of Phosphates and Phosphonates. *Journal of Medicinal Chemistry*, 2008, vol. 51, 2328-2345
- **BERGE et al.** *J. Pharmacol Sci*, 1977, vol. 66, 1-19
- **T. W. GREENE**. Protective Groups in Organic Synthesis. John Wiley & Sons, 1991
- **P. J. KOCIENSKI**. *Protecting Groups, Thieme*, 2005
- IUPAC-IUB Commission on Biochemical Nomenclature. *Biochem.*, 1972, vol. 11, 942-944
- Remington: The Science and Practice of Pharmacy. Lippincott Williams& Wilkins, 2005
- Encyclopedia of Pharmaceutical Technology. Marcel Dekker, 1988